(19) Europäisches Patentamt
European Patent Office
Office européen des brevets

(11) EP 3 126 365 B1

(12) # EUROPEAN PATENT SPECIFICATION

(45) Date of publication and mention of the grant of the patent:
**10.01.2018 Bulletin 2018/02**

(21) Application number: **15713523.7**

(22) Date of filing: **02.04.2015**

(51) Int Cl.:
***C07D 471/22*** (2006.01) ***C07D 498/22*** (2006.01)
***A61K 31/505*** (2006.01) ***A61P 35/00*** (2006.01)

(86) International application number:
**PCT/EP2015/057401**

(87) International publication number:
**WO 2015/150557 (08.10.2015 Gazette 2015/40)**

(54) **MACROCYLIC PYRIDINE DERIVATIVES**
MAKROCYCLISCHE PYRIDINDERIVATE
DÉRIVÉS DE PYRIDINE MACROCYCLIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **03.04.2014 EP 14163442**
**05.09.2014 EP 14183823**

(43) Date of publication of application:
**08.02.2017 Bulletin 2017/06**

(73) Proprietor: **Janssen Pharmaceutica, N.V.**
**2340 Beerse (BE)**

(72) Inventors:
- **DIELS, Gaston, Stanislas, Marcella**
**B-2340 Beerse (BE)**
- **SCHOENTJES, Bruno**
**F-92130 Ile-de-France (FR)**
- **VERSELE, Matthias, Luc, Aimé**
**B-2340 Beerse (BE)**
- **BERTHELOT, Didier, Jean-Claude**
**F-27100 Val de Reuil (FR)**
- **WILLEMS, Marc**
**B-2350 Vosselaar (BE)**
- **VIELLEVOYE, Marcel**
**B-2340 Beerse (BE)**
- **SOMMEN, François, Maria**
**B-2323 Wortel (BE)**
- **WROBLOWSKI, Berthold**
**B-2340 Beerse (BE)**
- **MEERPOEL, Lieven**
**B-2340 Beerse (BE)**

(74) Representative: **Lenaerts, Philip**
**Johnson & Johnson Patent Law Department**
**Turnhoutseweg 30**
**2340 Beerse (BE)**

(56) References cited:
**WO-A1-2009/112439**

- **ANDERS POULSEN ET AL: "Structure-based design of nitrogen-linked macrocyclic kinase inhibitors leading to the clinical candidate SB1317/TG02, a potent inhibitor of cyclin dependant kinases (CDKs), Janus kinase 2 (JAK2), and Fms-like tyrosine kinase-3 (FLT3)", JOURNAL OF MOLECULAR MODELING, SPRINGER, DE, vol. 19, no. 1, 22 July 2012 (2012-07-22), pages 119-130, XP035158034, ISSN: 0948-5023, DOI: 10.1007/S00894-012-1528-7**
- **ASHWINI K. DEVKOTA ET AL: "Investigating the Kinetic Mechanism of Inhibition of Elongation Factor 2 Kinase by NH125: Evidence of a Common in Vitro Artifact", BIOCHEMISTRY, vol. 51, no. 10, 13 March 2012 (2012-03-13), pages 2100-2112, XP055121644, ISSN: 0006-2960, DOI: 10.1021/bi201787p**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 126 365 B1

## Description

Field of the Invention

The present invention relates to substituted macrocylic pyridine derivatives having EF2K inhibitory activity and optionally also Vps34 inhibitory activity. The invention further relates to processes for preparing such compounds, pharmaceutical compositions comprising said compounds as an active ingredient as well as the use of said compounds as a medicament.

Background of the invention

In all eukaryotic cell types, protein elongation is a critical and energetically expensive step in the synthesis of new proteins. The rate of protein elongation is therefore strictly regulated to coordinate the availability of resources (energy, amino acids) with the demand for newly synthesised proteins. Eukaryotic elongation factor 2 (EF2) is essential for protein elongation: its affinity for the ribosome, and hence protein elongation rate, is controlled by its phosphorylation state. Phosphorylation of eEF2 at Threonine 56 by the elongation factor 2 kinase (EF2K or eEF2K) decreases the affinity of EF2 for the ribosome, and reduces protein elongation rates (Browne et al., Eur J Biochem. 2002, 269(22):5360-5368). This regulation is critical under various forms of cellular stress, such as nutrient limitation and hypoxia, or conditions of increased energy expenditure, such as muscle exercise. In addition, local subcellular regulation of EF2 phosphorylation by EF2K at nerve growth cones or at the synapse ensures preferential translation of certain nerve growth factors and neurotransmitters. Dysregulation of EF2 (Thr56) phosphorylation has been associated with several devastating pathologies, including cancer and depression. Tumour cells often experience various forms of stress (hypoxia, nutrient deprivation), and therefore activate eEF2K activity to balance protein elongation rates with the high demand for de novo protein synthesis. Indeed, EF2 is highly phosphoryated in tumour tissue compared to normal tissue as an adaptive response to nutrient limitation (Leprivier et al., Cell 2013, 153(5):1064-1079). Deregulation of this control through inhibition of eEF2K is thought to fatally increase energy expenditure in tumour cells, and represent an anti-tumour strategy through induction of metabolic crisis (Hait et al., Clin Cancer Res. 2006, 12:1961-1965; Jin et al., J Cell Sci. 2007, 120(3):379-83; Leprivier et al., Cell 2013, 153(5):1064-1079). Increased local translation of synaptic proteins such as BDNF (brain-derived neurotrophic factor) plays a critical role in the fast-acting anti-depressant activity of NMDA (N-Methyl-D-aspartic acid) antagonists (such as ketamine); reduced phosphorylation levels of EF2 are thought to be critical to enable BDNF translation, and hence EF2K inhibition has been proposed as a fast-acting anti-depressant therapy (Kavalali et al., Am J Psychiatry 2012, 169(11):1150-1156).

Consistent with its role under hypoxia and starvation, EF2K is activated by direct phosphorylation by AMPK, whereas EF2K is regulated through inhibitory phosphorylation by growth and cell cycle kinases, such as S6K and CDK2. In addition, EF2K is a Ca2+/calmodulin-dependent kinase; this regulation may be key for the synaptic regulation of EF2K. (Browne et al., Eur J Biochem. 2002, 269(22):5360-5368). EF2K is an atypical kinase: the primary sequence of its catalytic domain is only remotely related to that of canonical kinases, such as serine/threonine kinases, tyrosine kinases, or lipid kinases. Compounds with EF2K inhibitory activity, may prevent the stress-induced phosphorylation of eEF2 in cells and in xenografted tumours in mice.

In addition to strict regulation of protein synthesis under cellular stress as described above, many cell types utilize autophagy as a recycling mechanism to cope with low nutrient availability, hypoxia and other forms of cellular stress. Autophagy is a catabolic process, in which cytosolic content, including proteins, protein aggregates and entire organelles are engulfed in vesicles (autophagosomes) which fuse to lysosomes to enable degradation of macromolecules to recuperate building blocks (amino acids, fatty acids, nucleotides) and energy (Hait et al., Clin Cancer Res. 2006, 12:1961-1965). The double membrane of autophagosomes critically consists of phosphatidylinositol-(3)-phosphate [PI(3)P], the product of the class III PI3K, Vps34 (also called PIK3C3). Vps34, and the adaptor protein, Beclin1, are both essential for autophagy in mammalian cells (Amaravadi et al., Clin Cancer Res. 2011, 17:654-666). Autophagy is upregulated in tumors, and inhibition of autophagy using the lysosomotropic agent, chloroquine (which inhibits the fusion of lysosomes to autophagosomes), or RNAi approaches can impair tumorigenesis. Moreover, inhibition of autophagy has been shown to sensitize tumors to chemotherapeutic agents, radiation, proteasome inhibitors, and kinase inhibitors (such as the receptor tyrosine kinases EGFR, class I PI3K, mTOR, and Akt) (Amaravadi et al., Clin Cancer Res. 2011, 17:654-666). The clinical utility of chloroquine in treating patients with malaria, rheumatoid arthritis, lupus and HIV suggest potential utility of autophagy inhibitors for those pathologies as well (Ben-Zvi et al., Clin Rev Allergy Immunol. 2012, 42(2):145-53).

Inhibition of the class III PI3K, Vps34, may inhibit autophagy in cancer cells under stress. Moreover it was found that cancer cells, partially deficient in autophagy through knockdown of Beclin, are especially sensitive to Vps34 inhibition, suggesting that autophagy-deficient tumors (e.g. because of mono-allelic deletion of beclin1, as frequently found in breast, ovarian and prostate cancer, or other genetic lesions (Maiuri et al., Cell Death Differ. 2009, 16(1):87-93) may be

most susceptible to Vps34 inhibition.

WO 2009/112439 describes 4-aryl-2-anilino-pyrimidines as PLK kinase inhibitors.

There is a strong need for novel compounds which have EF2K inhibitory activity and optionally also have Vps34 inhibitory activity, thereby opening new avenues for the treatment of cancer. It is an object of the present invention to overcome or ameliorate at least one of the disadvantages of the prior art, or to provide a useful alternative. It is accordingly an object of the present invention to provide such novel compounds.

Summary of the invention

It has been found that the compounds of the present invention have EF2K inhibitory activity and optionally also have Vps34 inhibitory activity. The compounds according to the invention and the pharmaceutical compositions comprising such compounds may be useful for treating or preventing, in particular treating, diseases such as cancer, depression, and memory and learning disorders. In particular, the compounds according to the present invention and the pharmaceutical compositions thereof may be useful in the treatment of a haematological malignancy or solid tumour. In a specific embodiment said solid tumour is selected from the group consisting of glioblastoma, medulloblastoma, prostate cancer, breast cancer, ovarian cancer and colorectal cancer, and the like.

This invention concerns compounds of Formula (I)

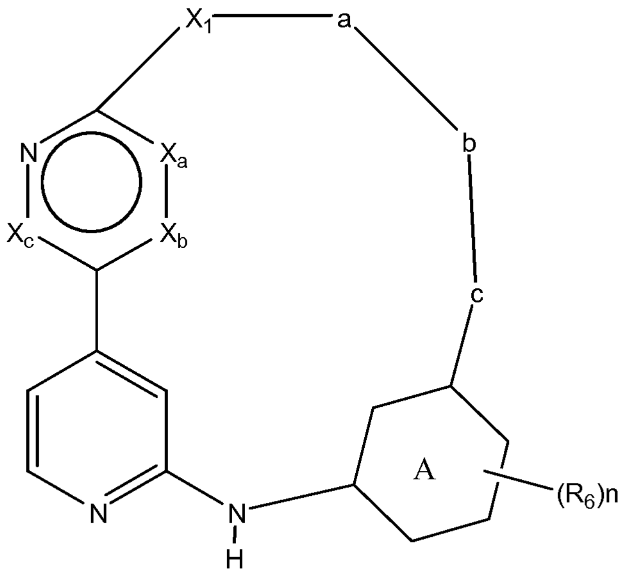

(I)

tautomers and stereochemically isomeric forms thereof, wherein

$X_a$, $X_b$ and $X_c$ each independently represent CH or N;

$-X_1-$ represents $-(CHR_{12})_s-NR_1-X_e-C_{1-4}$alkanediyl$-(SO_2)_{p3}-$ or

$-(CH_2)_s-O-X_e-C_{1-4}$alkanediyl$-(SO_2)_{p3}-$; wherein each of said $C_{1-4}$alkanediyl moieties are optionally substituted with hydroxyl or hydroxy$C_{1-4}$alkyl;

$-X_e-$ represents $-C(R_2)_2-$ or $-C(=O)-$;

a represents $-NR_4-C(=O)-[C(R_{5b})_2]_r-$ or $-NR_4-C(R_{5b})_2-C(=O)-$ or $-C(=O)-NR_4-C(R_{5b})_2-$;

b represents

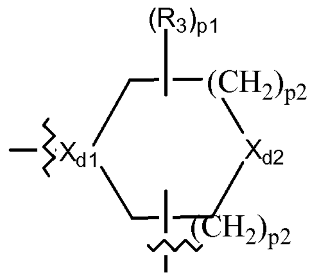

,

wherein said b ring may contain extra bonds to form a bridged ring system selected from 2,5-diazabicyclo[2.2.2]octanyl, 3,8-diazabicyclo[3.2.1]octanyl, 3,6-diazabicyclo[3.1.1]heptanyl, 3,9-diazabicyclo[3.3.1]nonyl;

$X_{d1}$ represents CH or N;

$X_{d2}$ represents $CH_2$ or NH;

provided that at least one of $X_{d1}$ and $X_{d2}$ represents nitrogen;

c represents a bond, $-[C(R_{5a})_2]_m-$, -C(=O)-, -O-, $-NR_{5a'}-$, $-SO_2-$, or -SO-;

ring

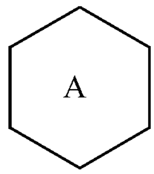

represents phenyl or pyridyl;

$R_1$ represents hydrogen, $C_{1-4}$alkyl, $C_{2-4}$alkenyl, $C_{2-4}$alkynyl, cyano$C_{1-4}$alkyl, -C(=O)-$C_{1-4}$alkyl, -C(=O)-halo$C_{1-4}$alkyl, hydroxy$C_{1-4}$alkyl, halo$C_{1-4}$alkyl, $C_{1-4}$alkyloxy$C_{1-4}$alkyl, halo$C_{1-4}$alkyloxy$C_{1-4}$alkyl, -C(=O)$NR_7R_8$, $-SO_2-NR_7R_8$, $-SO_2-R_9$, $R_{11}$, $C_{1-4}$alkyl substituted with $R_{11}$, -C(=O)-$R_{11}$, or -C(=O)-$C_{1-4}$alkyl-$R_{11}$;

each $R_2$ independently represents hydrogen, $C_{1-4}$alkyl, $C_{1-4}$alkyl substituted with $C_{3-6}$cycloalkyl, hydroxy$C_{1-4}$alkyl, $C_{1-4}$alkyloxy$C_{1-4}$alkyl, carboxyl, -C(=O)-O-$C_{1-4}$alkyl wherein $C_{1-4}$alkyl is optionally substituted with $C_{1-4}$alkyloxy, -C(=O)-$NH_2$, -C(=O)-NH($C_{1-4}$alkyl) wherein $C_{1-4}$alkyl is optionally substituted with $C_{1-4}$alkyloxy, or -C(=O)-N($C_{1-4}$alkyl)$_2$ wherein each $C_{1-4}$alkyl is optionally substituted with $C_{1-4}$alkyloxy;

or $R_1$ and one $R_2$ are taken together to form $C_{1-4}$alkanediyl or $C_{2-4}$alkenediyl, each of said $C_{1-4}$alkanediyl and $C_{2-4}$alkenediyl optionally being substituted with 1 to 4 substituents each independently selected from hydroxyl, oxo, halo, cyano, $N_3$, hydroxy$C_{1-4}$alkyl, $-NR_7R_8$, $-SO_2-NR_7R_8$, $-NH-SO_2-NR_7R_8$, $-C(=O)-NR_7R_8$, or $-NH-C(=O)-NR_7R_8$;

or $R_1$ and $R_{12}$ are taken together to form $C_{1-4}$alkanediyl or $C_{2-4}$alkenediyl, each of said $C_{1-4}$alkanediyl and $C_{2-4}$alkenediyl optionally being substituted with 1 to 4 substituents each independently selected from hydroxyl, oxo, halo, cyano, $N_3$, hydroxy$C_{1-4}$alkyl, - $NR_7R_8$, $-SO_2-NR_7R_8$, $-NH-SO_2-NR_7R_8$, $-C(=O)-NR_7R_8$, or $-NH-C(=O)-NR_7R_8$;

each $R_3$ independently represents hydrogen; oxo; hydroxyl; carboxyl; $-NR_{3a}R_{3b}$; $-C(=O)-NR_{3a}R_{3b}$; hydroxy$C_{1-4}$alkyl; halo$C_{1-4}$alkyl; -(C=O)-$C_{1-4}$alkyl;

-C(=O)-O-$C_{1-4}$alkyl wherein said $C_{1-4}$alkyl may optionally be substituted with phenyl; $C_{1-4}$alkyl optionally substituted with cyano, carboxyl, $C_{1-4}$alkyloxy,

-C(=O)-O-$C_{1-4}$alkyl, -O-C(=O)-$C_{1-4}$alkyl, $-NR_{3e}R_{3f}$, $-C(=O)-NR_{3e}R_{3f}$, $-SO_2-NR_{3e}R_{3f}$, Q, - C(=O)-Q, or $-SO_2$-Q; hydroxy$C_{1-4}$alkyloxy$C_{1-4}$alkyl; $C_{1-4}$alkyloxyhydroxy$C_{1-4}$alkyl; hydroxy$C_{1-4}$alkyloxyhydroxy$C_{1-4}$alkyl; or

$C_{1-4}$alkyloxy$C_{1-4}$alkyl optionally substituted with cyano, carboxyl, $C_{1-4}$alkyloxy, -C(=O)-O-$C_{1-4}$alkyl, -O-C(=O)-$C_{1-4}$alkyl, -$NR_{3e}R_{3f}$, - C(=O)-$NR_{3e}R_{3f}$, -$SO_2$-$NR_{3e}R_{3f}$, $R_{10}$, -C(=O)-$R_{10}$, or -$SO_2$-$R_{10}$; or

two $R_3$ substituents attached to the same carbon atom are taken together to form $C_{2-5}$alkanediyl or -$(CH_2)_p$-O-$(CH_2)_p$-;

each $R_{3a}$ and $R_{3b}$ independently represent hydrogen; -(C=O)-$C_{1-4}$alkyl; -$SO_2$-$NR_{3e}R_{3d}$; or $C_{1-4}$alkyl optionally substituted with $C_{1-4}$alkyloxy; or

$R_{3a}$ and $R_{3b}$ are taken together with the nitrogen to which they are attached to form a 4 to 7 membered saturated monocyclic heterocyclic ring which optionally contains 1 or 2 further heteroatoms selected from N, O or $SO_2$, said heterocyclic ring being optionally substituted with 1 to 4 substituents each independently selected from $C_{1-4}$alkyl, halo, hydroxyl, or halo$C_{1-4}$alkyl;

each $R_{3c}$ and $R_{3d}$ independently represent hydrogen, $C_{1-4}$alkyl or -(C=O)-$C_{1-4}$alkyl; or $R_{3c}$ and $R_{3d}$ are taken together with the nitrogen to which they are attached to form a 4 to 7 membered saturated monocyclic heterocyclic ring which optionally contains 1 or 2 further heteroatoms selected from N, O or $SO_2$, said heterocyclic ring being optionally substituted with 1 to 4 substituents each independently selected from $C_{1-4}$alkyl, halo, hydroxyl, or halo$C_{1-4}$alkyl;

each $R_{3e}$ and $R_{3f}$ independently represent hydrogen, $C_{1-4}$alkyl optionally substituted with $C_{1-4}$alkyloxy, -(C=O)-$C_{1-4}$alkyl, or -$SO_2$-$NR_{3e}R_{3d}$;

$R_4$ represents hydrogen, $C_{1-4}$alkyl or $C_{1-4}$alkyloxy$C_{1-4}$alkyl;

each $R_{5a}$ independently represents hydrogen or $C_{1-4}$alkyl; or

two $R_{5a}$ substituents attached to the same carbon atom are taken together to form $C_{2-5}$alkanediyl or -$(CH_2)_p$-O-$(CH_2)_p$-;

$R_{5a'}$ represents hydrogen or $C_{1-4}$alkyl;

each $R_{5b}$ independently represents hydrogen; $C_{1-4}$alkyl; $C_{1-4}$alkyl substituted with $NR_{5b1}R_{5b2}$; $C_{1-4}$alkyloxy$C_{1-4}$alkyl; hydroxy$C_{1-4}$alkyl; hydroxyl; $C_{3-6}$cycloalkyl; or phenyl optionally substituted with $C_{1-4}$alkyl, halo, hydroxyl or $C_{1-4}$alkyloxy; or

two $R_{5b}$ substituents attached to the same carbon atom are taken together to form $C_{2-5}$alkanediyl or -$(CH_2)_p$-O-$(CH_2)_p$-;

$R_{5b1}$ and $R_{5b2}$ independently represent hydrogen, $C_{1-4}$alkyl optionally substituted with $C_{1-4}$alkyloxy, -(C=O)-$C_{1-4}$alkyl, or -$SO_2$-$NR_{5b3}R_{5b4}$;

$R_{5b3}$ and $R_{5b4}$ independently represent hydrogen, $C_{1-4}$alkyl or -(C=O)-$C_{1-4}$alkyl; or

$R_{5b3}$ and $R_{5b4}$ are taken together with the nitrogen to which they are attached to form a 4 to 7 membered saturated monocyclic heterocyclic ring which optionally contains 1 or 2 further heteroatoms selected from N, O or $SO_2$, said heterocyclic ring being optionally substituted with 1 to 4 substituents each independently selected from $C_{1-4}$alkyl, halo, hydroxyl, or halo$C_{1-4}$alkyl;

each $R_6$ independently represents hydrogen, halo, hydroxyl, carboxyl, cyano, $C_{1-4}$alkyl, $C_{1-4}$alkyloxy$C_{1-4}$alkyl, hydroxy$C_{1-4}$alkyl, halo$C_{1-4}$alkyl, $C_{2-4}$alkenyl, $C_{2-4}$alkynyl, -$NR_{6a}R_{6b}$, or -C(=O)$NR_{6a}R_{6b}$;

each $R_{6a}$ and $R_{6b}$ independently represent hydrogen or $C_{1-4}$alkyl;

each $R_7$ and $R_8$ independently represent hydrogen, $C_{1-4}$alkyl, halo$C_{1-4}$alkyl, or $C_{3-6}$cycloalkyl; or

$R_7$ and $R_8$ are taken together with the nitrogen to which they are attached to form a 4 to 7 membered saturated monocyclic heterocyclic ring which optionally contains 1 further heteroatom selected from N, O or $SO_2$, said heterocyclic ring being optionally substituted with 1 to 4 substituents each independently selected from $C_{1-4}$alkyl, halo, hydroxyl, or halo$C_{1-4}$alkyl;

$R_9$ represents $C_{1-4}$alkyl, halo$C_{1-4}$alkyl, or $C_{3-6}$cycloalkyl;

each $R_{10}$ independently represents a 4 to 7 membered saturated monocyclic heterocyclic ring containing up to 2 heteroatoms selected from N, O or $SO_2$, said heterocyclic ring being optionally substituted with 1 to 4 substituents each independently selected from $C_{1-4}$alkyl, halo, hydroxyl or halo$C_{1-4}$alkyl;

each $R_{11}$ independently represents $C_{3-6}$cycloalkyl, phenyl, or a 4 to 7 membered monocyclic heterocyclic ring containing up to 3 heteroatoms selected from N, O or $SO_2$, said heterocyclic ring being optionally substituted with 1 to 4 substituents each independently selected from $C_{1-4}$alkyl, halo, hydroxyl, or halo$C_{1-4}$alkyl;

each $R_{12}$ independently represents hydrogen or $C_{1-4}$alkyl;

Q represents a 4 to 7 membered saturated monocyclic heterocyclic ring containing up to 3 heteroatoms selected from N, O or $SO_2$, said heterocyclic ring being optionally substituted with 1 to 4 substituents each independently selected from $C_{1-4}$alkyl, halo, hydroxyl or halo$C_{1-4}$alkyl;

n represents an integer of value 1 or 2;

m represents an integer of value 1 or 2 ;

p represents an integer of value 1 or 2;

p1 represents an integer of value 1 or 2;

each p2 independently represents an integer of value 0, 1 or 2;

r represents an integer of value 0, 1 or 2;

each $p_3$ independently represents an integer of value 0 or 1;

each s independently represents an integer of value 0, 1 or 2;

and the pharmaceutically acceptable addition salts, and the solvates thereof.

The present invention also concerns methods for the preparation of compounds of the present invention and pharmaceutical compositions comprising them.

The compounds of the present invention were found to have EF2K inhibitory activity and optionally also have Vps34 inhibitory activity. Therefore the compounds of the present invention may be useful in the treatment or prevention, in particular in the treatment, of diseases such as cancer, depression, neuroplasticity (synaptic plasticity and non-synaptic plasticity), and memory and learning disorders; in particular diseases such as cancer, depression, and memory and learning disorders. In particular, the compounds according to the present invention and the pharmaceutical compositions thereof may be useful in the treatment of a haematological malignancy or solid tumour. In a specific embodiment said solid tumour is selected from the group consisting of glioblastoma, medulloblastoma, prostate cancer, breast cancer, ovarian cancer and colorectal cancer, and the like.

In view of the aforementioned pharmacology of the compounds of Formula (I) and pharmaceutically acceptable addition salts, and solvates thereof, it follows that they may be suitable for use as a medicament.

In particular the compounds of Formula (I) and pharmaceutically acceptable addition salts, and solvates thereof, may be suitable in the treatment or prevention, in particular in the treatment, of cancer.

The present invention also concerns the use of compounds of Formula (I) and pharmaceutically acceptable addition salts, and solvates thereof, for the manufacture of a medicament for the treatment or prevention, in particular treatment, of diseases such as cancer, depression, neuroplasticity (synaptic plasticity and non-synaptic plasticity), and memory and learning disorders; in particular diseases such as cancer, depression, and memory and learning disorders.

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Detailed description

When describing the compounds of the invention, the terms used are to be construed in accordance with the following definitions, unless a context dictates otherwise.

Combinations of substituents and/or variables are permissible only if such combinations result in chemically stable compounds. "Stable compound" is meant to indicate a compound that is sufficiently robust to survive isolation to a useful degree of purity from a reaction mixture, and formulation into a therapeutic agent.

When any variable occurs more than one time in any constituent or in any Formula (e.g. Formula (I)), its definition in each occurence is independent of its definition at every other occurrence.

Whenever a radical or group is defined as "optionally substituted" in the present invention, it is meant that said radical or group is unsubstituted or is substituted.

Lines drawn from substituents into ring systems indicate that the bond may be attached to any of the suitable ring atoms.

Whenever the term "substituted with 1 to 4 substituents" is used in the present invention, it is meant, to indicate that from 1 to 4 hydrogens, in particular from 1 to 3 hydrogens, preferably 1 or 2 hydrogens, more preferably 1 hydrogen, on the atom or radical indicated in the expression using "substituted" are replaced with a selection from the indicated group, provided that the normal valency is not exceeded, and that the substitution results in a chemically stable compound, i.e. a compound that is sufficiently robust to survive isolation to a useful degree of purity from a reaction mixture, and formulation into a therapeutic agent.

Whenever the term "substituted with" without an indication of the number of substituents is used in the present invention, it is meant, unless otherwise is indicated or is clear from the context, to indicate that one 1 hydrogen, on the atom or radical indicated in the expression using "substituted" is replaced with a substituent from the indicated group, provided that the substitution results in a chemically stable compound, i.e. a compound that is sufficiently robust to survive isolation to a useful degree of purity from a reaction mixture, and formulation into a therapeutic agent. For example "$C_{1-4}$alkyl substituted with cyano" means a $C_{1-4}$alkyl group substituted with one cyano. "$C_{1-4}$alkyl optionally substituted with cyano" means unsubstituted $C_{1-4}$alkyl or $C_{1-4}$alkyl substituted with one cyano.

The prefix "$C_{x-y}$" (where x and y are integers) as used herein refers to the number of carbon atoms in a given group. Thus, a $C_{1-4}$alkyl group contains from 1 to 4 carbon atoms, a $C_{3-6}$cycloalkyl group contains from 3 to 6 carbon atoms, a $C_{1-4}$alkyloxy group contains from 1 to 4 carbon atoms, and so on.

The term "halo" as a group or part of a group is generic for fluoro, chloro, bromo, iodo unless otherwise is indicated or is clear from the context.

The term "$C_{1-4}$alkyl" as a group or part of a group refers to a hydrocarbyl radical of Formula $C_nH_{2n+1}$ wherein n is a number ranging from 1 to 4. $C_{1-4}$alkyl groups comprise from 1 to 4 carbon atoms, preferably from 1 to 3 carbon atoms, more preferably 1 to 2 carbon atoms. $C_{1-4}$alkyl groups may be linear or branched and may be substituted as indicated herein. When a subscript is used herein following a carbon atom, the subscript refers to the number of carbon atoms that the named group may contain.

$C_{1-4}$alkyl includes all linear, or branched alkyl groups with between 1 and 4 carbon atoms, and thus includes methyl, ethyl, n-propyl, i-propyl, 2-methyl-ethyl, butyl and its isomers (e.g. n-butyl, isobutyl and *tert*-butyl), and the like.

The term "$C_{1-4}$alkyloxy" as a group or part of a group refers to a radical having the Formula $-OR^c$ wherein $R^c$ is $C_{1-4}$alkyl. Non-limiting examples of suitable $C_{1-4}$alkyloxy include methyloxy (also methoxy), ethyloxy (also ethoxy), propyloxy, isopropyloxy, butyloxy, isobutyloxy, sec-butyloxy and tert-butyloxy.

The term "$C_{3-6}$cycloalkyl" alone or in combination, refers to a cyclic saturated hydrocarbon radical having from 3 to 6 carbon atoms. Non-limiting examples of suitable $C_{3-6}$cycloalkyl include cyclopropyl, cyclobutyl, cyclopentyl and cyclohexyl.

The term 'hydroxy$C_{1-4}$alkyl' as used herein as a group or part of a group refers to a $C_{1-4}$alkyl group as defined herein wherein one or more than one hydrogen atom is replaced with a hydroxyl group. The term 'hydroxy$C_{1-4}$alkyl' therefore includes monohydroxy$C_{1-4}$alkyl and also polyhydroxy$C_{1-4}$alkyl. There may be one, two, three or more hydrogen atoms replaced with a hydroxyl group, so the hydroxy$C_{1-4}$alkyl may have one, two, three or more hydroxyl groups. Examples of such groups include hydroxymethyl, hydroxyethyl, hydroxypropyl and the like.

In a particular embodiment 'hydroxy$C_{1-4}$alkyl' is limited to monohydroxy$C_{1-4}$alkyl.

The term 'hydroxy$C_{1-4}$alkyloxy' as used herein as a group or part of a group refers to a hydroxy$C_{1-4}$alkyl-O- group wherein "hydroxy$C_{1-4}$alkyl" is as defined before.

The term 'hydroxy$C_{1-4}$alkyloxy$C_{1-4}$alkyl' as used herein as a group or part of a group refers to a hydroxy$C_{1-4}$alkyl-O-$C_{1-4}$alkyl- group wherein "hydroxy$C_{1-4}$alkyl" and "$C_{1-4}$alkyl" are as defined before.

The term '$C_{1-4}$alkyloxyhydroxy$C_{1-4}$alkyl' as used herein as a group or part of a group refers to a $C_{1-4}$alkyl-O-hydroxy$C_{1-4}$alkyl- group wherein "hydroxy$C_{1-4}$alkyl" and "$C_{1-4}$alkyl" are as defined before.

The term 'hydroxy$C_{1-4}$alkyloxyhydroxy$C_{1-4}$alkyl' as used herein as a group or part of a group refers to a hydroxy$C_{1-4}$alkyl-O-hydroxy$C_{1-4}$alkyl- group wherein "hydroxy$C_{1-4}$alkyl" is as defined before.

The term 'halo$C_{1-4}$alkyl' as used herein as a group or part of a group refers to a $C_{1-4}$alkyl group as defined herein wherein one or more than one hydrogen atom is replaced with a halogen. The term 'halo$C_{1-4}$alkyl' therefore includes monohalo$C_{1-4}$alkyl and also polyhalo$C_{1-4}$alkyl. There may be one, two, three or more hydrogen atoms replaced with a halogen, so the halo$C_{1-4}$alkyl may have one, two, three or more halogens. Examples of such groups include fluoroethyl, fluoromethyl, trifluoromethyl or trifluoroethyl and the like.

The term "cyano$C_{1-4}$alkyl" as used herein refers to a $C_{1-4}$alkyl group as defined herein which is substituted with one cyano group.

The term '$C_{1-4}$alkoxy$C_{1-4}$alkyl' as used herein as a group or part of a group refers to a $C_{1-4}$alkyl-O-$C_{1-4}$alkyl group wherein $C_{1-4}$alkyl is as defined herein. Examples of such groups include methoxyethyl, ethoxyethyl, propoxymethyl, butoxypropyl, and the like.

The term 'halo$C_{1-4}$alkyloxy' as used herein as a group or part of a group refers to a -O-$C_{1-4}$alkyl group as defined herein wherein one or more than one hydrogen atom is replaced with a halogen. The term 'halo$C_{1-4}$alkyloxy' therefore include monohalo$C_{1-4}$alkyloxy and also polyhalo$C_{1-4}$alkyloxy. There may be one, two, three or more hydrogen atoms replaced with a halogen, so the halo$C_{1-4}$alkyloxy may have one, two, three or more halogens. Examples of such groups include 1-fluoroethyloxy, 2-fluoroethyloxy, difluoromethoxy or trifluoromethoxy and the like.

The term 'halo$C_{1-4}$alkyloxy$C_{1-4}$alkyl' as used herein as a group or part of a group means $C_{1-4}$alkyl substituted with one halo$C_{1-4}$alkyloxy. The term 'halo$C_{1-4}$alkyloxy$C_{1-4}$alkyl' therefore refers to a halo$C_{1-4}$alkyloxy-$C_{1-4}$alkyl- group wherein halo$C_{1-4}$alkyloxy and $C_{1-4}$alkyl are as defined above. Examples of such groups include 1-fluoroethyloxymethyl, 2-fluoroethyloxymethyl, 2-(2,2,2-trifluoroethoxy)-ethyl and the like.

The term "$C_{2-4}$alkenyl" as used herein as a group or part of a group refers to a linear or branched hydrocarbon group containing from 2 to 4 carbon atoms and containing a carbon carbon double bond such as, but not limited to, ethenyl, propenyl, butenyl, and the like.

The term "$C_{2-4}$alkynyl" as used herein as a group or part of a group refers to a linear or branched hydrocarbon group having from 2 to 4 carbon atoms and containing a carbon carbon triple bond.

Examples of 4 to 7 membered saturated monocyclic heterocyclic rings containing up to 2 heteroatoms selected from N, O or $SO_2$ (e.g. in the definition of $R_{10}$), include, but are not limited to, morpholinyl, piperidinyl, tetrahydropyranyl, tetrahydrofuranyl, and the like.

4 to 7 membered monocyclic heterocyclic rings containing up to 3 heteroatoms selected from N, O or $SO_2$ (e.g. in the definition of $R_{11}$), include both aromatic and non-aromatic ring systems. This includes unsaturated, partially saturated and saturated heterocyclic ring systems. Examples include, but are not limited to, pyridinyl, pyrimidinyl, morpholinyl, piperidinyl, tetrahydropyranyl, tetrahydrofuranyl, and the like.

The term "$C_{1-4}$alkanediyl" as a group or part of a group defines bivalent straight or branched chained saturated hydrocarbon radicals having from 1 to 4 carbon atoms such as, for example, methylene or methanediyl, ethan-1,2-diyl, ethan-1,1-diyl or ethylidene, propan-1,3-diyl, propan-1,2-diyl, butan-1,4-diyl, and the like.

The term "$C_{2-5}$alkanediyl" as a group or part of a group defines bivalent straight or branched chained saturated hydrocarbon radicals having from 2 to 5 carbon atoms such as, for example, ethan-1,2-diyl, ethan-1,1-diyl or ethylidene, propan-1,3-diyl, propan-1,2-diyl, butan-1,4-diyl, pentan-1,5-diyl, pentan-1,1-diyl, 2-methylbutan-1,4-diyl, and the like.

The term "$C_{2-4}$alkenediyl" as a group or part of a group defines straight or branched chain bivalent hydrocarbon radicals having from 2 to 4 carbon atoms and having a double bond such as 1,2-ethenediyl, 1,3-propenediyl, 1,4-butenediyl, and the like.

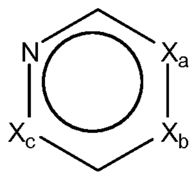

is an alternative representation for

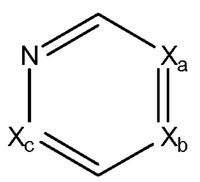

The bonds via which e.g. ring b is attached to the remainder of the molecule are indicated as:

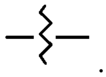

Whenever ring b is substituted with one or two $R_3$ substituents, those $R_3$ substituents may replace any hydrogen atom bound to a carbon or nitrogen atom in ring b, including atoms of the bridge, including NH and CH groups in the definition of $X_{d2}$, and including CH groups in the definition of $X_{d1}$. When two $R_3$ substituents are present, these may be present on the same or different atoms. For instance when $X_{d2}$ represents NH, then the $R_3$ substituent may be present on said nitrogen atom whenever possible. In said case, $X_{d2}$ represents $NR_3$. Or for instance, when $X_{d1}$ or $X_{d2}$ represent a carbon atom, then the $R_3$ substituent may be present on said carbon atom. In said case, $X_{d1}$ may represent $CR_3$ and $X_{d2}$ may represent $CHR_3$ or $C(R_3)_2$. Or for instance, when p2 is other than 0, the $R_3$ substituent may be present on any of the carbon atom represented by $(CH_2)_{p2}$.

Unless otherwise is indicated or is clear from the context, ring b can be attached to variable 'a' via replacement of a hydrogen atom on any carbon or nitrogen atom in ring b, including carbon and nitrogen atoms in the definition of $X_{d2}$.

In a particular embodiment, in the 'b substituent', the linker with the 'a substituent' is present on $X_{d2}$ or is present on a carbon atom in the alpha position of $X_{d2}$.

In a particular embodiment, in the 'b substituent', the linker with the 'a substituent' is present on $X_{d2}$.

In the present invention the b ring is linked to the remainder of the molecule as follows:

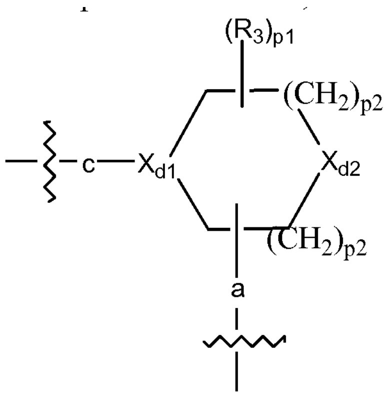

In the present invention, the a linker (-a-) is linked to the remainder of the molecule as depicted below:

- $X_1$-$NR_4$-C(=O)-$[C(R_{5b})_2]_r$-b-; -$X_1$-$NR_4$-$C(R_{5b})_2$-C(=O)-b-; -$X_1$-C(=O)- $NR_4$-$C(R_{5b})_2$-b-. In the present invention, $X_1$ being -$(CHR_{12})_s$-$NR_1$-$X_e$-$C_{1-4}$alkanediyl-$(SO_2)_{p3}$- or
- $(CH_2)_s$-O-$X_e$-$C_{1-4}$alkanediyl-$(SO_2)_{p3}$- is attached to the remainder of the molecule as follows:

$$\overset{\alpha}{-}(CHR_{12})_s\text{-}NR_1\text{-}X_e\text{-}C_{1-4}\text{alkanediyl-}(SO_2)_{p3}\overset{\beta}{-} \qquad (X_1'),$$

or

$$\overset{\alpha}{-}(CH_2)_s\text{-}O\text{-}X_e\text{-}C_{1-4}\text{alkanediyl-}(SO_2)_{p3}\overset{\beta}{-} \qquad (X_1'')$$

is attached with the carbon atom, the nitrogen atom (when s is 0 in Formula ($X_1$')) or the oxygen atom (when s is 0 in Formula ($X_1$")) in position $\alpha$ to the ring containing $X_a$, $X_b$ and $X_c$, and is attached with the group in position $\beta$($(SO_2)_{p3}$ or $C_{1-4}$alkanediyl (when p3 is 0)) to variable a. In both $X_1$ Formulas $C_{1-4}$alkanediyl is optionally substituted according to the scope.

For example when -$X_1$- represents-$(CHR_{12})_s$-$NR_1$-$X_e$-$C_{1-4}$alkanediyl-$(SO_2)_{p3}$-, a compound of Formula (I') is formed:

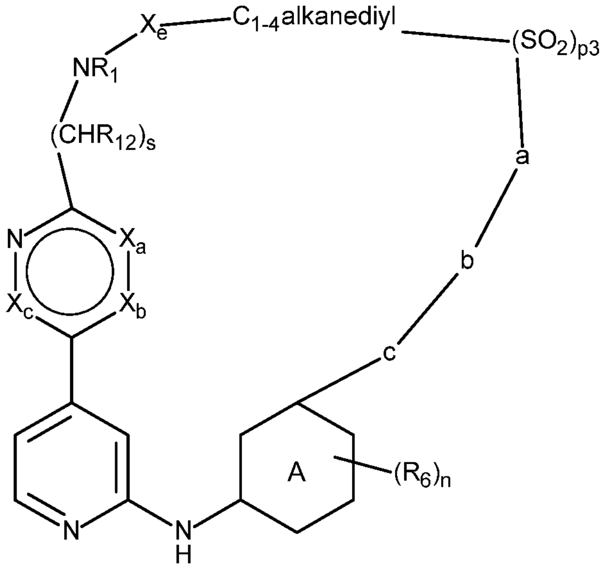

(I')

.

The term "subject" as used herein, refers to an animal, preferably a mammal (e.g. cat, dog, primate or human), more preferably a human, who is or has been the object of treatment, observation or experiment.

The term "therapeutically effective amount" as used herein, means that amount of active compound or pharmaceutical agent that elicits the biological or medicinal response in a tissue system, animal or human that is being sought by a researcher, veterinarian, medicinal doctor or other clinician, which includes alleviation or reversal of the symptoms of the disease or disorder being treated.

The term "composition" is intended to encompass a product comprising the specified ingredients in the specified amounts, as well as any product which results, directly or indirectly, from combinations of the specified ingredients in the specified amounts.

The term "treatment", as used herein, is intended to refer to all processes wherein there may be a slowing, interrupting, arresting or stopping of the progression of a disease, but does not necessarily indicate a total elimination of all symptoms.

The term "compounds of the invention" as used herein, is meant to include the compounds of Formula (I) and pharmaceutically acceptable addition salts, and solvates thereof.

As used herein, any chemical Formula with bonds shown only as solid lines and not as solid wedged or hashed wedged bonds, or otherwise indicated as having a particular configuration (e.g. *R, S*) around one or more atoms, contemplates each possible stereoisomer, or mixture of two or more stereoisomers.

Whenever one of the ring systems, is substituted with one or more substituents, those substituents may replace any hydrogen atom bound to a carbon or nitrogen atom of the ring system.

Hereinbefore and hereinafter, the term "compound of Formula (I)" is meant to include the stereoisomers thereof and the tautomeric forms thereof.

The terms "stereoisomers", "stereoisomeric forms" or "stereochemically isomeric forms" hereinbefore or hereinafter are used interchangeably.

The invention includes all stereoisomers of the compounds of the invention either as a pure stereoisomer or as a mixture of two or more stereoisomers.

Enantiomers are stereoisomers that are non-superimposable mirror images of each other. A 1:1 mixture of a pair of enantiomers is a racemate or racemic mixture.

Atropisomers (or atropoisomers) are stereoisomers which have a particular spatial configuration, resulting from a restricted rotation about a single bond, due to large steric hindrance. For the compounds of the present invention this may be caused by the linker ($-X_1$-a-b-c-) of the macrocycle. All atropisomeric forms of the compounds of Formula (I) are intended to be included within the scope of the present invention.

Diastereomers (or diastereoisomers) are stereoisomers that are not enantiomers, i.e. they are not related as mirror images. If a compound contains a double bond, the substituents may be in the E or the Z configuration. Substituents on bivalent cyclic (partially) saturated radicals may have either the cis- or trans-configuration; for example if a compound contains a disubstituted cycloalkyl group, the substituents may be in the cis or trans configuration. Therefore, the invention includes enantiomers, atropisomers, diastereomers, racemates, E isomers, Z isomers, cis isomers, trans isomers and mixtures thereof, whenever chemically possible.

The meaning of all those terms, i.e. enantiomers, atropisomers, diastereomers, racemates, E isomers, Z isomers, cis isomers, trans isomers and mixtures thereof are known to the skilled person.

The absolute configuration is specified according to the Cahn-Ingold-Prelog system. The configuration at an asymmetric atom is specified by either R or S. Resolved stereoisomers whose absolute configuration is not known can be designated by (+) or (-) depending on the direction in which they rotate plane polarized light. For instance, resolved enantiomers whose absolute configuration is not known can be designated by (+) or (-) depending on the direction in which they rotate plane polarized light.

When a specific stereoisomer is identified, this means that said stereoisomer is substantially free, i.e. associated with less than 50%, preferably less than 20%, more preferably less than 10%, even more preferably less than 5%, in particular less than 2% and most preferably less than 1%, of the other stereoisomers. Thus, when a compound of Formula (I) is for instance specified as (R), this means that the compound is substantially free of the (S) isomer; when a compound of Formula (I) is for instance specified as E, this means that the compound is substantially free of the Z isomer; when a compound of Formula (I) is for instance specified as cis, this means that the compound is substantially free of the trans isomer.

Some of the compounds of Formula (I) may also exist in their tautomeric form. Such forms in so far as they may exist, are intended to be included within the scope of the present invention.

It follows that a single compound may exist in both stereoisomeric and tautomeric form.

For therapeutic use, salts of the compounds of Formula (I) and solvates thereof, are those wherein the counterion is pharmaceutically acceptable. However, salts of acids and bases which are non-pharmaceutically acceptable may also find use, for example, in the preparation or purification of a pharmaceutically acceptable compound. All salts, whether pharmaceutically acceptable or not are included within the ambit of the present invention.

The pharmaceutically acceptable addition salts as mentioned hereinabove or hereinafter are meant to comprise the therapeutically active non-toxic acid and base addition salt forms which the compounds of Formula (I) and solvates thereof, are able to form. The pharmaceutically acceptable acid addition salts can conveniently be obtained by treating the base form with such appropriate acid. Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (i.e. butanedioic acid), maleic, fumaric, malic, tartaric, citric, methanesulfonic, ethanesulfonic, benzenesulfonic, p-toluenesulfonic, cyclamic, salicylic, p-aminosalicylic, pamoic and the like acids. Conversely said salt forms can be converted by treatment with an appropriate base into the free base form.

The compounds of Formula (I) and solvates thereof containing an acidic proton may also be converted into their non-toxic metal or amine addition salt forms by treatment with appropriate organic and inorganic bases. Appropriate base salt forms comprise, for example, the ammonium salts, the alkali and earth alkaline metal salts, e.g. the lithium, sodium, potassium, magnesium, calcium salts and the like, salts with organic bases, e.g. primary, secondary and tertiary aliphatic and aromatic amines such as methylamine, ethylamine, propylamine, isopropylamine, the four butylamine isomers, dimethylamine, diethylamine, diethanolamine, dipropylamine, diisopropylamine, di-n-butylamine, pyrrolidine, piperidine, morpholine, trimethylamine, triethylamine, tripropylamine, quinuclidine, pyridine, quinoline and isoquinoline; the benzathine, N-methyl-D-glucamine, hydrabamine salts, and salts with amino acids such as, for example, arginine, lysine and the like. Conversely the salt form can be converted by treatment with acid into the free acid form.

The term solvate comprises the hydrates and solvent addition forms which the compounds of Formula (I) are able to form, as well as pharmaceutically acceptable addition salts thereof. Examples of such forms are e.g. hydrates, alcoholates and the like.

The compounds of the invention as prepared in the processes described below may be synthesized in the form of mixtures of enantiomers, in particular racemic mixtures of enantiomers, that can be separated from one another following art-known resolution procedures. A manner of separating the enantiomeric forms of the compounds of Formula (I) and pharmaceutically acceptable addition salts, and solvates thereof, involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound would be synthesized by stereospecific methods of preparation. These methods will advantageously employ enantiomerically pure starting materials.

In the framework of this application, an element, in particular when mentioned in relation to a compound of Formula (I), comprises all isotopes and isotopic mixtures of this element, either naturally occurring or synthetically produced, either with natural abundance or in an isotopically enriched form. Radiolabelled compounds of Formula (I) may comprise a radioactive isotope selected from the group of $^{2}H$, $^{3}H$, $^{11}C$, $^{18}F$, $^{122}I$, $^{123}I$, $^{125}I$, $^{131}I$, $^{75}Br$, $^{76}Br$, $^{77}Br$ and $^{82}Br$. Preferably, the radioactive isotope is selected from the group of $^{2}H$, $^{3}H$, $^{11}C$ and $^{18}F$. More preferably, the radioactive isotope is $^{2}H$.

In particular, deuterated compounds are intended to be included within the scope of the present invention

As used in the specification and the appended claims, the singular forms "a", "an" and "the" also include plural referents unless the context clearly dictates otherwise. For example, "a compound" means 1 compound or more than 1

compound.

In an embodiment, the present invention concerns novel compounds of Formula (I), tautomers and stereoisomeric forms thereof, wherein

$X_a$, $X_b$ and $X_c$ each independently represent CH or N;

$-X_1-$ represents $-(CHR_{12})_s-NR_1-X_e-C_{1-4}$alkanediyl-$(SO_2)_{p3}-$;

$-X_e-$ represents $-C(R_2)_2-$;

a represents $-NR_4-C(=O)-[C(R_{5b})_2]_r-$ or $-NR_4-C(R_{5b})_2-C(=O)-$;

b represents

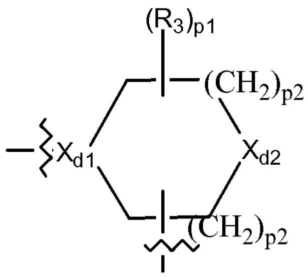

,

wherein said b ring may contain extra bonds to form a bridged ring system selected from 2,5-diazabicyclo[2.2.2]octanyl, 3,8-diazabicyclo[3.2.1]octanyl, 3,6-diazabicyclo[3.1.1]heptanyl, 3,9-diazabicyclo[3.3.1]nonyl;

$X_{d1}$ represents CH or N;

$X_{d2}$ represents NH;

provided that at least one of $X_{d1}$ and $X_{d2}$ represents nitrogen;

c represents a bond, $-[C(R_{5a})_2]_m-$, -O-, $-NR_{5a'}-$;

ring

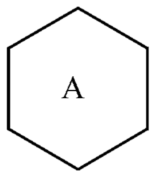

represents phenyl or pyridyl;

$R_1$ represents hydrogen, $C_{1-4}$alkyl, $C_{2-4}$alkenyl, $C_{2-4}$alkynyl, cyano$C_{1-4}$alkyl, $-C(=O)-C_{1-4}$alkyl, -C(=O)-halo$C_{1-4}$alkyl, hydroxy$C_{1-4}$alkyl, halo$C_{1-4}$alkyl, $C_{1-4}$alkyloxy$C_{1-4}$alkyl, halo$C_{1-4}$alkyloxy$C_{1-4}$alkyl, $-C(=O)NR_7R_8$, $-SO_2-NR_7R_8$, $-SO_2-R_9$, $R_{11}$, $C_{1-4}$alkyl substituted with $R_{11}$, $-C(=O)-R_{11}$, or $-C(=O)-C_{1-4}$alkyl-$R_{11}$; in particular $R_1$ represents hydrogen, $C_{1-4}$alkyl, $C_{2-4}$alkenyl, $C_{2-4}$alkynyl, cyano$C_{1-4}$alkyl, $-C(=O)-C_{1-4}$alkyl, -C(=O)-halo$C_{1-4}$alkyl, halo$C_{1-4}$alkyl, $-C(=O)NR_7R_8$, $-SO_2-NR_7R_8$, $-SO_2-R_9$, $R_{11}$, $C_{1-4}$alkyl substituted with $R_{11}$, $-C(=O)-R_{11}$, or $-C(=O)-C_{1-4}$alkyl-$R_{11}$;

each $R_2$ independently represents hydrogen, $C_{1-4}$alkyl, $C_{1-4}$alkyl substituted with $C_{3-6}$cycloalkyl, hydroxy$C_{1-4}$alkyl, $C_{1-4}$alkyloxy$C_{1-4}$alkyl, carboxyl, $-C(=O)-O-C_{1-4}$alkyl wherein $C_{1-4}$alkyl is optionally substituted with $C_{1-4}$alkyloxy, $-C(=O)-NH_2$, $-C(=O)-NH(C_{1-4}$alkyl) wherein $C_{1-4}$alkyl is optionally substituted with $C_{1-4}$alkyloxy, or $-C(=O)-N(C_{1-4}$alkyl$)_2$ wherein each $C_{1-4}$alkyl is optionally substituted with $C_{1-4}$alkyloxy;

or $R_1$ and one $R_2$ are taken together to form $C_{1-4}$alkanediyl or $C_{2-4}$alkenediyl, each of said $C_{1-4}$alkanediyl and $C_{2-4}$alkenediyl optionally being substituted with 1 to 4 substituents each independently selected from hydroxyl, oxo, halo, cyano, $N_3$, hydroxy$C_{1-4}$alkyl, $-NR_7R_8$, $-SO_2-NR_7R_8$, $-NH-SO_2-NR_7R_8$, $-C(=O)-NR_7R_8$, or $-NH-C(=O)-NR_7R_8$;

or $R_1$ and $R_{12}$ are taken together to form $C_{1-4}$alkanediyl or $C_{2-4}$alkenediyl, each of said $C_{1-4}$alkanediyl and

$C_{2-4}$alkenediyl optionally being substituted with 1 to 4 substituents each independently selected from hydroxyl, oxo, halo, cyano, $N_3$, hydroxy$C_{1-4}$alkyl, - $NR_7R_8$, -$SO_2$-$NR_7R_8$, -NH-$SO_2$-$NR_7R_8$, -C(=O)-$NR_7R_8$, or -NH-C(=O)-$NR_7R_8$;

each $R_3$ independently represents hydrogen; oxo; hydroxyl; carboxyl; -$NR_{3a}R_{3b}$; -C(=O)-$NR_{3a}R_{3b}$; hydroxy$C_{1-4}$alkyl; halo$C_{1-4}$alkyl; -(C=O)-$C_{1-4}$alkyl; -C(=O)-O-$C_{1-4}$alkyl wherein said $C_{1-4}$alkyl may optionally be substituted with phenyl; $C_{1-4}$alkyl optionally substituted with cyano, carboxyl, $C_{1-4}$alkyloxy, -C(=O)-O-$C_{1-4}$alkyl, -O-C(=O)-$C_{1-4}$alkyl, -$NR_{3e}R_{3f}$, - C(=O)-$NR_{3e}R_{3f}$, -$SO_2$-$NR_{3e}R_{3f}$, Q, -C(=O)-Q, or -$SO_2$-Q; hydroxy$C_{1-4}$alkyloxy$C_{1-4}$alkyl; $C_{1-4}$alkyloxyhydroxy$C_{1-4}$alkyl; hydroxy$C_{1-4}$alkyloxyhydroxy$C_{1-4}$alkyl; or $C_{1-4}$alkyloxy$C_{1-4}$alkyl optionally substituted with cyano, carboxyl, $C_{1-4}$alkyloxy, -C(=O)-O-$C_{1-4}$alkyl, -O-C(=O)-$C_{1-4}$alkyl, -$NR_{3e}R_{3f}$, -C(=O)-$NR_{3e}R_{3f}$, -$SO_2$-$NR_{3e}R_{3f}$, $R_{10}$, -C(=O)-$R_{10}$, or -$SO_2$-$R_{10}$; or

two $R_3$ substituents attached to the same carbon atom are taken together to form $C_{2-5}$alkanediyl or -$(CH_2)_p$-O-$(CH_2)_p$-;

each $R_{3a}$ and $R_{3b}$ independently represent hydrogen; -(C=O)-$C_{1-4}$alkyl; -$SO_2$-$NR_{3e}R_{3d}$; or $C_{1-4}$alkyl optionally substituted with $C_{1-4}$alkyloxy; or

$R_{3a}$ and $R_{3b}$ are taken together with the nitrogen to which they are attached to form a 4 to 7 membered saturated monocyclic heterocyclic ring which optionally contains 1 or 2 further heteroatoms selected from N, O or $SO_2$, said heterocyclic ring being optionally substituted with 1 to 4 substituents each independently selected from $C_{1-4}$alkyl, halo, hydroxyl, or halo$C_{1-4}$alkyl;

each $R_{3c}$ and $R_{3d}$ independently represent hydrogen, $C_{1-4}$alkyl or -(C=O)-$C_{1-4}$alkyl; or

$R_{3c}$ and $R_{3d}$ are taken together with the nitrogen to which they are attached to form a 4 to 7 membered saturated monocyclic heterocyclic ring which optionally contains 1 or 2 further heteroatoms selected from N, O or $SO_2$, said heterocyclic ring being optionally substituted with 1 to 4 substituents each independently selected from $C_{1-4}$alkyl, halo, hydroxyl, or halo$C_{1-4}$alkyl;

each $R_{3e}$ and $R_{3f}$ independently represent hydrogen, $C_{1-4}$alkyl optionally substituted with $C_{1-4}$alkyloxy, -(C=O)-$C_{1-4}$alkyl, or -$SO_2$-$NR_{3e}R_{3d}$;

$R_4$ represents hydrogen, $C_{1-4}$alkyl or $C_{1-4}$alkyloxy$C_{1-4}$alkyl;

each $R_{5a}$ independently represents hydrogen or $C_{1-4}$alkyl; or

two $R_{5a}$ substituents attached to the same carbon atom are taken together to form $C_{2-5}$alkanediyl or -$(CH_2)_p$-O-$(CH_2)_p$-;

$R_{5a'}$ represents hydrogen or $C_{1-4}$alkyl;

each $R_{5b}$ independently represents hydrogen; $C_{1-4}$alkyl; $C_{1-4}$alkyl substituted with $NR_{5b1}R_{5b2}$; $C_{1-4}$alkyloxy$C_{1-4}$alkyl; hydroxy$C_{1-4}$alkyl; hydroxyl; $C_{3-6}$cycloalkyl; or phenyl optionally substituted with $C_{1-4}$alkyl, halo, hydroxyl or $C_{1-4}$alkyloxy; or two $R_{5b}$ substituents attached to the same carbon atom are taken together to form $C_{2-5}$alkanediyl or -$(CH_2)_p$-O-$(CH_2)_p$-;

$R_{5b1}$ and $R_{5b2}$ independently represent hydrogen, $C_{1-4}$alkyl optionally substituted with $C_{1-4}$alkyloxy, -(C=O)-$C_{1-4}$alkyl, or -$SO_2$-$NR_{5b3}R_{5b4}$;

$R_{5b3}$ and $R_{5b4}$ independently represent hydrogen, $C_{1-4}$alkyl or -(C=O)-$C_{1-4}$alkyl; or

$R_{5b3}$ and $R_{5b4}$ are taken together with the nitrogen to which they are attached to form a 4 to 7 membered saturated monocyclic heterocyclic ring which optionally contains 1 or 2 further heteroatoms selected from N, O or $SO_2$, said heterocyclic ring being optionally substituted with 1 to 4 substituents each independently selected from $C_{1-4}$alkyl, halo, hydroxyl, or halo$C_{1-4}$alkyl;

each $R_6$ independently represents hydrogen, halo, hydroxyl, carboxyl, cyano, $C_{1-4}$alkyl, $C_{1-4}$alkyloxy$C_{1-4}$alkyl, hydroxy$C_{1-4}$alkyl, halo$C_{1-4}$alkyl, $C_{2-4}$alkenyl, $C_{2-4}$alkynyl, - $NR_{6a}R_{6b}$, or -C(=O)$NR_{6a}P_{6b}$;

each $R_{6a}$ and $R_{6b}$ independently represent hydrogen or $C_{1-4}$alkyl;

each $R_7$ and $R_8$ independently represent hydrogen, $C_{1-4}$alkyl, halo$C_{1-4}$alkyl, or $C_{3-6}$cycloalkyl; or

$R_7$ and $R_8$ are taken together with the nitrogen to which they are attached to form a 4 to 7 membered saturated monocyclic heterocyclic ring which optionally contains 1 further heteroatom selected from N, O or $SO_2$, said heterocyclic ring being optionally substituted with 1 to 4 substituents each independently selected from $C_{1-4}$alkyl, halo, hydroxyl, or halo$C_{1-4}$alkyl;

$R_9$ represents $C_{1-4}$alkyl, halo$C_{1-4}$alkyl, or $C_{3-6}$cycloalkyl;

each $R_{10}$ independently represents a 4 to 7 membered saturated monocyclic heterocyclic ring containing up to 2 heteroatoms selected from N, O or $SO_2$, said heterocyclic ring being optionally substituted with 1 to 4 substituents each independently selected from $C_{1-4}$alkyl, halo, hydroxyl or halo$C_{1-4}$alkyl;

each $R_{11}$ independently represents $C_{3-6}$cycloalkyl, phenyl, or a 4 to 7 membered monocyclic heterocyclic ring containing up to 3 heteroatoms selected from N, O or $SO_2$, said heterocyclic ring being optionally substituted with 1 to 4 substituents each independently selected from $C_{1-4}$alkyl, halo, hydroxyl, or halo$C_{1-4}$alkyl;

each $R_{12}$ independently represents hydrogen or $C_{1-4}$alkyl;

Q represents a 4 to 7 membered saturated monocyclic heterocyclic ring containing up to 3 heteroatoms selected from N, O or $SO_2$, said heterocyclic ring being optionally substituted with 1 to 4 substituents each independently selected from $C_{1-4}$alkyl, halo, hydroxyl or halo$C_{1-4}$alkyl;

n represents an integer of value 1 or 2;

m represents an integer of value 1 or 2 ;

p represents an integer of value 1 or 2;

p1 represents an integer of value 1 or 2;

each p2 independently represents an integer of value 0, 1 or 2;

r represents an integer of value 0, 1 or 2;

each $p_3$ independently represents an integer of value 0 or 1;

each s independently represents an integer of value 0, 1 or 2;

and the pharmaceutically acceptable addition salts, and the solvates thereof.

In an embodiment, the present invention concerns novel compounds of Formula (I), tautomers and stereoisomeric forms thereof, wherein
$X_a$, $X_b$ and $X_c$ each independently represent CH or N;
$-X_1-$ represents $-(CHR_{12})_s-NR_1-X_e-C_{1-4}$alkanediyl$-(SO_2)_{p3}-$;
$-X_e-$ represents $-C(R_2)_2-$;
a represents $-NR_4-C(=O)-[C(R_{5b})_2]_r-$ or $-NR_4-C(R_{5b})_2-C(=O)-$;
b represents

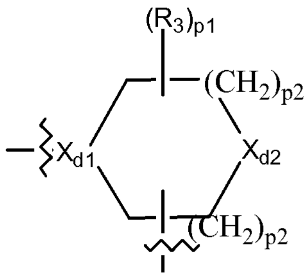

,

wherein said b ring may contain extra bonds to form a bridged ring system selected from 2,5-diazabicyclo[2.2.2]octanyl, 3,8-diazabicyclo[3.2.1]octanyl, 3,6-diazabicyclo[3.1.1]heptanyl, 3,9-diazabicyclo[3.3.1]nonyl;

$X_{d1}$ represents CH or N;
$X_{d2}$ represents NH;
provided that at least one of $X_{d1}$ and $X_{d2}$ represents nitrogen;
c represents a bond, $-[C(R_{5a})_2]_m-$, -O-, $-NR_{5a'}-$;
ring

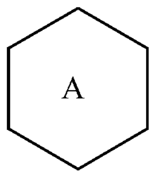

represents phenyl or pyridyl;

$R_1$ represents hydrogen, $C_{1-4}$alkyl, $C_{2-4}$alkenyl, $C_{2-4}$alkynyl, cyano$C_{1-4}$alkyl, -C(=O)-$C_{1-4}$alkyl, -C(=O)-halo$C_{1-4}$alkyl, halo$C_{1-4}$alkyl, -C(=O)$NR_7R_8$, $-SO_2-NR_7R_8$, $-SO_2-R_9$, $R_{11}$, $C_{1-4}$alkyl substituted with $R_{11}$, -C(=O)-$R_{11}$, or -C(=O)-$C_{1-4}$alkyl-$R_{11}$;

each $R_2$ independently represents hydrogen, $C_{1-4}$alkyl, $C_{1-4}$alkyl substituted with $C_{3-6}$cycloalkyl, hydroxy$C_{1-4}$alkyl, $C_{1-4}$alkyloxy$C_{1-4}$alkyl, carboxyl, -C(=O)-O-$C_{1-4}$alkyl wherein $C_{1-4}$alkyl is optionally substituted with $C_{1-4}$alkyloxy, $-C(=O)-NH_2$, -C(=O)-NH($C_{1-4}$alkyl) wherein $C_{1-4}$alkyl is optionally substituted with $C_{1-4}$alkyloxy, or -C(=O)-N($C_{1-4}$alkyl$)_2$ wherein each $C_{1-4}$alkyl is optionally substituted with $C_{1-4}$alkyloxy;

or $R_1$ and one $R_2$ are taken together to form $C_{3-4}$alkanediyl or $C_{3-4}$alkenediyl, each of said $C_{3-4}$alkanediyl and $C_{3-4}$alkenediyl optionally being substituted with 1 to 4 substituents each independently selected from hydroxyl, oxo, halo, cyano, $N_3$, hydroxy$C_{1-4}$alkyl, - $NR_7R_8$, $-SO_2-NR_7R_8$, $-NH-SO_2-NR_7R_8$, $-C(=O)-NR_7R_8$, or $-NH-C(=O)-NR_7R_8$;

each $R_3$ independently represents hydrogen; oxo; hydroxyl; carboxyl; $-NR_{3a}R_{3b}$; $-C(=O)-NR_{3a}R_{3b}$; hydroxy$C_{1-4}$alkyl; halo$C_{1-4}$alkyl; -(C=O)-$C_{1-4}$alkyl; -C(=O)-O-$C_{1-4}$alkyl wherein said $C_{1-4}$alkyl may optionally be substituted with phenyl; $C_{1-4}$alkyl optionally substituted with cyano, carboxyl, $C_{1-4}$alkyloxy, -C(=O)-O-$C_{1-4}$alkyl, -O-C(=O)-$C_{1-4}$alkyl, $-NR_{3e}R_{3f}$, - $C(=O)-NR_{3e}R_{3f}$, $-SO_2-NR_{3e}R_{3f}$, Q, -C(=O)-Q, or $-SO_2-Q$; hydroxy$C_{1-4}$alkyloxy$C_{1-4}$alkyl; $C_{1-4}$alkyloxyhydroxy$C_{1-4}$alkyl; hydroxy$C_{1-4}$alkyloxyhydroxy$C_{1-4}$alkyl; or $C_{1-4}$alkyloxy$C_{1-4}$alkyl optionally substituted with cyano, carboxyl, $C_{1-4}$alkyloxy, -C(=O)-O-$C_{1-4}$alkyl, -O-C(=O)-$C_{1-4}$alkyl, $-NR_{3e}R_{3f}$, $-C(=O)-NR_{3e}R_{3f}$, $-SO_2-NR_{3e}R_{3f}$, $R_{10}$, -C(=O)-$R_{10}$, or $-SO_2-R_{10}$; or

two $R_3$ substituents attached to the same carbon atom are taken together to form $C_{2-5}$alkanediyl or $-(CH_2)_p-O-(CH_2)_p-$;

each $R_{3a}$ and $R_{3b}$ independently represent hydrogen; -(C=O)-$C_{1-4}$alkyl; $-SO_2-NR_{3e}R_{3d}$; or $C_{1-4}$alkyl optionally substituted with $C_{1-4}$alkyloxy; or

$R_{3a}$ and $R_{3b}$ are taken together with the nitrogen to which they are attached to form a 4 to 7 membered saturated monocyclic heterocyclic ring which optionally contains 1 or 2 further heteroatoms selected from N, O or $SO_2$, said heterocyclic ring being optionally substituted with 1 to 4 substituents each independently selected from $C_{1-4}$alkyl, halo, hydroxyl, or halo$C_{1-4}$alkyl;

each $R_{3c}$ and $R_{3d}$ independently represent hydrogen, $C_{1-4}$alkyl or -(C=O)-$C_{1-4}$alkyl; or

$R_{3c}$ and $R_{3d}$ are taken together with the nitrogen to which they are attached to form a 4 to 7 membered saturated monocyclic heterocyclic ring which optionally contains 1 or 2 further heteroatoms selected from N, O or $SO_2$, said heterocyclic ring being optionally substituted with 1 to 4 substituents each independently selected from $C_{1-4}$alkyl, halo, hydroxyl, or halo$C_{1-4}$alkyl;

each $R_{3e}$ and $R_{3f}$ independently represent hydrogen, $C_{1-4}$alkyl optionally substituted with $C_{1-4}$alkyloxy,

-(C=O)-$C_{1-4}$alkyl, or -$SO_2$-$NR_{3e}R_{3d}$;
$R_4$ represents hydrogen, $C_{1-4}$alkyl or $C_{1-4}$alkyloxy$C_{1-4}$alkyl;
each $R_{5a}$ independently represents hydrogen or $C_{1-4}$alkyl; or
two $R_{5a}$ substituents attached to the same carbon atom are taken together to form $C_{2-5}$alkanediyl or -$(CH_2)_p$-O-$(CH_2)_p$-;
$R_{5a'}$ represents hydrogen or $C_{1-4}$alkyl;
each $R_{5b}$ independently represents hydrogen; $C_{1-4}$alkyl; $C_{1-4}$alkyl substituted with $NR_{5b1}R_{5b2}$; $C_{1-4}$alkyloxy$C_{1-4}$alkyl; hydroxy$C_{1-4}$alkyl; hydroxyl; $C_{3-6}$cycloalkyl; or phenyl optionally substituted with $C_{1-4}$alkyl, halo, hydroxyl or $C_{1-4}$alkyloxy; or
two $R_{5b}$ substituents attached to the same carbon atom are taken together to form $C_{2-5}$alkanediyl or -$(CH_2)_p$-O-$(CH_2)_p$-;
$R_{5b1}$ and $R_{5b2}$ independently represent hydrogen, $C_{1-4}$alkyl optionally substituted with $C_{1-4}$alkyloxy, -(C=O)-$C_{1-4}$alkyl, or -$SO_2$-$NR_{5b3}R_{5b4}$;
$R_{5b3}$ and $R_{5b4}$ independently represent hydrogen, $C_{1-4}$alkyl or -(C=O)-$C_{1-4}$alkyl; or
$R_{5b3}$ and $R_{5b4}$ are taken together with the nitrogen to which they are attached to form a 4 to 7 membered saturated monocyclic heterocyclic ring which optionally contains 1 or 2 further heteroatoms selected from N, O or $SO_2$, said heterocyclic ring being optionally substituted with 1 to 4 substituents each independently selected from $C_{1-4}$alkyl, halo, hydroxyl, or halo$C_{1-4}$alkyl;
each $R_6$ independently represents hydrogen, halo, hydroxyl, carboxyl, cyano, $C_{1-4}$alkyl, $C_{1-4}$alkyloxy$C_{1-4}$alkyl, hydroxy$C_{1-4}$alkyl, halo$C_{1-4}$alkyl, $C_{2-4}$alkenyl, $C_{2-4}$alkynyl, - $NR_{6a}R_{6b}$, or -C(=O)$NR_{6a}P_{6b}$;
each $R_{6a}$ and $R_{6b}$ independently represent hydrogen or $C_{1-4}$alkyl;
each $R_7$ and $R_8$ independently represent hydrogen, $C_{1-4}$alkyl, halo$C_{1-4}$alkyl, or $C_{3-6}$cycloalkyl; or
$R_7$ and $R_8$ are taken together with the nitrogen to which they are attached to form a 4 to 7 membered saturated monocyclic heterocyclic ring which optionally contains 1 further heteroatom selected from N, O or $SO_2$, said heterocyclic ring being optionally substituted with 1 to 4 substituents each independently selected from $C_{1-4}$alkyl, halo, hydroxyl, or halo$C_{1-4}$alkyl;
$R_9$ represents $C_{1-4}$alkyl, halo$C_{1-4}$alkyl, or $C_{3-6}$cycloalkyl;
each $R_{10}$ independently represents a 4 to 7 membered saturated monocyclic heterocyclic ring containing up to 2 heteroatoms selected from N, O or $SO_2$, said heterocyclic ring being optionally substituted with 1 to 4 substituents each independently selected from $C_{1-4}$alkyl, halo, hydroxyl or halo$C_{1-4}$alkyl;
each $R_{11}$ independently represents $C_{3-6}$cycloalkyl, phenyl, or a 4 to 7 membered monocyclic heterocyclic ring containing up to 3 heteroatoms selected from N, O or $SO_2$, said heterocyclic ring being optionally substituted with 1 to 4 substituents each independently selected from $C_{1-4}$alkyl, halo, hydroxyl, or halo$C_{1-4}$alkyl;
each $R_{12}$ independently represents hydrogen or $C_{1-4}$alkyl;
Q represents a 4 to 7 membered saturated monocyclic heterocyclic ring containing up to 3 heteroatoms selected from N, O or $SO_2$, said heterocyclic ring being optionally substituted with 1 to 4 substituents each independently selected from $C_{1-4}$alkyl, halo, hydroxyl or halo$C_{1-4}$alkyl;
n represents an integer of value 1 or 2;
m represents an integer of value 1 or 2 ;
p represents an integer of value 1 or 2;
p1 represents an integer of value 1 or 2;
each p2 independently represents an integer of value 0, 1 or 2;
r represents an integer of value 0, 1 or 2;
each $p_3$ independently represents an integer of value 0 or 1;
each s independently represents an integer of value 0, 1 or 2;
and the pharmaceutically acceptable addition salts, and the solvates thereof.

In an embodiment, the present invention concerns novel compounds of Formula (I), tautomers and stereoisomeric forms thereof, wherein
$X_a$, $X_b$ and $X_c$ each independently represent CH or N;
-$X_1$- represents -$(CHR_{12})_s$-$NR_1$-$X_e$-$C_{1-4}$alkanediyl-$(SO_2)_{p3}$;
-$X_e$- represents -$C(R_2)_2$-;
a represents -$NR_4$-C(=O)-$[C(R_{5b})_2]_r$- or -$NR_4$-$C(R_{5b})_2$-C(=O)-;
b represents

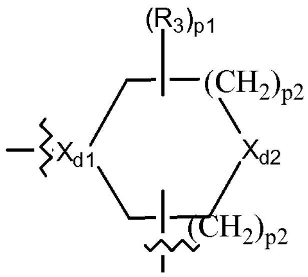

,

wherein said b ring may contain extra bonds to form a bridged ring system selected from 2,5-diazabicyclo[2.2.2]octanyl, 3,8-diazabicyclo[3.2.1]octanyl;

$X_{d1}$ represents CH or N;

$X_{d2}$ represents NH;

c represents a bond, $-[C(R_{5a})_2]_m-$, -O-, $-NR_{5a'}-$;

ring

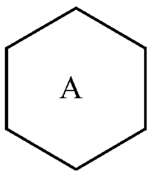

represents phenyl or pyridyl;

$R_1$ represents hydrogen, $C_{1-4}$alkyl, $C_{2-4}$alkenyl, hydroxy$C_{1-4}$alkyl, $C_{1-4}$alkyloxy$C_{1-4}$alkyl, $C_{1-4}$alkyl substituted with $R_{11}$, or $-C(=O)-R_{11}$; in particular $R_1$ represents hydrogen, $C_{1-4}$alkyl, $C_{2-4}$alkenyl, $C_{1-4}$alkyl substituted with $R_{11}$, or $-C(=O)-R_{11}$;

each $R_2$ independently represents hydrogen, $C_{1-4}$alkyl, $C_{1-4}$alkyl substituted with $C_{3-6}$cycloalkyl, carboxyl, -C(=O)-O-$C_{1-4}$alkyl, $-C(=O)-NH_2$, -C(=O)-NH($C_{1-4}$alkyl);

or $R_1$ and one $R_2$ are taken together to form $C_{1-4}$alkanediyl or $C_{2-4}$alkenediyl, each of said $C_{1-4}$alkanediyl and $C_{2-4}$alkenediyl optionally being substituted with 1 substituent selected from hydroxyl, oxo, halo, cyano, $N_3$, $-NR_7R_8$, $-NH-SO_2-NR_7R_8$;

or $R_1$ and $R_{12}$ are taken together to form $C_{1-4}$alkanediyl;

each $R_3$ independently represents hydrogen; hydroxy$C_{1-4}$alkyl; $C_{1-4}$alkyl; or

$C_{1-4}$alkyloxy$C_{1-4}$alkyl optionally substituted with cyano or $-NR_{3e}R_{3f}$, or

two $R_3$ substituents attached to the same carbon atom are taken together to form $C_{2-5}$alkanediyl;

each $R_{3e}$ and $R_{3f}$ independently represent hydrogen, or -(C=O)-$C_{1-4}$alkyl;

$R_4$ represents hydrogen or $C_{1-4}$alkyl;

each $R_{5a}$ independently represents hydrogen or $C_{1-4}$alkyl; or

two $R_{5a}$ substituents attached to the same carbon atom are taken together to form $C_{2-5}$alkanediyl or $-(CH_2)_p-O-(CH_2)_p-$;

$R_{5a'}$ represents hydrogen or $C_{1-4}$alkyl;

each $R_{5b}$ independently represents hydrogen; $C_{1-4}$alkyl; $C_{1-4}$alkyl substituted with $NR_{5b1}R_{5b2}$; $C_{1-4}$alkyloxy$C_{1-4}$alkyl; hydroxy$C_{1-4}$alkyl; hydroxyl; $C_{3-6}$cycloalkyl; or phenyl optionally substituted with $C_{1-4}$alkyl, halo, hydroxyl or $C_{1-4}$alkyloxy; or

two $R_{5b}$ substituents attached to the same carbon atom are taken together to form $C_{2-5}$alkanediyl or $-(CH_2)_p-O-(CH_2)_p-$;

$R_{5b1}$ and $R_{5b2}$ independently represent hydrogen, $-(C=O)-C_{1-4}$alkyl;

each $R_6$ independently represents hydrogen, halo, or $-C(=O)NR_{6a}R_{6b}$;

each $R_{6a}$ and $R_{6b}$ independently represent hydrogen or $C_{1-4}$alkyl;

each $R_7$ and $R_8$ independently represent hydrogen;

each $R_{11}$ independently represents $C_{3-6}$cycloalkyl;

each $R_{12}$ independently represents hydrogen or $C_{1-4}$alkyl;

n represents an integer of value 1;

m represents an integer of value 1;

p represents an integer of value 1;

p1 represents an integer of value 1 or 2;

each p2 independently represents an integer of value 0, 1 or 2;

r represents an integer of value 1;

each $p_3$ independently represents an integer of value 0 or 1;

each s independently represents an integer of value 0 or 1;

and the pharmaceutically acceptable addition salts, and the solvates thereof.

In an embodiment, the present invention concerns novel compounds of Formula (I), tautomers and stereoisomeric forms thereof, wherein
$X_a$ is N;
$X_b$ and $X_c$ represent CH;
$-X_1-$ represents $-NH-(CH_2)_3-$,

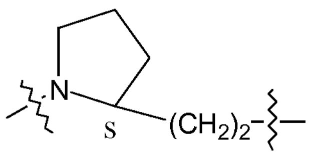 , 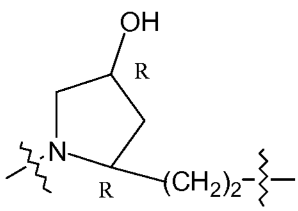 ,

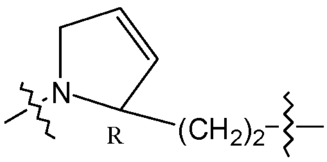,

or $-X_1-$ represents

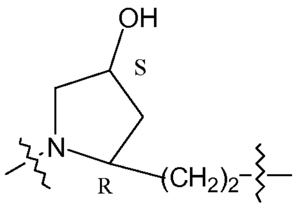;

a represents $-NR_4-C(=O)-[C(R_{5b})_2]_r-$;

b represents (b-1), (b-2), (b-3) or (b-4):

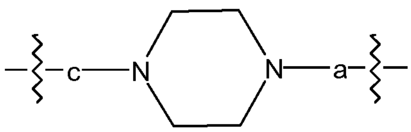

(b-1)

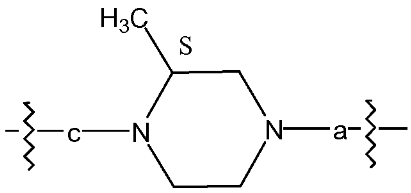

(b-2)

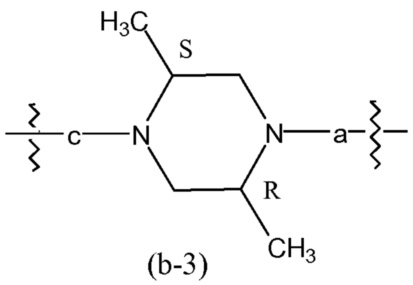

(b-3)

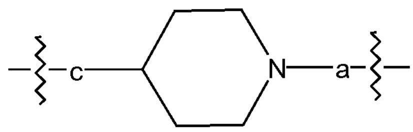

(b-4)

;

c represents $-[C(R_{5a})_2]_m-$ when b represents (b-1), (b-2) or (b-3); or c represents -O-when b represents (b-4);

ring

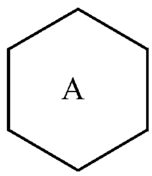

represents phenyl;

$R_4$ represents hydrogen;

each $R_{5a}$ independently represents hydrogen or $C_{1-4}$alkyl; in particular each $R_{5a}$ represents hydrogen;

each $R_{5b}$ independently represents hydrogen; or two $R_{5b}$ substituents attached to the same carbon atom are taken together to form $C_{2-5}$alkanediyl or $-(CH_2)_p-O-(CH_2)_p-$;

each $R_6$ independently represents hydrogen, or halo;

n represents an integer of value 1;

m represents an integer of value 1;

p represents an integer of value 1;

r represents an integer of value 1;

and the pharmaceutically acceptable addition salts, and the solvates thereof.

It will be clear for the skilled person that that in the above embodiment wherein $-X_1-$ represents e.g.

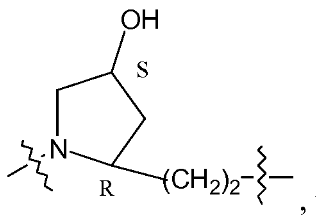

,

the $-(CH_2)_2-$ group is attached to 'variable a'.

Another embodiment of the present invention relates to those compounds of Formula (I) and the pharmaceutically acceptable addition salts, and the solvates thereof, or any subgroup thereof as mentioned in any of the other embodiments wherein one or more of the following restrictions apply:

(i) $X_a$, $X_b$ and $X_c$ each independently represent CH or N;
(ii) $-X_1-$ represents $-(CHR_{12})_s-NR_1-X_e-C_{1-4}$alkanediyl$-(SO_2)_{p3}$;
(iii) $-X_e-$ represents $-C(R_2)_2-$;
(iv) a represents $-NR_4-C(=O)-[C(R_{5b})_2]_r-$ or $-NR_4-C(R_{5b})_2-C(=O)-$;
(v) b represents

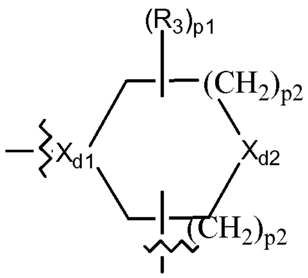

,

wherein said b ring may contain extra bonds to form a bridged ring system selected from 2,5-diazabicyclo[2.2.2]octanyl, 3,8-diazabicyclo[3.2.1]octanyl;
(vi) $X_{d1}$ represents CH or N;
(vii) $X_{d2}$ represents NH;
(viii) c represents a bond, $-[C(R_{5a})_2]_m-$, -O-, $-NR_{5a'}-$;
(ix) ring

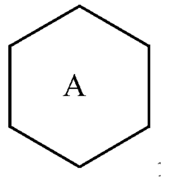

represents phenyl or pyridyl;

(x) $R_1$ represents hydrogen, $C_{1-4}$alkyl, $C_{2-4}$alkenyl, hydroxy$C_{1-4}$alkyl, $C_{1-4}$alkyloxy$C_{1-4}$alkyl, $C_{1-4}$alkyl substituted with $R_{11}$, or -C(=O)-$R_{11}$; in particular hydrogen, $C_{1-4}$alkyl, $C_{2-4}$alkenyl, $C_{1-4}$alkyl substituted with $R_{11}$, or - C(=O)-$R_{11}$; each $R_2$ independently represents hydrogen, $C_{1-4}$alkyl, $C_{1-4}$alkyl substituted with $C_{3-6}$cycloalkyl, carboxyl, -C(=O)-O-$C_{1-4}$alkyl, -C(=O)-$NH_2$, -C(=O)-NH($C_{1-4}$alkyl);

or $R_1$ and one $R_2$ are taken together to form $C_{1-4}$alkanediyl or $C_{2-4}$alkenediyl, each of said $C_{1-4}$alkanediyl and $C_{2-4}$alkenediyl optionally being substituted with 1 substituent selected from hydroxyl, oxo, halo, cyano, $N_3$, -$NR_7R_8$, -NH-$SO_2$-$NR_7R_8$;

(xi) each $R_3$ independently represents hydrogen; hydroxy$C_{1-4}$alkyl; $C_{1-4}$alkyl; or $C_{1-4}$alkyloxy$C_{1-4}$alkyl optionally substituted with cyano or -$NR_{3e}R_{3f}$, or two $R_3$ substituents attached to the same carbon atom are taken together to form $C_{2-5}$alkanediyl;

(xii) each $R_{3e}$ and $R_{3f}$ independently represent hydrogen, or -(C=O)-$C_{1-4}$alkyl;

(xiii) $R_4$ represents hydrogen or $C_{1-4}$alkyl;

(xiv) each $R_{5a}$ independently represents hydrogen or $C_{1-4}$alkyl; or two $R_{5a}$ substituents attached to the same carbon atom are taken together to form $C_{2-5}$alkanediyl or -$(CH_2)_p$-O-$(CH_2)_p$-;

(xv) $R_{5a'}$ represents hydrogen or $C_{1-4}$alkyl;

(xvi) each $R_{5b}$ independently represents hydrogen; $C_{1-4}$alkyl; $C_{1-4}$alkyl substituted with $NR_{5b1}R_{5b2}$; $C_{1-4}$alkyloxy$C_{1-4}$alkyl; hydroxy$C_{1-4}$alkyl; hydroxyl; $C_{3-6}$cycloalkyl; or phenyl optionally substituted with $C_{1-4}$alkyl, halo, hydroxyl or $C_{1-4}$alkyloxy; or

two $R_{5b}$ substituents attached to the same carbon atom are taken together to form $C_{2-5}$alkanediyl or -$(CH_2)_p$-O-$(CH_2)_p$-;

(xvii) $R_{5b1}$ and $R_{5b2}$ independently represent hydrogen, -(C=O)-$C_{1-4}$alkyl;

(xviii) each $R_6$ independently represents hydrogen, halo, or -C(=O)$NR_{6a}R_{6b}$;

(xix) each $R_{6a}$ and $R_{6b}$ independently represent hydrogen or $C_{1-4}$alkyl;

(xx) each $R_7$ and $R_8$ independently represent hydrogen;

(xxi) each $R_{11}$ independently represents $C_{3-6}$cycloalkyl;

(xxii) each $R_{12}$ independently represents hydrogen or $C_{1-4}$alkyl; in particular hydrogen;

(xxiii) n represents an integer of value 1;

(xxiv) m represents an integer of value 1;

(xxv) p represents an integer of value 1;

(xxvi) p1 represents an integer of value 1 or 2;

(xxvii) each p2 independently represents an integer of value 0, 1 or 2;

(xxviii)r represents an integer of value 1;

(xxix) each $p_3$ independently represents an integer of value 0 or 1;

(xxx) each s independently represents an integer of value 0 or 1.

Another embodiment of the present invention relates to those compounds of Formula (I) and the pharmaceutically acceptable addition salts, and the solvates thereof, or any subgroup thereof as mentioned in any of the other embodiments wherein one or more of the following restrictions apply:

(i) $X_a$ represents N; $X_b$ and $X_c$ represent CH;

(ii) -$X_1$- represents -$(CHR_{12})_s$-$NR_1$-$X_e$-$C_{1-4}$alkanediyl-;

(iii) -$X_e$- represents -$C(R_2)_2$-;

(iv) a represents -$NR_4$-C(=O)-$[C(R_{5b})_2]_r$- or -$NR_4$-$C(R_{5b})_2$-C(=O)-; in particular a represents -$NR_4$-C(=O)-$[C(R_{5b})_2]_r$-;

(v) b represents

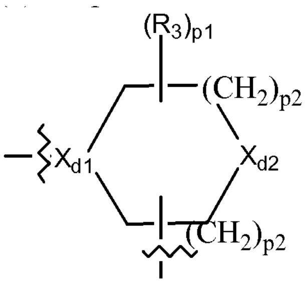

,

provided that the linker with the 'a substituent' is present on $X_{d2}$ or is present on a carbon atom in the alpha position of $X_{d2}$;
(vi) c represents $CH_2$;
(vii) r is 1.

In an embodiment, the present invention relates to those compounds of Formula (I) and the pharmaceutically acceptable addition salts, and the solvates thereof, or any subgroup thereof as mentioned in any of the other embodiments, wherein b represents

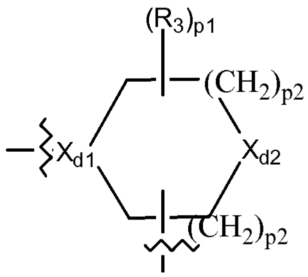

,

in particular wherein b represents

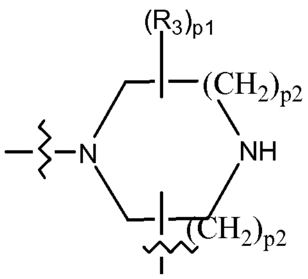

.

In an embodiment, the present invention relates to those compounds of Formula (I) and the pharmaceutically acceptable addition salts, and the solvates thereof, or any subgroup thereof as mentioned in any of the other embodiments, wherein b represents

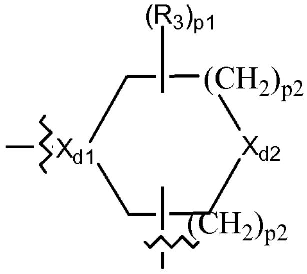

wherein said b ring may contain extra bonds to form a bridged ring system; in particular wherein b represents

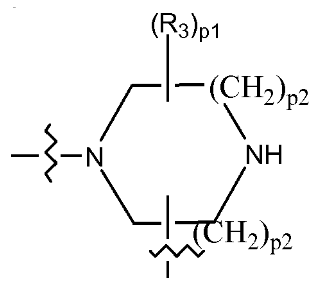

wherein said b ring may contain extra bonds to form a bridged ring system.

In an embodiment, the present invention relates to those compounds of Formula (I) and the pharmaceutically acceptable addition salts, and the solvates thereof, or any subgroup thereof as mentioned in any of the other embodiments, wherein b represents

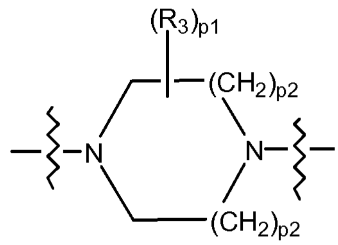

In an embodiment, the present invention relates to those compounds of Formula (I) and the pharmaceutically acceptable addition salts, and the solvates thereof, or any subgroup thereof as mentioned in any of the other embodiments, wherein b represents

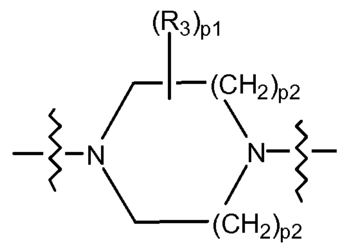

wherein said b ring may contain extra bonds to form a bridged ring system.

In an embodiment, the present invention relates to those compounds of Formula (I) and the pharmaceutically acceptable addition salts, and the solvates thereof, or any subgroup thereof as mentioned in any of the other embodiments, wherein

r is 1;
$-X_1-$ represents $-(CHR_{12})-NR_1-X_e-C_{1-4}$alkanediyl- wherein $C_{1-4}$alkanediyl is optionally substituted with hydroxyl or hydroxy$C_{1-4}$alkyl; or $-X_1-$ represents $-NR_1-X_e-C_{2-4}$alkanediyl- wherein $C_{2-4}$alkanediyl is optionally substituted with hydroxyl or hydroxy$C_{1-4}$alkyl;
m is 1;
$R_6$ is other than $C_{1-4}$alkyl;
$R_3$ is other than hydroxy$C_{1-4}$alkyloxy$C_{1-4}$alkyl; and
b represents

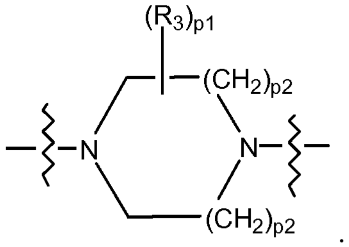

In an embodiment, the present invention relates to those compounds of Formula (I) and the pharmaceutically acceptable addition salts, and the solvates thereof, or any subgroup thereof as mentioned in any of the other embodiments, wherein

r is 1;

$-X_1-$ represents $-(CHR_{12})-NR_1-X_e-C_{1-4}$alkanediyl- wherein $C_{1-4}$alkanediyl is optionally substituted with hydroxyl or hydroxy$C_{1-4}$alkyl; or $-X_1-$ represents $-NR_1-X_e-C_{2-4}$alkanediyl- wherein $C_{2-4}$alkanediyl is optionally substituted with hydroxyl or hydroxy$C_{1-4}$alkyl;

c is $CH_2$;

$R_6$ is other than $C_{1-4}$alkyl;

$R_3$ is other than hydroxy$C_{1-4}$alkyloxy$C_{1-4}$alkyl; and b represents

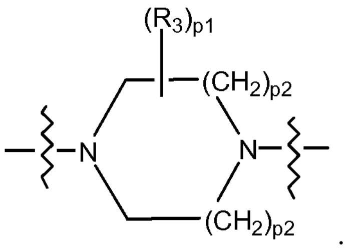

In an embodiment, the present invention relates to those compounds of Formula (I) and the pharmaceutically acceptable addition salts, and the solvates thereof, or any subgroup thereof as mentioned in any of the other embodiments, wherein b represents

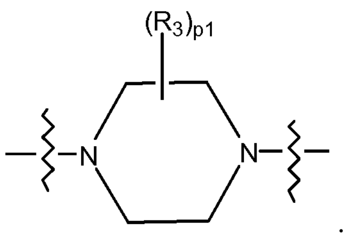

In an embodiment, the present invention relates to those compounds of Formula (I) and the pharmaceutically acceptable addition salts, and the solvates thereof, or any subgroup thereof as mentioned in any of the other embodiments, wherein b represents

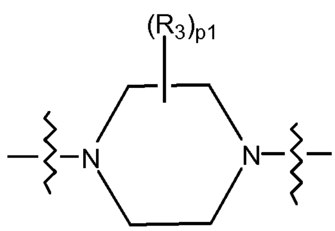

wherein said b ring may contain extra bonds to form a bridged ring system.

In an embodiment, the present invention relates to those compounds of Formula (I) and the pharmaceutically acceptable addition salts, and the solvates thereof, or any subgroup thereof as mentioned in any of the other embodiments,

wherein r is 1, and b represents

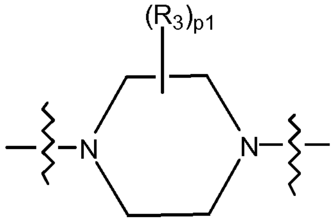

.

In an embodiment, the present invention relates to those compounds of Formula (I) and the pharmaceutically acceptable addition salts, and the solvates thereof, or any subgroup thereof as mentioned in any of the other embodiments, wherein r is 1, and b represents

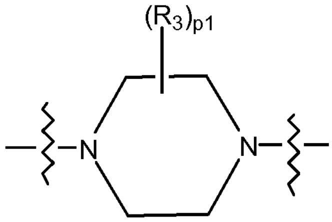

wherein said b ring may contain extra bonds to form a bridged ring system.

In an embodiment, the present invention relates to those compounds of Formula (I) and the pharmaceutically acceptable addition salts, and the solvates thereof, or any subgroup thereof as mentioned in any of the other embodiments, wherein ring b does not contain extra bonds to form a bridged ring system.

In an embodiment, the present invention relates to those compounds of Formula (I) and the pharmaceutically acceptable addition salts, and the solvates thereof, or any subgroup thereof as mentioned in any of the other embodiments, wherein r is 1 and $X_{d2}$ is NH.

In an embodiment, the present invention relates to those compounds of Formula (I) and the pharmaceutically acceptable addition salts, and the solvates thereof, or any subgroup thereof as mentioned in any of the other embodiments, wherein r is 1, $X_{d1}$ is N, and $X_{d2}$ is NH.

In an embodiment, the present invention relates to those compounds of Formula (I) and the pharmaceutically acceptable addition salts, and the solvates thereof, or any subgroup thereof as mentioned in any of the other embodiments, wherein $X_{d1}$ is N, and $X_{d2}$ is NH; and c represents a bond, $-[C(R_{5a})_2]_m-$, -C(=O)-, $-SO_2-$, or -SO-.

In an embodiment, the present invention relates to those compounds of Formula (I) and the pharmaceutically acceptable addition salts, and the solvates thereof, or any subgroup thereof as mentioned in any of the other embodiments, wherein $X_{d1}$ is CH, and $X_{d2}$ is NH; and c represents -O-.

In an embodiment, the present invention relates to those compounds of Formula (I) and the pharmaceutically acceptable addition salts, and the solvates thereof, or any subgroup thereof as mentioned in any of the other embodiments, wherein when $X_{d1}$ is N, then c represents a bond, $-[C(R_{5a})_2]_m-$, -C(=O)-, $-SO_2-$, or -SO-; in particular when $X_{d1}$ is N, then c represents a bond or $-[C(R_{5a})_2]_m-$, more in particular when $X_{d1}$ is N, then c represents $-[C(R_{5a})_2]_m-$, even more in particular when $X_{d1}$ is N, then c represents $-CH_2-$.

In an embodiment, the present invention relates to those compounds of Formula (I) and the pharmaceutically acceptable addition salts, and the solvates thereof, or any subgroup thereof as mentioned in any of the other embodiments, wherein when b represents

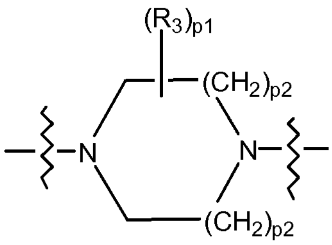

,

then c is other than -O- or -$NR_{5a'}$-.

In an embodiment, the present invention relates to those compounds of Formula (I) and the pharmaceutically acceptable addition salts, and the solvates thereof, or any subgroup thereof as mentioned in any of the other embodiments, wherein when b represents

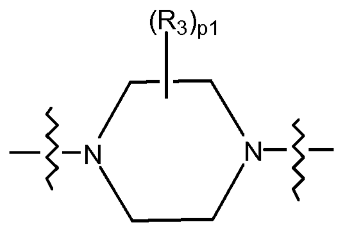

,

then c is other than -O- or -$NR_{5a'}$-.

In an embodiment, the present invention relates to those compounds of Formula (I) and the pharmaceutically acceptable addition salts, and the solvates thereof, or any subgroup thereof as mentioned in any of the other embodiments, wherein c represents a bond or - $[C(R_{5a})_{2]m}$- when $X_{d1}$ represents CH or N; or c may also represent -O- or -$NR_{5a'}$- when $X_{d1}$ represents CH.

In an embodiment, the present invention relates to those compounds of Formula (I) and the pharmaceutically acceptable addition salts, and the solvates thereof, or any subgroup thereof as mentioned in any of the other embodiments, wherein c represents a bond, - $[C(R_{5a})_2]_m$-, -C(=O)-, -$SO_2$-, or -SO- when $X_{d1}$ represents CH or N; or c may also represent -O- or -$NR_{5a'}$- when $X_{d1}$ represents CH.

In an embodiment, the present invention relates to those compounds of Formula (I) and the pharmaceutically acceptable addition salts, and the solvates thereof, or any subgroup thereof as mentioned in any of the other embodiments, wherein $X_{d1}$ represents CH and $X_{d2}$ represents NH.

In an embodiment, the present invention relates to those compounds of Formula (I) and the pharmaceutically acceptable addition salts, and the solvates thereof, or any subgroup thereof as mentioned in any of the other embodiments, wherein s is 1.

In an embodiment, the present invention relates to those compounds of Formula (I) and the pharmaceutically acceptable addition salts, and the solvates thereof, or any subgroup thereof as mentioned in any of the other embodiments, wherein p3 is 0.

In an embodiment, the present invention relates to those compounds of Formula (I) and the pharmaceutically acceptable addition salts, and the solvates thereof, or any subgroup thereof as mentioned in any of the other embodiments, wherein s is 0 or 1.

In an embodiment, the present invention relates to those compounds of Formula (I) and the pharmaceutically acceptable addition salts, and the solvates thereof, or any subgroup thereof as mentioned in any of the other embodiments, wherein s is 0.

In an embodiment, the present invention relates to those compounds of Formula (I) and the pharmaceutically acceptable addition salts, and the solvates thereof, or any subgroup thereof as mentioned in any of the other embodiments, wherein s is 0 and p3 is 0.

In an embodiment, the present invention relates to those compounds of Formula (I) and the pharmaceutically acceptable addition salts, and the solvates thereof, or any subgroup thereof as mentioned in any of the other embodiments, wherein s is 1, p3 is 0 and $R_{12}$ is H.

In an embodiment, the present invention relates to those compounds of Formula (I) and the pharmaceutically acceptable addition salts, and the solvates thereof, or any subgroup thereof as mentioned in any of the other embodiments, wherein m is 1.

In an embodiment, the present invention relates to those compounds of Formula (I) and the pharmaceutically acceptable addition salts, and the solvates thereof, or any subgroup thereof as mentioned in any of the other embodiments, wherein p2 is 1.

In an embodiment, the present invention relates to those compounds of Formula (I) and the pharmaceutically acceptable addition salts, and the solvates thereof, or any subgroup thereof as mentioned in any of the other embodiments, wherein $X_a$ is N.

In an embodiment, the present invention relates to those compounds of Formula (I) and the pharmaceutically acceptable addition salts, and the solvates thereof, or any subgroup thereof as mentioned in any of the other embodiments, wherein $X_a$ is N; $X_b$ and $X_c$ represent CH.

In an embodiment, the present invention relates to those compounds of Formula (I) and the pharmaceutically acceptable addition salts, and the solvates thereof, or any subgroup thereof as mentioned in any of the other embodiments,

wherein one of $X_a$, $X_b$ and $X_c$ is N, and the other are CH.

In an embodiment, the present invention relates to those compounds of Formula (I) and the pharmaceutically acceptable addition salts, and the solvates thereof, or any subgroup thereof as mentioned in any of the other embodiments, wherein

$X_a$ is N; $X_b$ and $X_c$ represent CH;

$R_1$ represents hydrogen, $C_{1-4}$alkyl, $C_{2-4}$alkenyl, $C_{2-4}$alkynyl, cyano$C_{1-4}$alkyl, -C(=O)-$C_{1-4}$alkyl, -C(=O)-halo$C_{1-4}$alkyl, halo$C_{1-4}$alkyl, -C(=O)$NR_7R_8$, -$SO_2$-$NR_7R_8$, -$SO_2$-$R_9$, $R_{11}$, $C_{1-4}$alkyl substituted with $R_{11}$, -C(=O)-$R_{11}$, or -C(=O)-$C_{1-4}$alkyl-$R_{11}$;

each $R_2$ independently represents hydrogen, $C_{1-4}$alkyl, $C_{1-4}$alkyl substituted with $C_{3-6}$cycloalkyl, hydroxy$C_{1-4}$alkyl, $C_{1-4}$alkyloxy$C_{1-4}$alkyl, carboxyl, -C(=O)-O-$C_{1-4}$alkyl wherein $C_{1-4}$alkyl is optionally substituted with $C_{1-4}$alkyloxy, or -C(=O)-$NH_2$; or $R_1$ and one $R_2$ are taken together to form $C_{3-4}$alkanediyl or $C_{3-4}$alkenediyl, each of said $C_{3-4}$alkanediyl and $C_{3-4}$alkenediyl optionally being substituted with 1 to 4 substituents each independently selected from hydroxyl, oxo, halo, cyano, $N_3$, hydroxy$C_{1-4}$alkyl, - $NR_7R_8$, -$SO_2$-$NR_7R_8$, -NH-$SO_2$-$NR_7R_8$, -C(=O)-$NR_7R_8$, or -NH-C(=O)-$NR_7R_8$;

$R_{12}$ is hydrogen.

In an embodiment, the present invention relates to those compounds of Formula (I) and the pharmaceutically acceptable addition salts, and the solvates thereof, or any subgroup thereof as mentioned in any of the other embodiments, wherein $X_a$ is N; $X_b$ and $X_c$ represent CH;

$R_1$ represents hydrogen, $C_{1-4}$alkyl, $C_{2-4}$alkenyl, $C_{2-4}$alkynyl, cyano$C_{1-4}$alkyl, -C(=O)-$C_{1-4}$alkyl, -C(=O)-halo$C_{1-4}$alkyl, halo$C_{1-4}$alkyl, -C(=O)$NR_7R_8$, -$SO_2$-$NR_7R_8$, -$SO_2$-$R_9$, $R_{11}$, $C_{1-4}$alkyl substituted with $R_{11}$, -C(=O)-$R_{11}$, or -C(=O)-$C_{1-4}$alkyl-$R_{11}$;

each $R_2$ independently represents hydrogen, $C_{1-4}$alkyl, $C_{1-4}$alkyl substituted with $C_{3-6}$cycloalkyl, hydroxy$C_{1-4}$alkyl, $C_{1-4}$alkyloxy$C_{1-4}$alkyl, carboxyl, -C(=O)-O-$C_{1-4}$alkyl wherein $C_{1-4}$alkyl is optionally substituted with $C_{1-4}$alkyloxy, or -C(=O)-$NH_2$; or $R_1$ and one $R_2$ are taken together to form $C_{3-4}$alkanediyl or $C_{3-4}$alkenediyl, each of said $C_{3-4}$alkanediyl and $C_{3-4}$alkenediyl optionally being substituted with 1 to 4 substituents each independently selected from hydroxyl, oxo, halo, cyano, $N_3$, hydroxy$C_{1-4}$alkyl, - $NR_7R_8$, -$SO_2$-$NR_7R_8$, -NH-$SO_2$-$NR_7R_8$, -C(=O)-$NR_7R_8$, or -NH-C(=O)-$NR_7R_8$;

s is 0.

In an embodiment, the present invention relates to those compounds of Formula (I) and the pharmaceutically acceptable addition salts, and the solvates thereof, or any subgroup thereof as mentioned in any of the other embodiments, wherein $X_a$ is N; $X_b$ and $X_c$ represent CH;

$R_1$ represents hydrogen, $C_{1-4}$alkyl, $C_{2-4}$alkenyl, $C_{1-4}$alkyl substituted with $R_{11}$, or -C(=O)-$R_{11}$;

each $R_2$ independently represents hydrogen, $C_{1-4}$alkyl, $C_{1-4}$alkyl substituted with $C_{3-6}$cycloalkyl, carboxyl, -C(=O)-O-$C_{1-4}$alkyl, or -C(=O)-$NH_2$;

or $R_1$ and one $R_2$ are taken together to form $C_{3-4}$alkanediyl or $C_{3-4}$alkenediyl, each of said $C_{3-4}$alkanediyl and $C_{3-4}$alkenediyl optionally being substituted with 1 substituent selected from hydroxyl, oxo, halo, cyano, $N_3$, -$NR_7R_8$, or -NH-$SO_2$-$NR_7R_8$;

s is 0.

In an embodiment, the present invention relates to those compounds of Formula (I) and the pharmaceutically acceptable addition salts, and the solvates thereof, or any subgroup thereof as mentioned in any of the other embodiments, wherein ring A is phenyl.

In an embodiment, the present invention relates to those compounds of Formula (I) and the pharmaceutically acceptable addition salts, and the solvates thereof, or any subgroup thereof as mentioned in any of the other embodiments, wherein ring A is pyridyl.

In an embodiment, the present invention relates to those compounds of Formula (I) and the pharmaceutically acceptable addition salts, and the solvates thereof, or any subgroup thereof as mentioned in any of the other embodiments, wherein $R_1$ represents $C_{1-4}$alkyl, $C_{2-4}$alkenyl, $C_{2-4}$alkynyl, -C(=O)-$C_{1-4}$alkyl, -C(=O)-halo$C_{1-4}$alkyl, hydroxy$C_{1-4}$alkyl, halo$C_{1-4}$alkyl, $C_{1-4}$alkyloxy$C_{1-4}$alkyl, halo$C_{1-4}$alkyloxy$C_{1-4}$alkyl, -C(=O)$NR_7R_8$, -$SO_2$-$R_9$, $R_{11}$, $C_{1-4}$alkyl substituted with $R_{11}$, -C(=O)-$R_{11}$, or -C(=O)-$C_{1-4}$alkyl-$R_{11}$.

In an embodiment, the present invention relates to those compounds of Formula (I) and the pharmaceutically acceptable addition salts, and the solvates thereof, or any subgroup thereof as mentioned in any of the other embodiments, wherein $R_1$ represents $C_{1-4}$alkyl, $C_{2-4}$alkenyl, $C_{2-4}$alkynyl, or $C_{1-4}$alkyloxy$C_{1-4}$alkyl; in particular $R_1$ represents $C_{1-4}$alkyl, $C_{2-4}$alkenyl, or $C_{1-4}$alkyloxy$C_{1-4}$alkyl.

In an embodiment, the present invention relates to those compounds of Formula (I) and the pharmaceutically acceptable addition salts, and the solvates thereof, or any subgroup thereof as mentioned in any of the other embodiments, wherein $R_1$ represents hydrogen, $C_{1-4}$alkyl, $C_{2-4}$alkenyl, $C_{2-4}$alkynyl, cyano$C_{1-4}$alkyl, -C(=O)-$C_{1-4}$alkyl, -C(=O)-halo$C_{1-4}$alkyl, halo$C_{1-4}$alkyl, -C(=O)$NR_7R_8$, -$SO_2$-$NR_7R_8$, -$SO_2$-$R_9$, $R_{11}$, $C_{1-4}$alkyl substituted with $R_{11}$, -C(=O)-$R_{11}$, or -C(=O)-$C_{1-4}$alkyl-$R_{11}$.

In an embodiment, the present invention relates to those compounds of Formula (I) and the pharmaceutically acceptable addition salts, and the solvates thereof, or any subgroup thereof as mentioned in any of the other embodiments,

wherein $R_1$ represents hydrogen, $C_{1-4}$alkyl, $C_{2-4}$alkenyl, $C_{2-4}$alkynyl, cyano$C_{1-4}$alkyl, -C(=O)-$C_{1-4}$alkyl, -C(=O)-halo$C_{1-4}$alkyl, halo$C_{1-4}$alkyl, -C(=O)$NR_7R_8$, -$SO_2$-$NR_7R_8$, -$SO_2$-$R_9$, $R_{11}$, $C_{1-4}$alkyl substituted with $R_{11}$, -C(=O)-$R_{11}$, or -C(=O)-$C_{1-4}$alkyl-$R_{11}$; or $R_1$ is taken together with one $R_2$ or $R_{12}$.

In an embodiment, the present invention relates to those compounds of Formula (I) and the pharmaceutically acceptable addition salts, and the solvates thereof, or any subgroup thereof as mentioned in any of the other embodiments, wherein $R_1$ represents $C_{1-4}$alkyl, $C_{2-4}$alkenyl, $C_{2-4}$alkynyl, -C(=O)-$C_{1-4}$alkyl, -C(=O)-halo$C_{1-4}$alkyl, hydroxy$C_{1-4}$alkyl, halo$C_{1-4}$alkyl, $C_{1-4}$alkyloxy$C_{1-4}$alkyl, halo$C_{1-4}$alkyloxy$C_{1-4}$alkyl, -C(=O)$NR_7R_8$, -$SO_2$-$R_9$, $R_{11}$, $C_{1-4}$alkyl substituted with $R_{11}$, -C(=O)-$R_{11}$, or -C(=O)-$C_{1-4}$alkyl-$R_{11}$; or $R_1$ is taken together with one $R_2$ or $R_{12}$.

In an embodiment, the present invention relates to those compounds of Formula (I) and the pharmaceutically acceptable addition salts, and the solvates thereof, or any subgroup thereof as mentioned in any of the other embodiments, wherein $R_1$ represents $C_{1-4}$alkyl, $C_{2-4}$alkenyl, $C_{2-4}$alkynyl, -C(=O)-$C_{1-4}$alkyl, -C(=O)-halo$C_{1-4}$alkyl, halo$C_{1-4}$alkyl, -C(=O)$NR_7R_8$, -$SO_2$-$R_9$, $R_{11}$, $C_{1-4}$alkyl substituted with $R_{11}$, -C(=O)-$R_{11}$, or -C(=O)-$C_{1-4}$alkyl-$R_{11}$; or $R_1$ is taken together with one $R_2$ or $R_{12}$.

In an embodiment, the present invention relates to those compounds of Formula (I) and the pharmaceutically acceptable addition salts, and the solvates thereof, or any subgroup thereof as mentioned in any of the other embodiments, wherein $R_1$ and $R_{12}$ are not taken together.

In an embodiment, the present invention relates to those compounds of Formula (I) and the pharmaceutically acceptable addition salts, and the solvates thereof, or any subgroup thereof as mentioned in any of the other embodiments, wherein

$R_1$ represents hydrogen, $C_{1-4}$alkyl, $C_{2-4}$alkenyl, $C_{1-4}$alkyl substituted with $R_{11}$, or -C(=O)-$R_{11}$;

each $R_2$ independently represents hydrogen, $C_{1-4}$alkyl, $C_{1-4}$alkyl substituted with $C_{3-6}$cycloalkyl, carboxyl, -C(=O)-O-$C_{1-4}$alkyl, -C(=O)-$NH_2$, -C(=O)-NH($C_{1-4}$alkyl); or $R_1$ and one $R_2$ are taken together to form $C_{1-4}$alkanediyl or $C_{2-4}$alkenediyl, each of said $C_{1-4}$alkanediyl and $C_{2-4}$alkenediyl optionally being substituted with 1 substituent selected from hydroxyl, oxo, halo, cyano, $N_3$, -$NR_7R_8$, -NH-$SO_2$-$NR_7R_8$;

$R_{12}$ is hydrogen.

In an embodiment, the present invention relates to those compounds of Formula (I) and the pharmaceutically acceptable addition salts, and the solvates thereof, or any subgroup thereof as mentioned in any of the other embodiments, wherein

$R_1$ is other than hydroxy$C_{1-4}$alkyl or $C_{1-4}$alkyloxy$C_{1-4}$alkyl;

s is 0.

In an embodiment, the present invention relates to those compounds of Formula (I) and the pharmaceutically acceptable addition salts, and the solvates thereof, or any subgroup thereof as mentioned in any of the other embodiments, wherein

$R_1$ is other than hydroxy$C_{1-4}$alkyl or $C_{1-4}$alkyloxy$C_{1-4}$alkyl;

$R_1$ and $R_{12}$ are not taken together;

$R_{12}$ is hydrogen.

In an embodiment, the present invention relates to those compounds of Formula (I) and the pharmaceutically acceptable addition salts, and the solvates thereof, or any subgroup thereof as mentioned in any of the other embodiments, wherein $R_1$ represents hydrogen, $C_{1-4}$alkyl, $C_{2-4}$alkenyl, $C_{2-4}$alkynyl, cyano$C_{1-4}$alkyl, -C(=O)-$C_{1-4}$alkyl, -C(=O)-halo$C_{1-4}$alkyl, halo$C_{1-4}$alkyl, -C(=O)$NR_7R_8$, -$SO_2$-$NR_7R_8$, -$SO_2$-$R_9$, $R_{11}$, $C_{1-4}$alkyl substituted with $R_{11}$, -C(=O)-$R_{11}$, or -C(=O)-$C_{1-4}$alkyl-$R_{11}$; or $R_1$ is taken together with one $R_2$.

In an embodiment, the present invention relates to those compounds of Formula (I) and the pharmaceutically acceptable addition salts, and the solvates thereof, or any subgroup thereof as mentioned in any of the other embodiments, wherein $R_1$ represents $C_{1-4}$alkyl, $C_{2-4}$alkenyl, $C_{2-4}$alkynyl, -C(=O)-$C_{1-4}$alkyl, -C(=O)-halo$C_{1-4}$alkyl, hydroxy$C_{1-4}$alkyl, halo$C_{1-4}$alkyl, $C_{1-4}$alkyloxy$C_{1-4}$alkyl, halo$C_{1-4}$alkyloxy$C_{1-4}$alkyl, -C(=O)$NR_7R_8$, -$SO_2$-$R_9$, $R_{11}$, $C_{1-4}$alkyl substituted with $R_{11}$, -C(=O)-$R_{11}$, or -C(=O)-$C_{1-4}$alkyl-$R_{11}$; or $R_1$ is taken together with one $R_2$.

In an embodiment, the present invention relates to those compounds of Formula (I) and the pharmaceutically acceptable addition salts, and the solvates thereof, or any subgroup thereof as mentioned in any of the other embodiments, wherein $R_1$ represents $C_{1-4}$alkyl, $C_{2-4}$alkenyl, $C_{2-4}$alkynyl, -C(=O)-$C_{1-4}$alkyl, -C(=O)-halo$C_{1-4}$alkyl, halo$C_{1-4}$alkyl, -C(=O)$NR_7R_8$, -$SO_2$-$R_9$, $R_{11}$, $C_{1-4}$alkyl substituted with $R_{11}$, -C(=O)-$R_{11}$, or -C(=O)-$C_{1-4}$alkyl-$R_{11}$; or $R_1$ is taken together with one $R_2$.

In an embodiment, the present invention relates to those compounds of Formula (I) and the pharmaceutically acceptable addition salts, and the solvates thereof, or any subgroup thereof as mentioned in any of the other embodiments, wherein $R_1$ represents hydrogen, $C_{1-4}$alkyl, $C_{2-4}$alkenyl, hydroxy$C_{1-4}$alkyl, $C_{1-4}$alkyloxy$C_{1-4}$alkyl, $C_{1-4}$alkyl substituted with $R_{11}$, or -C(=O)-$R_{11}$; in particular hydrogen, $C_{1-4}$alkyl, $C_{2-4}$alkenyl,

$C_{1-4}$alkyl substituted with $R_{11}$, or -C(=O)-$R_{11}$;

each $R_2$ independently represents hydrogen, $C_{1-4}$alkyl, $C_{1-4}$alkyl substituted with $C_{3-6}$cycloalkyl, carboxyl, -C(=O)-O-$C_{1-4}$alkyl, -C(=O)-$NH_2$, -C(=O)-NH($C_{1-4}$alkyl); or $R_1$ and one $R_2$ are taken together to form $C_{1-4}$alkanediyl or

$C_{2-4}$alkenediyl, each of said $C_{1-4}$alkanediyl and $C_{2-4}$alkenediyl optionally being substituted with 1 substituent selected from hydroxyl, oxo, halo, cyano, $N_3$, $-NR_7R_8$, $-NH-SO_2-NR_7R_8$.

In an embodiment, the present invention relates to those compounds of Formula (I) and the pharmaceutically acceptable addition salts, and the solvates thereof, or any subgroup thereof as mentioned in any of the other embodiments, wherein $R_1$ represents hydrogen, $C_{1-4}$alkyl, $C_{2-4}$alkenyl, $C_{1-4}$alkyl substituted with $R_{11}$, or $-C(=O)-R_{11}$.

In an embodiment, the present invention relates to those compounds of Formula (I) and the pharmaceutically acceptable addition salts, and the solvates thereof, or any subgroup thereof as mentioned in any of the other embodiments, wherein $R_1$ represents hydrogen, $C_{1-4}$alkyl, $C_{2-4}$alkenyl, $C_{1-4}$alkyl substituted with $R_{11}$, or $-C(=O)-R_{11}$; or $R_1$ is taken together with one $R_2$.

In an embodiment, the present invention relates to those compounds of Formula (I) and the pharmaceutically acceptable addition salts, and the solvates thereof, or any subgroup thereof as mentioned in any of the other embodiments, wherein $R_1$ represents hydrogen, or $R_1$ is taken together with one $R_2$.

In an embodiment, the present invention relates to those compounds of Formula (I) and the pharmaceutically acceptable addition salts, and the solvates thereof, or any subgroup thereof as mentioned in any of the other embodiments, wherein $R_1$ is other than hydrogen.

In an embodiment, the present invention relates to those compounds of Formula (I) and the pharmaceutically acceptable addition salts, and the solvates thereof, or any subgroup thereof as mentioned in any of the other embodiments, wherein when $R_1$ and $R_2$ are taken together, they form $C_{3-4}$alkanediyl or $C_{3-4}$alkenediyl, each of said $C_{3-4}$alkanediyl and $C_{3-4}$alkenediyl optionally being substituted with 1 to 4 substituents each independently selected from hydroxyl, oxo, halo, cyano, $N_3$, hydroxy$C_{1-4}$alkyl, $-NR_7R_8$, $-SO_2-NR_7R_8$, - $NH-SO_2-NR_7R_8$, $-C(=O)-NR_7R_8$, or $-NH-C(=O)-NR_7R_8$.

In an embodiment, the present invention relates to those compounds of Formula (I) and the pharmaceutically acceptable addition salts, and the solvates thereof, or any subgroup thereof as mentioned in any of the other embodiments, wherein when $R_1$ and $R_{12}$ are taken together, they form $C_{3-4}$alkanediyl or $C_{3-4}$alkenediyl, each of said $C_{3-4}$alkanediyl and $C_{3-4}$alkenediyl optionally being substituted with 1 to 4 substituents each independently selected from hydroxyl, oxo, halo, cyano, $N_3$, hydroxy$C_{1-4}$alkyl, $-NR_7R_8$, - $SO_2-NR_7R_8$, $-NH-SO_2-NR_7R_8$, $-C(=O)-NR_7R_8$, or $-NH-C(=O)-NR_7R_8$.

In an embodiment, the present invention relates to those compounds of Formula (I) and the pharmaceutically acceptable addition salts, and the solvates thereof, or any subgroup thereof as mentioned in any of the other embodiments, wherein

$R_1$ represents hydrogen, $C_{1-4}$alkyl, $C_{2-4}$alkenyl, $C_{2-4}$alkynyl, cyano$C_{1-4}$alkyl, $-C(=O)-C_{1-4}$alkyl, $-C(=O)$-halo$C_{1-4}$alkyl, halo$C_{1-4}$alkyl, $-C(=O)NR_7R_8$, $-SO_2-NR_7R_8$, $-SO_2-R_9$, $R_{11}$, $C_{1-4}$alkyl substituted with $R_{11}$, $-C(=O)-R_{11}$, or $-C(=O)-C_{1-4}$alkyl-$R_{11}$;

each $R_2$ independently represents hydrogen, $C_{1-4}$alkyl, $C_{1-4}$alkyl substituted with $C_{3-6}$cycloalkyl, hydroxy$C_{1-4}$alkyl, $C_{1-4}$alkyloxy$C_{1-4}$alkyl, carboxyl, $-C(=O)-O-C_{1-4}$alkyl wherein $C_{1-4}$alkyl is optionally substituted with $C_{1-4}$alkyloxy, $-C(=O)-NH_2$, $-C(=O)-NH(C_{1-4}$alkyl) wherein $C_{1-4}$alkyl is optionally substituted with $C_{1-4}$alkyloxy, or $-C(=O)-N(C_{1-4}$alkyl$)_2$ wherein each $C_{1-4}$alkyl is optionally substituted with $C_{1-4}$alkyloxy; or

$R_1$ and one $R_2$ are taken together to form $C_{3-4}$alkanediyl or $C_{3-4}$alkenediyl, each of said $C_{3-4}$alkanediyl and $C_{3-4}$alkenediyl optionally being substituted with 1 to 4 substituents each independently selected from hydroxyl, oxo, halo, cyano, $N_3$, hydroxy$C_{1-4}$alkyl, $-NR_7R_8$, $-SO_2-NR_7R_8$, $-NH-SO_2-NR_7R_8$, $-C(=O)-NR_7R_8$, or $-NH-C(=O)-NR_7R_8$; or $R_1$ and $R_{12}$ are taken together to form $C_{3-4}$alkanediyl or $C_{3-4}$alkenediyl, each of said $C_{3-4}$alkanediyl and $C_{3-4}$alkenediyl optionally being substituted with 1 to 4 substituents each independently selected from hydroxyl, oxo, halo, cyano, $N_3$, hydroxy$C_{1-4}$alkyl, -$NR_7R_8$, $-SO_2-NR_7R_8$, $-NH-SO_2-NR_7R_8$, $-C(=O)-NR_7R_8$, or $-NH-C(=O)-NR_7R_8$.

In an embodiment, the present invention relates to those compounds of Formula (I) and the pharmaceutically acceptable addition salts, and the solvates thereof, or any subgroup thereof as mentioned in any of the other embodiments, wherein $R_2$ represents hydrogen.

In an embodiment, the present invention relates to those compounds of Formula (I) and the pharmaceutically acceptable addition salts, and the solvates thereof, or any subgroup thereof as mentioned in any of the other embodiments, wherein $R_2$ represents hydrogen; or $R_1$ and $R_2$ are taken together.

In an embodiment, the present invention relates to those compounds of Formula (I) and the pharmaceutically acceptable addition salts, and the solvates thereof, or any subgroup thereof as mentioned in any of the other embodiments, wherein $R_1$ and one $R_2$ are taken together to form $C_{1-4}$alkanediyl optionally being substituted with 1 hydroxyl substituent; and wherein the other $R_2$ variables are hydrogen.

In an embodiment, the present invention relates to those compounds of Formula (I) and the pharmaceutically acceptable addition salts, and the solvates thereof, or any subgroup thereof as mentioned in any of the other embodiments, wherein $R_4$ represents hydrogen.

In an embodiment, the present invention relates to those compounds of Formula (I) and the pharmaceutically acceptable addition salts, and the solvates thereof, or any subgroup thereof as mentioned in any of the other embodiments, wherein each $R_{10}$ independently represents a 6 membered saturated monocyclic heterocyclic ring containing up to 2 heteroatoms selected from N or O, said heterocyclic ring being optionally substituted with 1 $C_{1-4}$alkyl substituent.

In an embodiment, the present invention relates to those compounds of Formula (I) and the pharmaceutically acceptable addition salts, and the solvates thereof, or any subgroup thereof as mentioned in any of the other embodiments, wherein each $R_{10}$ independently represents morpholinyl or piperazinyl optionally substituted with 1 $C_{1-4}$alkyl substituent.

In an embodiment, the present invention relates to those compounds of Formula (I) and the pharmaceutically acceptable addition salts, and the solvates thereof, or any subgroup thereof as mentioned in any of the other embodiments, wherein each $R_{11}$ independently represents $C_{3-6}$cycloalkyl.

In an embodiment, the present invention relates to those compounds of Formula (I) and the pharmaceutically acceptable addition salts, and the solvates thereof, or any subgroup thereof as mentioned in any of the other embodiments, wherein $CHR_{12}$ is $CH_2$.

In an embodiment, the present invention relates to those compounds of Formula (I) and the pharmaceutically acceptable addition salts, and the solvates thereof, or any subgroup thereof as mentioned in any of the other embodiments, wherein $R_{12}$ is H.

In an embodiment, the present invention relates to those compounds of Formula (I) and the pharmaceutically acceptable addition salts, and the solvates thereof, or any subgroup thereof as mentioned in any of the other embodiments, wherein c represents $CH_2$.

In an embodiment, the present invention relates to those compounds of Formula (I) and the pharmaceutically acceptable addition salts, and the solvates thereof, or any subgroup thereof as mentioned in any of the other embodiments, wherein c represents-$[C(R_{5a})_2]_m$-.

In an embodiment, the present invention relates to those compounds of Formula (I) and the pharmaceutically acceptable addition salts, and the solvates thereof, or any subgroup thereof as mentioned in any of the other embodiments, wherein a represents $-NR_4-C(=O)-[C(R_{5b})_2]_r-$ or $-NR_4-C(R_{5b})_2-C(=O)-$.

In an embodiment, the present invention relates to those compounds of Formula (I) and the pharmaceutically acceptable addition salts, and the solvates thereof, or any subgroup thereof as mentioned in any of the other embodiments, wherein a represents $-NR_4-C(=O)-[C(R_{5b})_2]_r-$.

In an embodiment, the present invention relates to those compounds of Formula (I) and the pharmaceutically acceptable addition salts, and the solvates thereof, or any subgroup thereof as mentioned in any of the other embodiments, wherein a represents $-NR_4-C(=O)-[C(R_{5b})_2]_r-$; and r is 1.

In an embodiment, the present invention relates to those compounds of Formula (I) and the pharmaceutically acceptable addition salts, and the solvates thereof, or any subgroup thereof as mentioned in any of the other embodiments, wherein a represents$-NR_4-C(R_{5b})_2-C(=O)-$.

In an embodiment, the present invention relates to those compounds of Formula (I) and the pharmaceutically acceptable addition salts, and the solvates thereof, or any subgroup thereof as mentioned in any of the other embodiments, wherein in the 'b substituent', the linker with the 'a substituent' is present on $X_{d2}$ or is present on a carbon atom in the alpha position of $X_{d2}$.

In an embodiment, the present invention relates to those compounds of Formula (I) and the pharmaceutically acceptable addition salts, and the solvates thereof, or any subgroup thereof as mentioned in any of the other embodiments, wherein in the 'b substituent', the linker with the 'a substituent' is present on $X_{d2}$.

In an embodiment, the present invention relates to those compounds of Formula (I) and the pharmaceutically acceptable addition salts, and the solvates thereof, or any subgroup thereof as mentioned in any of the other embodiments, wherein in the 'b substituent', the linker with the 'a substituent' is present on $X_{d2}$; and wherein p1 is 1.

In an embodiment, the present invention relates to those compounds of Formula (I) and the pharmaceutically acceptable addition salts, and the solvates thereof, or any subgroup thereof as mentioned in any of the other embodiments, wherein $-X_1-$ represents - $(CHR_{12})-NR_1-X_e-C_{1-4}$alkanediyl- wherein $C_{1-4}$alkanediyl is optionally substituted with hydroxyl or hydroxy$C_{1-4}$alkyl; or $-X_1-$ represents $-NR_1-X_e-C_{2-4}$alkanediyl- wherein $C_{2-4}$alkanediyl is optionally substituted with hydroxyl or hydroxy$C_{1-4}$alkyl.

In an embodiment, the present invention relates to those compounds of Formula (I) and the pharmaceutically acceptable addition salts, and the solvates thereof, or any subgroup thereof as mentioned in any of the other embodiments, wherein $-X_1-$ represents $-(CHR_{12})-NR_1-X_e-C_{1-4}$alkanediyl-; or $-X_1-$ represents $-NR_1-X_e-C_{2-4}$alkanediyl-.

In an embodiment, the present invention relates to those compounds of Formula (I) and the pharmaceutically acceptable addition salts, and the solvates thereof, or any subgroup thereof as mentioned in any of the other embodiments, wherein p is 1.

In an embodiment, the present invention relates to those compounds of Formula (I) and the pharmaceutically acceptable addition salts, and the solvates thereof, or any subgroup thereof as mentioned in any of the other embodiments, wherein $R_3$ is H.

In an embodiment, the present invention relates to those compounds of Formula (I) and the pharmaceutically acceptable addition salts, and the solvates thereof, or any subgroup thereof as mentioned in any of the other embodiments, wherein $R_6$ is H.

In an embodiment, the present invention relates to those compounds of Formula (I) and the pharmaceutically

acceptable addition salts, and the solvates thereof, or any subgroup thereof as mentioned in any of the other embodiments, wherein

$-X_1-$ represents $-CH_2-NR_1-CH_2-C_{1-4}$alkanediyl-, $-NR_1-CH_2-C_{2-4}$alkanediyl-, or $-X_1-$represents one of the following groups wherein $-(CH_2)_2-$ is attached to 'variable a':

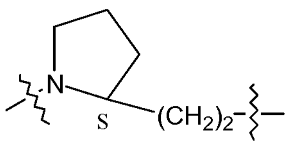 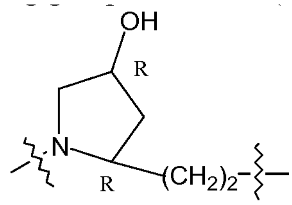 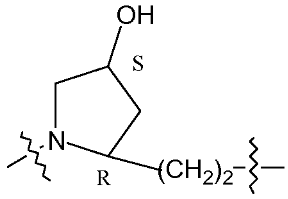

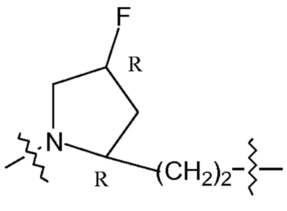 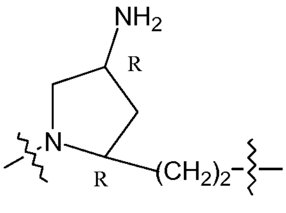 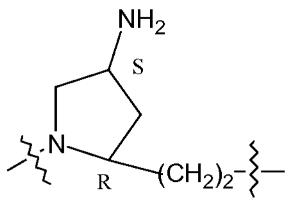

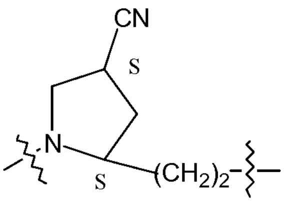 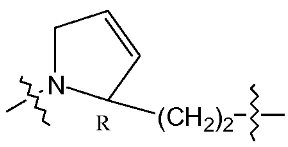 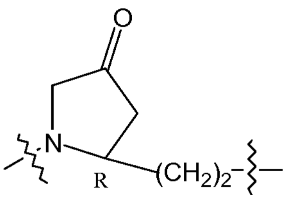

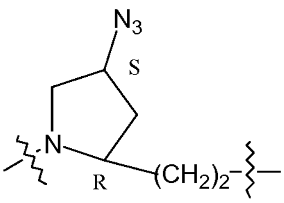 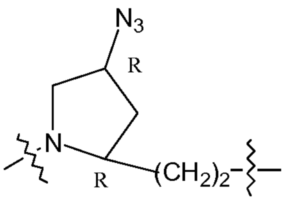 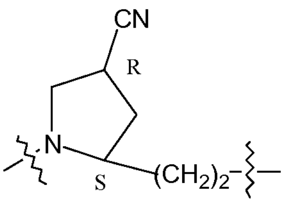

$R_1$ represents $C_{1-4}$alkyl, $C_{2-4}$alkenyl, $C_{2-4}$alkynyl, $C_{1-4}$alkyloxy$C_{1-4}$alkyl; in particular $R_1$ represents $C_{1-4}$alkyl, $C_{2-4}$alkenyl, or $C_{1-4}$alkyloxy$C_{1-4}$alkyl;

a represents $-NR_4-C(=O)-[C(R_{5b})_2]_r-$ or $-NR_4-C(R_{5b})_2-C(=O)-$; in particular a represents $-NR_4-C(=O)-[C(R_{5b})_2]_r-$; more in particular a represents $-NR_4-C(=O)-CH_2-$.

In an embodiment, the present invention relates to those compounds of Formula (I) and the pharmaceutically acceptable addition salts, and the solvates thereof, or any subgroup thereof as mentioned in any of the other embodiments, wherein

$-X_1-$ represents $-NH-(CH_2)_3-$, or $-X_1-$ represents one of the following groups wherein - $(CH_2)_2-$ is attached to 'variable a':

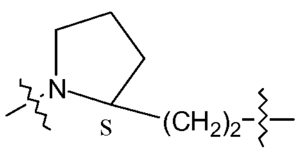 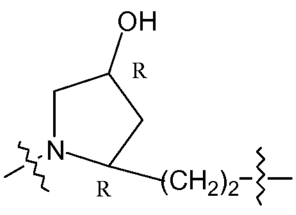 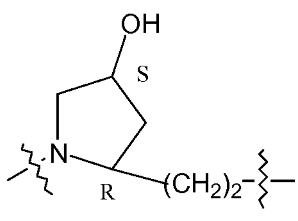 ;

a represents $-NR_4-C(=O)-[C(R_{5b})_2]_r-$ or $-NR_4-C(R_{5b})_2-C(=O)-$; in particular a represents $-NR_4-C(=O)-[C(R_{5b})_2]_r-$; more in particular a represents $-NR_4-C(=O)-CH_2-$.

In an embodiment, the present invention relates to those compounds of Formula (I) and the pharmaceutically acceptable addition salts, and the solvates thereof, or any subgroup thereof as mentioned in any of the other embodiments, wherein $-X_1-$ represents $-CH_2-NR_1-CH_2-C_{1-4}$alkanediyl- or $-NR_1-CH_2-C_{1-4}$alkanediyl-.

In an embodiment, the present invention relates to those compounds of Formula (I) and the pharmaceutically acceptable addition salts, and the solvates thereof, or any subgroup thereof as mentioned in any of the other embodiments, wherein $-X_1-$ represents one of the following groups wherein $-(CH_2)_2-$ is attached to 'variable a':

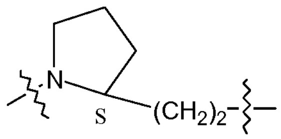 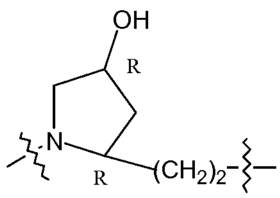 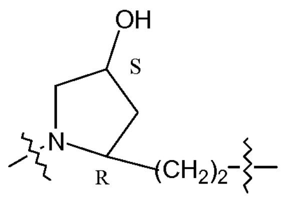

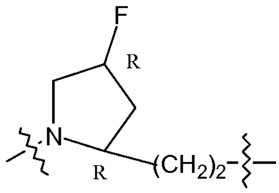 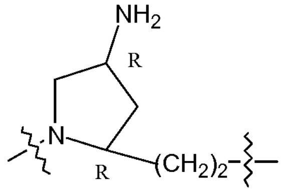 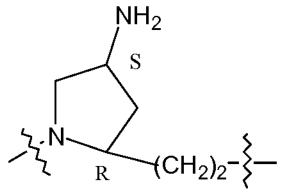

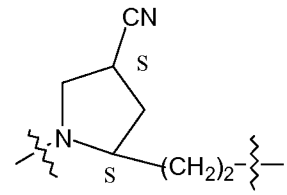 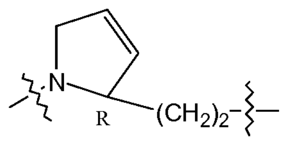 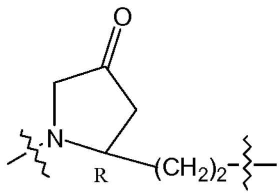

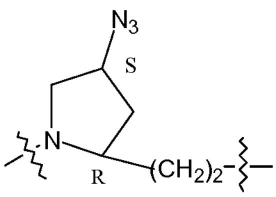 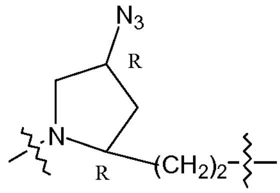 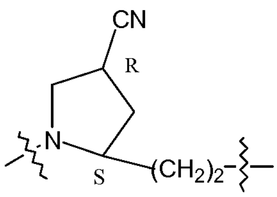 ;

a represents $-NR_4-C(=O)-[C(R_{5b})_2]_r-$ or $-NR_4-C(R_{5b})_2-C(=O)-$; in particular a represents $-NR_4-C(=O)-[C(R_{5b})_2]_r-$; more in particular a represents $-NR_4-C(=O)-CH_2-$.

In an embodiment, the present invention relates to those compounds of Formula (I) and the pharmaceutically acceptable addition salts, and the solvates thereof, or any subgroup thereof as mentioned in any of the other embodiments, wherein $-X_1-$ represents one of the following groups wherein $-(CH_2)_2-$ is attached to 'variable a':

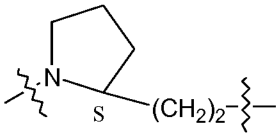 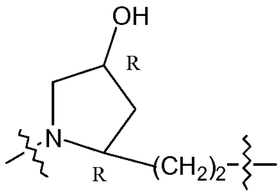 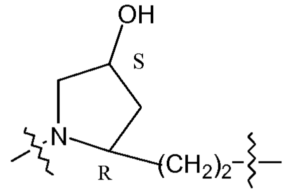 ;

a represents $-NR_4-C(=O)-[C(R_{5b})_2]_r-$ or $-NR_4-C(R_{5b})_2-C(=O)-$; in particular a represents $-NR_4-C(=O)-[C(R_{5b})_2]_r-$; more in particular a represents $-NR_4-C(=O)-CH_2-$.

In an embodiment, the present invention relates to those compounds of Formula (I) and the pharmaceutically acceptable addition salts, and the solvates thereof, or any subgroup thereof as mentioned in any of the other embodiments, wherein if $R_1$ is taken together with one $R_2$, the bond towards the second $R_2$ substituent is oriented as shown hereunder:

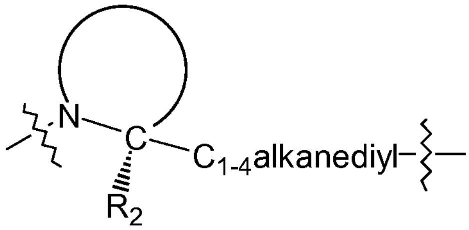

.

In an embodiment, the present invention relates to those compounds of Formula (I) and the pharmaceutically acceptable addition salts, and the solvates thereof, or any subgroup thereof as mentioned in any of the other embodiments, wherein if $R_1$ is taken together with one $R_2$, then $-X_1-$ represents the following group wherein $C_{1-4}$alkanediyl is attached to 'variable a':

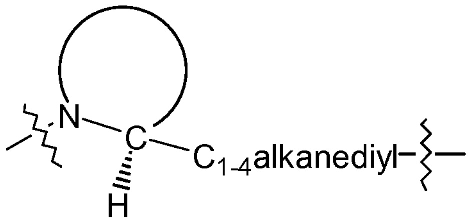

.

In an embodiment, the present invention relates to those compounds of Formula (I) and the pharmaceutically acceptable addition salts, and the solvates thereof, or any subgroup thereof as mentioned in any of the other embodiments, wherein $R_1$ is always taken together with one $R_2$.

In an embodiment, the present invention relates to those compounds of Formula (I) and the pharmaceutically acceptable addition salts, and the solvates thereof, or any subgroup thereof as mentioned in any of the other embodiments, wherein $R_1$ is always taken together with one $R_2$, and the bond towards the second $R_2$ substituent is oriented as shown hereunder:

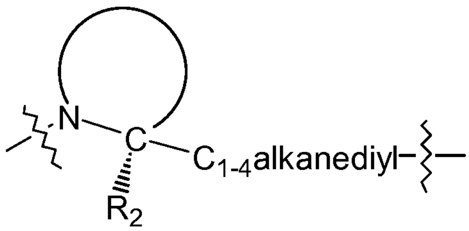

.

In an embodiment, the present invention relates to those compounds of Formula (I) and the pharmaceutically acceptable addition salts, and the solvates thereof, or any subgroup thereof as mentioned in any of the other embodiments, wherein each $R_3$ independently represents hydrogen; oxo; hydroxyl; carboxyl; $-NR_{3a}R_{3b}$; $-C(=O)-NR_{3a}R_{3b}$; hydroxy$C_{1-4}$alkyl; halo$C_{1-4}$alkyl; $-(C=O)-C_{1-4}$alkyl; $-C(=O)-O-C_{1-4}$alkyl wherein said $C_{1-4}$alkyl may optionally be substituted with phenyl; $C_{1-4}$alkyl optionally substituted with cyano, carboxyl, $C_{1-4}$alkyloxy, $-C(=O)-O-C_{1-4}$alkyl, $-O-C(=O)-C_{1-4}$alkyl, $-NR_{3e}R_{3f}$, $-C(=O)-NR_{3e}R_{3f}$, or $-SO_2-NR_{3e}R_{3f}$; hydroxy$C_{1-4}$alkyloxy$C_{1-4}$alkyl; $C_{1-4}$alkyloxyhydroxy$C_{1-4}$alkyl; hydroxy$C_{1-4}$alkyloxyhydroxy$C_{1-4}$alkyl; or $C_{1-4}$alkyloxy$C_{1-4}$alkyl optionally substituted with cyano, carboxyl, $C_{1-4}$alkyloxy, $-C(=O)-O-C_{1-4}$alkyl, $-O-C(=O)-C_{1-4}$alkyl, $-NR_{3e}R_{3f}$, $-C(=O)-NR_{3e}R_{3f}$, $-SO_2-NR_{3e}R_{3f}$, $R_{10}$, $-C(=O)-R_{10}$, or $-SO_2-R_{10}$.

In an embodiment, the present invention relates to those compounds of Formula (I) and the pharmaceutically acceptable addition salts, and the solvates thereof, or any subgroup thereof as mentioned in any of the other embodiments, wherein each $R_3$ independently represents hydrogen; hydroxy$C_{1-4}$alkyl; $C_{1-4}$alkyl; or $C_{1-4}$alkyloxy$C_{1-4}$alkyl optionally substituted with cyano or $-NR_{3e}R_{3f}$; or two $R_3$ substituents attached to the same carbon atom are taken together to form $C_{2-5}$alkanediyl.

In an embodiment, the present invention relates to those compounds of Formula (I) and the pharmaceutically acceptable addition salts, and the solvates thereof, or any subgroup thereof as mentioned in any of the other embodiments, wherein

a represents $-NR_4-C(=O)-[C(R_{5b})_2]_r-$; and

c represents a bond, $-[C(R_{5a})_2]_m-$, -O- or $-NR_{5a'}-$.

In an embodiment, the present invention relates to those compounds of Formula (I) and the pharmaceutically acceptable addition salts, and the solvates thereof, or any subgroup thereof as mentioned in any of the other embodiments, wherein

a represents $-NR_4-C(=O)-[C(R_{5b})_2]_r-$; r is 1; and

c represents a bond, $-[C(R_{5a})_2]_m-$, -O- or $-NR_{5a'}-$.

In an embodiment, the present invention relates to those compounds of Formula (I) and the pharmaceutically acceptable addition salts, and the solvates thereof, or any subgroup thereof as mentioned in any of the other embodiments, wherein $X_a$ is N;

a represents $-NR_4-C(=O)-[C(R_{5b})_2]_r-$, r is 1; and

c represents a bond, $-[C(R_{5a})_2]_m-$, -O- or $-NR_{5a'}-$.

In an embodiment, the present invention relates to those compounds of Formula (I) and the pharmaceutically acceptable addition salts, and the solvates thereof, or any subgroup thereof as mentioned in any of the other embodiments, wherein

a represents $-NR_4-C(=O)-[C(R_{5b})_2]_r-$, r is 1; and c represents $-[C(R_{5a})_2]_m-$.

In an embodiment, the present invention relates to those compounds of Formula (I) and the pharmaceutically acceptable addition salts, and the solvates thereof, or any subgroup thereof as mentioned in any of the other embodiments, wherein $X_a$ is N;

a represents $-NR_4-C(=O)-[C(R_{5b})_2]_r-$, r is 1; and c represents $-[C(R_{5a})_2]_m-$.

In an embodiment, the present invention relates to those compounds of Formula (I) and the pharmaceutically acceptable addition salts, and the solvates thereof, or any subgroup thereof as mentioned in any of the other embodiments, wherein

a represents $-NR_4-C(=O)-[C(R_{5b})_2]_r-$, r is 1; and c represents $-CH_2-$.

In an embodiment, the present invention relates to those compounds of Formula (I) and the pharmaceutically acceptable addition salts, and the solvates thereof, or any subgroup thereof as mentioned in any of the other embodiments, wherein $X_a$ is N;

a represents $-NR_4-C(=O)-[C(R_{5b})_2]_r-$, r is 1; and c represents $-CH_2-$.

In an embodiment, the present invention relates to those compounds of Formula (I) and the pharmaceutically acceptable addition salts, and the solvates thereof, or any subgroup thereof as mentioned in any of the other embodiments, wherein two $R_{5b}$ substituents attached to the same carbon atom are taken together to form $C_{2-5}$alkanediyl or $-(CH_2)_p-O-(CH_2)_p-$, in particular $C_{2-5}$alkanediyl.

In an embodiment, the present invention relates to those compounds of Formula (I) and the pharmaceutically acceptable addition salts, and the solvates thereof, or any subgroup thereof as mentioned in any of the other embodiments, wherein $X_a$ is N; and wherein two $R_{5b}$ substituents attached to the same carbon atom are taken together to form; $C_{2-5}$alkanediyl or $-(CH_2)_p-O-(CH_2)_p-$, in particular $C_{2-5}$alkanediyl.

In an embodiment, the present invention relates to those compounds of Formula (I) and the pharmaceutically acceptable addition salts, and the solvates thereof, or any subgroup thereof as mentioned in any of the other embodiments, wherein

a represents $-NR_4-C(=O)-[C(R_{5b})_2]_r-$; and wherein two $R_{5b}$ substituents attached to the same carbon atom are taken together to form $C_{2-5}$alkanediyl or $-(CH_2)_p-O-(CH_2)_p-$, in particular $C_{2-5}$alkanediyl.

In an embodiment, the present invention relates to those compounds of Formula (I) and the pharmaceutically acceptable addition salts, and the solvates thereof, or any subgroup thereof as mentioned in any of the other embodiments, wherein $X_a$ is N;

a represents $-NR_4-C(=O)-[C(R_{5b})_2]_r-$; and wherein two $R_{5b}$ substituents attached to the same carbon atom are taken together to form $C_{2-5}$alkanediyl or $-(CH_2)_p-O-(CH_2)_p-$, in particular $C_{2-5}$alkanediyl.

In an embodiment, the present invention relates to those compounds of Formula (I) and the pharmaceutically acceptable addition salts, and the solvates thereof, or any subgroup thereof as mentioned in any of the other embodiments, wherein

a represents $-NR_4-C(=O)-[C(R_{5b})_2]_r-$; r is 1; and wherein the two $R_{5b}$ substituents attached to the same carbon atom are taken together to form $C_{2-5}$alkanediyl or $-(CH_2)_p-O-(CH_2)_p-$, in particular $C_{2-5}$alkanediyl.

In an embodiment, the present invention relates to those compounds of Formula (I) and the pharmaceutically acceptable addition salts, and the solvates thereof, or any subgroup thereof as mentioned in any of the other embodiments, wherein $X_a$ is N;

a represents $-NR_4-C(=O)-[C(R_{5b})_2]_r-$; r is 1; and wherein the two $R_{5b}$ substituents attached to the same carbon atom are taken together to form $C_{2-5}$alkanediyl or $-(CH_2)_p-O-(CH_2)_p-$, in particular $C_{2-5}$alkanediyl.

In an embodiment, the present invention relates to those compounds of Formula (I) and the pharmaceutically acceptable addition salts, and the solvates thereof, or any subgroup thereof as mentioned in any of the other embodiments, wherein

a represents $-NR_4-C(=O)-[C(R_{5b})_2]_r-$; r is 1; wherein the two $R_{5b}$ substituents attached to the same carbon atom are taken together to form $C_{2-5}$alkanediyl or $-(CH_2)_p-O-(CH_2)_p-$, in particular $C_{2-5}$alkanediyl; and c represents $-CH_2-$.

In an embodiment, the present invention relates to those compounds of Formula (I) and the pharmaceutically acceptable addition salts, and the solvates thereof, or any subgroup thereof as mentioned in any of the other embodiments, wherein $X_a$ is N;
a represents $-NR_4-C(=O)-[C(R_{5b})_2]_r-$; r is 1; wherein the two $R_{5b}$ substituents attached to the same carbon atom are taken together to form $C_{2-5}$alkanediyl or $-(CH_2)_p-O-(CH_2)_p-$, in particular $C_{2-5}$alkanediyl; and c represents $-CH_2-$.

In an embodiment, the present invention relates to those compounds of Formula (I) and the pharmaceutically acceptable addition salts, and the solvates thereof, or any subgroup thereof as mentioned in any of the other embodiments, wherein
$-X_1-$ represents $-NR_1-X_e-C_{1-4}$alkanediyl- wherein said $C_{1-4}$alkanediyl moiety is optionally substituted with hydroxyl or hydroxy$C_{1-4}$alkyl;
$-X_e-$ represents $-C(R_2)_2-$; and
$R_1$ is taken together with $R_2$ to form $C_{1-4}$alkanediyl or $C_{2-4}$alkenediyl, each of said $C_{1-4}$alkanediyl and $C_{2-4}$alkenediyl optionally being substituted with 1 to 4 substituents each independently selected from hydroxyl, oxo, halo, cyano, $N_3$, hydroxy$C_{1-4}$alkyl, - $NR_7R_8$, $-SO_2-NR_7R_8$, $-NH-SO_2-NR_7R_8$, $-C(=O)-NR_7R_8$, or $-NH-C(=O)-NR_7R_8$.

In an embodiment, the present invention relates to those compounds of Formula (I) and the pharmaceutically acceptable addition salts, and the solvates thereof, or any subgroup thereof as mentioned in any of the other embodiments, wherein $X_a$ is N;
$-X_1-$ represents $-NR_1-X_e-C_{1-4}$alkanediyl- wherein said $C_{1-4}$alkanediyl moiety is optionally substituted with hydroxyl or hydroxy$C_{1-4}$alkyl;
$-X_e-$ represents $-C(R_2)_2-$; and
$R_1$ is taken together with $R_2$ to form $C_{1-4}$alkanediyl or $C_{2-4}$alkenediyl, each of said $C_{1-4}$alkanediyl and $C_{2-4}$alkenediyl optionally being substituted with 1 to 4 substituents each independently selected from hydroxyl, oxo, halo, cyano, $N_3$, hydroxy$C_{1-4}$alkyl, - $NR_7R_8$, $-SO_2-NR_7R_8$, $-NH-SO_2-NR_7R_8$, $-C(=O)-NR_7R_8$, or $-NH-C(=O)-NR_7R_8$.

In an embodiment, the present invention relates to those compounds of Formula (I) and the pharmaceutically acceptable addition salts, and the solvates thereof, or any subgroup thereof as mentioned in any of the other embodiments, wherein
$-X_1-$ represents $-NR_1-X_e-C_{1-4}$alkanediyl- wherein said $C_{1-4}$alkanediyl moiety is optionally substituted with hydroxyl or hydroxy$C_{1-4}$alkyl;
$-X_e-$ represents $-C(R_2)_2-$; and
$R_1$ is taken together with $R_2$ to form $C_{1-4}$alkanediyl substituted with 1 hydroxyl substituent.

In an embodiment, the present invention relates to those compounds of Formula (I) and the pharmaceutically acceptable addition salts, and the solvates thereof, or any subgroup thereof as mentioned in any of the other embodiments, wherein $X_a$ is N;
$-X_1-$ represents $-NR_1-X_e-C_{1-4}$alkanediyl- wherein said $C_{1-4}$alkanediyl moiety is optionally substituted with hydroxyl or hydroxy$C_{1-4}$alkyl;
$-X_e-$ represents $-C(R_2)_2-$; and
$R_1$ is taken together with $R_2$ to form $C_{1-4}$alkanediyl substituted with 1 hydroxyl substituent.

In an embodiment, the present invention relates to those compounds of Formula (I) and the pharmaceutically acceptable addition salts, and the solvates thereof, or any subgroup thereof as mentioned in any of the other embodiments, wherein $-X_1-$ represents

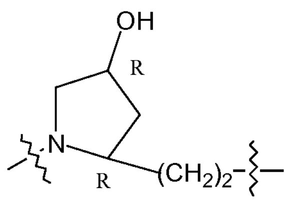

wherein $-(CH_2)_2-$ is attached to 'variable a'.

In an embodiment, the present invention relates to those compounds of Formula (I) and the pharmaceutically acceptable addition salts, and the solvates thereof, or any subgroup thereof as mentioned in any of the other embodiments, wherein $X_a$ is N; and $-X_1-$represents

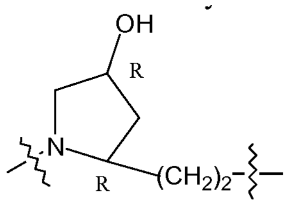

wherein $-(CH_2)_2-$ is attached to 'variable a'.

In an embodiment, the present invention relates to those compounds of Formula (I) and the pharmaceutically acceptable addition salts, and the solvates thereof, or any subgroup thereof as mentioned in any of the other embodiments, wherein $-X_1-$ represents one of the following groups wherein $-(CH_2)_2-$ is attached to 'variable a':

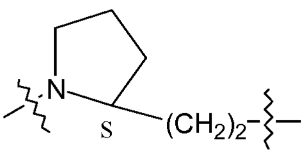 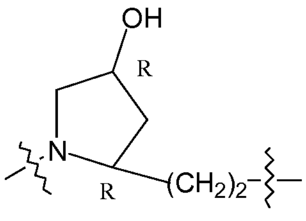 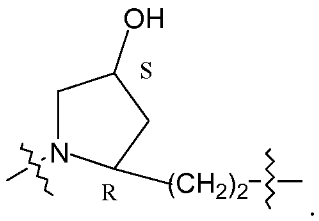.

In an embodiment, the present invention relates to those compounds of Formula (I) and the pharmaceutically acceptable addition salts, and the solvates thereof, or any subgroup thereof as mentioned in any of the other embodiments, wherein $X_a$ is N; and $-X_1-$represents one of the following groups wherein $-(CH_2)_2-$ is attached to 'variable a':

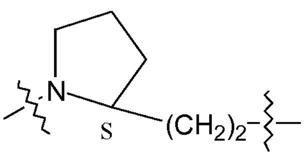 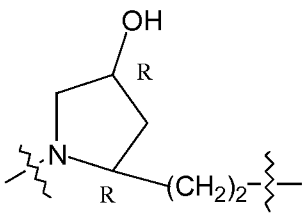 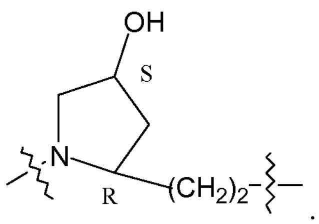.

In an embodiment, the present invention relates to those compounds of Formula (I) and the pharmaceutically acceptable addition salts, and the solvates thereof, or any subgroup thereof as mentioned in any of the other embodiments, wherein

a represents $-NR_4-C(=O)-[C(R_{5b})_2]_r-$; r is 1; wherein the two $R_{5b}$ substituents attached to the same carbon atom are taken together to form $C_{2-5}$alkanediyl;

c represents $-CH_2-$;

$-X_1-$ represents $-NR_1-X_e-C_{1-4}$alkanediyl- wherein said $C_{1-4}$alkanediyl moiety is optionally substituted with hydroxyl or hydroxy$C_{1-4}$alkyl;

$-X_e-$ represents $-C(R_2)_2-$; and

$R_1$ is taken together with $R_2$ to form $C_{1-4}$alkanediyl substituted with 1 hydroxyl substituent.

In an embodiment, the present invention relates to those compounds of Formula (I) and the pharmaceutically acceptable addition salts, and the solvates thereof, or any subgroup thereof as mentioned in any of the other embodiments, wherein $X_a$ is N;

a represents $-NR_4-C(=O)-[C(R_{5b})_2]_r-$; r is 1; wherein the two $R_{5b}$ substituents attached to the same carbon atom are taken together to form $C_{2-5}$alkanediyl;

c represents $-CH_2-$;

$-X_1-$ represents $-NR_1-X_e-C_{1-4}$alkanediyl- wherein said $C_{1-4}$alkanediyl moiety is optionally substituted with hydroxyl or hydroxy$C_{1-4}$alkyl;

$-X_e-$ represents $-C(R_2)_2-$; and

$R_1$ is taken together with $R_2$ to form $C_{1-4}$alkanediyl substituted with 1 hydroxyl substituent.

In an embodiment, the present invention relates to those compounds of Formula (I) and the pharmaceutically acceptable addition salts, and the solvates thereof, or any subgroup thereof as mentioned in any of the other embodiments, wherein

a represents $-NR_4-C(=O)-[C(R_{5b})_2]_r-$; r is 1; wherein the two $R_{5b}$ substituents attached to the same carbon atom are taken together to form $C_{2-5}$alkanediyl;
c represents $-CH_2-$;
$-X_1-$ represents $-NR_1-X_e-C_{1-4}$alkanediyl- wherein said $C_{1-4}$alkanediyl moiety is optionally substituted with hydroxyl or hydroxy$C_{1-4}$alkyl;
$-X_e-$ represents $-C(R_2)_2-$; and
$R_1$ is taken together with $R_2$ to form $C_{1-4}$alkanediyl or $C_{2-4}$alkenediyl, each of said $C_{1-4}$alkanediyl and $C_{2-4}$alkenediyl optionally being substituted with 1 to 4 substituents each independently selected from hydroxyl, oxo, halo, cyano, $N_3$, hydroxy$C_{1-4}$alkyl, $-NR_7R_8$, - $SO_2-NR_7R_8$, $-NH-SO_2-NR_7R_8$, $-C(=O)-NR_7R_8$, or $-NH-C(=O)-NR_7R_8$.

In an embodiment, the present invention relates to those compounds of Formula (I) and the pharmaceutically acceptable addition salts, and the solvates thereof, or any subgroup thereof as mentioned in any of the other embodiments, wherein $X_a$ is N;
a represents $-NR_4-C(=O)-[C(R_{5b})_2]_r-$; r is 1; wherein the two $R_{5b}$ substituents attached to the same carbon atom are taken together to form $C_{2-5}$alkanediyl;
c represents $-CH_2-$;
$-X_1-$ represents $-NR_1-X_e-C_{1-4}$alkanediyl- wherein said $C_{1-4}$alkanediyl moiety is optionally substituted with hydroxyl or hydroxy$C_{1-4}$alkyl;
$-X_e-$ represents $-C(R_2)_2-$; and
$R_1$ is taken together with $R_2$ to form $C_{1-4}$alkanediyl or $C_{2-4}$alkenediyl, each of said $C_{1-4}$alkanediyl and $C_{2-4}$alkenediyl optionally being substituted with 1 to 4 substituents each independently selected from hydroxyl, oxo, halo, cyano, $N_3$, hydroxy$C_{1-4}$alkyl, - $NR_7R_8$, $-SO_2-NR_7R_8$, $-NH-SO_2-NR_7R_8$, $-C(=O)-NR_7R_8$, or $-NH-C(=O)-NR_7R_8$.

In an embodiment, the present invention relates to those compounds of Formula (I) and the pharmaceutically acceptable addition salts, and the solvates thereof, or any subgroup thereof as mentioned in any of the other embodiments, wherein
a represents $-NR_4-C(=O)-[C(R_{5b})_2]_r-$, r is 1; wherein the two $R_{5b}$ substituents attached to the same carbon atom are taken together to form $C_{2-5}$alkanediyl;
c represents $-CH_2-$; and $-X_1-$ represents

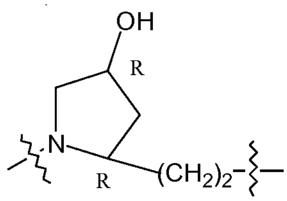

wherein $-(CH_2)_2-$ is attached to 'variable a'.

In an embodiment, the present invention relates to those compounds of Formula (I) and the pharmaceutically acceptable addition salts, and the solvates thereof, or any subgroup thereof as mentioned in any of the other embodiments, wherein $X_a$ is N; a represents $-NR_4-C(=O)-[C(R_{5b})_2]_r-$, r is 1; wherein the two $R_{5b}$ substituents attached to the same carbon atom are taken together to form $C_{2-5}$alkanediyl;
c represents $-CH_2-$; and $-X_1-$ represents

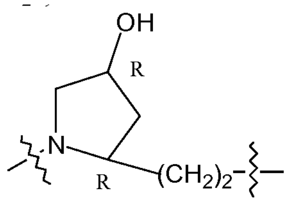

wherein $-(CH_2)_2-$ is attached to 'variable a'.

In an embodiment, the present invention relates to those compounds of Formula (I) and the pharmaceutically acceptable addition salts, and the solvates thereof, or any subgroup thereof as mentioned in any of the other embodiments, wherein
a represents $-NR_4-C(=O)-[C(R_{5b})_2]_r-$, r is 1; wherein the two $R_{5b}$ substituents attached to the same carbon atom are taken together to form $C_{2-5}$alkanediyl;
c represents $-CH_2-$; and $-X_1-$ represents one of the following groups wherein $-(CH_2)_2-$ is attached to 'variable a':

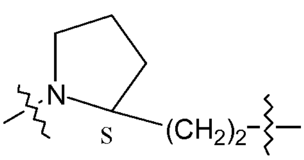 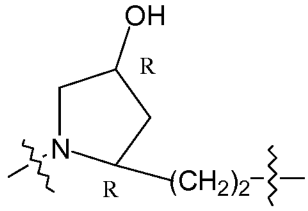 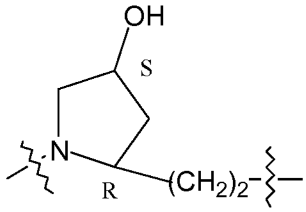 .

In an embodiment, the present invention relates to those compounds of Formula (I) and the pharmaceutically acceptable addition salts, and the solvates thereof, or any subgroup thereof as mentioned in any of the other embodiments, wherein $X_a$ is N;
a represents $-NR_4-C(=O)-[C(R_{5b})_2]_r-$, r is 1; wherein the two $R_{5b}$ substituents attached to the same carbon atom are taken together to form $C_{2-5}$alkanediyl;
c represents $-CH_2-$; and
$-X_1-$ represents one of the following groups wherein $-(CH_2)_2-$ is attached to 'variable a':

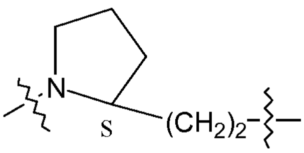 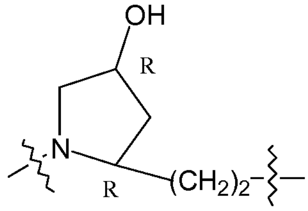 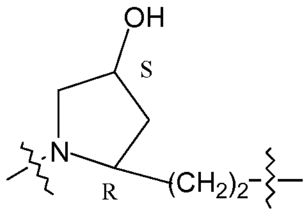 .

In an embodiment, the present invention relates to a subgroup of Formula (I) as defined in the general reaction schemes.

In an embodiment the compound of Formula (I) is selected from the group consisting of compounds 2, 6, 10, 23, 33, 36, 44, 46, 47, 53, 54, 55, 56, 57, 59, 62, 65, 66, 76, 104, and 107, tautomers and stereoisomeric forms thereof, and the pharmaceutically acceptable addition salts, and the solvates thereof.

In an embodiment the compound of Formula (I) is selected from the group consisting of compounds 43, 107, 1, 62, 57, 56, 64, 20, 22, 81, 65, 53, 97, 11, 35, 52, 89, 96 and 50, tautomers and stereoisomeric forms thereof,
and the pharmaceutically acceptable addition salts, and the solvates thereof.

In an embodiment the compound of Formula (I) is selected from the group consisting of any of the exemplified compounds, tautomers and stereoisomeric forms thereof,
and the free bases, the pharmaceutically acceptable addition salts, and the solvates thereof.

All possible combinations of the above-indicated embodiments are considered to be embraced within the scope of this invention.

**Methods for the Preparation of Compounds of Formula (I)**

In this section, as in all other sections unless the context indicates otherwise, references to Formula (I) also include all other sub-groups and examples thereof as defined herein.

The general preparation of some typical examples of the compounds of Formula (I) is described hereunder and in the specific examples, and are generally prepared from starting materials which are either commercially available or prepared by standard synthetic processes commonly used by those skilled in the art. The following schemes are only meant to represent examples of the invention and are in no way meant to be a limit of the invention.

Alternatively, compounds of the present invention may also be prepared by analogous reaction protocols as described in the general schemes below, combined with standard synthetic processes commonly used by those skilled in the art of organic chemistry. Additionally, compounds of the present invention may also be prepared by analogous reaction protocols as described in the general schemes below combined with methods described in WO2009112439. Starting materials may also be prepared by methods as described in the literature for example by the procedures described WO2009150230, WO2004105765, WO2005058318, WO2005058913, WO2006061415, WO2006061417, WO2009016132, WO2008155421 and WO2007003525.

The skilled person will realize that in the reactions described in the Schemes, it may be necessary to protect reactive functional groups, for example hydroxy, amino (for example $NHR_4$ in an intermediate of Formula (XXIII-a)), or carboxy groups, where these are desired in the final product, to avoid their unwanted participation in the reactions.

Conventional protecting groups can be used in accordance with standard practice. This is illustrated in the specific examples. The protecting groups may be removed at a convenient subsequent stage using methods known from the art.

The skilled person will realize that in the reactions described in the Schemes, it may be advisable or necessary to perform the reaction under an inert atmosphere, such as for example under $N_2$-gas atmosphere, for example when NaH is used in the reaction.

It will be apparent for the skilled person that it may be necessary to cool the reaction mixture before reaction work-up (refers to the series of manipulations required to isolate and purify the product(s) of a chemical reaction such as for example quenching, column chromatography, extraction).

The skilled person will realize that heating the reaction mixture under stirring may enhance the reaction outcome. In some reactions microwave heating may be used instead of conventional heating to shorten the overall reaction time.

The skilled person will realize that another sequence of the chemical reactions shown in the Schemes below, may also result in the desired compound of Formula (I).

The skilled person will realize that intermediates and final compounds shown in the schemes below may be further functionalized according to methods well-known by the person skilled in the art. Examples are shown in the specific experimental part.

The skilled person will realize that more Compounds of Formula (I) can be prepared by using analogous synthetic protocols as described in the Schemes below. For example, general schemes wherein $(SO_2)_{p3}$ is not present in the $X_1$ linker, typically can also be used to prepare compounds with $(SO_2)_{p3}$ as part of the $X_1$ linker.

In case one of the starting materials is available as a salt form, the skilled person will realize that it may be necessary to first treat the salt with a base, such as for example DIPEA.

Although not shown in the general schemes, the rings in the position of ring b, may also contain extra bonds to form a bridged ring according to the scope.

In the schemes below, the $C_{1-4}$alkanediyl moiety in the intermediates and the final compounds, such as for example the $C_{1-4}$alkanediyl moiety in the $-X_1$-linker, is optionally substituted as defined in the scope.

All variables are defined as mentioned hereabove unless otherwise is indicated or is clear from the context.

In general, compounds of Formula (I-a) can be prepared according to Scheme 1:

Scheme 1

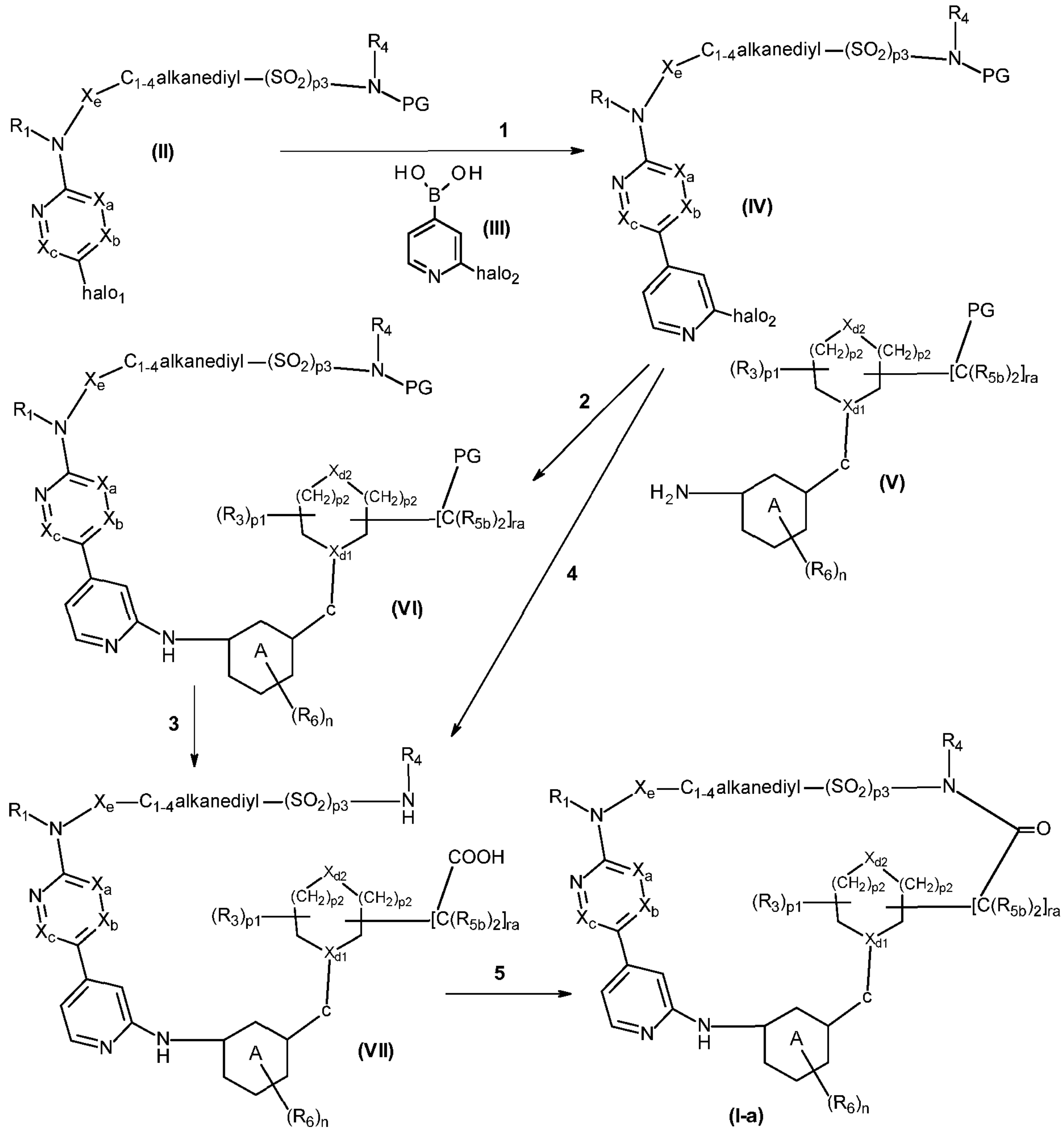


In scheme 1, 'halo$_1$' is defined as Br, I or Cl; 'halo$_2$' is defined as Cl or F; 'PG' is defined as a protecting group such as for example *tert*-butoxycarbonyl (Boc), methoxycarbonyl or ethoxycarbonyl; and 'ra' is defined as 1 or 2. All other variables in Scheme 1 are defined according to the scope of the present invention.

In Scheme 1, the following reaction conditions apply:

**1**: in a suitable mixture of solvents such as for example water/dioxane, in the presence of a suitable base, such as for example $Na_2CO_3$, in the presence of a catalyst such as for example tetrakis(triphenylphosphine)palladium ($Pd(PPh_3)_4$);

**2** (only for halo$_2$ is Cl): Buchwald-Hartwig amination; reaction between an intermediate of Formula (IV) and (V), typically in a suitable solvent such as for example dioxane, in the presence of a suitable base such as for example $Cs_2CO_3$, in the presence of a catalyst such as tris(dibenzylideneacetone)dipalladium ($Pd_2(dba)_3$), in the presence of a ligand such as for example 2-dicyclohexylphosphino-2',6'-dimethoxybiphenyl (S-Phos);

**3:** in the presence of an acid such as for example trifluoroacetic acid (TFA) in a solvent such as for example DCM; or alternatively in the presence of an acid such as for example HCl in a solvent such as for example 1,4-dioxane

optionally in the presence of water; or
alternatively first in the presence of a base such as for example NaOH, and subsequently in the presence of an acid such as for example HCl, in the presence of a suitable solvent such as for example THF;
**4:** coupling reaction between an intermediate of Formula (IV) with an intermediate of Formula (V) under acidic conditions; typically in a suitable solvent such as for example n-butanol, in the presence of an acid such as HCl (e.g. a 6M solution of HCl in 2-propanol);
**5:** in the presence of a coupling agent such as for example diethyl cyanophosphonate, (1H-benzotriazol-1-yloxy)(tripyrrolidin-1-yl)phosphonium hexafluorophosphate (PyBOP), 1-[bis(dimethylamino)methylene]-1H-benzotriazol-1-ium 3-oxide hexafluorophosphate (HBTU) or 1-[bis(dimethylamino)methylene]-1H-[1,2,3]triazolo[4,5-b]pyridin-1-ium 3-oxide hexafluorophosphate (HATU) in the presence of a base such as for example triethylamine ($Et_3N$) or N,N-diisopropylethylamine (DIPEA), in a suitable solvent such as for example DMF.

Intermediates of Formula (II), (III) and (V) are commercially available or can be prepared by standard means obvious to those skilled in the art or as described in the specific experimental part.
An intermediate of Formula (IV) wherein $X_a$ is N and $X_b$ and $X_c$ are CH, hereby named an intermediate of Formula (IV-a), alternatively may be prepared according to the method described in Scheme 1a:

Scheme 1a

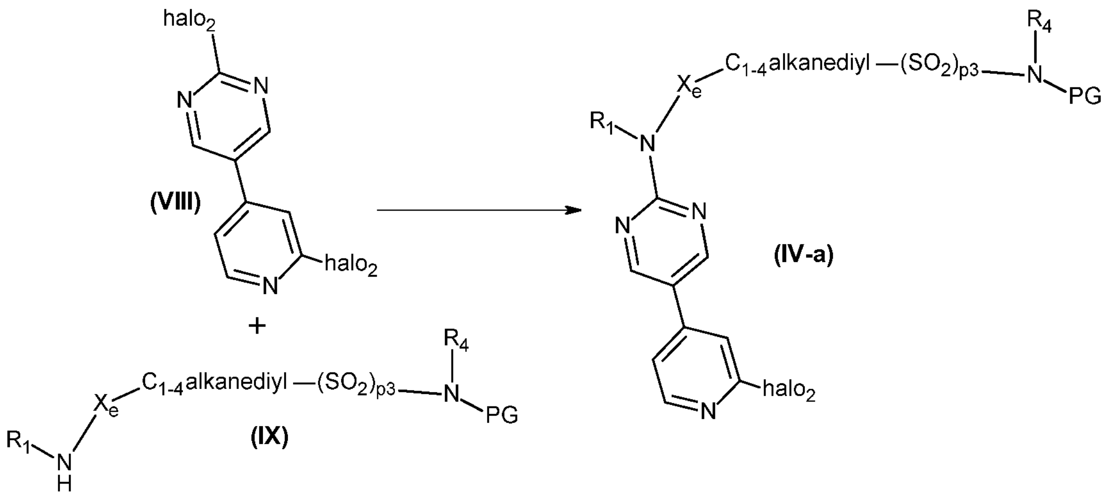

In scheme 1a, 'halo$_2$' is defined as F or Cl; 'PG' is defined as a protecting group such as for example *tert*-butoxycarbonyl, methoxycarbonyl or ethoxycarbonyl; and all other variables are defined according to the scope of the present invention.
Intermediates of Formula (VIII) and (IX) are commercially available or can be prepared by standard means obvious to those skilled in the art or as described in the specific experimental part.
An intermediate of Formula (VII) wherein $X_a$ is N and $X_b$ and $X_c$ are CH, and wherein $R_1$ and one $R_2$ are taken together to form $C_{1-4}$alkanediyl or $C_{2-4}$alkenediyl, each substituted with hydroxyl, wherein p3 is 0, and wherein $R_4$ is hydrogen, hereby named an intermediate of Formula (VII-a), alternatively may be prepared according to the method described in Scheme 1b:

Scheme 1b

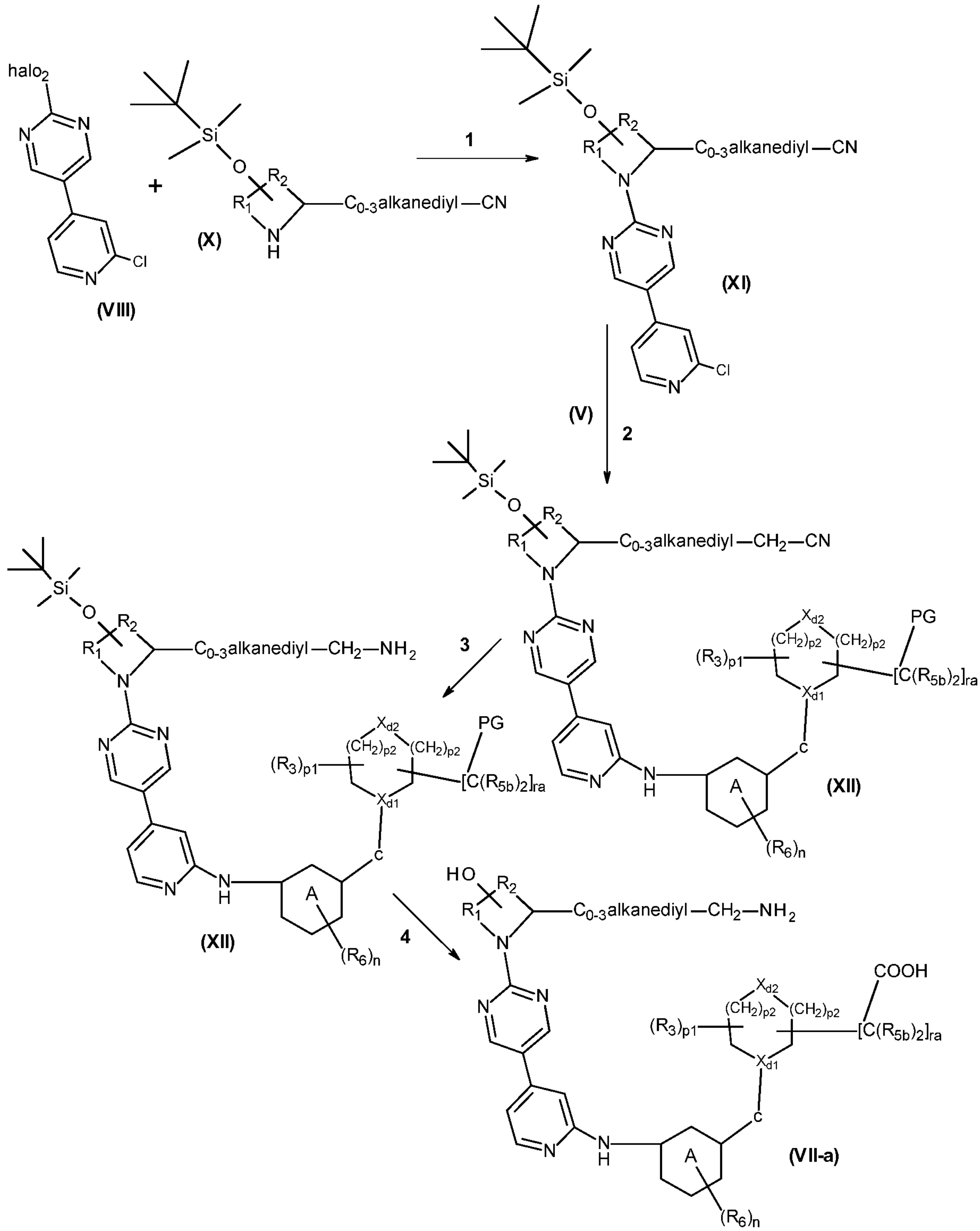


In scheme 1b, 'halo$_2$' is defined as Cl or F; 'PG' is defined as a protecting group such as for example *tert*-butoxycarbonyl, methoxycarbonyl or ethoxycarbonyl; and all other variables are defined as mentioned before.

In Scheme 1, the following reaction conditions apply:

**1**: coupling reaction between an intermediate of Formula (VIII) and (X) in the presence of a base such as for example $Na_2CO_3$, triethylamine ($Et_3N$) or N,N-diisopropylethylamine (DIPEA), in a suitable solvent such as for example DMF;
**2:** coupling reaction between an intermediate of Formula (XI) and (V) in a suitable solvent such as for example tert-butanol, in the presence of a suitable base, such as for example $K_2CO_3$, in the presence of a metal such as

$(Pd_2(dba)_3)$, in the presence of a ligand such as X-Phos (dicyclohexyl[2',4',6'-tris(1-methylethyl)[1,1'-biphenyl]-2-yl]-phosphine);

**3:** reduction of the cyano group in the presence of $H_2$-gas atmosphere in a suitable solvent such as for example methanol (MeOH) in the presence of a base such as for example $NH_4OH$, in the presence of a catalyst such as for example Raney Nickel;

**4:** in the presence of an acid such as for example trifluoroacetic acid (TFA) in a solvent such as for example DCM; or

alternatively in the presence of an acid such as for example HCl in a solvent such as for example 1,4-dioxane optionally in the presence of water; or

alternatively first in the presence of a base such as for example NaOH, and subsequently in the presence of an acid such as for example HCl, in the presence of a suitable solvent such as for example THF.

Intermediates of Formula (VIII) and (X) are commercially available or can be prepared by standard means obvious to those skilled in the art or as described in the specific experimental part.

In general, compounds of Formula (I-b) can be prepared according to Scheme 2:

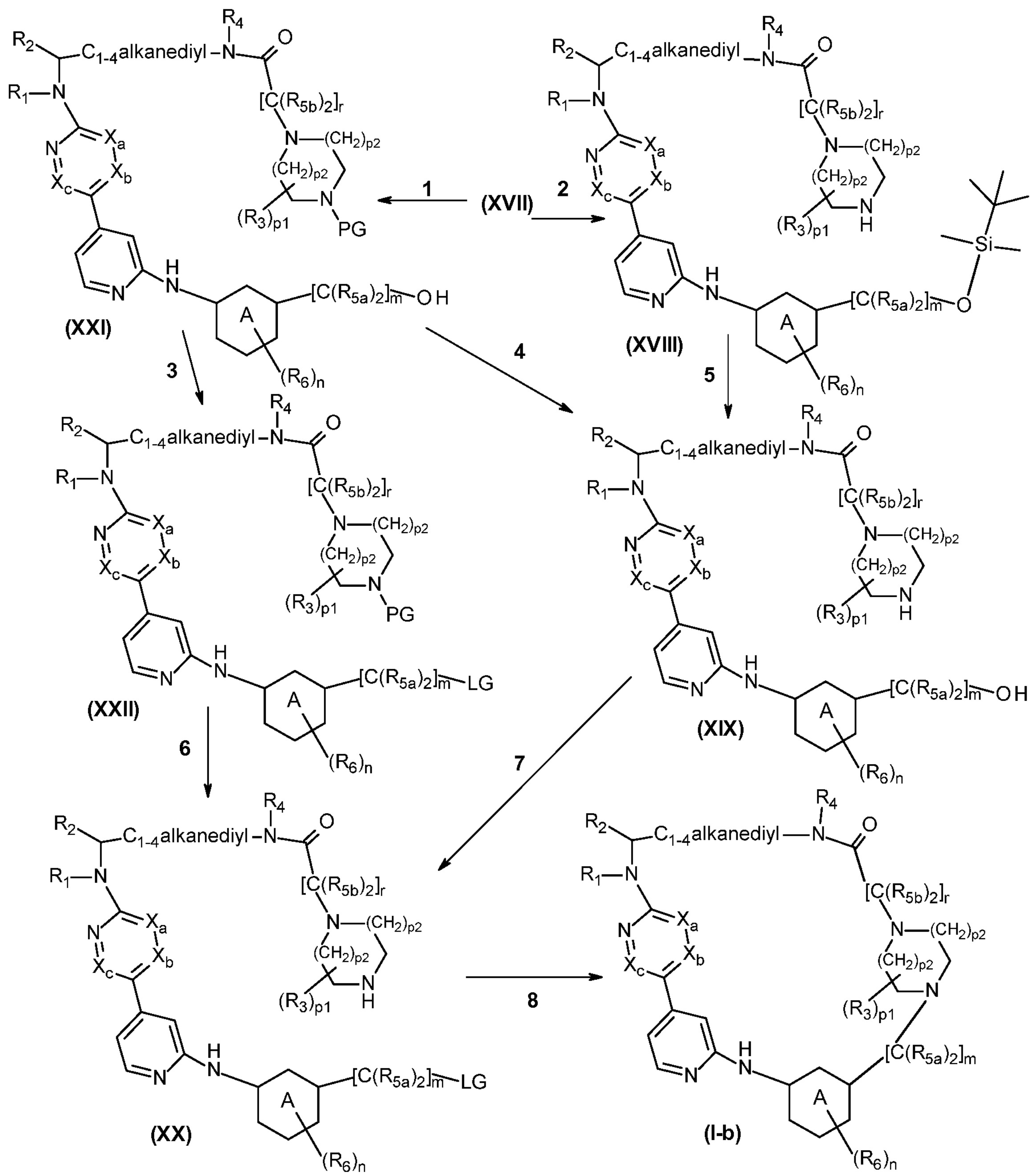

In scheme 2, 'PG' is as defined before and in this scheme additionally may also be a benzyl group; 'LG' means leaving group such as for example chloro or mesylate; and all other variables are defined according to the scope of the present invention.

The skilled person will realize that protecting groups can be easily converted into each other by using well-known reactions as illustrated in the specific examples.

In Scheme 2, the following reaction conditions apply:

**1**: deprotection of the hydroxyl group by addition of an appropriate deprotecting agent such as for example tetrabutylammonium fluoride, in the presence of a suitable solvent such as for example THF;

**2:** deprotection of the piperazinyl moiety in the presence of $H_2$-gas atmosphere and a catalyst such as for example Pd/C (for example 5 wt % or 10 wt %) in a suitable solvent such as for example MeOH;

**3:** introduction of a leaving group (LG) using sulfonyl chlorides such as for example methanesulfonyl chloride (MsCl) or p-toluenesulfonyl chloride (TsCl) in the presence of a suitable base such as for example DIPEA, in the presence of a suitable solvent such as for example DCM;

**4:** deprotection of the piperazinyl moiety in the presence of an acid such as for example TFA in a solvent such as for example DCM; or alternatively in the presence of an acid such as for example HCl in a solvent such as for example 1,4-dioxane optionally in the presence of water;

**5:** in the presence of a deprotecting agent such as for example TBAF in THF; or alternatively in the presence of an acid such as for example HCl in $H_2O$; or alternatively in the presence of $CH_3COOH$ optionally in the presence of water;

**6:** deprotection of the piperazinyl moiety in the presence of an acid such as for example TFA in a solvent such as for example DCM; or alternatively in the presence of an acid such as for example HCl in a solvent such as for example 1,4-dioxane optionally in the presence of water;

**7:** introduction of a leaving group (LG) using for example thionyl chloride in the presence of a suitable solvent such as for example 1,2-dichloroethane;

**8:** in the presence of a suitable base, such as for example $K_2CO_3$, in the presence of a suitable solvent such as for example DMF;

In general, an intermediate of Formula (XVII) can be prepared according to Scheme 2b:

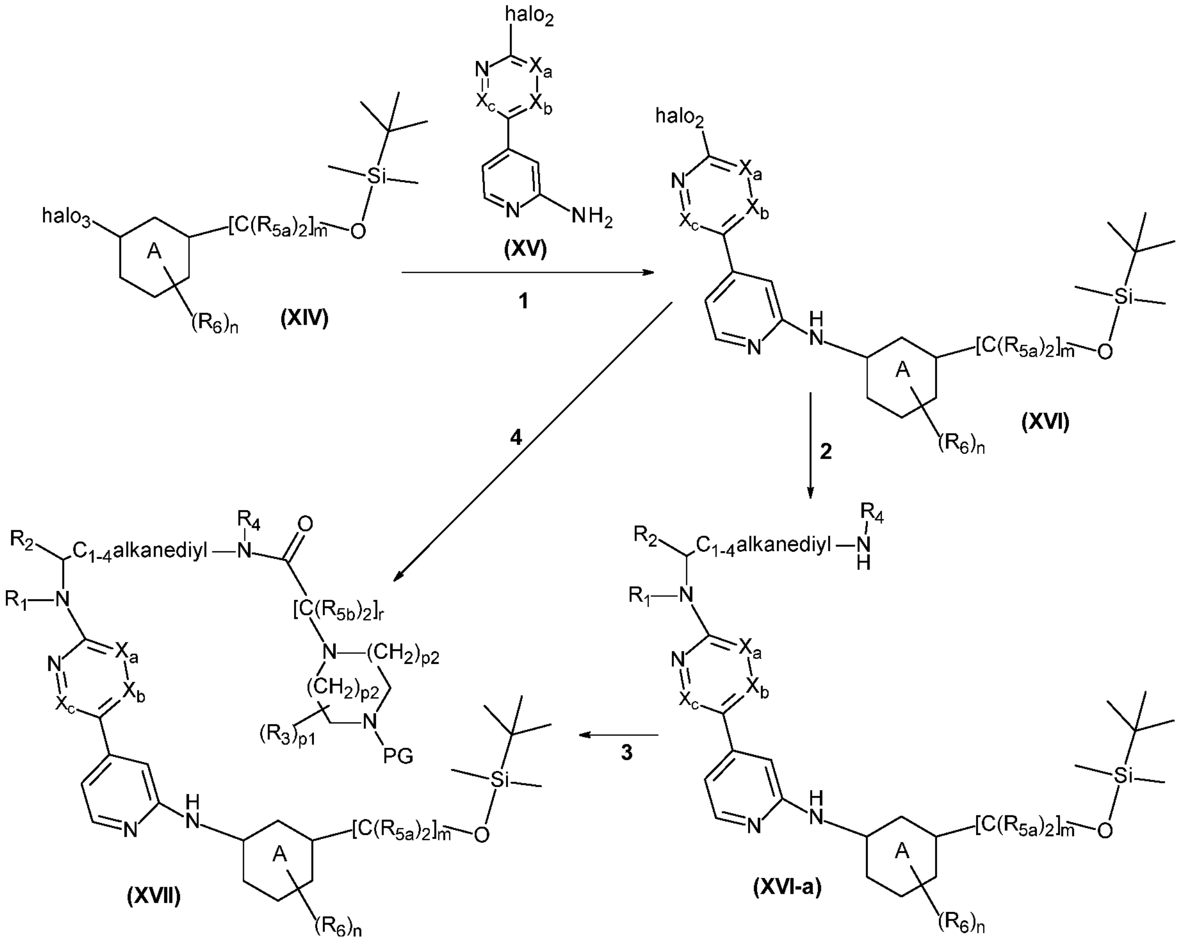


In scheme 2b, 'PG' is as defined before and in this scheme additionally may also be a benzyl group; 'halo$_3$' is defined as Br or I; 'halo2' is as defined before in the general reaction schemes; and all other variables are defined according to the scope of the present invention.

In Scheme 2b, the following reaction conditions apply:

**1**: in a solvent or a mixture of solvents such as dioxane/THF, in the presence of a suitable base such as for example $Cs_2CO_3$, in the presence of a catalyst such as for example Pd(II) acetate, together with a ligand such as 9,9-dimethyl-4,5-bis(diphenylphosphino)xanthene;

**2:** reaction with an intermediate of Formula (XVI-a1):

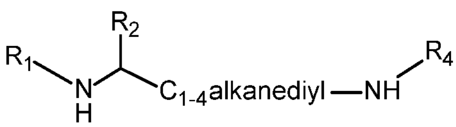

**(XVI-a1)**

optionally in the presence of a suitable base, such as for example $Na_2CO_3$, optionally in the presence of a suitable solvent such as for example DMA or NMP, or in a mixture of solvents such as for example DMA/DMSO ("DMSO" means dimethyl sulfoxide);

**3:** firstly reaction with an intermediate of Formula (XVII-a) in the presence of a suitable base, such as for example $Et_3N$, in the presence of a suitable solvent such as for example $CH_3CN$; and subsequently addition of (XVII-b) to the mixture:

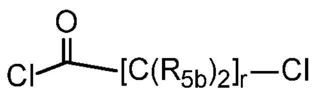 **(XVII-a)** 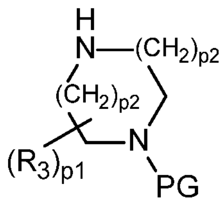 **(XVII-b)**

**4:** reaction with an intermediate of Formula (XVI-a2):

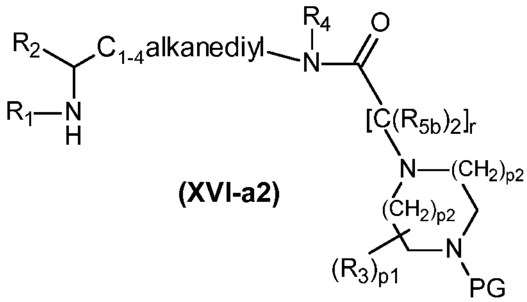

**(XVI-a2)**

in the presence of a suitable base, such as for example $K_2CO_3$, in the presence of a suitable solvent such as for example DMF.

The starting materials in scheme 2b are commercially available or can be prepared by standard means obvious to those skilled in the art or as described in the specific experimental part.

In general, compounds of Formula (I-c) can be prepared according to Scheme 3:

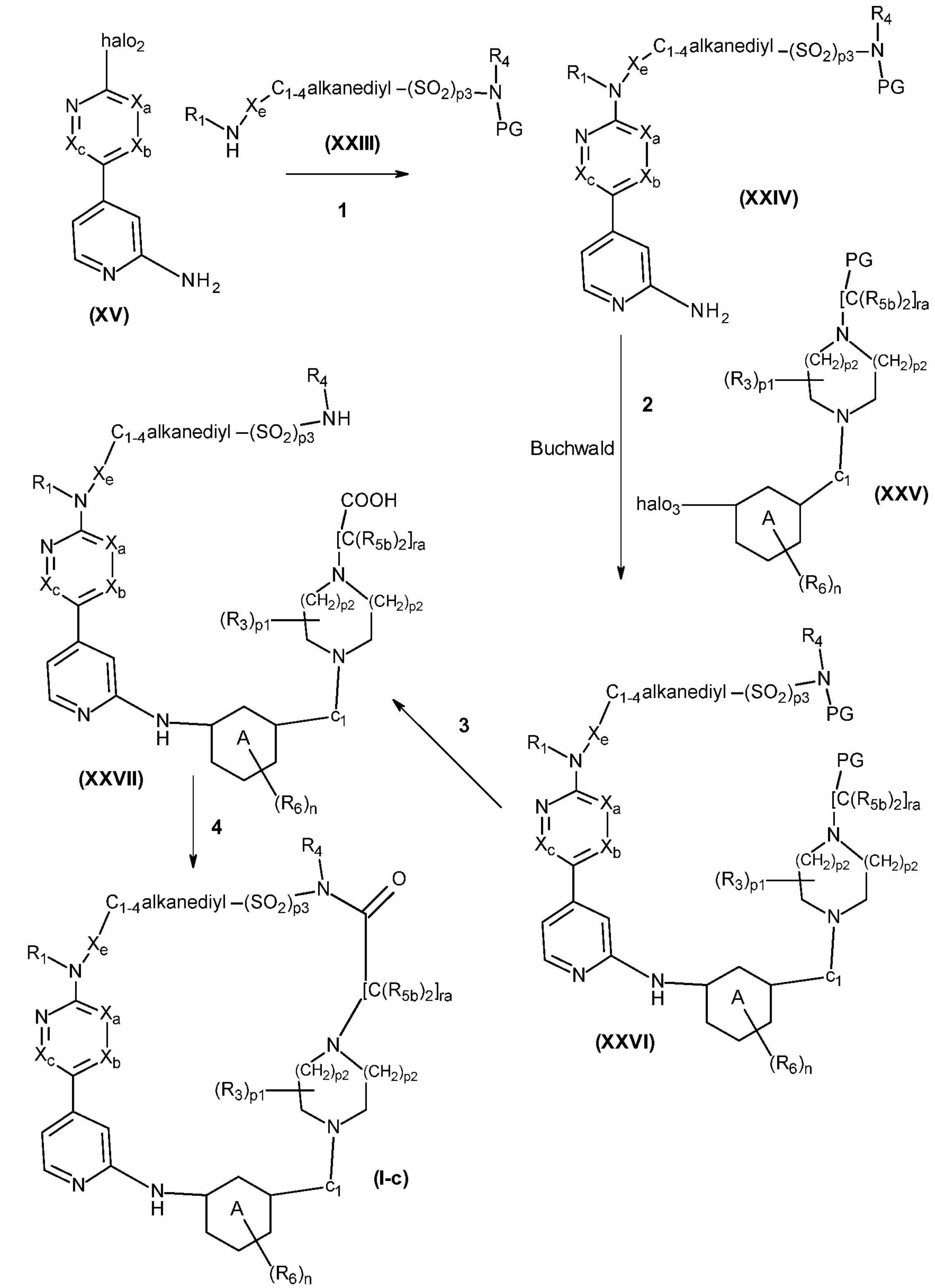


(XV) (XXIII) (XXIV) (XXV) (XXVI) (XXVII) (I-c)

In scheme 3, 'halo$_2$' andd'halo$_3$' are as defined before; 'PG' is defined as a protecting group such as for example *tert*-butoxycarbonyl, methoxycarbonyl or ethoxycarbonyl; 'c$_1$' is defined as a bond, $-[C(R_{5a})_2]_m-$, -C(=O)-, $-SO_2-$, or -SO-; and all other variables are defined as mentioned before.

In Scheme 3, the following reaction conditions apply:

**1**: coupling reacting between an intermediate of Formula (XV) and (XXIII) in a suitable solvent such as for example $CH_3CN$;

**2:** Buchwald-Hartwig amination; reaction between an intermediate of Formula (XXIV) and (XXV), typically in a suitable solvent such as for example dioxane, in the presence of a suitable base such as for example $Cs_2CO_3$, in the presence of a catalyst such as tris(dibenzylideneacetone)dipalladium ($Pd_2(dba)_3$), in the presence of a ligand such as for example S-Phos;

**3:** in the presence of an acid such as for example trifluoroacetic acid (TFA) in a solvent such as for example DCM; or alternatively in the presence of an acid such as for example HCl in a solvent such as for example 1,4-dioxane optionally in the presence of water; or

alternatively first in the presence of a base such as for example NaOH, and subsequently in the presence of an acid such as for example HCl, in the presence of a suitable solvent such as for example THF;

**4:** in the presence of a coupling agent such as for example diethyl cyanophosphonate, (1H-benzotriazol-1-yloxy)(tripyrrolidin-1-yl)phosphonium hexafluorophosphate (PyBOP), 1-[bis(dimethylamino)methylene]-1H-benzotriazol-1-ium 3-oxide hexafluorophosphate (HBTU) or 1-[bis(dimethylamino)methylene]-1H-[1,2,3]triazolo[4,5-b]pyridin-1-ium 3-oxide hexafluorophosphate (HATU) in the presence of a base such as for example triethylamine ($Et_3N$) or N,N-diisopropylethylamine (DIPEA), in a suitable solvent such as for example DMF.

The starting materials in scheme 3 are commercially available or can be prepared by standard means obvious to those skilled in the art or as described in the specific experimental part.

In general, compounds of Formula (I-d) can be prepared according to Scheme 4:

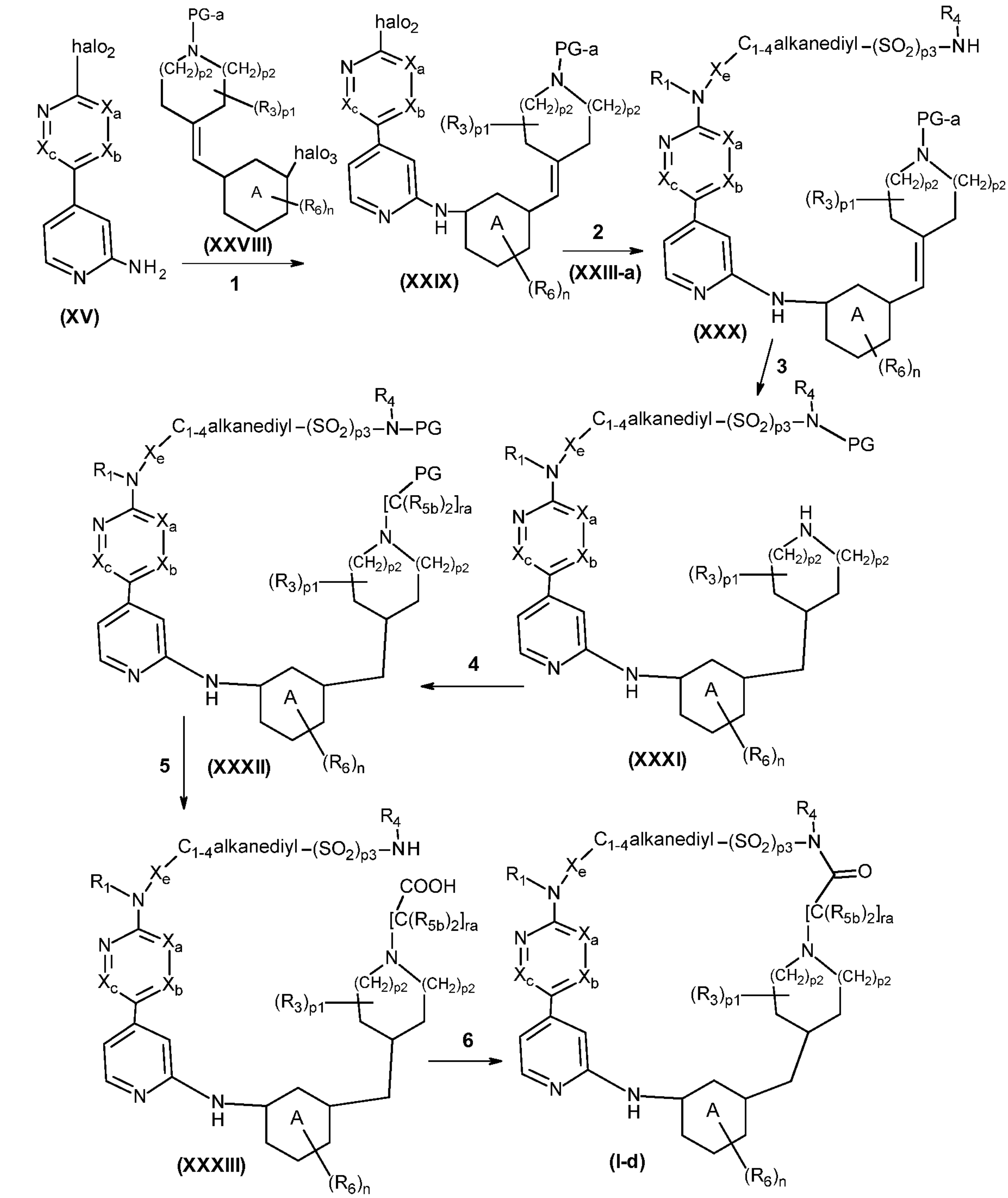


In scheme 4, 'PG', 'halo$_2$' and 'halo$_3$' are as defined before, 'PG-a' additionally may also be a benzyl group; and all other variables are as defined before.

In Scheme 4, the following reaction conditions apply:

**1:** in a solvent or a mixture of solvents such as dioxane/THF, in the presence of a suitable base such as for example $Cs_2CO_3$, in the presence of a catalyst such as for example Pd(II) acetate, together with a ligand such as 9,9-dimethyl-4,5-bis(diphenylphosphino)xanthene;

**2:** coupling reaction between an intermediate of Formula (XXIX) and an intermediate of Formula (XXIII-a):

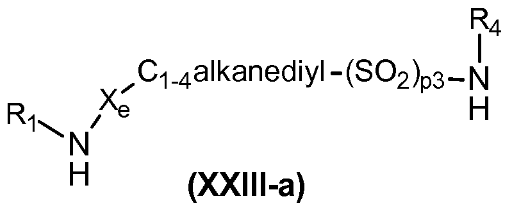

**(XXIII-a)**

,

optionally in the presence of a suitable base, such as for example $Na_2CO_3$, optionally in the presence of a suitable solvent such as for example *N*,*N*-dimethylacetamide (DMA) or 1-methyl-2-pyrrolidinone (NMP) or mixture of solvents such as for example DMA/DMSO ("DMSO" means dimethyl sulfoxide);

**3:** first, in case no protective group is present yet on $NR_4$, a protective group is introduced on $NR_4$ via reaction with *tert*-butoxycarbonyl anhydride in a suitable solvent such as for example DCM; then a reduction reaction in the presence of $H_2$-gas atmosphere and a catalyst such as for example Pd/C (for example 5 wt % or 10 wt %) in a suitable solvent such as for example MeOH or THF;

**4:** a substrate with a protecting group is introduced on the nitrogen atom of the piperidinyl by using for example tert-butyl bromoacetate, in the presence of a base such as for example $K_2CO_3$, in a suitable solvent such as DMF;

**5:** in the presence of an acid such as for example trifluoroacetic acid (TFA) in a solvent such as for example DCM; or alternatively in the presence of an acid such as for example HCl in a solvent such as for example 1,4-dioxane optionally in the presence of water; or

alternatively first in the presence of a base such as for example NaOH, and subsequently in the presence of an acid such as for example HCl, in the presence of a suitable solvent such as for example THF;

**6:** in the presence of a coupling agent such as for example diethyl cyanophosphonate, (1H-benzotriazol-1-yloxy)(tripyrrolidin-1-yl)phosphonium hexafluorophosphate (PyBOP), 1-[bis(dimethylamino)methylene]-1H-benzotriazol-1-ium 3-oxide hexafluorophosphate (HBTU) or 1-[bis(dimethylamino)methylene]-1H-[1,2,3]triazolo[4,5-b]pyridin-1-ium 3-oxide hexafluorophosphate (HATU) in the presence of a base such as for example triethylamine ($Et_3N$) or N,N-diisopropylethylamine (DIPEA), in a suitable solvent such as for example DMF.

In general, compounds of Formula (I-e) can be prepared according to Scheme 5:

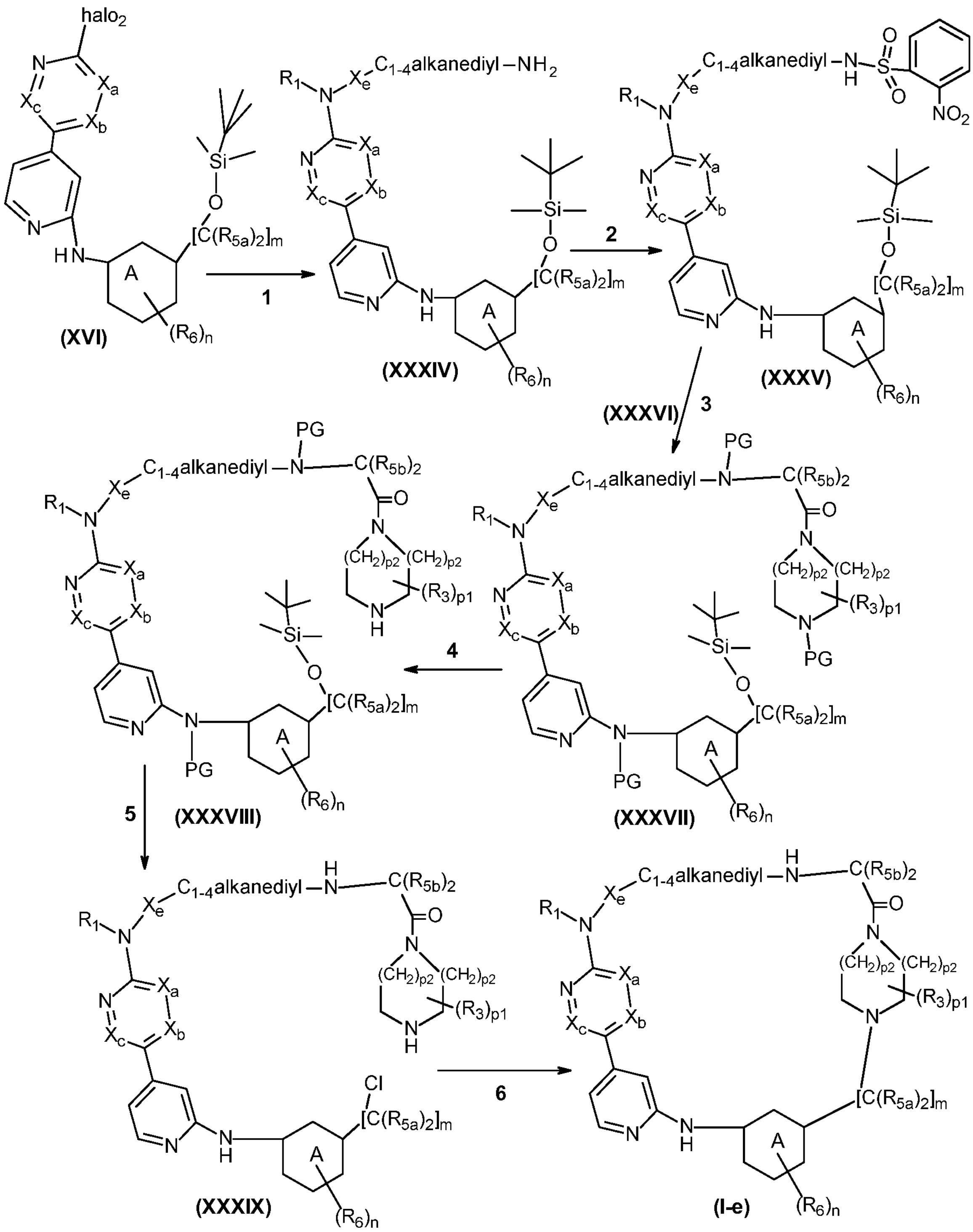


In scheme 5, 'PG' is as defined before; 'halo' is defined as Br, Cl or F; and all other variables are as defined before.

In Scheme 5, the following reaction conditions apply:

**1:** optionally in a suitable solvent such as for example DMF, and optionally in the presence of a base such as for example $K_2CO_3$;

**2:** in the presence of 2-nitrobenzenesulfonyl chloride, in the presence of a suitable base such as for example $Et_3N$ or DIPEA, in a suitable solvent such as for example DCM;

**3:** first, reaction between an intermediate of Formula (XXXV) and an intermediate of Formula (XXVI) (PG can also typically be benzyloxycarbonyl in an intermediate of Formula (XXXVI), in the presence of a suitable base such as for example $K_2CO_3$ or

$Cs_2CO_3$ in a suitable solvent such as for example DMF; and subsequently in the presence of a deprotecting group such as for example thiophenol; finally protecting groups are introduced with *tert*-butoxycarbonyl anhydride in a suitable solvent such as for example DCM;

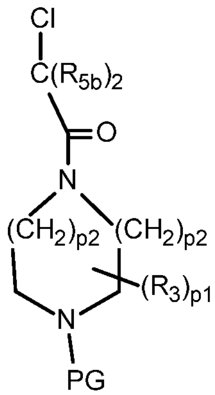

**(XXXVI)**

**4:** via reaction in the presence of $H_2$-gas atmosphere and a catalyst such as for example Pd/C (for example 5 wt % or 10 wt %) in a suitable solvent such as for example MeOH or THF;

**5:** firstly, in the presence of a deprotecting agent such as for example tetrabutylammonium fluoride (TBAF) in THF; or alternatively in the presence of an acid such as for example HCl in $H_2O$; or alternatively in the presence of $CH_3COOH$ optionally in the presence of water;

secondly, introduction of a leaving group (LG) using for example thionyl chloride in the presence of a suitable solvent such as for example 1,2-dichloroethane;

**6:** in the presence of a suitable base, such as for example $K_2CO_3$, in the presence of a suitable solvent such as for example DMF.

In general, compounds of Formula (I-f) can be prepared according to Scheme 6:

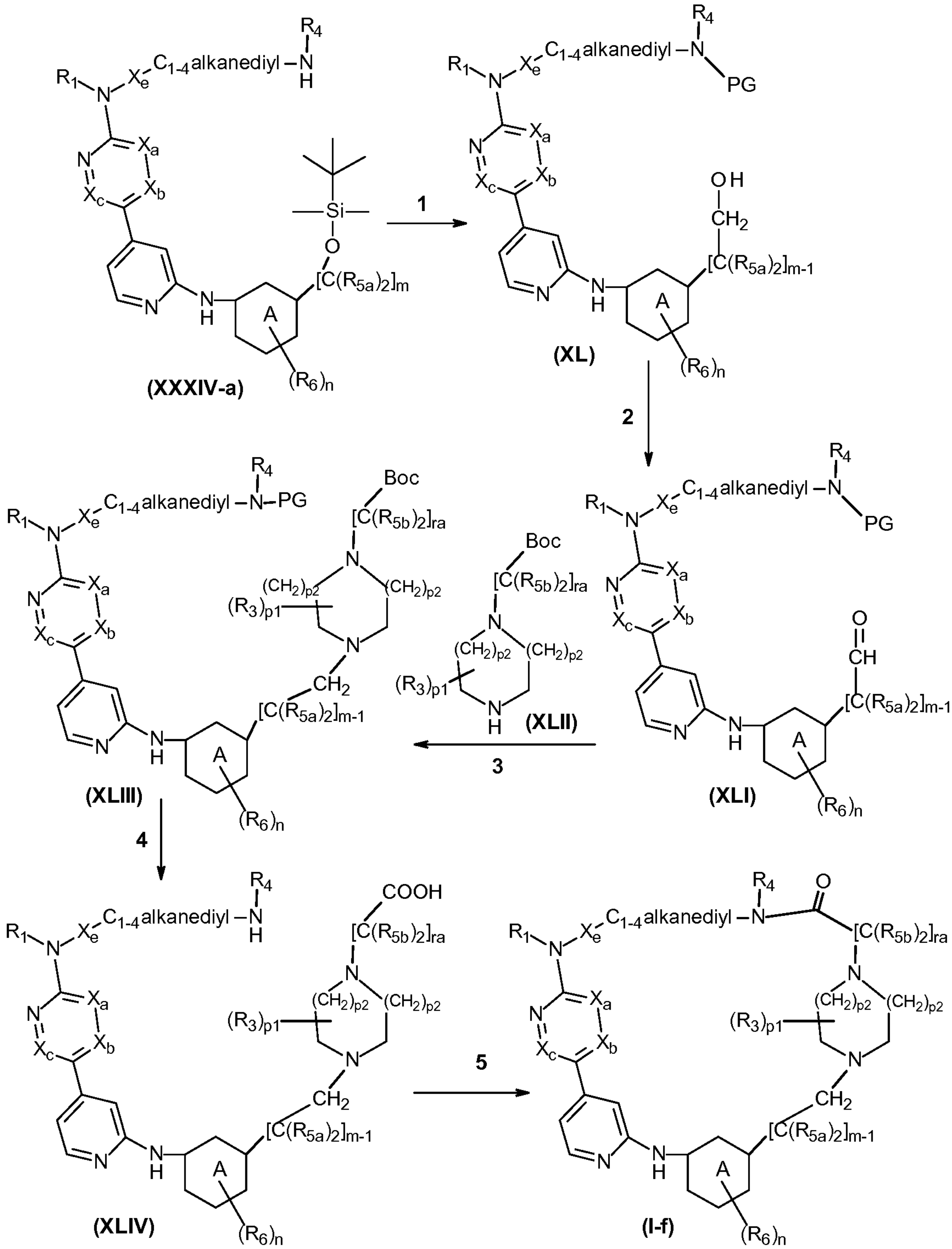


(XXXIV-a) (XL) (XLI) (XLII) (XLIII) (XLIV) (I-f)

In scheme 6, 'all variables are as defined before.

In Scheme 6, the following reaction conditions apply:

**1:** first a protecting group is introduced with for example *tert*-butoxycarbonyl anhydride in a suitable solvent such as for example DCM; secondly in the presence of a deprotecting agent such as for example tetrabutylammonium fluoride (TBAF) in THF;

**2:** in the presence of an oxidizing agent such as for example $MnO_2$, in the presence of a suitable solvent such as for example DCM;

**3:** in the presence of a reducing agent such as for example sodium triacetoxyborohydride ($NaBH(OAc)_3$), and in the presence of a suitable solvent such as for example 1,2-dichloroethane (DCE);

**4:** in the presence of an acid such as for example trifluoroacetic acid (TFA) in a solvent such as for example DCM; or alternatively in the presence of an acid such as for example HCl in a solvent such as for example 1,4-dioxane optionally in the presence of water; or
alternatively first in the presence of a base such as for example NaOH, and subsequently in the presence of an acid such as for example HCl, in the presence of a suitable solvent such as for example THF;
**5:** in the presence of a coupling agent such as for example diethyl cyanophosphonate, (1H-benzotriazol-1-yloxy)(tripyrrolidin-1-yl)phosphonium hexafluorophosphate (PyBOP), 1-[bis(dimethylamino)methylene]-1H-benzotriazol-1 -ium 3-oxide hexafluorophosphate (HBTU) or 1-[bis(dimethylamino)methylene]-1H-[1,2,3]triazolo[4,5-b]pyridin-1-ium 3-oxide hexafluorophosphate (HATU) in the presence of a base such as for example triethylamine ($Et_3N$) or N,N-diisopropylethylamine (DIPEA), in a suitable solvent such as for example DMF.

In general, compounds of Formula (I-g) can be prepared according to Scheme 7:

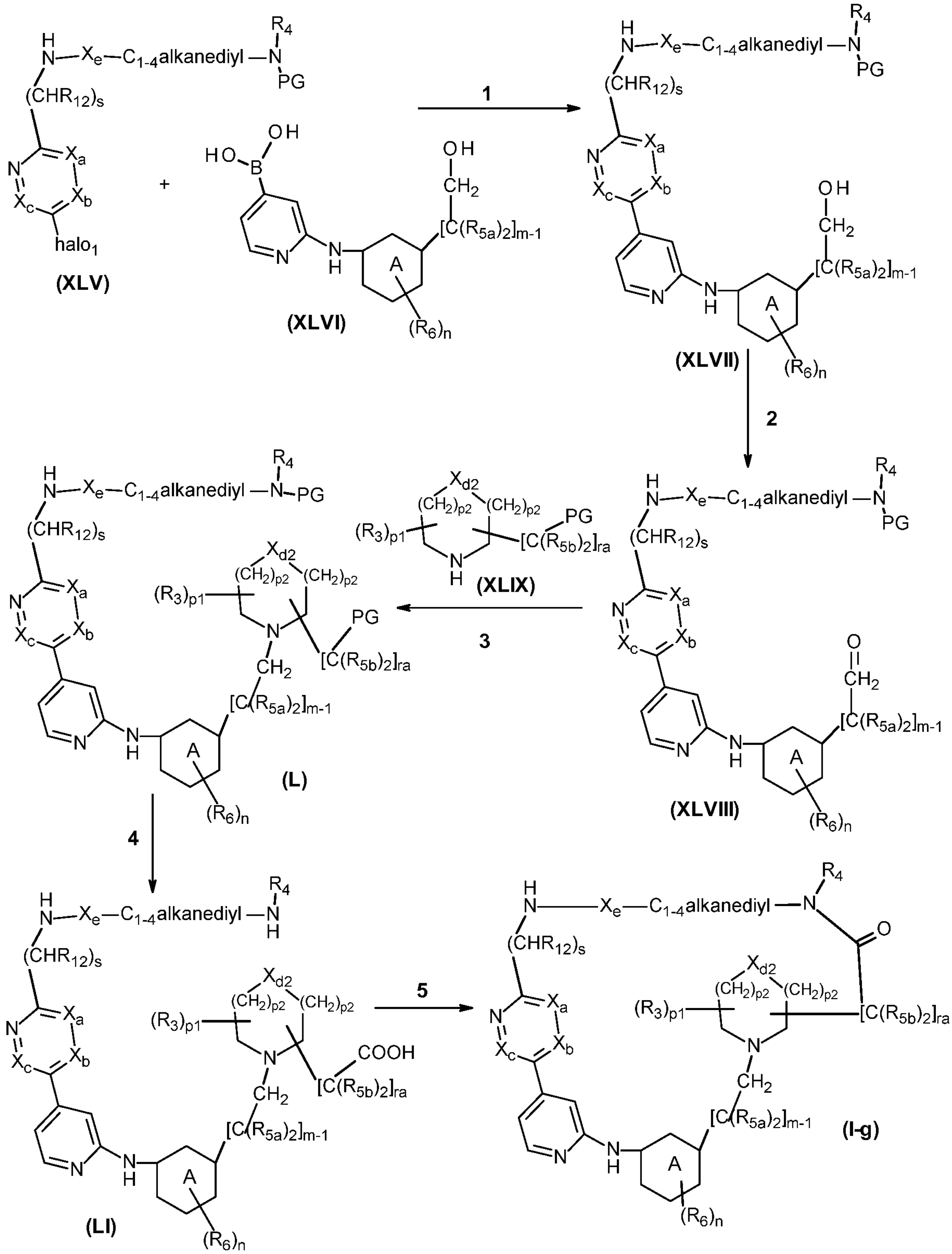


In scheme 7, 'all variables are as defined before. The skilled person will realize that additional protecting groups may be present if necessary.

In Scheme 7, the following reaction conditions apply:

**1:** coupling reaction between an intermediate of Formula (XLV) and (XLVI), in the presence of a suitable catalyst such as for example [1,1'-bis(diphenylphosphino-κP)ferrocene]dichloropalladium ($PdCl_2(dppf)$), in the presence of a suitable base such as for example $Na_2CO_3$, in the presence of a mixture of suitable solvents such as for example

water/1,4-dioxane;

**2:** in the presence of an oxidizing agent such as for example $MnO_2$, in the presence of a suitable solvent such as for example DCM or ethyl acetate (EtOAc);

**3:** in the presence of a reducing agent such as for example sodium triacetoxyborohydride ($NaBH(OAc)_3$), and in the presence of a suitable solvent such as for example DCM or 1,2-dichloroethane (DCE);

**4:** in the presence of an acid such as for example trifluoroacetic acid (TFA) in a solvent such as for example DCM; or alternatively in the presence of an acid such as for example HCl in a solvent such as for example 1,4-dioxane optionally in the presence of water; or

alternatively first in the presence of a base such as for example NaOH, and subsequently in the presence of an acid such as for example HCl, in the presence of a suitable solvent such as for example THF;

**5:** in the presence of a coupling agent such as for example diethyl cyanophosphonate, (1H-benzotriazol-1-yloxy)(tripyrrolidin-1-yl)phosphonium hexafluorophosphate (PyBOP), 1-[bis(dimethylamino)methylene]-1H-benzotriazol-1-ium 3-oxide hexafluorophosphate (HBTU) or 1-[bis(dimethylamino)methylene]-1H-[1,2,3]triazolo[4,5-b]pyridin-1-ium 3-oxide hexafluorophosphate (HATU) in the presence of a base such as for example triethylamine ($Et_3N$) or N,N-diisopropylethylamine (DIPEA), in a suitable solvent such as for example DMF.

An intermediate of Formula (XLVI) is commercially available or can be prepared by standard means obvious to those skilled in the art or as described in the specific experimental part.

In general, compounds of Formula (I-g) can be converted to compounds of Formula (I-g-2) as shown in Scheme 7b:

Scheme 7b

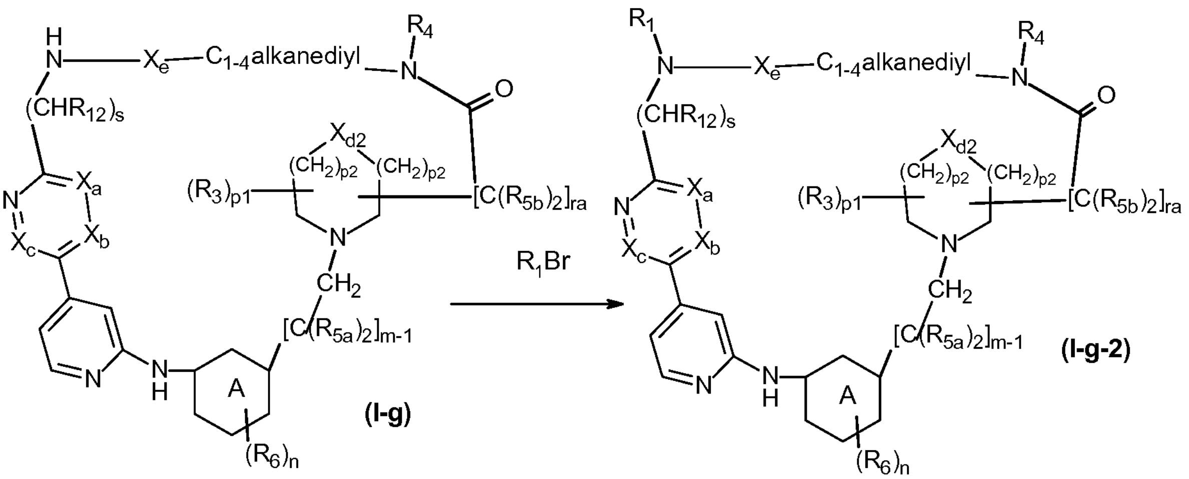


In Scheme 7b, a compound of Formula (I-g) is reacted with an intermediate of Formula $R_1$-Br, to result in a compound of Formula (I-g-2). This reaction typically is performed in the presence of a suitable base such as for example DIPEA, in the presence of a suitable solvent such as for example DMF.

Analogous functionalization reactions can be performed by replacing $R_1Br$, for example, with alkylsulfonyl chlorides, acid chlorides or sulfamides. Other functional groups can also be introduced via reductive amination. All these reactions can be performed under standard reaction conditions well-known by the skilled person.

In general, compounds of Formula (I-h) can be prepared according to Scheme 8:

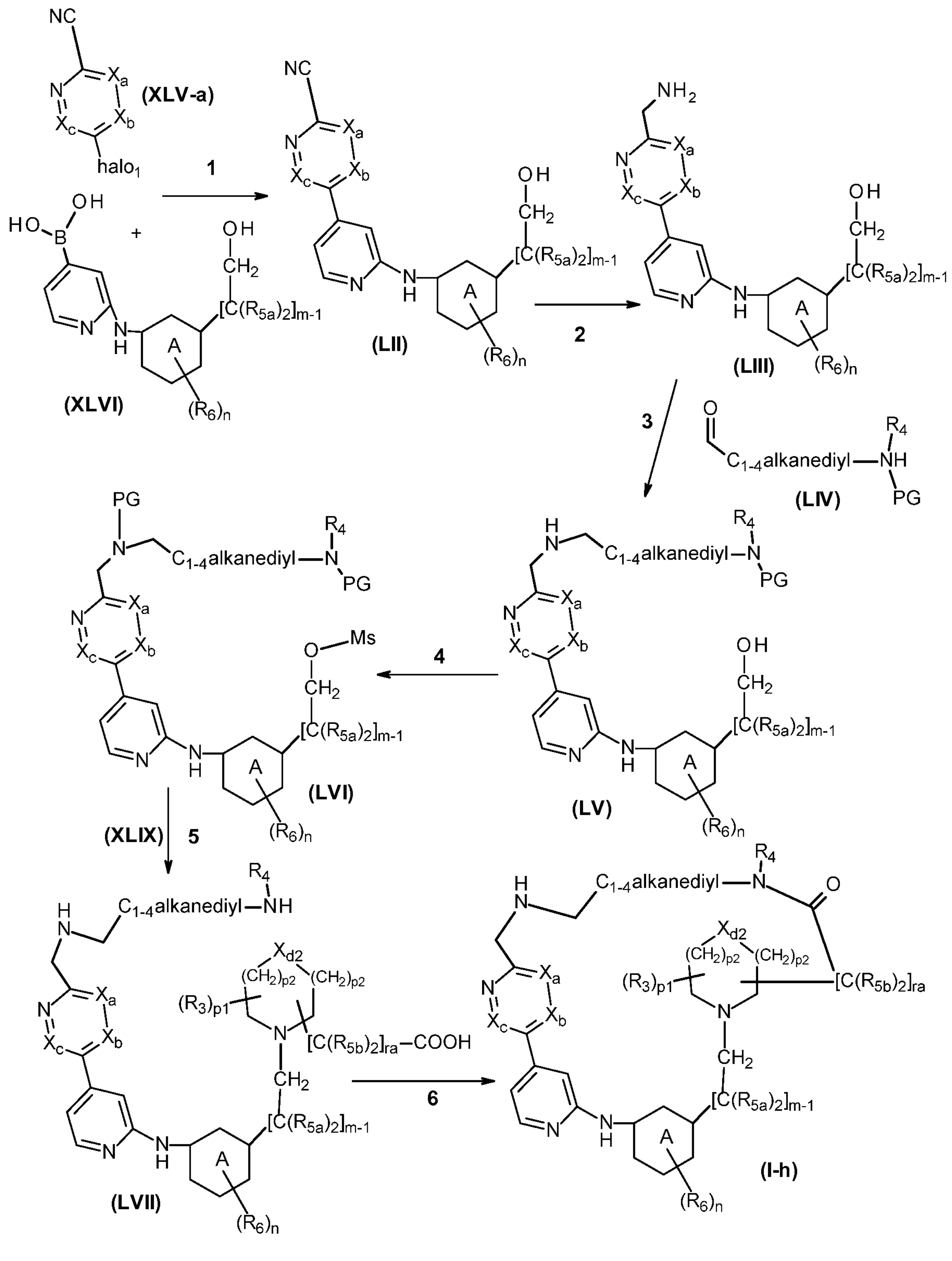


(XLV-a) (XLVI) (LII) (LIII) (LIV) (LV) (LVI) (LVII) (I-h)

In scheme 8, 'Ms' means mesyl (methanesulfonyl), and all other variables are as defined before.

In Scheme 8, the following reaction conditions apply:

**1:** coupling reaction between an intermediate of Formula (XLV-a) and (XLVI), in the presence of a suitable catalyst such as for example $PdCl_2$(dppf), in the presence of a suitable base such as for example $Na_2CO_3$, in the presence of a suitable solvent or a mixture of suitable solvents such as for example water/1,4-dioxane;

**2:** via reduction in the presence of $H_2$-gas atmosphere and a catalyst such as for example Pt/C or Pd/C (for example 5 wt % or 10 wt %) in a suitable solvent or a mixture of suitable solvents such as for example EtOAc/acetic acid;

**3:** in the presence of a reducing agent such as for example sodium triacetoxyborohydride ($NaBH(OAc)_3$), and in the presence of a suitable solvent such as for example 1,2-dichloroethane (DCE) or a mixture of suitable solvent such as for example *N*,*N*-dimethylacetamide (DMA)/acetic acid;

**4:** first a protecting group is introduced with for example *tert*-butoxycarbonyl anhydride in a suitable solvent such as for example DCM, optionally in the presence of a base such as for example $Et_3N$; secondly reaction with mesylchloride in a suitable solvent such as DCM in the presence of a suitable base such as for example $Et_3N$ or DIPEA;

**5:** first a coupling reaction between an intermediate of Formula (LVI) and (XLIX) optionally in the presence of a suitable solvent such as for example DMF;

secondly removal of the protecting group in the presence of an acid such as for example trifluoroacetic acid (TFA) in a solvent such as for example DCM; or alternatively in the presence of an acid such as for example HCl in a solvent such as for example 1,4-dioxane optionally in the presence of water; or alternatively first in the presence of a base such as for example NaOH, and subsequently in the presence of an acid such as for example HCl, in the presence of a suitable solvent such as for example THF;

**6:** in the presence of a coupling agent such as for example diethyl cyanophosphonate, (1H-benzotriazol-1-yloxy)(tripyrrolidin-1-yl)phosphonium hexafluorophosphate (PyBOP), 1-[bis(dimethylamino)methylene]-1H-benzotriazol-1-ium 3-oxide hexafluorophosphate (HBTU) or 1-[bis(dimethylamino)methylene]-1H-[1,2,3]triazolo[4,5-b]pyridin-1-ium 3-oxide hexafluorophosphate (HATU) in the presence of a base such as for example triethylamine ($Et_3N$) or N,N-diisopropylethylamine (DIPEA), in a suitable solvent such as for example DMF.

Analogous reactions as described in Scheme 7b can also be performed on compound of Formula (I-h).

In general, compounds of Formula (I-i) can be prepared according to Scheme 9:

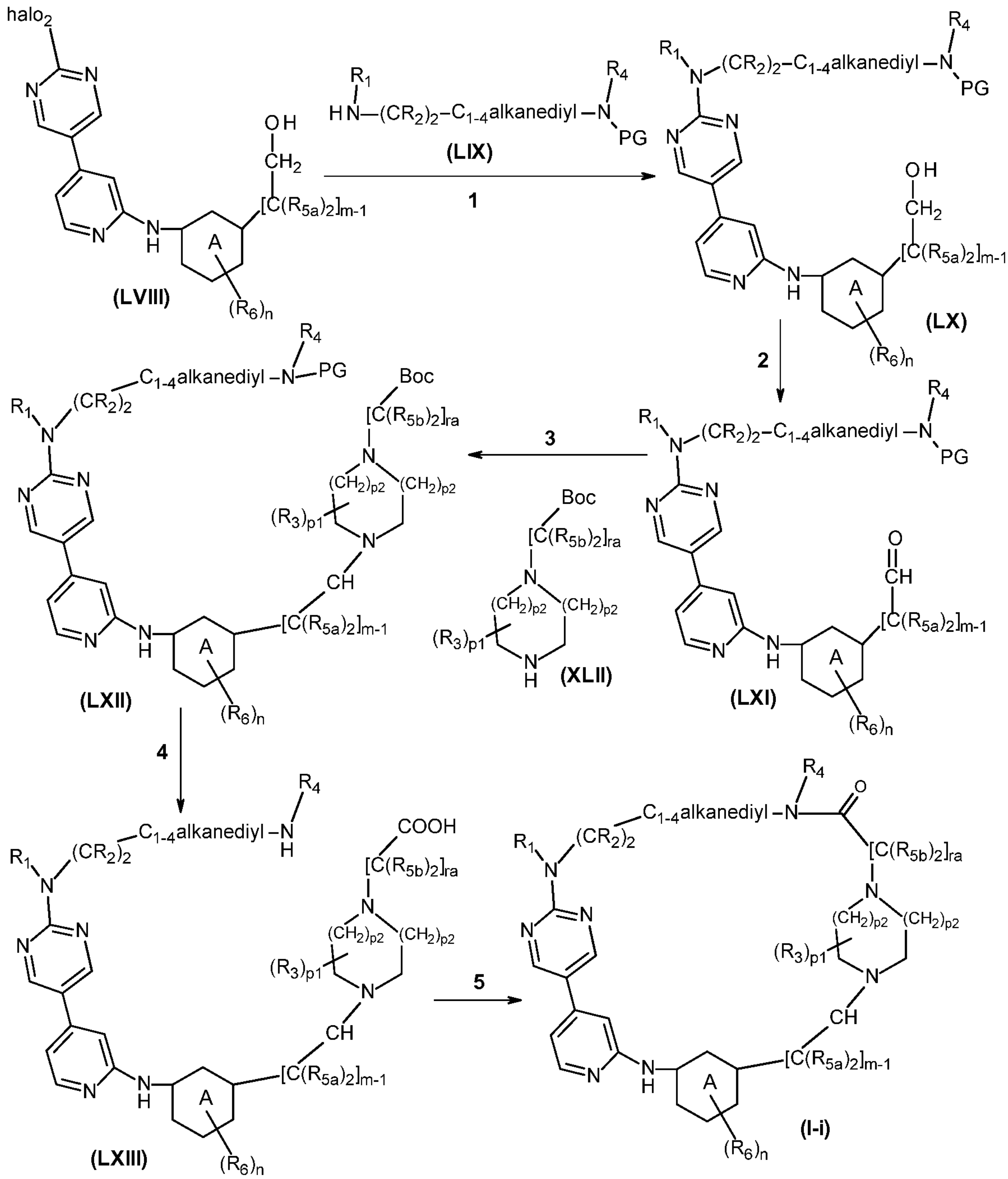


In scheme 9, 'PG' is as defined before; 'halo2' is as defined before (Cl or F); and all other variables are as defined before.

In Scheme 9, the following reaction conditions apply:

**1:** in a suitable solvent such as for example 2-methyl-2-propanol or NMP, optionally in the presence of a base such as for example DIPEA;
**2:** in the presence of an oxidizing agent such as for example $MnO_2$, in the presence of a suitable solvent such as for example DCM;
**3:** in the presence of a reducing agent such as for example sodium triacetoxyborohydride ($NaBH(OAc)_3$), and in the presence of a suitable solvent such as for example DCM or 1,2-dichloroethane (DCE);
**4:** in the presence of an acid such as for example trifluoroacetic acid (TFA) in a solvent such as for example DCM; or alternatively in the presence of an acid such as for example HCl in a solvent such as for example 1,4-dioxane optionally in the presence of water; or alternatively first in the presence of a base such as for example NaOH, and subsequently in the presence of an acid such as for example HCl, in the presence of a suitable solvent such as for

example THF;

**5:** in the presence of a coupling agent such as for example diethyl cyanophosphonate, (1H-benzotriazol-1-yloxy)(tripyrrolidin-1-yl)phosphonium hexafluorophosphate (PyBOP), 1-[bis(dimethylamino)methylene]-1H-benzotriazol-1-ium 3-oxide hexafluorophosphate (HBTU) or 1-[bis(dimethylamino)methylene]-1H-[1,2,3]triazolo[4,5-b]pyridin-1-ium 3-oxide hexafluorophosphate (HATU) in the presence of a base such as for example triethylamine ($Et_3N$) or N,N-diisopropylethylamine (DIPEA), in a suitable solvent such as for example DMF.

The skilled person will realize that dependent on the choice of (LIX) in scheme 9, the reactions described in scheme 9 can also be used to prepare compounds wherein $X_a$, $X_b$ and $X_c$ have the meaning as defined in the scope ($X_a$, $X_b$ and $X_c$ each independently represent CH or N).

Compounds of Formula (I-i) wherein $R_2$ represents -C(=O)-O-$C_{1-4}$alkyl can be converted to compounds wherein $R_2$ represents COOH (via e.g. basic hydrolysis reaction), which in turn can be converted by methods known by those skilled in the art to compounds wherein $R_2$ represents an amide.

In general, intermediates of Formula (LXVI) can be prepared according to Scheme 10 starting from intermediates of Formula (LXIV) and (LXV), wherein all variables are as defined before. Intermediates of Formula (LXVI) can be further reacted to final compounds of Formula (I) by using analogous reaction protocols as described before in the other general schemes. Intermediates of Formula (LXIV) and (LXV) are commercially available or can be prepared by standard means obvious to those skilled in the art or as described in the specific experimental part.

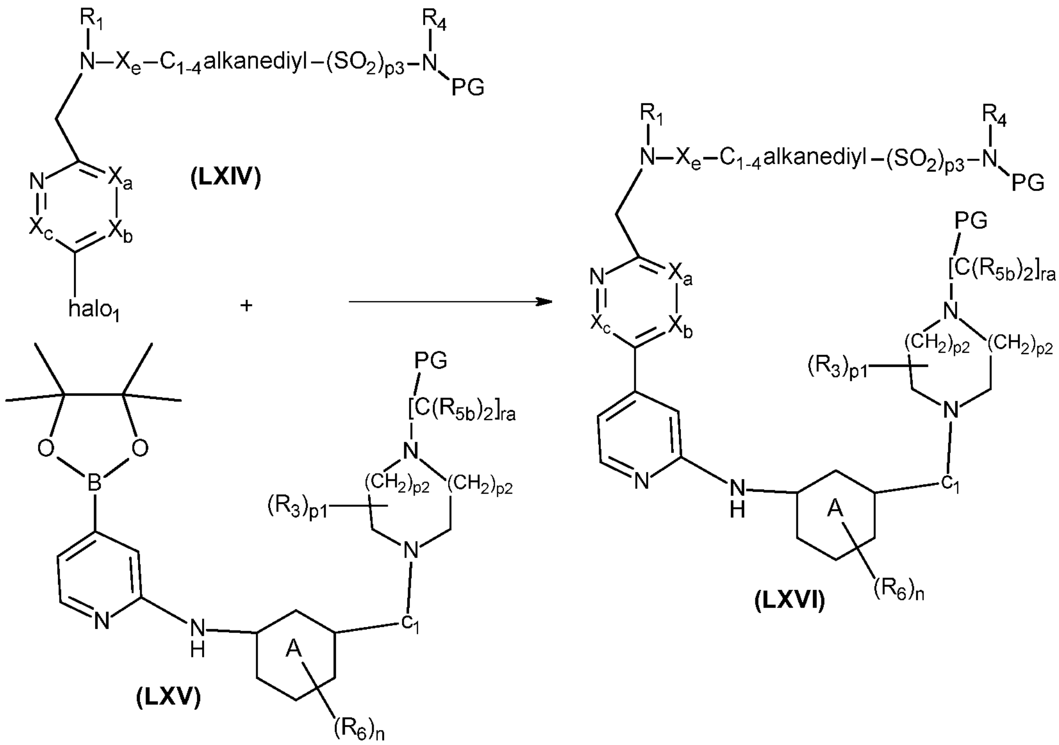

In general, intermediates of Formula (LXX) can be prepared according to Scheme 11, wherein all variables are as defined before. Intermediates of Formula (LXX) can be further reacted to final compounds of Formula (I) by using analogous reaction protocols as described before in Scheme 1b.

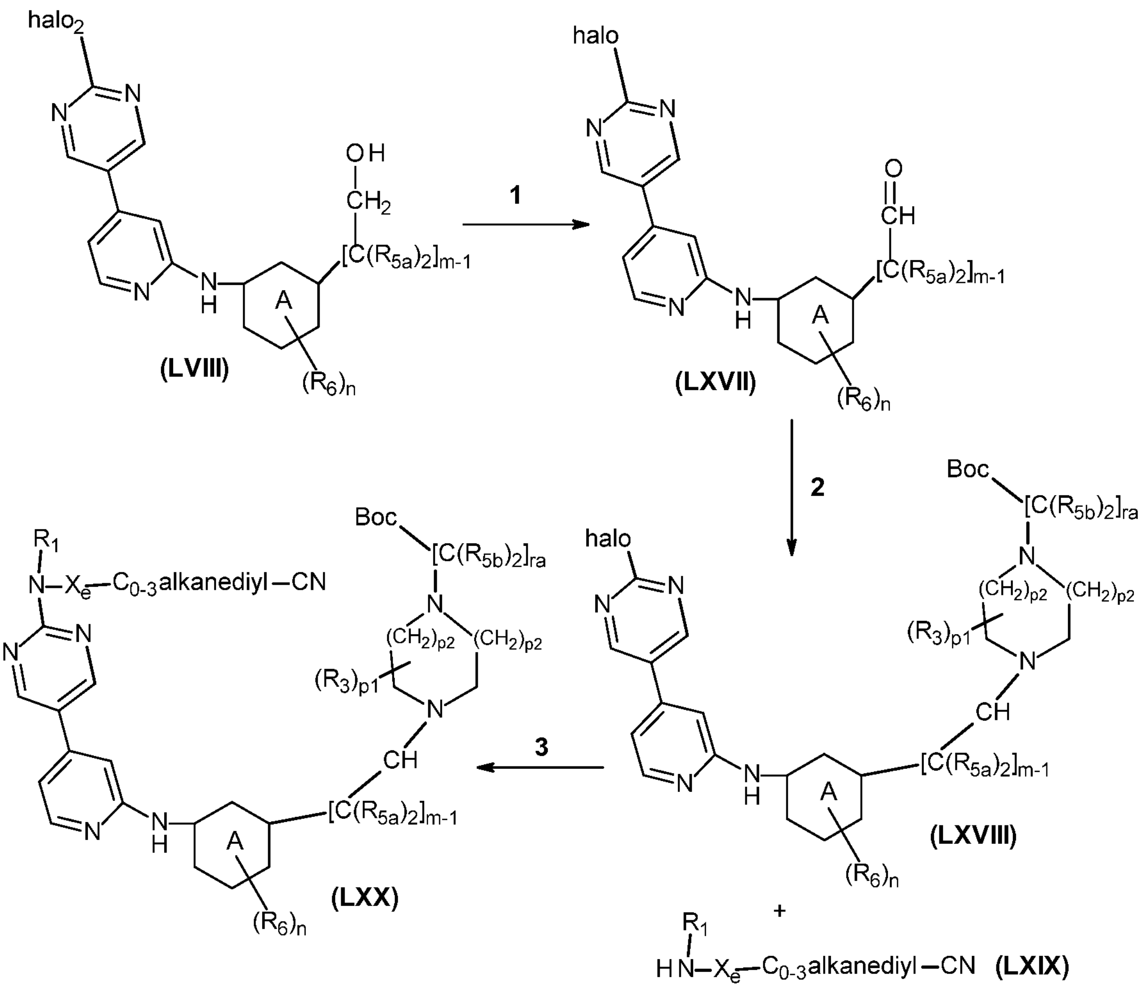


(LVIII) (LXVII) (LXVIII) + (LXIX) (LXX)

The compounds of Formula (I) may also be converted into each other via art-known reactions or functional group transformations.

For instance, a compound of Formula (I), wherein $R^6$ represents aminocarbonyl can be converted to a compound wherein $R^6$ represents carboxyl, by reaction with a suitable acid such as for example HCl. During this reaction, ring-opening of the macrocycle may occur. In this case, it is necessary to react the outcome of the reaction with a coupling agent such as for example diethyl cyanophosphonate, in the presence of a base such as for example triethylamine ($Et_3N$), in a suitable solvent such as for example DMF, to close the macrocylic ring.

Compounds of Formula (I) wherein $R_1$ and $R_2$, or $R_1$ and $R_{12}$, are taken together to form $C_{1-4}$alkanediyl or $C_{2-4}$alkenediyl, and which are substituted with hydroxyl on said $C_{1-4}$alkanediyl or $C_{2-4}$alkenediyl, may be converted to other compounds of Formula (I) by the following reactions:

- hydroxyl to azide ion: in a suitable solvent such as THF, in the presence of a ligand such as triphenylphosphine ($PPh_3$), an azide source such as diphenylphosphoryl azide (DPPA) and in the presence of an azodicarboxylate such as for example diisopropyl azodicarboxylate (DIAD);

- azide to $NH_2$: via reduction reaction in the presence of $H_2$-gas atmosphere and a catalyst such as for example Pt/C or Pd/C (for example 5 wt % or 10 wt %) in a suitable solvent such as for example MeOH or THF;

- $NH_2$ to $NH_2$-S(=O)$_2$-NH-: via reaction with sulfamide in a suitable solvent such as for example dioxane;

- hydroxyl to oxo: Swern oxidation to a ketone using oxalyl chloride, dimethyl sulfoxide (DMSO) and an organic base such as for example $Et_3N$;

- hydroxyl to cyano: first conversion of the hydroxyl group to $CH_3$-S(=O)$_2$-O-via reaction with mesylchloride in a suitable solvent such as DCM in the presence of a suitable base such as for example DIPEA; second conversion

of $CH_3$-S(=O)$_2$-O- to the cyano group by reaction with e.g. NaCN in a suitable solvent such as for example DMSO;

- hydroxyl to fluoro: in a suitable solvent such as THF in the presence of a suitable base (promotor) such as for example 1,8-diazabicyclo[5.4.0]undecene-7 (DBU) in the presence of a fluorinating reagent such as (diethylamino)difluorosulfonium tetrafluoroborate (XtalFluor-E®).

Although not shown explicitly in the general reaction schemes, the skilled person will realize that compounds wherein $NR_1$ is replaced by O, can be prepared according to analogous reaction protocols as outlined hereabove in combination with methods known by the skilled person.

In all these preparations, the reaction products may be isolated from the reaction medium and, if necessary, further purified according to methodologies generally known in the art such as, for example, extraction, crystallization, trituration and chromatography. In particular, stereoisomers can be isolated chromatographically using a chiral stationary phase such as, for example, Chiralpak® AD (amylose 3,5 dimethylphenyl carbamate) or Chiralpak® AS, both purchased from Daicel Chemical Industries, Ltd, in Japan, or by Supercritical Fluid Chromatography (SFC).

The chirally pure forms of the compounds of Formula (I) form a preferred group of compounds. It is therefore that the chirally pure forms of the intermediates and their salt forms are particularly useful in the preparation of chirally pure compounds of Formula (I). Also enantiomeric mixtures of the intermediates are useful in the preparation of compounds of Formula (I) with the corresponding configuration.

## Pharmacology

It has been found that the compounds of the present invention have EF2K inhibitory activity and optionally may also have Vps34 inhibitory activity.

The compounds according to the invention and the pharmaceutical compositions comprising such compounds may be useful for treating or preventing, in particular treating, diseases such as cancer, depression, neuroplasticity (synaptic plasticity and non-synaptic plasticity), and memory and learning disorders; in particular diseases such as cancer, depression, and memory and learning disorders.

In particular, the compounds according to the present invention and the pharmaceutical compositions thereof may be useful in the treatment or the prevention, in particular in the treatment, of a haematological malignancy or solid tumour.

In a specific embodiment said solid tumour is selected from the group consisting of glioblastoma, medulloblastoma, prostate cancer, breast cancer, ovarian cancer and colorectal cancer.

Examples of other cancers which may be treated (or inhibited) include, but are not limited to, a carcinoma, for example a carcinoma of the bladder, breast, colon (e.g. colorectal carcinomas such as colon adenocarcinoma and colon adenoma), kidney, urothelial, uterus, epidermis, liver, lung (for example adenocarcinoma, small cell lung cancer and non-small cell lung carcinomas, squamous lung cancer), oesophagus, head and neck, gall bladder, ovary, pancreas (e.g. exocrine pancreatic carcinoma), stomach, gastrointestinal (also known as gastric) cancer (e.g. gastrointestinal stromal tumours), cervix, endometrium, thyroid, prostate, or skin (for example squamous cell carcinoma or dermatofibrosarcoma protuberans); pituitary cancer, a hematopoietic tumour of lymphoid lineage, for example leukemia, acute lymphocytic leukemia, chronic lymphocytic leukemia, B-cell lymphoma (e.g. diffuse large B-cell lymphoma), T-cell lymphoma, Hodgkin's lymphoma, non-Hodgkin's lymphoma, hairy cell lymphoma, or Burkett's lymphoma; a hematopoietic tumour of myeloid lineage, for example leukemias, acute and chronic myelogenous leukemias, chronic myelomonocytic leukemia (CMML), myeloproliferative disorder, myeloproliferative syndrome, myelodysplastic syndrome, or promyelocytic leukemia; multiple myeloma; thyroid follicular cancer; hepatocellular cancer, a tumour of mesenchymal origin (e.g. Ewing's sarcoma), for example fibrosarcoma or rhabdomyosarcoma; a tumour of the central or peripheral nervous system, for example astrocytoma, neuroblastoma, glioma (such as glioblastoma multiforme) or schwannoma; melanoma; seminoma; teratocarcinoma; osteosarcoma; xeroderma pigmentosum; keratoctanthoma; thyroid follicular cancer; or Kaposi's sarcoma. In particular, squamous lung cancer, breast cancer, colorectal cancer, glioblastoma, astrocytomas, prostate cancer, small cell lung cancer, melanoma, head and neck cancer, thyroid cancer, uterine cancer, gastric cancer, hepatocellular cancer, cervix cancer, multiple myeloma, bladder cancer, endometrial cancer, urothelial cancer, colon cancer, rhabdomyosarcoma, pituitary gland cancer.

The compounds according to the invention and the pharmaceutical compositions comprising such compounds may also be useful for treating or preventing, in particular treating, diseases such as malaria, rheumatoid arthritis, lupus and HIV.

The compounds of the invention and compositions thereof can also be used in the treatment of hematopoetic diseases of abnormal cell proliferation whether pre-malignant or stable such as myeloproliferative diseases. Myeloproliferative diseases ("MPD"s) are a group of diseases of the bone marrow in which excess cells are produced. They are related to, and may evolve into, myelodysplastic syndrome. Myeloproliferative diseases include polycythemia vera,

essential thrombocythemia and primary myelofibrosis. A further haematological disorder is hypereosinophilic syndrome. T-cell lymphoproliferative diseases include those derived from natural Killer cells.

Thus, in the disclosed pharmaceutical compositions, uses or methods for treating a disease or condition comprising abnormal cell growth, the disease or condition comprising abnormal cell growth in one embodiment is a cancer.

The compounds of the present invention also have therapeutic applications in sensitising tumour cells for radiotherapy and chemotherapy.

Hence the compounds of the present invention can be used as "radiosensitizer" and/or "chemosensitizer" or can be given in combination with another "radiosensitizer" and/or "chemosensitizer".

The term "radiosensitizer", as used herein, is defined as a molecule, preferably a low molecular weight molecule, administered to animals in therapeutically effective amounts to increase the sensitivity of the cells to ionizing radiation and/or to promote the treatment of diseases which are treatable with ionizing radiation.

The term "chemosensitizer", as used herein, is defined as a molecule, preferably a low molecular weight molecule, administered to animals in therapeutically effective amounts to increase the sensitivity of cells to chemotherapy and/or promote the treatment of diseases which are treatable with chemotherapeutics.

Several mechanisms for the mode of action of radiosensitizers have been suggested in the literature including: hypoxic cell radiosensitizers (e.g., 2- nitroimidazole compounds, and benzotriazine dioxide compounds) mimicking oxygen or alternatively behave like bioreductive agents under hypoxia; non-hypoxic cell radiosensitizers (e.g., halogenated pyrimidines) can be analogoues of DNA bases and preferentially incorporate into the DNA of cancer cells and thereby promote the radiation-induced breaking of DNA molecules and/or prevent the normal DNA repair mechanisms; and various other potential mechanisms of action have been hypothesized for radiosensitizers in the treatment of disease.

Many cancer treatment protocols currently employ radiosensitizers in conjunction with radiation of x-rays. Examples of x-ray activated radiosensitizers include, but are not limited to, the following: metronidazole, misonidazole, desmethylmisonidazole, pimonidazole, etanidazole, nimorazole, mitomycin C, RSU 1069, SR 4233, EO9, RB 6145, nicotinamide, 5-bromodeoxyuridine (BUdR), 5- iododeoxyuridine (IUdR), bromodeoxycytidine, fluorodeoxyuridine (FudR), hydroxyurea, cisplatin, and therapeutically effective analogs and derivatives of the same.

Photodynamic therapy (PDT) of cancers employs visible light as the radiation activator of the sensitizing agent. Examples of photodynamic radiosensitizers include the following, but are not limited to: hematoporphyrin derivatives, Photofrin, benzoporphyrin derivatives, tin etioporphyrin, pheoborbide-a, bacteriochlorophyll-a, naphthalocyanines, phthalocyanines, zinc phthalocyanine, and therapeutically effective analogs and derivatives of the same.

Radiosensitizers may be administered in conjunction with a therapeutically effective amount of one or more other compounds, including but not limited to: compounds which promote the incorporation of radiosensitizers to the target cells; compounds which control the flow of therapeutics, nutrients, and/or oxygen to the target cells; chemotherapeutic agents which act on the tumour with or without additional radiation; or other therapeutically effective compounds for treating cancer or other diseases. Chemosensitizers may be administered in conjunction with a therapeutically effective amount of one or more other compounds, including but not limited to: compounds which promote the incorporation of chemosensitizers to the target cells; compounds which control the flow of therapeutics, nutrients, and/or oxygen to the target cells; chemotherapeutic agents which act on the tumour or other therapeutically effective compounds for treating cancer or other disease. Calcium antagonists, for example verapamil, are found useful in combination with antineoplastic agents to establish chemosensitivity in tumor cells resistant to accepted chemotherapeutic agents and to potentiate the efficacy of such compounds in drug-sensitive malignancies.

The invention relates to compounds of Formula (I) and pharmaceutically acceptable addition salts, and solvates thereof, for use as a medicament.

The invention also relates to compounds of Formula (I) and pharmaceutically acceptable addition salts, and solvates thereof, for use in the inhibition of EF2K and optionally also for use in the inhibition of Vps34.

The compounds of the present invention can be "anti-cancer agents", which term also encompasses "anti-tumor cell growth agents" and "anti-neoplastic agents".

The invention also relates to compounds of Formula (I) and pharmaceutically acceptable addition salts, and solvates thereof, for use in the treatment of diseases mentioned above.

The invention also relates to compounds of Formula (I) and pharmaceutically acceptable addition salts, and solvates thereof, for the treatment or prevention, in particular for the treatment, of said diseases.

The invention also relates to compounds of Formula (I) and pharmaceutically acceptable addition salts, and solvates thereof, for the treatment or prevention, in particular in the treatment, of EF2K mediated diseases or conditions.

The invention also relates to compounds of Formula (I) and pharmaceutically acceptable addition salts, and solvates thereof, for the treatment or prevention, in particular in the treatment, of EF2K and optionally Vps34 mediated diseases or conditions.

The invention also relates to the use of compounds of Formula (I) and pharmaceutically acceptable addition salts, and solvates thereof, for the manufacture of a medicament.

The invention also relates to the use of compounds of Formula (I) and pharmaceutically acceptable addition

salts, and solvates thereof, for the manufacture of a medicament for the inhibition of EF2K and optionally also for the inhibition of Vps34.

The invention also relates to the use of compounds of Formula (I) and pharmaceutically acceptable addition salts, and solvates thereof, for the manufacture of a medicament for the treatment or prevention, in particular for the treatment, of any one of the disease conditions mentioned hereinbefore.

The invention also relates to the use of compounds of Formula (I) and pharmaceutically acceptable addition salts, and solvates thereof, for the manufacture of a medicament for the treatment of any one of the disease conditions mentioned hereinbefore.

The compounds of Formula (I) and pharmaceutically acceptable addition salts, and solvates thereof, can be administered to mammals, preferably humans for the treatment or prevention of any one of the diseases mentioned hereinbefore.

The compounds of the present invention may also be used in the optimisation of industrial protein production.

In view of the utility of the compounds of Formula (I) and pharmaceutically acceptable addition salts, and solvates thereof, a method is disclosed of treating warm-blooded animals, including humans, suffering from or a method of preventing warm-blooded animals, including humans, to suffer from any one of the diseases mentioned hereinbefore.

Said methods comprise the administration, i.e. the systemic or topical administration, preferably oral administration, of an effective amount of a compound of Formula (I) and pharmaceutically acceptable addition salts, and solvates thereof, to warm-blooded animals, including humans.

Those of skill in the treatment of such diseases could determine the effective therapeutic daily amount from the test results presented hereinafter. An effective therapeutic daily amount would be from about 0.005 mg/kg to 50 mg/kg, in particular 0.01 mg/kg to 50 mg/kg body weight, more in particular from 0.01 mg/kg to 25 mg/kg body weight, preferably from about 0.01 mg/kg to about 15 mg/kg, more preferably from about 0.01 mg/kg to about 10 mg/kg, even more preferably from about 0.01 mg/kg to about 1 mg/kg, most preferably from about 0.05 mg/kg to about 1 mg/kg body weight. The amount of a compound according to the present invention, also referred to here as the active ingredient, which is required to achieve a therapeutically effect will of course, vary on case-by-case basis, for example with the particular compound, the route of administration, the age and condition of the recipient, and the particular disorder or disease being treated.

A method of treatment may also include administering the active ingredient on a regimen of between one and four intakes per day. In these methods of treatment the compounds according to the invention are preferably formulated prior to administration. As described herein below, suitable pharmaceutical formulations are prepared by known procedures using well known and readily available ingredients.

The compounds of the present invention, that can be suitable to treat or prevent cancer or cancer-related conditions, may be administered alone or in combination with one or more additional therapeutic agents. Combination therapy includes administration of a single pharmaceutical dosage formulation which contains a compound of Formula (I), a pharmaceutically acceptable addition salt, or a solvate thereof, and one or more additional therapeutic agents, as well as administration of the compound of Formula (I), a pharmaceutically acceptable addition salt, or a solvate thereof, and each additional therapeutic agents in its own separate pharmaceutical dosage formulation. For example, a compound of Formula (I), a pharmaceutically acceptable addition salt, or a solvate thereof, and a therapeutic agent may be administered to the patient together in a single oral dosage composition such as a tablet or capsule, or each agent may be administered in separate oral dosage formulations.

While it is possible for the active ingredient to be administered alone, it is preferable to present it as a pharmaceutical composition.

Accordingly, the present invention further provides a pharmaceutical composition comprising a pharmaceutically acceptable carrier and, as active ingredient, a therapeutically effective amount of a compound of Formula (I), a pharmaceutically acceptable addition salt, or a solvate thereof.

The carrier or diluent must be "acceptable" in the sense of being compatible with the other ingredients of the composition and not deleterious to the recipients thereof.

For ease of administration, the subject compounds may be formulated into various pharmaceutical forms for administration purposes. The compounds according to the invention, in particular the compounds of Formula (I) and pharmaceutically acceptable addition salts, and solvates thereof, or any subgroup or combination thereof may be formulated into various pharmaceutical forms for administration purposes. As appropriate compositions there may be cited all compositions usually employed for systemically administering drugs.

To prepare the pharmaceutical compositions of this invention, an effective amount of the particular compound as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, in particular, for administration orally, rectally, percutaneously, by parenteral injection or by inhalation. For example, in preparing the compositions in oral dosage form, any of the usual pharmaceutical media may be employed such as, for example, water, glycols, oils, alcohols and the like in the case of

oral liquid preparations such as suspensions, syrups, elixirs, emulsions and solutions; or solid carriers such as starches, sugars, kaolin, diluents, lubricants, binders, disintegrating agents and the like in the case of powders, pills, capsules and tablets. Because of their ease in administration, tablets and capsules represent the most advantageous oral dosage unit forms in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable solutions, for example, may be prepared in which the carrier comprises saline solution, glucose solution or a mixture of saline and glucose solution. Injectable solutions containing a compound of Formula (I), a pharmaceutically acceptable addition salt, or a solvate thereof, may be formulated in an oil for prolonged action. Appropriate oils for this purpose are, for example, peanut oil, sesame oil, cottonseed oil, corn oil, soybean oil, synthetic glycerol esters of long chain fatty acids and mixtures of these and other oils. Injectable suspensions may also be prepared in which case appropriate liquid carriers, suspending agents and the like may be employed. Also included are solid form preparations that are intended to be converted, shortly before use, to liquid form preparations. In the compositions suitable for percutaneous administration, the carrier optionally comprises a penetration enhancing agent and/or a suitable wetting agent, optionally combined with suitable additives of any nature in minor proportions, which additives do not introduce a significant deleterious effect on the skin. Said additives may facilitate the administration to the skin and/or may be helpful for preparing the desired compositions. These compositions may be administered in various ways, e.g., as a transdermal patch, as a spot-on, as an ointment. Acid or base addition salts of compounds of Formula (I) due to their increased water solubility over the corresponding base or acid form, are more suitable in the preparation of aqueous compositions.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, powder packets, wafers, suppositories, injectable solutions or suspensions and the like, and segregated multiples thereof.

In order to enhance the solubility and/or the stability of the compounds of Formula (I) and pharmaceutically acceptable addition salts, and solvates thereof, in pharmaceutical compositions, it can be advantageous to employ α-, β- or γ-cyclodextrins or their derivatives, in particular hydroxyalkyl substituted cyclodextrins, e.g. 2-hydroxypropyl-β-cyclodextrin or sulfobutyl-β-cyclodextrin. Also co-solvents such as alcohols may improve the solubility and/or the stability of the compounds according to the invention in pharmaceutical compositions.

Depending on the mode of administration, the pharmaceutical composition will preferably comprise from 0.05 to 99 % by weight, more preferably from 0.1 to 70 % by weight, even more preferably from 0.1 to 50 % by weight of the compound of Formula (I), a pharmaceutically acceptable addition salt, or a solvate thereof, and from 1 to 99.95 % by weight, more preferably from 30 to 99.9 % by weight, even more preferably from 50 to 99.9 % by weight of a pharmaceutically acceptable carrier, all percentages being based on the total weight of the composition.

As another aspect of the present invention, a combination of a compound of the present invention with another anticancer agent is envisaged, especially for use as a medicine, more specifically for use in the treatment of cancer or related diseases.

For the treatment of the above conditions, the compounds of the invention may be advantageously employed in combination with one or more other medicinal agents, more particularly, with other anti-cancer agents or adjuvants in cancer therapy. Examples of anti-cancer agents or adjuvants (supporting agents in the therapy) include but are not limited to:

- platinum coordination compounds for example cisplatin optionally combined with amifostine, carboplatin or oxaliplatin;
- taxane compounds for example paclitaxel, paclitaxel protein bound particles (Abraxane™) or docetaxel;
- topoisomerase I inhibitors such as camptothecin compounds for example irinotecan, SN-38, topotecan, topotecan hcl;
- topoisomerase II inhibitors such as anti-tumour epipodophyllotoxins or podophyllotoxin derivatives for example etoposide, etoposide phosphate or teniposide;
- anti-tumour vinca alkaloids for example vinblastine, vincristine or vinorelbine;
- anti-tumour nucleoside derivatives for example 5-fluorouracil, leucovorin, gemcitabine, gemcitabine hcl, capecitabine, cladribine, fludarabine, nelarabine;
- alkylating agents such as nitrogen mustard or nitrosourea for example cyclophosphamide, chlorambucil, carmustine, thiotepa, mephalan (melphalan), lomustine, altretamine, busulfan, dacarbazine, estramustine, ifosfamide optionally in combination with mesna, pipobroman, procarbazine, streptozocin, temozolomide, uracil;
- anti-tumour anthracycline derivatives for example daunorubicin, doxorubicin optionally in combination with dexrazoxane, doxil, idarubicin, mitoxantrone, epirubicin, epirubicin hcl, valrubicin;
- molecules that target the IGF-1 receptor for example picropodophilin;
- tetracarcin derivatives for example tetrocarcin A;
- glucocorticoids for example prednisone;
- antibodies for example trastuzumab (HER2 antibody), rituximab (CD20 antibody), gemtuzumab, gemtuzumab ozogamicin, cetuximab, pertuzumab, bevacizumab, alemtuzumab, eculizumab, ibritumomab tiuxetan, nofetumomab, panitumumab, tositumomab, CNTO 328;
- estrogen receptor antagonists or selective estrogen receptor modulators or inhibitors of estrogen synthesis for example tamoxifen, fulvestrant, toremifene, droloxifene, faslodex, raloxifene or letrozole;
- aromatase inhibitors such as exemestane, anastrozole, letrazole, testolactone and vorozole;
- differentiating agents such as retinoids, vitamin D or retinoic acid and retinoic acid metabolism blocking agents (RAMBA) for example accutane;
- DNA methyl transferase inhibitors for example azacytidine or decitabine;
- antifolates for example premetrexed disodium;
- antibiotics for example antinomycin D, bleomycin, mitomycin C, dactinomycin, carminomycin, daunomycin, levamisole, plicamycin, mithramycin;
- antimetabolites for example clofarabine, aminopterin, cytosine arabinoside or methotrexate, azacitidine, cytarabine, floxuridine, pentostatin, thioguanine;
- apoptosis inducing agents and antiangiogenic agents such as Bcl-2 inhibitors for example YC 137, BH 312, ABT 737, gossypol, HA 14-1, TW 37 or decanoic acid;
- tubuline-binding agents for example combrestatin, colchicines or nocodazole;
- kinase inhibitors (e.g. EGFR (epithelial growth factor receptor) inhibitors, MTKI (multi target kinase inhibitors), mTOR inhibitors) for example

flavoperidol, imatinib mesylate, erlotinib, gefitinib, dasatinib, lapatinib, lapatinib ditosylate, sorafenib, sunitinib, sunitinib maleate, temsirolimus;

- farnesyltransferase inhibitors for example tipifarnib;
- histone deacetylase (HDAC) inhibitors for example sodium butyrate, suberoylanilide hydroxamic acid (SAHA), depsipeptide (FR 901228), NVP-LAQ824, R306465, JNJ-26481585, trichostatin A, vorinostat;
- Inhibitors of the ubiquitin-proteasome pathway for example PS-341, MLN .41 or bortezomib;
- Yondelis;
- Telomerase inhibitors for example telomestatin;
- Matrix metalloproteinase inhibitors for example batimastat, marimastat, prinostat or metastat;
- Recombinant interleukins for example aldesleukin, denileukin diftitox, interferon alfa 2a, interferon alfa 2b, peginterferon alfa 2b;
- MAPK inhibitors;
- Retinoids for example alitretinoin, bexarotene, tretinoin;
- Arsenic trioxide;
- Asparaginase;
- Steroids for example dromostanolone propionate, megestrol acetate, nandrolone (decanoate, phenpropionate), dexamethasone;
- Gonadotropin releasing hormone agonists or antagonists for example abarelix, goserelin acetate, histrelin acetate, leuprolide acetate;
- Thalidomide, lenalidomide;
- Mercaptopurine, mitotane, pamidronate, pegademase, pegaspargase, rasburicase;
- BH3 mimetics for example ABT-737;
- MEK inhibitors for example PD98059, AZD6244, CI-1040;
- colony-stimulating factor analogs for example filgrastim, pegfilgrastim, sargramostim; erythropoietin or analogues thereof (e.g. darbepoetin alfa); interleukin 11; oprelvekin; zoledronate, zoledronic acid; fentanyl; bisphosphonate; palifermin;
- a steroidal cytochrome P450 17alpha-hydroxylase-17,20-lyase inhibitor (CYP17), e.g. abiraterone, abiraterone acetate;
- Glycolysis inhibitors, such as 2-deoxyglucose;
- mTOR inhibitors such as rapamycins and rapalogs, and mTOR kinase inhibitors;
- PI3K inhibitors and dual mTOR/PI3K inhibitors;
- autophagy inhibitors, such as chloroquine and hydroxy-chloroquine;
- B-raf inhibitors, e.g. vemurafenib;

- androgen receptor antagonist drugs, e.g. enzalutamide or ARN-509.

The present invention further relates to a product containing as first active ingredient a compound according to the invention and as further active ingredient one or more anticancer agents, as a combined preparation for simultaneous, separate or sequential use in the treatment of patients suffering from cancer.

The one or more other medicinal agents and the compound according to the present invention may be administered simultaneously (e.g. in separate or unitary compositions) or sequentially in either order. In the latter case, the two or more compounds will be administered within a period and in an amount and manner that is sufficient to ensure that an advantageous or synergistic effect is achieved. It will be appreciated that the preferred method and order of administration and the respective dosage amounts and regimes for each component of the combination will depend on the particular other medicinal agent and compound of the present invention being administered, their route of administration, the particular tumour being treated and the particular host being treated. The optimum method and order of administration and the dosage amounts and regime can be readily determined by those skilled in the art using conventional methods and in view of the information set out herein.

The weight ratio of the compound according to the present invention and the one or more other anticancer agent(s) when given as a combination may be determined by the person skilled in the art. Said ratio and the exact dosage and frequency of administration depends on the particular compound according to the invention and the other anticancer agent(s) used, the particular condition being treated, the severity of the condition being treated, the age, weight, gender, diet, time of administration and general physical condition of the particular patient, the mode of administration as well as other medication the individual may be taking, as is well known to those skilled in the art. Furthermore, it is evident that the effective daily amount may be lowered or increased depending on the response of the treated subject and/or depending on the evaluation of the physician prescribing the compounds of the instant invention. A particular weight ratio for the present compound of Formula (I) and another anticancer agent may range from 1/10 to 10/1, more in particular from 1/5 to 5/1, even more in particular from 1/3 to 3/1.

The platinum coordination compound is advantageously administered in a dosage of 1 to 500mg per square meter ($mg/m^2$) of body surface area, for example 50 to 400 $mg/m^2$, particularly for cisplatin in a dosage of about 75 $mg/m^2$ and for carboplatin in about 300$mg/m^2$ per course of treatment.

The taxane compound is advantageously administered in a dosage of 50 to 400 mg per square meter ($mg/m^2$) of body surface area, for example 75 to 250 $mg/m^2$, particularly for paclitaxel in a dosage of about 175 to 250 $mg/m^2$ and for docetaxel in about 75 to 150 $mg/m^2$ per course of treatment.

The camptothecin compound is advantageously administered in a dosage of 0.1 to 400 mg per square meter ($mg/m^2$) of body surface area, for example 1 to 300 $mg/m^2$, particularly for irinotecan in a dosage of about 100 to 350 $mg/m^2$ and for topotecan in about 1 to 2 $mg/m^2$ per course of treatment.

The anti-tumour podophyllotoxin derivative is advantageously administered in a dosage of 30 to 300 mg per square meter ($mg/m^2$) of body surface area, for example 50 to 250$mg/m^2$, particularly for etoposide in a dosage of about 35 to 100 $mg/m^2$ and for teniposide in about 50 to 250 $mg/m^2$ per course of treatment.

The anti-tumour vinca alkaloid is advantageously administered in a dosage of 2 to 30 mg per square meter ($mg/m^2$) of body surface area, particularly for vinblastine in a dosage of about 3 to 12 $mg/m^2$ , for vincristine in a dosage of about 1 to 2 $mg/m^2$ , and for vinorelbine in dosage of about 10 to 30 $mg/m^2$ per course of treatment.

The anti-tumour nucleoside derivative is advantageously administered in a dosage of 200 to 2500 mg per square meter ($mg/m^2$) of body surface area, for example 700 to 1500 $mg/m^2$, particularly for 5-FU in a dosage of 200 to 500$mg/m^2$, for gemcitabine in a dosage of about 800 to 1200 $mg/m^2$ and for capecitabine in about 1000 to 2500 $mg/m^2$ per course of treatment.

The alkylating agents such as nitrogen mustard or nitrosourea is advantageously administered in a dosage of 100 to 500 mg per square meter ($mg/m^2$) of body surface area, for example 120 to 200 $mg/m^2$, particularly for cyclophosphamide in a dosage of about 100 to 500 $mg/m^2$, for chlorambucil in a dosage of about 0.1 to 0.2 mg/kg, for carmustine in a dosage of about 150 to 200 $mg/m^2$ , and for lomustine in a dosage of about 100 to 150 $mg/m^2$ per course of treatment.

The anti-tumour anthracycline derivative is advantageously administered in a dosage of 10 to 75 mg per square meter ($mg/m^2$) of body surface area, for example 15 to 60 $mg/m^2$, particularly for doxorubicin in a dosage of about 40 to 75 $mg/m^2$, for daunorubicin in a dosage of about 25 to 45$mg/m^2$, and for idarubicin in a dosage of about 10 to 15 $mg/m^2$ per course of treatment.

The antiestrogen agent is advantageously administered in a dosage of about 1 to 100 mg daily depending on the particular agent and the condition being treated. Tamoxifen is advantageously administered orally in a dosage of 5 to 50 mg, preferably 10 to 20 mg twice a day, continuing the therapy for sufficient time to achieve and maintain a therapeutic effect. Toremifene is advantageously administered orally in a dosage of about 60 mg once a day, continuing the therapy for sufficient time to achieve and maintain a therapeutic effect. Anastrozole is advantageously administered orally in a dosage of about 1mg once a day. Droloxifene is advantageously administered orally in a dosage of about

20-100mg once a day. Raloxifene is advantageously administered orally in a dosage of about 60mg once a day. Exemestane is advantageously administered orally in a dosage of about 25mg once a day.

Antibodies are advantageously administered in a dosage of about 1 to 5 mg per square meter ($mg/m^2$) of body surface area, or as known in the art, if different. Trastuzumab is advantageously administered in a dosage of 1 to 5 mg per square meter ($mg/m^2$) of body surface area, particularly 2 to 4 $mg/m^2$ per course of treatment.

These dosages may be administered for example once, twice or more per course of treatment, which may be repeated for example every 7, 14, 21 or 28 days.

The following examples illustrate the present invention. In case no specific stereochemistry is indicated for a stereocenter of a compound or an intermediate, this means that the compound or the intermediate was obtained as a mixture of the R and the S enantiomers.

When an intermediate is indicated as 'HCl salt' or 'TFA salt', this means that the number of equivalents of HCl or TFA was not determined.

## Examples

Hereinafter, the term "NaH" means sodium hydride (60% in mineral oil); "DCM" means dichloromethane; "aq." means aqueous; "TFE" means 2,2,2-trifluoroethanol; "q.s." means quantum sufficit; "MTBE" means methyl tert-butyl ether; "Int." means intermediate; "TBAF" means tetrabutylammonium fluoride; "DPPA" means diphenylphosphoryl azide; "XtalFluor-E®" means (diethylamino)difluorosulfonium tetrafluoroborate; "DBU" means 1,8-diazabicyclo[5.4.0]undecene-7; "AcOH" means acetic acid; "tBuOH" means tert-butanol; "Co." means compound; "r.t." means room temperature; "DCE" means 1,2-dichloroethane; "DIPE" means diisopropyl ether; "DIAD" means diisopropyl azodicarboxylate; "Boc" means *tert*-butoxycarbonyl; "$(BOC)_2O$" means di-*tert*-butyl dicarbonate; "ACN" means acetonitrile; "$NH_4Ac$" means ammonium acetate; "X-Phos" means dicyclohexyl[2',4',6'-tris(1-methylethyl)[1,1'-biphenyl]-2-yl]-phosphine; "S-Phos" means 2-dicyclohexylphosphino-2',6'-dimethoxybiphenyl; "DEA" means diethanolamine; "BDS" means base deactivated silica"; "NMP" means 1-methyl-2-pyrrolidinone; "DMA" means *N*,*N*-dimethylacetamide; "MeOH" means methanol; "LC" means liquid chromatography; "LCMS" means Liquid Chromatography/Mass spectrometry; "HATU" means 1-[bis(dimethylamino)methylene]-1H-[1,2,3]triazolo[4,5-b]pyridin-1-ium 3-oxide hexafluorophosphate; "HPLC" means high-performance liquid chromatography; "BINAP" means [1,1'-binaphthalene]-2,2'-diylbis[diphenylphosphine] (racemic); "TFA" means trifluoroacetic acid; "m.p." means melting point; "$N_2$" means nitrogen; "RP" means reversed phase; "min" means minute(s); "h" means hour(s); "EtOAc" means ethyl acetate; "$Et_3N$" means triethylamine; "PE" means petroleum ether; "Xantphos" means (9,9-dimethyl-9H-xanthene-4,5-diyl)bis[diphenylphosphine]; "EtOH" means ethanol; "THF" means tetrahydrofuran; "Celite®" means diatomaceous earth; "DMF" means *N*,*N*-dimethyl formamide; "DMSO" means dimethyl sulfoxide; "DECP" means diethyl cyanophosphonate; 'iPrOH" means 2-propanol; "$iPrNH_2$" means isopropylamine; "SFC" means Supercritical Fluid Chromatography; "DIPEA" means N,N-diisopropylethylamine; "$Pd(PPh_3)_4$" means tetrakis(triphenylphosphine)palladium; "HBTU" means 1-[bis(dimethylamino)methylene]-1H-benzotriazol-1-ium 3-oxide hexafluorophosphate; "w/v" means weight/volume; "$NaBH(OAc)_3$" means sodium triacetoxyborohydride; "$PPh_3$" means triphenylphosphine; "$Et_2O$" means diethyl ether; "Pd/C" means palladium on carbon; "Pt/C" means platina on carbon; "$Pd(OAc)_2$" means palladium(II) acetate; "$Pd_2(dba)_3$" means tris(dibenzylideneacetone)dipalladium; "Et" means ethyl; "Me" means methyl; "$PdCl_2(dppf)$-DCM" means [1,1'-bis(diphenylphosphino-κP)ferrocene]dichloropalladium-dichloromethane (1:1); "$PdCl_2(dppf)$" means [1,1'-bis(diphenylphosphino-κP)ferrocene]dichloropalladium; and "TLC" means thin layer chromatography.

## A. Preparation of the Intermediates

### Example A1

#### a) Preparation of Int. 1

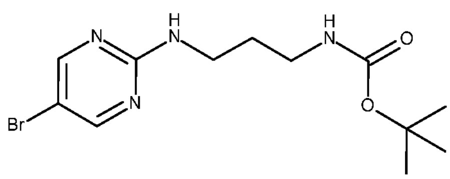

2-Chloro-5-bromo pyrimidine (23.4 g; 121 mmol) was dissolved in EtOH (450 ml). N-(3-aminopropyl)carbamic acid tert-butyl ester (52.7 g; 302.5 mmol) was added. The mixture was refluxed for 6 h, then cooled to r.t. The mixture was filtered. The filter cake was washed with aq. $Na_2CO_3$ and dried yielding 45 g of intermediate 1 (100 %).

The intermediates in the table below were prepared according to an analogous reaction protocol as used for Int. 1:

| | |
|---|---|
| 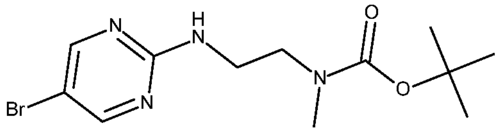<br>Int. 2 (from 2-chloro-5-bromo pyrimidine and *N*-(2-aminoethyl)-*N*-methyl-carbamic acid, 1,1-dimethylethyl ester) | 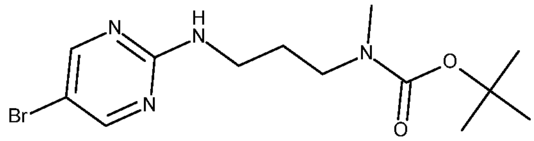<br>Int. 3 (from 2-chloro-5-bromo pyrimidine and *N*-(3-aminopropyl)-*N*-methyl-carbamic acid, 1,1-dimethylethyl ester) |

b) Preparation of Int. 4

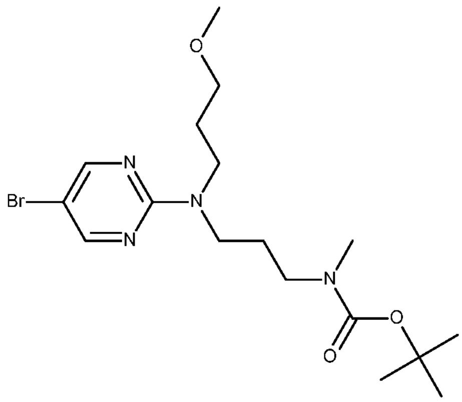

NaH 60 % (603 mg; 15.081 mmol) was added to a stirred solution of Int. 3 (1.7 g; 5.027 mmol) in DMF (9.7 mL) at 0 °C. The reaction mixture was stirred for 20 min. 1-Bromo-3-methoxypropane (1 g; 6.535 mmol) was added at 0 °C. The reaction mixture was allowed to stir at r.t. for 1 h, hydrolysed with water and extracted with EtOAc. The organic layer was washed with water, dried over $MgSO_4$, filtered and evaporated. The residue was purified by preparative LC on (irregular 15-40 μm 90g Merck). Mobile phase (85 % heptane, 15 % EtOAc). The desired fractions were collected and the solvent was evaporated to give Int. 4 (1.66 g; 79 %).

The intermediates in the table below were prepared according to an analogous reaction protocol as used for Int. 4:

| | |
|---|---|
| 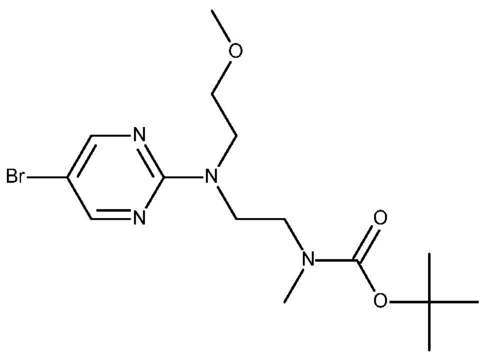<br>Int. 5 (from Int. 2 and 2-bromoethyl methyl ether) | 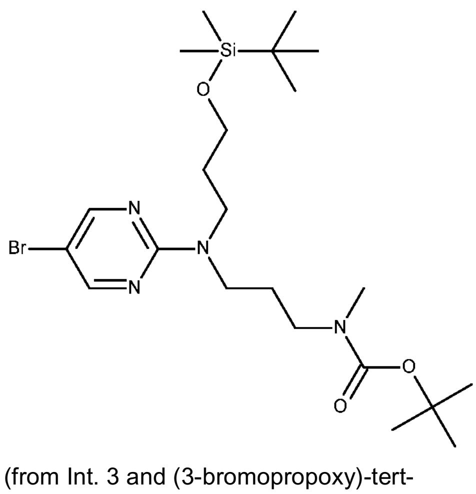<br>Int. 6 (from Int. 3 and (3-bromopropoxy)-tert-butyldimethylsilane) |

(continued)

| | |
|---|---|
| 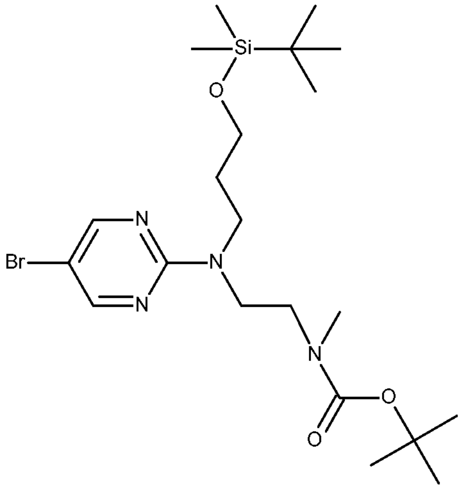 Int. 7 (from Int. 2 and (3-bromopropoxy)-tert-butyldimethylsilane) | |

c) Preparation of Int. 8

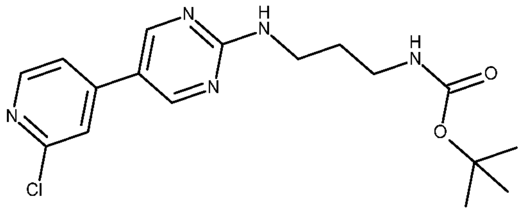

Int. 1 (34.4 g; 104 mmol) was dissolved in dioxane (1000 ml). 2-Chloropyridine-4-boronic acid (18 g; 114 mmol), $Pd(PPh_3)_4$ (6.12 g; 2.12 mmol) and $Na_2CO_3$ (2 M aq. solution) (235 ml) were added under $N_2$ gas atmosphere. The mixture was refluxed overnight. The mixture was filtered and the filtrate was concentrated. The residue was purified by column chromatography over silica gel (eluent: PE / EtOAc 10/1). The desired fractions were collected and the solvent was evaporated yielding 19 g of Int. 8 (53.0 %).

The intermediates in the table below were prepared according to an analogous reaction protocol as used for Int. 8:

| | |
|---|---|
| 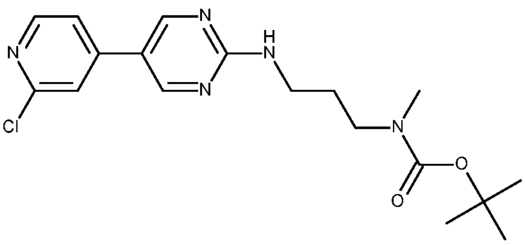 Int. 9 (from Int. 3) | 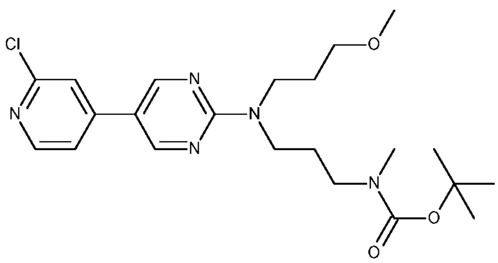 Int. 10 (from Int. 4) |

(continued)

| | |
|---|---|
| 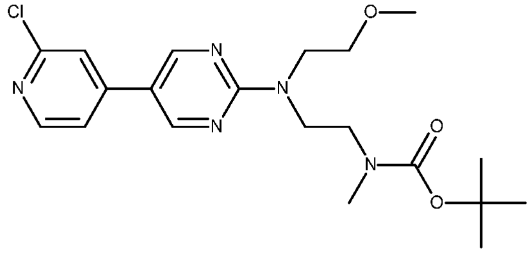 Int. 11 (from Int. 5) | 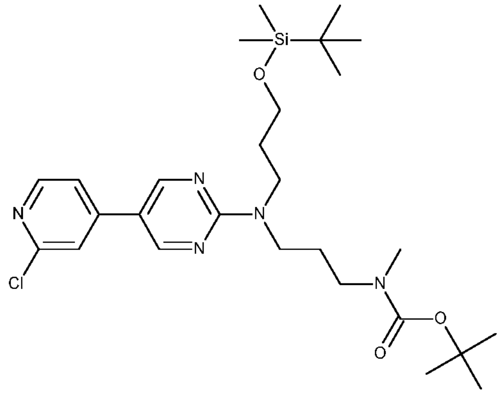 Int. 12 (from Int. 6) |
| 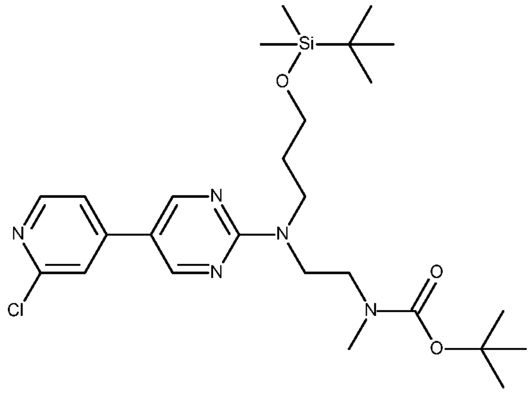 Int. 13 (from Int. 7) | |

The intermediates in the table below were prepared by using successively analogous reaction protocols as used for Int. 1 and Int. 8:

| |
|---|
| 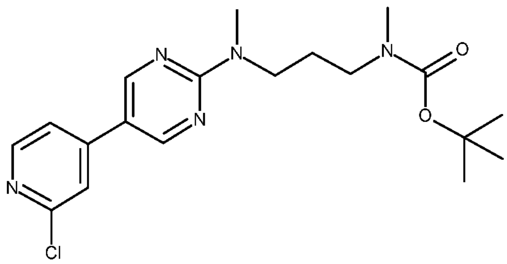 |
| Int. 86 (from 2-chloro-5-bromo pyrimidine and *N*-methyl-*N*-[3-(methylamino)propyl]-carbamic acid, 1,1-dimethylethyl ester) |

Example A2-a

a) Preparation of Int. 14

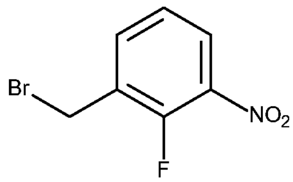

A mixture of 2-fluoro-3-nitrotoluene (33 g; 213 mmol), N-bromosuccinimide (41.7 g; 234.3 mmol) and a catalytic amount of azobisisobutyronitrile in carbon tetrachloride (300 ml) was heated to reflux for 24 h. The mixture was filtered. The organic solvent was evaporated *in vacuo* to yield 50 g of Int. 14 (100 %).

b) Preparation of Int. 15

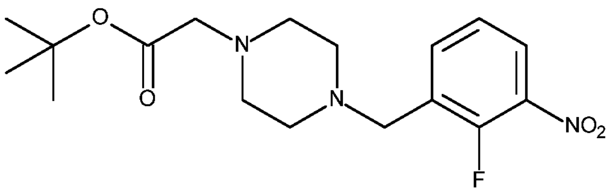

Piperazine-1-acetic acid tert-butyl ester (42.6 g, 213 mmol) was added to a suspension of Int. 14 (50 g, 213 mmol) and potassium carbonate in ACN (200 ml). The mixture was stirred at r.t. for 2 h. The mixture was filtered. The organic solvent was evaporated *in vacuo*. The residue was purified by chromatography on silica gel (PE/EtOAc 8/1 to pure EtOAc). The pure fractions were collected and the solvent was evaporated to yield 38 g of Int. 15 (50 %).

c) Preparation of Int. 16

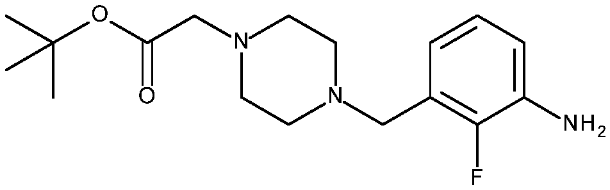

Int. 15 (38 g; 108 mmol) was dissolved in a mixture of THF (120 ml), water (60 ml) and MeOH (60 ml). Fe (60.3 g;1080 mmol) and ammonium chloride (57.8 g; 1080 mmol) were added. The mixture was refluxed for 2 h. The mixture was filtered. Brine and DCM were added to the filtrate. The organic layer was separated, dried over $Na_2SO_4$ and evaporated to dryness to give 26 g of Int. 16 (74 %).

Example A2-b

a) Preparation of Int. 17

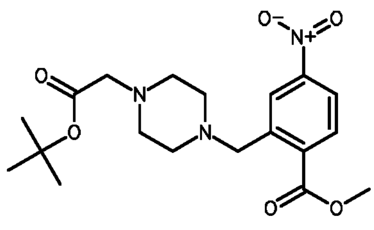

A solution of 2-bromomethyl-4-nitrobenzoic acid methyl ester (110 g, 401 mmol), piperazine-1-acetic acid tert-butyl ester (81 g, 405 mmol) and $K_2CO_3$ (q.s.) in ACN (1000 ml) was stirred for 6 h at 50 °C. The precipitate was filtered off and the solvent was removed. The residue was purified by column chromatography over silica gel (gradient eluent: PE/EtOAc from 10/1 to 1/1). The desired fractions were collected and the solvent was evaporated. Yield: 130 g of Int. 17 (93 %).

b) Preparation of Int. 18

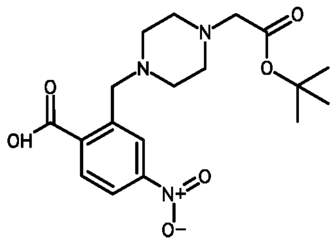

A solution of Int. 17 (91 g, 231.3 mmol) and LiOH (1 mol/L in water; 693.9 mL, 693.9 mmol) in THF (700 mL) was stirred for 3 h at r.t.. The pH of the reaction was adjusted to to pH 4-5 by addition of 2 N HCl. The organic solvent was evaporated under reduced pressure. The mixture was cooled to r.t., and the precipitate was filtered off and dried to give 70 g of Int. 18 (80 %).

c) Preparation of Int. 19

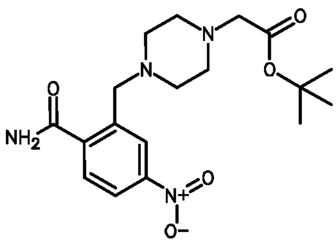

A solution of Int. 18 (33 g, 87 mmol), ammonium hydrochloride (6.52g, 121.8 mmol), 1-hydroxy-1H-benzotriazole hydrate (14.11 g, 104.4 mmol), 3-ethyl-1-(3-dimethylaminopropyl)carbodiimide .HCl (20.01 g, 104.4 mmol) and $Et_3N$ (35.21 g, 348 mmol) in DMF (250ml) was stirred overnight at r.t.. The mixture was evaporated *in vacuo,* water was added to the residue and this aqueous mixture was extracted with DCM. The organic phase was washed with water, brine, dried over $Na_2SO_4$ and filtered. The solvent was evaporated and the crude product was purified by column chromatography over silica gel (eluent: EtOAc). The desired fractions were collected and the solvent was evaporated. Yield: 18.8 g of Int. 19 (57 %).

The intermediates in the table below were prepared according to an analogous reaction protocol as used for Int. 19:

| |
|---|
| 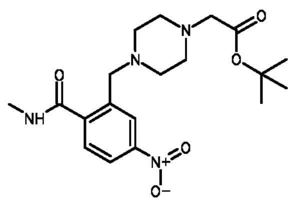 Int. 20 (starting from Int. 18 and methylamine hydrochloride) |

d) Preparation of Int. 21

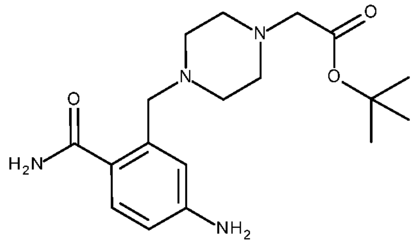

Pt/C (5 %)(1 g, 5.1 mmol) was added to a solution of Int. 19 (18.8 g , 49.7 mmol) in EtOH (350 ml) and the resulting suspension was hydrogenated under a hydrogen atmosphere for 15 h at 40 °C. The catalyst was removed by filtration and the filtrate was evaporated under reduced pressure. Yield: 16.0 g of Int. 21 (92 %).

The intermediates in the table below were prepared according to an analogous reaction protocol as used for Int. 21:

| |
|---|
| 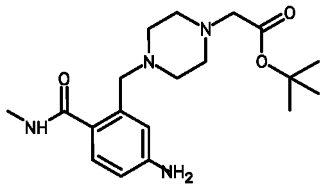 Int. 22 (starting from Int. 20) |

Example A2-c

a) Preparation of Int. 23

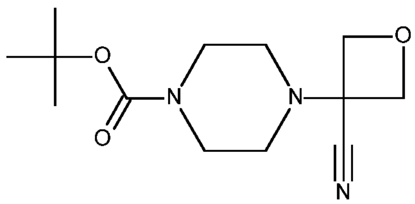

A solution of 3-oxetanone (4 g; 55 mmol), trimethylsilylcyanide (5.46 g; 55 mmol) and $ZnI_2$ (0.010 g) in tert-butyl methyl ether (10 ml) was stirred overnight. Then 1-piperazinecarboxylic acid, 1,1-dimethylethyl ester, acetate (1:1) was added and the solution was further stirred overnight. The solvent was removed. The product was directly used as such for the next reaction step.Yield: 14 g of Int. 23 (98 %).

b) Preparation of Int. 24

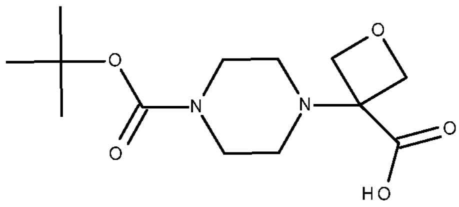

A solution of Int. 23 (14 g; 52 mmol) in 2 M NaOH (aqueous; 50 ml) and MeOH(100 ml) was refluxed for 6 h and then concentrated. The remaining solution was cooled to 0 °C, acidified to pH 5 by the addition of concentrated HCl, and then extracted with EtOAc. The combined organic extracts were dried ($MgSO_4$), filtered and concentrated to give the product as a white solid. Yield: 14 g of Int. 24 (94 %).

<u>c) Preparation of Int. 25</u>

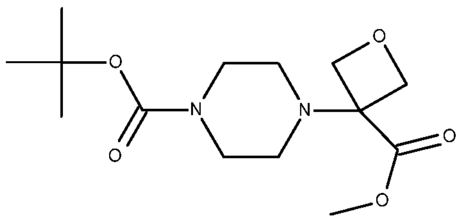

To a mixture of Int. 24 (10 g; 34.92 mmol) and $Cs_2CO_3$ (16.9 g; 52 mmol) in DMF(30 ml) was added MeI (10.50 g; 74 mmol) at r.t.. After stirring for 12 h, the mixture was filtered over Celite®. The filtrate was evaporated and the residue was purified with column chromatography (eluent: 100/10 PE/EtOAc) over silica gel. The desired fraction was collected and the solvent was removed to give 7.2 g of Int. 25 as white solid which was used as such in the next reaction step.

<u>d) Preparation of Int. 26</u>

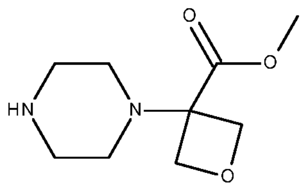

TFA salt

Int. 25 (1.2 g; 4 mmol) in 20 % TFA in DCM (10 ml) was stirred for 1 h. The solvent was removed to give the crude product, which was directly used as such for next reaction step.

<u>e) Preparation of Int. 27</u>

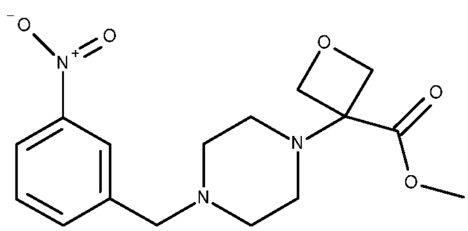

A mixture of Int. 26 (crude; approximately 4 mmol) , 3-nitrobenzylbromide (0.86 g; 4 mmol), $K_2CO_3$ (2.2 g; 16 mmol) and ACN (20 ml) was stirred overnight at r.t. After filtration, the solvent was removed and the crude product was purified by column chromatography over silica gel (eluent: EtOAc). The desired fraction was collected and the solvent was removed to give the product. Yield: 0.67 g of Int. 27.

f) Preparation of Int. 28

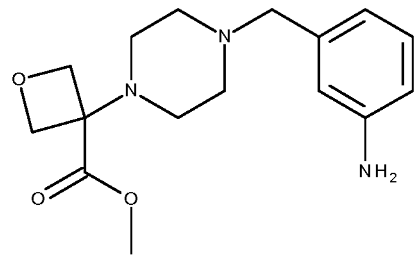

Int. 27 (0.67 g; 2 mmol) with Pt/C (0.1 g) as a catalyst in MeOH (10 ml) was hydrogenated under $H_2$ (20 Psi) at r.t. for 16h. Then the catalyst was filtered off and the solvent was evaporated to give Int. 28 (0.6 g; 92 % yield).

Example A2-d

a) Preparation of Int. 29

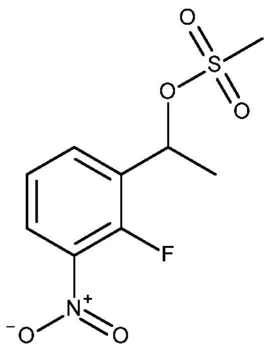

To a solution of 2-fluoro-3-nitro-benzene-1-ethanol (4.6 g; 23.602 mmol) in DCM (50 ml) was added DIPEA (10.611 ml; 61.572 mmol) and mesylchloride (2.383 ml; 30.786 mmol) at 0 °C. The resulting mixture was stirred at r.t. for 2 h. Saturated aqueous $NaHCO_3$ was added and EtOAc was added to extract the product. The organic layer was washed with brine, dried over $Na_2SO_4$ and filtered. The solvent was removed *in vacuo* to give 5 g of Int. 29 (80 %).

b) Preparation of Int. 30

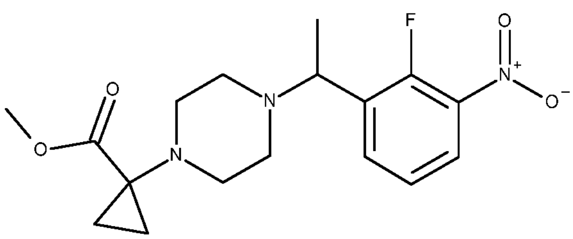

1-(1-Piperazinyl)-cyclopropanecarboxylic acid, methyl ester .HCl (3.521 g; 15.955 mmol) was added to the mixture of Int. 29 (4.2 g; 15.955 mmol) and $K_2CO_3$ (8.832 g; 64 mmol) in DMF(50 ml) and the reaction mixture was stirred at r.t. for 4 h. The mixture was poured into water and extracted with EtOAc. The organic layer was washed with brine, dried, filtered and evaporated *in vacuo.* The crude was dissolved in DCM and 10 ml HCl/dioxane (4 M)was added. The precipitate was filtered and was then dissolved in $H_2O$ and adjusted to pH > 7. The water layer was extracted with EtOAc and evaporated. The residue was separated by HPLC Column: Chiralpak OJ-H 250×4.6mm I.D., 5 μm, Mobile phase: methanol (0.05% ethanolamine) in $CO_2$ from 5% to 40% , Flow rate: 2.35mL/min. The desired fractions were collected and the solvent was evaporated to give 150 mg of Int. 30 (40%).

c) Preparation of Int. 31

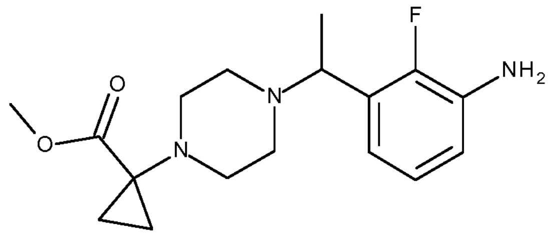

The mixture of Int. 30 (2.8 g; 7.969 mmol), Fe (4.768 g; 85.38 mmol), $NH_4Cl$ (4.567 g; 85.38 mmol) in MeOH/$H_2O$/THF 1/1/2 (60 ml) was refluxed for 30 min. The organic solvent was removed *in vacuo.* The residue was extracted with EtOAc. The organic layer was collected and evaporated *in vacuo* to give 2.473 g of Int. 31 which was used as such for the next reaction step.

Example A2-e

a) Preparation of Int. 32

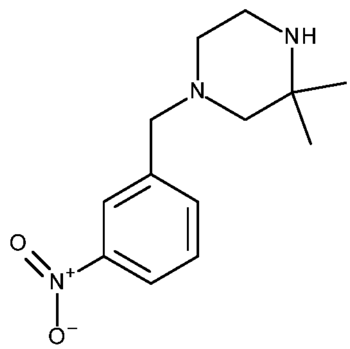

2,2-Dimethylpiperazine (2.3 g; 20 mmol) and $K_2CO_3$(5.5 g; 40 mmol) were dissolved in ACN (50 ml) at 25 °C. 3-Nitrobenzyl bromide (4.3 g; 20 mmol) was added dropwise at 25 °C and the reaction was stirred for another 16 h. The reaction mixture was filtered and the filtrate was concentrated. The crude was purified by chromatography column (DCM/MeOH 5/1). The desired fractions were collected and the solvent was evaporated to give a yellow solid. Yield: 2 g of Int. 32 (40%).

b) Preparation of Int. 33

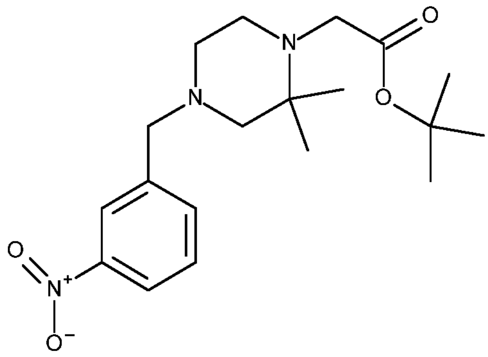

Int. 32 (2 g; 8 mmol) and $K_2CO_3$ (2.5 g; 18 mmol) were stirred in ACN (30 ml) at 25 °C. 2-Bromo-acetic acid, 1,1-dimethylethyl ester (1.9 g; 9 mmol) was added dropwise at 25 °C and the mixture was stirred for 16 h. The mixture was filtered and the filtrate was concentrated. The crude was purified by chromatography column (PE/EtOAc 5/1). The desired fractions were collected and the solvent was evaporated to give a yellow solid. Yield: Int. 33 (1.6 g; 63 %).

c) Preparation of Int. 34

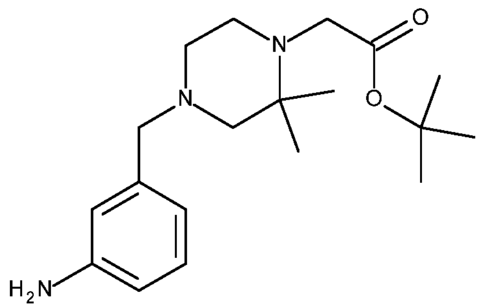

Under $H_2$ gas atmosphere, Pt/C(0.2 g) was added to Int. 33 (1.6 g; 4.4 mmol) dissolved in MeOH (20 ml) and the mixture was hydrogenated at 20 °C for 12 h. The catalyst was filtered off and the filtrate was concentrated to give an oil. Yield: Int. 34 (1.5 g; 81.7 %).

Intermediate 73 was prepared by using successively analogous reaction protocols as used for Int. 32, Int. 33, Int. 34 and Int. 35, starting from 4,7-diazaspiro[2.5]octane, hydrochloride (1:2) and 3- nitrobenzyl bromide:

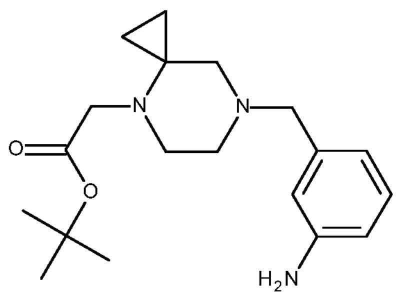

Int. 73

Example A2-f

a) Preparation of Int. 35

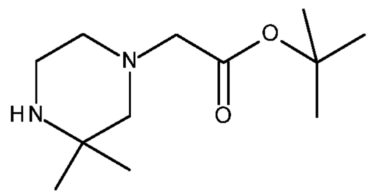

The mixture of 2,2-dimethyl-piperazine (2 g; 17.5 mmol), 2-bromo-acetic acid, 1,1-dimethylethyl ester (3.4 g; 17.5 mmol) and $Na_2CO_3$ (3.7 g; 35 mmol) in ACN (30 ml) was stirred at r.t. overnight. The solid was filtered. The filtrate was concentrated to give crude product. Yield: 4.0 g of Int. 35 (100 %).

b) Preparation of Int. 36

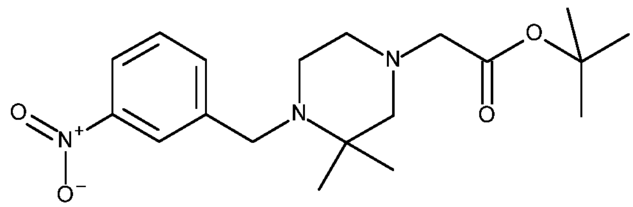

The mixture of Int. 35 (4 g; 17.5 mmol), 3- nitro benzyl bromide (3.8 g; 17.5 mmol) and $Na_2CO_3$ (3.7 g; 35 mmol) in ACN (30 ml) was stirred at r.t. overnight. The solid was filtered. The filtrate was concentrated to give crude product. Yield: 4.5 g of intermediate 36 (70.3 %).

c) Preparation of Int. 37

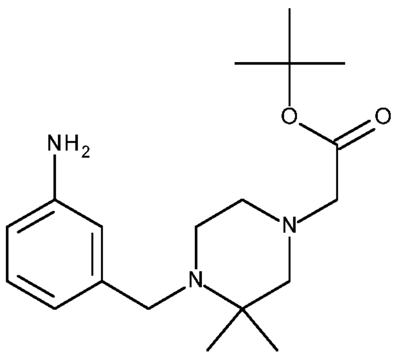

Int. 36 (4.4 g; 12 mmol) in EtOH (100 ml) was hydrogenated under $H_2$ gas atmosphere (20 Psi) with Pt/C (0.5 g) as catalyst at r.t. After consumption of 3 eq. of $H_2$ the catalyst was filtered off and the filtrate evaporated to give the desired product. Yield: 4 g of Int. 37 (100 %).

Intermediate 74 was prepared by using successively analogous reaction protocols as used for Int. 35, Int. 36 and Int. 37, starting from 4,7-diazaspiro[2.5]octane, hydrochloride (1:2) and 2-bromo-acetic acid, 1,1-dimethylethyl ester:

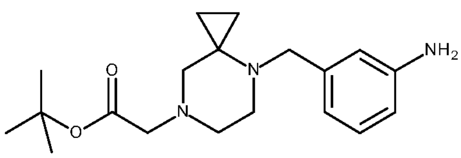

Int. 74

Example A2-g

a) Preparation of Int. 40

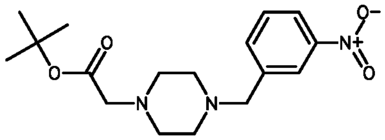

The synthesis protocol was conducted twice on the same quantities of 1-(3-nitrobenzyl)piperazine (20 g; 84.74 mmol).

NaH (60 % in mineral oil) (8.7 g; 216.94 mmol) was added portionwise to a stirred solution of 1-(3-Nitrobenzyl)piperazine (40 g; 180.784 mmol) in DMF (190 mL) at r.t.. The reaction mixture was stirred for 20 minutes. Tert-butyl bromoacetate (26.5 mL; 180.784 mmol) was added dropwise at 5 °C. The reaction mixture was stirred for 20 minutes. Water and EtOAc were added and the layers were separated. The organic layer was dried ($MgSO_4$), filtered and evaporated to dryness. The solid was purified by preparative LC (Irregular SiOH 20-45 μm 1000 g DAVISIL). Mobile phase (60 % Heptane, 3 % MeOH, 37 % EtOAc). The desired fractions were collected and the solvent was evaporated.

Total yield: 44.5 g of Int. 40 (73 %).

b) Preparation of Int. 41

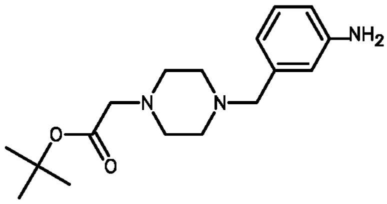

The synthesis protocol was conducted twice on the same quantities of Int. 40 (9 g; 26.833 mmol).

A mixture of Int. 40 (18 g; 53.667 mmol) in MeOH (650 mL) was hydrogenated under $H_2$-gas atmosphere at atmospheric pressure at r.t. in the presence of Raney nickel (19 g; 322.819 mmol) as a catalyst. The catalyst was filtered off on a pad of Celite® and the filtrate was evaporated. Total yield 15.3 g of Int. 41 (93 %).

Example A2-h

a) Preparation of Int. 62

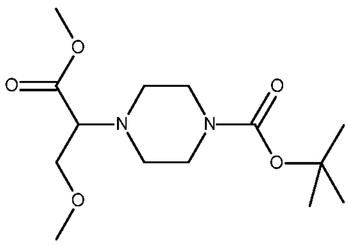

A mixture of 1-piperazinecarboxylic acid, 1,1-dimethylethyl ester, acetate (1:1) (9.5 g; 50.8 mmol), 2-bromo-3-methoxy-propanoic acid, methyl ester (10.0 g; 50.8 mmol) and $K_2CO_3$ (10.5 g; 76.2 mmol) in ACN (150 ml) was stirred overnight at r.t.. Then the mixture was poured into water and extracted with EtOAc. The organic phase was washed with water, brine, dried over $Na_2SO_4$ and evaporated in vacuum. The residue was purified by column chromatography over

silica gel (eluent: DCM/EtOAc 1/1). The desired fractions were collected and the solvent was evaporated to afford 13.9 g of Int. 62 (51 %).

b) Preparation of Int. 63

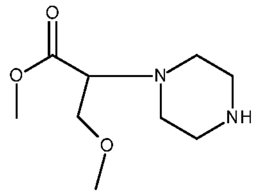

HCl salt

A solution of Int. 62 (2.6 g; 8.6 mmol) in HCl/dioxane (15 ml) was stirred for 16 h at r.t.. The reaction mixture was concentrated under vacuum. 2 g of crude Int. 63 was obtained which was used as such in the next reaction step.

c) Preparation of Int. 64

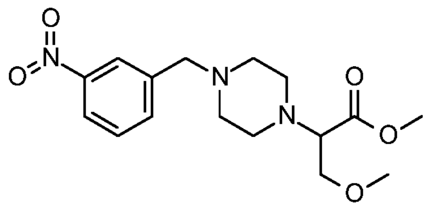

A solution of Int. 63 (2 g) in ACN (15 ml) was stirred at r.t.. Then $K_2CO_3$ (4.6 g; 33.6 mmol) was added. After 10 minutes 3- nitro benzylchloride (2 g; 9.26 mmol) was added. The reaction mixture was stirred for 20 h. Then the mixture was concentrated under vacuum and the residue was taken up into water and extracted with EtOAc. The organic layer was dried over $MgSO_4$, filtered and evaporated. The residue was purified by column chromatography on silica gel (PE/EtOAc 3/1). The desired fractions were collected and the solvent was evaporated. Yield: 2.2 g of Int. 64.

d) Preparation of Int. 65

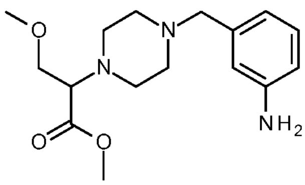

A mixture of Int. 64 (2.2 g; 6.5 mmol) with Pt/C (0.2 g) as a catalyst in MeOH (15 ml) was hydrogenated at r.t. for 20 h under $H_2$ gas flow. The catalyst was filtered off and the filtrate was concentrated under vacuum. The residue was used as such in the next reaction step. Yield: 2 g of Int. 65 (100 %).

Intermediate 66 was prepared by using successively analogous reaction protocols as used for Int. 62, Int. 63, Int. 64 and Int. 65, starting from 2-bromo-3-hydroxy-propanoic acid, methyl ester and piperazinecarboxylic acid, 1,1-dimethylethyl ester, acetate (1:1):

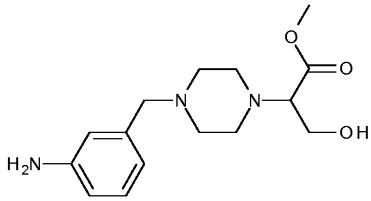

Int. 66

Example A2-i

a) Preparation of Int. 67

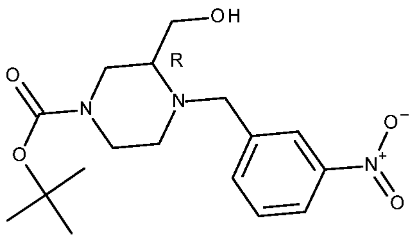

The mixture of (3R)-3-(hydroxymethyl)-1-piperazinecarboxylic acid, 1,1-dimethylethyl ester (2.41 g; 11.15 mmol), 3- nitro benzylbromide (2.53 g; 11.7mmol) and $K_2CO_3$(1.34 g; 29 mmol) in ACN (50 ml) was stirred at r.t. for 12 h. The precipitate was filtered off. The filtrate was concentrated *in vacuo.* The crude was purified by column chromatography (eluent: PE/ EtOAc 4/1). The desired fractions were collected and the solvent was evaporated. Yield: 3.1 g of Int. 67 (88 % yield).

b) Preparation of Int. 68

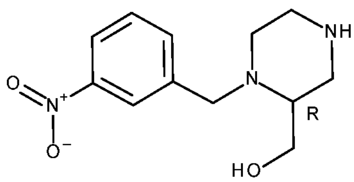

HCl salt

The mixture of Int. 67 (3.1 g; 8.83 mmol) in HCl (4 M in dioxane) (30 ml)was stirred at r.t. for 3 h. The solvent was removed *in vacuo* to give 4 g of crude Int. 68 which was used as such in the next reaction step.

c) Preparation of Int. 69

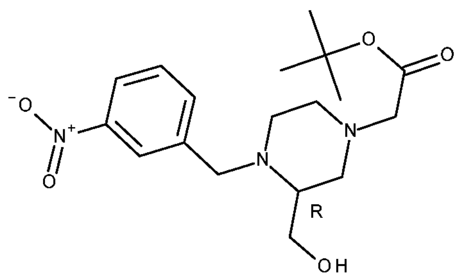

The mixture of Int. 68 (4 g), 2-bromo-acetic acid, 1,1-dimethylethyl ester (1.8 g; 9.3 mmol) and $K_2CO_3$ (3.6 g; 25.5 mmol) in ACN(60 ml) was stirred at r.t. for 12 h. The precipitate was filtered off. The filtrate was concentrated *in vacuo.* The crude was purified by column chromatography (eluent: PE/EtOAc 4/1). The desired fractions were collected and the solvent was evaporated. Yield: 2.8 g of Int. 69 (88% yield for two steps).

d) Preparation of Int. 70

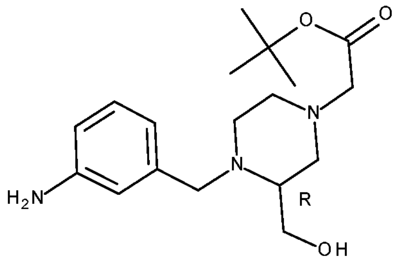

A mixture of Int. 69 (2.8 g; 7.66 mmol) in MeOH(20 ml) was hydrogenated at r.t. under atmospheric pressure of $H_2$ gas with Pt/C (0.1 g) as a catalyst. After uptake of $H_2$ (3 eq.), the catalyst was filtered off and the filtrate was evaporated. Yield: 2.4 g of Int. 70 (93 %).

The intermediates in the table below were prepared by using successively analogous reaction protocols as used for Int. 67, Int. 68, Int. 69 and Int. 70:

| | |
|---|---|
| 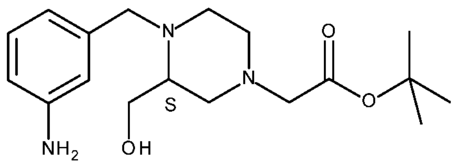 | 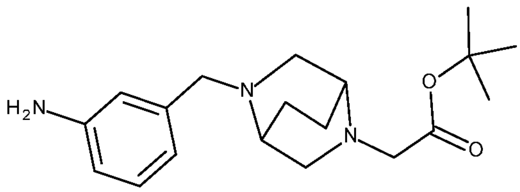 |
| Int. 71 (starting from (3S)-3-(hydroxymethyl)-1-piperazinecarboxylic acid, 1,1-dimethylethyl ester and 3-nitrobenzylbromide) | Int. 72 (starting from 2,5-diazabicyclo[2.2.2]octane-2-carboxylic acid, 1,1-dimethylethyl ester and 3 - nitro benzyl bromide) |

Example A2-i

a) Preparation of Int. 75

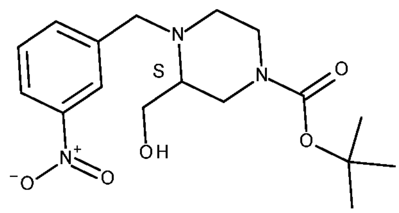

The mixture of (3S)-3-(hydroxymethyl)-1-piperazinecarboxylic acid, 1,1-dimethylethyl ester (3.5g; 16.183 mmol), 3- nitro benzyl bromide (5.243 g; 24.27 mmol), $K_2CO_3$(6.71 g; 48.55 mmol) and ACN(50 ml) was stirred at r.t. for 12 h. The solid was filtered off. The solvent was evaporated. The residue was purified by short column chromatography on silica gel (PE/EtOAc 1/1). The desired fractions were collected and the solvent was evaporated to give 2.5 g of Int. 75 (88 %).

b) Preparation of Int. 76

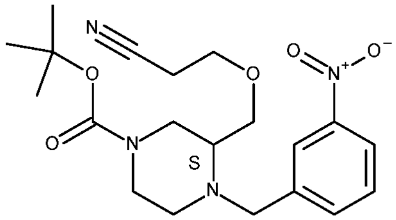

Tetrabutylammonium iodide (0.665 g; 1.8 mmol) was added to the mixture of Int. 75 (5 g; 14.229 mmol) in NaOH (80 ml; 640 mmol) and toluene (8 ml) at r.t. Then acrylonitrile (20.4 g; 384.5 mmol) was added to the mixture. The mixture was stirred at r.t. for 0.5 hour, poured into water and extracted with EtOAc. The organic layer was dried and evaporated. The crude product was purified by short column chromatography on silica gel (PE/EtOAc 1/1). The desired fractions were collected and the solvent was evaporated to give 5.7 g of Int. 76 (95 %).

c) Preparation of Int. 77

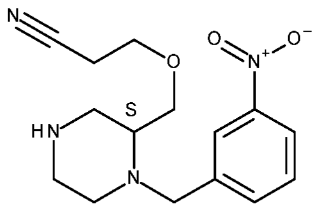

TFA salt

A mixture of Int. 76 (5 g; 11.75 mmol), TFA(9 ml) and DCM(27 ml) was stirred at r.t. for 2 h. The solvent was removed to obtain 5.17 g of Int. 77.

d) Preparation of Int. 78

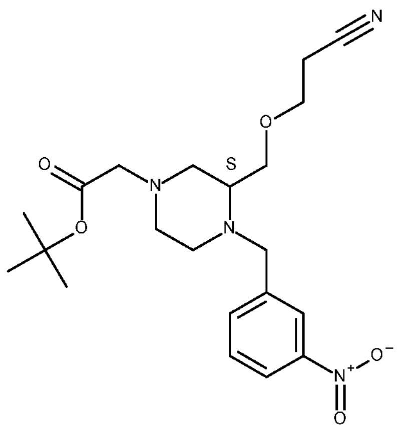

The mixture of Int. 77 (5.172 g), 2-bromo-acetic acid, 1,1-dimethylethyl ester (3.511 g; 18 mmol), $K_2CO_3$ (8.28 g; 60 mmol) and ACN (100 ml) was stirred at r.t. for 12 h. The solid was filtered off. The solvent was evaporated. The residue was purified by short column chromatography on silica gel (PE/EtOAc 4/1). the desired fractions were collected and the solvent was evaporated to give 3.5 g of Int. 78.

e) Preparation of Int. 79

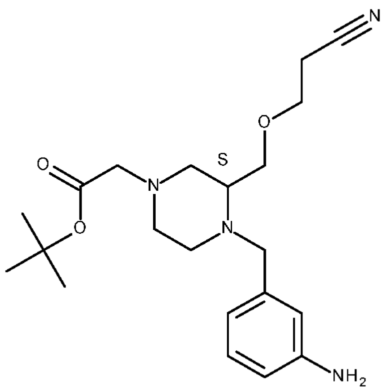

Int. 78 (3.5 g; 7.95 mmol) was dissolved in THF (50 ml), MeOH (25 ml) and water (25 ml). Iron (4.4 g; 79 mmol) and $NH_4Cl$ (4.23 mmol; 79 mmol) were added. The mixture was refluxed for 2 h. The mixture was filtered. Brine and DCM were added to the filtrate. The organic layer was separated, dried ($Na_2SO_4$), filtered and evaporated. Yield: 3 g of Int. 79 (92 %).

Example A3

a) Preparation of Int. 38

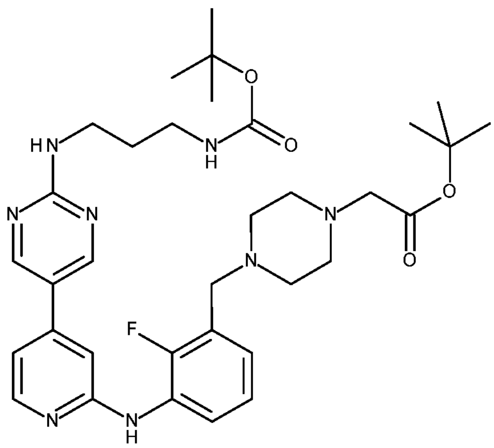

Int. 8 (727.693 mg; 2 mmol) and Int. 16 (905.55 mg; 2.8 mmol) were dissolved in dioxane (6 ml). $Pd_2(dba)_3$ (183.147 mg; 0.2 mmol), S-phos (82.1 mg; 0.2 mmol) and $Cs_2CO_3$ (1303.277 mg; 4 mmol) were added. The mixture was heated at 160 °C for 55 min. The reaction mixture was poured into dioxane (100 ml). The mixture was filtered and the filtrate concentrated. The residue was purified by Prep HPLC on (RP Vydac Denali C18 - 10 μm, 200 g, 5 cm). Mobile phase (0.25 % $NH_4HCO_3$ solution in water, ACN). The desired fractions were collected, evaporated, solved in MeOH and evaporated again. Yield: 740 mg of Int. 38 (56.9 %).

b) Preparation of Int. 39

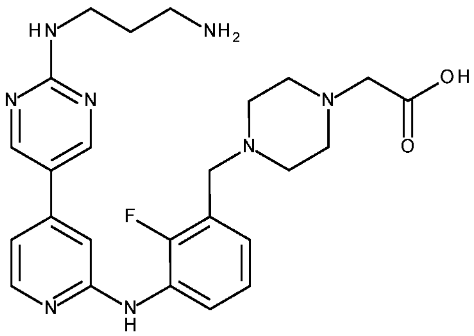

TFA salt

A solution of Int. 38 (740 mg; 1.137 mmol) in TFA (9.326 g; 81.789 mmol) and DCM (18.652 ml) was stirred at r.t. for 48 h. The reaction mixture was concentrated under reduced pressure and the residue was used as such in the next step. Yield 1.211 g of Int. 39.

The intermediates in the table below were prepared by first using an analogous reaction protocol as used for Int. 38, followed by an analogous reaction protocol as used for Int. 39.

| | |
|---|---|
| 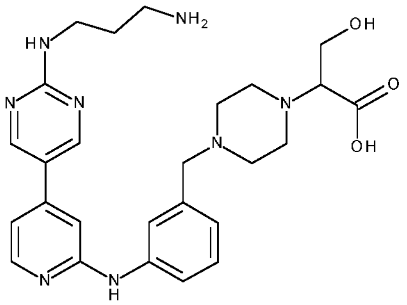 TFA salt<br>Int. 42 (starting from Int. 8 and Int. 66; used for Co. 30) | 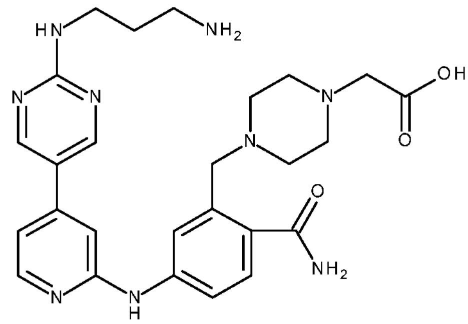 TFA salt<br>Int. 43 (starting from Int. 8 and Int. 21; used for Co. 4) |
| 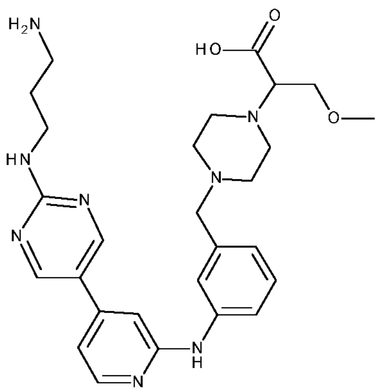 TFA salt<br>Int. 44 (starting from Int. 8 and Int. 65; used for Co. 31) | 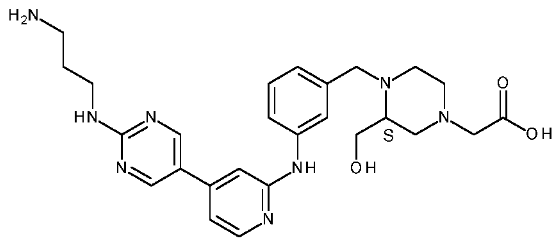 TFA salt<br>Int. 45 (starting from Int. 8 and Int. 71; used for Co. 7) |

(continued)

| | |
|---|---|
| 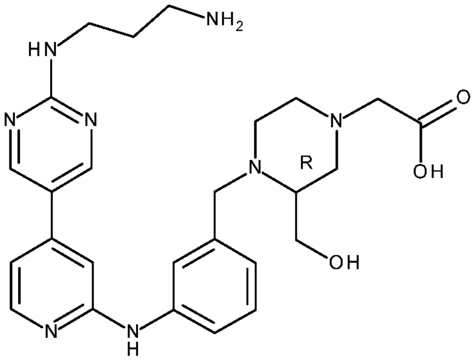 TFA salt<br>Int. 46 (starting from Int. 8 and Int. 70; used for Co. 8) | 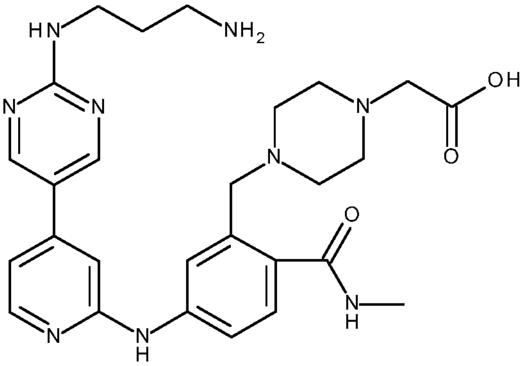 TFA salt<br>Int. 47 (starting from Int. 8 and Int. 22; used for Co. 9) |
| 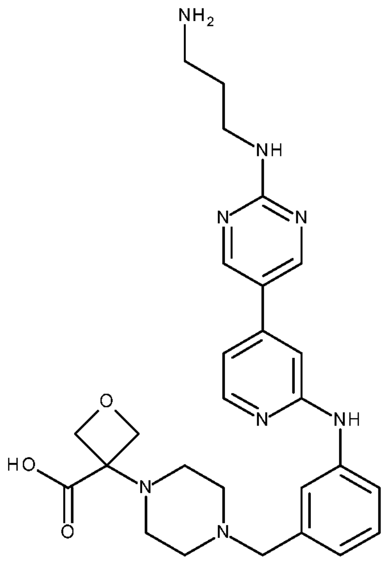 TFA salt<br>Int. 48 (starting from Int. 8 and Int. 28; used for Co. 10) | 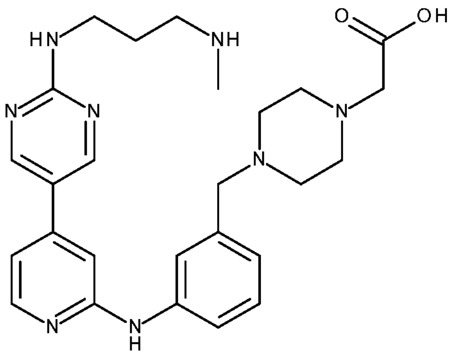 HCl salt<br>Int.49 (starting fromInt.9 andInt.41;used forCo. 13) |
| 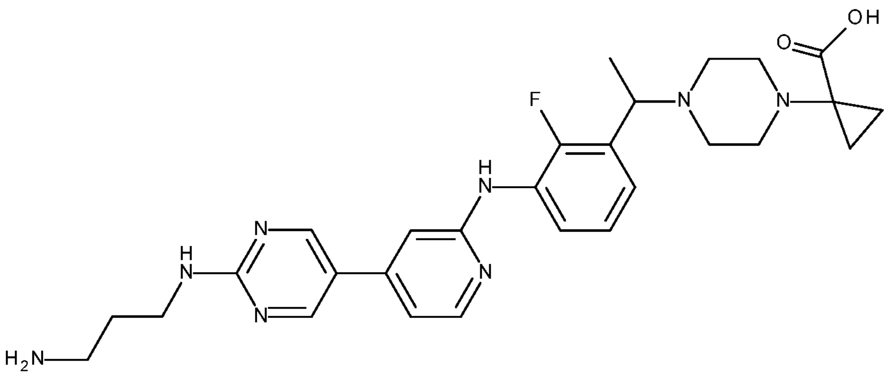 HCl salt<br>Int. 50 (starting from Int. 8 and Int. 31; used for Co. 32) | |

(continued)

| | |
|---|---|
| 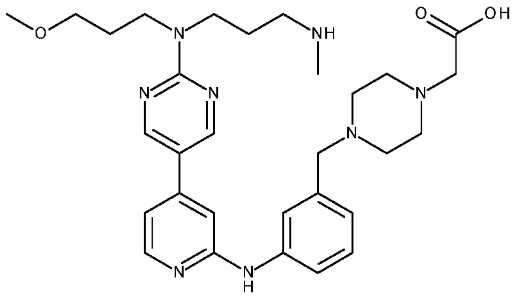<br>HCl salt<br>Int. 51 (starting from Int. 10 and Int. 41; used for Co. 14) | 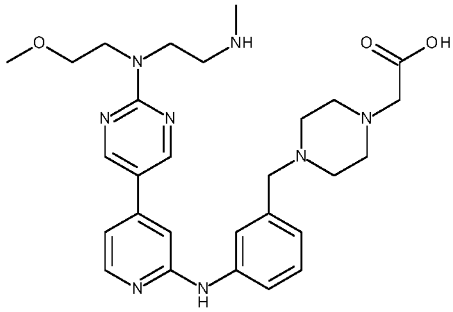<br>HCl salt<br>Int. 52 (starting from Int. 11 and Int. 41; used for Co. 15) |
| 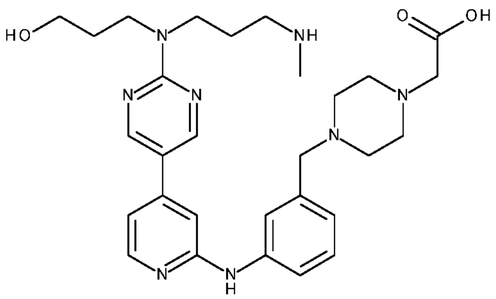<br>HCl salt<br>Int. 54 (starting from Int. 12 and Int. 41; used for Co. 17) | 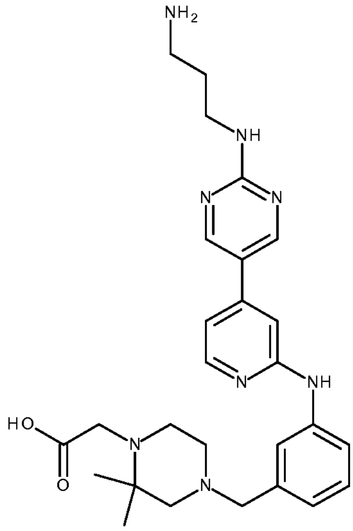<br>TFA salt<br>Int. 55 (starting from Int. 8 and Int. 34; used for Co. 18) |
| 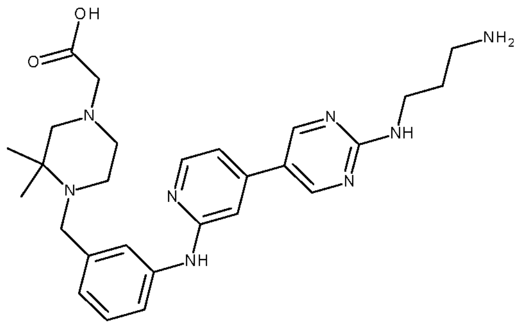<br>TFA salt<br>Int. 56 (starting from Int. 8 and Int. 37; used for Co. 19) | 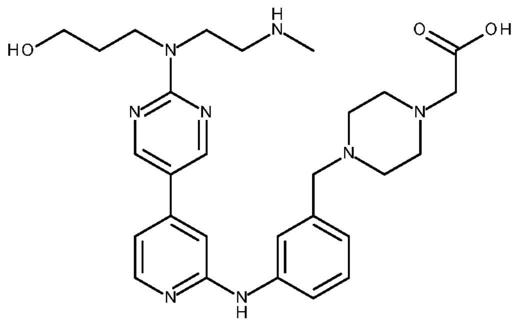<br>HCl salt<br>Int. 53 (starting from Int. 13 and Int. 41; used for Co. 16) |

(continued)

| | |
|---|---|
| 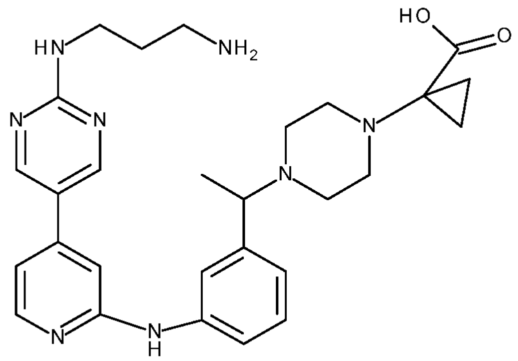<br>HCl salt<br>Int. 81 (starting from Int. 8 and Int. 61; used for Co. 21) | 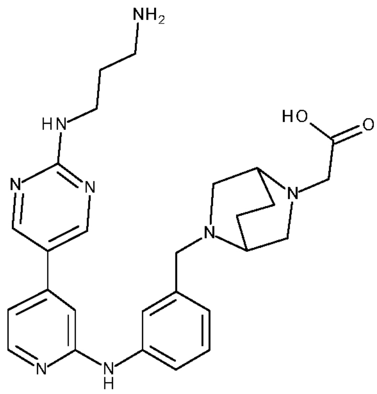<br>TFA salt<br>Int. 82 (starting from Int. 8 and Int. 72; used for Co. 24) |
| 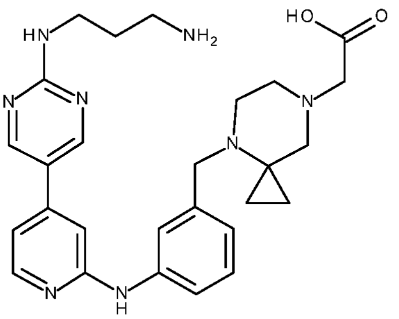<br>TFA salt<br>Int. 83 (starting from Int. 8 and Int. 73; used for Co. 27) | 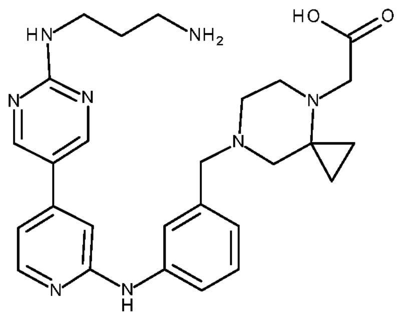<br>TFA<br>salt<br>Int. 84 (starting from Int. 8 and Int. 74; used for Co. 28) |
| 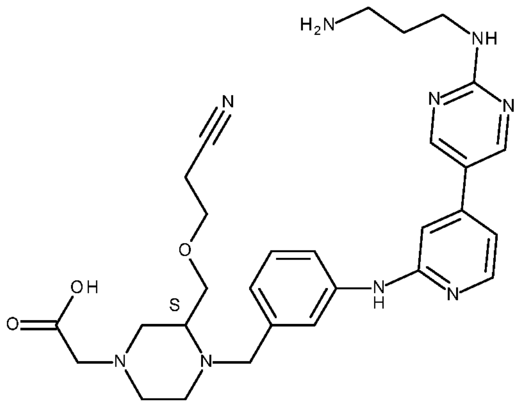<br>TFA salt<br>Int. 85 (starting from Int. 8 and Int. 79; used for Co. 29) | |

(continued)

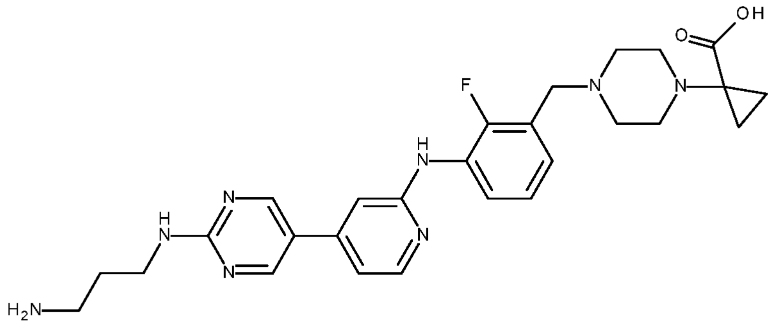

HCl salt

Int. 80 (starting from Int. 59 and Int. 8; used for Co. 20)

Example A4-a

a) Preparation of Int. 57

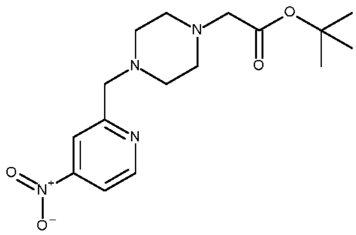

2-Chloromethyl-4-nitropyridine (2.75 g; 15.936 mmol) was added to the mixture of 1-piperazineacetic acid, 1,1-dimethylethyl ester (3.2 g; 15.978 mmol) and $K_2CO_3$(4.4 g; 31.837 mmol) in ACN (50 ml). The resulting mixture was stirred at r.t. for 16 h. The precipitate was filtered off and the filtrate was evaporated in vacuum. The crude product was purified by column chromatography (eluent: PE/EtOAc 3/1). The desired fractions were collected and the solvent was evaporated to give 4 g of Int. 57 (74 %).

b) Preparation of Int. 58

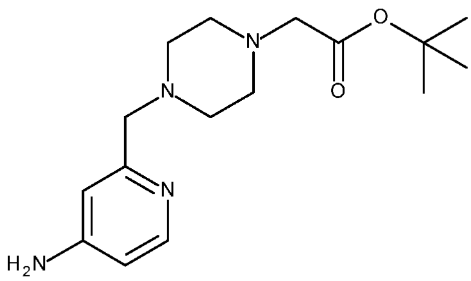

Int. 57 (4 g; 11.778 mmol) was dissolved in THF (60 ml), MeOH (30 ml) and water(15 ml).
Iron (6.577 g; 117.78 mmol) and $NH_4Cl$ (6.3 g; 117.78 mmol) were added. The mixture was refluxed for 1 h. EtOAc was added and the mixture was filtered. The filtrate was concentrated. Water was added and the mixture was basified with 10 % $NaHCO_3$ aqueous solution to pH > 7. The mixture was extracted with DCM and MeOH (DCM/MeOH 5/1).
The organic layer was separated, washed with brine, dried over $Na_2SO_4$ and evaporated to give 3 g of Int. 58 (76 %).
The intermediates in the table below were prepared by first using an analogous reaction protocol as used for

Int. 57, followed by an analogous reaction protocol as used for Int. 58.

| | |
|---|---|
| 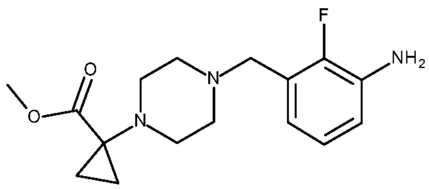 Int. 59 (starting from 1-(chloromethyl)-2-fluoro-3-nitro-benzene and 1-(1-piperazinyl)-cyclopropanecarboxylic acid, methyl ester) | 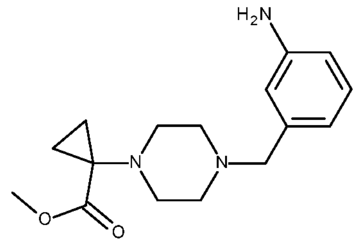 Int. 60 (starting from 3-nitro benzyl bromide and 1-(1-piperazinyl)-cyclopropanecarboxylic acid, methyl ester) |
| 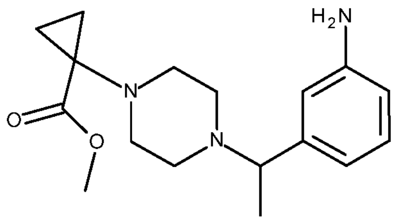 Int. 61 (starting from 1-(1-chloro ethyl)-3-nitro-benzene and 1-(1-piperazinyl)-cyclopropanecarboxylic acid, methyl ester) | |

Example A5a

a) Preparation of Int. 92

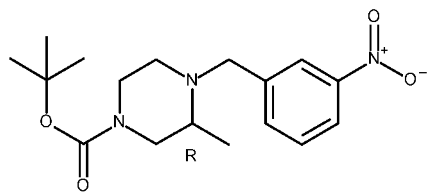

To a solution of (3R)-3-methyl-1-piperazinecarboxylic acid, 1,1-dimethylethyl ester (10 g ; 49.93 mmol), 3-nitrobenzyl bromide (10.79 g; 49.93 mmol) in ACN(200 ml) was added $K_2CO_3$ (13.8 g; 99.86 mmol) and the reaction mixture was stirred overnight. Water was added and the aqueous layer was extracted with EtOAc. The organic layer was separated, dried ($MgSO_4$), filtered and the solvent was evaporated. Yield: 18 g of Int. 92 (100 %).

b) Preparation of Int. 93

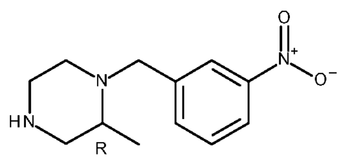

HCl salt

Int. 92 (18 g; 53.67 mmol) was dissolved in DCM(150 ml). HCl in dioxane(60 ml) was added. The solution was stirred overnight, and the solvent was evaporated. The crude residue (15 g) (containing Int. 93) was used as such in the next reaction step.

c) Preparation of Int. 94

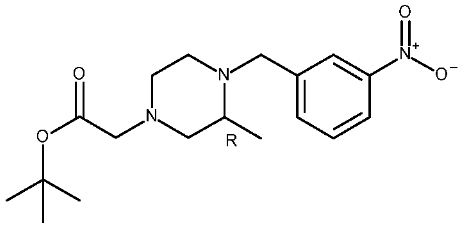

To a solution of Int. 93 (15 g) and 2-bromo-acetic acid, 1,1-dimethylethyl ester (11.31 g; 57.96 mmol) in DCM (200 ml) was added DIPEA (21.4g; 165.6 mmol) and the reaction mixture was stirred overnight. Water was added and the aqueous layer was extracted with EtOAc. The organic layer was separated, dried ($MgSO_4$), filtered and the solvent was evaporated. The crude product was purified by column chromatography over silica gel (PE / EtOAc 2/1). The desired fractions were collected and the solvent was evaporated. Yield: 14 g of Int. 94.

d) Preparation of Int. 95

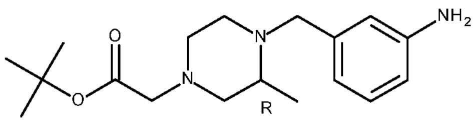

A mixture of Int. 94 (14 g; 40.07 mmol) with Pt/C (0.9 g) as a catalyst in MeOH(140 ml) was hydrogenated under a 40 psi pressure of $H_2$ gas for 5 h. The catalyst was filtered off on a Celite® pad which was washed several times with MeOH . The combined filtrates were evaporated to dryness. Yield: 12 g of Int. 95 (94 %).

The intermediates in the table below were prepared by using successively analogous reaction protocols as used for Int. 92, Int. 93, Int. 94 and Int. 95.

| |
|---|
| 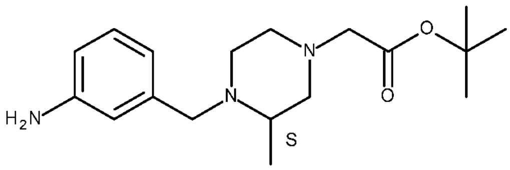 |
| Int. 96 (starting from (3S)-3-methyl-1-piperazinecarboxylic acid, 1,1-dimethylethyl ester, and 3-nitrobenzyl bromide) |

Example A5b

a) Preparation of Int. 97

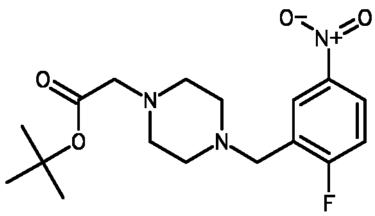

Piperazine-1-acetic acid tert-butyl ester (25.67 g, 128mmol) was added to a suspension of 3-bromomethyl-4-fluoronitrobenzene (Journal of Medicinal Chemistry (1994), 37(9), 1362-70) (30 g, 128 mmol) and $K_2CO_3$ (35.3 g, 256 mmol) in ACN (400 ml). The mixture was stirred at r.t. for 2 h and was then filtered.The filtrate was evaporated *in vacuo.* The residue was purified by chromatography on silica gel (PE/EtOAc 8/1 to pure EtOAc). The desired fractions were collected and the solvent was evaporated. Yield: 28 g of Int. 97 (62 % yield).

b) Preparation of Int. 98

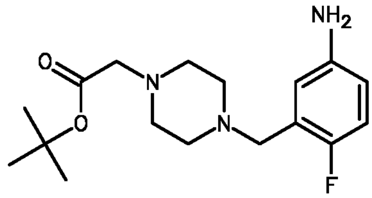

Int. 97 (28 g, 79.2 mmol) was dissolved in a mixture of THF (40 ml), $H_2O$ (40 ml) and MeOH (80 ml). Fe (44.2 g, 792 mmol) and $NH_4Cl$ (42.3 g,792 mmol) were added. The mixture was refluxed for 2 h. After cooling, the mixture was filtered. Brine and DCM were added to the filtrate. The organic layer was separated, dried over $Na_2SO_4$ and evaporated to dryness. Yield: 24.3 g of Int. 98 (95 %).

Example A5c

a) Preparation of Int. 99

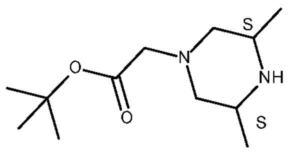

(2S,6S)-2,6-dimethyl-piperazine (1.142 g; 10 mmol) was stirred in THF (q.s.). First DIPEA (5.17 ml; 30 mmol) and then tert-butyl bromoacetate (1.624 ml; 11 mmol) was added. The reaction mixture was stirred overnight at r.t.. The reaction mixture was heated at 50 °C for 2 h. The reaction mixture was evaporated, dissolved in DCM and the organic layer was washed with a $NaHCO_3$-solution, dried $MgSO_4$ and evaporated. The residue was purified on silicagel (eluent: gradient DCM 100% to 90 % / MeOH-$NH_3$ 0% to 10%). The pure fractions were collected and evaporated. Yield: 0.56 g of Int. 99 (24.5 %).

b) Preparation of Int. 100

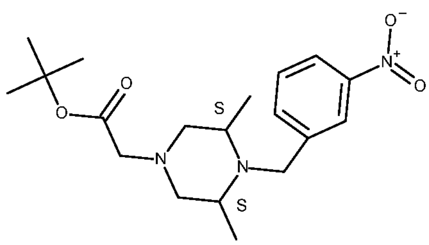

A mixture of Int. 99 (0.56 g; 0.00245 mol), 3-nitrobenzyl bromide (0.583g; 2.698 mmol) and DIPEA (1.268 ml; 7.358 mmol) in DMF (23.738 ml) was stirred at 75 °C for 18 h. The reaction mixture was evaporated. A $NaHCO_3$-solution in water was added to the residue. The product was extracted twice with DCM. The organic layer was washed with water, dried with $MgSO_4$, filtered and the solvents of the filtrate were evaporated. The residue was purified by column

chromatpgraphy on silicagel (eluent: gradient DCM 100% to 90 % / MeOH-$NH_3$ 0% to 10%). The pure fraction was collected and evaporated. The residue was dissolved in heptanes and filtered. The filtrate was evaporated. Yield: 0.59 g of Int. 100 (66 %).

c) Preparation of Int. 101

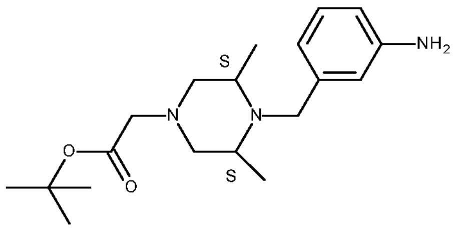

Int. 100 (0.59 g; 1.266 mmol) with Pt/C 5 % (0.2 g) as a catalyst was hydrogenated at r.t. in THF (25.762 ml) under hydrogen atmosphere until 3 eq. hydrogen were absorbed. The catalyst was removed by filtration over Dicalite®. The filtrate was evaporated and the residue was purified by column chromatography over silicagel (eluent: gradient DCM 100% to 90 % / MeOH-$NH_3$ 0% to 10%). The pure fractions were collected and evaporated. Yield: 0.4 g of Int. 101 (94.7 %).

The intermediates in the table below were prepared by using successively analogous reaction protocols as used for Int. 100 and Int. 101 (for Int. 102), and Int. 99, Int. 100 and Int. 101 (for Int. 103).

| | |
|---|---|
| 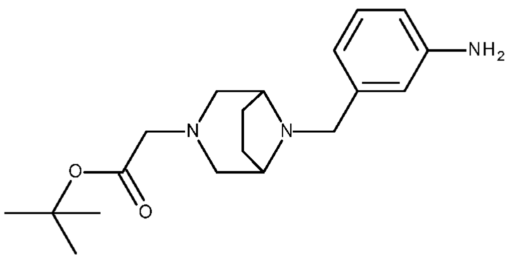<br>Int. 102 (starting from 3,8-diazabicyclo[3.2.1]octane-3-acetic acid, 1,1-dimethylethyl ester, and 3-nitrobenzyl bromide) | 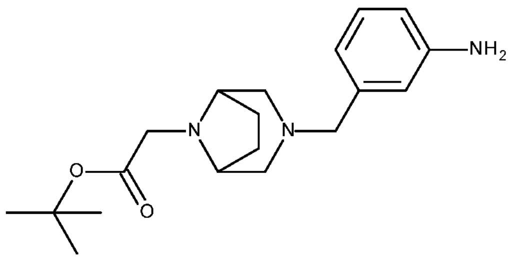<br>Int. 103 (starting from 3-(phenylmethyl)-3,8-diazabicyclo[3.2.1]octane, and tert-butyl bromoacetate) |

Example A5d

a) Preparation of Int. 104

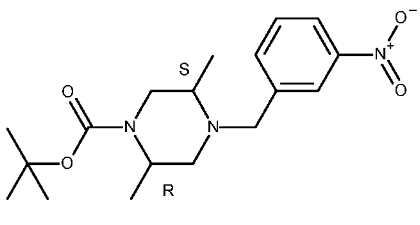

To a mixture of (2R,5S)-2,5-dimethyl-1-piperazinecarboxylic acid, 1,1-dimethylethyl ester (1 g; 3.99 mmol) in DMF (30.877 ml) was added DIPEA (2.749 ml; 15.951 mmol) and the mixture was stirred for 5 min. 3-Nitrobenzyl bromide (0.948 g; 4.387 mmol) was added. The reaction mixture was stirred at 75 °C for 18 h. The reaction mixture was evaporated, dissolved in DCM and a $NaHCO_3$ solution in water was added. The organic layer was separated, washed with water, dried with $MgSO_4$, filtered and evaporated.

The residue was purified by column chromatography on silicagel (eluent: gradient DCM 100% to 90% / MeOH-

$NH_3$ 0% to 10%). The pure fractions were collected and evaporated. Yield: 1.22 g of Int. 104 (87.5 %).

b) Preparation of Int. 105

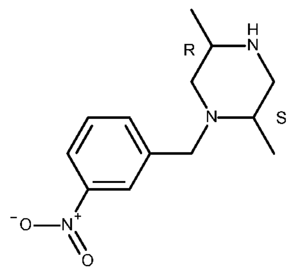

HCl salt

Int. 104 (1.22g; 0.00349 mol) was dissolved in iPrOH (80 ml). HCl (6 M in iPrOH) (8.728 ml) was added. The reaction mixture was stirred for 1.5 h at reflux temperature and was evaporated. Yield: 0.87 g of Int. 105.

c) Preparation of Int. 106

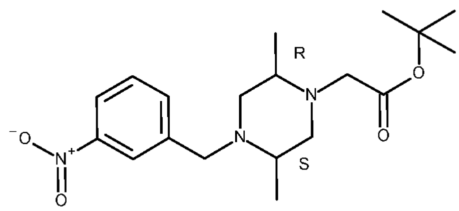

Int. 105 (0.75 g) was suspended in ACN(47.136 ml). DIPEA (1.037 ml; 0.00602 mol) was added, and tert-butyl bromoacetate (0.645g; 0.00331 mol) was added dropwise. The reaction mixture was stirred for 5 h at r.t. and was then evaporated. The residue was dissolved in DCM and a $NaHCO_3$ solution in water. The organic layer was separated, washed with water, dried $MgSO_4$ and evaporated. Yield: 1.22 g of Int. 106.

d) Preparation of Int. 107

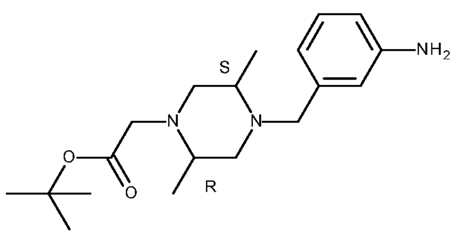

Int. 106 (1.22g; 2.618 mmol) with Pt/C 5 % (0.2 g) as a catalyst was hydrogenated at r.t. in THF (53.27 ml) under hydrogen atmosphere until 3 eq. hydrogen were absorbed. The catalyst was removed by filtration over Dicalite®. The filtrate was evaporated and the residue was purified by column chromatography on silicagel (eluent:gradient DCM 100% to 85% / MeOH-$NH_3$ 0% to 15%). The pure fractions were collected and evaporated. Yield: 0.88 g of Int. 107 (100 %).

Example A5e

a) Preparation of Int. 108

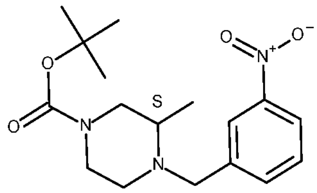

(3*S*)-3-Methyl-1-piperazinecarboxylic acid, 1,1-dimethylethyl ester (17.13 g; 85.6 mmol) was dissolved in ACN (150 ml). 3-Nitrobenzyl bromide (15.57 g; 77 mmol) and $K_2CO_3$(13.6 g; 171.3 mmol) were added. The mixture was stirred at r.t. overnight. The mixture was filtered. The filtrate was concentrated. Yield: 28.2 g of Int. 108.

b) Preparation of Int. 109

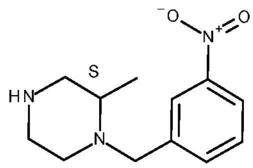

HCl salt

Int. 108 (28.2g ; 84 mmol) was dissolved in HCl/EtOAc (200 ml). The mixture was stirred at r.t. for 1 h. The precipitate was filtered and dried *in vacuo.* Yield: 11 g of Int. 109.

c) Preparation of Int. 110

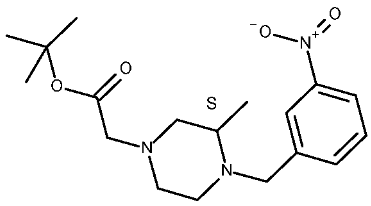

Int. 109 (0.73 g) was dissolved in DCM (20 ml). Tert-butyl bromoacetate (1.17 g; 3.7 mmol) and $Et_3N$ (1.2 g; 9.3 mmol) were added. The mixture was stirred at r.t. for 3 h. The mixture was washed with water and brine, dried over $Na_2SO_4$, filtered and concentrated to give 2.4 g of crude Int. 110 which was used as such in the next reaction step.

d) Preparation of Int. 111

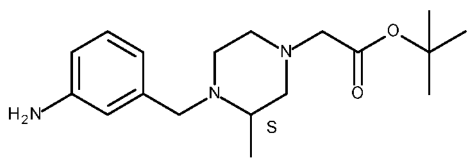

Int. 110 (2.4 g) was dissolved in MeOH(10 ml). Pt/C (0.2 g) was added. The mixture was hydrogenated at 40 °C under hydrogen gas atmosphere (40 psi). The catalyst was filtered. The filtrate was concentrated. Yield: 0.2 g of Int. 111.

Example A6

a) Preparation of Int. 87

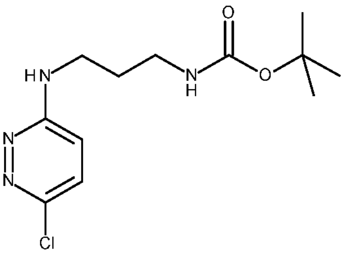

The mixture of 3,6-dichloro pyridazine (3.0 g; 20 mmol) and N-(3-aminopropyl)carbamic acid tert-butyl ester (7.0 g; 40 mmol) in MeOH (30 ml) was heated at reflux overnight. The mixture was evaporated in vacuum to give the crude intermediate. This crude intermediate was purified by column chromatography over silica gel (eluent: DCM/MeOH 10/1). The desired fractions were collected and the solvent was evaporated. Yield: 4.5 g of Int. 87 (79 %).

b) Preparation of Int. 88

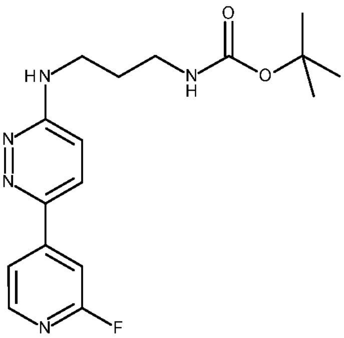

A mixture of Int. 87 (2.55 g; 8.89 mmol), 2-fluoro pyridine 4-boronic acid (1.87 g; 13.3 mmol), $Pd(PPh_3)_4$ (0.2 g; 0.18 mmol) and 2 M $Na_2CO_3$ (18 ml; 36 mmol) in dioxane (60 ml) was heated to reflux for 12 h. Then 100 ml of $H_2O$ was added and the mixture was extracted with EtOAc. The organic layer was separated, dried over $MgSO_4$, filtered and evaporated. The residue was purified by column chromatography on silica gel (PE/EtOAc 5/1). The desired fractions were collected and the solvent was evaporated. Yield: 2 g of Int. 88 (65 %).

The intermediates in the table below were prepared by first using an analogous reaction protocol as used for Int. 87, followed by an analogous reaction protocol as used for Int. 88.

| | |
|---|---|
| 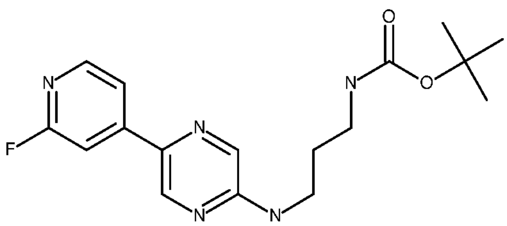 Int. 89 (starting from 2,5-dichloro-pyrazine and N-(3-aminopropyl)carbamic acid tert-butyl ester) | |

Example A7

a) Preparation of Int. 90

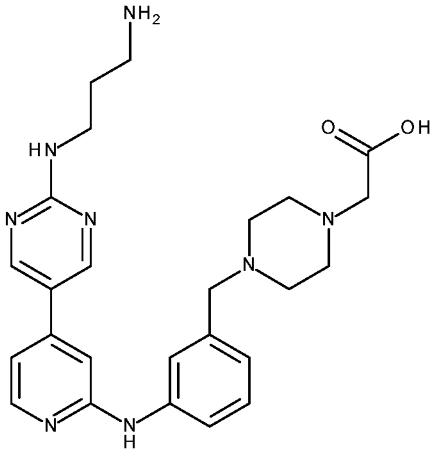

HCl salt

A mixture of Int. 8 (1.41 g; 3.604 mmol) and Int. 41 (1.79 g; 5.406 mmol) in n-butanol (11 ml) and HCl (6 M in iPrOH) (6.007 ml) was stirred and heated at 140 °C for 3 h using microwave irradiation. The solvents were evaporated. Yield: 3.17 g of Int. 90 which was used as such in the next reaction step.

The intermediates in the table below were prepared by using an analogous reaction protocol as used for Int. 90.

| | |
|---|---|
| 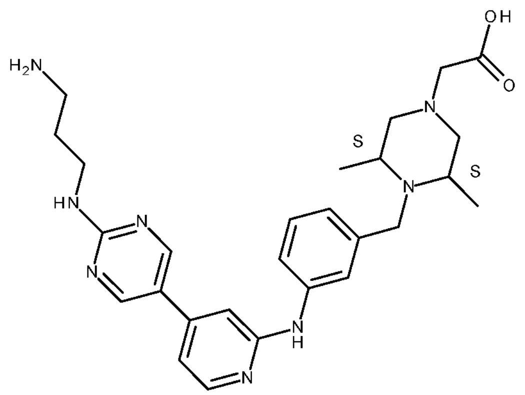 HCl salt | 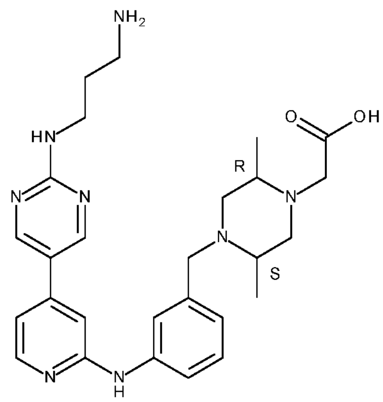 HCl salt |
| Int. 91 (starting from Int. 8 and Int. 101; used for Co. 34) | Int. 112 (starting from Int. 8 and Int. 107; used for Co. 35) |

(continued)

| | |
|---|---|
| 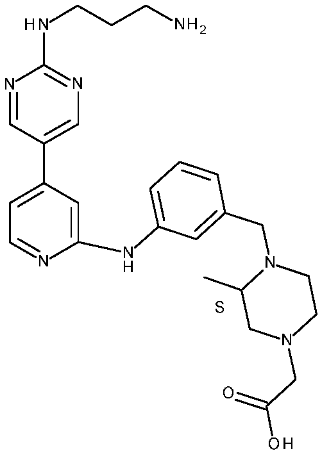<br>Int. 113 (starting from Int. 8 and Int. 96; used for Co. 36) | 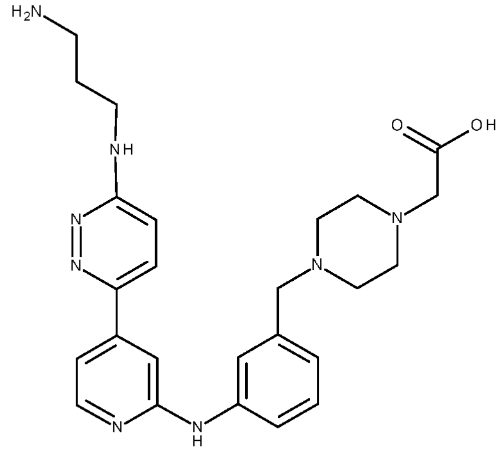<br>TFA salt<br>Int. 114 (starting from Int. 88 and Int. 41; used for Co. 37) |
| 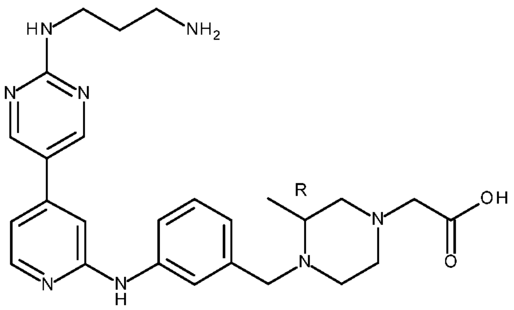<br>TFA salt<br>Int. 115 (starting from Int. 8 and Int. 95; used for Co. 40) | 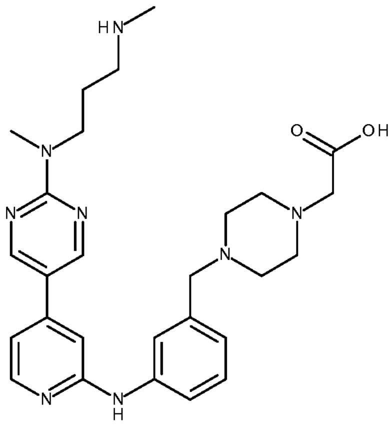<br>HCl salt<br>Int. 116 (starting from Int. 86 and Int. 41; used for Co. 38) |
| 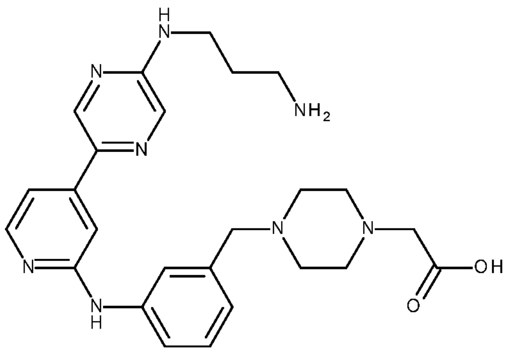<br>HCl salt<br>Int. 117 (starting from Int. 89 and Int. 41; used for Co. 39) | 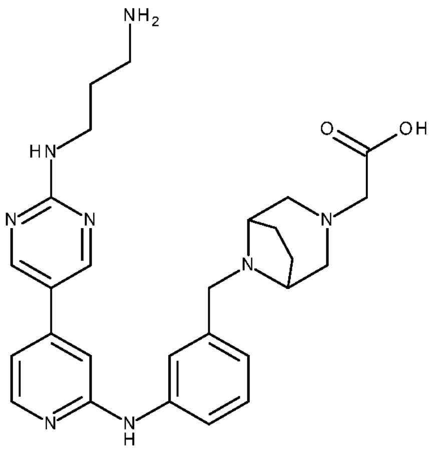<br>HCl salt<br>Int. 118 (starting from Int. 8 and Int. 102; used for Co. 41) |

(continued)

| | |
|---|---|
| 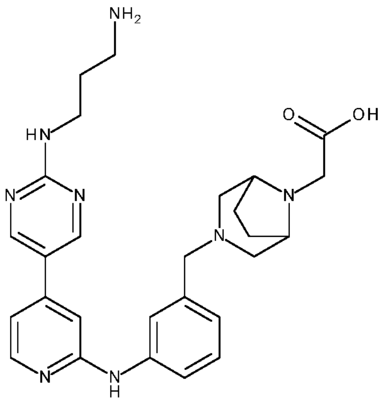<br>HCl salt<br>Int. 119 (starting from Int. 8 and Int. 103; used for Co. 42) | |

Example A8a

a) Preparation of Int. 120

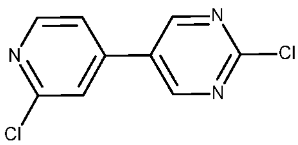

A solution of (2-chloropyrimidin-5-yl)boronic acid (31.6 g; 200 mmol), 2-chloro 4-bromo pyridine (40.4 g; 209 mmol) and $Na_2CO_3$(42.4 g) in dioxane(1000 ml) was degassed with $N_2$ for 30 min. $Pd(PPh_3)_4$ was added and the mixture was refluxed overnight. The mixture was filtered over Celite® and poured into water. The precipitate was filtered and washed with tert-butyl methyl ether. The residue was purified by column chromatography (eluent: PE / EtOAc 2/1). The desired fractions were collected and the solvent was evaporated. Yield: 12 g of Int. 120 (26.7 %).

b) Preparation of Int. 121

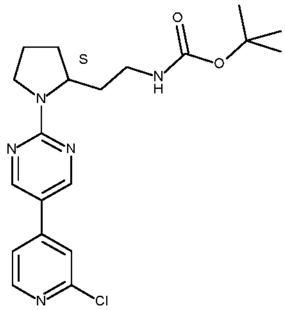

To a solution of [2-(2S)-2-pyrrolidinylethyl]-carbamic acid, 1,1-dimethylethyl ester (1.90 g; 8.85 mmol) in ACN (80 ml) was added Int. 120 (2 g; 8.85 mmol) and DIPEA(10 ml) at r.t.. The mixture was stirred overnight. The solvent was removed. The crude product was purified by column chromatography over silica gel (PE/EtOAc 4/1). The desired fractions were collected and the solvent was evaporated. Yield: 1.40 g of Int. 121 (39 %).

Int. 121b

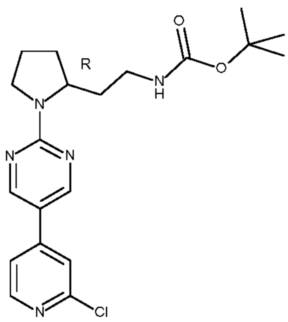

was prepared according to an analogous reaction protocol, but [2-(2R)-2-pyrrolidinylethyl]-carbamic acid, 1,1-dimethylethyl ester was used as starting material.

Example A8b

a-1) Preparation of Int. 122

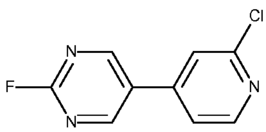

The mixture of Int. 120 (3 g; 13.271 mmol), KF (2.503g; 43.084 mmol) and 18-crown-6 (350.747 mg; 1.327 mmol) in ACN (40 ml) was stirred at 40 °C overnight under $N_2$ atmosphere. The mixture was poured into water and extracted with DCM. The organic layer was dried and concentrated *in vacuo.* The residue was purified by column over silica gel (eluent: PE/EtOAc 4/1). The product fractions were collected and the solvent was evaporated to give the product. Yield: 2.8 g of Int. 122 (90 %).

a-2) Preparation of Int. 123

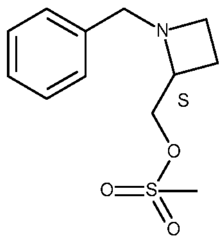

A mixture of (2S)-1-(phenylmethyl)-2-azetidinemethanol (1.77 g; 10 mmol), mesylchloride (1374.612 mg; 12 mmol) and $Et_3N$ (2.168 ml; 15 mmol) in DCM (60 ml) was stirred at r.t. for 10 h. The solution was washed with aq. $NaHCO_3$, water and the organic layer was separated. The organic layer was dried over $MgSO_4$, filtered and evaporated to give 2.55 g of Int. 123 as a sticky oil.

b) Preparation of Int. 124

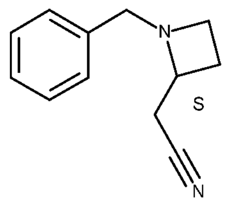

A mixture of Int. 123 (2553.33 mg; 10 mmol), NaCN (1470.3 g; 30 mmol) and KI (0.1 mg) in DMSO (10 ml) was heated to 50 °C for 10 h. The reaction mixture was poured into water and extracted with EtOAc. The organic layer was dried over $MgSO_4$, filtered and evaporated. The residue was purified by column chromatography over silica gel (eluent: EtOAc/PE 4/1).The desired fractions were collected and the solvent was removed to give a sticky oil.
Yield: 700 mg of Int. 124 (37.6 %).

c) Preparation of Int. 125

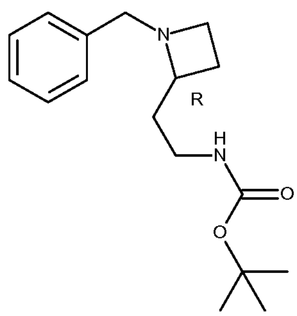

To a solution of Int. 124 (800 mg; 4.3 mmol), dicarbonic acid, *C*,*C*'-bis(1,1-dimethylethyl) ester (1876.924 mg; 8.6 mmol) and NiCl.6$H_2$O (868.621 mg; 4.295 mmol) in MeOH (30 ml) was added $NaBH_4$ (816.694 mg; 21.475 mmol) at 0 °C in portions. Stirring was continued for 30 minutes, and then the solvent was removed and the residue was poured into water and extracted with EtOAc. The organic layer was dried over $MgSO_4$, filtered and evaporated to give the crude product which was further purified by column chromatography over silica gel (eluent: hexane/EtOAc 10/1).

The desired fraction was collected and evaporated to give 300 mg of the desired product as an oil (24 %).

d) Preparation of Int. 126

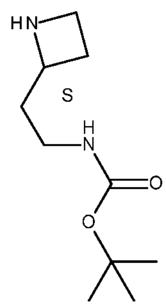

A mixture of Int. 125 (300 mg; 1 mmol) in MeOH (20 ml) was hydrogenated at r.t. under atmospheric pressure of $H_2$ gas with Pd/C (0.3 g) as a catalyst. After uptake of $H_2$ (1 eq.), the catalyst was filtered off and the filtrate was evaporated. Yield: 200 mg of Int. 126 (99 %).

e) Preparation of Int. 127

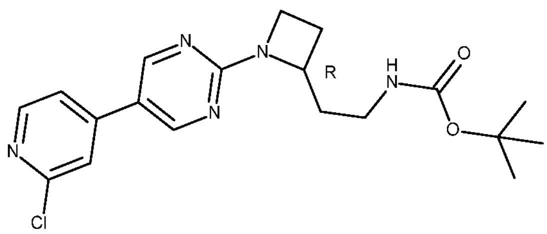

A solution of Int. 126 (200.278 mg;1 mmol), Int. 122 (209.607 mg; 1 mmol) and $Et_3N$ (151.785 mg; 1.5 mmol) in THF (5 ml) was stirred overnight. The solvent was removed and the residue was purified by column chromatography over silica gel (eluent: PE/EtOAc 4/1). The desired fractions were collected and the solvent was removed to give the desired product.
Yield: 310 mg of Int. 127 (65 %).

Example A9a

a) Preparation of Int. 136

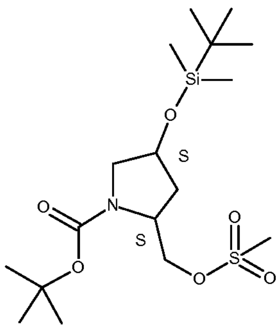

(2*S*,4*S*)-4-[[(1,1-dimethylethyl)dimethylsilyl]oxy]-2-(hydroxymethyl)-1-pyrrolidinecarboxylic acid, 1,1-dimethylethyl ester (134 g; 404.195 mmol) was dissolved in pyridine (330 ml). Mesylchloride (61.9 g; 540.371 mmol) was added at 0 °C. The reaction mixtures was stirred for 2 h at r.t.. Then the reaction mixture was evaporated under reduced pressure. The residue was dissolved in EtOAc and the organic layer was washed with a 10 % $NaHCO_3$ solution. The organic layer was dried over anhydrous sodium sulfate and concentrated to give the crude product which was used as such in the next reaction step without purification. Yield: 146.0 g of Int. 136.

b) Preparation of Int. 137

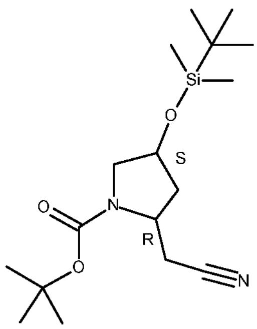

NaCN (73.5 g; 1499.78 mmol) was added to a solution of Int. 136 (163 g; 397.937 mmol) in DMSO (300 ml) and the reaction mixture was heated to 60 °C for 6 h. After completion of the reaction the mixture was dissolved in EtOAc.

The organic layer was washed with water and brine. The organic layer was concentrated under reduced pressure. The crude intermediate was purified by column chromatography over silica gel (eluent: PE/EtOAc 20/1). The desired fractions were collected and the solvent was evaporated. Yield: 108.0 g of Int. 137.

c) Preparation of Int. 138

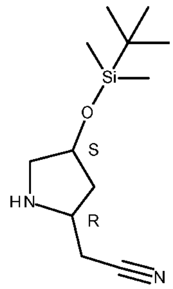

A mixture of Int. 137 (40 g; 117.463 mmol) and TFA (133.935 g; 1174.63 mmol) in DCM (400 ml) was stirred overnight at r.t.. The mixture was treated with saturated $NaHCO_3$, and extracted with DCM. The organic phase was washed with brine, dried over $Na_2SO_4$, filtered and evaporated in vacuum to give the crude intermediate which was purified by column chromatography over silica gel (eluent: PE/EtOAc 2/1). The desired fractions were collected and the solvent was evaporated. Yield: 21.0 g of Int. 138 (70.6 %).

The intermediates in the table below were prepared by using successively analogous reaction protocols as used for Int. 136, Int. 137, and Int. 138.

| |
|---|
| 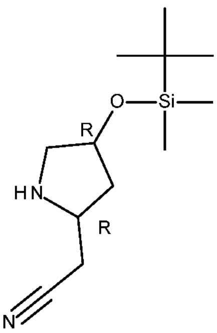<br>Int. 139 (starting from (2S,4R)-4-[[(1,1-dimethylethyl)dimethylsilyl]oxy]-2-(hydroxymethyl)-1-pyrrolidinecarboxylic acid, 1,1-dimethylethyl ester) |

d) Preparation of Int. 140

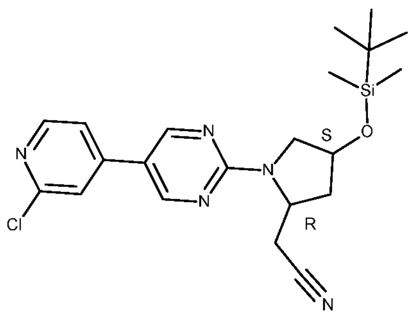

The mixture of Int. 120 (11.754g; 51.993 mmol), Int. 138 (21g; 87.348 mmol) and DIPEA (33.599 g; 259.965

mmol) in DMF(150 ml) was stirred at 90 °C for 2 h. To the mixture was added water and the water layer was extracted with EtOAc. The organic layer was washed by water, brine, dried over $Na_2SO_4$, filtered, and evaporated in vacuum to give the crude intermediate which was purified by column chromatography over silica gel (eluent: PE/EtOAc 1/1). The desired fractions were collected and the solvent was evaporated. Yield: 12.0 g of Int. 140 (52.6 %).

Int. 142

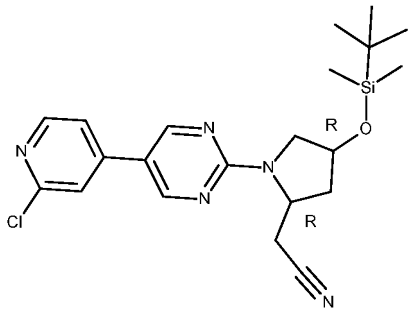

was prepared according to an analogous reaction protocol, but Int. 120 and Int. 139 were used as starting materials.

e) Preparation of Int. 141

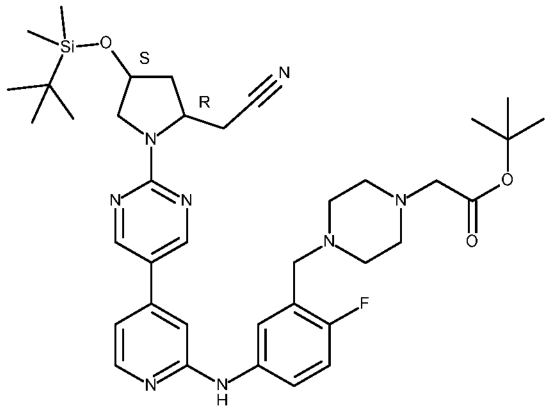

Under $N_2$ atmosphere $Pd_2(dba)_3$ (192mg; 0.21 mmol) was added to the mixture of Int. 140 (1 g; 2.093 mmol), Int. 98 (0.787g; 2.093 mmol), X-Phos (400 mg; 0.839 mmol) and $K_2CO_3$ (580 mg; 4.197 mmol) in tBuOH (30 ml) and the mixture was refluxed overnight. The precipitate was filtered off. The filtrate was concentrated in vacuum to give the cude product. The crude product was purified by column (eluent: PE/EtOAc 1/4). The desired fractions were collected and the solvent was evaporated. Yield: 1.05 g of Int. 141 (50.45 %; solid).

f) Preparation of Int. 143

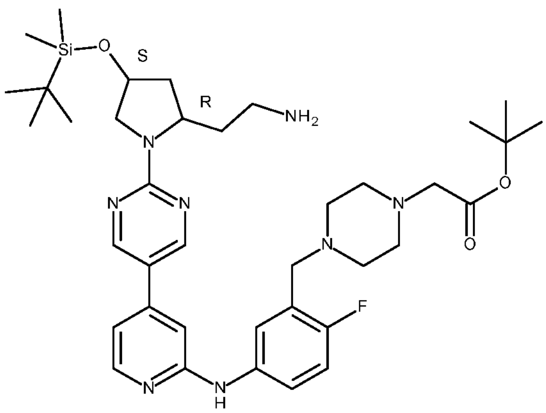

A solution of Int. 141 (1.05 g; 1.336 mmol) in MeOH (30 ml) was hydrogenated at 85 °C (atmospheric pressure) with Raney Ni (1 g) as a catalyst in the presence of $NH_4OH$ (6 ml). After consumption of $H_2$ (2 eq.), the catalyst was filtered off and the filtrate was evaporated to give 830 mg of Int. 143.

g) Preparation of Int. 144

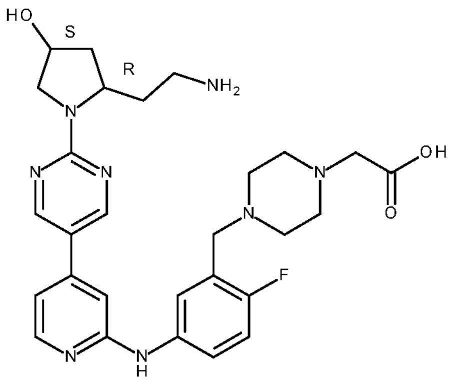

HCl salt

Int. 143 (800 mg; 1.037 mmol) was treated with 4 N HCl in dioxane (20 ml). The mixture was stirred overnight at r.t.. The mixture was evaporated in vacuum to give 610 mg of Int. 144.

The intermediates in the table below were prepared by using successively analogous reaction protocols as used for Int. 141, Int. 143, and Int. 144.

| | |
|---|---|
| 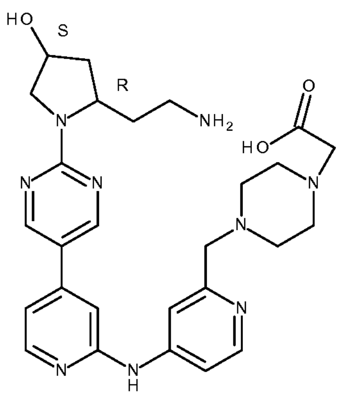 HCl salt | 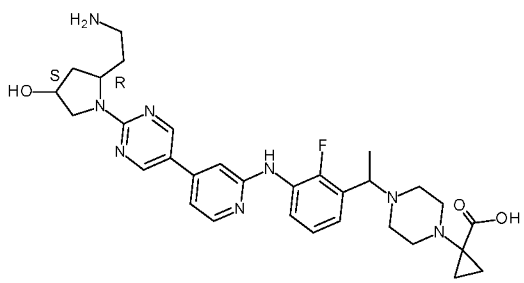 HCl salt |
| Int. 145 (starting from Int. 58 and Int. 140; used for Co. 51) | Int. 146 (starting from Int. 31 and Int. 140; used for Co. 52) |

(continued)

| | |
|---|---|
| 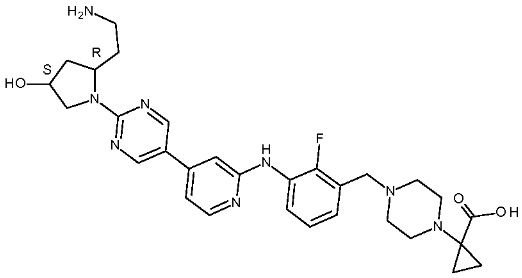 HCl salt<br>Int. 147 (starting from Int. 59 and Int. 140; used for Co.53) | 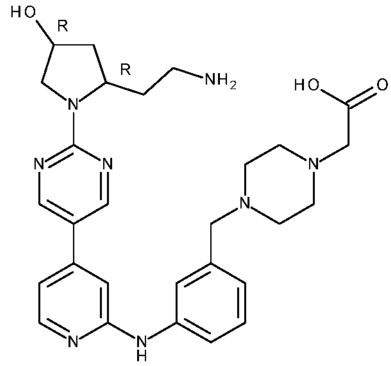 TFA salt<br>Int. 148 (starting from Int. 41 and Int. 142; used for Co. 54) |
| 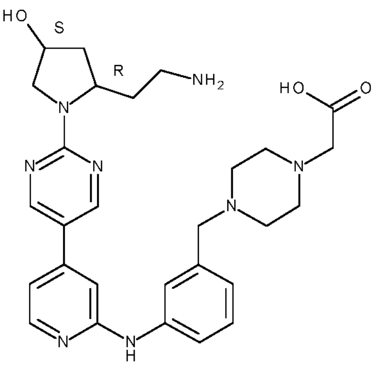 TFA salt<br>Int. 149 (starting from Int. 41 and Int. 140; used for Co. 55) | 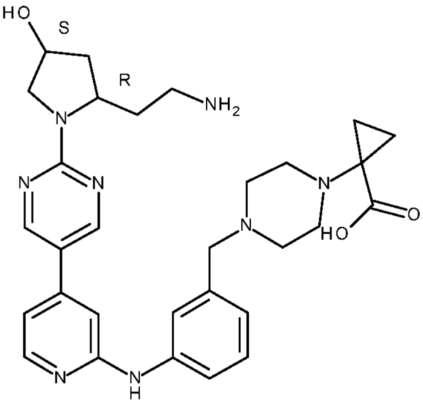 HCl salt<br>Int. 150 (starting from Int. 60 and Int. 140; used for Co. 56) |
| 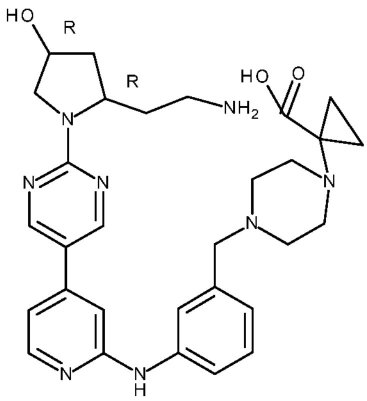 HCl salt<br>Int. 151 (starting from Int. 60 and Int. 142; used for Co. 57) | 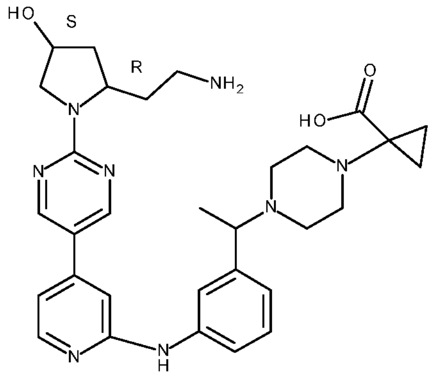 HCl salt<br>Int. 152 (starting from Int. 61 and Int. 140; used for Co. 58) |

(continued)

| | |
|---|---|
| 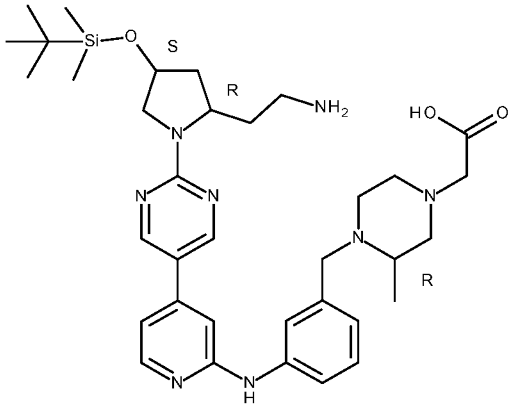<br>TFA salt<br>Int. 153 (starting from Int. 95 and Int. 140; used for Co. 59) | 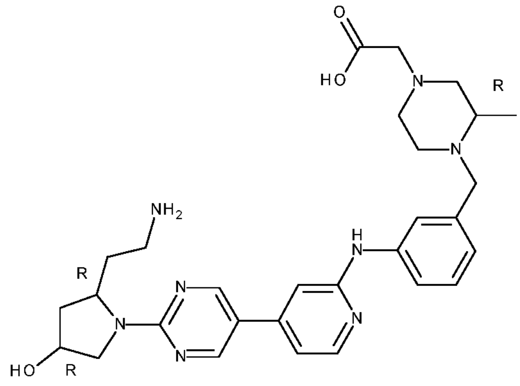<br>TFA salt<br>Int. 154 (starting from Int. 95 and Int. 142; used for Co. 60) |
| 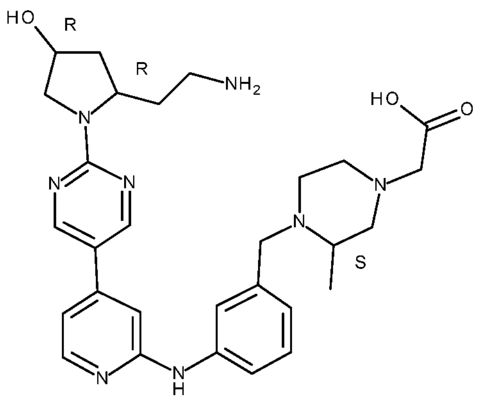<br>TFA salt<br>Int. 155 (starting from Int. 96 and Int. 142; used for Co. 61) | 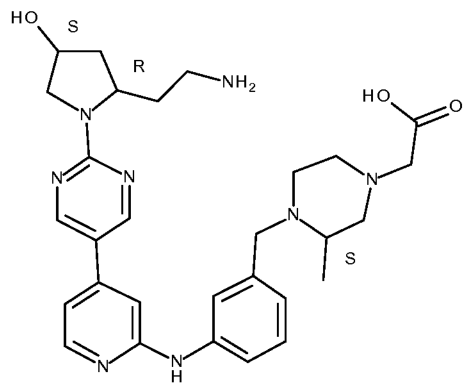<br>TFA salt<br>Int. 156 (starting from Int. 96 and Int. 140; used for Co. 62) |
| | 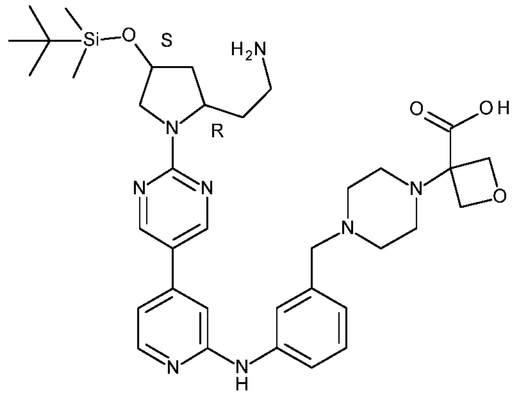<br>Int. 158 (starting from Int. 28 and Int. 140; used for Co. 64) |

(continued)

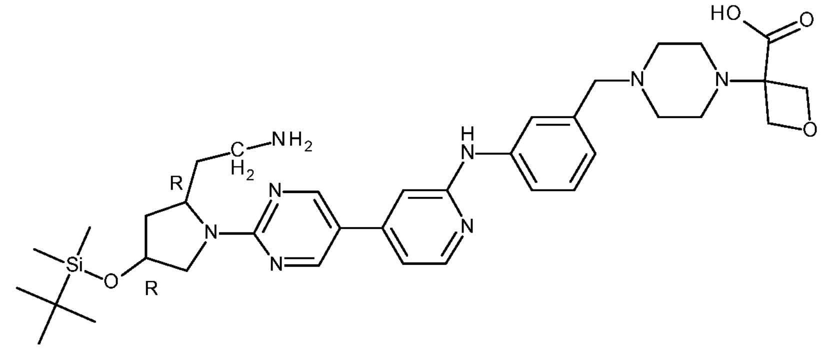

Int. 157 (starting from Int. 28 and Int. 142; used for Co. 63)

Example A9b

a) Preparation of Int. 128

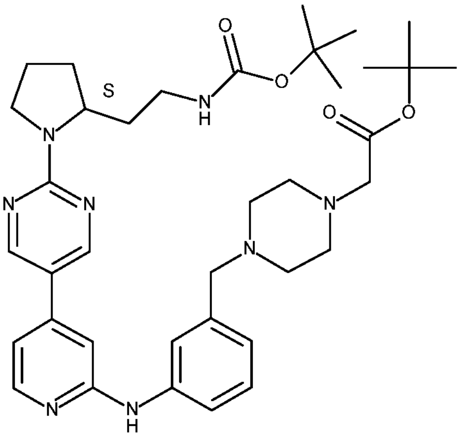

To a solution of Int. 121 (1.20 g; 2.97 mmol) in dioxane(50 ml) was added Int. 41 (1.00 g; 3.27 mmol), S-phos (0.617 g; 1.485 mmol), $Pd_2(dba)_3$ (0.135 g; 0.1485 mmol) and $Cs_2CO_3$ (1.94 g; 5.94 mmol) under $N_2$ atmosphere. The mixture was heated to reflux for 3 h. EtOAc was added and the mixture was filtered. The filtrate was collected and was evaporated. The crude product was purified by column chromatography over silica gel (PE/EtOAc ratio 1/5). The desired fractions were collected and the solvent was evaporated. Yield: 1.48 of Int. 128 (74 %).

b) Preparation of Int. 129

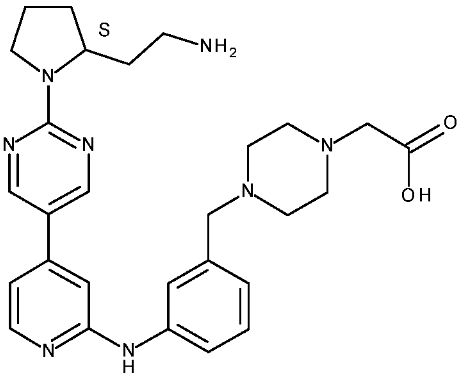

A solution of Int. 128 (1.48 g; 2.20 mol) in 20 % $CF_3COOH$ in DCM (30 ml) was stirred for 2 h. The solvent was evaporated. A $NaHCO_3$ solution was added to adjust the pH to 7-8. The mixture was extracted with DCM. The organic layer was separated, dried and evaporated. Yield: 1.13 g of Int. 129 (99 %).

The intermediates in the table below were prepared by first using an analogous reaction protocol as used for Int. 128, followed by an analogous reaction protocol as used for Int. 129.

| | |
|---|---|
| 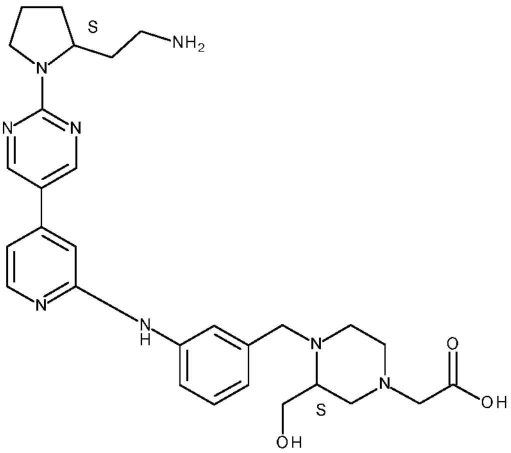 TFA salt<br>Int. 130 (starting from Int. 121 and Int. 71; used for Co. 44) | 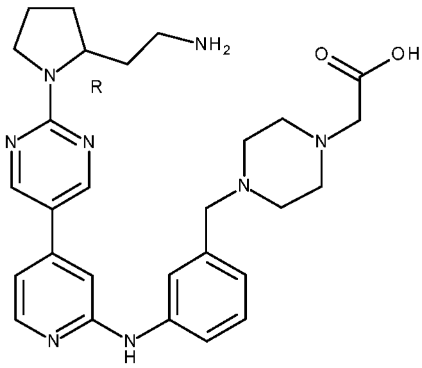 TFA salt<br>Int. 131 (starting from Int. 121b and Int. 41; used for Co. 45) |
| 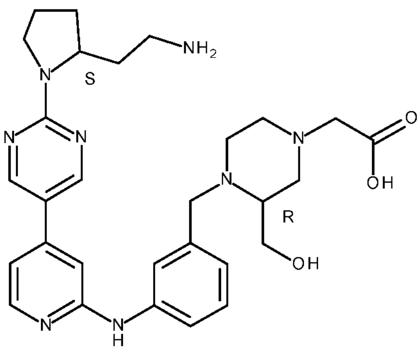 TFA salt<br>Int. 132 (starting from Int. 121 and Int. 70; used for Co. 46) | 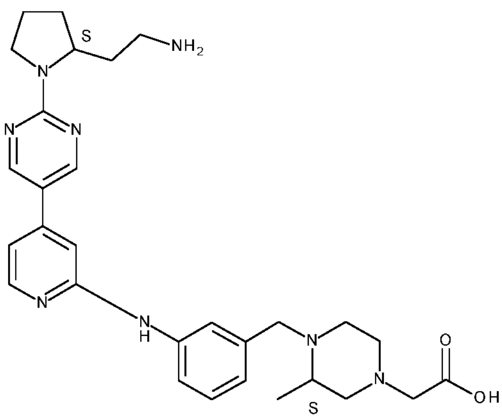 TFA salt<br>Int. 133 (starting from Int. 121 and Int. 96; used for Co. 47) |

(continued)

| | |
|---|---|
| 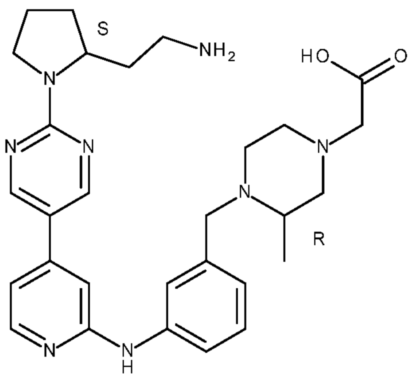 TFA salt | 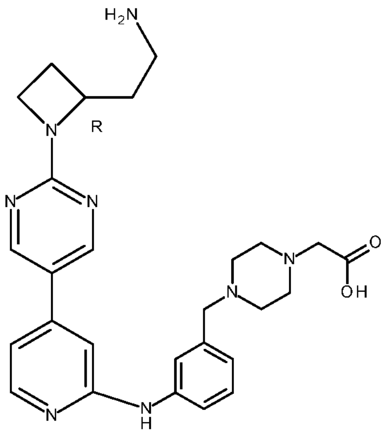 TFA salt |
| Int. 134 (starting from Int. 121 and Int. 95; used for Co. 48) | Int. 135 (starting from Int. 127 and Int. 41; used for Co. 49) |

Example A10

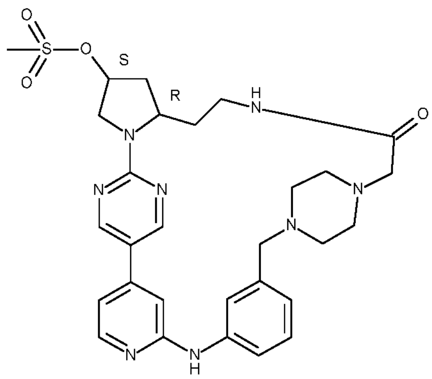

a) Preparation of Int. 190

Compound 55 (102.9 mg; 0.2 mmol) and DIPEA (155 mg; 1.2 mmol) in DCM (5 ml) were stirred at 5 °C. Mesylchloride (144.5 mg; 1 mmol) was added dropwise. Stirring was continued for 1 h at r.t. Water was added. The reaction mixture was extracted with DCM and the organic layer was dried with $MgSO_4$, filtered and evaporated yielding Int; 190 which was used as such in the next reaction step.

Int. 191 (used for Co. 79)

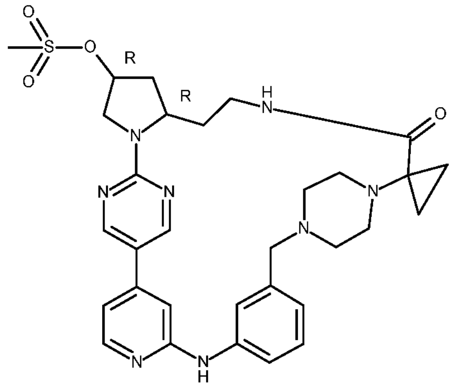

was prepared according to an analogous reaction protocol, but Compound 57 was used as starting material.

Example A11a

a) Preparation of Int. 192a

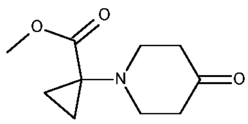

A solution of 1-ethyl-1-methyl-4-oxo-piperidinium, iodide (1:1) (30 g) in $H_2O$ (70 ml) was added over a period of 30 min to a refluxing mixture of 1-amino-cyclopropanecarboxylic acid, methyl ester, hydrochloride (1:1) (11.253 g, 74.233 mmol) and $K_2CO_3$ (1.026 g, 7.423 mmol) in MeOH (200 ml) under $N_2$ atmosphere. The reaction mixture was heated to reflux temperature for 1 h. Then more 1-ethyl-1-methyl-4-oxo-piperidinium, iodide (1:1) (12 g) in $H_2O$ (20 ml) was added over a period of 10 min to the refluxing mixture. The reaction mixture was stirred again for 1 h and was then slowly cooled to r.t. (being stirred for 2 h). The solution was concentrated and the concentrate was diluted with $H_2O$. The aqueous mixture was extracted with DCM. The organic layer was dried and evaporated. The crude product was purified over silica gel with flash chromatography (eluent: PE/EtOAc 9/1). The desired fractions were collected and the solvent was evaporated. Yield: 4.32 g of Int. 192a (29 %).

b) Preparation of Int. 192

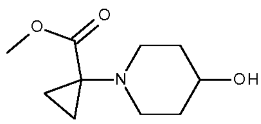

To a solution of Int. 192a (10 g; 50.7 mmol) in MeOH (100 ml) was added $NaBH_4$ (2.88 g; 76.05 mmol) portionwise at r.t.. The solvent was evaporated and water was added. The mixture was extracted with DCM. The organic layer was separated, collected, dried and evaporated. Yield: 10.102 g of Int. 192 (95 %).

c) Preparation of Int. 193

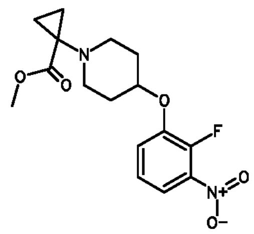

To a solution of Int. 192 (3.49 g; 17.5 mmol), 2- fluoro-3-hydroxy nitrobenzene (2.5 g; 15.9 mmol) and $PPh_3$ (4.59 g; 17.5 mmol) in THF (60 ml) was added DEAD (3.05 g; 17.5 mmol) at 0°C under $N_2$ atmosphere. The mixture was then warmed to r.t. and stirred overnight. The solvent was evaporated and the crude product was purified by flash chromatography over silica (eluens: PE/EtOAc 5/1). The desired fractions were collected and the solvent was evaporated. Yield: 4.2 g of Int. 193 (50.7 %).

d) Preparation of Int. 202

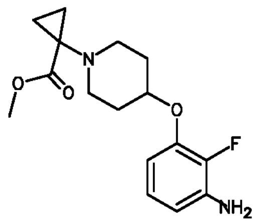

Int. 193 (2.4 g; 4.97 mmol) was dissolved in a mixture of THF (20 ml), water (10 ml) and MeOH (10 ml). Fe (2.38 g; 42.56 mmol) and $NH_4Cl$ (2.28 g; 42.56 mmol) was added. The mixture was refluxed for 2 h and was then filtered. Brine and DCM were added to the filtrate.

The organic layer was collected, dried and evaporated. The crude product was purified over silica gel on flash chromatography (eluent: PE/EtOAc 3/1). The desired fractions were collected and the solvent was evaporated. Yield: 1.38 g of Int. 202 (88.3 %).

Int. 212

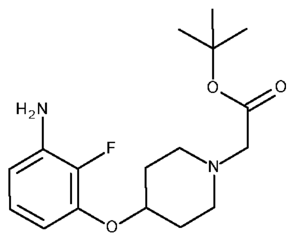

was prepared starting from Int. 197 according to an analogous rection protocol as was used for Int. 202.

e) Preparation of Int. 203

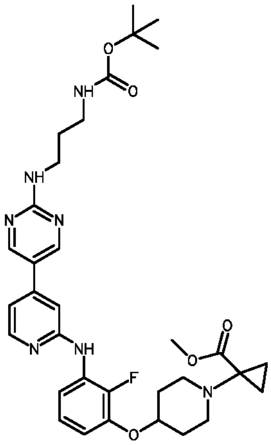

To a solution of Int. 202 (1.38g; 4.38 mmol) in tBuOH (100 ml) was added Int. 8 (1.77 mg; 4.38 mmol), $K_2CO_3$ (1.21 g; 0.439 mmol), X-phos (209.1 mg; 0.439 mmol) and $Pd_2(dba)_3$ (200.8 mg; 0.219 mmol) under $N_2$ atmosphere. The mixture was stirred at 80 °C overnight. The reaction was filtered and evaporated. The crude product was purified over silica gel on flash chromatography (eluent: PE/EtOAc 1/10). The desired fractions were collected and the solvent was evaporated. Yield: 1.7 g of Int. 203 (57.9 %).

f) Preparation of Int. 204

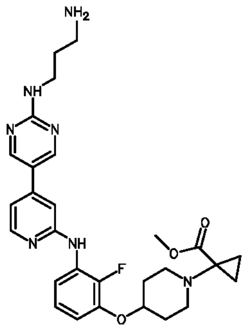

TFA salt

Int. 203 (1.7 g; 2.54 mmol) in TFA 25% in DCM (100 ml) was stirred at r.t. for 2 h. The solvent was evaporated Yield: 1.737 g of Int. 204.

g) Preparation of Int. 205

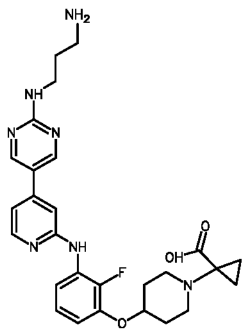

HCl salt

Int. 204 (1.637 g) in 6 N HCl (50 ml) was stirred at 100°C overnight. The solvent was evaporated and the residue

(1.406 g) was used as such in the next reaction step.

The intermediates in the table below were prepared by using successively analogous reaction protocols as used for Int. 202, Int. 203, Int. 204 and Int. 205.

| | |
|---|---|
| 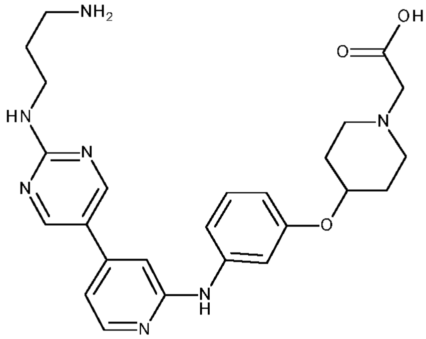 TFA salt<br>Int. 206 (starting from Int. 194; used for Co. 90) | 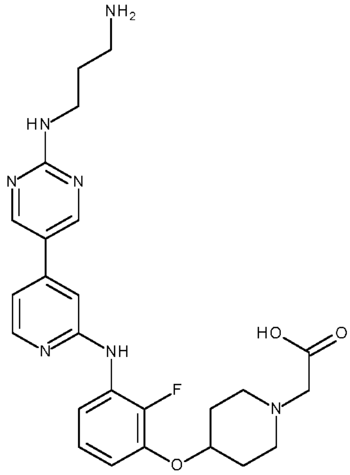 TFA salt<br>Int. 207 (starting from Int. 195; used for Co. 91) |
| 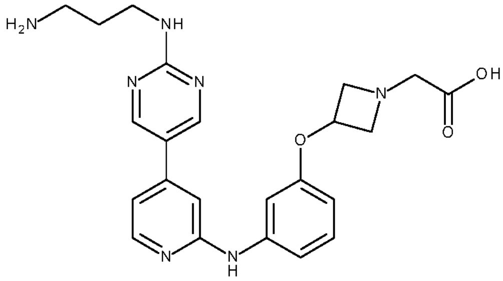 TFA salt<br>Int. 208 (starting from Int. 198; used for Co. 92) | 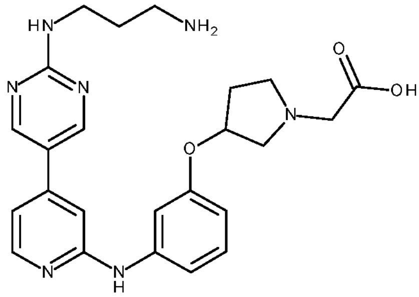 TFA salt<br>Int. 209 (starting from Int. 199; used for Co. 93) |
| 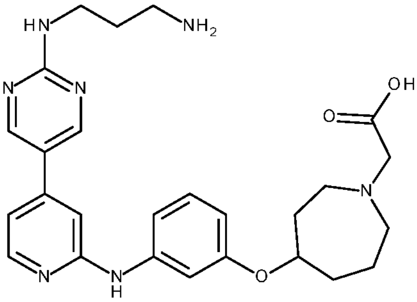 TFA salt<br>Int. 210 (starting from Int. 200; used for Co. 94) | 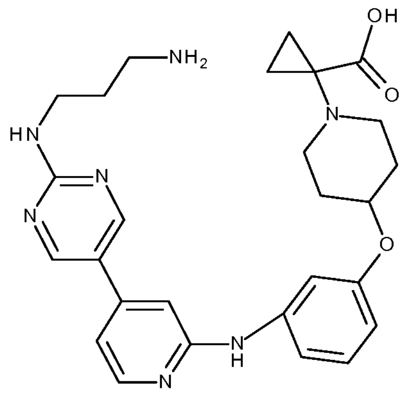 HCl salt<br>Int. 211 (starting from Int. 201; used for Co. 95) |

Example A11b

a) Preparation of Int. 194

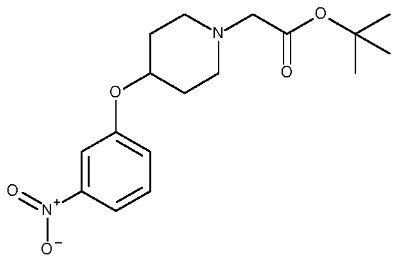

4-(3-nitrophenoxy)-piperidine, hydrochloride (1:1) (4.8 g; 18.55 mol) was dissolved in ACN (150 ml). 2-Bromo-acetic acid, 1,1-dimethylethyl ester (4 g; 20.5 mmol) and DIPEA (6 g; 46.51 mmol) were added. The mixture was stirred at r.t. overnight. The mixture was concentrated. The residue was dissolved in EtOAc and the organic layer was washed with water and brine, dried and concentrated. The residue was purified by chromatography (eluent: PE/EtOAc 3/1). The desired fractions was collected and concentrated. Yield: 5.2 g of Int. 194 (83.2 %).

Example A11c

a) Preparation of Int. 195

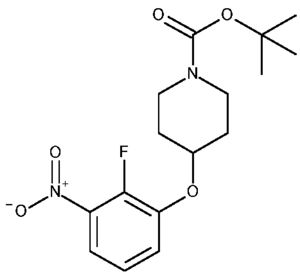

To a solution of 4-hydroxy-1-piperidinecarboxylic acid, 1,1-dimethylethyl ester (3.52 g; 17.5 mmol), 2-fluoro-3-hydroxy nitrobenzene (2.5 g; 15.9 mmol) and $PPh_3$ (4.59 g; 17.5 mmol) in THF (60 ml) was added DEAD (3.049 g; 17.5 mmol) at 0 °C under $N_2$ atmosphere. The mixture was then warmed to r.t. and stirred overnight. The solvent was removed and the crude product was purified over silica gel on flash chromatography (eluent: PE/EtOAc 5/1). The desired fractions were collected and the solvent was evaporated. Yield: 4.2 g of Int. 195 (52.8 %).

b) Preparation of Int. 196

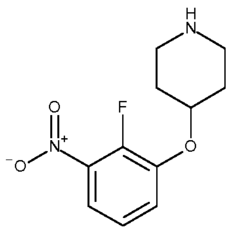

HCl salt

Int. 195 (4.2 g; 8.0 mmol) in HCl (50 ml) in dioxane was stirred at r.t. for 2 h. The solvent was evaporated. DCM was added and the solid was filtered off, washed with DCM and dried. Yield: 2.31 g of Int. 196 (110 %).

c) Preparation of Int. 197

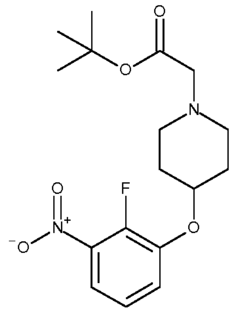

2-bromo-acetic acid, 1,1-dimethylethyl ester (3.284 g; 16.8 mmol) was added to the mixture of Int. 196 (2.31 g; 7.93 mmol) and $K_2CO_3$ (3.49 g; 25.25 mmol) in ACN (100 ml) at r.t.. The reaction mixture was stirred overnight and was then filtered. The filtrate was evaporated *in vacuo.* The residue was dissolved in water and EtOAc. The organic phase was washed with water, brine, dried over $Na_2SO_4$ and filtered. The crude product was purified over silica gel by flash chromatography (eluent: PE/EtOAc 3/2). The desired fractions were collected and the solvent was evaporated. Yield: 1.8 g of Int. 197 (64.0 %).

The intermediates in the table below were prepared by using successively analogous reaction protocols as used for Int. 195, Int. 196 and Int. 197.

| | |
|---|---|
| 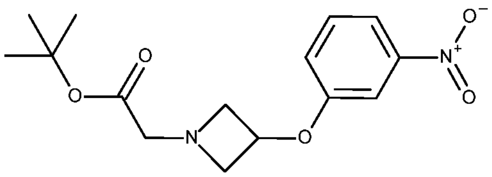<br>Int. 198 (starting from 3-nitrophenol and 3-hydroxy-1-azetidinecarboxylic acid, 1,1-dimethylethyl ester) | 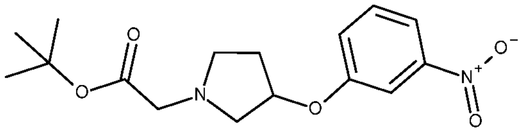<br>Int. 199 (starting from 3-nitrophenol and 3-hydroxy-1-pyrrolidinecarboxylic acid, 1,1-dimethylethyl ester) |
| 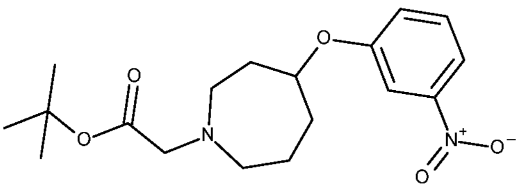<br>Int. 200 (starting from 3-nitrophenol and hexahydro-4-hydroxy-1*H*-azepine-1-carboxylic acid, 1,1-dimethylethyl ester) | 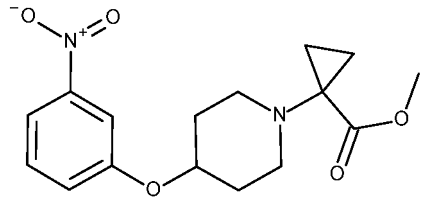<br>Int. 201 (starting from 3-nitrophenol and Int. 192) |

Example A12a

a) Preparation of Int. 213

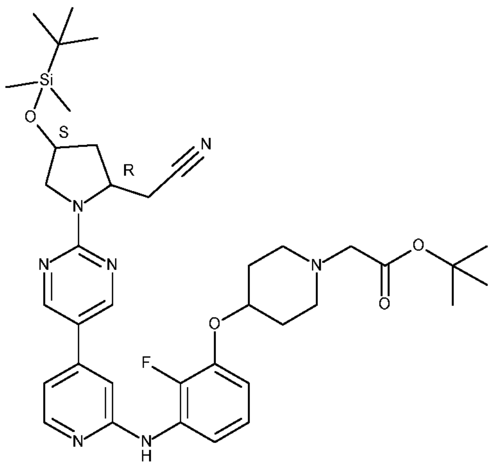

To a solution of Int. 212 (1g; 2.93 mmol) in tBuOH (60 ml) was added Int. 140 (1.26 g; 2.93 mmol), $K_2CO_3$ (0.191 g; 0.586 mmol), X-phos (140 mg; 0.293 mmol) and $Pd_2(dba)_3$ (134 mg; 0.146 mmol) under $N_2$ atmosphere. The mixture was stirred at 80 °C overnight and was then filtered and evaporated. The crude product was purified over silica gel on flash chromatography (eluent: PE/EtOAc 1/1). The desired fractions were collected and the solvent was evaporated. Yield: 0.68 g of Int. 213 (30.7 %).

b) Preparation of Int. 214

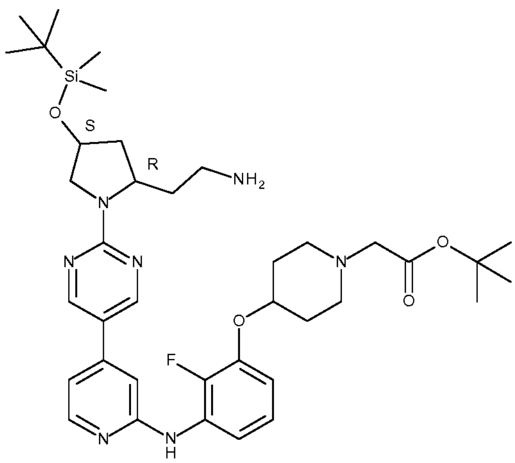

To a solution of Int. 213 (0.68 g; 0.9 mmol) in MeOH (50 ml) was added NH4OH (5 ml) and Raney Nickel (0.5 g) under $H_2$ atmosphere. The mixture was hydrogenated at 50 °C overnight. The catalyst was filtered off and the filtrate was evaporated. Yield: 0.55 g of Int. 214 (76.2 %).

c) Preparation of Int. 215

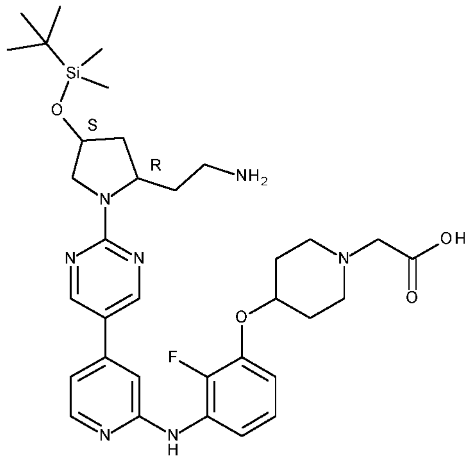

TFA salt

Int. 214 (550 mg; 0.686 mmol) in TFA 30% in DCM (30 ml) was stirred at r.t. overnight. The reaction mixture was evaporated. Yield: 0.507 g of Int. 215.

Int. 216

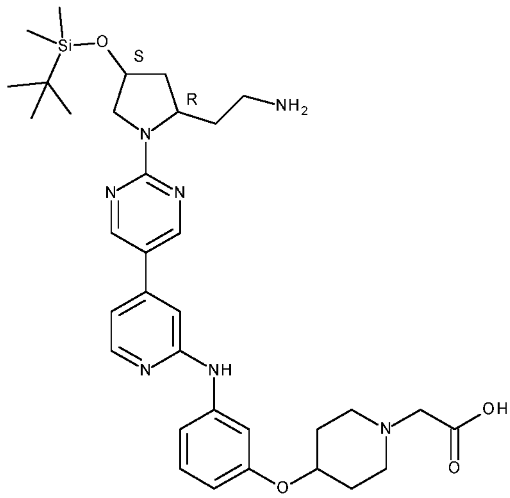

(TFA salt) was prepared starting from Int. 194 by using successively analogous reaction protocols as used for Int. 212, Int. 213, Int. 214 and Int. 215.

d) <u>Preparation of Int. 215a and Int. 216a</u>

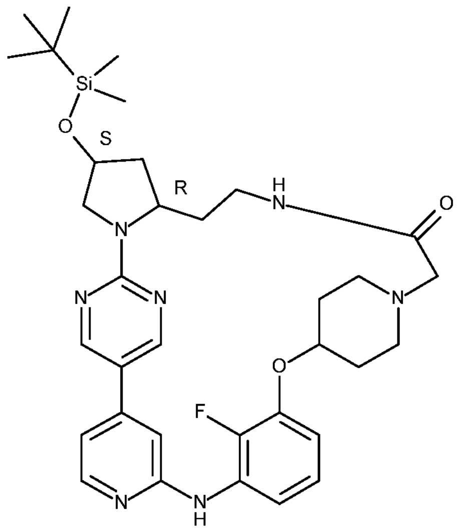

Intermediate 215a

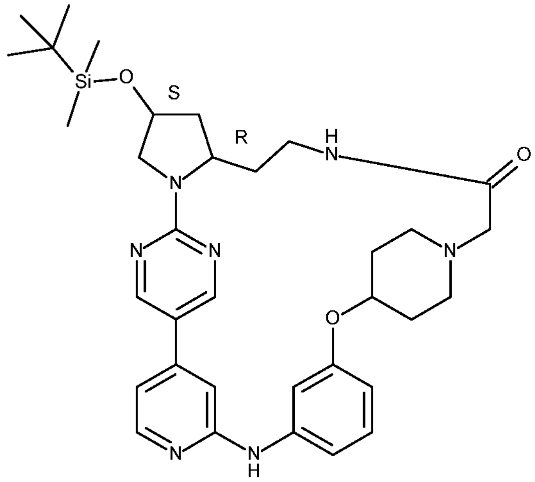

Intermediate 216a

Int. 215a and Int. 216a were prepared respectively from Int. 215 and Int. 216 by following an analogous reaction protocol as was described for Compound 89 (B5).

Example A12b

a) Preparation of Int. 217

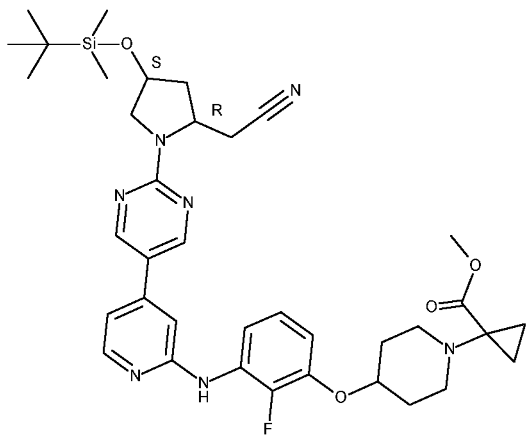

To a solution of Int. 202 (1.4g; 4.22 mmol) in dioxane (70 ml) was added Int. 140 (1.82 g; 4.22 mmol), $Cs_2CO_3$ (2.75 g; 8.446 mmol), S-phos (86.68 mg; 0.211mmol) and $Pd_2(dba)_3$ (96.67 mg; 0.106 mmol) under $N_2$ atmosphere. The mixture was refluxed for 3 h. The reaction was filtered and evaporated. The crude product was purified over silica gel on flash chromatography (eluent: PE/EtOAc 3/2). The desired fractions were collected and the solvent was evaporated. Yield: 1.8 g of Int. 217 (59.8 %).

b) Preparation of Int. 218

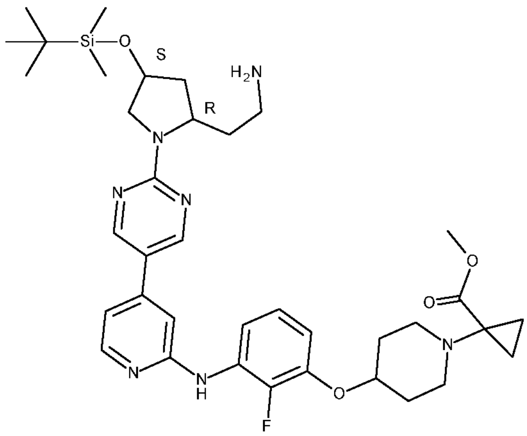

To a solution of Int. 217 (1.8 g; 2.53 mmol) in MeOH (100 ml) was added $NH_4OH$ (9 ml) and Raney Nickel (1 g) under $H_2$ atmosphere. The mixture was hydrogenated at 50 °C overnight. The catalyst was filtered off and the filtrate was evaporated. Yield: 1.78 g of Int. 218 (95 %).

c) Preparation of Int. 219

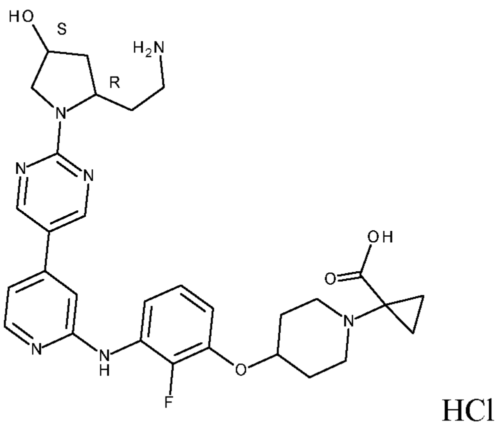

HCl salt

Int. 218 (1.783 g; 2.4 mmol) in 6 N HCl (70 ml) was stirred at 100 °C overnight. The solvent was evaporated and the residue was used as such in the next reaction step. Yield: 1.556 g of Int. 219 (used for Co. 96).

Int. 220 (HCl salt) (used for Co. 97)

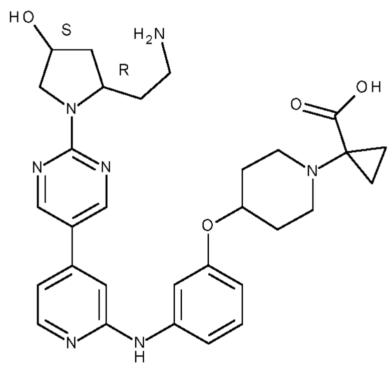

was prepared starting from Int. 201 by using successively analogous reaction protocols as used for Int. 202, Int. 217, Int. 218 and Int. 219.

Example A13a

a) Preparation of Int. 221

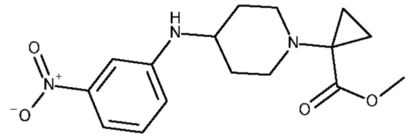

To a solution of Int. 192a (3 g; 15 mmol), and 3-nitro-aniline (1.73 g; 12.55mmol) in DCE (60 ml) was added acetic acid (1.055 g; 17.6 mmol), and the solution was stirred for 1 h. Then sodium triacetoxyborohydride (3.457 g; 16.31 mmol) was added and the reaction mixture was stirred at r.t. overnight. Then water was added and the reaction mixture was extracted twice with DCM. The organic layer was washed with brine, dried, filtered and the solvent was evaporated. The crude product was purified by column chromatography over silica gel (DCM/EtOAc 20/1). The desired fractions were collected and the solvent was evaporated. Yield: 2.2 g of Int. 221 (54%).

Example A13b

a) Preparation of Int. 222

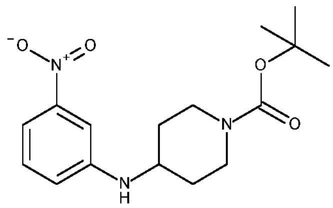

Acetic acid (4.32 g; 72 mmol) was added to a solution of 3-nitroaniline (5.53 g; 40 mmol), 4-oxo-1-piperidinecarboxylic acid, 1,1-dimethylethyl ester (9.56 g; 48 mmol) in DCM (50 ml) and stirring was continued for 30 min. Then sodiumtriacetoxyborohydride (10.17 g; 48 mmol) was added and stirring was continued for 16 h. Then water was added and the mixture was extracted 2x with DCM. The organic layer was washed with brine, dried, filtered and the solvent was evaporated. This crude product was purified by column chromatography over silica (eluent: PE/EtOAc 2/1). The desired fractions were collected and the solvent was evaporated. Yield:12.87 g of Int. 222 (100%).

b) Preparation of Int. 223

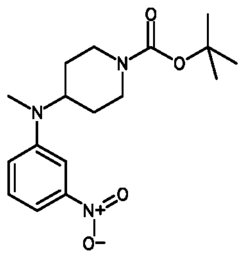

To a solution of Int. 222 (8 g; 24.893 mmol) in DMF (240 ml) at 0 °C under $N_2$ gas atmosphere was added NaH 60% (5 g), and the mixture was stirred for 1 h at r.t.. Then $CH_3I$ (19.4 g; 136.68 mmol) was added and the mixture was stirred overnight at r.t.. The mixture was decomposed with water, and extracted with EtOAc. The organic phase was washed by water, brine, and dried over $Na_2SO_4$, filtered, and evaporated *in vacuo* to give the crude intermediate. Yield:

8.35 g of Int. 223 (100 %).

c) Preparation of Int. 224

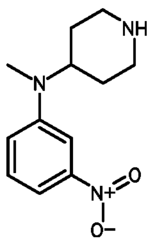

HCl salt

A mixture of Int. 223 (8.35 g; 24.896 mmol) and MeOH/HCl (150 ml) in DCM (150 ml) was stirred overnight at r.t.. The mixture was evaporated *in vacuo*. This crude intermediate was used directly for the next reaction step. Yield: 7.67 g of Int. 224.

d) Preparation of Int. 225

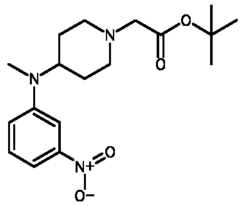

The mixture of Int. 224 (7.67 g), 2-bromo-acetic acid, 1,1-dimethylethyl ester (7.28 g; 37.33 mmol) and $K_2CO_3$ (17.19 g; 124.43 mmol) in ACN (200 ml) was stirred overnight at r.t.. The mixture was filtered, and the filtrate was evaporated *in vacuo* to give the crude intermediate which was purified by column chromatography over silica gel (eluent: PE/EtOAc 1/1). The desired fractions were collected and the solvent was evaporated. Yield:7.5 g of Int. 225.

Example A13c

a) Preparation of Int. 226

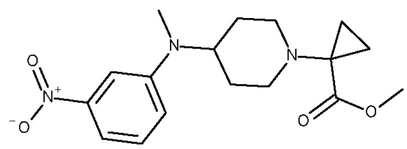

To a solution of Int. 221 (0.65 g; 2.035 mmol) in DMF (30 ml) at 0 °C under $N_2$ gas atmosphere was added NaH 60% (0.407g; 10.175 mmol) and the mixture was stirred for 1 h at r.t.. Then $CH_3I$ (1.44 g; 10.175 mmol) was added and the mixture was stirred overnight at r.t.. Water was added and the mixture was extracted with DCM. The organic phase was washed by water, brine, and dried over $Na_2SO_4$, filtered, and evaporated *in vacuo* to give the crude intermediate which was used as such in the next reaction step. Yield: 0.692 g of Int. 226 (100 %).

Example A13d

a) Preparation of Int. 227

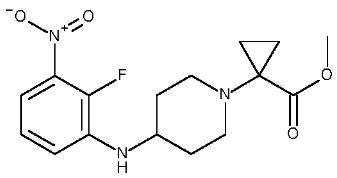

A mixture of 2-fluoro-3- nitro aniline (5 g; 32 mmol), Int. 192a (6.3 g; 32 mmol) and acetic acid (2.885 g; 48 mmol) in DCE (50 ml) was stirred for 1 h at r.t.. Sodium triacetoxy borohydride (10.1 g; 48 mmol) was added. The resulting mixture was stirred at r.t. overnight. The mixture was poured into water and extracted with DCM. The organic layer was collected, dried and evaporated *in vacuo*. The residue was purified by column over silica gel (eluent: PE/EtOAc 3/1). The product fractions were collected and the solvent was evaporated. Yield: 3 g of Int. 227.

b) Preparation of Int. 228

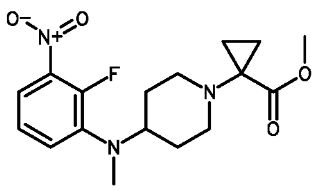

To the solution of Int. 227 (2.4 g; 7.11 mmol) in MeOH (30 ml) was added formaldehyde (1.73 g; 21.34 mmol), sodium cyano borohydride (3.14 g; 50 mmol) and acetic acid (1.58 g; 26.32 mmol). The mixture was stirred at r.t. overnight. The reaction mixture was partitioned between DCM and sat. NaCl. The organic layer was dried over $Na_2SO_4$, filtered and evaporated. The crude was purified by column chromatography (eluent: PE/EtOAc 5/1). The desired fractions were collected and the solvent was evaporated. Yield: 2 g of Int. 228.

Example A13e

a) Preparation of Int. 229

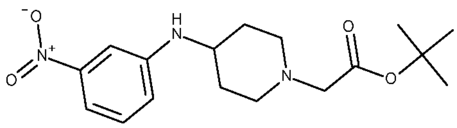

3-Nitro-aniline (5.0 g; 36.2 mmol) was dissolved in DCE (75 ml). Tert-butyl 4-oxopiperidine-1-acetate (15.4 g; 72.4 mmol) and acetic acid (4.3 g; 72.4 mmol) were added. The mixture was stirred at r.t. for 1 h, then sodiumacetoxyborohydride (15.3 g; 72.4 mmol) was added in portions. The mixture was stirred at r.t. overnight. The mixture was washed with water, brine, dried, and concentrated to give 3.0 g of Int. 229 (69.5 %).

Example A14a

a) Preparation of Int. 230

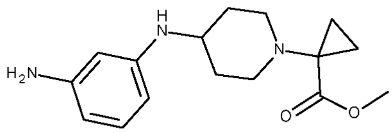

To a solution of Int. 221 (0.67 g; 2.098 mmol) in MeOH (100 ml) was added Pt/C (0.3 g) under $H_2$ atmosphere. The reaction was stirred overnight, then filtered and evaporated. Yield: 0.57 g of Int. 230 (93 %).

Int. 226b (used for Int. 237)

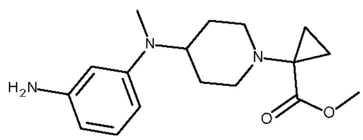

was prepared according to an analogous reaction protocol, but Int. 226 was used as starting material.

Int. 228b (used for Int. 240)

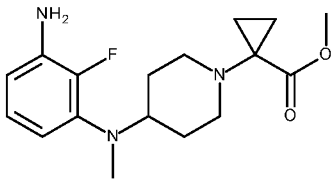

was prepared according to an analogous reaction protocol, but Int. 228 was used as starting material.

b) Preparation of Int. 231

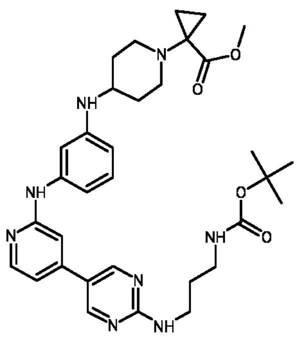

To a solution of Int. 230 (570 mg; 1.97 mmol) in dioxane (50 ml) was added Int. 8 (788.5 mg; 2.167 mmol), $Cs_2CO_3$ (1.28 g; 3.93 mmol), S-phos (40.4 mg; 0.0985 mmol) and $Pd_2(dba)_3$ (45.099 mg; 0.0493 mmol) under $N_2$ atmosphere. The mixture was refluxed for 3 h. EtOAc was added, and the mixture was filtered. The filtrate was evaporated. The crude intermediate was purified by column chromatography over silica gel (PE/EtOAc 1/9). The desired fractions were collected and the solvent was evaporated. Yield: 0.59 g of Int. 231 (43 %).

c) Preparation of Int. 232

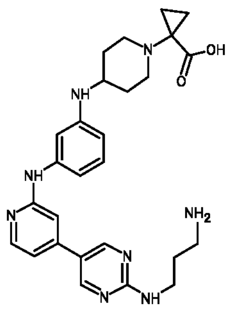

HCl salt

Int. 231 (0.59 g; 0.861 mmol) in HCl in dioxane (30 ml) was stirred at room temperature for 2 h. The solvent was removed. The residue was dissolved in 6 N HCl aqueous (50 ml) and refluxed overnight. The mixture was evaporated and the crude Int. 232 (0.52 g) was used as such in the next reaction step.

The intermediates in the table below were prepared by using successively analogous reaction protocols as used for Int. 230, Int. 231 and Int. 232.

| | |
|---|---|
| 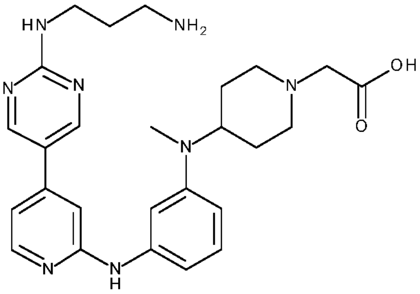 TFA salt<br>Int. 233 (starting from Int. 225; used for Co. 101) | 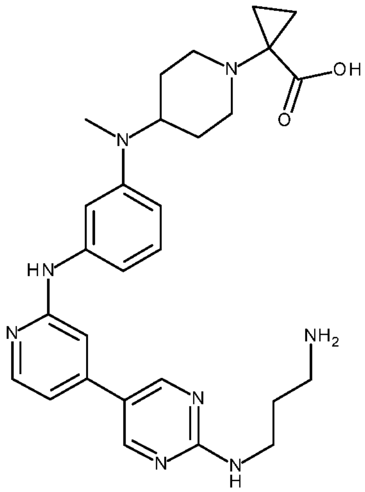 HCl salt<br>Int. 234 (starting from Int. 226; used for Co. 102) |
| 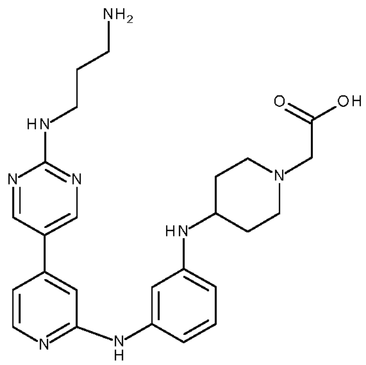 TFA salt<br>Int. 236 (starting from Int. 229; used for Co. 106) | 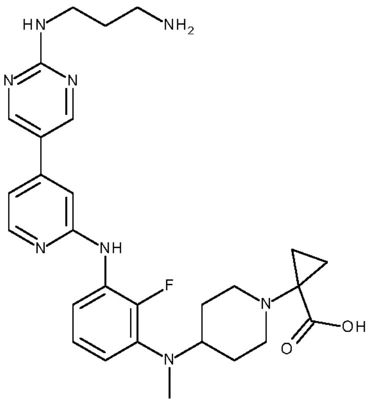 HCl salt<br>Int. 235 (starting from Int. 228; used for Co. 103) |

Example A14b

a) Preparation of Int. 237

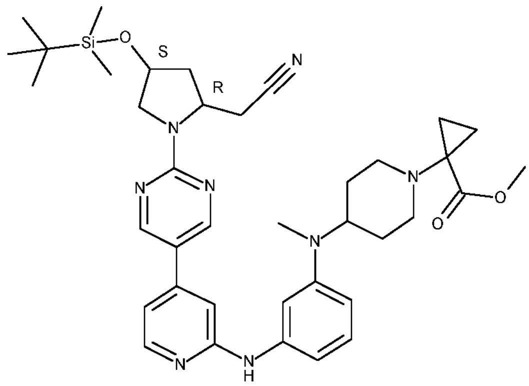

To a solution of Int. 140 (0.875 g; 2.035 mmol) in dioxane (40 ml) was added Int. 226b (0.65 g; 2.035 mmol), $Cs_2CO_3$ (1.33 g; 4.07 mmol), S-phos (0.0423 g; 0.102 mmol) and $Pd_2(dba)_3$ (0.047 g; 0.051 mmol) under $N_2$ atmosphere. The mixture was heated to reflux for 3 h. The reaction was filtered and evaporated. The crude intermediate was purified by flash chromatography over silica gel (eluent: PE/EtOAc 3/1). The desired fractions were collected and the solvent was evaporated. Yield: 0.6 g of Int. 237 (41 %).

b) Preparation of Int. 238

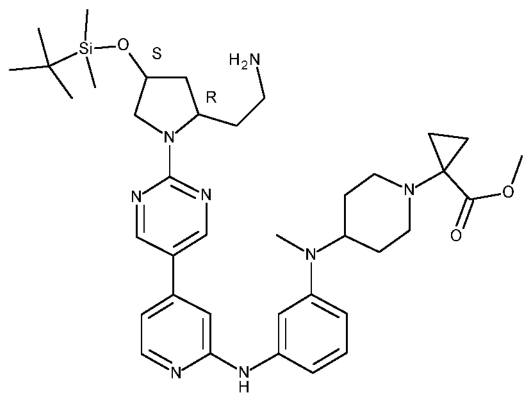

To a solution of Int. 237 (0.6 g; 0.85 mmol) in MeOH (50 ml) was added $NH_4OH$ (3 ml) and Raney Nickel (0.5 g) under $H_2$ atmosphere. The mixture was hydrogenated at 50 °C overnight. The catalyst was filtered off and the filtrate was evaporated. Yield: 0.597 g of Int. 238 (100 %).

c) Preparation of Int. 239

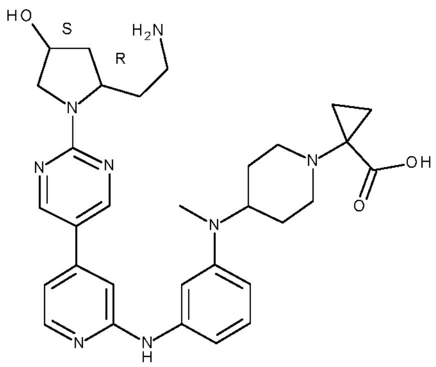

HCl salt

A solution of Int. 238 (597 mg; 0.85 mmol) in 6N HCl (50 ml) was stirred at 100°C overnight. The solvent was evaporated and the residue was used directly for the next reaction step. Yield: 0.529 g of Int. 239 (used for Co. 104).

Int. 240 (used for Co. 105)

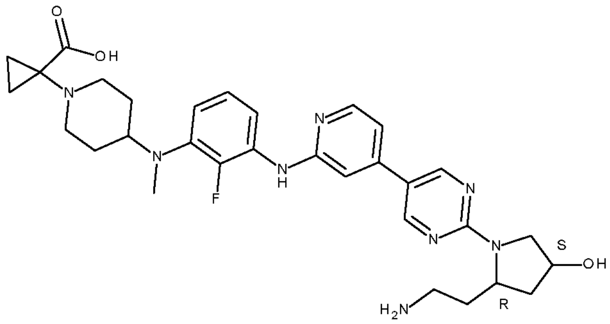

HCl salt was prepared by using successively analogous reaction protocols as used for Int. 237, Int. 238 and Int. 239, starting from Int. 228b.

Example A15

a) Preparation of Int. 176

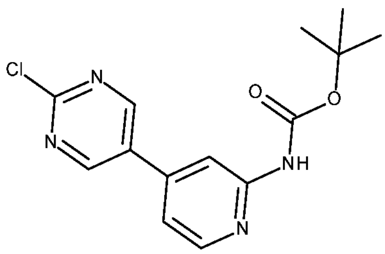

To a solution of N-[4-(4,4,5,5-tetramethyl-1,3,2-dioxaborolan-2-yl)-2-pyridinyl]-carbamic acid, 1,1-dimethylethyl ester (34 g; 90.258 mmol) in dioxane(200 ml) was added 2 chloro-5-bromo pyrimidine (9.699 g; 50.143 mmol). $PdCl_2$(dppf)(1.101 g; 1.504 mmol) and 20 ml 2 M aq. $Na_2CO_3$ were added under $N_2$ atmosphere. The reaction mixture was stirred at 80 °C for 3 h. The mixture was filtered and evaporated. DCM was added and the organic layer was washed with water and brine and dried. The solution was filtered and evaporated. The residue was stirred in tert-butyl methyl ether and the solid was filtered off and dried. Yield: 11 g of Int. 176 (60.7 %).

b) Preparation of Int. 177

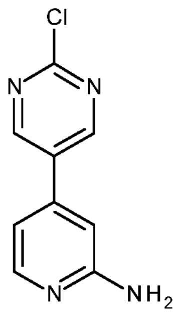

HCl salt

Int. 176 (11g; 30.481 mmol) in HCl in dioxane (100 ml) was stirred at r.t. for 2 h. The solid was filtered off, washed with DCM and dried. Yield: 6.2 g of Int. 177.

c) Preparation of Int. 178

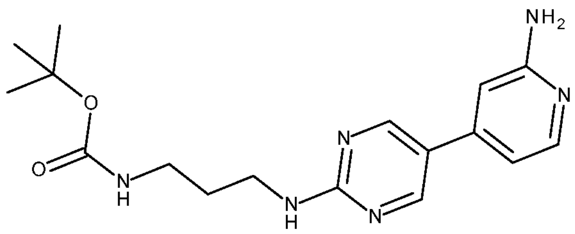

A mixture of Int. 177 (20 g) and N-(3-aminopropyl)carbamic acid tert-butyl ester (20.238 g; 116.147 mmol) in ACN (200 ml) was stirred at 80 °C for 18 h. The reaction was quenched by the addition of water. The product was extracted 3 x from the mixture with DCM. The combined organic layer was washed with water, dried with $MgSO_4$, filtered and the solvents of the filtrate were evaporated. The residue was triturated in DIPE. The precipitate was filtered off, washed with DIPE and dried *in vacuo* at 50 °C. Yield: 20.93 g of Int. 178.

Example A16

a) Preparation of Int. 179

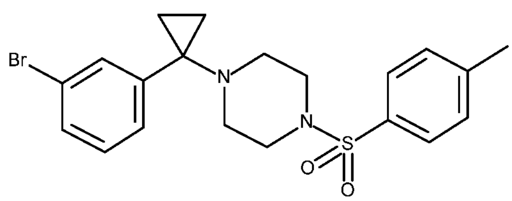

A mixture of 1-(3-bromophenyl)-cyclopropanamine, hydrochloride (1:1) (2.5 g; 10.058 mmol) and N,N-bis(2-chloroethyl)-p-toluenesulphonamide (3.277 g; 11.064 mmol) in DIPEA (10 ml) was stirred at 120 °C for 20 h. The reaction mixture was cooled to r.t. and dissolved in DCM. This organic layer was washed twice with water and once with brine, dried over $MgSO_4$, filtered and the solvents were evaporated. The residue was dissolved in DCM and purified over a $SiO_2$ column, type Grace Reveleris SRC, 120 g, Si 40, on a Armen Spot II Ultimate purification system using heptanes, DCM and MeOH as eluens in a gradient starting from 50 % heptanes and 50 % DCM going to 100 % DCM and ending

with 5 % MeOH and 95 % DCM. The fractions containing product were combined and the solvents were evaporated yielding 2.31 g of Int. 179 (52.75 %).

b) Preparation of Int. 180

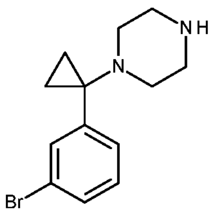

HBr salt

A mixture of Int. 179 (1.9 g; 4.364 mmol) and 33 % HBr in AcOH (25 ml) was stirred at 80 °C for 3 h. The solvents were evaporated. The residue was triturated in DIPE. The precipitate was filtered off, washed 3 x with DIPE and then dried on the air yielding 2.021 g of Int. 180.

c) Preparation of Int. 181

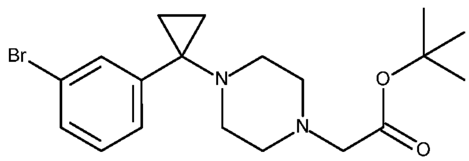

A mixture of Int. 180 (1.33 g), tert-butyl bromoacetate (0.618 ml; 4.188 mmol) and $Et_3N$ (1.94 ml; 13.96 mmol) in DCM (15 ml) was stirred at r.t. for 1 h. The reaction was quenched by the addition of water. The product was extracted 3 x from the mixture with DCM. The combined organic layer was washed with water, dried with $MgSO_4$, filtered and the solvents of the filtrate were evaporated yielding 1.35 g of Int. 181.

The intermediates in the table below were prepared by using successively analogous reaction protocols as used for Int. 179, Int. 180 and Int. 181.

| | |
|---|---|
| 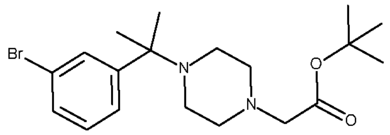 Int. 182 (starting from 2-(3-bromophenyl)propan-2-amine and N,N-bis(2-chloroethyl)-p-toluenesulphonamide) | 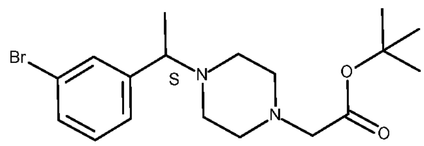 Int. 183 (starting from (S)-3-bromo-alpha-methylbenzylamine and N,N-bis(2-chloroethyl)-p-toluenesulphonamide) |
| 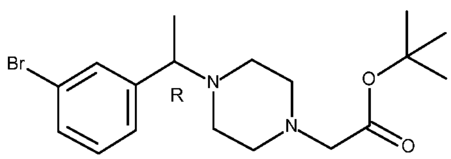 Int. 184 (starting from (R)-3-bromo-alpha-methylbenzylamine and N,N-bis(2-chloroethyl)-p-toluenesulphonamide) | |

Example A17

a) Preparation of Int. 185

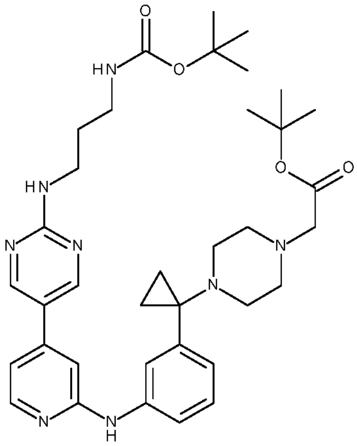

A mixture of Int. 181 (1.35 g; 3.415 mmol), Int. 178 (1.278 g; 3.415 mmol), 2-dicyclohexylphosphino-2',6'-dimethoxy-biphenyl (0.28 g; 0.683 mmol), tris(dibenzylideneacetone)dipalladium(0) (0.313 g; 0.341 mmol) and cesium carbonate (4.45 g; 13.659 mmol) in dioxane (15 ml) was flushed through with $N_2$ gas. After 15 minutes the vial was closed and stirred and heated at 100°C for 18 h. The solvents were evaporated. DCM and water were added. The product was extracted 3x from the mixture with DCM. The combined organic layer was washed with water, dried with $MgSO_4$, filtered and the solvents of the filtrate were evaporated. The residue was dissolved in DCM and purified over a $SiO_2$ column, type Grace Reveleris SRC, 12 g, Si 40, on a Armen Spot II Ultimate purification system using DCM and MeOH as eluens in a gradient starting from 100% DCM and ending with 5% MeOH and 95% DCM. The fractions containing product were combined and the solvents were evaporated yielding Int. 185 (0.668 g; 26.425 %).

b) Preparation of Int. 186

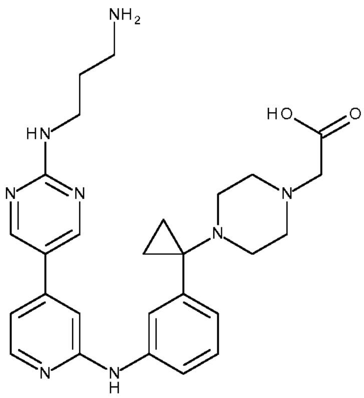

HCl salt

HCl (4 M in dioxane) (2.256 ml) was added to a stirred solution of Int. 185 (0.668 g; 0.902 mmol) in 1,4-dioxane (25 ml) at r.t.. The reaction mixture was stirred at 80 °C for 2 h. The solvents were evaporated. The residue was triturated in DIPE. The precipitate was filtered off, washed with DIPE and then dissolved in MeOH. The solvents were evaporated yielding Int. 186 (0.534 g).

The intermediates in the table below were prepared by using successively analogous reaction protocols as used for Int. 185 and Int. 186.

| | |
|---|---|
| 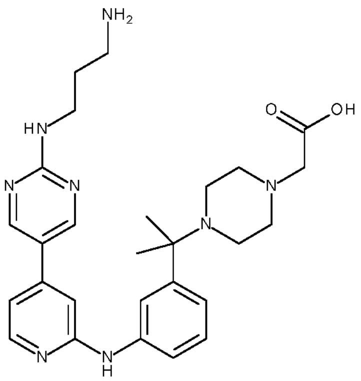 HCl salt | 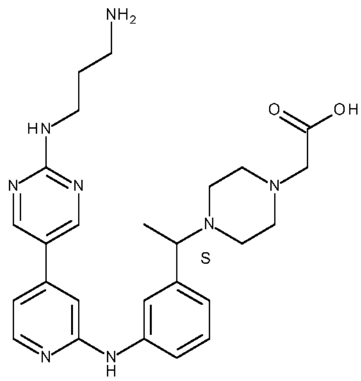 HCl salt |
| Int. 187 (starting from Int. 182; used for Co. 74) | Int. 188 (starting from Int. 183; used for Co. 75) |

| | |
|---|---|
| 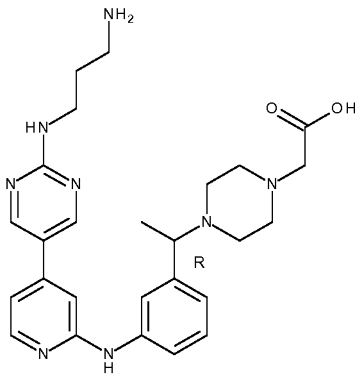 HCl salt | |
| Int. 189 (starting from Int. 184; used for Co. 76) | |

Example A18a

a) Preparation of Int. 241

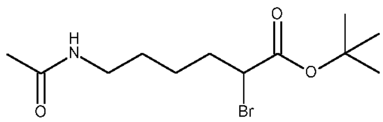

A solution of 6-(acetylamino)-2-bromo-haxanoic acid (9.5 g; 37.7 mmol) and $HClO_4$ (1.5 ml) in acetic acid, 1,1-dimethylethyl ester (400 ml)was stirred overnight at room temperature. The mixture was poured into water and extracted with EtOAc. The organic phase was washed with a sat. $NaHCO_3$ solution, dried over $Na_2SO_4$, and evaporated *in vacuo* to give 5.5 g of Int. 241 as a crude (37.9 %).

b) Preparation of Int. 242

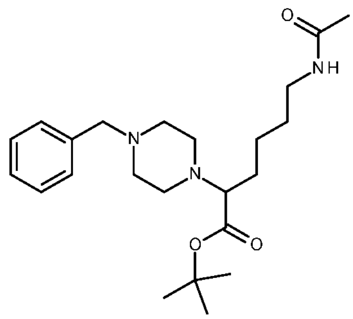

A mixture of Int. 241 (5.5 g; 17.85 mmol), 1-(phenylmethyl)-piperazine (3.145 g) and $K_2CO_3$ (7.4 g; 53.5 mmol) in ACN (200 ml) was stirred overnight at r.t. The mixture was filtered, and the filtrate was evaporated. This crude intermediate was purified by HPLC (HPLC condition: BASE Column: gemini, Flow rate: 80ml/min, Mobile Phase B: ACN, Gradient: 24-54 % (%B) from 0-9 min). The desired fraction was collected, evaporated and basified with a saturated $NaHCO_3$ solution aqueous. The precipitate was filtered to give 1.5 g of Int. 242 (20.8 %).

c) Preparation of Int. 243

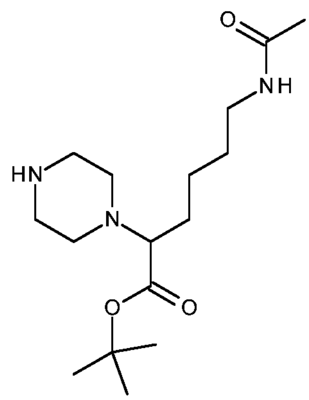

A mixture of Int. 242 (2.4 g; 5.947 mmol) in MeOH (100 ml) was hydrogenated at 20 °C under a $H_2$ gas atmosphere (50 psi) with Pd/C (1.5 g) as a catalyst. After uptake of 1 eq. of $H_2$ gas, the catalyst was filtered off and the filtrate was evaporated to give 1.74 g of Int. 243 (93.3%) which was used directly for the next reaction step.

Example A18b

a) Preparation of Int. 244

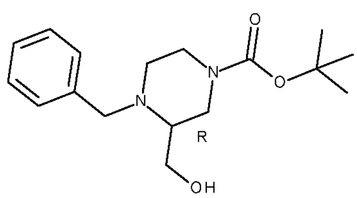

A mixture of (3R)-3-(hydroxymethyl)-1-piperazinecarboxylic acid, 1,1-dimethylethyl ester (3g; 13.87 mmol), benzaldehyde (1.77 g; 16.65 mmol) and acetic acid (1. 3g; 20.8 mmol) in DCE (20 ml) was stirred for 1 h at r.t. Sodiumacetoxyborohydride (3.52 g; 16.65 mmol) was added. The resulting mixture was stirred overnight, poured into water and extracted with DCM. The organic layer was collected, dried and evaporated *in vacuo.* The residue was purified by

column over silica gel (eluent: PE/EtOAc 3/1). The product fractions were collected and the solvent was evaporated, yielding 3 g of Int. 244 (70.6 %).

b) Preparation of Int. 245

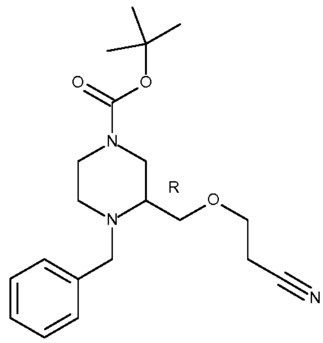

Int. 244 (3 g; 9.8 mmol) was added to a mixture of acrylonitrile (2.6 g; 49 mmol) and tetrabutylammonium iodide(400 mg) in 40 % NaOH aqueous (30 ml) and toluene (10 ml) at r.t. The resulting mixture was stirred at room temperature overnight. The mixture was extracted with EtOAc and the organic layer was collected, dried and evaporated *in vacuo.* The crude was purified by column chromatography (eluent: PE/EtOAc 3/1). The desired fractions were collected and the solvent was evaporated. Yield: 3 g of Int. 245 (85 %).

c) Preparation of Int. 246

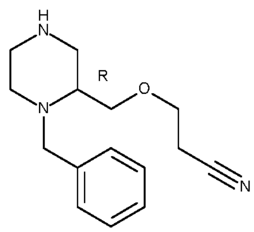

HCl salt

The mixture of Int. 245 (3 g; 8.346 mmol) in HCl/dioxane (20 ml) was stirred at r.t. for 4 h. The solvent was removed *in vacuo,* yielding 2.77 g of Int. 246 which was used directly for the next reaction step.

d) Preparation of Int. 247

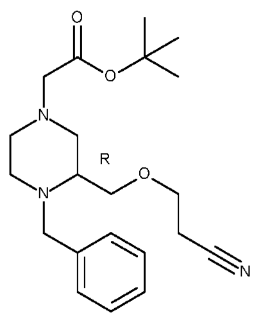

2-Bromo-acetic acid, 1,1-dimethylethyl ester (1.95 g; 10 mmol) was added to the mixture of Int. 246 (2.756 g) and $K_2CO_3$ (3.44 g; 25 mmol) in ACN(50 ml). The resulting mixture was stirred at r.t. overnight. The solid was filtered

and the filtrate was evaporated and purified by column chromatography (eluent: PE/EtOAc 3/1). The desired fractions were collected and the solvent was evaporated, yielding 1.8 g of Int. 247.

e) Preparation of Int. 248

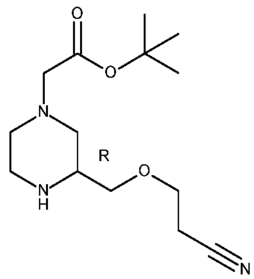

To a solution of Int. 247 (1.8 g; 4.8 mmol) in DCE (100 ml) was added carbonochloridic acid, 1-chloroethyl ester (1.373 g; 9.6 mmol). The mixture was refluxed overnight and was then concentrated. The residue was dissolved in MeOH and refluxed for 1 h. The solvent was removed and the residue was taken up into a sat. $NaHCO_3$ solution aqueous and extracted with EtOAc. The organic layer was washed with brine, dried, filtered and evaporated *in vacuo.* 1.16 g of crude Int. 248 was obtained which was used directly for the next reaction step.

Example A19

a) Preparation of Int. 249

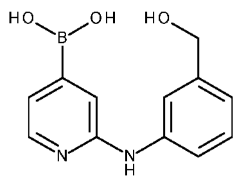

A mixture of 2-fluoro-4-boronic acid (15 g; 106.5 mmol), 3- amino benzylalcohol (14.4 g; 117 mmol) and 4N HCl in dioxane (26.6 ml) in dioxane (100 ml) and water (20 ml) was stirred at 100 °C for 64 h. The reaction mixture was cooled and $NaHCO_3$ (18 g) was slowly added. Then the solvent was concentrated under reduced pressure until a volume of 50 ml. The residue was treated with $H_2O$ (300 mL) and EtOAc (200 ml). The solids not dissolving in $H_2O$ and EtOAc were filtered off. The solids were washed with DIPE and dried *in vacuo.*
Yield: 17.9 g of Int. 249.

b) Preparation of Int. 250

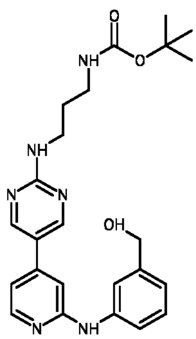

A mixture of Int. 249 (1 g; 4.097 mmol), Int. 1 (1.493 g; 4.507 mmol), $PdCl_2$(dppf) (0.3g; 0.41 mmol) and $Na_2CO_3$ (1.303 g; 12.292 mmol) in water (3.8 ml) and 1,4-dioxane (38 ml) was flushed through with $N_2$ gas for 15 min. The reaction mixture was stirred at 80 °C for 2 h and then cooled down to r.t. The reaction mixture was poured out into ice/water. The mixture was stirred for 20 min and then the precipitate was filtered off, washed with water and then dried on the air. The precipitate was dissolved in a mixture of DCM/MeOH and then the solvents were evaporated. Yield: 1.84 g of Int. 250 (99.7 %).

c) Preparation of Int. 251

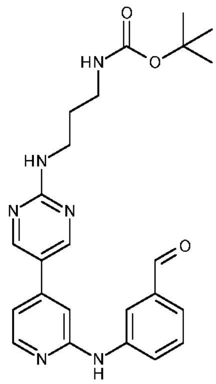

$MnO_2$ (1.78 g; 20.4 mmol) was added portionwise to a solution of Int. 250 (0.46 g; 1 mmol) in EtOAc (40 ml) at r.t. The reaction mixture was stirred at r.t. for 1 day. The mixture was filtered over a plug of Dicalite®. The residue was washed 5x with EtOAc. The solvents of the filtrate were evaporated yielding 0.56 g of Int. 251.

d) Preparation of Int. 252

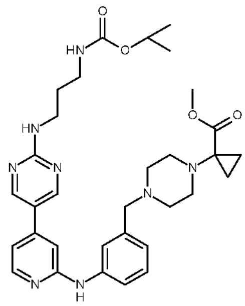

Sodiumacetoxyborohydride (0.794 g; 3.746 mmol) was added portionwise to a stirred mixture of Int. 251 (0.56 g; 1.249 mmol) and 1-(1-piperazinyl)-cyclopropanecarboxylic acid, methyl ester (0.318 g; 1.498 mmol) in DCM (5.6 ml) at r.t. The reaction mixture was stirred at room temperature for 2 h. The reaction was quenched by the addition of a saturated aqueous $NH_4Cl$ solution. Water was added and the mixture was extracted twice with DCM. The organic layer was separated, dried with $MgSO_4$, filtered and the solvents were evaporated. The residue was dissolved in DCM and purified over a $SiO_2$ column, type Grace Reveleris SRC, 12 g, Si 40, on a Armen Spot II Ultimate purification system using DCM and MeOH as eluents in a gradient starting from 100% DCM to 5% MeOH and 95% DCM. The fractions containing product were combined and the solvents were evaporated yielding 0.29 g of Int. 252.

e) Preparation of Int. 253

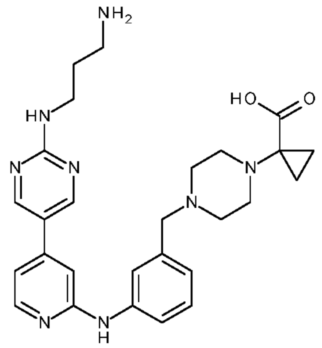

HCl salt

HCl (4M in dioxane) (3.385 ml) was added to a stirred solution of Int. 252 (0.29g; 0.451 mmol) in 1,4-dioxane (8 ml) at r.t. The reaction mixture was stirred at 80 °C for 20 h. The solvents were evaporated. HCl (37% in $H_2O$) (8 ml) was added to the residue and the mixture was stirred at 80°C for 18 h. The solvents were evaporated yielding 337 mg of Int. 253.

The intermediates in the table below were prepared from Int. 251 by using successively analogous reaction protocols as used for Int. 252 and Int. 253.

| | |
|---|---|
| 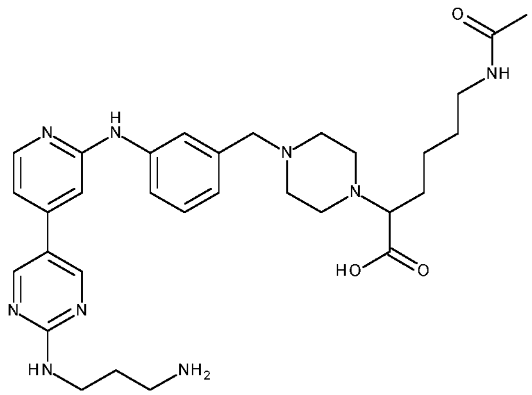 TFA salt | 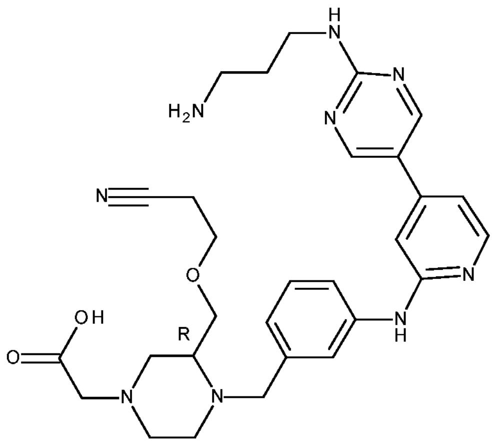 TFA salt |
| Int. 254 (starting from Int. 251 and Int. 243; used for Co. 108) | Int. 255 (starting from Int. 251 and Int. 248; used for Co. 111) |

Example A20

a) Preparation of Int. 256

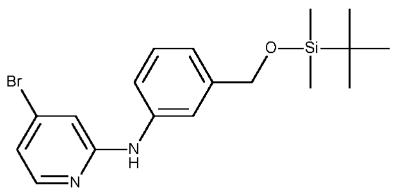

A flask was charged with 2-amino 4-bromo pyridine (19.1 g; 110.5 mmol), 1-[[[(1,1-dimethylethyl)dimethylsilyl]oxy]methyl]-3-iodo-benzene (38.5 g; 110.5 mmol), $Cs_2CO_3$ (126.0 g; 386.8 mmol), dioxane (545 ml) and THF(90 ml). Under $N_2$ gas atmosphere Xantphos (3.84 g; 6.63 mmol) and $Pd(OAc)_2$ (1.24 g; 5.53 mmol) were added and the reaction

mixture was heated at 90 °C for 3 h. The mixture was filtered. The filtrate was evaporated *in vacuo.* The crude product was purified by column chromatography over silica gel (eluent: DCM/MeOH 15/1). The desired fractions were collected and the solvent was evaporated. Yield: 34 g of Int. 256 (78 %).

b) Preparation of Int. 257

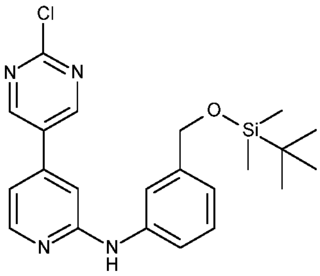

A flask was charged with Int. 256 (32.0 g; 81 mmol), 2-chloropyrimidine-5-boronic acid (15.4 g; 97 mmol), $Na_2CO_3$ (2M aqueous) and dioxane (q.s.). Bis[tris(1,1-dimethylethyl)phosphine]-palladium (2.1 g; 4.1 mmol) was added to the reaction mixture and the mixture was heated at 100 °C for 2 h. The mixture was extracted with EtOAc. The organic phase was evaporated *in vacuo* to give the crude intermediate which was purified by column chromatography over silica gel (eluent: DCM/MeOH 20/1). The desired fractions were collected and the solvent was evaporated. Yield: 32 g of Int. 257 (92 %).

c) Preparation of Int. 258 and Int. 258a

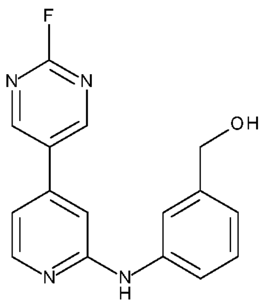

Int. 258

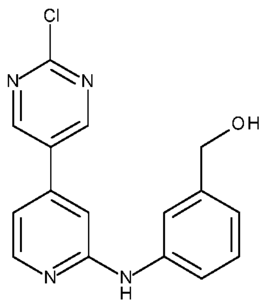

Int. 258a

The mixture of Int. 257 (32.0 g; 75 mmol) and TBAF (29.5 g; 113 mmol) in THF (400 ml) was stirred overnight. The organic phase was evaporated *in vacuo.* The residue was purified by HPLC (HPLC condition: Column: YMC PACK QDS-AQ 150*30 mm, 5μm, Flow rate: 50ml/min, Mobile Phase A: Purified water (containing 0.075 % TFA), Mobile Phase B: ACN, Gradient: 24-54 %(% B) from 0-9 min. Two different product fractions were collected, evaporated *in vacuo* and made alkaline with a saturated $NaHCO_3$ solution (aqueous). The mixtures were filtered and evaporated. Yield: 9.5 g of Int. 258a (41 %) and 2.7 g of Int. 258 (12 %).

d) Preparation of Int. 259

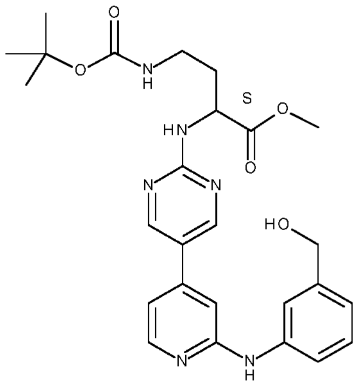

The mixture of Int. 258 (1.5 g; 5.06 mmol), (2*S*)-2-amino-4-[[(1,1-dimethylethoxy)carbonyl]-amino]-butanoic acid, methyl ester, hydrochloride (1:1) (1.35 g; 5.06 mmol) and DIPEA (5 ml) in NMP (30 ml) was stirred at room temperature for 24 h. The resulting mixture was poured into water and extracted with EtOAc. The organic layer was washed with brine, dried, filtered and evaperated *in vacuo.* The residue was purified by flash column chromatography (eluent: DCM/MeOH from 100/0 to 95/5). The desired fractions were collected and the solvent was evaporated. Yield: 1.9 g of Int. 259 (74 %).

e) Preparation of Int. 260

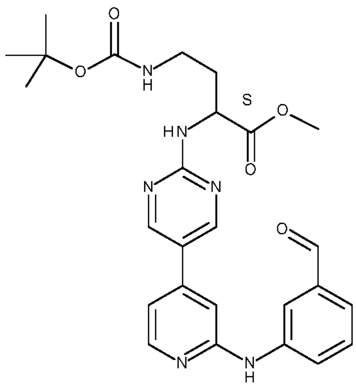

The mixture of Int. 259 (1.9 g; 3.74 mmol) and $MnO_2$ (3.25 g; 37.4 mmol) in DCM (30 ml) was stirred at r.t. overnight. The $MnO_2$ was filtered off over Celite®. The filtrate was evaporated *in vacuo,* yielding 1.6 g of Int. 260 (84.4 %).

f) Preparation of Int. 261

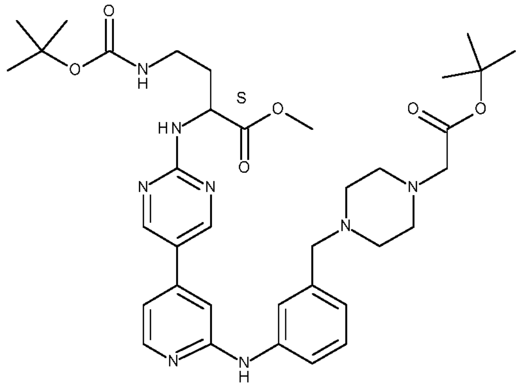

The mixture of 1-piperazineacetic acid, 1,1-dimethylethyl ester (0.76 g; 3.79 mmol), Int. 260 (1.6 g; 3.16 mmol) and $CH_3COOH$ (0.28 g; 4.74 mmol) in DCE was stirred for 1 h at r.t. $NaBH(OAc)_3$ (0.80 g; 3.79 mmol) was added. The resulting mixture was stirred overnight. The resulting mixture was poured into water and extracted with EtOAc. The organic layer was washed with aq. $NaHCO_3$ and brine, dried over $Na_2SO_4$ and evaporated *in vacuo.* The residue was purified by column chromatography (eluent: 100 % EtOAc). The desired fractions were collected and the solvent was evaporated. Yield: 1.3 g of Int. (59 %).

Int. 262

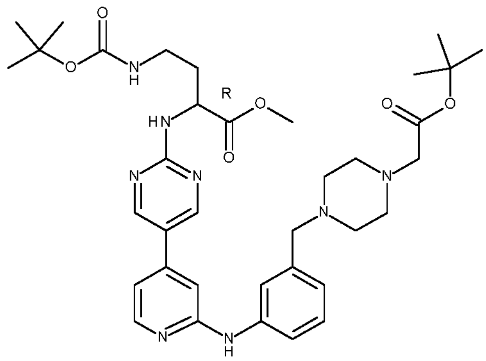

was prepared by using successively analogous reaction protocols as used for Int. 259, Int. 260 and Int. 261, starting from Int. 258 and (2*R*)- 2-amino-4-[[(1,1-dimethylethoxy)carbonyl]amino]-butanoic acid, methyl ester, hydrochloride (1:1).

Example A21

a) Preparation of Int. 263

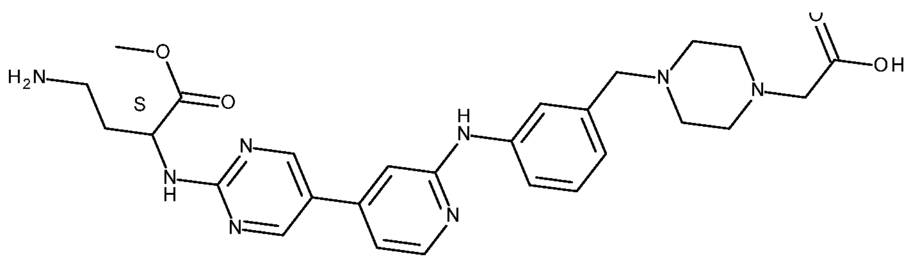

TFA salt

A mixture of Int. 261 (1.3 g; 1.88 mmol) and TFA (4 ml) in DCM (12 ml) was stirred at r.t. overnight. The solvent was removed *in vacuo.* The residue was used directly in the next reaction step. Yield: 1.5 g of Int. 263.

Int. 264 (used for Co. 118)

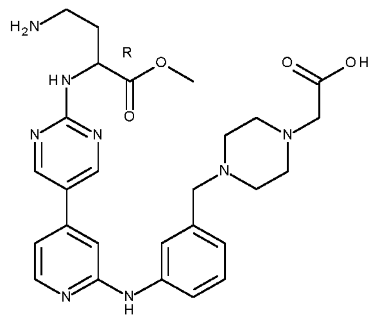

was prepared by using an analogous reaction protocol as used for Int. 263 starting from Int. 262.

Example A22

a) Preparation of Int. 265

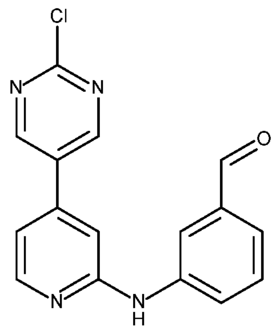

Int. 258a (1g; 3.2 mmol) and EtOAc (100 ml) were stirred at r.t. $MnO_2$(5 g) was added portionwise. Stirring was continued for 16 h. The catalyst was filtered off hot (3x repeated). The combined filtrates were evaporated to dryness yielding 790 mg of Int. 265 which was used as such in the next reaction step.

b) Preparation of Int. 266

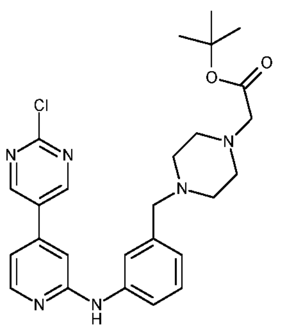

1-Piperazineacetic acid, 1,1-dimethylethyl ester hydrochloric acid (1/2) (0.865mg; 3.17 mmol) was suspended in DCM (50 ml), sodium acetate (0.487g; 5.94 mmol) and acetic acid (5 ml). Int. 265 (0.82 g) was added and stirred for 10 min. $NaBH(OAc)_3$ (1.395 g; 6.60 mmol) was added. The reaction mixture was stirred for 1.5 h and then additional $NaBH(OAc)_3$ (1.5 g) was added. The reaction mixture was stirred for 4h and was then poured out in a sat. $NaHCO_3$-solution (aqueous). This mixture was extracted with DCM/iPrOH. The organic layer was separated, dried and evaporated. The residue was purified by column chromatography over silicagel (eluent gradient DCM 100% to 85 % / MeOH-$NH_3$ 0% to

15%). The desired fractions were collected and evaporated yielding 0.25 g of Int. 266.

c) Preparation of Int. 267

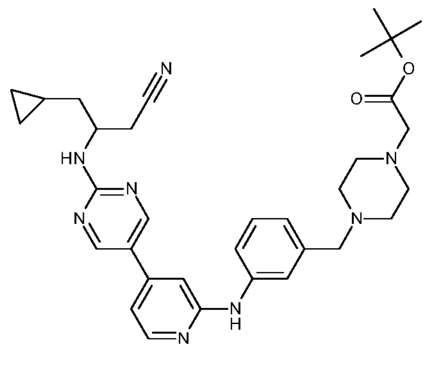

β-Amino-cyclopropanebutanenitrile (0.224 g; 1.394 mmol) was suspended in ACN (10 mL). DIPEA (0.32 ml) was added. The reaction mixture was stirred for 5 min. Int. 266 (0.23 g; 0.465 mmol) in ACN (10 mL) was added. The reaction mixture was heated 80 h at 130 °C. The solvent was evaporated and the residue was dissolved in DCM. The organic layer was separated, washed with a $NaHCO_3$-solution (aqueous), and water and was then dried and evaporated, yielding 0.17 g of Int. 267 (62.8 %).

d) Preparation of Int. 268

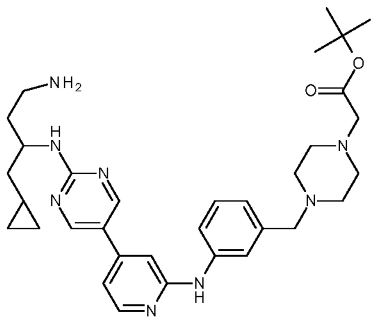

Raney Nickel (24 mg) was suspended in 7 N $NH_3$ in MeOH (50 ml) under $N_2$ atmosphere. Int. 267 (0.25 g; 0.412 mmol) dissolved in MeOH (20 ml) was added at r.t. The reaction mixture was hydrogenated under an atmosphere pressure of $H_2$ gas. The catalyst was filtered off and the filtrate was evaporated. The residue was purified by column chromatography on silicagel (eluent gradient DCM 100% to 85 % / MeOH-$NH_3$ 0% to 15%). The desired fractions were collected and evaporated yielding 0.15 g of Int. 268 (62 %).

e) Preparation of Int. 269

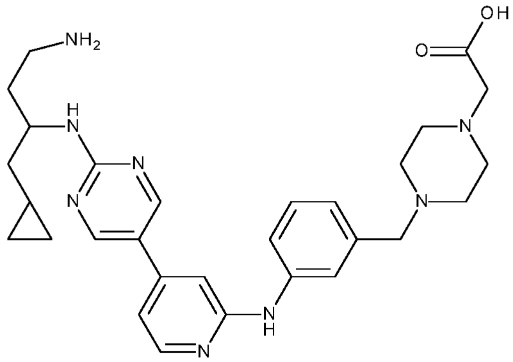

Int. 268 (0.15 g; 0.256 mmol) was dissolved in dioxane (30 ml) and HCl (4 M in dioxane; 1 ml). The reaction mixture was heated for 18 h at 100 °C. The reaction mixture was evaporated and dried *in vacuo* overnight. The crude compound such obtained was used as such in the next reaction step.

Example A23

a) Preparation of Int. 270

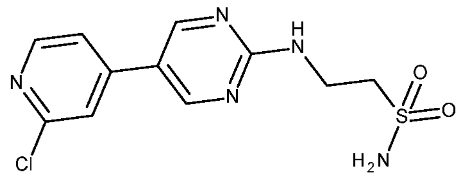

A mixture of Int. 120 (1.4 g; 6.23 mmol) and 2-amino-ethanesulfonamide, hydrochloride (1/1) (1 g; 6.23 mmol) in $Et_3N$ (1.82 ml; 13.1 mmol) and ACN (50 ml) was stirred at 60 °C for 48h. The reaction mixture was cooled to r.t. The precipitate that was formed was filtered off, washed with ACN and dried *in vacuo* at 45 °C yielding 1.47 g Int. 270 (72.2 %).

b) Preparation of Int. 271

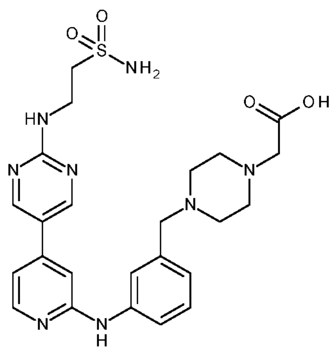

A mixture of Int. 270 (0.47 g; 1.44 mmol)) and Int. 41 (0.66 g; 2.16 mmol) in n-butanol (4.5 ml) and HCl (6 M in iPrOH) (3 ml) was stirred and heated at 140 °C for 3 h using microwave irradiation. The solvents were evaporated. The residue was purified by Prep HPLC on (RP Vydac Denali C18 - 10μm, 200g, 5cm). Mobile phase (0.25% $NH_4HCO_3$ solution in water, ACN). The desired fractions were collected, evaporated, solved in MeOH and evaporated again, yielding 0.20 g of Int. 271 (26.4 %).

Example A24

a) Preparation of Int. 272

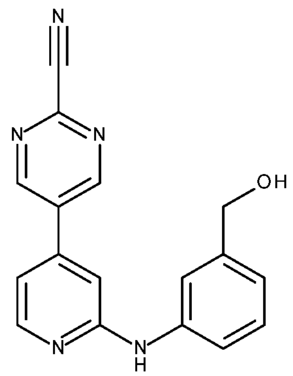

1,1'-Bis(diphenylphosphino)ferrocenedichloro palladium(II) (0.75 g; 1.022 mmol) was added to Int. 249 (8 g; 32.779 mmol) and 5-bromopyrimidine-2-carbonitrile (7.237 g; 39.335 mmol) in dioxane (160 ml) at r.t. The reaction mixture was stirred at 80 °C for 30 min. A $Na_2CO_3$ solution in $H_2O$ (24.584 ml; 49.169 mmol) was added to the reaction mixture at 80 °C. The reaction mixture was stirred at 80 °C for 1 h. The reaction mixture was poured on ice/water. The water layer was stirred at r.t. for 1 h. The precipitate was filtered off and dried under vacuum yielding 9.2 g of Int. 272 (92.53 %).

b) Preparation of Int. 273

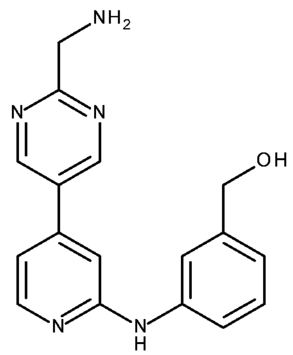

A suspension of Int. 272 (4.3 g; 14.176 mmol) and Pd 5 % wt on active carbon wet degussa type (430.0 mg; 4.041 mmol) in EtOAc/acetic acid (1/1) (150 ml) was hydrogenated at r.t. under atmospheric pressure of $H_2$ for 16 h. The catalyst was filtered off and was washed with MeOH (250ml). The filtrate was evaporated to dryness. The residue was dissolved in water (200 ml). The water layer was basified with a saturated $NaHCO_3$ solution (aqueous). The water layer was filtered through Dicalite®. The filtrate was stirred at r.t. for 16 h. The precipitate was filtered off and dried yielding Int. 273 (2.2 g; 50.5 %).

c) Preparation of Int. 274

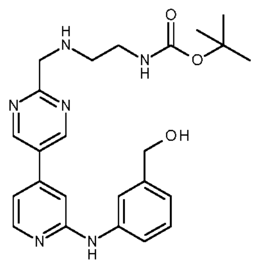

Int. 273 (2.2 g; 7.158 mmol) and tert-butyl N-(2-oxoethyl)carbamate (11.394 ml; 7.158 mmol) were stirred in DMA (50 ml) at r.t. for 30 min. The reaction mixture was added dropwise over 30 min to a solution of $NaBH(OAC)_3$ (4.551g; 21.474 mmol) in acetic acid at r.t. The reaction mixture was stirred 1 h at r.t. and was then poured out on ice/water. The water layer was stirred at r.t. for 1 h. The water layer was concentrated under reduced pressure. The residue was stirred in DIPE (250 ml). The precipitate was filtered off. The precipitate was dissolved in MeOH (150 ml). The MeOH layer was filtered through Dicalite®. The filtrate was evaporated to dryness. The residue was purified by Prep HPLC on (RP Vydac Denali C18 - 10μm, 200g, 5cm). Mobile phase (0.25 % $NH_4HCO_3$ solution in water, ACN). The desired fractions were collected and the solvent was evaporated. Yield: 600 mg of Int. 274 (18.6 %).

d) Preparation of Int. 275

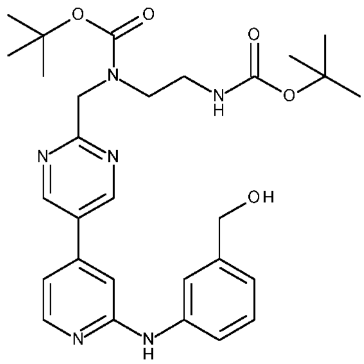

Di-tert-butyl dicarbonate (871.952 mg; 3.995 mmol) was added to Int. 274 (600 mg; 1.332 mmol) and $Et_3N$ (1.111 ml; 7.99 mmol) in DCM (13.833 ml) at r.t. The reaction mixture was stirred at r.t. for 1 h and was then concentrated. The residue was stirred in DIPE (30 ml). The DIPE-layer was decanted. The residue was dried under vacuum at 50 °C. Yield: 650 mg of Int. 275 (88.6 %).

e) Preparation of Int. 276

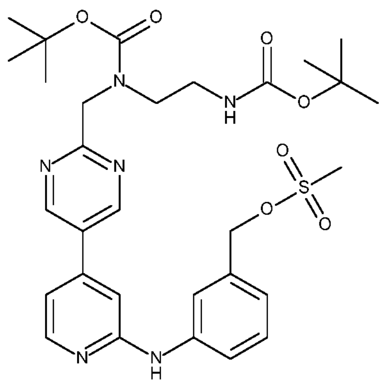

Methanesulfonyl chloride (0.274 ml; 3.541 mmol) was added dropwise to a solution of Int. 275 (650 mg; 1.18 mmol) and $Et_3N$ (0.656 ml; 4.722 mmol) in DCM (15 ml) at 0 °C. The reaction mixture was stirred for 30 min at 0 °C yielding a reaction mixture containing Int. 276 which was used as such in the next reaction step.

f) Preparation of Int. 277

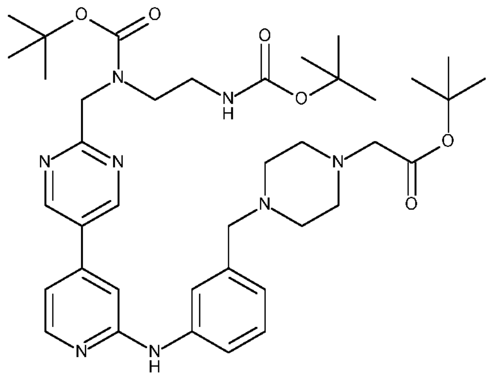

1-piperazineacetic acid, 1,1-dimethylethyl ester (1.779g; 8.26 mmol) was added to the reaction mixture of the previous step (containing Int. 276) at r.t. The reaction mixture was stirred at r.t. for 3 h. The reaction mixture was washed with water. The organic layer was separated, dried, filtered and concentrated The residue was purified by silicagel column chromatography. Eluents: DCM/MeOH // gradient 99.5/0.5 to 96/4. The pure fractions were collected and concentrated under reduced pressure yielding 582 mg of Int. 277 (67.3 %).

g) Preparation of Int. 278

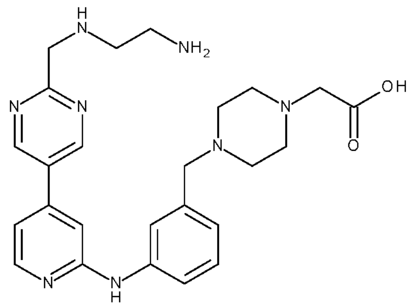

HCl salt

HCl (4 M in dioxane) (0.5 ml; 2 mmol) was added to Int. 277 (60 mg; 0.0819 mmol) in 1,4-dioxane (3.974 ml) at 60 °C. The reaction mixture was stirred at 60 °C for 2 h. The reaction mixture was concentrated under reduced pressure. The residue was two times co-evaporated with toluene (2x 50 ml) and the crude Int. 278 was used as such in the next reaction step.

Example A25a

a) Preparation of Int. 324

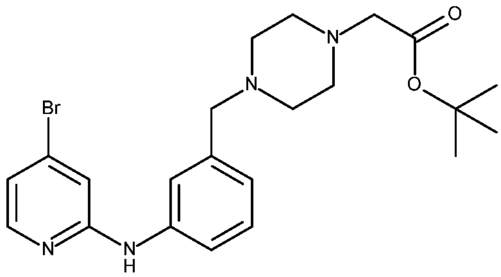

A mixture of 2-amino-4-bromopyridine(91.4 g; 528.3 mmol), 4-[(3-iodophenyl)methyl]-1-piperazinecarboxylic acid, 1,1-dimethylethyl ester (220 g; 528.3 mmol), $Pd(OAc)_2$ (3.56 g; 0.03 eq.) Xanthphos (9.15 g; 0.03eq.) and $Cs_2CO_3$ (516.1 g; 1585 mmol) were stirred in dioxane (2.21). The mixture was charged with $N_2$-gas for 15 min and then heated between 95 °C and 105 °C for 21 h. The reaction mixture was cooled, poured into water and the mixture was then extracted 3x with EtOAc. The combined organic layer was washed with brine, dried with $Na_2SO_4$ and filtered. The filtrate was evaporated and the residue was purified by column chromatography. The desired fractions were collected and the solvent was evaporated, yielding 140 g of Int. 324 (46.8 %).

b) Preparation of Int. 325

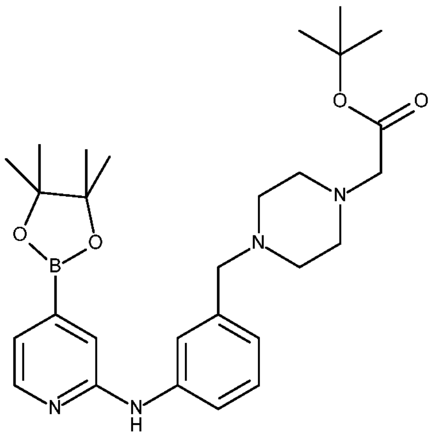

A mixture of int. 324(130 g; 281.7 mmol), 2,2'-Bi-1,3,2-dioxaborolane, 4,4,4',4',5,5,5',5'-octamethyl-(77.27 g; 281.7 mmol) and potassium acetate(96.8 g, 3 eq.) was stirred in DMF(1.3 1). The mixture was charged with $N_2$ gas for 30 minutes.

Then $Pd(dppf)_2Cl_2$ (6.186g, 0.03eq)was added and then the reaction was heated at 80°C for 12hours. The rm was cooled , poured into water and the mixture was then extracted 3 times with ethylacetate. The combined organic layer was washed with brine, dried with $Na_2SO_4$ and filtered. The filtrate was evaporated and the crude residue was recrystallized from MTBE and hexane, yielding 100 g of Int. 325 (64.9 %).

Example A25b

a) Preparation of Int. 279

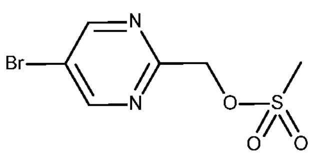

Methanesulfonyl chloride (6.14 ml; 79.4 mmol) was added dropwise to a stirred suspension of 5-bromo-2-pyrimidinemethanol (5 g; 26.45 mmol) in a mixture of DCM (300 ml) and $Et_3N$ (11ml; 79.4 mmol) at 0 °C. After addition the reaction mixture was stirred at 0 °C for 1 h. The reaction was quenched by the addition of 100 mL water. The organic layer was separated, washed with water, dried with $MgSO_4$, filtered and the solvents were evaporated. Yield: 7.82 g of Int. 279 (92.3 %) which was used as such in the next reaction step.

b) Preparation of Int. 280

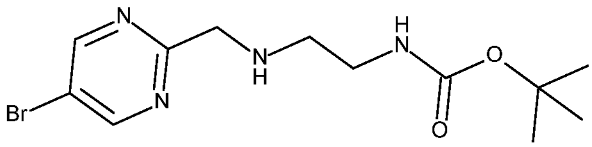

A solution of Int. 279 (7.82 g; 29.28 mmol) in ACN (20 ml) was added dropwise to a stirred suspension of tert-butyl N-(2-aminoethyl)carbamate (13.85 ml; 87.8 mmol) and $Na_2CO_3$ (3.72g; 35.1 mmol) in ACN (480 ml). After addition the reaction mixture was stirred at r.t. for 18 h. The reaction was quenched by the addition of water. DCM was added. The organic layer was separated, washed with water, dried with $MgSO_4$, filtered and the solvents were evaporated. The residue was dissolved in DCM and purified over a $SiO_2$ column, type Grace Reveleris SRC, 80 g, Si 40, on a Armen Spot II Ultimate purification system using DCM and MeOH as eluents in a gradient starting from 100 % DCM and ending with 5 % MeOH and 95 % DCM. The fractions containing product were combined and the solvents were evaporated. Yield: 4.44 g of Int. 280 (38 %).

c) Preparation of Int. 281

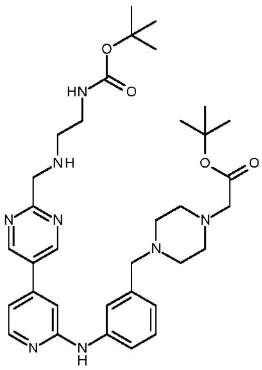

A mixture of Int. 280 (4.44g; 11.12 mmol), Int. 325 (6.22 g; 12.23 mmol), dichloro(diphenyl-phosphinoferrocene)palladium (0.814g; 1.113 mmol) and $Na_2CO_3$ (3.538 g; 33.379 mmol) in water (9.8 ml) and 1,4-dioxane (98.5 ml) was flushed through with $N_2$ gas. The reaction mixture was stirred at 80 °C for 1 h and then cooled down to room temperature. The reaction was diluted with water and the mixture was extracted twice with DCM. The organic layer was washed with water, dried with $MgSO_4$, filtered and the solvents of the filtrate evaporated. The residue was dissolved in DCM and purified over a $SiO_2$ column, type Grace Reveleris SRC, 4 g, Si 40, on a Armen Spot II Ultimate purification system using DCM and MeOH as eluents in a gradient starting from 100 % DCM and ending with 5 % MeOH and 95 % DCM. The fractions containing product were combined and the solvents were evaporated. The residue was purified by Prep HPLC (Stationary phase: RP Vydac Denali C18 - 10 μm, 200 g, 5 cm, Mobile phase: 0.25% $NH_4HCO_3$ solution in water, ACN). The desired fractions were collected, evaporated and re-purified by Prep HPLC (Stationary phase: Uptisphere C18 ODB - 10 μm, 200 g, 5 cm) (Mobile phase: gradient 0.1 % TFA aq. solution 95% / 5 % ACN to 100% ACN). Yield: 1.72 g Int. 281 (19.8 %).

d) Preparation of Int. 282

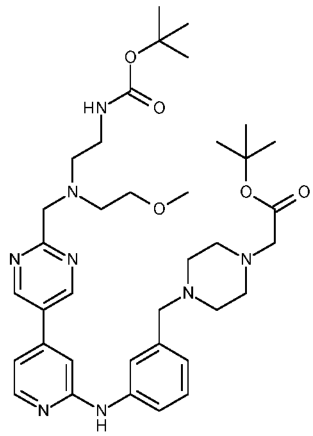

A solution of 2-bromoethyl methyl ether (0.0609 g; 0.64 mmol) in DMF (6 ml) was added dropwise to a solution of Int. 281 (0.5 g; 0.64 mmol) and DIPEA (0.44 ml; 2.56 mmol) in DMF (9 ml) at 50 °C over a period of 1 h. After addition the reaction mixture was stirred for 18 h at 50 °C. Water (50 ml) and DCM (300 ml) were added. The mixture was shaken vigorously. The organic layer was separated, dried with $MgSO_4$, filtered and the solvents were evaporated. The residue was dissolved in DCM and purified over a $SiO_2$ column, type Grace Reveleris SRC, 12 g, Si 40, on a Armen Spot II Ultimate purification system using DCM and methanol as eluens in a gradient starting from 100% dichloromethane and ending with 5 % MeOH and 95 % DCM. The fractions containing product were combined and the solvents were evaporated, yielding 70 mg of Int. 282 which was used as such in the next reaction step.

Int. 283

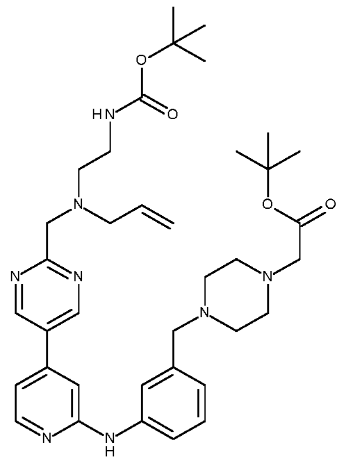

was prepared by using an analogous reaction protocol as used for Int. 282 starting from Int. 281 and allyl bromide.

Int. 284

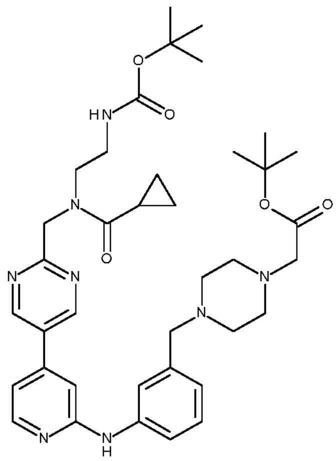

was prepared by using an analogous reaction protocol as used for Int. 282 starting from Int. 281 and cyclopropanecarbonyl chloride.

Example A26

a) Preparation of Int. 285

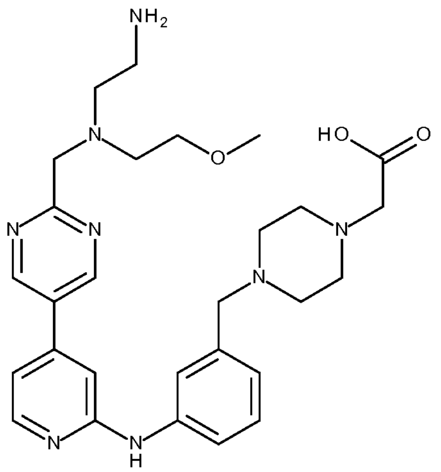

HCl salt

HCl (4 M in dioxane) (0.6 ml) was added to a stirred solution of Int. 282 (70 mg; 0.077 mmol) in 1,4-dioxane (5.9 ml) at r.t. The reaction mixture was stirred at r.t. for 2 h. The solvents were evaporated yielding 86 mg of Int. 285.

Int. 286 (used for Co. 130)

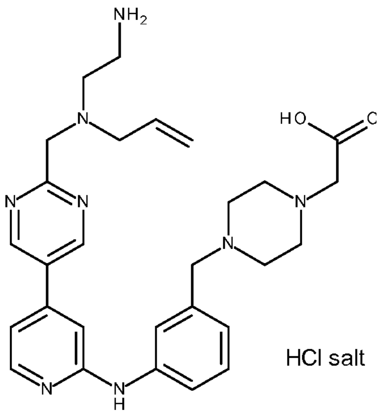

HCl salt

was prepared by using an analogous reaction protocol as used for Int. 285 starting from Int. 283.

Int. 287 (used for Co. 131)

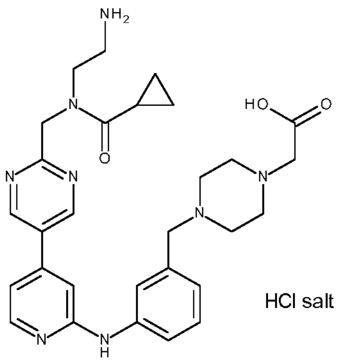

HCl salt

was prepared by using an analogous reaction protocol as used for Int. 285 starting from Int. 284.

Example A27a

a) Preparation of Int. 288

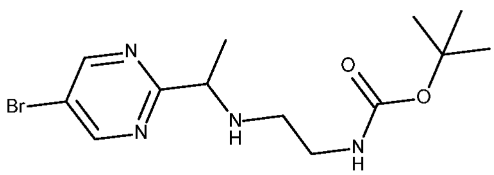

The mixture of 1-(5-bromo-2-pyrimidinyl)-ethanone (10 g; 50 mmol) and N-(2-aminoethyl)-carbamic acid, 1,1-dimethylethyl ester (8 g; 50 mmol) was stirred in TFE (60 ml). Then $NaBH_4$ (5.675g;150 mmol) was added and the mixture was stirred under r.t. After completion of the reaction, the mixture was filtered and the residue was washed with TFE (2 mL). The solvent was distilled off. The crude product was purified by column chromatography on silica gel (eluent: PE/EtOAc 2/1). The product fractions were collected and the solvent was evaporated to Int. 288 (7 g; 40 %).

b) Preparation of Int. 289

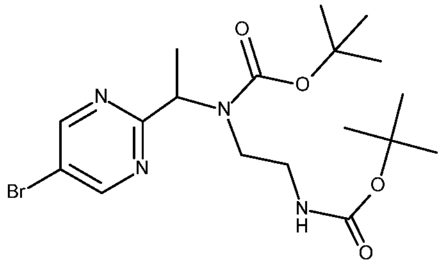

Dicarbonic acid, C,C'-bis(1,1-dimethylethyl) ester (3.42g; 15.7 mmol) was added to the mixture of Int. 288 (7 g; 20.3 mmol) in $Et_3N$ (5ml) and DCM (50 ml) at r.t. The mixture was stirred overnight. Sat. citric acid was added. The mixture was stirred for 10 min. and was then extracted with DCM. The organic layer was dried, filtered and evaporated *in vacuo.* The crude was purified by column chromatography (eluent: PE/EtOAc 4/1). The product fractions were collected and the solvent was evaporated to give 6.5 g of Int. 289 (72 %).

Example A27b

a) Preparation of Int. 290

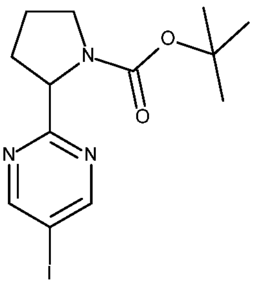

(2S)-2-Pyrrolidinecarboximidamide (10 g; 40 mmol) was dissolved in EtOH (500 ml). 3-(Dimethylamino)-2-iodo-2-propenal (10.8g; 48 mmol) and $NaHCO_3$ were added. The mixture was refluxed overnight. The mixture was concentrated. The residue was dissolved in DCM and the organic layer was then washed with brine. The organic layer was dried and concentrated. The residue was purified by chromatography over silica gel (eluent: EtOAc/PE 10/80). The desired fractions were collected and the solvent was evaporated. Yield: 5.5 g of Int. 290 (36.6 %; racemic).

b) Preparation of Int. 291

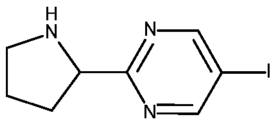

HCl salt

Int. 290 (4 g; 10. 66 mmol) was dissolved in HCl/dioxane (100 ml). The mixture was stirred at r.t. for 2h. The mixture was concentrated. The residue was stirred in methyl t-butyl ether and the solid was filtered off and dried. Yield: 3.54 g of Int. 291.

c) Preparation of Int. 292

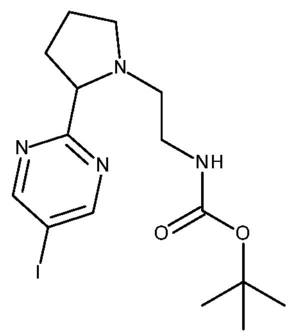

Int. 291 (2 g) was dissolved in ACN (150 ml). $K_2CO_3$ (2.21 g; 16 mmol) was added. The mixture was stirred at r.t. for 20 min. N-(2-bromoethyl)-carbamic acid, 1,1-dimethylethyl ester (2.88 g; 12.8 mmol) was added. The mixture was stirred at 50 °C overnight. The mixture was concentrated. The residue was dissolved in DCM and the organic phase was washed with brine, dried and concentrated. The residue was purified by chromatography over silica gel (eluent: EtOAc/PE 1/1). The desired fractions were collected and concentrated. Yield: 2.0g of Int. 292.

Example A28

a) Preparation of Int. 293

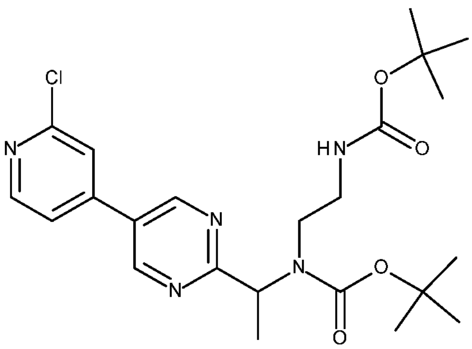

The mixture of Int. 289 (6.5 g; 14.6 mmol), B-(2-chloro-4-pyridinyl)-boronic acid (2.4 g; 15.3 mmol), $Pd(PPh_3)_4$(1.69g; 1.46 mmol) and sat. aq. $Na_2CO_3$ (20 ml) in dioxane (60 ml) was refluxed for 3 h under $N_2$ atmosphere. The resulting mixture was poured into water and the precipitate was filtered, washed with water and dried. The crude was purified by column chromatography (eluent: PE/EtOAc 3/1). The desired fractions were collected and the solvent was evaporated. Yield: 5.5 g of Int. 293 (79 %).

Int. 294

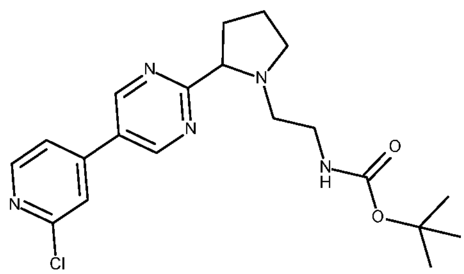

was prepared by using an analogous reaction protocol as used for Int. 293 starting from Int. 292 and B-(2-chloro-4-pyridinyl)-boronic acid.

b) Preparation of Int. 295

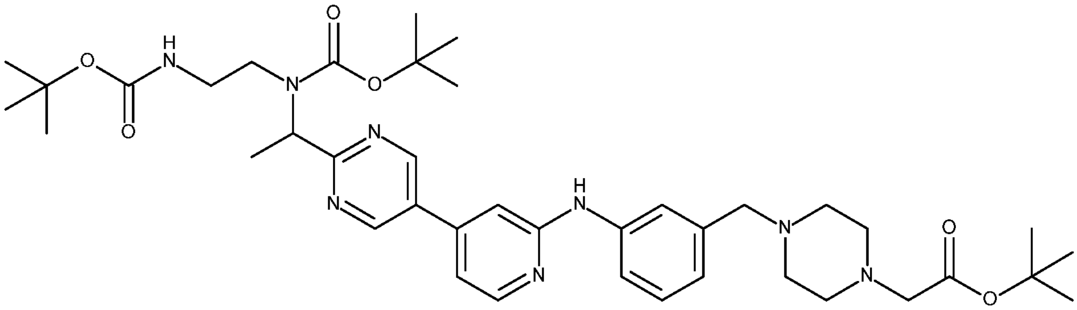

A mixture of Int. 293 (5.5 g; 11.5 mmol), Int. 41 (3.5 g; 11.5 mmol), $Pd_2dba_3$ (526.537mg; 0.575 mmol), S-phos (961.536 mg; 2.314 mmol) and $Cs_2CO_3$ (7.539g; 23.14 mmol) in dioxane (100 ml) was refluxed for 4 h under $N_2$ atmosphere. The precipitate was filtered off. The filtrate was concentrated *in vacuo.* The crude was purified by column chromagraphy (eluent: PE/EtOAc 1/3). The desired fractions were collected and the solvent was evaporated to give 6.12 g of Int. 295 (64 %).

c) Preparation of Int. 296

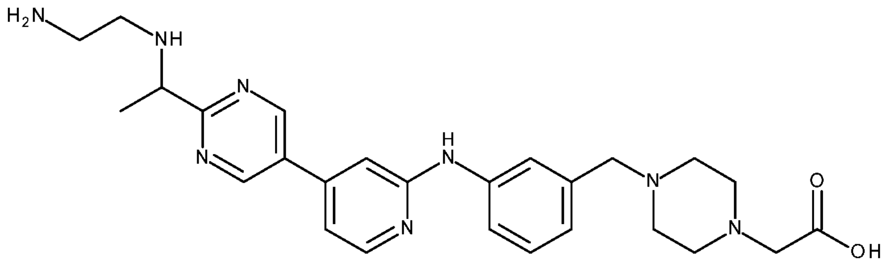

TFA salt

A mixture of Int. 295 (6.1 g; 8.03 mmol) in TFA (30 ml) and DCM (90 ml) was refluxed overnight. The solvent was removed to give 6.70 g of Int. 296.

Int. 297 (used for Co. 135)

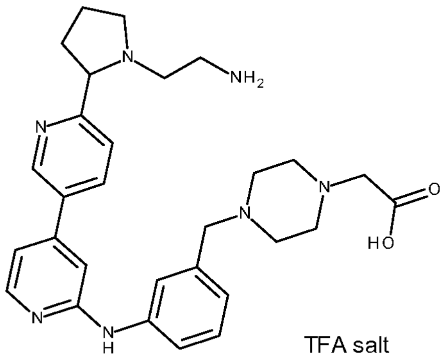

TFA salt

was prepared by using analogous reaction protocols as used for Int. 295 and Int. 296 starting from Int. 294 and Int. 41.

Example A29

a) Preparation of Int. 159

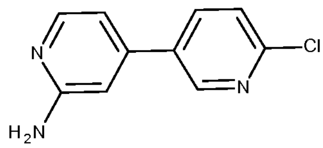

The reaction was performed in 4 batches of the same quantities.

A solution of 6-chloropyridine-3-boronic acid (5 g; 31.773 mmol), 2-amino-4-bromopyridine (5.5 g ;31.773 mmol), $K_2CO_3$ (11.9 g, 85.788 mmol), water (16 mL) in THF (50 mL) was degassed with $N_2$ flow at r.t. for 15 min. Triphenylphosphine (833 mg, 3.177 mmol) and palladium(II) acetate (214 mg, 0.953 mmol) were added and the reaction mixture was stirred at 70 °C for 6 h. The combined reaction mixtures were poured into water and EtOAc was added. The reaction mixture was filtered on a short pad of Celite®. The organic layer was washed with water then brine, dried over $MgSO_4$, filtered, and the solvent was evaporated to give a yellow solid which was stirred in a mixture of DCM/ MeOH, filtered off and dried yielding 9.9 g of Int. 159 (80 %).

b) Preparation of Int. 160

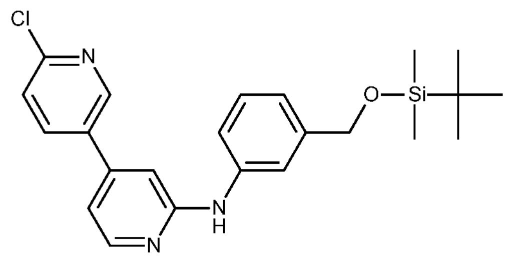

A mixture of Int. 159 (4.75 g; 18.478 mmol), 1-[[(1,1-dimethylethyl)dimethylsilyl]oxy]-3-iodo-benzene (6.4 g; 18.478 mmol), cesium carbonate (21.1 g; 64.674 mmol), 9,9-dimethyl-4,5-bis(diphenylphosphino)xanthene (1.28 g; 2.217 mmol) and palladium(II) acetate (47% Pd) (414 mg; 1.848 mmol) in dioxane (30 mL) and THF (5 mL) was stirred at 90 °C for 9 h . The reaction mixture was poured into water and DCM was added. The mixture was filtered through Celite®. The filtrate was extracted with DCM (3x).The organic layer was washed with water, dried over $MgSO_4$, filtered and evaporated to give an orange oil .The residue was purified by preparative LC on (Irregular SiOH 20-45μm 450g MATREX). Mobile phase (70% heptane, 30% EtOAc). The desired fractions were collected and the solvent was evaporated. Yield: 3.7 g of Int. 160 (47 %).

c) Preparation of Int. 161

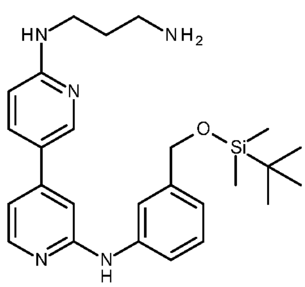

The reaction was performed in a microwave (biotage) in a sealed tube (monomode, 400W) on 3 equal quantities of Int. 160 (2 g , 4.7 mmol).

A mixture of Int. 160 (2 g; 4.7 mmol), 1,3-diaminopropane (2 mL; 23.5 mmol) in NMP (12 mL) was stirred at 170 °C for 90 min. The 3 reaction mixtures were combined and evaporated. The residue was purified by preparative LC on (Stability Silica 5μm 150 x 30.0 mm). Mobile phase (Gradient from 100 % DCM to 1 % $NH_4OH$, 85 % DCM, 14 % MeOH). The desired fractions were collected and the solvent was evaporated. Yield: 4.3 g of Int. 161 (66 %).

d) Preparation of Int. 162

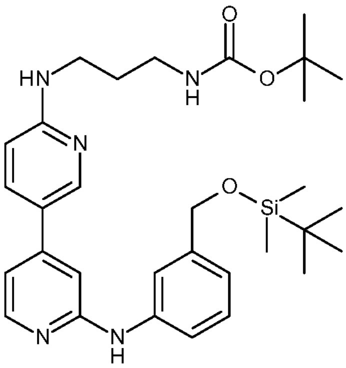

A solution of Int. 161 (4.3 g; 9.273 mmol) and $(BOC)_2O$ (3 g, 13.91 mmol) in DCM (30 mL) was stirred for 6 h at r.t..Water and DCM were added. The mixture was extracted with DCM. The organic layer was washed with brine, dried over $MgSO_4$, filtered and the solvent was evaporated The crude product was purified by preparative LC on (Stability Silica 5μm 150 x 30.0 mm). Mobile phase (Gradient) (100 % DCM to 0.1 % $NH_4OH$, 96 % DCM, 4 % MeOH). The desired fractions were collected and the solvent was evaporated. Yield: 5.3 g of Int. 162.

The intermediates in the table below were prepared by first using an analogous reaction protocol as used for Int. 161, followed by an analogous reaction protocol as used for Int. 162.

| | |
|---|---|
| 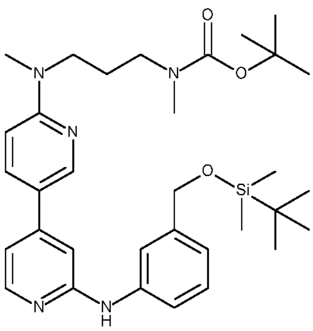 | 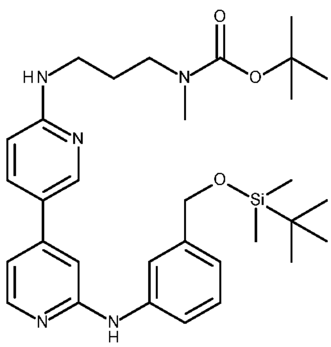 |
| Int. 163 (starting from Int. 160 and N, N'-dimethyl-1,3-propanediamine) | Int. 164 (starting from Int. 160 and N-methyl-1,3-propanediamine) |

(continued)

| | |
|---|---|
| 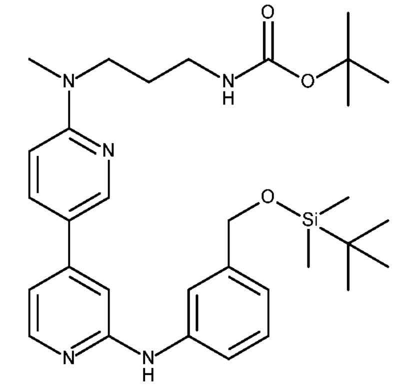 | 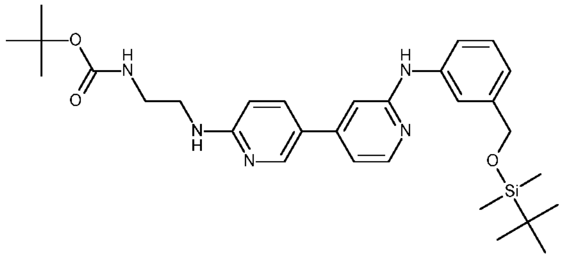 |
| Int. 165 (starting from Int. 160 and N-methyl-1,3-propanediamine) | Int. 166 (starting from Int. 160 and tert-butyl N-(2-aminoethyl)carbamate) |

Example A30

a) Preparation of Int. 167

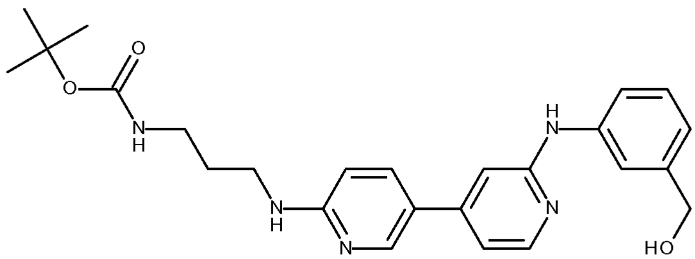

Tetrabutylammonium fluoride 1 M (10.43mL, 10.34 mmol) was added dropwise to a solution of a mixture of Int. 162 (5.3 g) in THF (120mL) at r.t. and stirred overnight. Water was added and the organic solvent was evaporated. The mixture was extracted with DCM. The organic layer was washed with water, dried over $MgSO_4$, filtered and evaporated. The crude product was purified by preparative LC on (Stability Silica 5 μm 150 x 30.0 mm). Mobile phase (Gradient) (98 % DCM, 2 % MeOH to 0.5 % $NH_4OH$, 90 % DCM, 10 % MeOH). The desired fractions were collected and the solvent was evaporated to give 2.1 g of Int. 167 (50 %).

b) Preparation of Int. 168

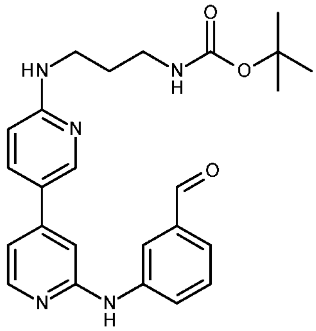

A solution of Int. 167 (2.1 g; 3.503 mmol) in DCM (40 mL) was stirred at ambient temperature and manganese

dioxide (16.8 g; 192.87 mmol) was added. The suspension was stirred at r.t. overnight. The reaction mixture was filtered through a pad of Celite®, the residue was washed with DCM and the filtrate was evaporated to give Int. 168 (1.32 g; 84%).

c) Preparation of Int. 169

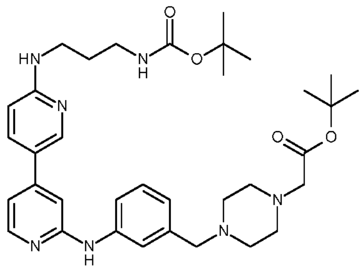

The reaction was performed in a microwave (biotage) in a sealed tube (monomode, 400W).

Sodiumacetoxyborohydride (938 mg, 4.424 mmol) was added to a stirred solution of Int. 168 (1.32 g, 2.949 mmol) and 1-piperazineacetic acid, 1,1-dimethylethyl ester (1.18 g, 5.899 mmol) in DCM (16 mL) and DIPEA (1 mL, 5.899 mmol). The mixture was stirred at 120 °C for 20 min. Water, $K_2CO_3$ 10 % and DCM were added. The reaction mixture was extracted 3 times with DCM. The organic layer was separated, dried over $MgSO_4$, filtered and the solvent was evaporated. The crude product was stirred in a mixture of ACN and DIPE and the precipitate was filtered off and dried to give Int. 169 (1.5 g; 80%).

d) Preparation of Int. 170

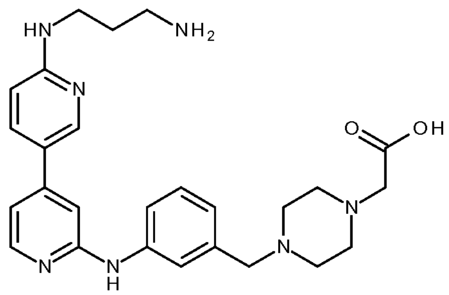

HCl salt

HCl (37 % in $H_2O$) (991 µL; 11.87 mmol) and water (3.2 mL) were added to a solution of Int. 169 (1.5g; 2.374 mmol) in dioxane (40 mL). The reaction mixture was stirred at 100 °C for 2 h. The solution was evaporated under reduced pressure to give 1.9 g Int. 170 as a yellow oil The crude product was used as such without further purification for the next reaction step.

The intermediates in the table below were prepared by using successively analogous reaction protocols as used for Int. 167, Int. 168, Int. 169 and Int. 170.

| | |
|---|---|
| 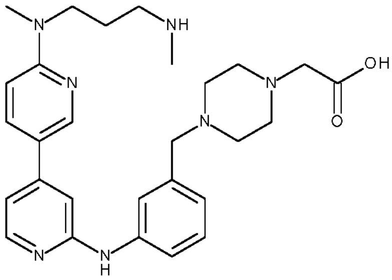<br>HCl salt<br>Int. 171 (starting from Int. 163 and 1-piperazineacetic acid, 1,1-dimethylethyl ester; used for Co. 68) | 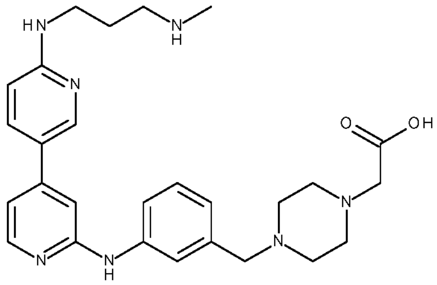<br>HCl salt<br>Int. 172 (starting from Int. 164 and 1-piperazineacetic acid, 1,1-dimethylethyl ester; used for Co. 69) |

| | |
|---|---|
| 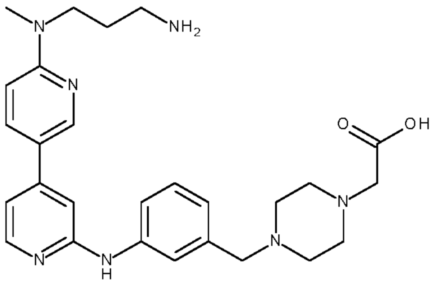<br>HCl salt<br>Int. 173 (starting from Int. 165 and 1-piperazineacetic acid, 1,1-dimethylethyl ester; used for Co 72) | 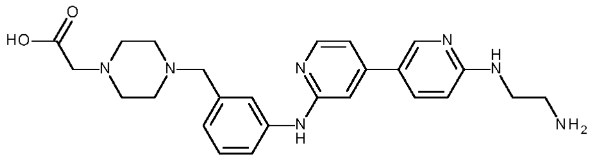<br>TFA salt<br>Int. 174 (starting from Int. 166 and 1-piperazineacetic acid, 1,1-dimethylethyl ester; used for Co. 70) |
| 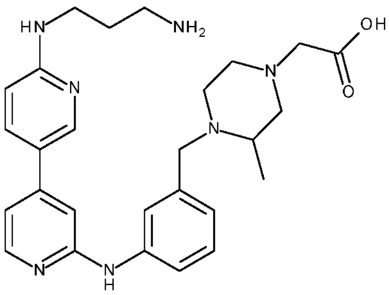<br>HCl salt<br>Int. 175 (starting from Int. 162 and 3-methyl-1-piperazineacetic acid, 1,1-dimethylethyl ester; used for Co. 71) | |

Example A31

a) Preparation of Int. 298

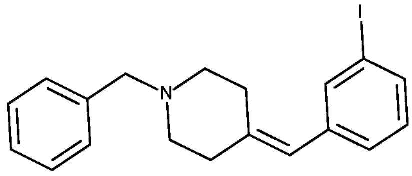

A suspension of [(3-iodophenyl)methyl]triphenyl-phosphonium bromide (29.3 g; 52.39 mmol), 1-benzyl-4-piperidone (9.4 mL; 52.39 mmol) and $K_2CO_3$ (11.6 g; 83.83 mmol) in iPROH (229 ml) was heated under reflux for 24 h After cooling to r.t., water and DCM were added. The organic layer was separated, dried over $MgSO_4$, filtered and the solvent was evaporated. The residue was purified by preparative LC on (Irregular SiOH 20-45μm 450g MATREX). Mobile phase (80% Heptane, 20% EtOAc). The desired fractions were collected and the solvent was evaporated. Yield: 7.8 g of Int. 298 as a yellow oil (38 %).

b) Preparation of Int. 299

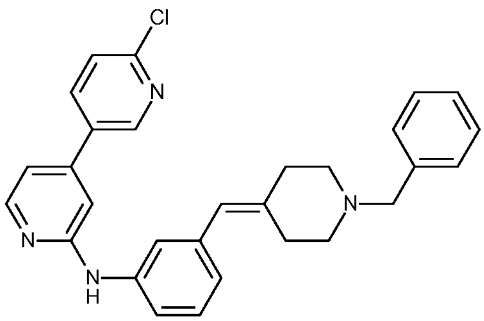

A mixture of Int. 159 (2 g; 9.73 mmol), Int. 298 (3.8 g; 9.73 mmol), cesium carbonate (11 g; 34.04 mmol), 9,9-dimethyl-4,5-bis(diphenylphosphino)xanthene (675 mg; 1.17 mmol) and Pd(II) acetate (218 mg; 0.97 mmol) in dioxane (12.5 mL) and THF (2 mL) was stirred at 90 °C overnight After cooling to r.t., water was added and the reaction mixture was extracted with DCM. The organic layer was dried over $MgSO_4$, filtered and evaporated. The residue was purified by preparative LC on (Irregular SiOH 20-45μm 450g MATREX). Mobile phase (2 % MeOH, 60 % heptane, 38 % EtOAc). The desired fractions were collected and the solvent was evaporated. Yield: 2.08 g of Int. 299 as a yellow oil (27.5 %).

c) Preparation of Int. 300

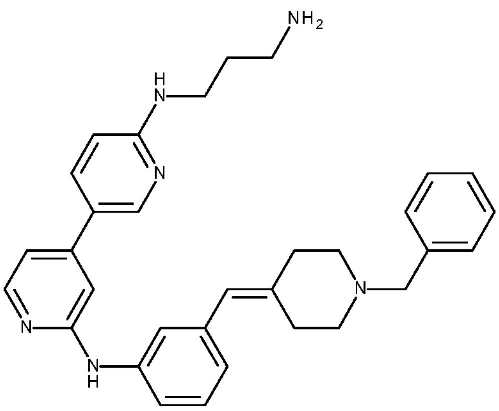

Int. 299 (1.96 g; 2.52 mmol) and 1,3-diaminopropane (14.3 ml; 12.59 mmol) in NMP (2 ml) were stirred at 140 °C for 4 h .Water was added. The precipitate was filtered off and dried yielding 2.38 g of crude intermediate. Part of the crude (100 mg) was purified by preparative LC (Stability Silica 30-45μm, 10g, Mobile phase Gradient (from 100% DCM

to 95% DCM, 5% MeOH, 0.1% $NH_4OH$)). The pure fractions were collected and the solvent was evaporated. This residue was repurified by preparative LC on (irregular 15-40μm 30g Merck). Mobile phase (1% $NH_4OH$, 84% DCM, 15% MeOH). The desired fractions were collected and the solvent was evaporated, yielding a colourless oil which was freeze-dried with dioxane yielding 42 mg of Int. 300 as a white solid.

d) Preparation of Int. 301

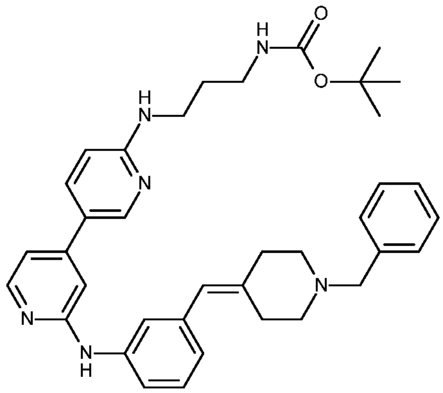

Dicarbonic acid, C,C'-bis(1,1-dimethylethyl) ester (862 mg; 3.95 mmol) was added to a solution of Int. 300 (2.06 g, 3.59 mmol) in DCM (14 mL) at 0 °C. The reaction mixture was stirred at r.t. for 1 h. Water and DCM were added. A precipitate was filtered off. The filtrate was separated and the water layer was further extracted with DCM. The organic layer was washed with brine, dried over $MgSO_4$, filtered and the solvent was evaporated. The residue was purified by preparative LC on (irregular SiOH 15-40μm 300g MERCK). Mobile phase (40 % Heptane, 8 % MeOH, 52 % EtOAc). The desired fractions were collected and the solvent was evaporated, yielding 600 mg of Int. 301 as a colourless oil (28 %).

e) Preparation of Int. 302

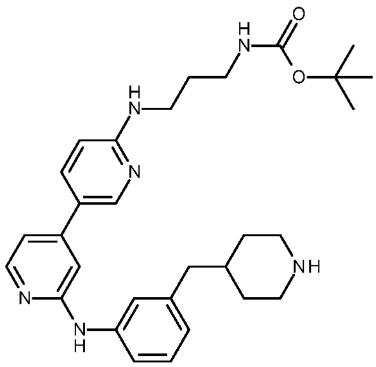

A mixture of Int. 301 (436 mg; 0.72 mmol) was hydrogenated at 50 °C in MeOH (5 mL) with Pd/C (10 %) (100 mg) as catalyst at 3 bars of $H_2$ gas atmosphere in a pressure vessel reactor for 5 h. The catalyst was filtered off on a pad of Celite®. Celite® was washed with a mixture of DCM/MeOH (3x). The filtrate was evaporated and the residue was purified by preparative LC on (Stability Silica 5μm 150x30.0mm). Mobile phase (Gradient from $NH_4OH$/DCM/ MeOH 0.2/98/2 to $NH_4OH$/ DCM/MeOH 1.8/82/18). The desired fractions were collected and the solvent was evaporated, yielding 150 mg of Int. 302 as a colourless oil (40%).

f) Preparation of Int. 303

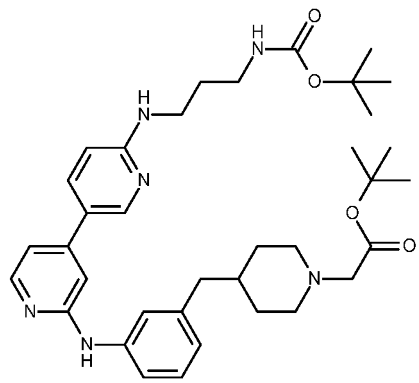

Tert-butyl bromoacetate (41 μL; 0.28 mmol) was added dropwise to a solution of Int. 302 (144 mg; 0.28 mmol) and $K_2CO_3$ (58 mg; 0.42mmol) in DMF (615 μL) at r.t. The reaction mixture was stirred at r.t. for 90 min. Water and EtOAc were added. The mixture was extracted with EtOAc (3x). The organic layer was washed with brine, dried over $MgSO_4$, filtered and the solvent was evaporated. The residue was purified by preparative LC on (Stability Silica 5μm 150x30.0mm). Mobile phase (Gradient from $NH_4OH$/DCM/MeOH 0/100/0 to $NH_4OH$/ DCM/ MeOH 0.8/92/8). The desired fractions were collected and the solvent was evaporated, yielding 85 mg of Int. 303 as a yellow oil (48 %).

g) Preparation of Int. 304

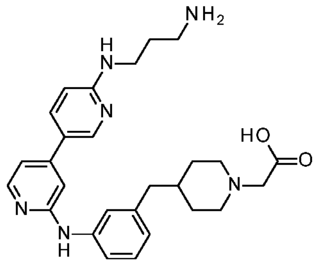

HCl salt

HCl (37 % in $H_2O$) (46 μL; 0.55 mmol) and water (0.6 mL) were added to a solution of Int. 303 (81 mg; 0.11 mmol) in dioxane (3.2 ml). The reaction mixture was stirred at 100 °C for 2 h. The solution was evaporated under reduced pressure. The residue was dried *in vacuo* at 70 °C yielding 108 mg of Int. 304 as a yellow oil, used as such in the next reaction step.

Example A32

a) Preparation of Int. 305

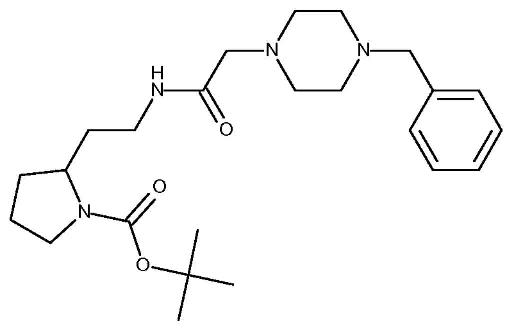

Chloroacetyl chloride (723 μL; 9.1 mmol) in ACN (6 mL) was added dropwise to a stirred solution of 2-(aminoethyl)-1-N-boc-pyrrolidine (1.5 g; 7 mmol) and $Et_3N$ (1.9 mL; 14 mmol) in ACN (18 ml) at r.t. The reaction mixture was stirred for 1 h. 1-benzyl piperazine (3.7 g; 21 mmol) was added and the reaction mixture was stirred at 60 °C for 2 h. Water was added and the reaction mixture was extracted with DCM. The organic layer was dried over $MgSO_4$, filtered and evaporated. The residue was purified by preparative LC on (Irregular SiOH 20-45μm 450g MATREX). Mobile phase (Gradient from 40 % Heptane, 7 % MeOH, 53 % EtOAc to 40 % heptane, 10 % MeOH, 50 % EtOAc). The pure fractions were collected and the solvent was evaporated. Yield: 2.3 g of Int. 305.

b) Preparation of Int. 306

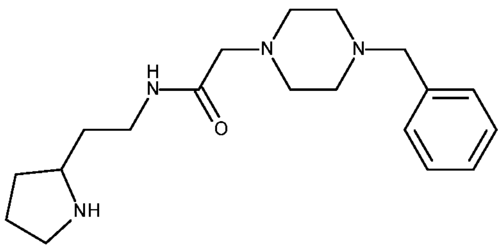

TFA (8 mL; 107 mmol) was added to a solution of Int. 305 (2.3 g; 5.3 mmol) in DCM (40 mL) at 0-5 °C. The reaction mixture was stirred at r.t. for 4 h. TFA (8 mL; 107 mmol) was added. The reaction mixture was stirred for 24 h. Water and $K_2CO_3$ were added. The mixture was extracted with DCM, dried over $MgSO_4$, filtered and evaporated to give 1.9 g of Int. 306.

Example A33

a) Preparation of Int. 307

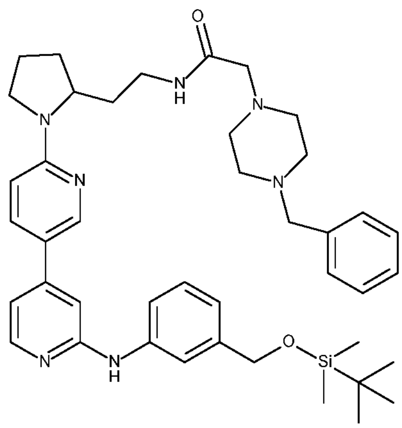

Int. 160 (700 mg; 1.6 mmol), Int. 306 (1 g; 3 mmol) and $K_2CO_3$ (1.1 g; 8.2 mmol) in DMF (3 mL) were stirred at 100 °C for 2 days. Water was added and the reaction mixture was extracted with DCM. The organic layer was dried over $MgSO_4$, filtered and evaporated. The residue was purified by preparative LC on (irregular SiOH 15-40 μm 40 g). Mobile phase (from 100 % DCM to $NH_4OH$/ DCM/MeOH 0.5/90/10). The pure fractions were collected and the solvent evaporated. Yield: 570mg of Int. 307 (48.2 %).

b) Preparation of Int. 308

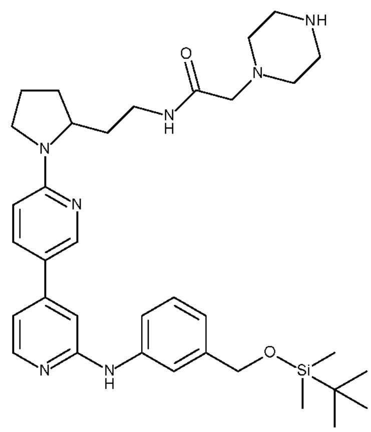

A mixture of Int. 307 (570 mg; 0.79 mmol) was hydrogenated at 50 °C in MeOH (10 ml) with Pd/C 10 % (55 mg) as catalyst at 5 bars of $H_2$ gas in a pressure vessel reactor for 24 h . The catalyst was filtered off on a pad of Celite®. Celite® was washed with a mixture of DCM/MeOH (3x). The filtrate was evaporated to give 474 mg of Int. 308 (oily; 95.2 %).

c) Preparation of Int. 309

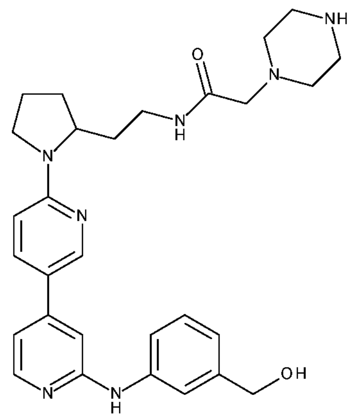

Tetrabutylammonium fluoride 1 M (1.5 mL; 1.51 mmol) was added dropwise to a solution of Int. 308 (474 mg; 0.75 mmol) in THF (10 ml) at r.t. The reaction mixture was stirred at r.t. for 3 h. Water was added and the THF was evaporated. The water layer was extracted with DCM. The organic layer was washed with water, dried over $MgSO_4$, filtered and the solvent was evaporated. The residue was purified by preparative LC on (Stability Silica 5μm 150 x 30.0 mm). Mobile phase (Gradient from $NH_4OH$/DCM/MeOH 0.5/95/5 to $NH_4OH$/DCM/MeOH 1.8/82/18). The desired fractions were collected and the solvent was evaporated. Yield: 167 mg of Int. 309 as a colourless oil (40 %).

d) Preparation of Int. 310

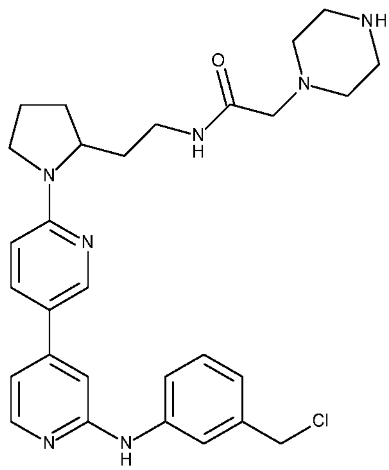

HCl salt

$SOCl_2$ (1.09 mL; 14.90 mmol) was added dropwise to a stirred solution of Int. 309 (167 mg; 0.30 mmol) in DCE (39 ml) at r.t. The reaction mixture was stirred at 60 °C for 6 h. The solvent was evaporated to dryness yielding 249 mg of crude Int. 310 which was used as such in the next reaction step.

Example A34

a) Preparation of Int. 311

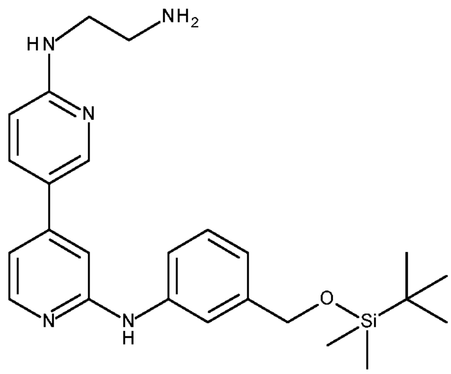

A mixture of Int. 160 (700 mg; 1.6 mmol) and ethylenediamine (1.1 mL; 16 mmol) in a sealed tube was heated at 170 °C using one single mode microwave (Biotage Initiator EXP 60) with a power output ranging from 0 to 400 W for 90 min. The mixture was poured into water. The gum was decanted, taken up into DCM/MeOH 95/5, dried over $MgSO_4$ and evaporated, yielding 740 mg of Int. 311 (oily).

b) Preparation of Int. 312

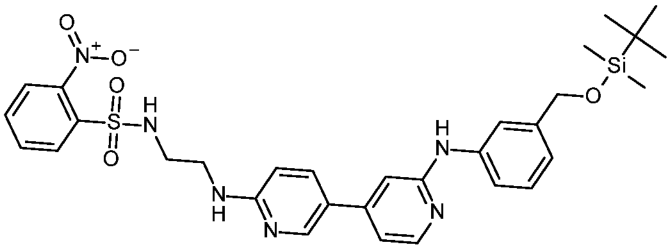

2-Nitrobenzenesulfonyl chloride (401 mg; 1.81 mmol) in DCM (10 mL) was added dropwise to a mixture of Int. 311 (740 mg; 1.65 mmol) and $Et_3N$ (0.34 mL; 2.5 mmol) in DCM (45 mL) at r.t. The reaction mixture was stirred for 1 h. Water was added. The organic layer was separated and dried over $MgSO_4$, filtered and evaporated. The residue was purified by preparative LC on (irregular SiOH 15-40μm 300g MERCK). Mobile phase ($NH_4OH$, DCM, MeOH 0.1/97.5/2.5 The pure fractions were combined and the solvent was evaporated to give 760mg (72.7 %) of Int. 312 as a brown foam.

c) Preparation of Int. 313

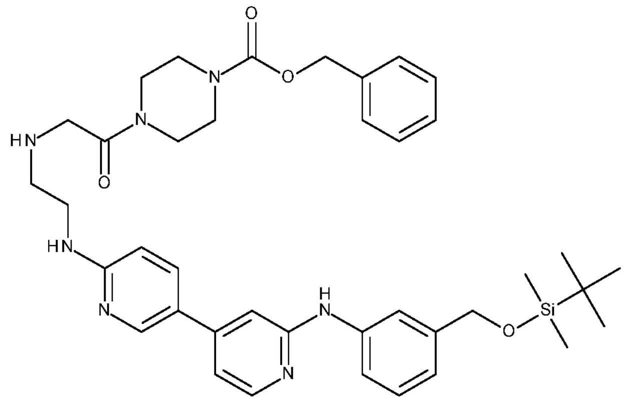

Int. 312 (580 mg; 0.91 mmol), 4-(2-chloroacetyl)-1-piperazinecarboxylic acid, phenylmethyl ester (0.44 g; 1.2 mmol) and $K_2CO_3$ (0.25 g; 1.8 mmol) in DMF (9 mL) were stirred at r.t. for 90 min. Thiophenol (0.28 mL; 2.7 mmol) was added and the mixture was stirred at r.t. for 3 h. Water was added and the reaction mixture was extracted twice with EtOAc. The combined organic layers were washed with water, dried over $MgSO_4$, filtered and evaporated. The residue was purified by preparative LC on (irregular SiOH 15-40 μm 300 g MERCK). Mobile phase ($NH_4OH$/DCM/MeOH 0.1/93/7). The pure fractions were combined and the solvent was evaporated to give 380 mg of Int. 313 (58.5 %).

d) Preparation of Int. 314

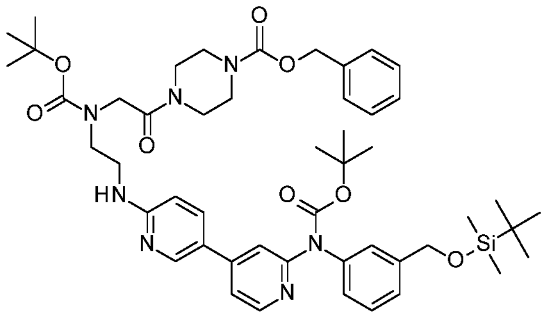

Int. 313 (370 mg; 0.52 mmol) and di-tert-butyl dicarbonate (227 mg; 1 mmol) in DCM (10 mL) were stirred at r.t. overnight. The solvent was evaporated. The residue was purified by preparative LC (Stability Silica 5μm 150 x 30.0 mm, mobile phase Gradient from pure DCM, to DCM/MeOH/$NH_4OH$ 85/15/0.5). The pure fractions were collected and the solvent evaporated yielding 380mg of Int. 314 (80.1 %).

e) Preparation of Int. 315

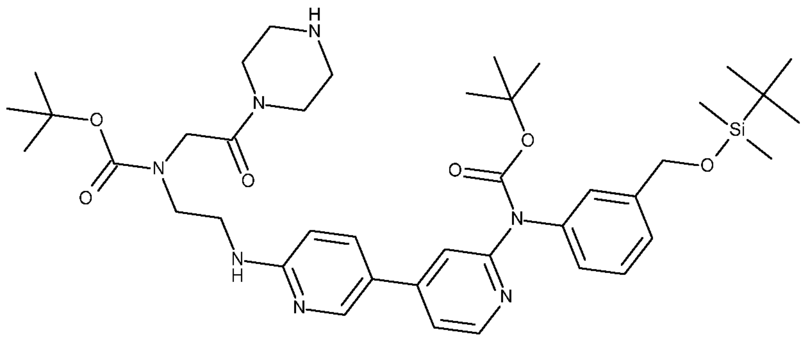

A solution of Int. 314 (380 mg; 0.42 mmol) in MeOH (12 mL) was hydrogenated at r.t. with Pd/C (35 mg) as a catalyst at atmospheric pressure of $H_2$ gas. The reaction mixture was stirred at r.t. for 12 h. The catalyst was filtered off on a pad of Celite®. Celite® was washed with DCM/MeOH. The filtrate was evaporated yielding 260 mg of Int. 315 (oily).

f) Preparation of Int. 316

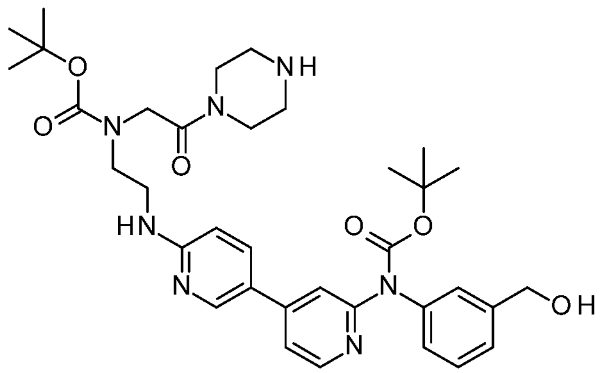

Tetrabutylammonium fluoride 1 M (0.67 mL; 0.67 mmol) was added dropwise to a solution of Int. 315 (0.26 g; 0.34 mmol) in THF (10 mL) at r.t. The reaction mixture stirred at r.t. for 1 h. Water was added and THF was evaporated. The mixture was extracted with DCM. The organic layer was washed with water, dried over $MgSO_4$, filtered and the solvent was evaporated. The residue was purified by preparative LC on (irregular SiOH 15-40μm 24g). Mobile phase (from pure DCM to $NH_4OH$/DCM/MeOH 0.5/82/20). The pure fractions were collected and the solvent evaporated until dryness to give 150 mg of Int. 316 (67.6 %).

g) Preparation of Int. 317

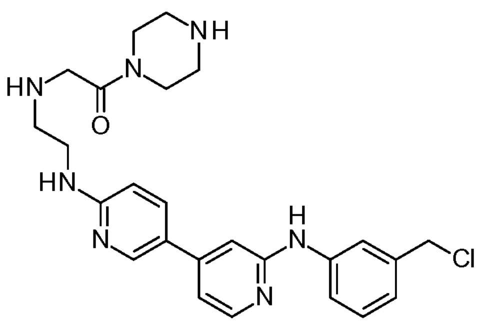

HCl salt

$SOCl_2$ (827 μL; 11.3 mmol) was added dropwise to a stirred solution of Int. 316 (150 mg; 0.23 mmol) in DCE (25 mL) at r.t. The reaction mixture was stirred at 60 °C for 3 h. The solvent was evaporated to dryness yielding 133mg

of crude Int. 317 which was used as such without purification for the next reaction step.

Example A35

a) Preparation of Int. 318

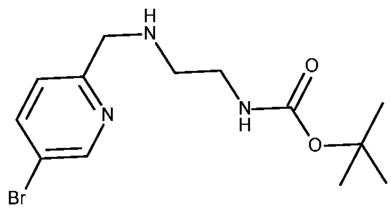

*N*-(2-aminoethyl)-carbamic acid, 1,1-dimethylethyl ester (17.73 g; 110.64 mmol) and $MgSO_4$ (19.025g; 158.06 mmol) were added to a solution of 5- bromo-2-pyridine carboxaldehyde (20 g ;105.37 mmol) in DCM(500 ml). The reaction mixture was stirred 1 h at r.t. under $N_2$ atmosphere. $NaBH(OAc)_3$ (29.033 g;136.985 mmol) was added portionwise. The reaction mixture was stirred overnight. Water was added and the organic layer was collected, dried and evaporated. The crude was purified by column chromatography over silica gel (eluent: DCM/MeOH 9/1). The desired fractions were collected and the solvent was evaporated. Yield: 18.3 g of Int. 318 (51.5 %).

b) Preparation of Int. 319

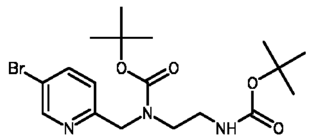

dicarbonic acid, C,C-bis(1,1-dimethylethyl) ester (267.174 g;1224.18mmol) ester was added to a solution of Int. 318 (210 g; 489.672 mmol) in DCM (1500 ml). The reaction mixture was stirred overnight at r.t. Water was added and the organic layer was separated, dried and evaporated. The crude intermediate was stirred with tert-butyl methyl ether, filtered, and the solid was dried. Yield: 126 g of Int. 319 (58 %).

c) Preparation of Int. 320

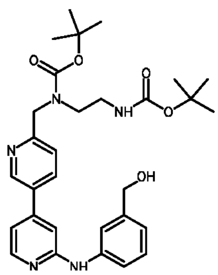

A mixture of Int. 319 (10 g;23.238 mmol), Int. 249 (6.8 g;25.076 mmol), $PdCl_2dppf$ (1.7 g; 2.323 mmol) and $Na_2CO_3$ (7.37 g; 69.535 mmol) in dioxane(225 ml) and water (75 ml) was stirred at 90 °C for 3 h under $N_2$ flow. The mixture was filtered. The filtrate was concentrated. The residue was purified by chromatography over silica gel (eluent: EtOAc/PE 1/1). The desired fractions were collected and the solvent was evaporated. Yield: 11.5 g of Int. 320 (90 %).

d) Preparation of Int. 321

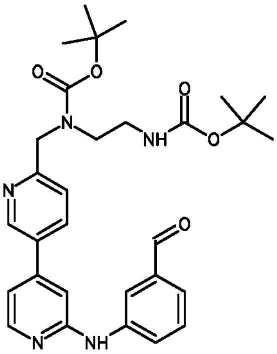

Int 320 (21.5 g;39.115 mmol) was dissolved in DCM (500 ml). $MnO_2$ (28 g; 322.061 mmol) was added. The mixture was stirred at r.t. overnight. The mixture was refluxed for 4 h. The mixture was filtered. The filtrate was concentrated. Yield: 19 g of Int. 321 (88.6 %).

e) Preparation of Int. 322

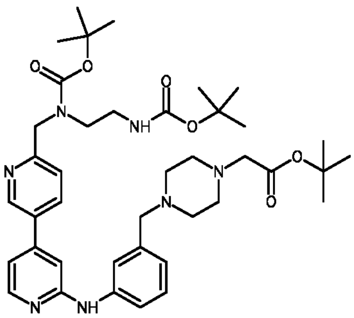

Int. 321 (19 g;34.694 mmol) was dissolved in DCE (300 ml). 1-piperazineacetic acid, 1,1-dimethylethyl ester (11.78 g;58.818 mmol) and acetic acid (4.24 g; 70.605 mmol) were added. The mixture was refluxed for 6 h and then cooled to r.t. $NaBH(OAc)_3$ (10 g; 47.183 mmol) was added. The mixture was stirred at r.t. overnight. The mixture was treated with water and extracted with DCM. The organic phase was separated, washed with brine, dried and concentrated. The crude intermediate was used directly for the next reaction step without further purification. Yield: 33 g of Int. 322.

e) Preparation of Int. 323

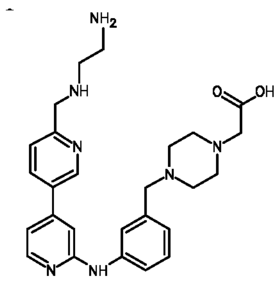

TFA salt

Int. 322 (24 g; 32.79 mmol) was dissolved in TFA (70 ml) and DCM (200 ml). The mixture was stirred at r.t.

overnight. The mixture was concentrated to give 15 g of crude intermediate 323 which was directly used as such for the next reaction step.

B. Preparation of the final compounds

Example B 1

Preparation of Compound 1

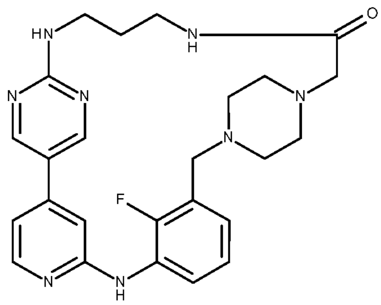

DECP (0.73 ml; 4.88 mmol) was added to a solution of Int. 39 (1.211 g) and $Et_3N$ (0.679 ml; 4.88 mmol) in DMF (72.477 ml) at r.t. The r.m. was stirred at r.t. for 16 h. The reaction mixture was concentrated under reduced pressure. The residue was dissolved in 100 ml water. The water layer was alkalified with sat. $NaHCO_3$ solution (aqueous). The water layer was extracted with DCM (2x 50 ml). The organic layer was dried, filtered and evaporated and the residue was purified by Prep HPLC on (RP Vydac Denali C18 - 10 μm, 200 g, 5 cm). Mobile phase (0.25 % $NH_4HCO_3$ solution in water, ACN). The desired fractions were collected, evaporated, solved in MeOH and evaporated again. Yield: 103 mg of compound 1.

Example B2

Preparation of Compound 33

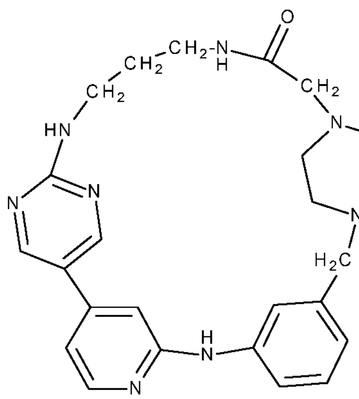

Diethyl cyanophosphonate (1.039 ml; 6.254 mmol) was added to a stirred solution of Int. 90 (3.17 g) and $Et_3N$ (8.694ml; 62.545 mmol) in DMF(140 ml) at r.t.. The reaction mixture was stirred at r.t. for 18 h. A saturated aqueous $NaHCO_3$ solution was added to the reaction mixture. This mixture was stirred for 10 min and was then diluted with water and a mixture of 10 % MeOH and 90 % DCM. The organic layer was separated. The water layer was extracted two additional times with a mixture of 10 % MeOH and 90 % DCM. The combined organic layer was washed with water, dried with $MgSO_4$, filtered and the solvents of the filtrate evaporated The residue was purified by Prep HPLC on (RP Vydac Denali C18 - 10μm, 200g, 5cm). Mobile phase (0.5 % $NH_4Ac$ solution in water + 10 % ACN, ACN), The combined fractions were alkalized with ammonia and evaporated till water. The precipitate was filtered off and washed with water.

Yield: 152 mg of compound 33.

Example B3

Preparation of Compound 43

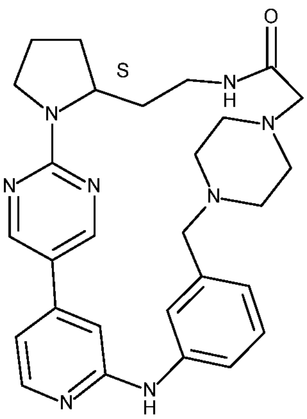

A solution of Int. 129 (1.13 g; 2.20 mmol) in DMF (50 ml) was added dropwise to a stirred solution of DECP (1.80 ml; 11.0 mmol) and DIPEA (5 ml) in DMF (50 ml) at r.t. under $N_2$ atmosphere over a period of 1 h. The solvent was evaporated. The crude compound was purified by high-performance liquid chromatography (Column: synergi 20*250mm, Mobile Phase A: Purified water (containing 0.1 % TFA), Mobile Phase B: ACN, Gradient: 0-25% (% B). A $NaHCO_3$ solution was added to adjust the pH >7. The solvent was concentrated and extracted with EtOAc (3x100 ml). The desired organic layers were washed with brine, dried over $Na_2SO_4$, filtered and the solvent was evaporated *in vacuo.* Yield: Compound 43 (0.4427 g; 40 %).

Example B4

Preparation of Compounds 77 and 78

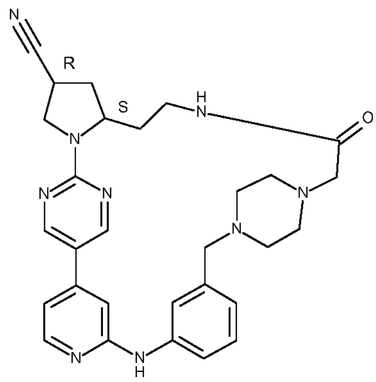

Compound 77

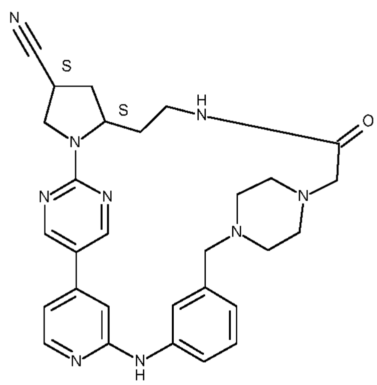

Compound 78

Int. 190 (118.5 mg; 0.2 mmol), NaCN (100 mg; 2.041mmol) and DMSO (2 ml) were stirred at 90 °C for 16 h. The reaction mixture was cooled, poured into water and extracted with EtOAc. The organic layer was dried with $MgSO_4$, filtered and evaporated. The residue (150 mg) was purified by Prep HPLC on (RP Vydac Denali C18 - 10 μm, 200 g, 5 cm). Mobile phase (0.25 % $NH_4HCO_3$ solution in water, ACN). The desired fractions were collected, evaporated, solved in MeOH and evaporated again. This second residue still contained 2 isomers. The second residue was purified by Prep SFC (Stationary phase: Chiralpak Diacel AD 30 x 250 mm), Mobile phase: CO2, EtOH with 0.2 % $iPrNH_2$). The desired

fractions were collected, evaporated, solved in MeOH and evaporated again, yielding 1 mg of Compound 78 (1%) and 18 mg of Compound 77 (17 %).

Example B5

Preparation of Compound 89

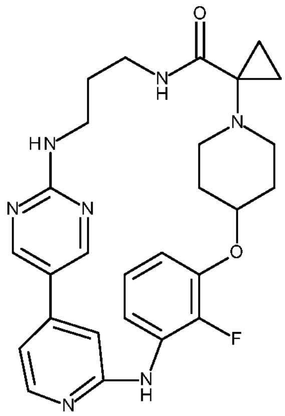

A solution of Int. 205 (1.406 g) in DMF (70 ml) was added dropwise to a stirred solution of DECP (1.71 g; 10.5 mmol) and DIPEA (2.71 g) in DMF (80 ml) at r.t. under $N_2$ atmosphere over a period of 1 h. The reaction mixture was filtered and the filtrate was evaporated and the solid was purified by high-performance liquid chromatography (Column: Gemini 150*30mm, 5 μm, Flow rate: 35ml/min, Mobile Phase A: Purified water (containing 0.1% TFA), Mobile Phase B: ACN, Gradient: 12-42% (%B). $NaHCO_3$ solution was added to adjust the pH>7. This solvent was concentrated and the precipitate was filtered off and dried. Yield: 217.90 mg of Compound 89.

Example B5a

Preparation of Compound 98

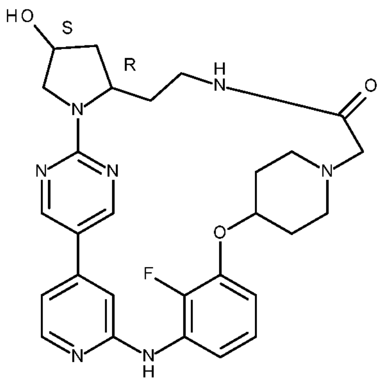

Int. 215a (0.4 g; 0.309 mmol) in IN HCl (20 ml) was stirred at r.t. for 30 min.
The aqueous layer was washed with DCM. A $NaHCO_3$ solution was added to the aqueous layer to adjust the pH to 7-8. The aqueous layer was extracted with DCM and the organic layer was dried, filtered and evaporated. The crude was purified by high-performance liquid chromatography (Column: Gemini 250*20mm, 5 μm, Flow rate: 25ml/min, Mobile Phase A: Purified water (containing 0.1% TFA), Mobile Phase B: ACN, Gradient: 2-32% (%B). A $NaHCO_3$ solution was added to adjust the pH>7. This solvent was concentrated and the precipitate was filtered off, washed with water and dried *in vacuo.* Yield: 46.50 mg of Compound 98 (28.1 %).

Example B6

Preparation of Compound 100

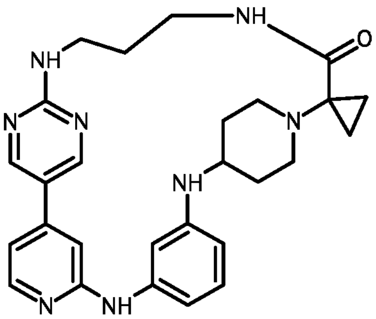

A solution of Int. 232 (480.99 mg) in DMF (50 ml) was added dropwise to a stirred solution of DECP (780.5 mg; 4.785 mmol) and DIPEA (1.237 g; 9.57 mmol) in DMF (50 ml) at room temperature under $N_2$ atmosphere over a period of 1 h. The solvent was evaporated. The crude was purified by HPLC (Column: Synergi 150*30mm, 5μm, Flow rate: 30ml/min, Mobile Phase A: Purified water (containing 0.1% TFA), Mobile Phase B: ACN, Gradient: 3-33% (%B). $NaHCO_3$ solution was added to adjust the pH>7. This solvent was concentrated and the precipitate was filtered off and washed with water. The solid was dried. Yield: 0.1162 g of Compound 100.

Example B7

Preparation of Compound 73

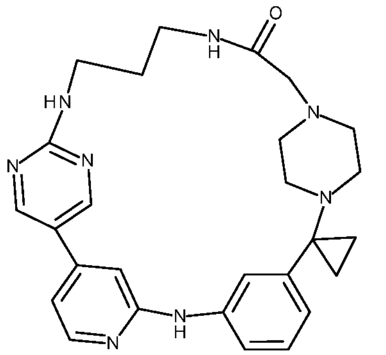

Diethyl cyanophosphonate (0.487 ml; 2.932 mmol) was added to a stirred solution of Int. 186 (0.801 g) and $Et_3N$ (1.019 ml; 7.331 mmol) in DMF (30 ml) at r.t.. The reaction mixture was stirred at r.t. for 1 h. The solvents were evaporated and the residue was purified by Prep HPLC on (Uptisphere C18 ODB - 10μm, 200g, 5cm). Mobile phase (0.25% $NH_4HCO_3$ solution in water, ACN), yielding Compound 73 (100 mg).

Example B8

Preparation of Compound 107

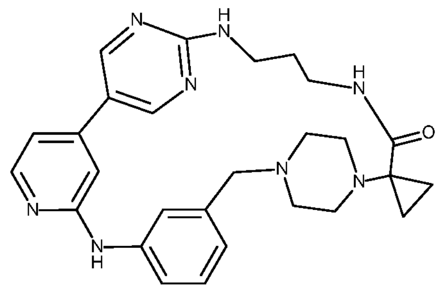

Diethyl cyanophosphonate (0.137 ml; 0.827 mmol) was added dropwise to a stirred solution of Int. 253 (337 mg)and $Et_3N$ (0.574 ml; 4.133 mmol) in DMF (20 ml) at r.t. After addition the reaction mixture was stirred for 1 h. The solvents were evaporated. The residue was dissolved in DCM with some MeOH and then washed with a 10% aqueous $Na_2CO_3$ solution, washed with water, dried with $MgSO_4$, filtered and the solvents of the filtrate were evaporated. The residue was dissolved in DCM and purified over a $SiO_2$ column, type Grace Reveleris SRC, 4 g, Si 40, on a Armen Spot II Ultimate purification system using DCM, MeOH and 7 N $NH_3$ in MeOH as eluents in a gradient starting from 100% DCM going to 5% MeOH and 95% DCM and ending with 5% MeOH and 5% 7 N $NH_3$ in MeOH and 90% DCM. The fractions containing product were combined and the solvents were evaporated yielding 151 mg of Compound 107.

Example B9

Preparation of Compound 116

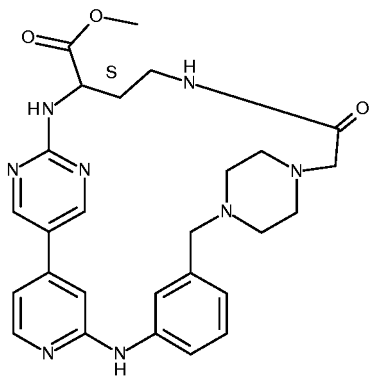

Under $N_2$ atmosphere, Int. 263 (1.5 g) in DMF (75 ml) was added dropwise into a solution of DECP (1.53 g; 9.4 mmol) and DIPEA (3.3 ml; 18.8 mmol) in DMF (75 ml) at r.t. over 1 h. The resulting mixture was stirred for 30 min. The solvent was removed *in vacuo* and the residue was poured into water. The precipitate was filtered and dried to give 1.1 g of crude product. 0.3 g of crude product was purified by prep-HPLC Column: YMC-pack ODS-AQ 150*30mm*5μm. Mobile Phase: 10-30% ACN% (0.1 % TFA). Flow Rate: 30 ml/min. The desired fractions were collected and ACN was removed *in vacuo.* The aqueous layer was adjusted to pH >7 and extracted with EtOAc.The organic layer was dried ($Na_2SO_4$), filtered and concentrated *in vacuo* to give 100.2 mg of Compound 116

Example B10

Preparation of Compound 125

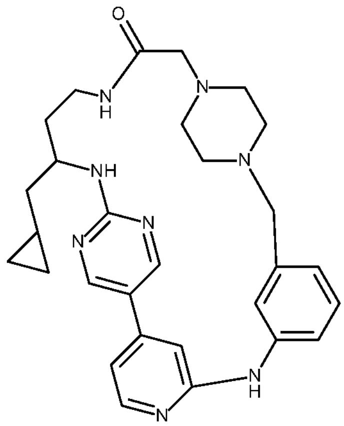

Diethyl cyanophosphonate (0.0851 ml; 0.512 mmol) was added to DMF (50 mL). A solution of Int. 269 (0.20 g) in DMF (100 mL) and $Et_3N$ (0.712 ml; 5.12 mmol) were added dropwise over a periode of 30 min at r.t. The reaction mixture was stirred for 5 h at r.t.

The reaction mixture was evaporated and the residue was dissolved in solution of sat. $NaHCO_3$, DCM and iPrOH. The organic layer was separated, washed with water, dried and evaporated. The residue was purified by column chromatography over silicagel: eluent gradient DCM 100% to 85% / MeOH-$NH_3$ 0% to 15%. The desired fractions were collected and the solvent was evaporated. The residue was repurified by Prep SFC (Stationary phase: Chiralcel Diacel OJ 20 x 250 mm), Mobile phase: $CO_2$, iPrOH with 0.2% iPr$NH_2$). The desired fractions were collected, evaporated, solved in MeOH and evaporated again. Yield: 6 mg of Compound 125.

Example B11

Preparation of Compound 126

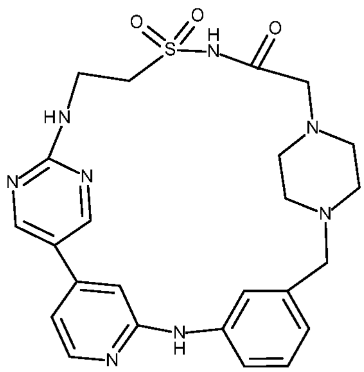

A mixture of Int. 271 (0.15 g; 0.285 mmol) and HATU (0.162 g; 0.427 mmol) in DIPEA (0.245 ml; 1.42 mol) and DMF (7.5 ml) was stirred at r.t. for 18 h. The solvents were evaporated. The residue was purified by Prep HPLC on RP XBridge Prep C18 OBD-10μm, 30x150mm. Mobile phase: 0.25% $NH_4HCO_3$ solution in water, MeOH. The desired fractions were collected and the solvent was evaporated. Yield: 7 mg of Compound 126 (4.8 %).

Example B12

Preparation of Compound 127

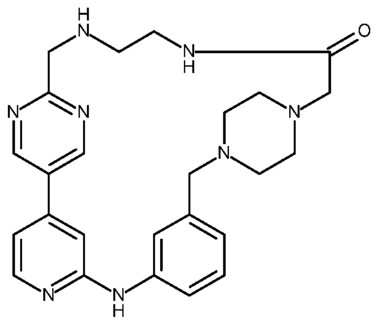

Diethyl cyanophosphonate (0.0272ml; 0.164 mmol) was added to a solution of Int. 278 (39.08 mg) and $Et_3N$ (0.228 ml; 1.64 mmol) in DMF (3.671 ml) at r.t. The reaction mixture was stirred at r.t. for 1 h to give a reaction mixture containing Compound 127 which was used as such in the next reaction step.

Example B13

Preparation of Compound 129

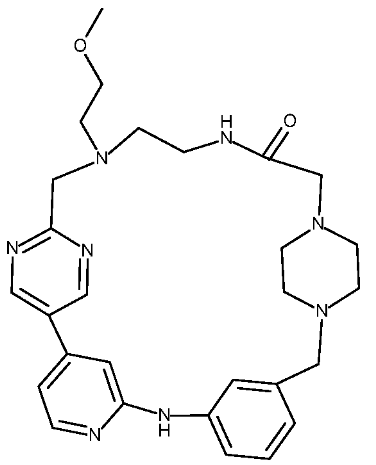

Diethyl cyanophosphonate (23.5 µl; 0.142 mmol) was added dropwise to a stirred solution of Int. 285 (86 mg) and $Et_3N$ (98.4 µl; 0.708 mmol) in DMF (4 ml) at r.t. After addition the reaction mixture was stirred for 1 h. The solvents were evaporated. The residue was purified by Prep HPLC (Stationary phase: RP SunFire Prep C18 OBD-10 µm, 30x150 mm) (Mobile phase: 0.25% $NH_4HCO_3$ solution in water, ACN). The desired fractions were collected and the solvent was evaporated. Yield: 8 mg of Compound 129.

Example B14

Preparation of Compound 132

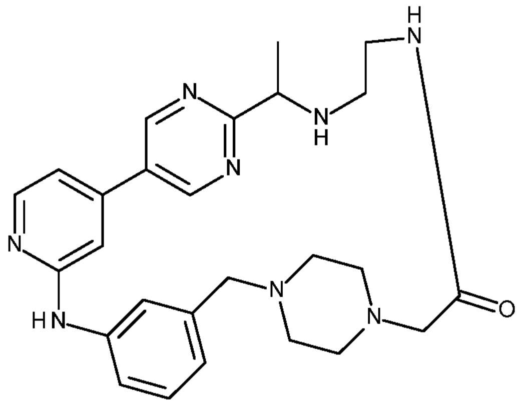

Int. 296 (6.69 mg; 8 mmol) in DMF (300 ml) was added dropwise into the solution of DECP (6.55 g; 40 mmol) and DIPEA (13.66 ml; 80 mmol) in DMF (300 ml) at r.t. over 1 h under $N_2$ atmosphere. The resulting mixture was stirred for 30 min. The solvent was removed *in vacuo* and the residue was poured into water. The precipitate was filtered and dried. The crude was washed with aq. $NaHCO_3$, $H_2O$, MTBE and DCM to give 1.2 g of Compound 132.

Example B15

Preparation of Compound 67

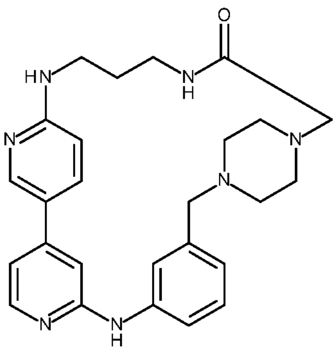

Diethyl cyanophosphonate (1.08 mL; 7.191 mmol) was added slowly to a solution of Int. 170 (1.9 g) and DIPEA (4.1 mL; 23.97 mmol) in DMF (340 mL). After the addition, the reaction mixture was heated at 100 °C for 4 h and was then evaporated. The residue was purified by preparative LC on (irregular SiOH 15-40 μm 300 g MERCK). Mobile phase (0.5 % $NH_4OH$, 93 % DCM, 7 % MeOH) and then repurified by achiral SFC on (AMINO 6 μm 150 x 21.2 mm). Mobile phase (0.3 % isopropylamine, 75 % $CO_2$, 25 % MeOH). The pure fractions were collected, evaporated and stirred in DIPE/ACN. The precipitate was filtered off and dried, yielding 412 mg of Compound 67.

Example B16

Preparation of Compound 140

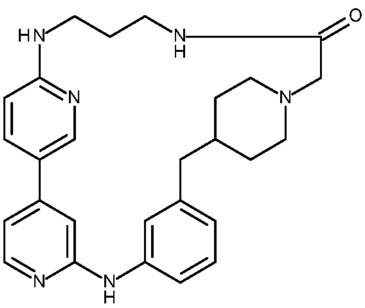

Diethyl cyanophosphonate (70 µL; 0.47 mmol) was added dropwise to a solution of Int. 304 (108 mg) and DIPEA (268 µL; 1.55 mmol) in DMF (66 mL). After addition, the reaction mixture was heated to 100 °C for 4 h. Then DMF was evaporated. The residue was purified by preparative LC on (Stability Silica 5µm 150 x 30.0 mm). Mobile phase (Gradient from $NH_4OH$/DCM/MeOH 0.2/98/2 to $NH_4OH$/DCM/MeOH 1.2/88/12). The desired fractions were collected and the solvent was evaporated. The residue was freeze-dried with water/ACN, yielding 18 mg of Compound 140 as a white powder.

Example B17

Preparation of Compound 141

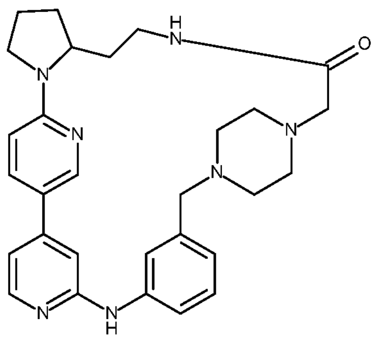

$K_2CO_3$ (1.18 g; 8.51 mmol) was added to a solution of Int. 310 (249 mg) in DMF (50 mL) at r.t. The reaction mixture was stirred at 50 °C for 3 h. Water and DCM were added. The mixture was extracted with DCM (3 x). The organic layer was dried over $MgSO_4$, filtered and the solvent was evaporated. The residue was purified by preparative LC on (Stability Silica 5µm 150x30.0mm). Mobile phase (Gradient from $NH_4OH$/DCM/MeOH 0.2/98/2 to $NH_4OH$/DCM/MeOH 1.2/88/12). The desired fractions were collected and the solvent was evaporated. Yield: 86 mg of Compound 141 as a off-white powder.

Example B18

Preparation of Compound 144

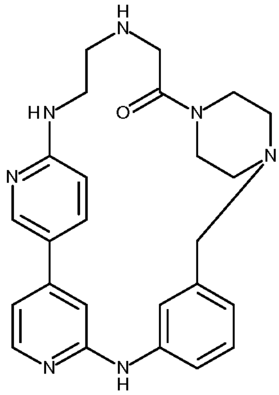

$K_2CO_3$ (686 mg; 5 mmol) was added to a solution of Int. 317 (133 mg) in DMF (35 mL) at r.t. The reaction mixture was stirred at 50 °C for 3h and then at 70 °C for 18h. The reaction mixture was evaporated and the residue was purified by preparative LC (Stability Silica 5μm 150 x 30.0 mm, mobile phase Gradient from pure DCM to DCM/MeOH/$NH_4OH$ 90/10/0.1). The pure fractions were evaporated and the solvent evaporated. This residue was repurified by preparative LC on (Stability Silica 5μm 150x30.0mm). Mobile phase (Gradient from $NH_4OH$/DCM/MeOH 0.2/98/2 to $NH_4OH$/DCM/MeOH 1.2/88/12). The pure fractions were evaporated and the solvent evaporated until dryness to give 19 mg of Compound 144.

Example B19

Preparation of Compound 145a

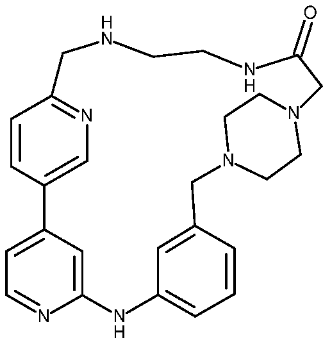

To as solution of DECP (22.488 ml;150 mmol) and DIPEA (60 ml; 344.467 mmol) in DMF (750 ml) was added dropwise a solution of Int. 323 (15 g) in DMF (750ml) for 2 h. The mixture was stirred at r.t. for 30 minutes and was then concentrated. The residue was purified by high-performance liquid chromatography over DYA101810 C18 (C18 column type) (eluent: (0.5 % $NH_3$ in $H_2O$)/ACN 35/65 v/v). The product fractions were collected and the solvent was evaporated. Yield: 1.992 g of Compound 145a.

C. Conversion reactions and chiral separations of final compounds

Example C1

a) Preparation of Compounds 5 and 6

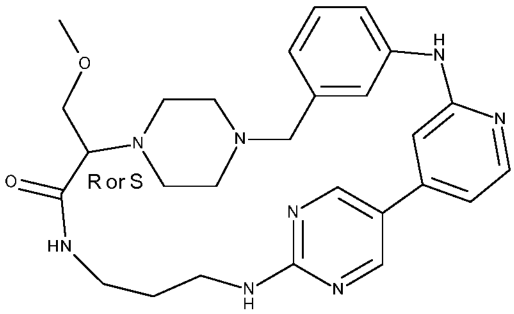

Compound 5

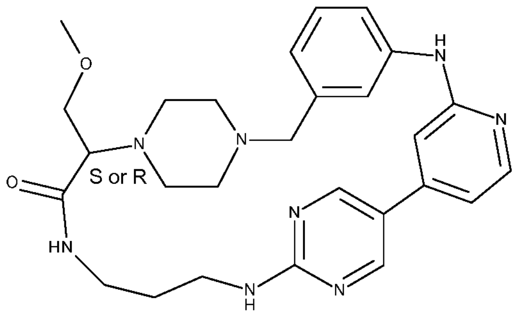

Compound 6

Compound 31 (200 mg) was separated by SFC separation on Chiralcel OJ, 20 μm; Supercritical $CO_2$ / MeOH (0.2 % DEA), v/v, 200 ml/min). The desired fraction were collected and the solvent was evaporated. Yield: 0.05 g of compound 6 (S or R) and 0.04 g of compound 5 (R or S).

b) Preparation of Compounds 25 and 26

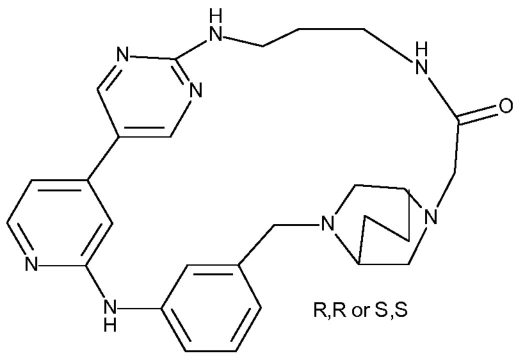

Compound 25

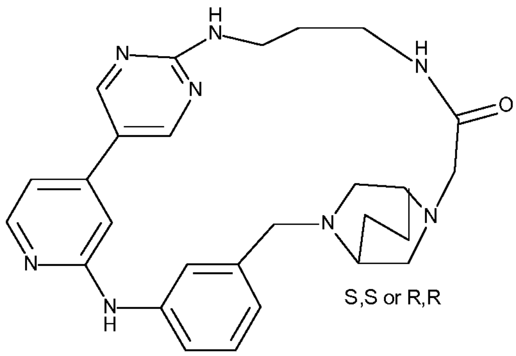

Compound 26

Compound 24 (500 mg) was separated by SFC separation on Chiralcel OJ, 20 μm; Supercritical $CO_2$ / MeOH (0.2 % DEA), v/v, 200 ml/min). The desired fraction were collected and the solvent was evaporated. Yield: 0.14 g of compound 26 and 0.148 g of compound 25.

c) Preparation of Compounds 22 and 23

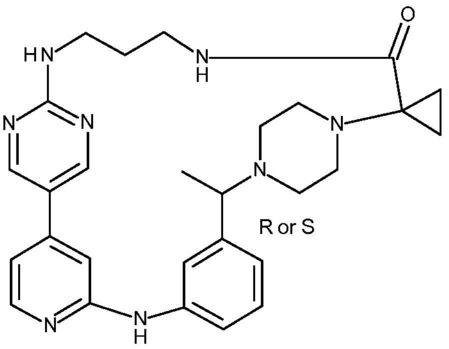

Compound 22

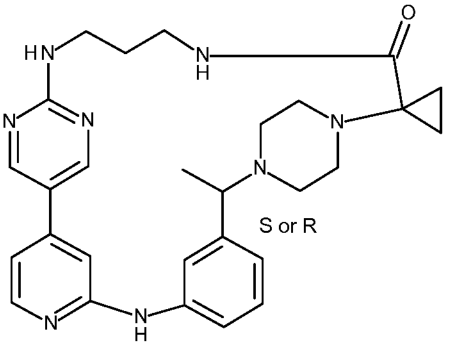

Compound 23

Compound 21 was separated by SFC (Column: OD-H 250×30 mm I.D, 10 μm, Flow rate: 80 ml/min, Mobile Phase A: Supercritical $CO_2$/ MeOH (0.2 %$NH_3H_2O$) 50/50. The desired fractions were collected and the solvent was evaporated. Yield: 0.4 g of compound 22 and 0.12 g of compound 23.

<u>d) Preparation of Compounds 2 and 3</u>

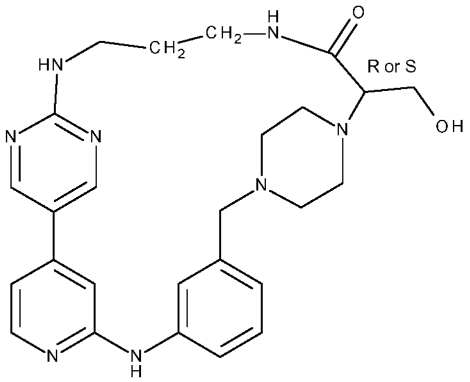

Compound 3

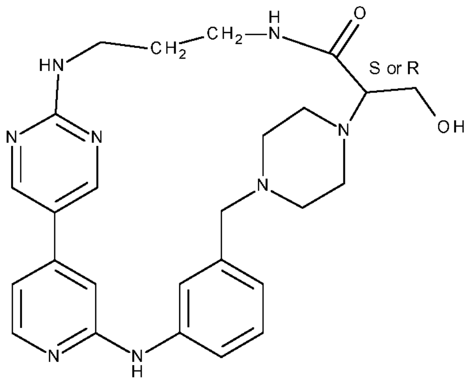

Compound 2

By similar SFC separation methods as described above, compound 30 was separated into its enantiomers, compounds 2 (37.9 mg) and 3 (49 mg).

<u>e) Preparation of Compounds 11 and 12</u>

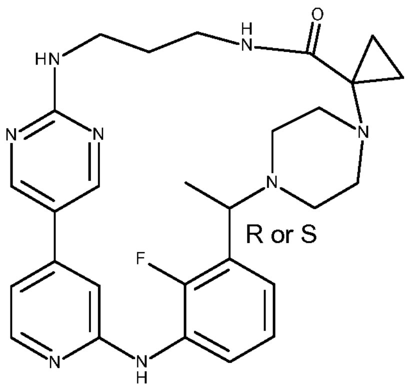

Compound 11

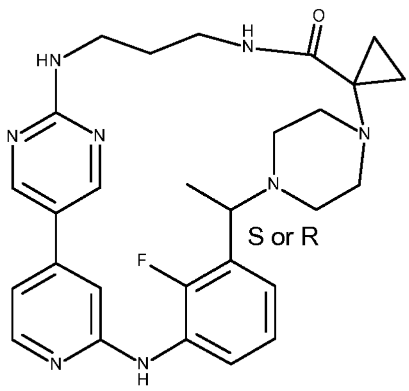

Compound 12

By similar SFC separation methods as described above, compound 32 (500 mg) was separated into its enantiomers, compounds 11 (210 mg) and 12 (36 mg).

f) Preparation of Compounds 65 and 66

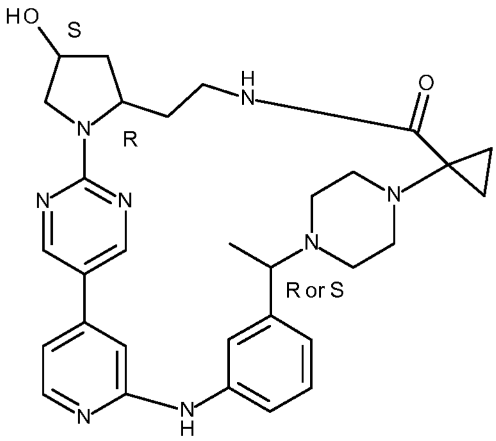

Compound 65

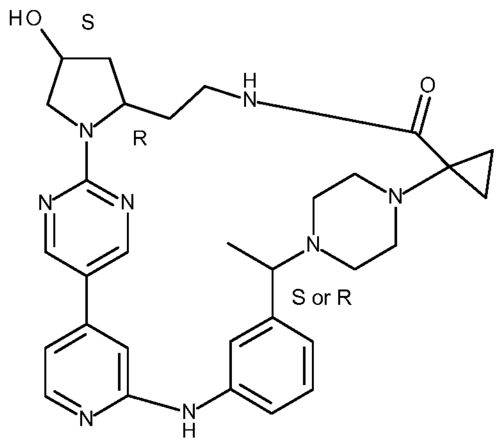

Compound 66

By similar SFC separation methods as described above, compound 58 was separated into compounds 65 (345.6 mg) and 66 (93.4 mg).

g) Preparation of Compounds 109 and 110

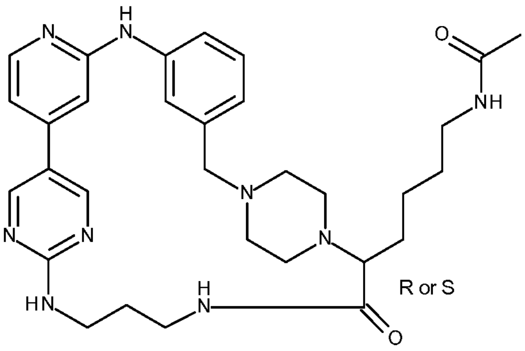

Compound 109

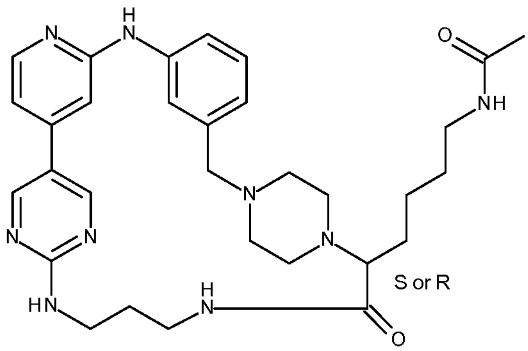

Compound 110

By similar SFC separation methods as described above, compound 108 was separated into compounds 109 and 110.

h) Preparation of Compounds 138 and 139

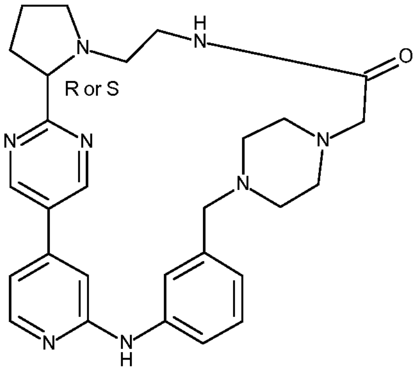

Compound 138

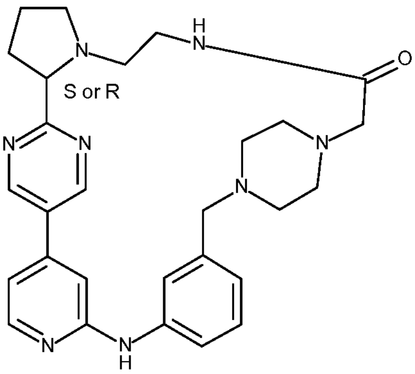

Compound 139

By similar SFC separation methods as described above, compound 135 was separated into compounds 138 and 139.

i) Preparation of Compounds 142 and 143

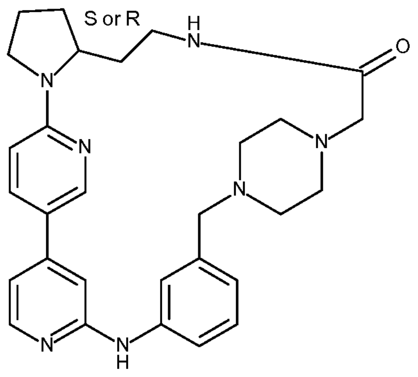

Compound 142

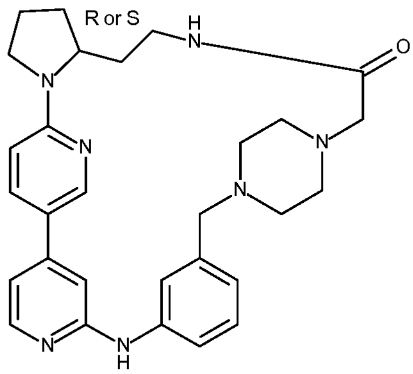

Compound 143

Compound 141 was separated by chiral SFC (Column: Chiralpak AD-H 5μm 250×20 mm). Mobile phase: 0.3 % isopropylamine, 55 % $CO_2$, 45 % iPrOH. The desired fractions were collected and the solvent was evaporated. Yield: 30 mg of compound 142 and 30 mg of compound 143.

j) Preparation of Compounds 133 and 134

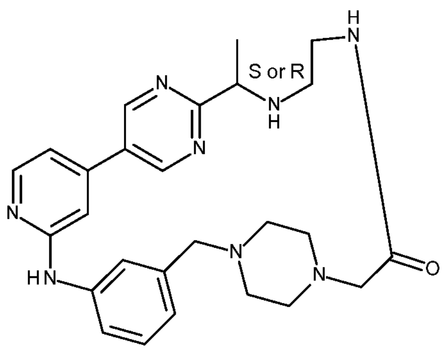

Compound 133

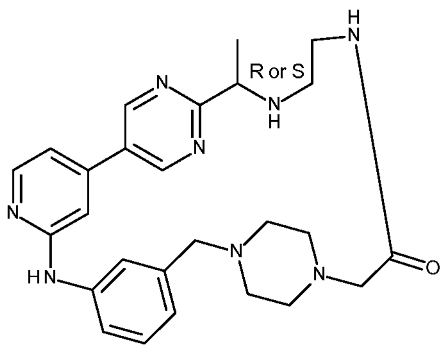

Compound 134

Compound 132 was separated into compounds 133 and 134 by chiral HPLC. (Compound 134 was used for the preparation of Compound 137).

Example C2

a) Preparation of Compounds 80 and 81

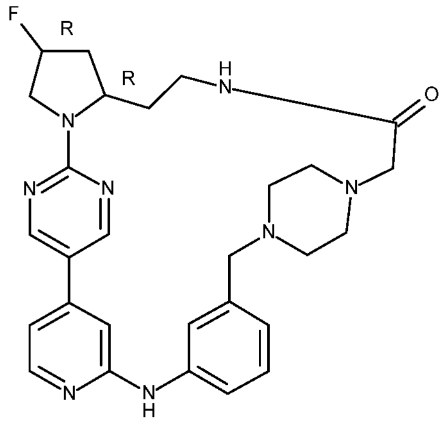

Compound 80

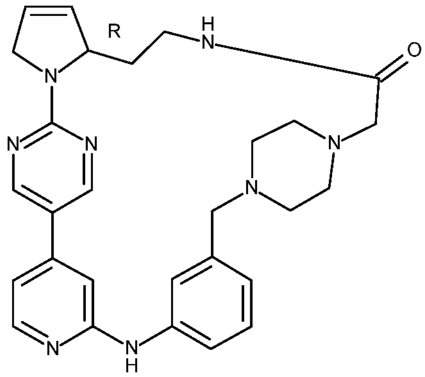

Compound 81

Compound 55 (514.6 mg; 1 mmol) in THF (10 ml) was stirred at -78 °C under $N_2$ flow. DBU (2283.6 mg; 15 mmol) was added. Then XtalFluor-E® (1144 mg; 5mmol) was added. Stirring was continued at -78 °C for 30 min and then the reaction mixture was stirred at r.t. for 2 h. The reaction mixture was poured into water, basified with $NaHCO_3$ and extracted 3x with DCM. The organic layer was dried with $MgSO_4$, filtered and evaporated. The residue purified by flash chromatography on silica(eluens: DCM/MeOH 90/10). The desired fractions were collected and evaporated. The residue (2.8 g) was repurified by Prep HPLC on (RP Vydac Denali C18 - 10μm, 200g, 5cm). Mobile phase (0.25% $NH_4HCO_3$ solution in water, ACN). The desired fractions were collected, evaporated, solved in MeOH and evaporated again, yielding 41 mg of Compound 80 (8 %) and 64 mg of Compound 81 (13 %).

b) Preparation of Compound 82

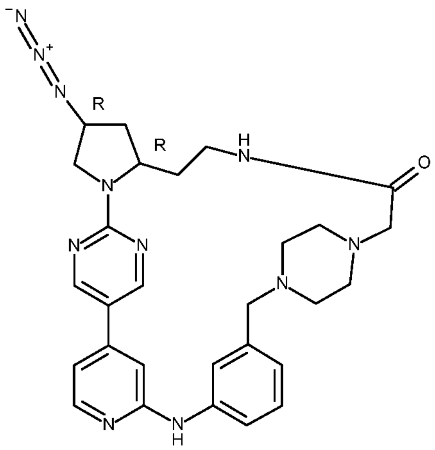

Compound 55 (514.6 mg; 1 mmol), $PPh_3$ (2623 mg; 10 mmol) and DPPA (2752 mg;10 mmol) were stirred in THF (25 ml) at r.t. DIAD (2022 mg; 10 mmol) was added dropwise. Stirring was continued for 16 h. The reaction mixture was evaporated to dryness. The residue was stirred in DIPE and the precipitate was filtered off. The precipitate was stirred in ACN for 16 h and filtered. The filtrate was evaporated to dryness. This residue (2.8 g) was purified by Prep HPLC on (RP Vydac Denali C18 - 10μm, 200g, 5cm). Mobile phase (0.25 % $NH_4HCO_3$ solution in water, MeOH). The desired fractions were collected, evaporated, solved in MeOH and evaporated again. This fraction was repurified using ACN instead of MeOH as solvent. Yield: 292 mg of Compound 82 (54 %).

Compound 83 was prepared according to an analogous reaction protocol.

c) Preparation of Compound 84

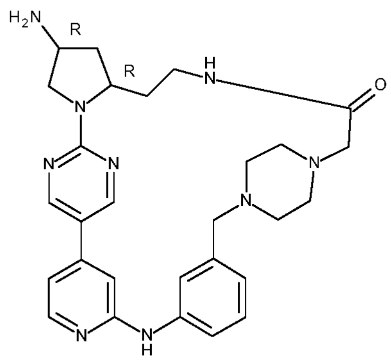

Compound 82 (156 mg; 0.289 mmol) was hydrogenated in 20 ml MeOH under 1 atm. $H_2$ gas at r.t. with 100 mg Pt 5 % on activated charcoal as catalyst. The catalyst was filtered off and the filtrate was evaporated. The residue was purified by Prep HPLC (Stationary phase: RP SunFire Prep C18 OBD-10 μm,30x 150 mm), Mobile phase: 0.25 % $NH_4HCO_3$ solution in water, ACN). The desired fractions were collected and the solvent was evaporated, yielding 47 mg of Compound 84 (31.6 %).

Compound 85 was prepared according to an analogous reaction protocol.

d) Preparation of Compound 86

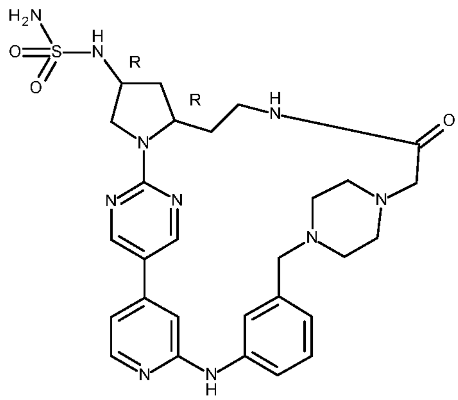

Compound 84 (65 mg; 0.127 mmol) and sulfamide (121.6 mg; 1.265 mmol) were stirred in 1,4 dioxane (3 ml) at 80 °C for 16 h. The reaction mixture was evaporated to dryness. The residue was purified by Prep HPLC (Stationary phase: RP Vydac Denali C18 - 10 μm, 200 g, 5 cm). Mobile phase: 0.25% $NH_4HCO_3$ solution in water, ACN). The desired fractions were collected, evaporated, solved in MeOH and evaporated again. Yield: 50 mg of Compound 86 (66.7 %).

Compound 87 was prepared according to an analogous reaction protocol.

e) Preparation of Compound 88

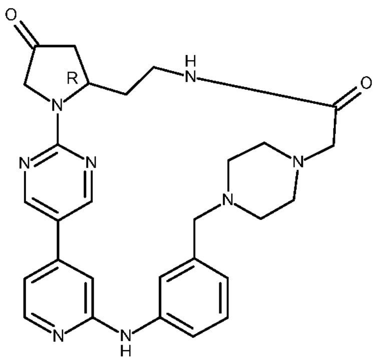

Oxalylchloride (5 ml; 2 M solution in DCM; 10 mmol) was stirred in DCM (10 ml) at - 78 °C under $N_2$ atmosphere. DMSO (1562.7 mg; 20 mmol) in DCM (10 ml) was added dropwise. After 5 min. Compound 55 (103 mg; 0.2 mmol) in DCM (3 ml) was added dropwise. The reaction mixture was stirred at -78 °C for 1 h. Then $Et_3N$ (3035.7 mg; 30 mmol) in DCM (2 ml) was added dropwise. The temperature was raised to r.t. for 16 h. The reaction mixture was poured into water and extracted with DCM. The organic layer was dried with $MgSO_4$, filtered and evaporated. The residue (200 mg) was purified by Prep HPLC (Stationary phase: RP SunFire Prep C18 OBD-10μm,30x 150 mm), Mobile phase: 0.25 % $NH_4HCO_3$ solution in water, ACN) The desired fractions were collected and evaporated.

The residue was repurified by Prep SFC (Stationary phase: Chiralcel Diacel OJ 20 x 250 mm), Mobile phase: $CO_2$, MeOH with 0.2 % $iPrNH_2$). The desired fractions were collected, evaporated, solved in MeOH and evaporated again, yielding 9 mg of Compound 88 (8.8 %).

Example C3

a) Preparation of Compound 112

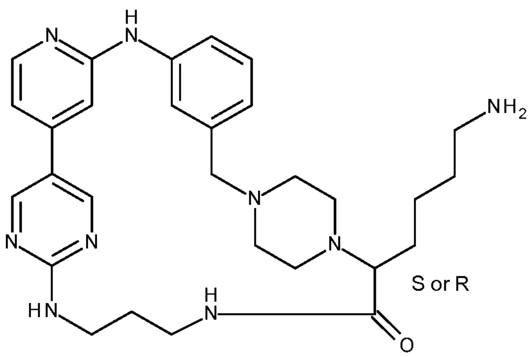

A solution of Compound 110 (170 mg; 0.297 mmol) in 4M HCl (17 ml) was refluxed overnight. The reaction mixture was evaporated *in vacuo*. The residue was repurified on Prep SFC stationary phase: Chiralcel Diacel OD 20 x 250 mm, Mobile phase: $CO_2$, MeOH with 0.2% $iPrNH_2$. The desired fractions were collected, evaporated, solved in MeOH and evaporated again, yielding 45 mg of the desired compound 112.

Example C4

a) Preparation of Compound 114

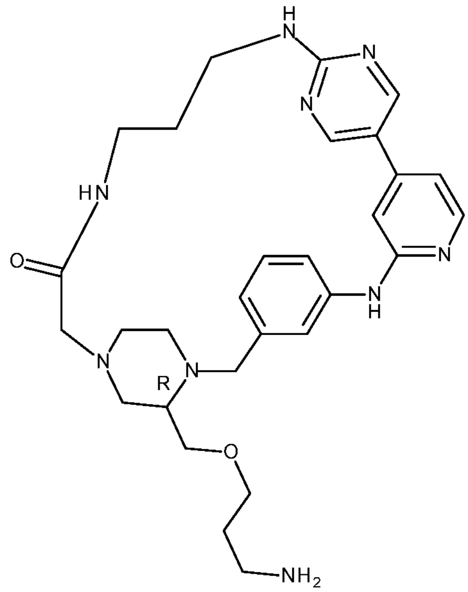

A mixture of Compound 111 (350 mg; 0.646 mmol) in MeOH (20 ml) was hydrogenated at 50 °C under atmospheric pressure of $H_2$ gas with Raney nickel (100 mg) as a catalyst in the presence of $NH_4OH$ (1 ml). After uptake of $H_2$ (2 eq.), the catalyst was filtered off and the filtrate was evaporated. The crude was purified by SFC Column: Chiralcel OD-H 150×4.6mm I.D., 5μm, Mobile phase: 40% ethanol (0.1% ethanolamine) in $CO_2$, Flow rate: 2.35 mL/min and repurified by prep-HPLC. Conditions for prep-HPLC: Column: DYA101810 C18-10μm (C18 column type with 10 μm particle size). Mobile Phase: gradient 5 to 35% ACN and 95 to 65% (0.1% TFA sol. in water). Flow Rate: 80 ml/min. The desired fraction was collected and ACN was removed *in vacuo.* The aqueous layer was adjusted to pH>7 and the precipitate was filtered and dried *in vacuo* to give 130 mg of Compound 114 (37 %).

b) Preparation of Compound 115

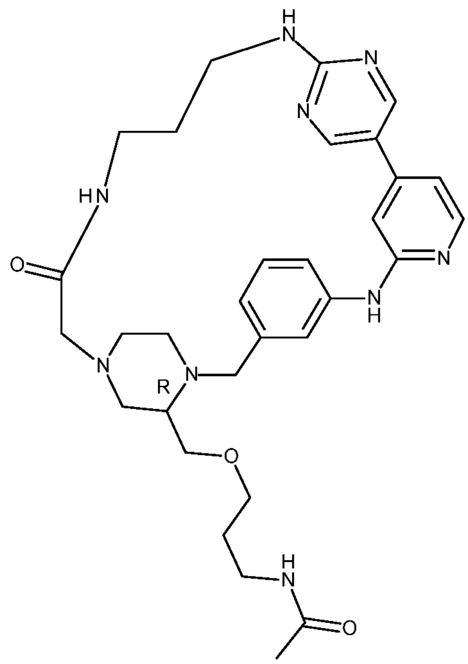

Compound 114 (50 mg; 0.0916mol) was dissolved in THF (3 ml). Acetic anhydride (9.3 mg; 0.092 mmol) and DIPEA (23.8 mg; 0.184 mmol) were added. The mixture was stirred at r.t. for 2 h. The mixture was concentrated. The

residue was recrystallized from EtOAc yielding 12.2 mg of Compound 115 (22 %).

Example C5

a) Preparation of Compound 117

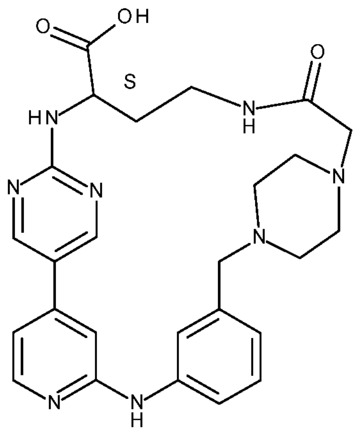

.3HCl

NaOH (0.15 g) was added to Compound 116 (0.8 g) in MeOH/$H_2O$ 1/1 (10 ml). The mixture was stirred at r.t. for 4 h. MeOH was removed *in vacuo.* Then the pH was adjusted to approximately 7. The resulting mixture was evaporated *in vacuo.* The residue was dissolved in MeOH and filtered. The filtrate was purified by prep-HPLC. Column: YMC-pack ODS-AQ 150*30mm*5μm. Mobile Phase: gradient 5 to 25 % ACN and 95 to 75% (0.1% TFA sol. in water). The desired fraction was collected and the solvent was removed *in vacuo.* The residue was stirred in HCl/dioxane (4M) for 30 min. The solvent was removed *in vacuo* to give 300 mg of Compound 117.

Compound 119 (starting from Compound 118) and Compound 120 were prepared by using an analogous reaction protocol as was used for Compound 117.

b) Preparation of Compound 121

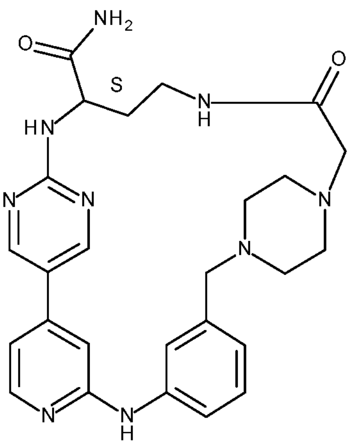

.3 TFA

A mixture of Compound 117 (150 mg), ammonia 4 M sol. in THF (0.61 ml) and 2,4,6-tripropyl-1,3,5,2,4,6-trioxatriphosphorinane 2,4,6-trioxide (187 mg; 0.294 mmol) in DIPEA (0.218 ml; 1.225 mmol) and DMF (10 ml) was stirred at 20 °C overnight. The DMF was removed *in vacuo.* The residue was purified by HPLC over Synergi (eluent: ACN/(0.5 % TFA in $H_2O$) 1% to 8%). The product fractions were collected and the solvent was evaporated. Yield: 6.3 mg of Compound 121.

Compound 122 was prepared by using an analogous reaction protocol as was used for Compound 121, but Compound 117 and methylamine were used as starting materials. Compound 123 was prepared by using an analogous reaction protocol as was used for Compound 121, but Compound 119 was used as starting material.

Compound 124 was prepared by using an analogous reaction protocol as was used for Compound 121, but Compound 119 and methylamine were used as starting materials.

Example C6

a) Preparation of Compound 128

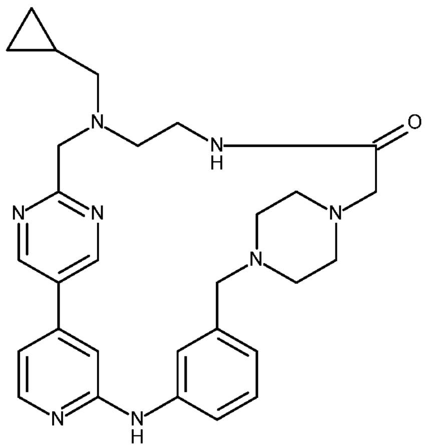

Cyclopropylmethyl bromide (44.281 mg; 0.328 mmol) was added to the reaction mixture containing compound 127 at 70°C. The reaction mixture was stirred at 80 °C for 4 h and was then concentrated to dryness. The residue was purified by Prep HPLC on (RP Vydac Denali C18 - 10μm, 200g, 5cm). Mobile phase (0.25% $NH_4HCO_3$ solution in water, ACN). The desired fractions were collected, evaporated, solved in MeOH and evaporated again, yielding 4.8 mg of Compound 128.

Example C7

a) Preparation of Compound 136

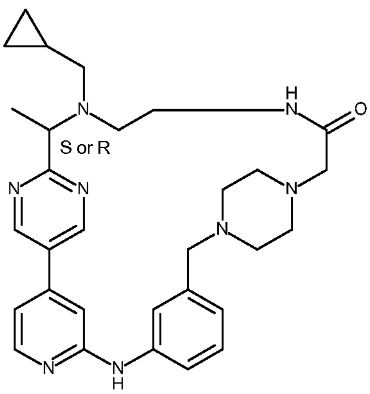

Cyclopropylmethyl bromide (288 mg; 2.13 mmol) was added to a solution of compound 133 (101 mg; 0.214 mmol) and DIPEA (0.737 ml; 4.274 mmol) in DMF (7 ml). The reaction mixture was stirred at 50 °C for 48 h. The reaction mixture was concentrated and the residue was purified by Prep HPLC (Stationary phase: RP Vydac Denali C18 - 10μm, 200g, 5cm), Mobile phase: 0.25% $NH_4HCO_3$ solution in water, ACN). The desired fractions were collected and the

solvent was evaporated, yielding 55 mg of Compound 136 (48.8 %).

Example C8

a) Preparation of Compound 146

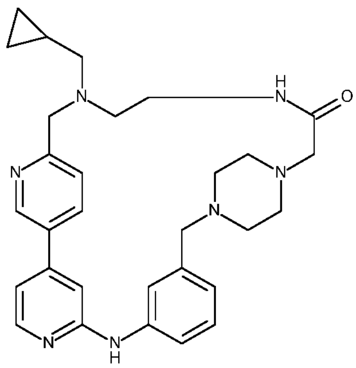

Cyclopropylmethyl bromide (44.65 mg; 0.331 mmol) dissolved in DMF (2 ml) was added portionwise to compound 145 (.4 HCl) (99.785 mg) and DIPEA (0.228 ml; 1.323 mmol) in DMF (8 ml) at 60 °C over 30 min. The reaction mixture was stirred at 70 °C for 16 h and was then evaporated. The residue was purified by Prep HPLC on (RP Vydac Denali C18 - 10 μm, 200g, 5cm). Mobile phase (0.25% $NH_4HCO_3$ solution in water, ACN). The desired fractions were collected, evaporated, solved in MeOH and evaporated again, yielding 45 mg of Compound 146 (53.18 %).

Example C9

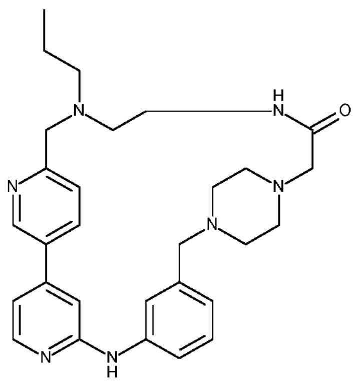

a) Preparation of Compound 147

$NaBH(OAc)_3$ (210.7 mg; 0.994 mmol) was added portionwise to a suspension of compound 145 (.4 HCl) (200 mg; 0.33 mol) and propionaldehyde (38.5 mg; 0.66 mol) in DMF (6 ml) at r.t. The reaction mixture was stirred further at r.t. for 1h and was then concentrated. The residue was purified by Prep HPLC (Stationary phase: RP Vydac Denali C18 - 10μm, 200g, 5cm), Mobile phase: 0.25% $NH_4HCO_3$ solution in water, ACN). The desired fractions were collected, evaporated, solved in MeOH and evaporated again, yielding 94 mg of compound 147 (56.76 %).

Example C10

a) Preparation of Compound 148

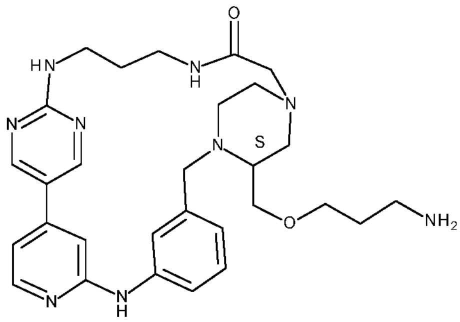

A mixture of Compound 29 (1 g; 1.30 mmol) in MeOH (30 ml) was hydrogenated at 50 °C under a $H_2$ gas pressure of 50 psi with Raney Nickel (0.5 g) as a catalyst in the presence of 25 % $NH_4OH$ (0.5 ml) overnight. After uptake of $H_2$ (2 eq.), the catalyst was filtered off and the filtrate was evaporated. The residue was purified by SFC (Column: Chiralcel OD 250×30mm I.D., 10μm; Mobile phase: Supercritical $CO_2$/EtOH (0.2 % $NH_3H_2O$) 60/40; Flow rate: 80ml/min, wavelength: 220nm). The desired fractions were collected and the solvent was evaporated. Yield: 0.06 g of Compound 148 (8 %).

b) Preparation of Compound 149

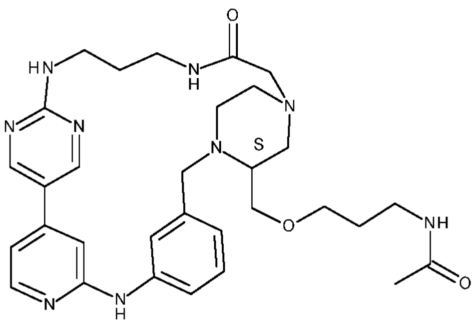

The mixture of Compound 148 (100 mg; 0.183 mmol), acetic anhydride (18.68 mg; 0.183 mmol), $Et_3N$ (64.64 mg; 0.64 mmol) and THF (10 ml) was stirred at r.t. for 2 h. The solvent was evaporated. The residue was purified by SFC (Column: Chiralcel OD 250×30mm I.D., 10μm; Mobile phase: Supercritical $CO_2$/EtOH (0.2 % $NH_3H_2O$) 60/40; Flow rate: 80ml/min; wavelength: 220nm). The desired fractions were collected and the solvent was evaporated. Yield: 0.042 g of Compound 149 (37.2 %).

By using analogous reaction protocols as described in the foregoing examples, the compounds listed in the Table below have been prepared.

'Method' refers to the Example number in analogy to which protocol the compound was synthesized.

In case no specific stereochemistry is indicated for a stereocenter of a compound, this means that the compound was obtained as a mixture of the R and the S enantiomers.

The values of salt stoichiometry or acid content in the compounds as provided herein, are those obtained experimentally and may vary dependent on the analytical method used (for the compounds in Table 1, $^1H$ NMR and/or elemental analysis was used). In case no salt form is indicated, the compound was obtained as a free base.

Table 1: compounds ("TFA" means trifluoroacetic acid)

| | |
|---|---|
| 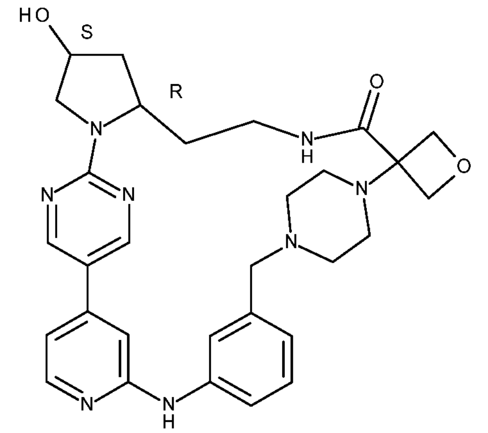 | 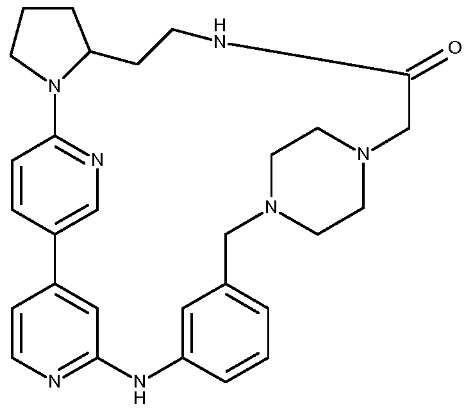 |
| Compound 64; Method B3 | Compound 141; Method B17 |
| 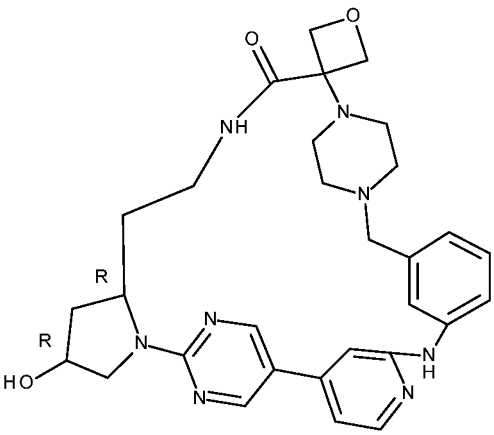 | 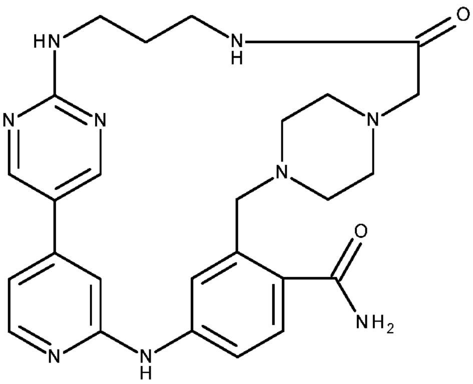 |
| Compound 63; Method B3 | Compound 4; Method B1 |
| 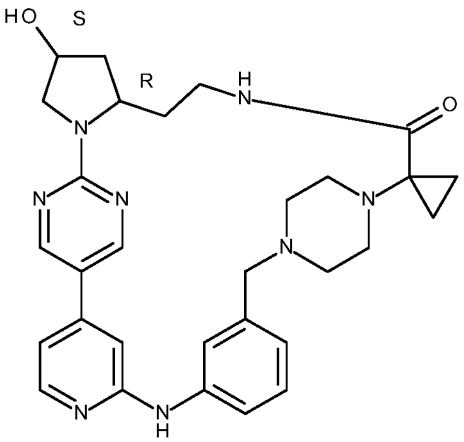 | 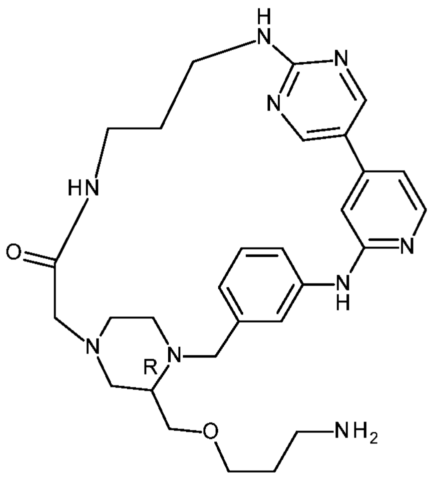 |
| Compound 56; Method B3 | Compound 114; Method C4.a |

(continued)

| | |
|---|---|
| 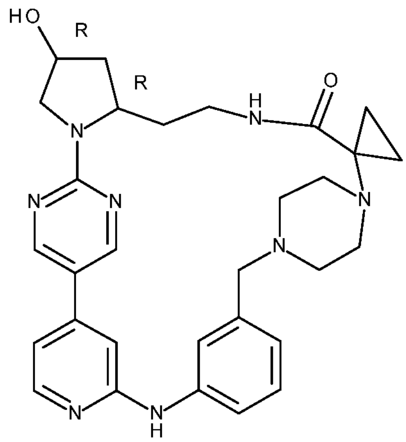 | 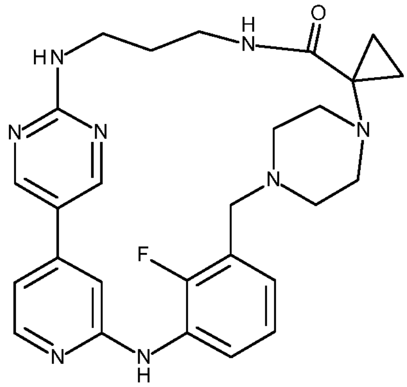 |
| Compound 57; Method B3 | Compound 20; Method B1 |
| 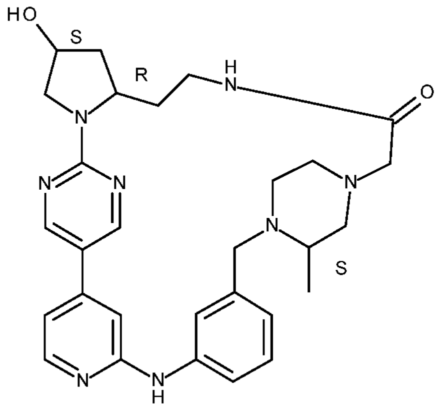 | 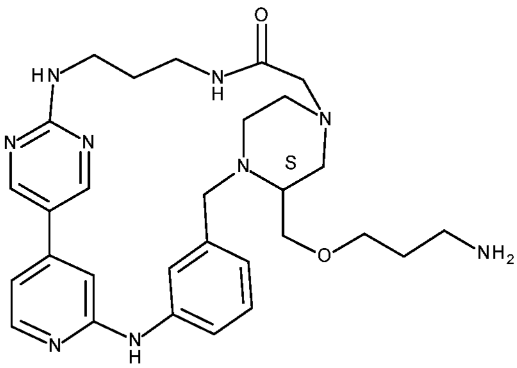 |
| Compound 62; Method B3 | Compound 148; Method C10.a |
| 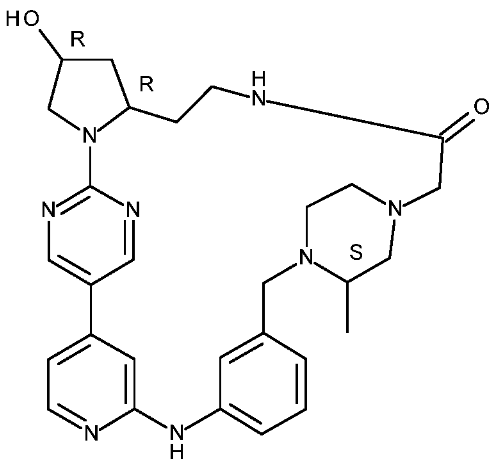 | 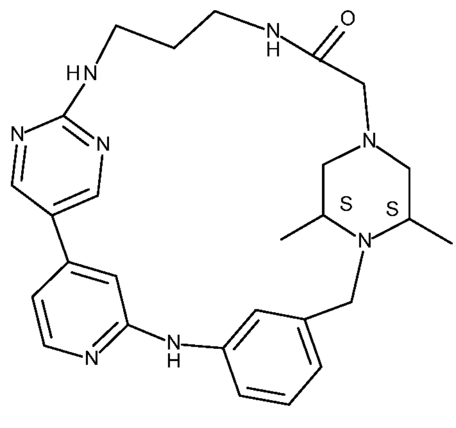 |
| Compound 61; Method B3 | Compound 34; Method B2 |

(continued)

| | |
|---|---|
| 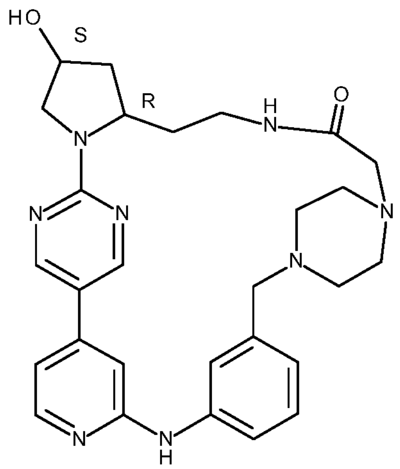 | 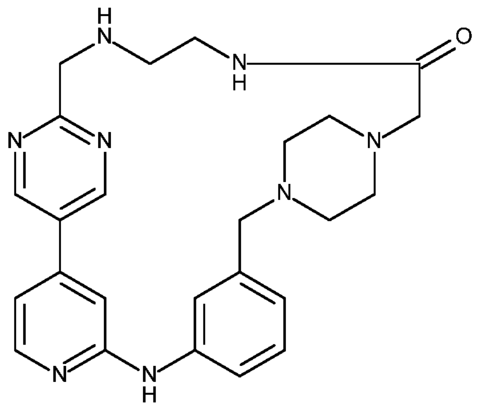 |
| Compound 55; Method B3 | Compound 127; Method B12 |
| 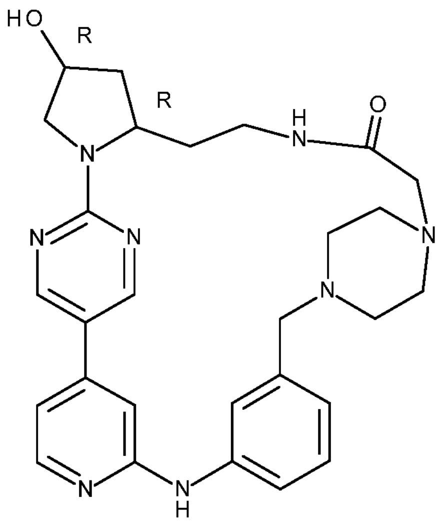 | 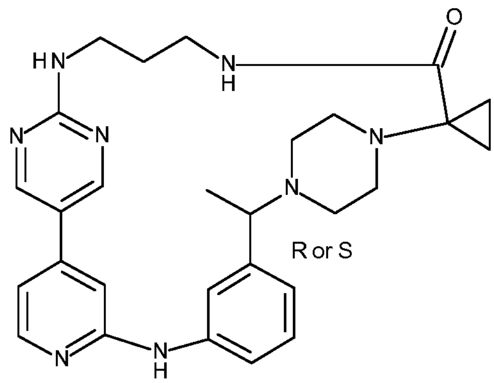 |
| Compound 54; Method B3 | Compound 22; Method C1 |
| 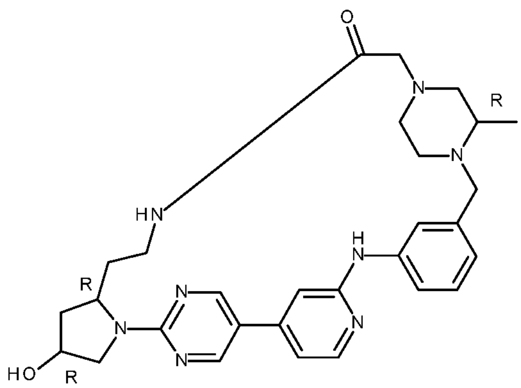 | 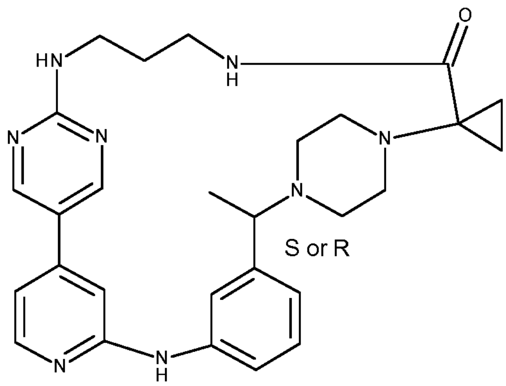 |
| Compound 60; Method B3 | Compound 23; Method C1 |

(continued)

| | |
|---|---|
| 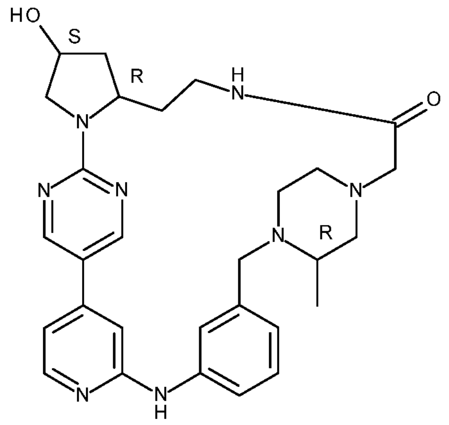 | 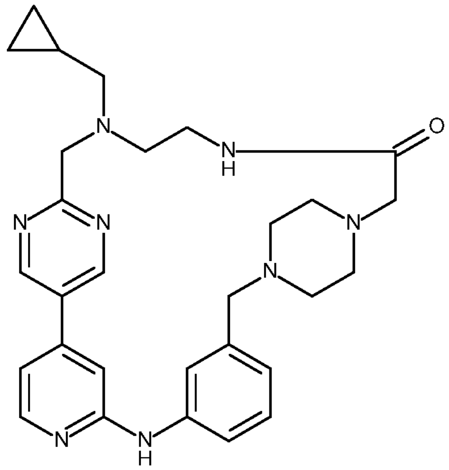 |
| Compound 59; Method B3 | Compound 128; Method C6 |
| 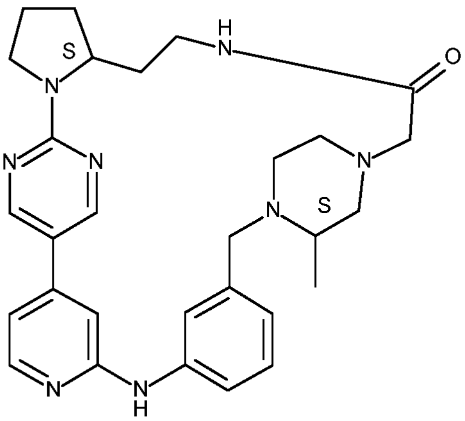 | 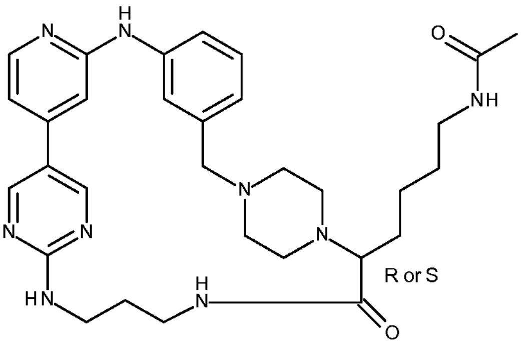 |
| Compound 47; Method B3 | Compound 109; Method C1 |
| 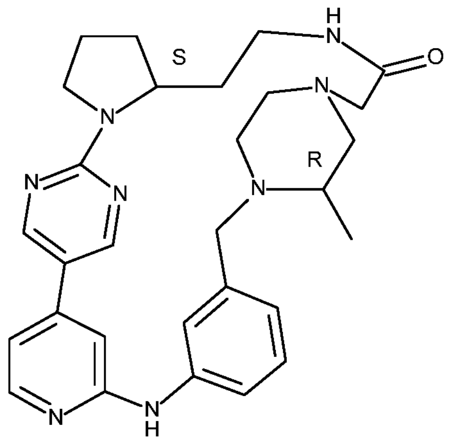 | 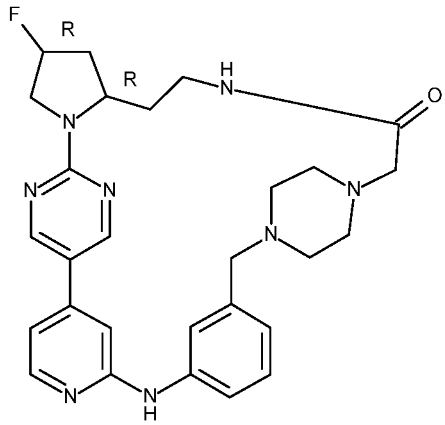 |
| Compound 48; Method B3 | Compound 80; Method C2.a |

(continued)

| | |
|---|---|
| 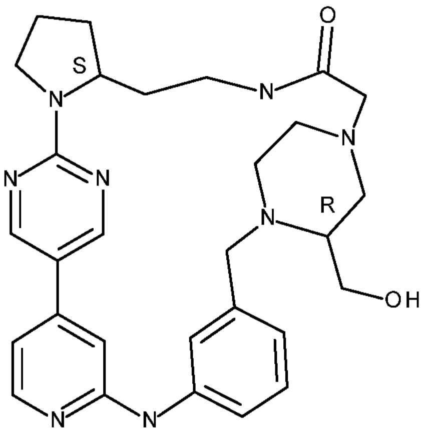 | 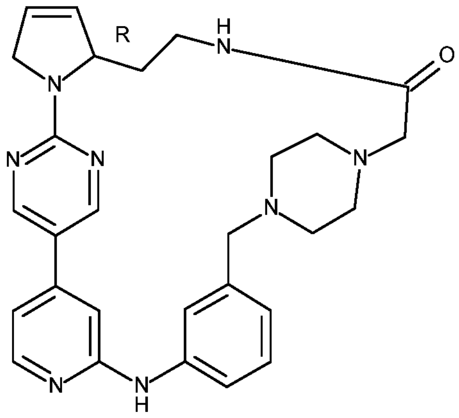 |
| Compound 46; Method B3 | Compound 81; Method C2.a |
| 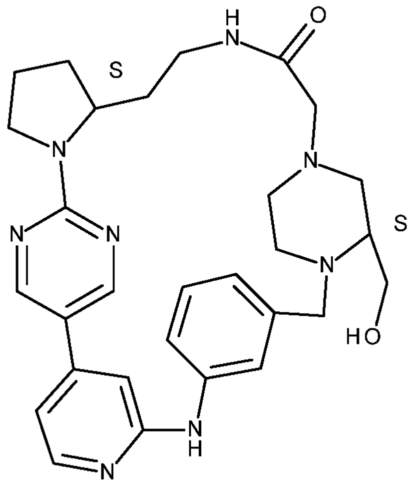 | 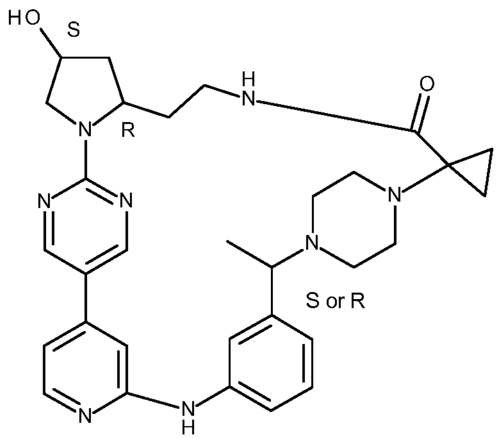 |
| Compound 44; Method B3 | Compound 66; Method C1 |
| 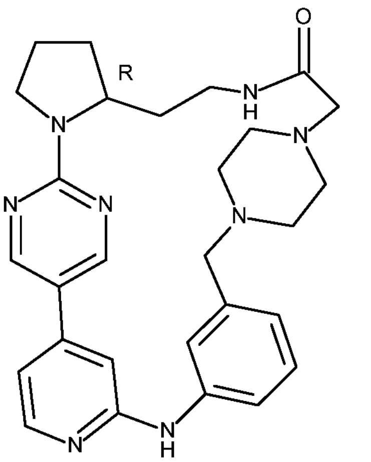 | 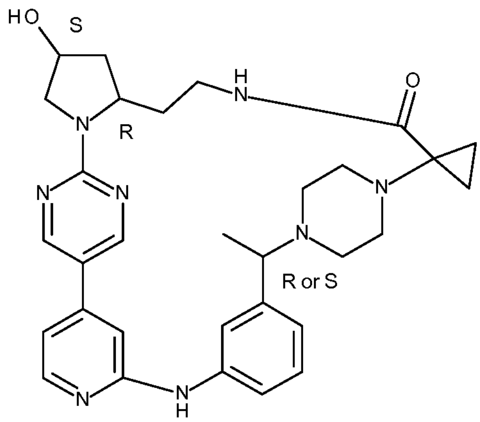 |
| Compound 45; Method B3 | Compound 65; Method C1 |

(continued)

| | |
|---|---|
| 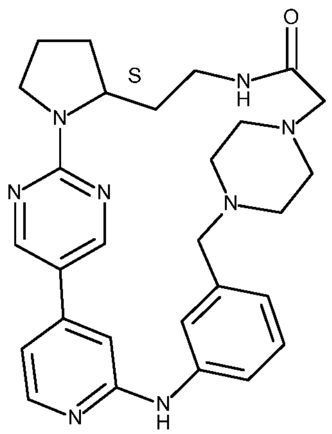 | 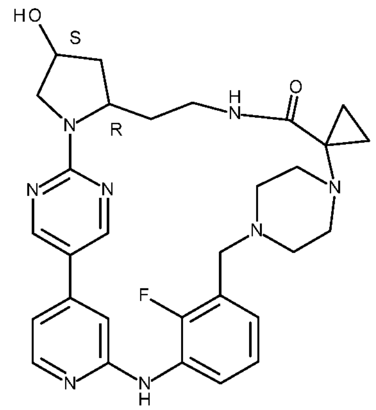 |
| Compound 43; Method B3 | Compound 53; Method B3 |
| 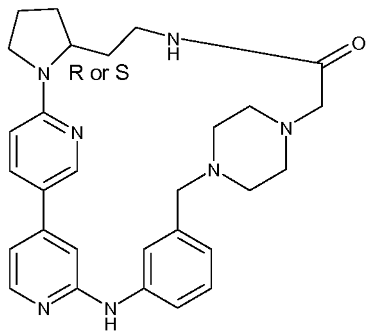 | 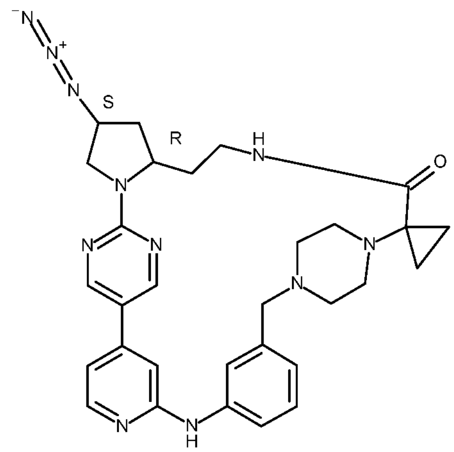 |
| Compound 143; Method C1 | Compound 83; Method C2.a |
| 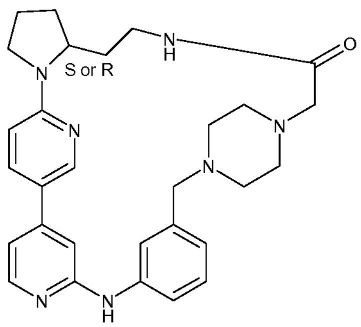 | 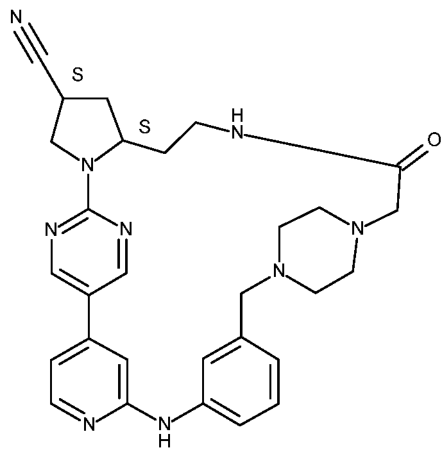 |
| Compound 142; Method C1 | Compound 78; Method B4 |

(continued)

| | |
|---|---|
| 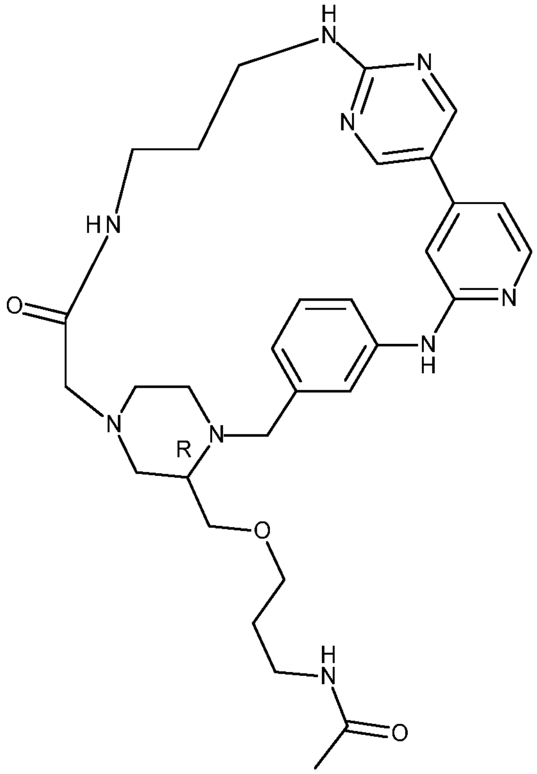 | 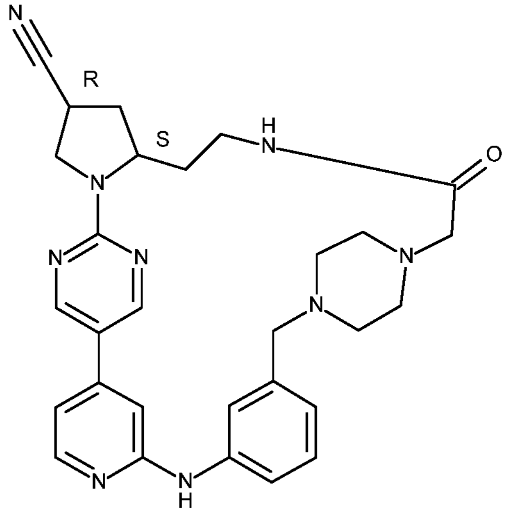 |
| Compound 115; Method C4.b | Compound 77; Method B4 |
| 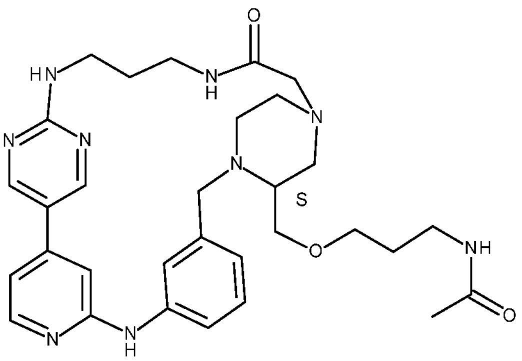 | 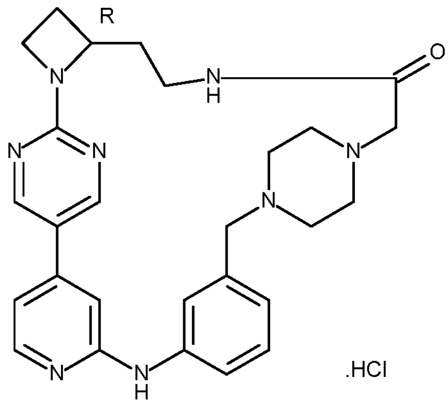 |
| Compound 149; Method C10.b | Compound 49; Method B3 |
| 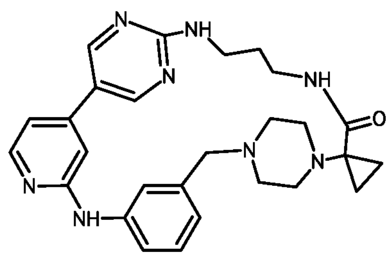 | 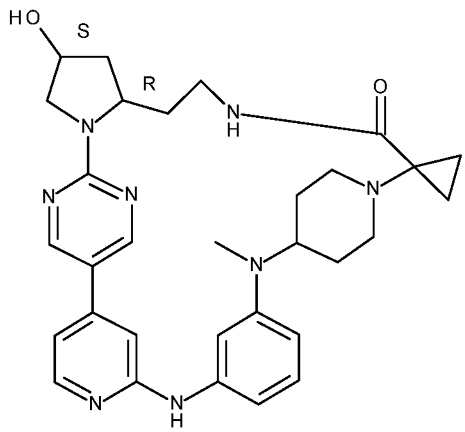 |

(continued)

| Compound 107; Method B8 | Compound 104; Method B6 |
|---|---|
| 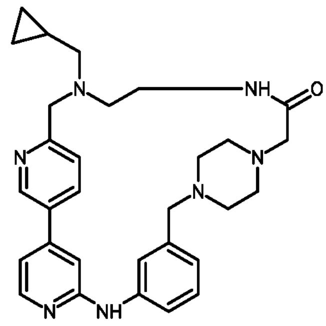 | 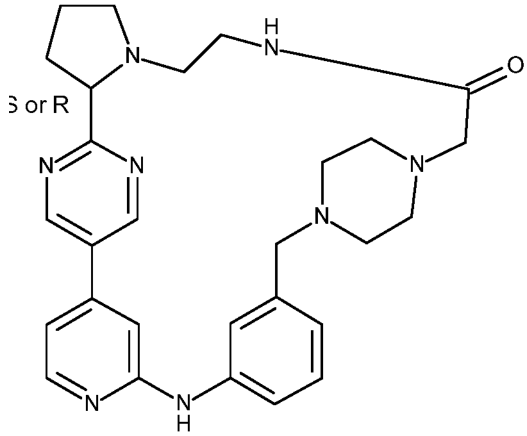 |
| Compound 146; Method C8 | Compound 139; Method C1 |
| 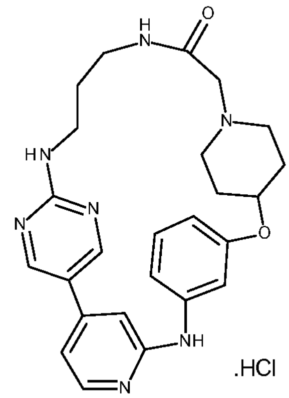 | 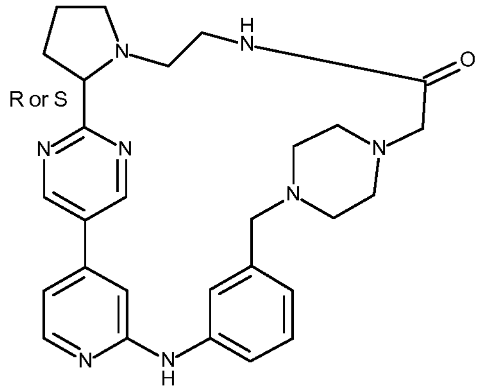 |
| Compound 90; Method B5 | Compound 138; Method C1 |
| 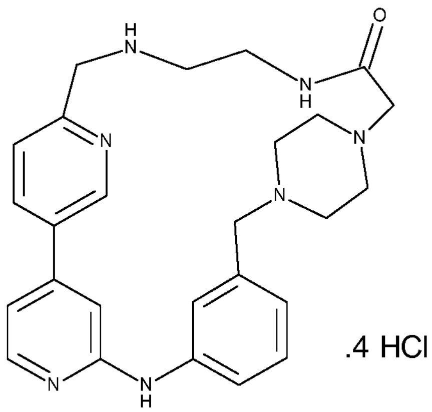 | 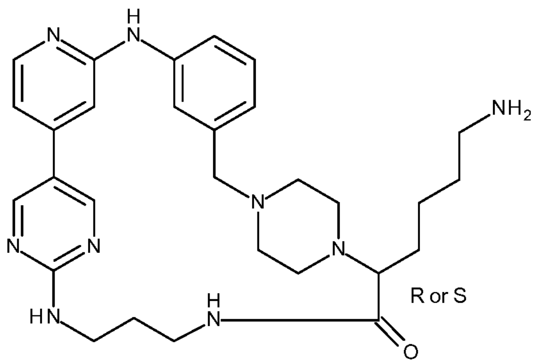 |
| Compound 145; Method B19 | Compound 113; Method C3 |

(continued)

| | |
|---|---|
| 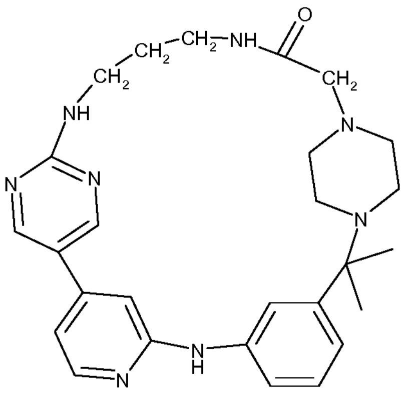 | 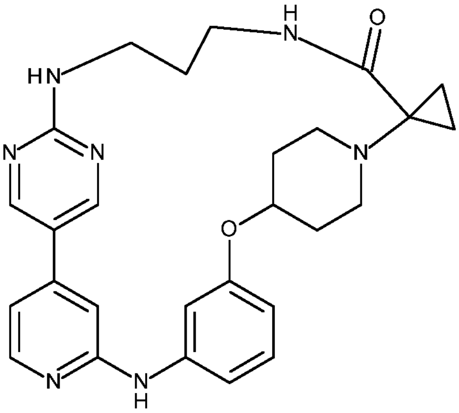 |
| Compound 74; Method B7 | Compound 95; Method B5 |
| 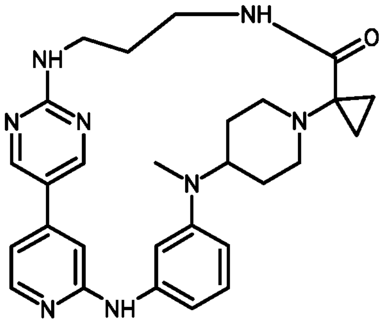 | 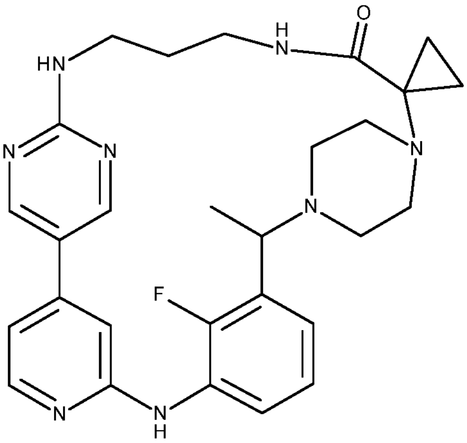 |
| Compound 102; Method B6 | Compound 32; Method B1 |
| 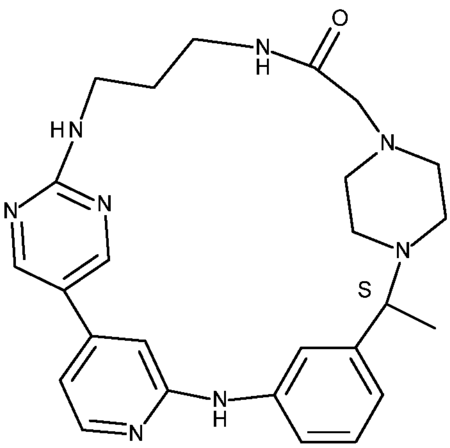 | 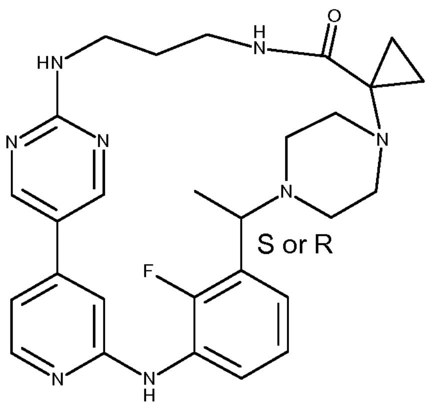 |
| Compound 75; Method B7 | Compound 12; Method C1 |

(continued)

| | |
|---|---|
| 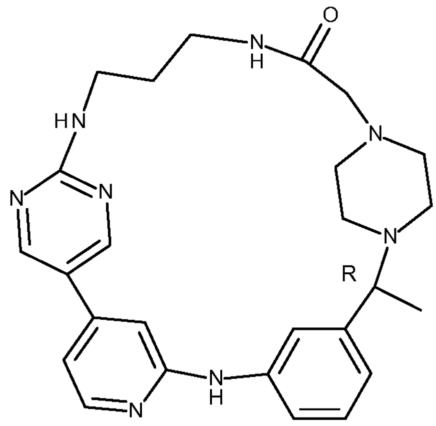 | 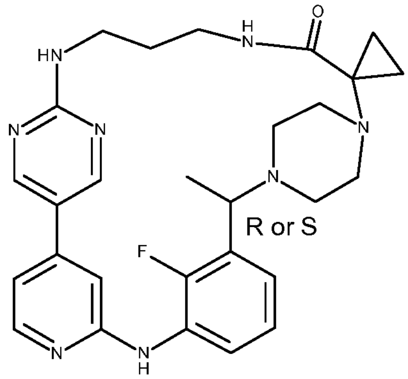 |
| Compound 76; Method B7 | Compound 11; Method C1 |
| 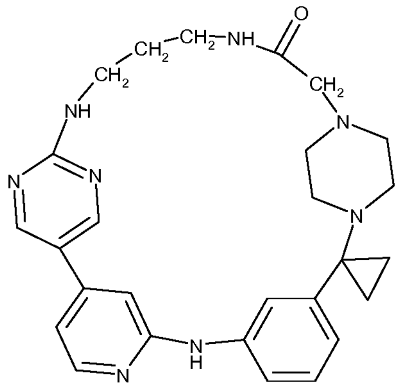 | 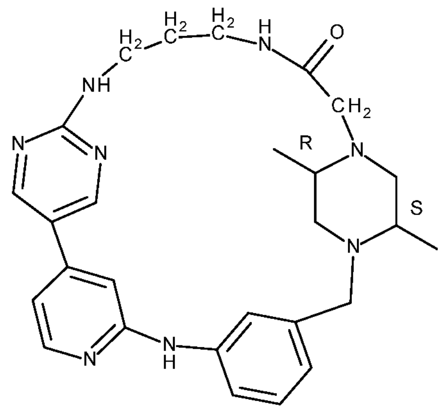 |
| Compound 73; Method B7 | Compound 35; Method B2 |
| 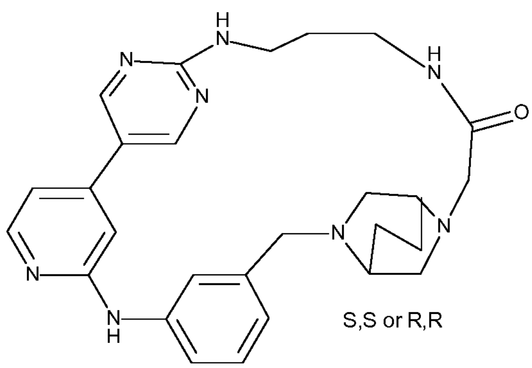 | 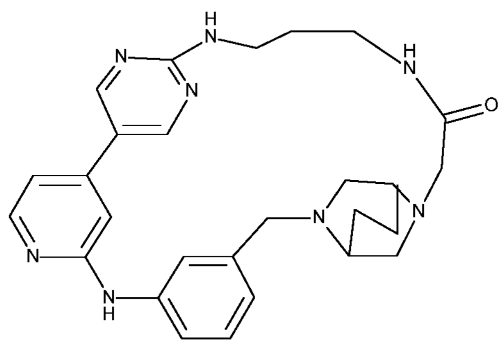 |
| S,S or R,R Compound 26; Method C | Compound 24; Method B 1 |

(continued)

| | |
|---|---|
| 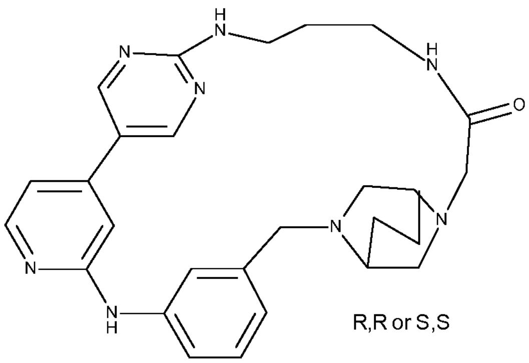 | 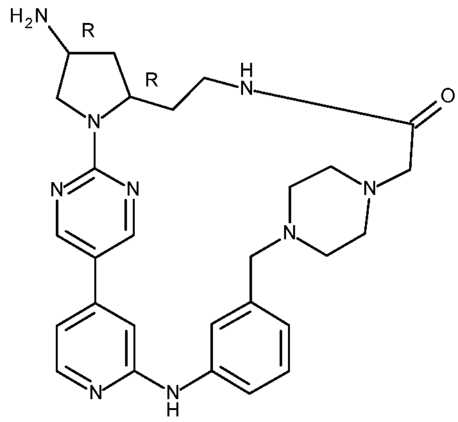 |
| R,R or S,S Compound 25; Method C | Compound 84; Method C2.c |
| 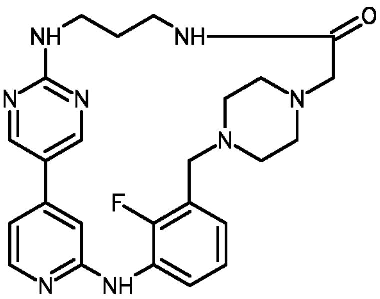 | 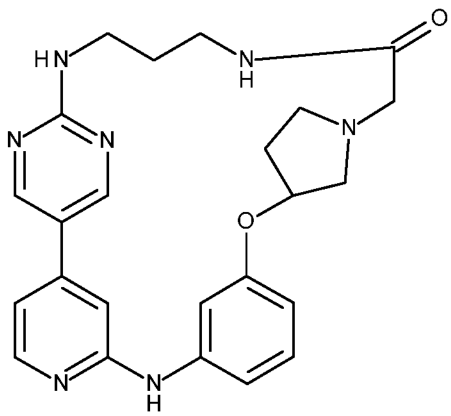 |
| Compound 1; Method B1 | Compound 93; Method B5 |
| 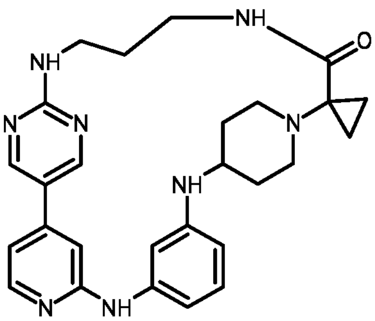 | 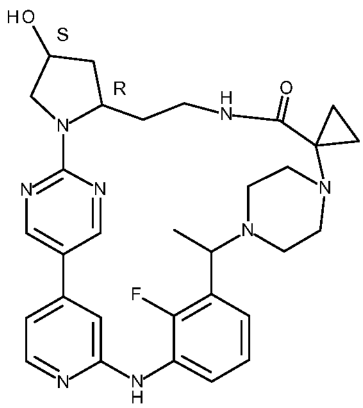 |
| Compound 100; Method B6 | Compound 52; Method B3 |

(continued)

| | |
|---|---|
| 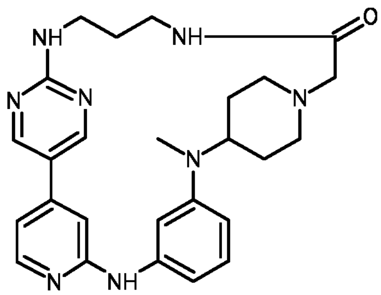 | 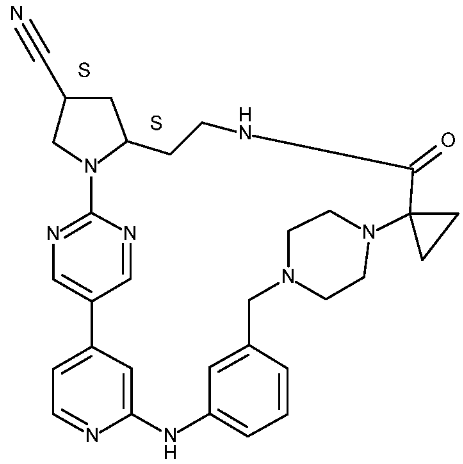 |
| Compound 101; Method B6 | Compound 79; Method B4 |
| 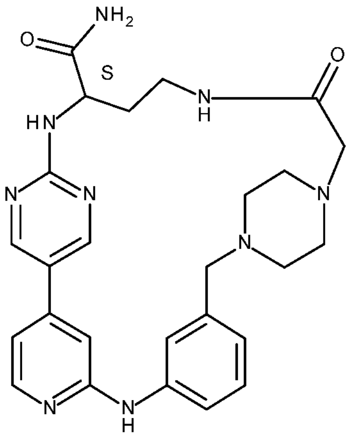 .3 TFA | 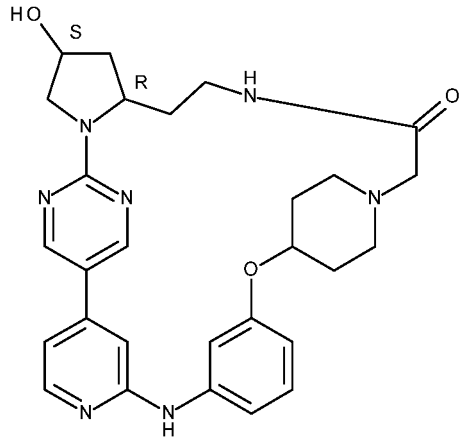 |
| Compound 121; Method C5.b | Compound 99; Method B5a |
| 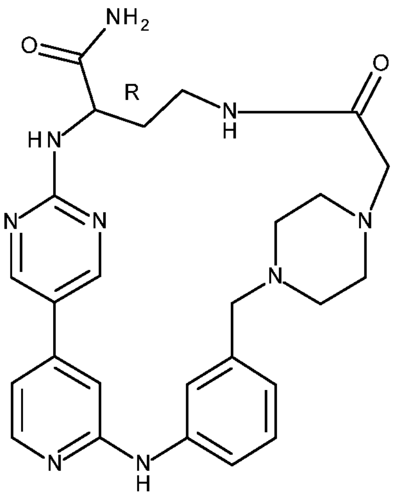 .3 TFA | 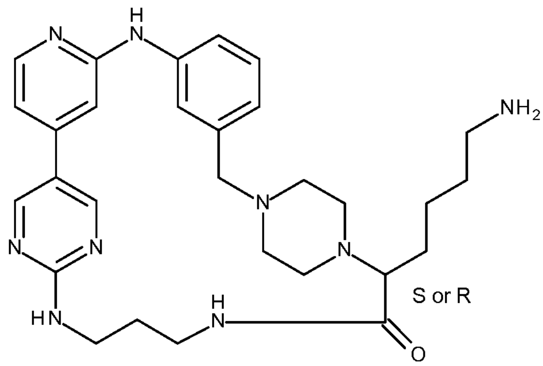 |
| Compound 123; Method C5.b | Compound 112; Method C3 |

(continued)

| | |
|---|---|
| 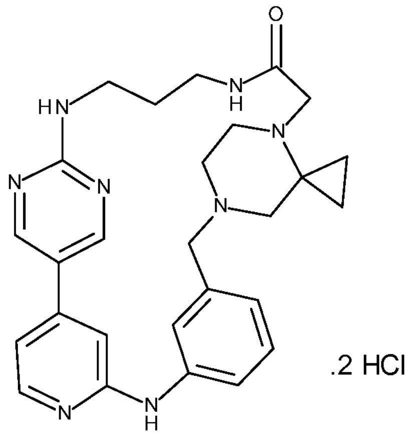 | 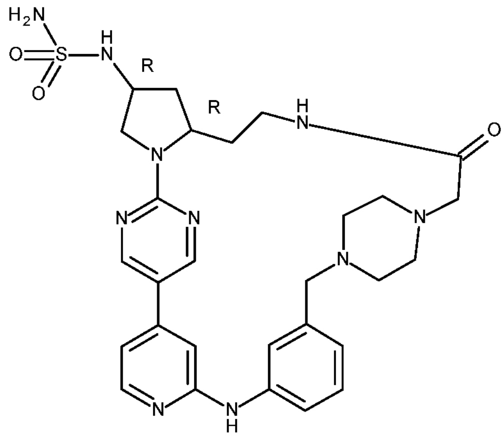 |
| Compound 28; Method B1 | Compound 86; Method C2.d |
| 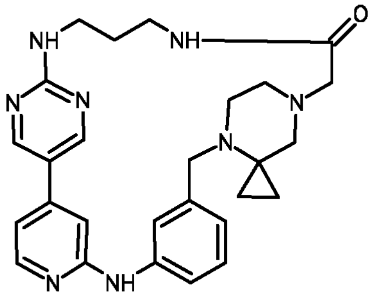 | 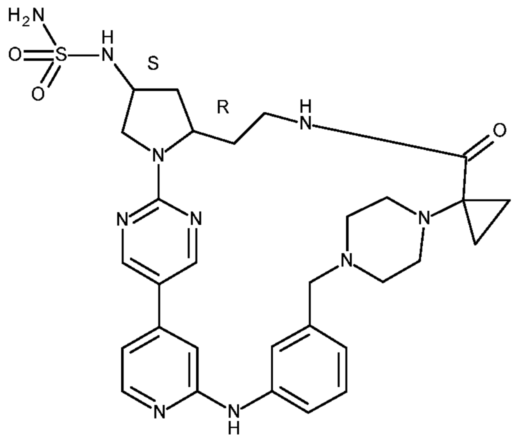 |
| Compound 27; Method B1 | Compound 87; Method C2.d |
| 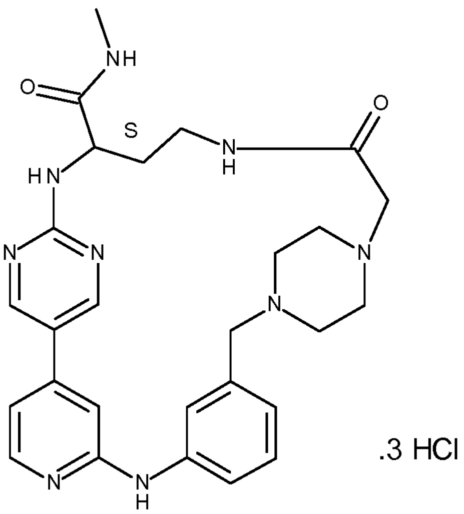 | 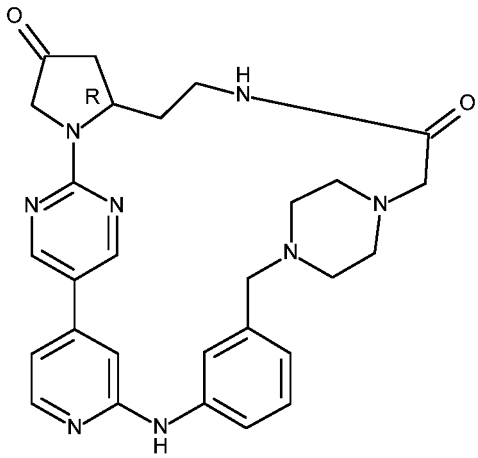 |
| Compound 122; Method C5.b | Compound 88; Method C2.e |

(continued)

| | |
|---|---|
| 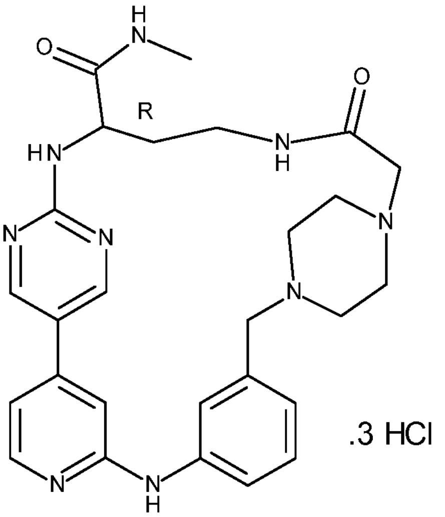 | 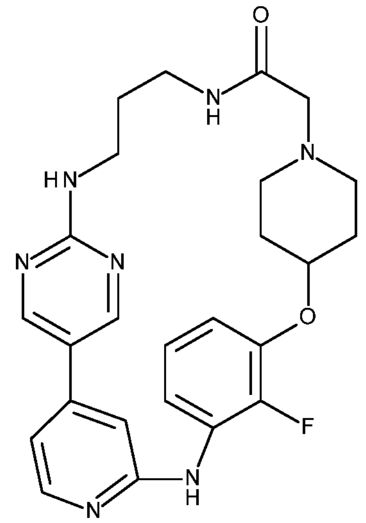 |
| Compound 124; Method C5.b | Compound 91; Method B5 |
| 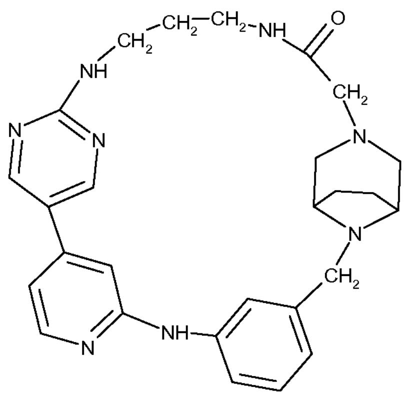 | 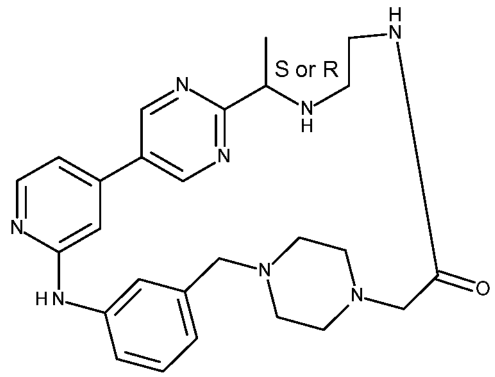 |
| Compound 41; Method B2 | Compound 133; Method C1 |
| 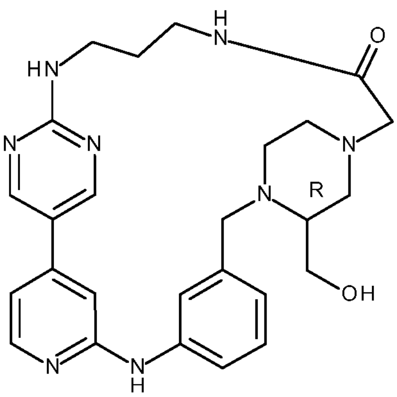 | 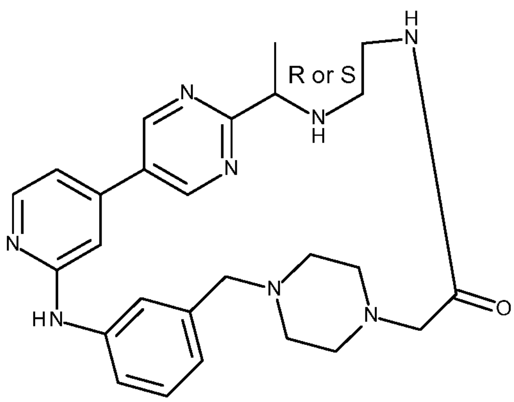 |
| Compound 8; Method B1 | Compound 134; Method C1 |

(continued)

| | |
|---|---|
| 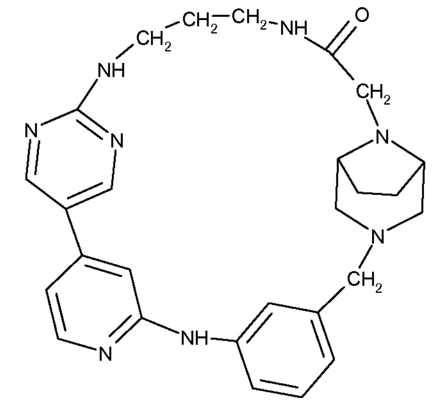 | 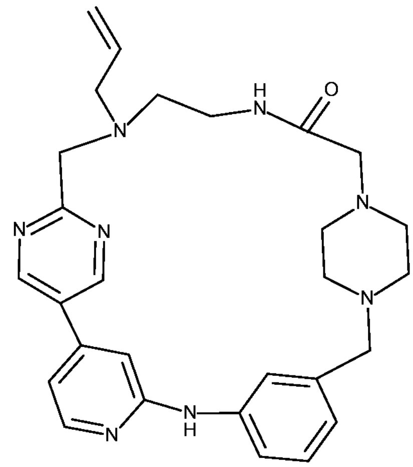 |
| Compound 42; Method B2 | Compound 130; Method B13 |
| 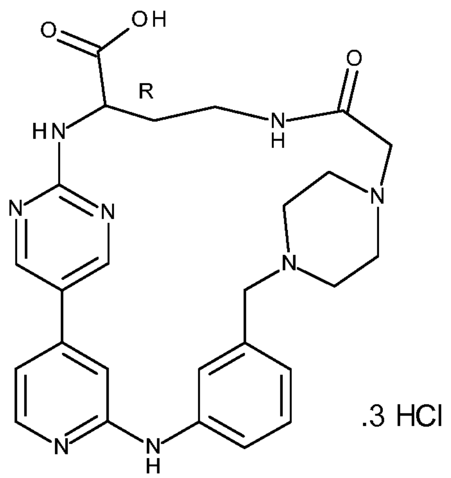 | 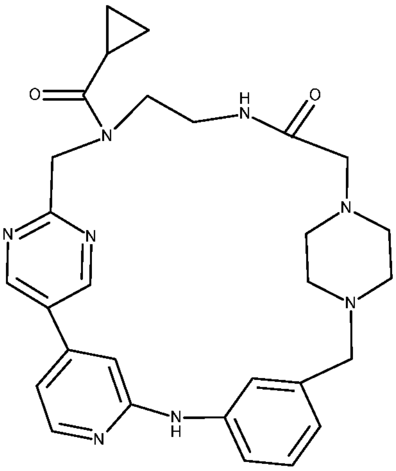 |
| Compound 119; Method C5.a | Compound 131; Method B13 |
| 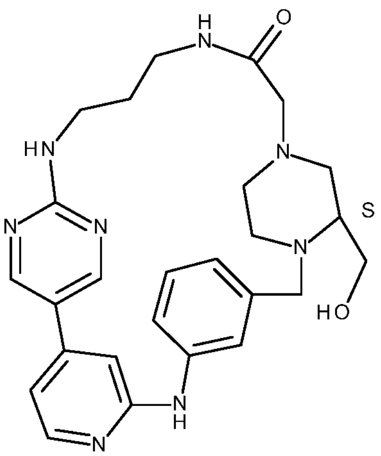 | 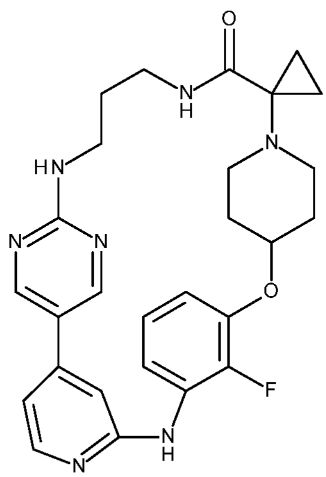 |
| Compound 7; Method B1 | Compound 89; Method B5 |

(continued)

| | |
|---|---|
| 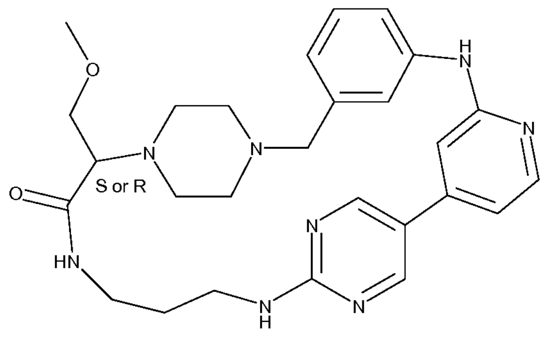 | 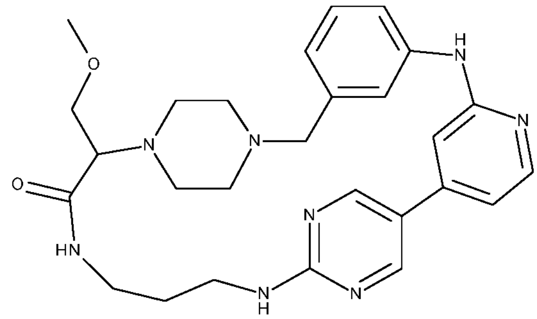 |
| Compound 6; Method C1 | Compound 31; Method B1 |
| 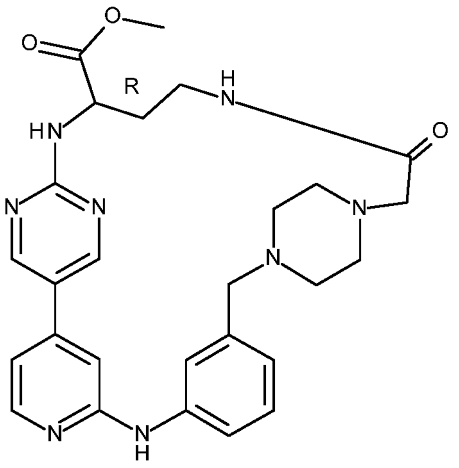 | 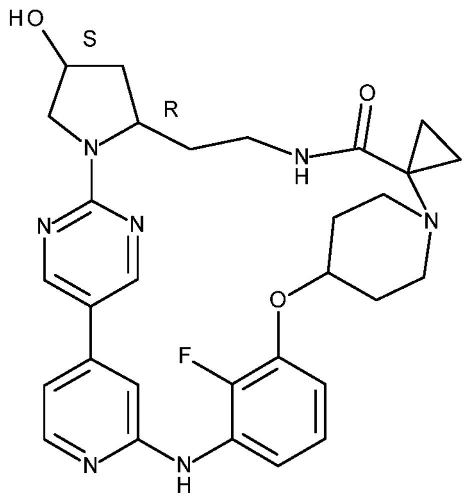 |
| Compound 118; Method B9 | Compound 96; Method B5 |
| 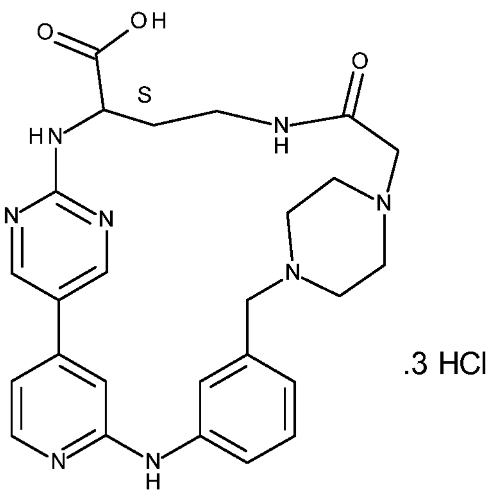 | 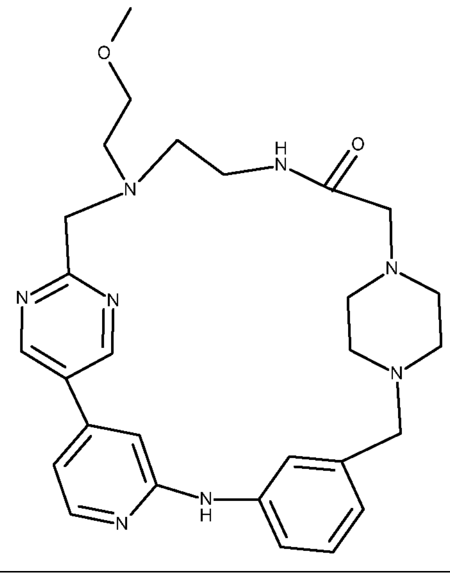 |
| Compound 117; Method C5.a | Compound 129; Method B13 |

(continued)

| | |
|---|---|
| 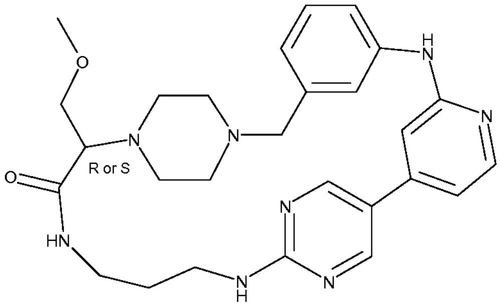 | 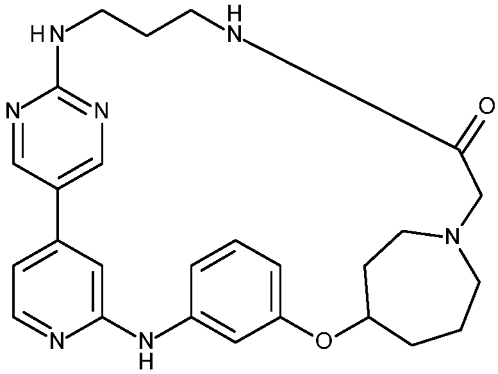 |
| Compound 5; Method C1 | Compound 94; Method B5 |
| 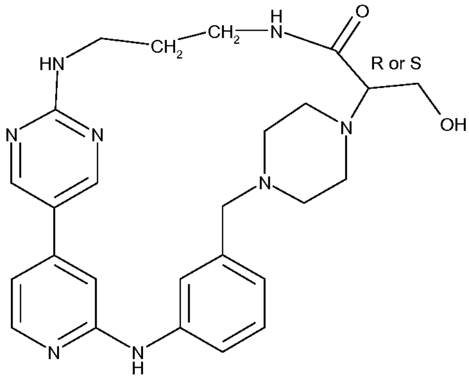 | 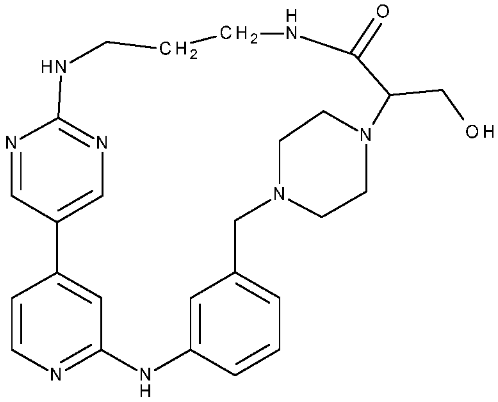 |
| Compound 3; Method C1 | Compound 30; Method B1 |
| 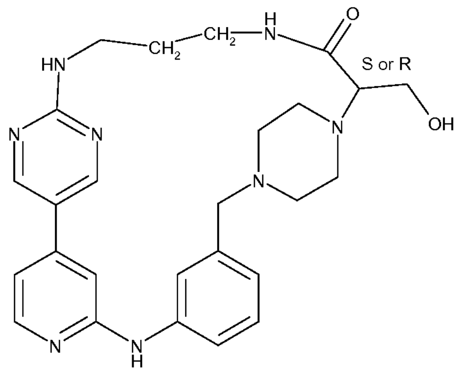 | 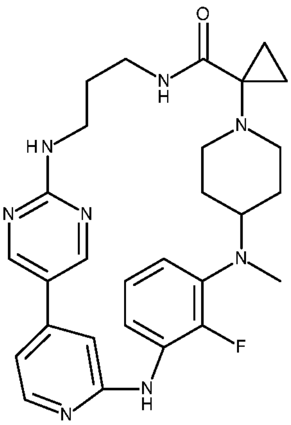 |
| Compound 2; Method C1 | Compound 103; Method B6 |

(continued)

| | |
|---|---|
| 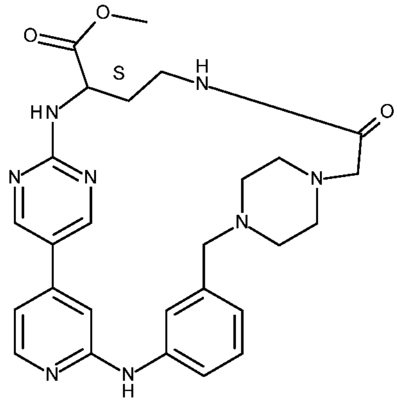 | 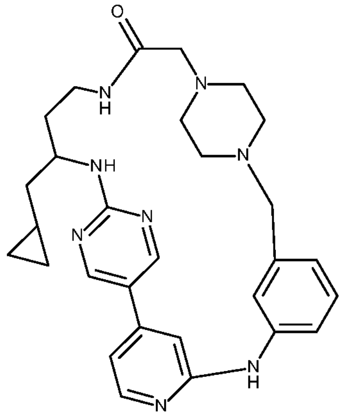 |
| Compound 116; Method B9 | Compound 125; Method B10 |
| 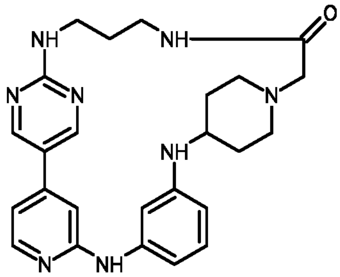 | 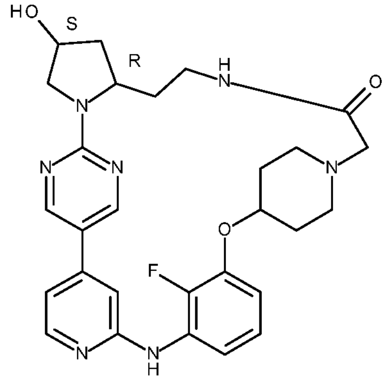 |
| Compound 106; Method B6 | Compound 98; Method B5a |
| 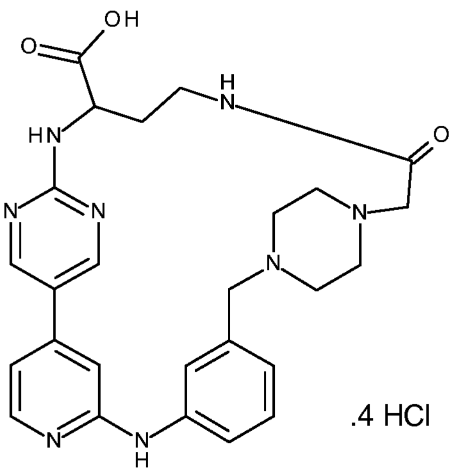 | 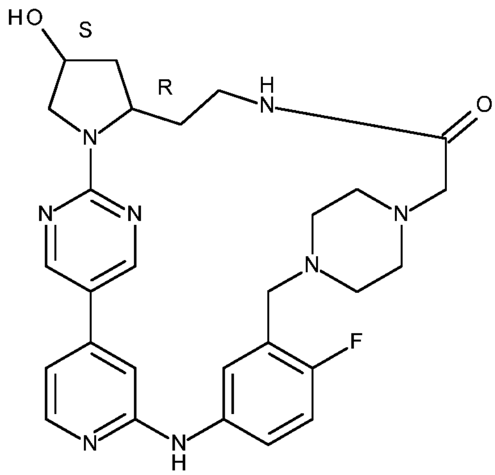 |
| Compound 120; Method C5.a | Compound 50; Method B3 |

(continued)

| | |
|---|---|
| 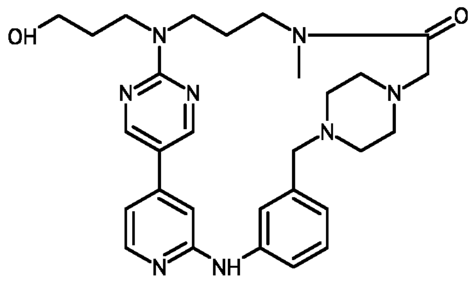 | 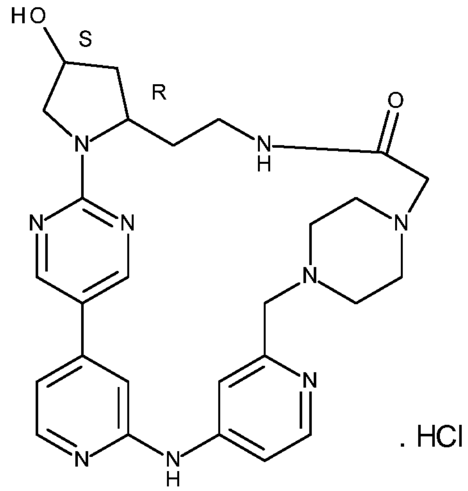 |
| Compound 16; Method B1 | Compound 51; Method B3 |
| 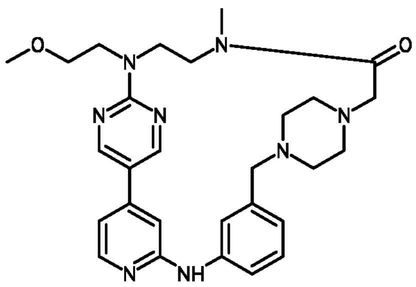 | 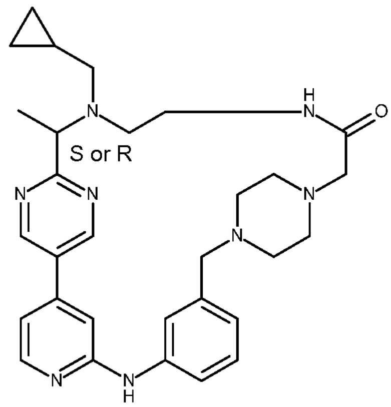 |
| Compound 15; Method B1 | Compound 136; Method C7 |
| 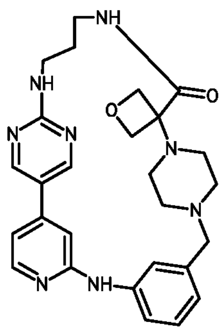 | 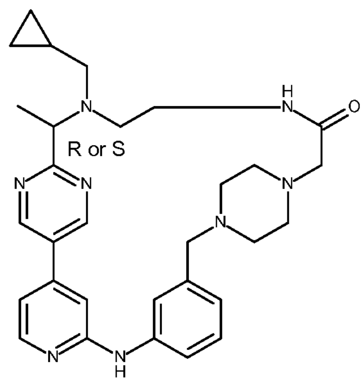 |
| Compound 10; Method B1 | Compound 137; Method C7 |

(continued)

| | |
|---|---|
| 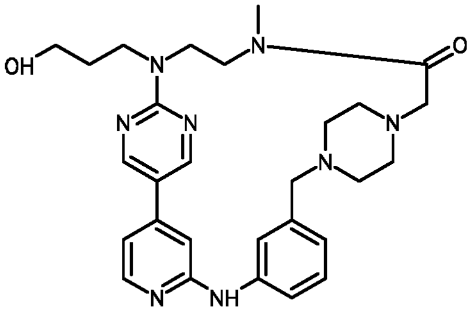 | 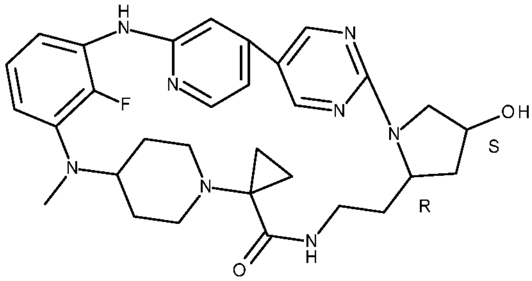 |
| Compound 17; Method B1 | Compound 105; Method B6 |
| 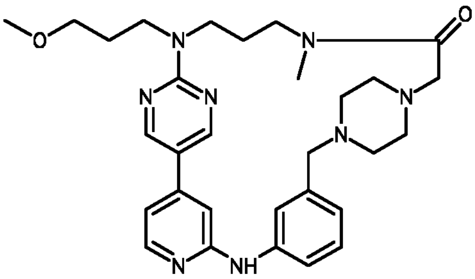 | 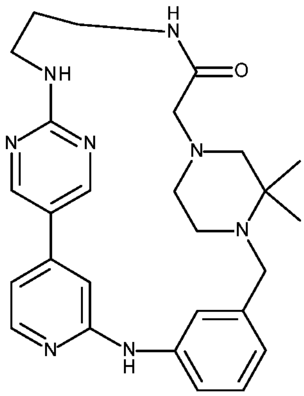 .3 HCl |
| Compound 14; Method B1 | Compound 19; Method B1 |
| 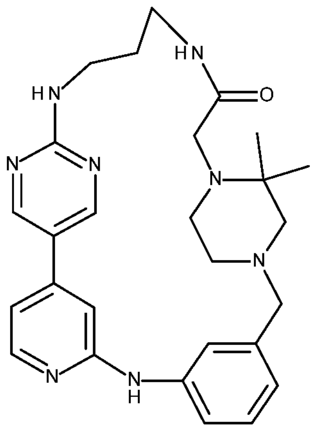 | 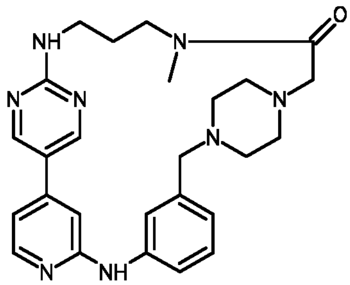 |
| Compound 18; Method B1 | Compound 13; Method B1 |
| 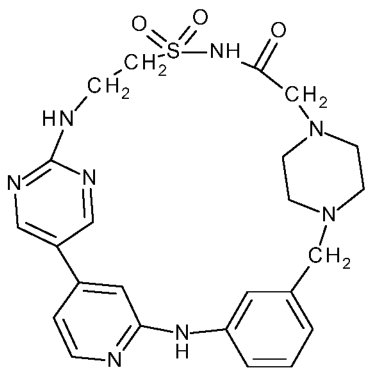 | 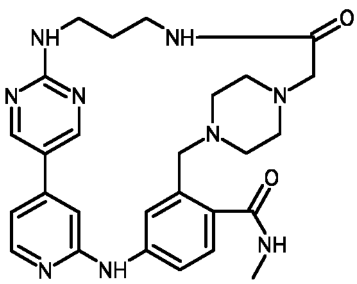 |

(continued)

| | |
|---|---|
| Compound 126; Method B11 | Compound 9; Method B1 |
| 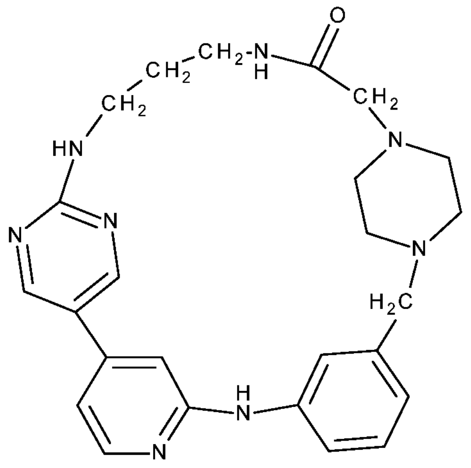 | 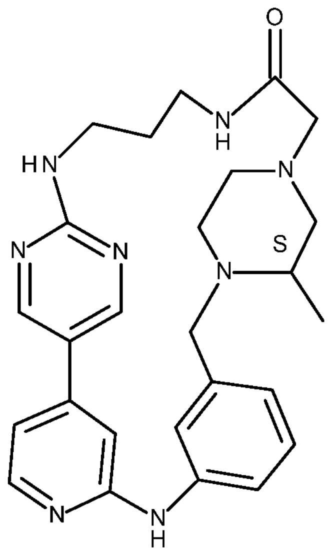 |
| Compound 33; Method B2 | Compound 36; Method B2 |
| 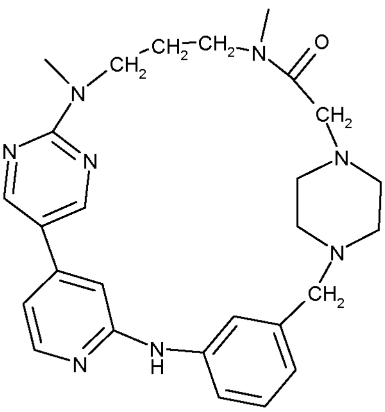 | 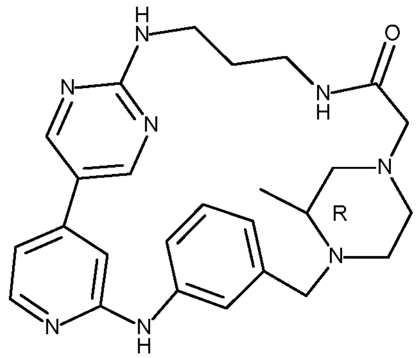 |
| Compound 38; Method B2 | Compound 40; Method B2 |
| 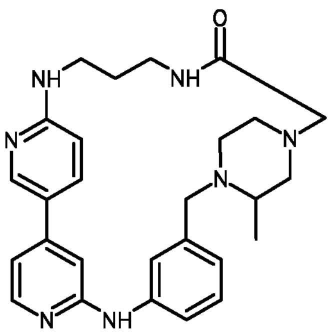 | 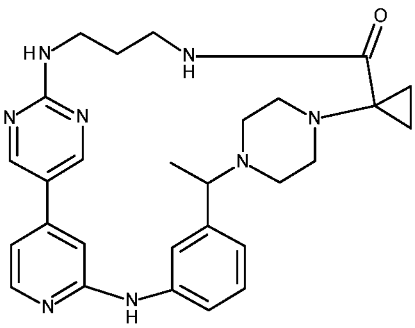 |
| Compound 71; Method B15 | Compound 21; Method B1 |

(continued)

| | |
|---|---|
| 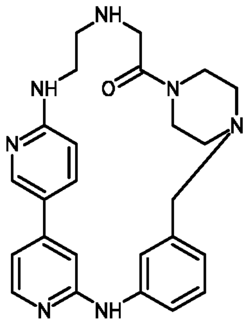 | 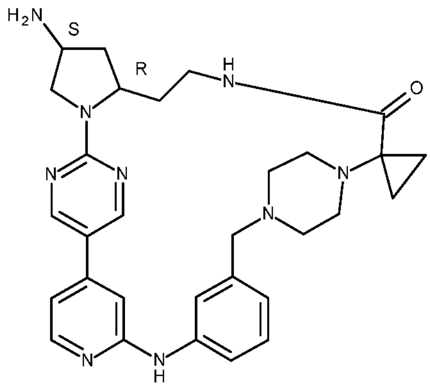 |
| Compound 144; Method B18 | Compound 85; Method C2.c |
| 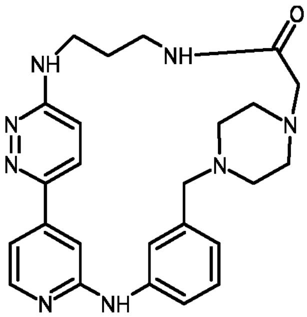 | 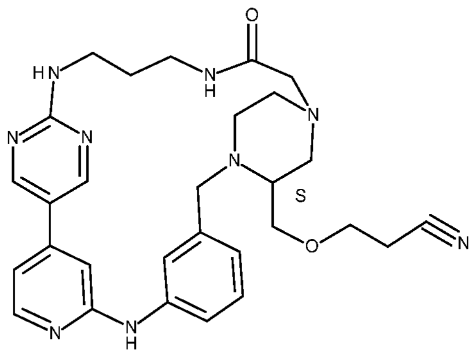 |
| Compound 37; Method B2 | Compound 29; Method B1 |
| 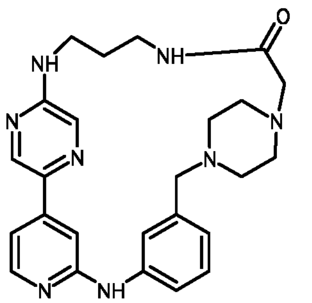 | 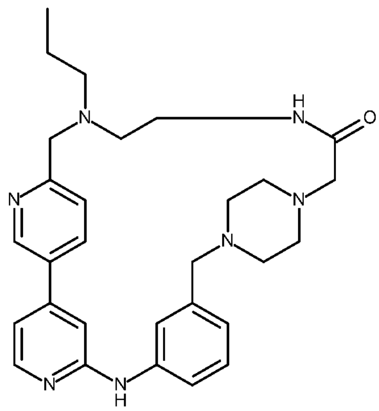 |
| Compound 39; Method B2 | Compound 147; Method C9 |
| 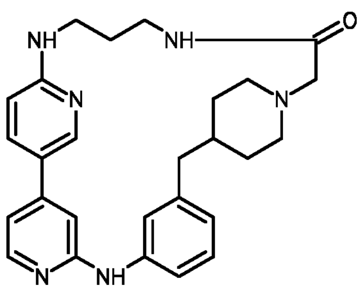 | 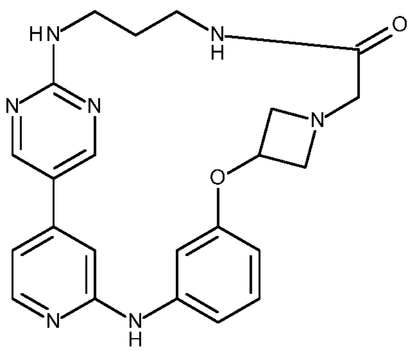 |
| Compound 140; Method B16 | Compound 92; Method B5 |

(continued)

| | |
|---|---|
| 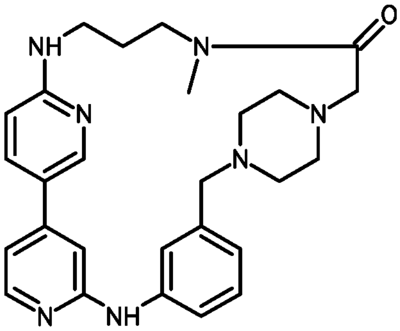 | 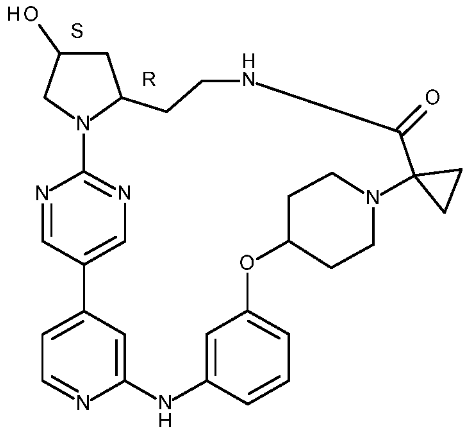 |
| Compound 69; Method B15 | Compound 97; Method B5 |
| 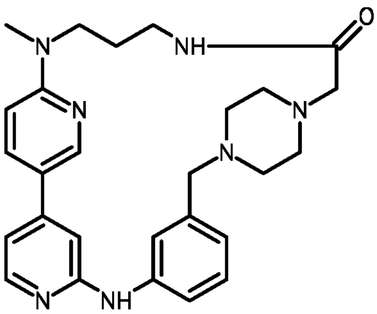 | 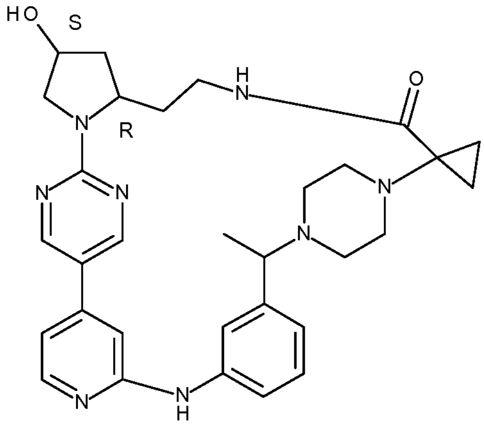 |
| Compound 72; Method B15 | Compound 58; Method B3 |
| 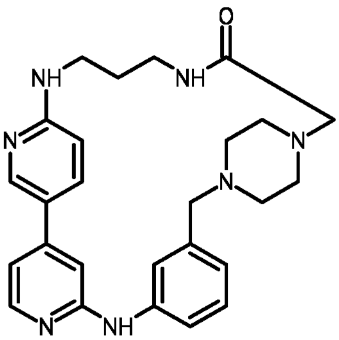 | 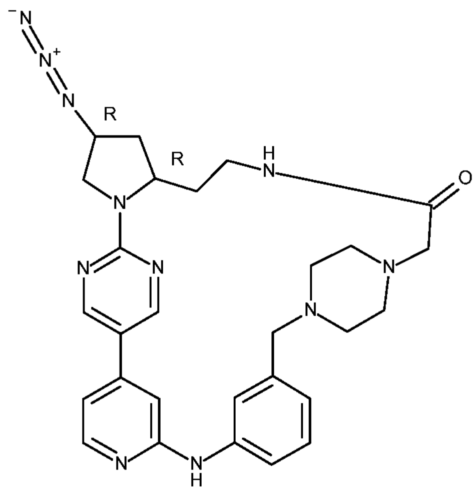 |
| Compound 67; Method B15 | Compound 82; Method C2.b |

(continued)

| | |
|---|---|
| 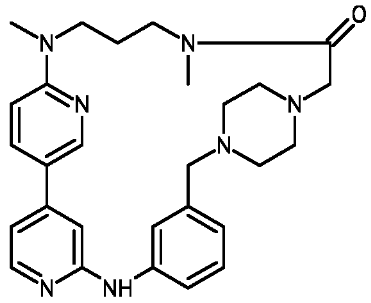 | 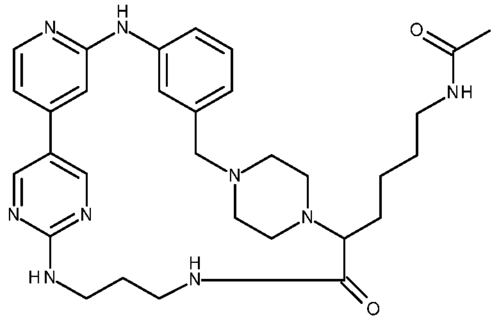 |
| Compound 68; Method B15 | Compound 108; Method B8 |
| 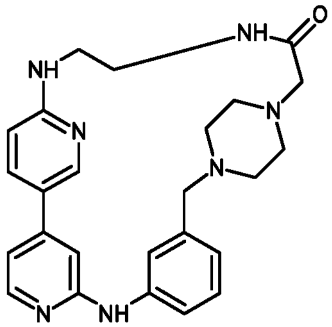 | 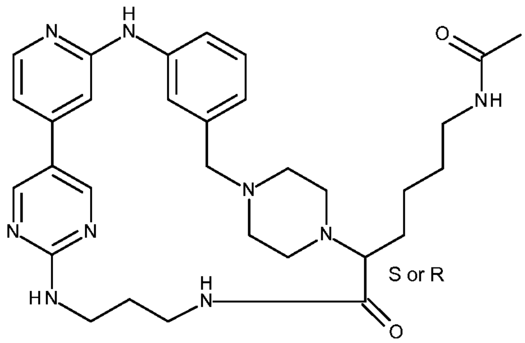 |
| Compound 70; Method B15 | Compound 110; Method C1 |
| 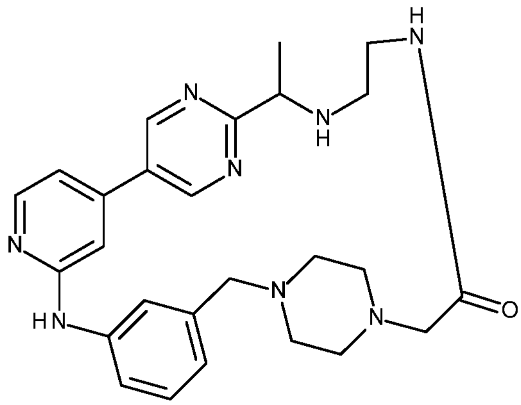 | 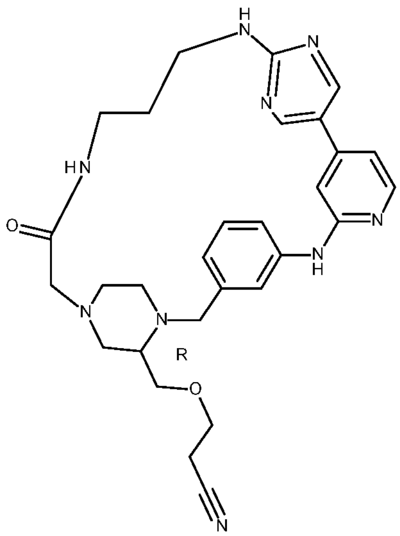 |
| Compound 132; Method B14 | Compound 111; Method B8 |

(continued)

| | |
|---|---|
| 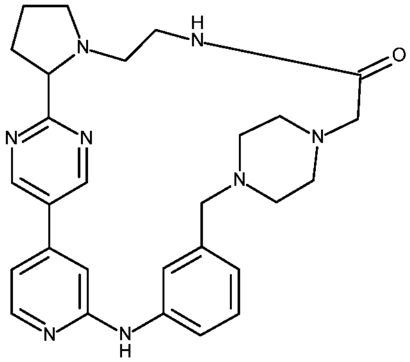 | 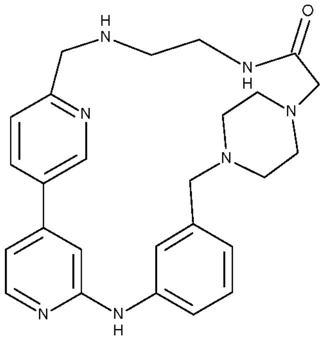 |
| Compound 135; Method B14 | Compound 145a; Method B19 |

Analytical Part

LCMS (Liquid Chromatography/Mass spectrometry)

*LCMS General procedure*

The High Performance Liquid Chromatography (HPLC) measurement was performed using a LC pump, a diode-array (DAD) or a UV detector and a column as specified in the respective methods. If necessary, additional detectors were included (see table of methods below).

Flow from the column was brought to the Mass Spectrometer (MS) which was configured with an atmospheric pressure ion source. It is within the knowledge of the skilled person to set the tune parameters (e.g. scanning range, dwell time...) in order to obtain ions allowing the identification of the compound's nominal monoisotopic molecular weight (MW). Data acquisition was performed with appropriate software.

Compounds are described by their experimental retention times ($R_t$) and ions. If not specified differently in the table of data, the reported molecular ion corresponds to the $[M+H]^+$ (protonated molecule) and/or $[M-H]^-$ (deprotonated molecule). In case the compound was not directly ionizable the type of adduct is specified (i.e. $[M+NH_4]^+$, $[M+HCOO]^-$, etc...). For molecules with multiple isotopic patterns (e.g. Br or Cl), the reported value is the one obtained for the lowest isotope mass. All results were obtained with experimental uncertainties that are commonly associated with the method used.

Hereinafter, "SQD" means Single Quadrupole Detector, "MSD" Mass Selective Detector, "RT" room temperature, "BEH" bridged ethylsiloxane/silica hybrid, "DAD" Diode Array Detector, "HSS" High Strength silica.

Table 2: LCMS Method codes (Flow expressed in mL/min; column temperature (T) in °C; Run time in minutes).

| LCMS Method | Instrument | Column | Mobile phase | gradient | Flow / Column T | Run time |
|---|---|---|---|---|---|---|
| 1 | Waters: Acquity® UPLC®-DAD and SQD | Waters : HSS T3 (1.8μm, 2.1* 100mm) | A: 10mM $CH_3COONH_4$ in 95% $H_2O$ + 5% $CH_3CN$ B: $CH_3CN$ | From 100% A to 5% A in 2.10min, to 0% A in 0.90min, to 5% A in 0.5min | 0.8 / 55 | 3.5 |
| 2 | Waters: Acquity® UPLC® - DAD and SQD | Waters : HSS T3 (1.8μm, 2.1*100mm) | A: 10mM $CH_3COONH_4$ in 95% $H_2O$ + 5% $CH_3CN$ B: $CH_3CN$ | From 100% A to 5% A in 2.10min, to 0% A in 0.90min, to 5% A in 0.5min | 0.7 / 55 | 3.5 |

(continued)

| LCMS Method | Instrument | Column | Mobile phase | gradient | Flow / Column T | Run time |
|---|---|---|---|---|---|---|
| 3 | Waters: Acquity UPLC® - DAD and Quattro | Waters: BEH C18 (1.7μm, 2.1x100mm) | A: 95% $CH_3COONH_4$ 7mM / 5% | 84.2% A for 0.49min, to 10.5% A in 2.18min, held | 0.343 / 40 | 6.2 |
| | Micro™ | | $CH_3CN$, B: $CH_3CN$ | for 1.94min, back to 84.2% A in 0.73min, held for 0.73min. | | |
| 4 | Agilent 1100-UV 220nm | YMC-PACK ODS-AQ, 50×2.0mm 5μm | A: 0.1% TFA in $H_2O$ B: 0.05 TFA in $CH_3CN$ | 100% A held for 1min from 100% A to 40% A in 4 min, held for 2.5 min, to 100% A in 0.5 min held for 2min. | 0.8 / 50 | 10.0 |
| 5 | Agilent 1100-UV 220nm | XBridge ShieldRP18, 50*2.1mm 5μm | A: 0.05%$NH_3$ in $H_2O$ B: $CH_3CN$ | 100% A held for 1min from 100% to 40% A in 4 min, held for 2.5 min, to 100% A in 0.5 min held for 2min. | 0.8 / 40 | 10.0 |
| 6 | Waters: Acquity® UPLC®-DAD and SQD | Waters : BEH C18 (1.7μm, 2.1*50mm) | A: 0.1% HCOOH + 5% $CH_3OH$ in $H_2O$ B: $CH_3CN$ | From 95% A to 0% A in 2.5 min, to 5% A in 0.5min. | 0.8 / 55 | 3 |

Melting Points

For compounds 14, 15, 70, 71 and 144, melting points (m.p.) were obtained with a Kofler hot bench, consisting of a heated plate with linear temperature gradient, a sliding pointer and a temperature scale in degrees Celsius.

For compounds 7 and 106, melting points were determined with a WRS-2A melting point apparatus that was purchased from Shanghai Precision and Scientific Instrument Co. Ltd. Melting points were measured with a linear heating up rate of 0.2-5.0 °C/minute The reported values are melt ranges. The maximum temperature was 300 °C.

The results of the analytical measurements are shown in table 3.

**Table 3:** Retention time ($R_t$) in min., $[M+H]^+$ peak (protonated molecule), LCMS method and m.p. (melting point in °C).

| Co. No. | $R_t$ | $[M+H]^+$ | LCMS Method | m.p. (°C) |
|---|---|---|---|---|
| 1 | 1.30 | 477 | 1 | |
| 2 | 1.23 | 489 | 2 | |
| 3 | 1.23 | 489 | 2 | |
| 5 | 1.35 | 503 | 1 | |
| 6 | 1.35 | 503 | 1 | |
| 7 | 1.17 | 489 | 1 | 267.8-267.9 |
| 8 | 1.17 | 489 | 1 | |
| 9 | 1.23 | 516 | 1 | |
| 10 | 1.35 | 501 | 1 | |

| Co. No. | $R_t$ | $[M+H]^+$ | LCMS Method | m.p. (°C) |
|---|---|---|---|---|
| 11 | 1.65 | 517 | 1 | |
| 12 | 1.65 | 517 | 1 | |
| 13 | 1.28 | 473 | 1 | |
| 14 | 1.78 | 545 | 2 | 240 |
| 15 | 1.52 | 517 | 1 | 240 |
| 16 | 1.42 | 531 | 1 | |
| 17 | 1.33 | 517 | 1 | |
| 18 | 1.68 | 487 | 1 | |
| 19 | 1.53 | 487 | 1 | |
| 20 | 1.60 | 503 | 1 | |
| 22 | 1.66 | 499 | 1 | |
| 23 | 1.66 | 499 | 1 | |
| 25 | 1.36 | 485 | 1 | |
| 26 | 1.36 | 485 | 1 | |
| 27 | 1.56 | 485 | 1 | |
| 28 | 1.66 | 485 | 1 | |
| 33 | 1.29 | 459 | 1 | |
| 34 | 1.52 | 487 | 2 | |
| 35 | 1.42 | 487 | 1 | |
| 36 | 1.37 | 473 | 1 | |
| 37 | 1.21 | 459 | 1 | |
| 38 | 1.57 | 487 | 1 | |
| 39 | 1.31 | 459 | 1 | |
| 40 | 1.37 | 473 | 1 | |
| 41 | 1.23 | 485 | 1 | |
| 42 | 1.61 | 485 | 1 | |
| 43 | 1.63 | 499 | 1 | |
| 44 | 1.45 | 529 | 1 | |
| 45 | 1.63 | 499 | 1 | |
| 46 | 1.47 | 529 | 1 | |
| 47 | 1.75 | 513 | 1 | |
| 48 | 1.7 | 513 | 1 | |
| 49 | 1.25 | 485 | 1 | |
| 50 | 1.38 | 533 | 1 | |
| 51 | 1.10 | 516 | 1 | |
| 52 | 1.63 | 573 | 1 | |
| 53 | 1.55 | 559 | 1 | |
| 54 | 1.35 | 515 | 1 | |
| 55 | 1.31 | 515 | 1 | |

| Co. No. | $R_t$ | $[M+H]^+$ | LCMS Method | m.p. (°C) |
|---|---|---|---|---|
| 56 | 1.53 | 541 | 1 | |
| 57 | 1.54 | 541 | 1 | |
| 59 | 1.43 | 529 | 2 | |
| 60 | 1.34 | 529 | 1 | |
| 61 | 1.46 | 529 | 1 | |
| 62 | 1.41 | 529 | 1 | |
| 63 | 1.35 | 557 | 1 | |
| 64 | 1.34 | 557 | 1 | |
| 65 | 1.60 | 555 | 1 | |
| 66 | 1.61 | 555 | 1 | |
| 67 | 1.37 | 458 | 1 | |
| 68 | 1.61 | 486 | 1 | |
| 69 | 2.07 | 472 | 3 | |
| 70 | 1.27 | 444 | 1 | 252 |
| 71 | 1.46 | 472 | 1 | 162 |
| 72 | 1.66 | 472 | 1 | |
| 73 | 1.56 | 485 | 1 | |
| 74 | 1.50 | 487 | 1 | |
| 75 | 1.36 | 473 | 1 | |
| 76 | 1.35 | 473 | 1 | |
| 77 | 1.49 | 524 | 1 | |
| 78 | 1.58 | 524 | 2 | |
| 79 | 1.75 | 550 | 1 | |
| 80 | 1.54 | 517 | 1 | |
| 81 | 1.69 | 497 | 1 | |
| 82 | 1.65 | 540 | 1 | |
| 83 | 1.99 | 566 | 1 | |
| 84 | 1.23 | 514 | 1 | |
| 85 | 1.38 | 540 | 1 | |
| 86 | 1.33 | 593 | 1 | |
| 87 | 1.50 | 619 | 1 | |
| 88 | 1.49 | 513 | 2 | |
| 89 | 1.69 | 504 | 1 | |
| 90 | 1.45 | 460 | 1 | |
| 91 | 1.42 | 478 | 1 | |
| 92 | 1.42 | 432 | 1 | |
| 93 | 1.49 | 446 | 1 | |
| 94 | 1.61 | 474 | 1 | |
| 95 | 1.75 | 486 | 1 | |

| Co. No. | $R_t$ | $[M+H]^+$ | LCMS Method | m.p. (°C) |
|---|---|---|---|---|
| 96 | 1.71 | 560 | 2 | |
| 97 | 1.72 | 542 | 1 | |
| 98 | 1.49 | 534 | 2 | |
| 99 | 1.47 | 516 | 1 | |
| 100 | 1.62 | 485 | 1 | |
| 101 | 1.44 | 473 | 1 | |
| 102 | 1.88 | 499 | 1 | |
| 103 | 1.85 | 517 | 1 | |
| 104 | 1.80 | 555 | 1 | |
| 105 | 1.78 | 573 | 1 | |
| 106 | 1.34 | 459 | 1 | 255.3-256.8 |
| 107 | 1.62 | 485 | 1 | |
| 109 | 1.28 | 572 | 2 | |
| 110 | 3.31 | 572 | 4 | |
| 111 | 3.56 | 542 | 4 | |
| 112 | 3.17 | 530 | 4 | |
| 113 | 3.15 | 530 | 4 | |
| 114 | 1.11 | 546 | 1 | |
| 115 | 1.24 | 588 | 1 | |
| 116 | 1.26 | 517 | 1 | |
| 117 | 0.92 | 503 | 1 | |
| 118 | 1.26 | 517 | 1 | |
| 119 | 0.92 | 503 | 1 | |
| 120 | 0.96 | 503 | 2 | |
| 121 | 1.12 | 502 | 1 | |

| Co. No. | $R_t$ | $[M+H]^+$ | LCMS Method | m.p. (°C) |
|---|---|---|---|---|
| 122 | 1.18 | 516 | 1 | |
| 123 | 1.15 | 502 | 2 | |
| 124 | 1.17 | 516 | 1 | |
| 125 | 1.56 | 513 | 6 | |
| 126 | 0.97 | 509 | 1 | |
| 128 | 1.48 | 513 | 1 | |
| 129 | 1.31 | 517 | 1 | |
| 130 | 1.45 | 499 | 1 | |
| 131 | 1.27 | 527 | 1 | |
| 133 | 4.24 | 473 | 5 | |
| 134 | 4.28 | 473 | 5 | |
| 136 | 1.62 | 527 | 1 | |
| 137 | 1.62 | 527 | 1 | |
| 138 | 1.32 | 499 | 2 | |
| 139 | 1.34 | 499 | 2 | |
| 140 | 1.67 | 457 | 1 | |
| 141 | 2.64 | 498 | 3 | |
| 142 | 1.74 | 498 | 1 | |
| 143 | 1.74 | 498 | 1 | |
| 144 | 1.21 | 444 | 1 | 143 |
| 145 | 1.15 | 458 | 1 | |
| 146 | 1.56 | 512 | 1 | |
| 147 | 1.67 | 500 | 2 | |
| 148 | 1.11 | 546 | 2 | |
| 149 | 1.24 | 588 | 1 | |

OR (Optical Rotation)

Compound 2: +140° (590 nm; 20 °C; 2.21 w/v %; DCM)
Compound 3: -142° (590 nm; 20 °C; 2.11 w/v %; DCM)
Compound 5: -16.7° (589 nm; 20 °C; 5.10 w/v %; DCM)
Compound 6: +10.02° (589 nm; 20 °C; 3.12 w/v %; DCM)
Compound 11: -57° (589 nm; 20 °C; 0.018 w/v %; MeOH/DCM 1/2)
Compound 12: -134° (589 nm; 20 °C; 0.016 w/v %; MeOH/DCM 1/2)
Compound 22: +48.5° (589 nm; 20 °C; 0.2 w/v %; DCM)
Compound 23: -47.5° (589 nm; 20 °C; 0.16 w/v %; DCM)
Compound 43: +118.14° (589 nm; 20 °C; 0.25225 w/v %; DMSO)
Compound 65: +154.17° (589 nm; 20 °C; 0.072 w/v %; MeOH)
Compound 66: +100.00° (589 nm; 20 °C; 0.060 w/v %; MeOH)
Compound 105: +294° (589 nm; 20 °C; 0.12 w/v %; 30 % MeOH in DCM)
Compound 133: -276° (589 nm; 20 °C; 0.308 w/v %; MeOH)

Compound 134: -4.5° (589 nm; 20 °C; 0.150 w/v %; MeOH)
Compound 138: +24.62° (589 nm; 20 °C; 0.052 w/v %; DCM)
Compound 139: -57.27° (589 nm; 20 °C; 0.044 w/v %; DCM)
Compound 142: +128.99° (589 nm; 20 °C; 0.238 w/v %; DMF)
Compound 143: -127.78° ((589 nm; 20 °C; 0.234 w/v %; DMF)

SFC-MS

For SFC-MS, an analytical SFC system from Berger Instruments (Newark, DE, USA) was used comprising a dual pump control module (FCM-1200) for delivery of $CO_2$ and modifier, a thermal control module for column heating (TCM2100) with temperature control in the range 1-150 °C and column selection valves (Valco, VICI, Houston, TX, USA) for 6 different columns. The photodiode array detector (Agilent 1100, Waldbronn, Germany) is equipped with a high-pressure flow cell (up to 400 bar) and configured with a CTC LC Mini PAL auto sampler (Leap Technologies, Carrboro, NC , USA). A ZQ mass spectrometer (Waters, Milford, MA, USA) with an orthogonal Z-electrospray interface is coupled with the SFC-system. Instrument control, data collection and processing were performed with an integrated platform consisting of the SFC ProNTo software and Masslynx software.

Co. No. 112-113: SFC-MS was carried out on a OD-H column (250 x 4.6 mm) (Daicel Chemical Industries Ltd) with a flow rate of 3 ml/min. Two mobile phases (mobile phase A: $CO_2$; mobile phase B: MeOH containing 0.2 % isopropylamine ($iPrNH_2$)) were employed. A gradient was applied from 10 % B to 40 % B in 18.75 min. Then a gradient was applied from 40 % B to 50 % B in 2 min, and 50 % B was hold for 3.6 min. Column temperature was set at 30°C. Under these conditions, Co. No. 113 had a shorter $R_t$ on the column than Co. No. 112. The measurement was compared against the mixture of the compounds. NMR

For a number of compounds, $^1$H NMR spectra were recorded on a Bruker DPX-400 spectrometer operating at 400 MHz, on a Bruker DPX-360 operating at 360 MHz, on a Bruker Avance 600 spectrometer operating at 600 MHz, or a Bruker Avance 500 III operating at 500 MHz using internal deuterium lock. As solvents CHLOROFORM-*d* (deuterated chloroform, $CDCl_3$) or DMSO-$d_6$ (deuterated DMSO, dimethyl-d6 sulfoxide) were used. Chemical shifts (δ) are reported in parts per million (ppm) relative to tetramethylsilane (TMS), which was used as internal standard.

Compound 1:
$^1$H NMR (360 MHz, DMSO-$d_6$) δ ppm 1.62 (br. s., 2 H) 2.58 (br. s., 8 H) 2.91 (br. s., 3 H) 3.23 (br. s., 3 H) 3.62 (s, 2 H) 6.35 (d, *J*=55 Hz, 1 H) 7.09 (dd, *J*=5.3, 1.3 Hz, 1 H) 7.15 - 7.28 (m, 3 H) 7.62 (t, *J*=6.2 Hz, 1 H) 7.71 (t, *J*=6.2 Hz, 1 H) 8.15 (d, *J*=5.5 Hz, 1 H) 8.32 (br. s., 1 H) 8.69 (br. s., 1 H) 8.82 (s, 1 H)

Compound 13: $^1$H NMR (400 MHz, DMSO-$d_6$) δ ppm 1.51 - 1.89 (m, 2 H) 2.17 - 2.56 (m, 8 H-partially obscured by solvent) 2.70 - 3.16 (m, 6 H) 3.22 - 3.44 (m, 5 H-partially obscured by solvent) 6.68 - 7.08 (m, 3 H) 7.10 - 7.33 (m, 2 H) 7.33 - 7.46 (m, 1 H) 7.47 - 7.65 (m, 1 H) 8.01 - 8.25 (m, 1 H) 8.31 - 8.70 (m, 2 H) 8.72 - 8.92 (m, 1 H)

Compound 14: $^1$H NMR (500 MHz, DMSO-$d_6$) δ ppm 1.58 - 1.97 (m, 4 H) 2.26 - 2.67 (m, 9 H-partially obscured by solvent) 2.99 - 3.27 (m, 8 H) 3.37 - 3.44 (m, 5 H) 3.48 - 3.79 (m, 4 H) 6.67 - 7.32 (m, 5 H) 7.34 - 7.52 (m, 1 H) 8.06 - 8.25 (m, 1 H) 8.28 - 9.07 (m, 3 H)

Compound 15: $^1$H NMR (500 MHz, DMSO-$d_6$) δ ppm 2.23 - 3.30 (m, 16 H-partially obscured by solvent) 3.37 - 5.13 (m, 10 H) 6.67 - 7.31 (m, 5 H) 7.36 - 7.52 (m, 1 H) 8.12 - 8.25 (m, 1 H) 8.35 - 8.78 (m, 2 H) 8.80 - 9.02 (m, 1 H)

Compound 16: $^1$H NMR (500 MHz, DMSO-$d_6$) δ ppm 1.62 - 1.96 (m, 4 H) 1.99 - 2.82 (m, 9 H-partially obscured by solvent) 3.04 - 3.17 (m, 4 H) 3.21 - 3.32 (m, 2 H) 3.39 - 3.50 (m, 4 H) 3.52 - 3.75 (m, 4 H) 4.52 (m, 1 H) 6.70 - 7.63 (m, 6 H) 8.05 - 8.26 (m, 1 H) 8.34 - 9.06 (m, 3 H)

Compound 17: $^1$H NMR (500 MHz, DMSO-$d_6$) δ ppm 1.62 - 1.83 (m, 2 H) 2.59 - 3.31 (m, 9 H) 3.40 - 3.52 (m, 3 H) 3.62 - 4.08 (m, 7 H) 4.12 - 4.99 (m, 3 H) 6.23 - 7.73 (m, 6 H) 8.03 - 8.28 (m, 1 H) 8.35 - 9.15 (m, 2 H) 9.72 (br. s, 1 H)

Compound 33 :
$^1$H NMR (400 MHz, CHLOROFORM-*d*) δ ppm 1.76 - 1.86 (m, 2 H) 2.56 - 2.75 (m, 8 H) 3.07 (s, 2 H) 3.33 - 3.42 (m, 2 H) 3.49 (q, *J*=7.1 Hz, 2 H) 3.55 (s, 2 H) 5.55 (t, *J*=6.7 Hz, 1 H) 6.80 (s, 1 H) 6.90 (dd, *J*=5.2, 1.6 Hz, 1 H) 6.93 - 7.00 (m, 1 H) 7.12 (d, *J*=0.8 Hz, 1 H) 7.16 (ddd, *J*=7.5, 1.0, 0.8 Hz, 1 H) 7.33 - 7.44 (m, 3 H) 8.22 (d, *J*=5.2 Hz, 1 H) 8.52 (s, 2 H)

Compound 43: $^1$H NMR (400 MHz, DMSO-$d_6$) δ ppm 1.39 (dddd, *J*=15.5, 7.7, 5.0, 2.4 Hz, 1 H) 1.81 - 1.92 (m, 2 H) 1.93 - 2.06 (m, 3 H) 2.55 - 2.75 (m, 8 H) 2.84 (d, *J*=15.7 Hz, 1 H) 2.95 - 3.02 (m, 1 H) 2.99 (d, *J*=15.7 Hz, 1 H) 3.31 (d, *J*=12.5 Hz, 1 H) 3.43 - 3.61 (m, 3 H) 3.66 (d, *J*=12.1 Hz, 1 H) 4.04 - 4.18 (m, 1 H) 6.96 - 7.05 (m, 3 H) 7.08 (s, 1 H) 7.29 (t, *J*=7.7 Hz, 1 H) 7.35 (s, 1 H) 7.44 (br. s., 1 H) 8.15 (d, *J*=5.2 Hz, 1 H) 8.33 (s, 1 H) 8.59 (s, 2 H)

Compound 67 : $^1$H NMR (500 MHz, DMSO-$d_6$) δ ppm 1.55 - 1.65 (m, 2 H) 2.53 - 2.71 (m, 8 H) 2.90 (s, 2 H) 3.10 - 3.15 (m, 2 H) 3.22 - 3.30 (m, 2 H) 3.43 (s, 2 H) 6.46 (d, *J*=8.8 Hz, 1 H) 6.90 - 7.03 (m, 4 H) 7.18 (s, 1 H) 7.30 (t, *J*=8.8 Hz, 1 H) 7.35 (br. s, 1 H) 7.65 (t, *J*=6.0 Hz, 1 H) 7.72 (dd, *J*=8.8, 2.2 Hz, 1 H) 8.12 (d, *J*=5.4 Hz, 1 H) 8.22 (d, *J*=1.6 Hz, 1 H) 8.76 (s, 1 H)

Compound 68 : $^1$H NMR (400 MHz, DMSO-$d_6$) δ ppm 1.50 - 1.98 (m, 2 H) 2.06 - 2.71 (m, 8 H-partially obscured by solvent peak) 2.75 - 3.27 (m, 10 H) 3.33 - 3.45 (m, 2 H) 3.48 - 3.73 (m, 2 H) 6.37 - 7.06 (m, 4 H) 7.10 - 7.46 (m, 3 H) 7.64 - 7.91 (m, 1 H) 8.03 - 8.22 (m, 1 H) 8.27 - 8.42 (m, 1 H) 8.65 - 8.90 (m, 1 H)

Compound 69 : $^1$H NMR (400 MHz, DMSO-$d_6$) δ ppm 1.66 - 1.93 (m, 2 H) 2.34 - 2.47 (m, 8 H) 2.74 - 2.97 (m, 4 H) 3.27-3.37 (m, 5 H) 3.40 (br. s., 2 H) 6.43 - 6.57 (m, 1 H) 6.59 - 6.70 (m, 1 H) 6.75 - 7.03 (m, 3 H) 7.13 (br. s, 1 H) 7.17 - 7.31 (m, 1 H) 7.39 (br. s., 1 H) 7.63 (d, *J*=8.6 Hz, 1 H) 8.14 (d, *J*=5.1 Hz, 1 H) 8.23 (br. s, 1 H) 8.40 (br. s., 1 H)

Compound 70 : $^1$H NMR (500 MHz, DMSO-$d_6$) δ ppm 2.03 - 2.45 (m, 8 H) 2.72 (s, 2H) 3.18 - 3.27 (m, 2 H) 3.45 (br. s, 2 H) 3.50 - 3.55 (m, 2 H) 6.46 (d, *J*=8.8 Hz, 1 H) 6.74 (t, *J*=5.2 Hz, 1 H) 6.83 - 6.88 (m, 1 H) 6.91 (dd, *J*=5.2, 1.4 Hz, 1 H) 6.94 (dd, *J*=8.8, 1.3 Hz, 1 H) 7.06 (s, 1 H) 7.26 (t, *J*=8.8 Hz, 1 H) 7.35 (s, 1 H) 7.56-7.61 (m, 2 H) 8.13 (d, *J*=5.2 Hz, 1 H) 8.15 (d, *J*=2.2 Hz, 1 H) 8.78 (s, 1 H)

Compound 72 : $^1$H NMR (500 MHz, DMSO-$d_6$) δ ppm 1.55 - 1.70 (m, 2 H) 2.53 - 2.72 (m, 8 H-partially obscured by solvent peak) 2.91 (s, 2 H) 2.99 (s, 3 H) 3.10 - 3.22 (m, 2 H) 3.43 (s, 2 H) 3.47 - 3.55 (m, 2 H) 6.60 (d, *J*=8.8 Hz, 1 H) 6.95 - 7.00 (m, 2 H) 7.02 (dd, *J*=5.4, 1.3 Hz, 1 H) 7.20 (br. s, 1 H) 7.30 (t, *J*=8.8 Hz, 1 H) 7.35 (br. s, 1 H) 7.73 (t, *J*=6.3 Hz, 1 H) 7.85 (dd, *J*=8.8, 2.4 Hz, 1 H) 8.14 (d, *J*=5.4 Hz, 1 H) 8.34 (d, *J*=2.2 Hz, 1 H) 8.77 (s, 1 H)

Compound 76: $^1$H NMR (360 MHz, DMSO-$d_6$) δ ppm 1.33 (d, *J*=6.2 Hz, 3 H) 1.52 - 1.68 (m, 2 H) 2.53 (br. s., 8 H) 2.78 (d, *J*=15.4 Hz, 1 H) 2.96 (d, *J*=15.7 Hz, 1 H) 2.99 - 3.09 (m, 1 H) 3.09 - 3.19 (m, 1 H) 3.21 - 3.32 (m, 2 H) 3.44 - 3.55 (m, 1 H) 6.90 - 7.09 (m, 4 H) 7.25 - 7.39 (m, 2 H) 7.50 - 7.59 (m, 1 H) 7.62 - 7.72 (m, 1 H) 8.15 (d, *J*=5.1 Hz, 1 H) 8.45 (br. s., 1 H) 8.66 (br. s., 1 H) 8.78 (s, 1 H)

Compound 83: $^1$H NMR (400 MHz, DMSO-$d_6$) δ ppm 0.77 - 0.97 (m, 2 H) 0.97 - 1.10 (m, 2 H) 1.48 - 1.66 (m, 1 H) 1.94 (ddd, *J*=13.8, 2.8, 2.7 Hz, 1 H) 2.17 - 2.32 (m, 1 H) 2.32 - 2.41 (m, 1 H) 2.41 - 2.47 (m, 2 H) 2.55 - 2.70 (m, 6 H) 2.88 - 3.02 (m, 1 H) 3.11 (br. s., 1 H) 3.54 (dd, *J*=12.5, 2.0 Hz, 1 H) 3.61 - 3.67 (m, 1 H) 3.70 (d, *J*=11.7 Hz, 1 H) 3.89 (dd, *J*=12.5, 6.1 Hz, 1 H) 4.01 - 4.17 (m, 1 H) 4.47 (tdd, *J*=6.0, 6.0, 2.9, 2.8 Hz, 1 H) 6.95 (d, *J*=7.7 Hz, 1 H) 6.99 (dt, *J*=8.1, 1.0 Hz, 1 H) 7.09 (dd, *J*=5.2, 1.6 Hz, 1 H) 7.22 - 7.31 (m, 2 H) 7.45 (s, 1 H) 7.85 (dd, *J*=9.3, 2.8 Hz, 1 H) 8.18 (d, *J*=5.2 Hz, 1 H) 8.69 (s, 1 H) 8.74 (s, 2 H)

Compound 107: $^1$H NMR (600 MHz, DMSO-$d_6$) δ ppm 0.89 - 0.94 (m, 2 H) 1.01 - 1.09 (m, 2 H) 1.59 - 1.71 (m, 2 H) 2.41 - 2.56 (m, 4 H) 2.67 - 2.79 (m, 4 H) 3.15 - 3.19 (m, 2 H) 3.34 - 3.39 (m, 2 H) 3.41 (br. s., 2 H) 6.89 (d, *J*=7.3 Hz, 1 H) 7.00 (d, *J*=7.6 Hz, 1 H) 7.10 (d, *J*=5.1 Hz, 1 H) 7.25 (t, *J*=7.7 Hz, 1 H) 7.34 (s, 1 H) 7.36 - 7.42 (m, 1 H) 7.48 (br. s., 1 H) 7.49 (s, 1 H) 8.18 (d, *J*=5.3 Hz, 1 H) 8.66 (br. s., 1 H) 8.71 (s, 2 H)

Compound 126: $^1$H NMR (400 MHz, DMSO-$d_6$) δ ppm 2.62 - 2.76 (m, 4 H) 2.89 - 3.02 (m, 4 H) 3.35 (s, 2 H) 3.40 - 3.48 (m, 2 H) 3.55 (s, 2 H) 3.61 - 3.69 (m, 2 H) 6.94 (d, *J*=7.7 Hz, 1 H) 6.98 - 7.08 (m, 3 H) 7.20 (t, *J*=6.1 Hz, 1 H) 7.29 (t, *J*=7.7 Hz, 1 H) 7.36 (s, 1 H) 8.16 (d, *J*=4.8 Hz, 1 H) 8.47 (br. s., 1 H) 8.51 (s, 2 H)

Compound 129: $^1$H NMR (360 MHz, CHLOROFORM-*d*) δ ppm 2.59 (s, 4 H) 2.54 (s, 4 H) 2.80 (t, *J*=5.7 Hz, 2 H) 2.86 (t, *J*=5.7 Hz, 2 H) 3.07 (s, 2 H) 3.28 (s, 3 H) 3.39 - 3.50 (m, 4 H) 3.54 (s, 2 H) 4.01 (s, 2 H) 6.94 (dd, *J*=5.1, 1.5 Hz, 1 H) 6.98 (dd, *J*=8.1, 1.1 Hz, 1 H) 7.09 - 7.21 (m, 3 H) 7.32 - 7.41 (m, 1 H) 7.43 (s, 1 H) 7.69 (t, *J*=4.8 Hz, 1 H) 8.31 (d, *J*=5.1 Hz, 1 H) 8.88 (s, 2 H)

Compound 140 : $^1$H NMR (500 MHz, DMSO-$d_6$) δ ppm 1.37 - 1.51 (m, 3 H) 1.56 - 1.65 (m, 2 H) 1.69 - 1.79 (m, 2 H) 2.00 (t, *J*=10.6 Hz, 2 H) 2.45 - 2.51 (m, 2 H-partially obscured by solvent peak) 2.87 (s, 2 H) 3.00 (d, *J*=10.6 Hz, 2 H) 3.11 - 3.20 (m, 2 H) 3.21 - 3.29 (m, 2 H) 6.47 (d, *J*=8.8 Hz, 1 H) 6.85 (d, *J*=8.8 Hz, 1 H) 6.89 (d, *J*=8.8 Hz, 1 H) 6.96 (dd, *J*=5.4, 0.9 Hz, 1 H) 7.00 (t, *J*=6.0 Hz, 1 H) 7.16 - 7.32 (m, 3 H) 7.64 (t, *J*=6.1 Hz, 1 H) 7.74 (dd, *J*=8.8, 2.5 Hz, 1 H) 8.13 (d, *J*=5.4 Hz, 1 H) 8.34 (d, *J*=2.2 Hz, 1 H) 8.74 (s, 1 H)

Compound 141 : $^1$H NMR (500 MHz, DMSO-$d_6$) δ ppm 1.23 - 1.32 (m, 2 H) 1.70 - 2.11 (m, 3 H) 2.22 - 2.81 (m, 9 H-partially obscured by solvent peak) 2.89-2.98 (m, 1 H) 3.10 (d, *J*=15.8 Hz, 1 H) 3.16 (d, *J*=12.0 Hz, 1 H) 3.19 - 3.26 (m, 1 H) 3.28 - 3.32 (m, 1 H-partially obscured by solvent peak) 3.37 - 3.61 (m, 2 H) 3.69 (d, *J*=12.0 Hz, 1 H) 3.83 - 4.06 (m, 1 H) 6.45 (d, *J*=9.1 Hz, 1 H) 6.91 - 7.05 (m, 3 H) 7.13 (s, 1 H) 7.30 (t, *J*=7.7 Hz, 1 H) 7.37 (t, *J*=2.4 Hz, 1 H) 7.79 (d, *J*=9.1 Hz, 2 H) 8.13 (d, *J*=5.4 Hz, 1 H) 8.33 (br. s., 1 H) 8.77 (s, 1 H)

Compound 142 :

$^1$H NMR (500 MHz, DMSO-$d_6$) δ ppm 1.23 - 1.32 (m, 2 H) 1.70 - 2.11 (m, 3 H) 2.22 - 2.81 (m, 9 H-partially obscured by solvent peak) 2.89-2.98 (m, 1 H) 3.10 (d, *J*=15.8 Hz, 1 H) 3.16 (d, *J*=12.0 Hz, 1 H) 3.19 - 3.26 (m, 1 H) 3.28 - 3.32 (m, 1 H-partially obscured by solvent peak) 3.37 - 3.61 (m, 2 H) 3.69 (d, *J*=12.0 Hz, 1 H) 3.83 - 4.06 (m, 1 H) 6.45 (d, *J*=9.1 Hz, 1 H) 6.91 - 7.05 (m, 3 H) 7.13 (s, 1 H) 7.30 (t, *J*=7.7 Hz, 1 H) 7.37 (t, *J*=2.4 Hz, 1 H) 7.79 (d, *J*=9.1 Hz, 2 H) 8.13 (d, *J*=5.4 Hz, 1 H) 8.33 (br. s., 1 H) 8.77 (s, 1 H)

Compound 143 : $^1$H NMR (500 MHz, DMSO-$d_6$) δ ppm 1.23 - 1.32 (m, 2 H) 1.70 - 2.11 (m, 3 H) 2.22 - 2.81 (m, 9 H-partially obscured by solvent peak) 2.89-2.98 (m, 1 H) 3.10 (d, *J*=15.8 Hz, 1 H) 3.16 (d, *J*=12.0 Hz, 1 H) 3.19 - 3.26 (m, 1 H) 3.28 - 3.32 (m, 1 H-partially obscured by solvent peak) 3.37 - 3.61 (m, 2 H) 3.69 (d, *J*=12.0 Hz, 1 H) 3.83 - 4.06 (m, 1 H) 6.45 (d, *J*=9.1 Hz, 1 H) 6.91 - 7.05 (m, 3 H) 7.13 (s, 1 H) 7.30 (t, *J*=7.7 Hz, 1 H) 7.37 (t, *J*=2.4 Hz, 1 H) 7.79 (d, *J*=9.1 Hz, 2 H) 8.13 (d, *J*=5.4 Hz, 1 H) 8.33 (br. s., 1 H) 8.77 (s, 1 H)

Compound 144 :$^1$H NMR (500 MHz, DMSO-$d_6$) δ ppm 2.30 - 2.43 (m, 4 H) 2.53 - 2.59 (m, 2 H) 3.36 - 3.67 (m, 11 H) 6.51 (d, *J*=8.8 Hz, 1 H) 6.84 (d, *J*=8.8 Hz, 1 H) 6.89 - 7.04 (m, 3 H) 7.14 - 7.31 (m, 2 H) 7.49 (s, 1 H) 7.71 (dd,

*J*=8.8, 2.5 Hz, 1 H) 8.15 (d, *J*=5.4 Hz, 1 H) 8.35 (d, *J*=2.5 Hz, 1 H) 8.87 (s, 1 H)

Pharmacology

Biochemical EF2K lysate-based kinase assay

LN-229 cells were purchased from ATCC (CRL-2611); these are glioblastoma cells. Cell lysates from LN229 were used in this kinase assay to provide both the kinase and the substrate (EF2). The AlphaLISA p-eEF2 (Thr56) detection assay was developed using a sandwich assay format with two specific antibodies recognizing different epitopes of the target, including one antibody against the phosphorylation site of interest. One anti-eEF2 antibody was conjugated onto AlphaLISA Acceptor beads, while the second antibody was biotinylated and captured by streptavidin coated Donor beads.

Compound was mixed with LN-229 cell lysates in the presence of a kinase buffer (e.g. HEPES) at a pH of 6.6, containing 10 mM $Mg^{2+}$ (e.g. magnesium acetate) and 10 mM adenosine-tri-phosphate (ATP) and incubated at room temperature for 15 minutes. The kinase reaction was stopped with excess ethylenediaminetetraacetic acid disodium salt and the biotinylated -anti phospho eEF2 antibody (3nM) was added for 1 hour. Then the anti-EF2 acceptor beads (10 μg/ml) as well as the streptavidin coated donor beads (20 μg/ml) were added for 1 hour, and the AlphaLISA signal was measured in an Envision instrument once, left overnight, and measured again for the final read.

EF2K cell-based assay

In this assay, 2.5 mM 2-deoxyglucose was used to deplete intracellular ATP and activate 5' adenosine monophosphate- activated protein kinase (AMPK) in the immortalized epithelial breast cell lines, MCF10A. MCF 10A cells were purchased from ATCC (CRL-10317). This resulted in a rapid activation of eEF2K and an increase in phosphorylation of EF2 at Thr 56, which was determined using a phospho-specific ELISA (AlphaLISA) as described above in the lysate-based EF2k kinase assay.

MCF10A cells are seeded at a density of 1.25 x 10 5 Cells/ ml at 100 μl /well in a 96-well plate and incubated for 24 hours (37 °C, 5 % $CO_2$). Compound is added for 1 hour, and cell are stimulated with 2.5 mM of 2-deoxy-glucose for 4 hours. Medium is then removed, and cells are lysed in an ice-cold buffer M-PER (Thermo Scientific, 78501), containing protease and phosphatase inhibitors. P-EF2 levels are determined in these lysates using the P-EF2 AlphaLISA described above.

Biochemical Vps34 lipid kinase assay

A non-radiometric kinase assay (ADP-Glo™ Assay, Promega, Madison, Wi, USA) was used for measuring the kinase activity of the PIK3C3 lipid kinase. All kinase assays were performed in 96-well half-area microtiter plates in a 25 μl reaction volume. The reaction cocktail was pipetted in 3 steps in the following order:

10 μl of ATP solution (in assay buffer, see below)
5 μl of test sample in 5% DMSO 10 μl of enzyme/substrate mixture

All lipid kinase assays contained 50 mM HEPES (4-(2-hydroxyethyl)-1-piperazineethanesulfonic acid) - NaOH, pH 7.5, 1 mM EGTA ((ethylene glycol tetraacetic acid), 100 mM NaCl, 0.03 % CHAPS (3-[(3-Cholamidopropyl) dimethylammonio]-1- propanesulfonate), 2 mM DTT (Dithiothreitol), 20 μM ATP (corresponding to the apparent ATP-Km), kinase (7.6 nM) and substrate (50 μM). The assay for PIK3C3 additionally contained 3 mM $MnCl_2$.

The reaction cocktails were incubated at 30° C for 60 minutes. The reaction was stopped with 25 μl ADP-Glo™ reagent per well. Plates were incubated for 40 minutes at room temperature, followed by addition of 50 μl kinase detection reagent per well and incubation for 60 minutes at room temperature. Signal was determined with a microplate luminescence reader (Victor, Perkin Elmer). The assay was either performed using a single dose of compound (1 μM final concentration in the assay reaction) with resulting data expressed as residual activity compared to control (DMSO), or using a serial (half-log) dilution of compounds starting at 10 μM and down to 0.3 nM (final concentrations in the assay) with data expressed as the pIC50.

The results of the above described assays are shown in table 4: ($pIC_{50}$ is $-logIC_{50}$ where $IC_{50}$ represents the concentration expressed in M at which the test compound gives 50% inhibition)

| Comp No. | eEF2K_C_alphalisa pIC50 | eEF2K_C_PThr56 pIC50 | VPS34_1microM_ %ofcntrl | VPS34 pIC50 |
|---|---|---|---|---|
| Compound 67 | ~6.45 | 5.40 | 34.00 | |

(continued)

| Comp No. | eEF2K_C_alphalisa pIC50 | eEF2K_C_PThr56 pIC50 | VPS34_1microM_ %ofcntrl | VPS34 pIC50 |
|---|---|---|---|---|
| Compound 70 | 5.25 | <4.52 | 72.57 | |
| Compound 68 | 6.29 | <4.52 | | 5.33 |
| Compound 72 | 6.54 | 5.23 | 50.80 | |
| Compound 69 | 4.85 | <4.52 | | |
| Compound 33 | ~7.66 | ~6.1 | | 6.66 |
| Compound 140 | 5.42 | 5.07 | 46.41 | |
| Compound 141 | | ~5.44 | | |
| Compound 142 | 7.45 | 5.80 | 40.04 | |
| Compound 143 | 4.86 | <4.52 | | 5.67 |
| Compound 39 | 6.56 | 4.91 | 45.71 | |
| Compound 37 | ~4.61 | <4.52 | 84.50 | |
| Compound 144 | ~4.82 | <4.52 | 53.74 | |
| Compound 71 | 6.48 | 4.61 | 32.15 | |
| Compound 40 | 6.79 | ~5.23 | 10.99 | 6.58 |
| Compound 38 | 7.07 | <4.52 | 43.29 | |
| Compound 36 | 7.79 | 6.01 | | 6.14 |
| Compound 43 | 8.34 | 6.53 | 40.75 | |
| Compound 45 | 6.52 | 4.73 | | 6.55 |
| Compound 13 | 6.19 | 5.04 | 34.94 | |
| Compound 9 | 7.46 | ~5.26 | 7.10 | 6.78 |
| Compound 116 | 5.57 | 4.72 | 33.55 | |
| Compound 126 | 6.55 | <4.52 | 52.70 | |
| Compound 18 | 7.06 | 5.98 | 8.79 | 6.76 |
| Compound 19 | 7.47 | 6.15 | | 6.54 |
| Compound 14 | <4.52 | <4.52 | 50.38 | |
| Compound 17 | <4.52 | <4.52 | 62.44 | |
| Compound 10 | 7.66 | 5.83 | 18.61 | 6.34 |
| Compound 107 | 8.17 | 6.45 | | 6.48 |
| Compound 15 | <4.52 | <4.52 | 67.04 | |
| Compound 16 | <4.52 | <4.52 | 46.10 | |
| Compound 120 | 6.20 | <4.52 | 23.74 | 6.53 |
| Compound 106 | 6.12 | 4.54 | 22.36 | 6.23 |
| Compound 2 | 7.32 | ~5.6 | | 6.26 |
| Compound 3 | 6.81 | 5.24 | 15.27 | 6.64 |
| Compound 5 | 6.95 | 5.60 | 22.45 | 6.35 |
| Compound 117 | 6.34 | <4.52 | 16.31 | 6.85 |
| Compound 118 | 6.51 | 4.81 | 70.61 | |
| Compound 6 | 7.35 | 5.95 | | 6.12 |

(continued)

| Comp No. | eEF2K_C_alphalisa pIC50 | eEF2K_C_PThr56 pIC50 | VPS34_1microM_ %ofcntrl | VPS34 pIC50 |
|---|---|---|---|---|
| Compound 7 | 6.95 | 4.79 | 12.51 | 6.97 |
| Compound 119 | 6.56 | <4.52 | 58.90 | |
| Compound 42 | 7.01 | ~5.64 | | 6.15 |
| Compound 44 | 7.61 | 5.06 | | 5.81 |
| Compound 76 | 7.80 | ~5.94 | | 6.40 |
| Compound 8 | 7.38 | 5.59 | 9.64 | 6.78 |
| Compound 46 | 7.88 | 6.19 | | 5.63 |
| Compound 41 | 6.98 | 5.77 | 23.31 | 6.13 |
| Compound 124 | 6.40 | ~4.6 | 65.50 | |
| Compound 122 | 4.72 | 4.85 | 39.48 | |
| Compound 27 | 7.97 | ~5.91 | | 7.59 |
| Compound 28 | 4.65 | <5 | | <5 |
| Compound 48 | 7.10 | 5.50 | 44.15 | |
| Compound 123 | 6.35 | 5.27 | 73.39 | |
| Compound 121 | 5.29 | <5 | | 5.58 |
| Compound 47 | 8.07 | 6.38 | | 5.04 |
| Compound 101 | 6.70 | 5.40 | 31.08 | |
| Compound 100 | 7.21 | 5.56 | 14.88 | 6.45 |
| Compound 1 | 7.98 | 6.44 | | 7.31 |
| Compound 73 | 5.28 | <5 | | <5 |
| Compound 59 | 7.46 | 6.07 | | 6.38 |
| Compound 60 | 7.18 | 5.32 | 86.82 | |
| Compound 54 | 7.96 | 6.10 | | 5.01 |
| Compound 55 | 8.07 | 6.11 | 29.55 | 6.26 |
| Compound 25 | 5.63 | <5 | | 6.22 |
| Compound 26 | 5.97 | <5 | 4.67 | 7.18 |
| Compound 61 | 7.90 | 6.37 | 80.70 | |
| Compound 62 | 8.26 | 7.07 | | 5.91 |
| Compound 57 | 7.99 | 6.51 | | 5.30 |
| Compound 56 | 8.47 | 6.52 | | 6.58 |
| Compound 75 | 6.20 | 5.01 | 45.04 | |
| Compound 102 | 7.09 | 5.88 | | |
| Compound 74 | 6.92 | 5.82 | 70.06 | |
| Compound 63 | 7.71 | 5.76 | | |
| Compound 64 | 8.29 | 6.41 | | |
| Compound 145 | ~5.26 | <5 | | |
| Compound 90 | 7.72 | 6.28 | | 7.21 |
| Compound 146 | 7.41 | 6.18 | 54.65 | |

(continued)

| Comp No. | eEF2K_C_alphalisa pIC50 | eEF2K_C_PThr56 pIC50 | VPS34_1microM_ %ofcntrl | VPS34 pIC50 |
|---|---|---|---|---|
| Compound 114 | | <5 | 29.15 | 6.29 |
| Compound 20 | 8.40 | 6.78 | | |
| Compound 149 | 5.20 | <5 | | |
| Compound 148 | | <5 | 9.53 | 7.03 |
| Compound 115 | 6.82 | 6.36 | | |
| Compound 34 | 6.48 | ~5.58 | 23.53 | 6.44 |
| Compound 22 | ~7.98 | 6.44 | | |
| Compound 23 | 7.02 | ~5.53 | | 5.76 |
| Compound 128 | 6.54 | 6.38 | 74.48 | |
| Compound 109 | 6.45 | <5 | | |
| Compound 80 | 8.00 | 6.20 | | |
| Compound 81 | 8.31 | 6.51 | | |
| Compound 66 | 7.21 | 6.04 | | 5.95 |
| Compound 65 | 8.25 | 6.42 | | 6.71 |
| Compound 53 | 8.39 | 7.07 | | 6.75 |
| Compound 82 | 7.73 | 6.15 | | |
| Compound 83 | 8.06 | 6.18 | 42.32 | |
| Compound 78 | 7.43 | 5.65 | 43.17 | |
| Compound 77 | 7.37 | 5.42 | 86.17 | |
| Compound 49 | 4.60 | <5 | | |
| Compound 104 | 7.93 | 5.85 | | 5.81 |
| Compound 139 | 5.12 | <5 | | |
| Compound 138 | ~5.8 | <5 | | |
| Compound 113 | | 5.26 | | |
| Compound 95 | 8.17 | ~6.19 | | |
| Compound 97 | 8.74 | 6.48 | | |
| Compound 12 | 6.84 | <5 | | |
| Compound 11 | 8.56 | 6.54 | | |
| Compound 35 | 7.19 | 6.61 | 20.85 | 6.53 |
| Compound 85 | 7.56 | 5.96 | | |
| Compound 84 | 7.14 | 5.99 | 21.55 | 6.49 |
| Compound 93 | 7.10 | 6.20 | | |
| Compound 52 | 8.27 | 7.12 | | |
| Compound 79 | 7.01 | 5.94 | | |
| Compound 99 | 7.51 | 5.97 | | |
| Compound 86 | 6.47 | <5 | 68.01 | |
| Compound 87 | 7.71 | ~5.64 | 33.75 | |
| Compound 88 | 7.19 | 6.34 | 66.48 | |

(continued)

| Comp No. | eEF2K_C_alphalisa pIC50 | eEF2K_C_PThr56 pIC50 | VPS34_1microM_ %ofcntrl | VPS34 pIC50 |
|---|---|---|---|---|
| Compound 91 | 7.87 | 6.14 | | |
| Compound 133 | <4.52 | ~5 | | |
| Compound 134 | 4.95 | <5 | | |
| Compound 130 | 6.00 | 5.80 | 57.01 | |
| Compound 131 | 7.21 | 5.60 | 56.21 | |
| Compound 89 | ~8.35 | 6.49 | | |
| Compound 92 | 6.90 | 5.93 | | |
| Compound 96 | 8.40 | 7.09 | | |
| Compound 129 | 4.57 | <5 | | |
| Compound 94 | 7.18 | 6.17 | | |
| Compound 147 | 6.40 | 5.98 | | |
| Compound 103 | 6.72 | 5.79 | | |
| Compound 125 | 5.24 | <5 | | |
| Compound 50 | 7.99 | 6.45 | | |
| Compound 51 | | ~5 | | |
| Compound 105 | | 6.10 | | |

Composition examples

"Active ingredient" (a.i.) as used throughout these examples relates to a compound of Formula (I), including any tautomer or stereoisomeric form thereof, or a pharmaceutically acceptable addition salt or a solvate thereof; in particular to any one of the exemplified compounds.

Typical examples of recipes for the formulation of the invention are as follows:

*1. Tablets*

| | |
|---|---|
| Active ingredient | 5 to 50 mg |
| Di-calcium phosphate | 20 mg |
| Lactose | 30 mg |
| Talcum | 10 mg |
| Magnesium stearate | 5 mg |
| Potato starch | ad 200 mg |

*2. Suspension*

An aqueous suspension is prepared for oral administration so that each milliliter contains 1 to 5 mg of active ingredient, 50 mg of sodium carboxymethyl cellulose, 1 mg of sodium benzoate, 500 mg of sorbitol and water ad 1 ml.

*3. Injectable*

A parenteral composition is prepared by stirring 1.5 % (weight/volume) of active ingredient in 0.9 % NaCl solution or in 10 % by volume propylene glycol in water.

*4. Ointment*

| | |
|---|---|
| Active ingredient | 5 to 1000 mg |
| Stearyl alcohol | 3 g |
| Lanoline | 5 g |
| White petroleum | 15 g |
| Water | ad 100 g |

In this Example, active ingredient can be replaced with the same amount of any of the compounds according to the present invention, in particular by the same amount of any of the exemplified compounds.

## Claims

**1.** A compound of Formula (I)

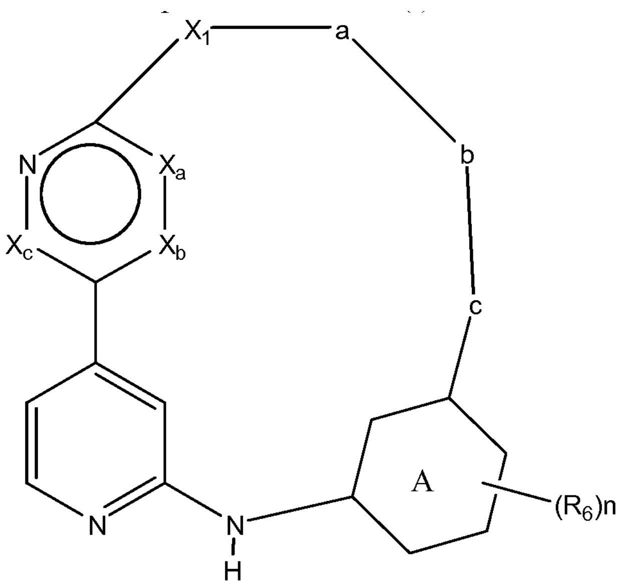

(I)

a tautomer or a stereoisomeric form thereof, wherein

$X_a$, $X_b$ and $X_c$ each independently represent CH or N;
$-X_1-$ represents $-(CHR_{12})_s-NR_1-X_e-C_{1-4}$alkanediyl-$(SO_2)_{p3}-$ or $-(CH_2)_s-O-X_e-C_{1-4}$alkanediyl-$(SO_2)_{p3}-$; wherein each of said $C_{1-4}$alkanediyl moieties are optionally substituted with hydroxyl or hydroxy$C_{1-4}$alkyl;
$-X_e-$ represents $-C(R_2)_2-$ or $-C(=O)-$;
a represents $-NR_4-C(=O)-[C(R_{5b})_2]_r-$ or $-NR_4-C(R_{5b})_2-C(=O)-$ or $-C(=O)-NR_4-C(R_{5b})_2-$;
b represents

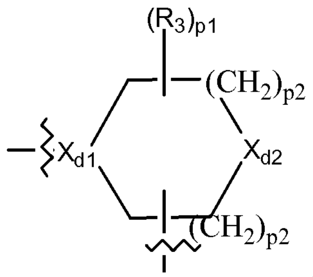

,

wherein said b ring may contain extra bonds to form a bridged ring system selected from 2,5-diazabicyclo[2.2.2]octanyl, 3,8-diazabicyclo[3.2.1]octanyl, 3,6-diazabicyclo[3.1.1]heptanyl, 3,9-diazabicyclo[3.3.1]nonyl;
$X_{d1}$ represents CH or N;
$X_{d2}$ represents $CH_2$ or NH;
provided that at least one of $X_{d1}$ and $X_{d2}$ represents nitrogen;
c represents a bond, $-[C(R_{5a})_2]_m-$, -C(=O)-, -O-, $-NR_{5a'}-$, $-SO_2-$, or -SO-;
ring

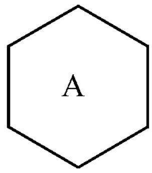

represents phenyl or pyridyl;
$R_1$ represents hydrogen, $C_{1-4}$alkyl, $C_{2-4}$alkenyl, $C_{2-4}$alkynyl, cyano$C_{1-4}$alkyl, -C(=O)-$C_{1-4}$alkyl, -C(=O)-halo$C_{1-4}$alkyl, hydroxy$C_{1-4}$alkyl, halo$C_{1-4}$alkyl, $C_{1-4}$alkyloxy$C_{1-4}$alkyl, halo$C_{1-4}$alkyloxy$C_{1-4}$alkyl, -C(=O)$NR_7R_8$, $-SO_2-NR_7R_8$, $-SO_2-R_9$, $R_{11}$, $C_{1-4}$alkyl substituted with $R_{11}$, -C(=O)-$R_{11}$, or -C(=O)-$C_{1-4}$alkyl-$R_{11}$;
each $R_2$ independently represents hydrogen, $C_{1-4}$alkyl, $C_{1-4}$alkyl substituted with $C_{3-6}$cycloalkyl, hydroxy$C_{1-4}$alkyl, $C_{1-4}$alkyloxy$C_{1-4}$alkyl, carboxyl, -C(=O)-O-$C_{1-4}$alkyl wherein $C_{1-4}$alkyl is optionally substituted with $C_{1-4}$alkyloxy, -C(=O)-$NH_2$, -C(=O)-NH($C_{1-4}$alkyl) wherein $C_{1-4}$alkyl is optionally substituted with $C_{1-4}$alkyloxy, or -C(=O)-N($C_{1-4}$alkyl$)_2$ wherein each $C_{1-4}$alkyl is optionally substituted with $C_{1-4}$alkyloxy;
or $R_1$ and one $R_2$ are taken together to form $C_{1-4}$alkanediyl or $C_{2-4}$alkenediyl, each of said $C_{1-4}$alkanediyl and $C_{2-4}$alkenediyl optionally being substituted with 1 to 4 substituents each independently selected from hydroxyl, oxo, halo, cyano, $N_3$, hydroxy$C_{1-4}$alkyl, $-NR_7R_8$, $-SO_2-NR_7R_8$, $-NH-SO_2-NR_7R_8$, $-C(=O)-NR_7R_8$, or $-NH-C(=O)-NR_7R_8$;
or $R_1$ and $R_{12}$ are taken together to form $C_{1-4}$alkanediyl or $C_{2-4}$alkenediyl, each of said $C_{1-4}$alkanediyl and $C_{2-4}$alkenediyl optionally being substituted with 1 to 4 substituents each independently selected from hydroxyl, oxo, halo, cyano, $N_3$, hydroxy$C_{1-4}$alkyl, $-NR_7R_8$, $-SO_2-NR_7R_8$, $-NH-SO_2-NR_7R_8$, $-C(=O)-NR_7R_8$, or $-NH-C(=O)-NR_7R_8$;
each $R_3$ independently represents hydrogen; oxo; hydroxyl; carboxyl; $-NR_{3a}R_{3b}$; $-C(=O)-NR_{3a}R_{3b}$; hydroxy$C_{1-4}$alkyl; halo$C_{1-4}$alkyl; -(C=O)-$C_{1-4}$alkyl;
-C(=O)-O-$C_{1-4}$alkyl wherein said $C_{1-4}$alkyl may optionally be substituted with phenyl; $C_{1-4}$alkyl optionally substituted with cyano, carboxyl, $C_{1-4}$alkyloxy, -C(=O)-O-$C_{1-4}$alkyl, -O-C(=O)-$C_{1-4}$alkyl, $-NR_{3e}R_{3f}$, $-C(=O)-NR_{3e}R_{3f}$, $-SO_2-NR_{3e}R_{3f}$, Q, -C(=O)-Q, or $-SO_2$-Q; hydroxy$C_{1-4}$alkyloxy$C_{1-4}$alkyl; $C_{1-4}$alkyloxyhydroxy$C_{1-4}$alkyl; hydroxy$C_{1-4}$alkyloxyhydroxy$C_{1-4}$alkyl; or $C_{1-4}$alkyloxy$C_{1-4}$alkyl optionally substituted with cyano, carboxyl, $C_{1-4}$alkyloxy, -C(=O)-O-$C_{1-4}$alkyl, -O-C(=O)-$C_{1-4}$alkyl, $-NR_{3e}R_{3f}$, $-C(=O)-NR_{3e}R_{3f}$, $-SO_2-NR_{3e}R_{3f}$, $R_{10}$, -C(=O)-$R_{10}$, or $-SO_2-R_{10}$; or
two $R_3$ substituents attached to the same carbon atom are taken together to form $C_{2-5}$alkanediyl or $-(CH_2)_p-O-(CH_2)_p-$;
each $R_{3a}$ and $R_{3b}$ independently represent hydrogen; -(C=O)-$C_{1-4}$alkyl; $-SO_2-NR_{3c}R_{3d}$; or $C_{1-4}$alkyl optionally substituted with $C_{1-4}$alkyloxy; or
$R_{3a}$ and $R_{3b}$ are taken together with the nitrogen to which they are attached to form a 4 to 7 membered saturated monocyclic heterocyclic ring which optionally contains 1 or 2 further heteroatoms selected from N, O or $SO_2$, said heterocyclic ring being optionally substituted with 1 to 4 substituents each independently selected from $C_{1-4}$alkyl, halo, hydroxyl, or halo$C_{1-4}$alkyl;

each $R_{3c}$ and $R_{3d}$ independently represent hydrogen, $C_{1-4}$alkyl or -(C=O)-$C_{1-4}$alkyl; or $R_{3c}$ and $R_{3d}$ are taken together with the nitrogen to which they are attached to form a 4 to 7 membered saturated monocyclic heterocyclic ring which optionally contains 1 or 2 further heteroatoms selected from N, O or $SO_2$, said heterocyclic ring being optionally substituted with 1 to 4 substituents each independently selected from $C_{1-4}$alkyl, halo, hydroxyl, or halo$C_{1-4}$alkyl;

each $R_{3e}$ and $R_{3f}$ independently represent hydrogen, $C_{1-4}$alkyl optionally substituted with $C_{1-4}$alkyloxy, -(C=O)-$C_{1-4}$alkyl, or -$SO_2$-$NR_{3c}R_{3d}$;

$R_4$ represents hydrogen, $C_{1-4}$alkyl or $C_{1-4}$alkyloxy$C_{1-4}$alkyl;

each $R_{5a}$ independently represents hydrogen or $C_{1-4}$alkyl; or

two $R_{5a}$ substituents attached to the same carbon atom are taken together to form $C_{2-5}$alkanediyl or $-(CH_2)_p-O-(CH_2)_p-$;

$R_{5a'}$ represents hydrogen or $C_{1-4}$alkyl;

each $R_{5b}$ independently represents hydrogen; $C_{1-4}$alkyl; $C_{1-4}$alkyl substituted with $NR_{5b1}R_{5b2}$; $C_{1-4}$alkyloxy$C_{1-4}$alkyl; hydroxy$C_{1-4}$alkyl; hydroxyl; $C_{3-6}$cycloalkyl; or phenyl optionally substituted with $C_{1-4}$alkyl, halo, hydroxyl or $C_{1-4}$alkyloxy; or

two $R_{5b}$ substituents attached to the same carbon atom are taken together to form $C_{2-5}$alkanediyl or $-(CH_2)_p-O-(CH_2)_p-$;

$R_{5b1}$ and $R_{5b2}$ independently represent hydrogen, $C_{1-4}$alkyl optionally substituted with $C_{1-4}$alkyloxy, -(C=O)-$C_{1-4}$alkyl, or -$SO_2$-$NR_{5b3}R_{5b4}$;

$R_{5b3}$ and $R_{5b4}$ independently represent hydrogen, $C_{1-4}$alkyl or -(C=O)-$C_{1-4}$alkyl; or

$R_{5b3}$ and $R_{5b4}$ are taken together with the nitrogen to which they are attached to form a 4 to 7 membered saturated monocyclic heterocyclic ring which optionally contains 1 or 2 further heteroatoms selected from N, O or $SO_2$, said heterocyclic ring being optionally substituted with 1 to 4 substituents each independently selected from $C_{1-4}$alkyl, halo, hydroxyl, or halo$C_{1-4}$alkyl;

each $R_6$ independently represents hydrogen, halo, hydroxyl, carboxyl, cyano, $C_{1-4}$alkyl, $C_{1-4}$alkyloxy$C_{1-4}$alkyl, hydroxy$C_{1-4}$alkyl, halo$C_{1-4}$alkyl, $C_{2-4}$alkenyl, $C_{2-4}$alkynyl, $-NR_{6a}P_{6b}$, or $-C(=O)NR_{6a}R_{6b}$;

each $R_{6a}$ and $R_{6b}$ independently represent hydrogen or $C_{1-4}$alkyl;

each $R_7$ and $R_8$ independently represent hydrogen, $C_{1-4}$alkyl, halo$C_{1-4}$alkyl, or $C_{3-6}$cycloalkyl; or

$R_7$ and $R_8$ are taken together with the nitrogen to which they are attached to form a 4 to 7 membered saturated monocyclic heterocyclic ring which optionally contains 1 further heteroatom selected from N, O or $SO_2$, said heterocyclic ring being optionally substituted with 1 to 4 substituents each independently selected from $C_{1-4}$alkyl, halo, hydroxyl, or halo$C_{1-4}$alkyl;

$R_9$ represents $C_{1-4}$alkyl, halo$C_{1-4}$alkyl, or $C_{3-6}$cycloalkyl;

each $R_{10}$ independently represents a 4 to 7 membered saturated monocyclic heterocyclic ring containing up to 2 heteroatoms selected from N, O or $SO_2$, said heterocyclic ring being optionally substituted with 1 to 4 substituents each independently selected from $C_{1-4}$alkyl, halo, hydroxyl or halo$C_{1-4}$alkyl;

each $R_{11}$ independently represents $C_{3-6}$cycloalkyl, phenyl, or a 4 to 7 membered monocyclic heterocyclic ring containing up to 3 heteroatoms selected from N, O or $SO_2$, said heterocyclic ring being optionally substituted with 1 to 4 substituents each independently selected from $C_{1-4}$alkyl, halo, hydroxyl, or halo$C_{1-4}$alkyl;

each $R_{12}$ independently represents hydrogen or $C_{1-4}$alkyl;

Q represents a 4 to 7 membered saturated monocyclic heterocyclic ring containing up to 3 heteroatoms selected from N, O or $SO_2$, said heterocyclic ring being optionally substituted with 1 to 4 substituents each independently selected from $C_{1-4}$alkyl, halo, hydroxyl or halo$C_{1-4}$alkyl;

n represents an integer of value 1 or 2;

m represents an integer of value 1 or 2 ;

p represents an integer of value 1 or 2;

p1 represents an integer of value 1 or 2;

each p2 independently represents an integer of value 0, 1 or 2;

r represents an integer of value 0, 1 or 2;

each $p_3$ independently represents an integer of value 0 or 1;

each s independently represents an integer of value 0, 1 or 2;

or a N-oxide, a pharmaceutically acceptable addition salt or a solvate thereof.

**2.** The compound according to claim 1, wherein

$X_a$, $X_b$ and $X_c$ each independently represent CH or N;

$-X_1-$ represents $-(CHR_{12})_s-NR_1-X_e-C_{1-4}$alkanediyl-$(SO_2)_{p3}-$;

$-X_e-$ represents $-C(R_2)_2-$;

a represents $-NR_4-C(=O)-[C(R_{5b})_2]_r-$ or $-NR_4-C(R_{5b})_2-C(=O)-$;
b represents

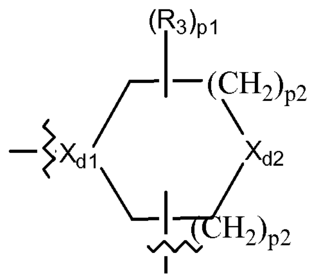

,

wherein said b ring may contain extra bonds to form a bridged ring system selected from 2,5-diazabicyclo[2.2.2]octanyl, 3,8-diazabicyclo[3.2.1]octanyl, 3,6-diazabicyclo[3.1.1]heptanyl, 3,9-diazabicyclo[3.3.1]nonyl;

$X_{d1}$ represents CH or N;
$X_{d2}$ represents NH;
provided that at least one of $X_{d1}$ and $X_{d2}$ represents nitrogen;
c represents a bond, $-[C(R_{5a})_2]_m-$, -O-, $-NR_{5a'}-$;
ring

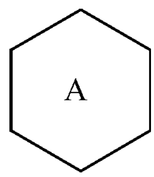

represents phenyl or pyridyl;
$R_1$ represents hydrogen, $C_{1-4}$alkyl, $C_{2-4}$alkenyl, $C_{2-4}$alkynyl, cyano$C_{1-4}$alkyl, $-C(=O)-C_{1-4}$alkyl, $-C(=O)-$halo$C_{1-4}$alkyl, halo$C_{1-4}$alkyl, $-C(=O)NR_7R_8$, $-SO_2-NR_7R_8$, $-SO_2-R_9$, $R_{11}$, $C_{1-4}$alkyl substituted with $R_{11}$, $-C(=O)-R_{11}$, or $-C(=O)-C_{1-4}$alkyl$-R_{11}$;
each $R_2$ independently represents hydrogen, $C_{1-4}$alkyl, $C_{1-4}$alkyl substituted with $C_{3-6}$cycloalkyl, hydroxy$C_{1-4}$alkyl, $C_{1-4}$alkyloxy$C_{1-4}$alkyl, carboxyl, $-C(=O)-O-C_{1-4}$alkyl wherein $C_{1-4}$alkyl is optionally substituted with $C_{1-4}$alkyloxy, $-C(=O)-NH_2$, $-C(=O)-NH(C_{1-4}$alkyl) wherein $C_{1-4}$alkyl is optionally substituted with $C_{1-4}$alkyloxy, or $-C(=O)-N(C_{1-4}$alkyl$)_2$ wherein each $C_{1-4}$alkyl is optionally substituted with $C_{1-4}$alkyloxy;
or $R_1$ and one $R_2$ are taken together to form $C_{3-4}$alkanediyl or $C_{3-4}$alkenediyl, each of said $C_{3-4}$alkanediyl and $C_{3-4}$alkenediyl optionally being substituted with 1 to 4 substituents each independently selected from hydroxyl, oxo, halo, cyano, $N_3$, hydroxy$C_{1-4}$alkyl, $-NR_7R_8$, $-SO_2-NR_7R_8$, $-NH-SO_2-NR_7R_8$, $-C(=O)-NR_7R_8$, or $-NH-C(=O)-NR_7R_8$;
each $R_3$ independently represents hydrogen; oxo; hydroxyl; carboxyl; $-NR_{3a}R_{3b}$; $-C(=O)-NR_{3a}R_{3b}$; hydroxy$C_{1-4}$alkyl; halo$C_{1-4}$alkyl; $-(C=O)-C_{1-4}$alkyl; $-C(=O)-O-C_{1-4}$alkyl wherein said $C_{1-4}$alkyl may optionally be substituted with phenyl; $C_{1-4}$alkyl optionally substituted with cyano, carboxyl, $C_{1-4}$alkyloxy, $-C(=O)-O-C_{1-4}$alkyl, $-O-C(=O)-C_{1-4}$alkyl, $-NR_{3e}R_{3f}$, $-C(=O)-NR_{3e}R_{3f}$, $-SO_2-NR_{3e}R_{3f}$, Q, $-C(=O)-Q$, or $-SO_2-Q$; hydroxy$C_{1-4}$alkyloxy$C_{1-4}$alkyl; $C_{1-4}$alkyloxyhydroxy$C_{1-4}$alkyl; hydroxy$C_{1-4}$alkyloxyhydroxy$C_{1-4}$alkyl; or $C_{1-4}$alkyloxy$C_{1-4}$alkyl optionally substituted with cyano, carboxyl, $C_{1-4}$alkyloxy, $-C(=O)-O-C_{1-4}$alkyl, $-O-C(=O)-C_{1-4}$alkyl, $-NR_{3e}R_{3f}$, $-C(=O)-NR_{3e}R_{3f}$, $-SO_2-NR_{3e}R_{3f}$, $R_{10}$, $-C(=O)-R_{10}$, or $-SO_2-R_{10}$; or
two $R_3$ substituents attached to the same carbon atom are taken together to form $C_{2-5}$alkanediyl or $-(CH_2)_p-O-(CH_2)_p-$;
each $R_{3a}$ and $R_{3b}$ independently represent hydrogen; $-(C=O)-C_{1-4}$alkyl; $-SO_2-NR_{3c}R_{3d}$; or $C_{1-4}$alkyl optionally substituted with $C_{1-4}$alkyloxy; or
$R_{3a}$ and $R_{3b}$ are taken together with the nitrogen to which they are attached to form a 4 to 7 membered saturated monocyclic heterocyclic ring which optionally contains 1 or 2 further heteroatoms selected from N, O or $SO_2$, said heterocyclic ring being optionally substituted with 1 to 4 substituents each independently selected from $C_{1-4}$alkyl, halo, hydroxyl, or halo$C_{1-4}$alkyl;
each $R_{3c}$ and $R_{3d}$ independently represent hydrogen, $C_{1-4}$alkyl or $-(C=O)-C_{1-4}$alkyl; or $R_{3c}$ and $R_{3d}$ are taken

together with the nitrogen to which they are attached to form a 4 to 7 membered saturated monocyclic heterocyclic ring which optionally contains 1 or 2 further heteroatoms selected from N, O or $SO_2$, said heterocyclic ring being optionally substituted with 1 to 4 substituents each independently selected from $C_{1-4}$alkyl, halo, hydroxyl, or halo$C_{1-4}$alkyl;

each $R_{3e}$ and $R_{3f}$ independently represent hydrogen, $C_{1-4}$alkyl optionally substituted with $C_{1-4}$alkyloxy, -(C=O)-$C_{1-4}$alkyl, or -$SO_2$-$NR_{3c}R_{3d}$;

$R_4$ represents hydrogen, $C_{1-4}$alkyl or $C_{1-4}$alkyloxy$C_{1-4}$alkyl;

each $R_{5a}$ independently represents hydrogen or $C_{1-4}$alkyl; or

two $R_{5a}$ substituents attached to the same carbon atom are taken together to form $C_{2-5}$alkanediyl or $-(CH_2)_p$-O-$(CH_2)_p$-;

$R_{5a'}$ represents hydrogen or $C_{1-4}$alkyl;

each $R_{5b}$ independently represents hydrogen; $C_{1-4}$alkyl; $C_{1-4}$alkyl substituted with $NR_{5b1}R_{5b2}$; $C_{1-4}$alkyloxy$C_{1-4}$alkyl; hydroxy$C_{1-4}$alkyl; hydroxyl; $C_{3-6}$cycloalkyl; or phenyl optionally substituted with $C_{1-4}$alkyl, halo, hydroxyl or $C_{1-4}$alkyloxy; or

two $R_{5b}$ substituents attached to the same carbon atom are taken together to form $C_{2-5}$alkanediyl or $-(CH_2)_p$-O-$(CH_2)_p$-;

$R_{5b1}$ and $R_{5b2}$ independently represent hydrogen, $C_{1-4}$alkyl optionally substituted with $C_{1-4}$alkyloxy, -(C=O)-$C_{1-4}$alkyl, or -$SO_2$-$NR_{5b3}R_{5b4}$;

$R_{5b3}$ and $R_{5b4}$ independently represent hydrogen, $C_{1-4}$alkyl or -(C=O)-$C_{1-4}$alkyl; or

$R_{5b3}$ and $R_{5b4}$ are taken together with the nitrogen to which they are attached to form a 4 to 7 membered saturated monocyclic heterocyclic ring which optionally contains 1 or 2 further heteroatoms selected from N, O or $SO_2$, said heterocyclic ring being optionally substituted with 1 to 4 substituents each independently selected from $C_{1-4}$alkyl, halo, hydroxyl, or halo$C_{1-4}$alkyl;

each $R_6$ independently represents hydrogen, halo, hydroxyl, carboxyl, cyano, $C_{1-4}$alkyl, $C_{1-4}$alkyloxy$C_{1-4}$alkyl, hydroxy$C_{1-4}$alkyl, halo$C_{1-4}$alkyl, $C_{2-4}$alkenyl, $C_{2-4}$alkynyl, -$NR_{6a}R_{6b}$, or -C(=O)$NR_{6a}R_{6b}$;

each $R_{6a}$ and $R_{6b}$ independently represent hydrogen or $C_{1-4}$alkyl;

each $R_7$ and $R_8$ independently represent hydrogen, $C_{1-4}$alkyl, halo$C_{1-4}$alkyl, or $C_{3-6}$cycloalkyl; or

$R_7$ and $R_8$ are taken together with the nitrogen to which they are attached to form a 4 to 7 membered saturated monocyclic heterocyclic ring which optionally contains 1 further heteroatom selected from N, O or $SO_2$, said heterocyclic ring being optionally substituted with 1 to 4 substituents each independently selected from $C_{1-4}$alkyl, halo, hydroxyl, or halo$C_{1-4}$alkyl;

$R_9$ represents $C_{1-4}$alkyl, halo$C_{1-4}$alkyl, or $C_{3-6}$cycloalkyl;

each $R_{10}$ independently represents a 4 to 7 membered saturated monocyclic heterocyclic ring containing up to 2 heteroatoms selected from N, O or $SO_2$, said heterocyclic ring being optionally substituted with 1 to 4 substituents each independently selected from $C_{1-4}$alkyl, halo, hydroxyl or halo$C_{1-4}$alkyl;

each $R_{11}$ independently represents $C_{3-6}$cycloalkyl, phenyl, or a 4 to 7 membered monocyclic heterocyclic ring containing up to 3 heteroatoms selected from N, O or $SO_2$, said heterocyclic ring being optionally substituted with 1 to 4 substituents each independently selected from $C_{1-4}$alkyl, halo, hydroxyl, or halo$C_{1-4}$alkyl;

each $R_{12}$ independently represents hydrogen or $C_{1-4}$alkyl;

Q represents a 4 to 7 membered saturated monocyclic heterocyclic ring containing up to 3 heteroatoms selected from N, O or $SO_2$, said heterocyclic ring being optionally substituted with 1 to 4 substituents each independently selected from $C_{1-4}$alkyl, halo, hydroxyl or halo$C_{1-4}$alkyl;

n represents an integer of value 1 or 2;

m represents an integer of value 1 or 2 ;

p represents an integer of value 1 or 2;

p1 represents an integer of value 1 or 2;

each p2 independently represents an integer of value 0, 1 or 2;

r represents an integer of value 0, 1 or 2;

each $p_3$ independently represents an integer of value 0 or 1;

each s independently represents an integer of value 0, 1 or 2.

**3.** The compound according to claim 1, wherein

$X_a$, $X_b$ and $X_c$ each independently represent CH or N;

-$X_1$- represents -$(CHR_{12})_s$-$NR_1$-$X_e$-$C_{1-4}$alkanediyl-$(SO_2)_{p3}$;

-$X_e$- represents -$C(R_2)_2$-;

a represents -$NR_4$-C(=O)-$[C(R_{5b})_2]_r$- or -$NR_4$-$C(R_{5b})_2$-C(=O)-;

b represents

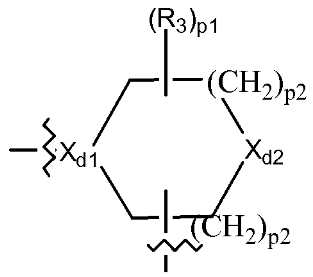

,

wherein said b ring may contain extra bonds to form a bridged ring system selected from 2,5-diazabicyclo[2.2.2]octanyl, 3,8-diazabicyclo[3.2.1]octanyl;

$X_{d1}$ represents CH or N;
$X_{d2}$ represents NH;
c represents a bond, $-[C(R_{5a})_2]_m-$, -O-, $-NR_{5a'}-$;
ring

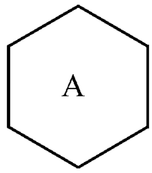

represents phenyl or pyridyl;
$R_1$ represents hydrogen, $C_{1-4}$alkyl, $C_{2-4}$alkenyl, hydroxy$C_{1-4}$alkyl, $C_{1-4}$alkyloxy$C_{1-4}$alkyl, $C_{1-4}$alkyl substituted with $R_{11}$, or $-C(=O)-R_{11}$; in particular $R_1$ represents hydrogen, $C_{1-4}$alkyl, $C_{2-4}$alkenyl, $C_{1-4}$alkyl substituted with $R_{11}$, or $-C(=O)-R_{11}$;
each $R_2$ independently represents hydrogen, $C_{1-4}$alkyl, $C_{1-4}$alkyl substituted with $C_{3-6}$cycloalkyl, carboxyl, $-C(=O)-O-C_{1-4}$alkyl, $-C(=O)-NH_2$, $-C(=O)-NH(C_{1-4}$alkyl);
or $R_1$ and one $R_2$ are taken together to form $C_{1-4}$alkanediyl or $C_{2-4}$alkenediyl, each of said $C_{1-4}$alkanediyl and $C_{2-4}$alkenediyl optionally being substituted with 1 substituent selected from hydroxyl, oxo, halo, cyano, $N_3$, $-NR_7R_8$, $-NH-SO_2-NR_7R_8$;
or $R_1$ and $R_{12}$ are taken together to form $C_{1-4}$alkanediyl;
each $R_3$ independently represents hydrogen; hydroxy$C_{1-4}$alkyl; $C_{1-4}$alkyl; or $C_{1-4}$alkyloxy$C_{1-4}$alkyl optionally substituted with cyano or $-NR_{3e}R_{3f}$; or
two $R_3$ substituents attached to the same carbon atom are taken together to form $C_{2-5}$alkanediyl;
each $R_{3e}$ and $R_{3f}$ independently represent hydrogen, or $-(C=O)-C_{1-4}$alkyl;
$R_4$ represents hydrogen or $C_{1-4}$alkyl;
each $R_{5a}$ independently represents hydrogen or $C_{1-4}$alkyl; or
two $R_{5a}$ substituents attached to the same carbon atom are taken together to form $C_{2-5}$alkanediyl or $-(CH_2)_p-O-(CH_2)_p-$;
$R_{5a'}$ represents hydrogen or $C_{1-4}$alkyl;
each $R_{5b}$ independently represents hydrogen; $C_{1-4}$alkyl; $C_{1-4}$alkyl substituted with $NR_{5b1}R_{5b2}$; $C_{1-4}$alkyloxy$C_{1-4}$alkyl; hydroxy$C_{1-4}$alkyl; hydroxyl; $C_{3-6}$cycloalkyl; or phenyl optionally substituted with $C_{1-4}$alkyl, halo, hydroxyl or $C_{1-4}$alkyloxy; or
two $R_{5b}$ substituents attached to the same carbon atom are taken together to form $C_{2-5}$alkanediyl or $-(CH_2)_p-O-(CH_2)_p-$;
$R_{5b1}$ and $R_{5b2}$ independently represent hydrogen, $-(C=O)-C_{1-4}$alkyl;
each $R_6$ independently represents hydrogen, halo, or $-C(=O)NR_{6a}R_{6b}$;
each $R_{6a}$ and $R_{6b}$ independently represent hydrogen or $C_{1-4}$alkyl;
each $R_7$ and $R_8$ independently represent hydrogen;
each $R_{11}$ independently represents $C_{3-6}$cycloalkyl;
each $R_{12}$ independently represents hydrogen or $C_{1-4}$alkyl;
n represents an integer of value 1;
m represents an integer of value 1;

p represents an integer of value 1;
p1 represents an integer of value 1 or 2;
each p2 independently represents an integer of value 0, 1 or 2;
r represents an integer of value 1;
each $p_3$ independently represents an integer of value 0 or 1;
each s independently represents an integer of value 0 or 1.

**4.** The compound according to claim 1, wherein
$X_a$ is N; $X_b$ and $X_c$ represent CH;
$R_1$ represents hydrogen, $C_{1-4}$alkyl, $C_{2-4}$alkenyl, $C_{2-4}$alkynyl, cyano$C_{1-4}$alkyl, -C(=O)-$C_{1-4}$alkyl, -C(=O)-halo$C_{1-4}$alkyl, halo$C_{1-4}$alkyl, -C(=O)$NR_7R_8$, -$SO_2$-$NR_7R_8$, -$SO_2$-$R_9$, $R_{11}$, $C_{1-4}$alkyl substituted with $R_{11}$, -C(=O)-$R_{11}$, or -C(=O)-$C_{1-4}$alkyl-$R_{11}$; each $R_2$ independently represents hydrogen, $C_{1-4}$alkyl, $C_{1-4}$alkyl substituted with $C_{3-6}$cycloalkyl, hydroxy$C_{1-4}$alkyl, $C_{1-4}$alkyloxy$C_{1-4}$alkyl, carboxyl, -C(=O)-O-$C_{1-4}$alkyl wherein $C_{1-4}$alkyl is optionally substituted with $C_{1-4}$alkyloxy, or -C(=O)-$NH_2$; or
$R_1$ and one $R_2$ are taken together to form $C_{3-4}$alkanediyl or $C_{3-4}$alkenediyl, each of said $C_{3-4}$alkanediyl and $C_{3-4}$alkenediyl optionally being substituted with 1 to 4 substituents each independently selected from hydroxyl, oxo, halo, cyano, $N_3$, hydroxy$C_{1-4}$alkyl, -$NR_7R_8$, -$SO_2$-$NR_7R_8$, -NH-$SO_2$-$NR_7R_8$, -C(=O)-$NR_7R_8$, or -NH-C(=O)-$NR_7R_8$;
$R_{12}$ is hydrogen.

**5.** The compound according to claim 1, wherein
-$X_1$- represents -$CH_2$-$NR_1$-$CH_2$-$C_{1-4}$alkanediyl-, -$NR_1$-$CH_2$-$C_{2-4}$alkanediyl-, or -$X_1$-represents one of the following groups wherein -$(CH_2)_2$- is attached to 'variable a':

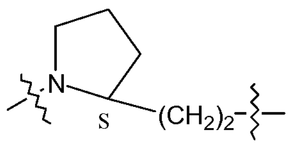 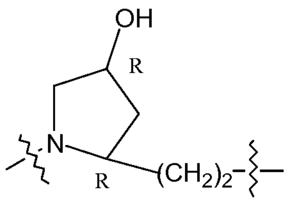 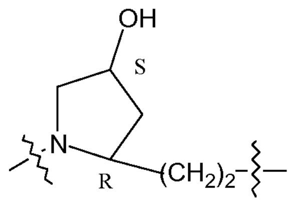

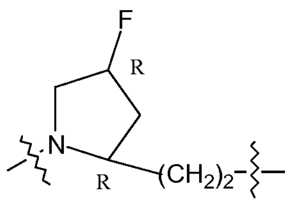 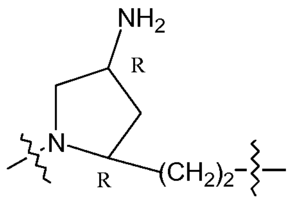 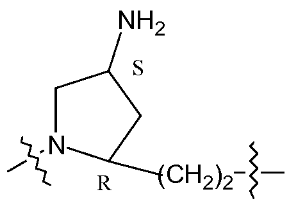

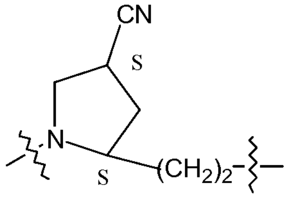 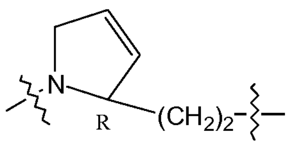 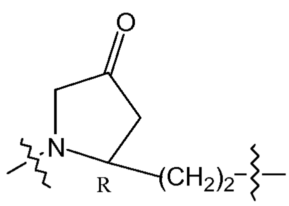

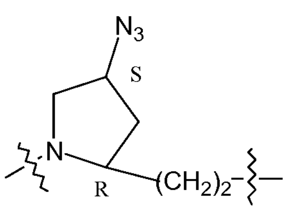 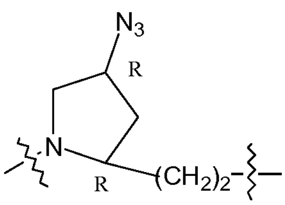 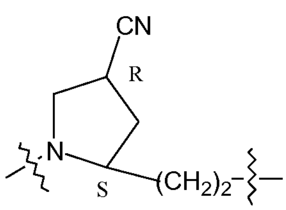

$R_1$ represents $C_{1-4}$alkyl, $C_{2-4}$alkenyl, $C_{2-4}$alkynyl, $C_{1-4}$alkyloxy$C_{1-4}$alkyl;
a represents -$NR_4$-C(=O)-$[C(R_{5b})_2]_r$- or -$NR_4$-$C(R_{5b})_2$-C(=O)-.

**6.** The compound according to claim 1, wherein if $R_1$ is taken together with one $R_2$, the bond towards the second $R_2$ substituent is oriented as shown hereunder:

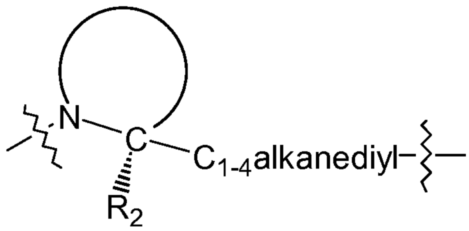

.

**7.** The compound according to claim 1, wherein b represents

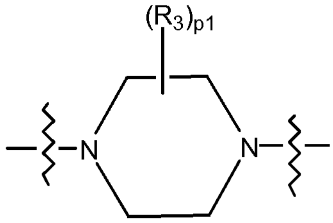

.

**8.** The compound according to claim 1, wherein
$R_1$ represents hydrogen, $C_{1-4}$alkyl, $C_{2-4}$alkenyl, hydroxy$C_{1-4}$alkyl, $C_{1-4}$alkyloxy$C_{1-4}$alkyl, $C_{1-4}$alkyl substituted with $R_{11}$, or -C(=O)-$R_{11}$;
each $R_2$ independently represents hydrogen, $C_{1-4}$alkyl, $C_{1-4}$alkyl substituted with $C_{3-6}$cycloalkyl, carboxyl, -C(=O)-O-$C_{1-4}$alkyl, -C(=O)-$NH_2$, -C(=O)-NH($C_{1-4}$alkyl); or $R_1$ and one $R_2$ are taken together to form $C_{1-4}$alkanediyl or $C_{2-4}$alkenediyl, each of said $C_{1-4}$alkanediyl and $C_{2-4}$alkenediyl optionally being substituted with 1 substituent selected from hydroxyl, oxo, halo, cyano, $N_3$, -$NR_7R_8$, -NH-$SO_2$-$NR_7R_8$.

**9.** The compound according to claim 1, wherein a represents -$NR_4$-C(=O)-$[C(R_{5b})_2]_r$-.

**10.** The compound according to claim 1 wherein c is $CH_2$.

**11.** The compound according to any one of claims 1-10, wherein
$X_a$ is N; $X_b$ and $X_c$ represent CH.

**12.** A pharmaceutical composition comprising a pharmaceutically acceptable carrier and, as active ingredient, a therapeutically effective amount of a compound according to any one of claims 1 to 11.

**13.** A compound as defined in any one of claims 1 to 11 for use as a medicament.

**14.** A compound as defined in any one of claims 1 to 11 for use in the treatment or prevention of a disease or condition selected from cancer, depression, and memory and learning disorders.

**15.** The compound according to claim 14 wherein the disease or condition is selected from glioblastoma, medulloblastoma, prostate cancer, breast cancer, ovarian cancer and colorectal cancer.

**Patentansprüche**

**1.** Verbindung der Formel (I)

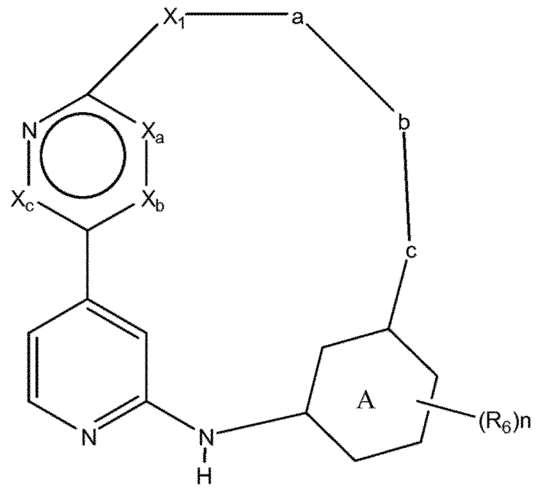

(I)

,

ein Tautomer oder eine stereoisomere Form davon, wobei $X_a$, $X_b$ und $X_c$ jeweils unabhängig voneinander für CH oder N stehen;

$-X_1-$ für - $(CHR_{12})_s-NR_1-X_e-C_{1-4}$-Alkandiyl-$(SO_2)_{p3}$- oder $-(CH_2)_s-O-X_e-C_{1-4}$-Alkandiyl- $(SO_2)_{p3}$- steht; wobei jede $C_{1-4}$-alkandiyl-Einheit wahlweise substituiert isted mit Hydroxyl oder Hydroxy-$C_{1-4}$-Alkyl;
$-X_e-$ für $-C(R_2)_2-$ oder -C(=O)- steht;
a für $-NR_4-C(=O)-[C(R_{5b})_2]_r-$ oder $-NR_4-C(R_{5b})_2-C(=O)-$ oder $-C(=O)-NR_4-C(R_{5b})2-$ steht;
b für Folgendes steht

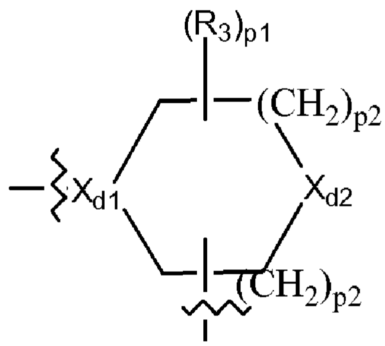

,

wobei der b-Ring zusätzliche Bindungen enthalten kann, um ein verbrücktes Ringsystem zu bilden, das ausgewählt ist aus 2,5-Diazabicyclo[2.2.2]octanyl, 3,8-Diazabicyclo[3.2.1]octanyl, 3,6-Diazabicyclo[3.1.1]heptanyl, 3,9-Diazabicyclo[3.3.1]nonyl;
$X_{d1}$ für CH oder N steht;
$X_{d2}$ für $CH_2$ oder NH steht;
mit der Maßgabe, dass mindestens eines von $X_{d1}$ und $X_{d2}$ für Stickstoff steht;
c für eine Bindung, $-[C(R_{5a})_2]_m-$, -C(=O)-, -O-, $-NR_{5a'}-$, $-SO_2-$ oder -SO- steht;
Ring

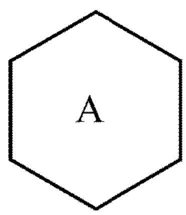

für Phenyl oder Pyridyl steht;
$R_1$ für Wasserstoff, $C_{1-4}$-Alkyl, $C_{2-4}$-Alkenyl, $C_{2-4}$-Alkynyl, Cyano-$C_{1-4}$-alkyl, -C(=O)-$C_{1-4}$-Alkyl, -C(=O)-Halo-

$C_{1-4}$-alkyl, Hydroxy-$C_{1-4}$-alkyl, Halo-$C_{1-4}$-alkyl, $C_{1-4}$-Alkyloxy-$C_{1-4}$-alkyl, Halo-$C_{1-4}$-Alkyloxy-$C_{1-4}$-alkyl, -C(=O)$NR_7R_8$, -$SO_2$-$NR_7R_8$, -$SO_2$-$R_9$, $R_{11}$, $C_{1-4}$-Alkyl, substituiert mit $R_{11}$, -C(=O)-$R_{11}$ oder -C (=O)-$C_{1-4}$-Alkyl-$R_{11}$ steht;

jedes $R_2$ unabhängig für Wasserstoff, $C_{1-4}$-Alkyl, $C_{1-4}$-Alkyl substituiert mit $C_{3-6}$-Cycloalkyl, Hydroxy-$C_{1-4}$alkyl, $C_{1-4}$-Alkyloxy-$C_{1-4}$-Alkyl, Carboxyl, -C(=O)-O-$C_{1-4}$-Alkyl steht, wobei $C_{1-4}$-Alkyl wahlweise substituiert ist mit $C_{1-4}$-Alkyloxy, -C(=O)-$NH_2$, -C(=O)-NH($C_{1-4}$-Alkyl), wobei $C_{1-4}$-Alkyl wahlweise substituiert ist mit $C_{1-4}$-Alkyloxy oder -C(=O)-N($C_{1-4}$-Alkyl)$_2$, wobei jedes $C_{1-4}$-Alkyl wahlweise substituiert ist mit $C_{1-4}$-Alkyloxy;

oder $R_1$ und $R_2$ zusammen genommen werden, um $C_{1-4}$-Alkandiyl oder $C_{2-4}$-Alkendiyl zu bilden, wobei jedes der $C_{1-4}$-Alkandiyle und $C_{2-4}$-Alkendiyl wahlweise mit 1 bis 4 Substituenten substituiert ist, die jeweils unabhängig ausgewählt sind aus Hydroxyl, Oxo, Halo, Cyano, $N_3$, Hydroxy-$C_{1-4}$-alkyl, -$NR_7R_8$, -$SO_2$-$NR_7R_8$, -NH-$SO_2$-$NR_7R_8$, -C(=O)-$NR_7R_8$ oder -NH-C(=O)-$NR_7R_8$;

oder $R_1$ und $R_{12}$ zusammen genommen werden, um $C_{1-4}$-Alkandiyl oder $C_{2-4}$-Alkendiyl zu bilden, wobei jedes der $C_{1-4}$-Alkandiyle und $C_{2-4}$-Alkendiyl wahlweise mit 1 bis 4 Substituenten substituiert ist, die jeweils unabhängig ausgewählt sind aus Hydroxyl, Oxo, Halo, Cyano, $N_3$, Hydroxy-$C_{1-4}$-alkyl, -$NR_7R_8$, -$SO_2$-$NR_7R_8$, -NH-$SO_2$-$NR_7R_8$, -C(=O)-$NR_7R_8$ oder -NH-C (=O)-$NR_7R_8$;

jedes $R_3$ unabhängig steht für Wasserstoff; Oxo; Hydroxyl; Carboxyl; -$NR_{3a}R_{3b}$; -C(=O)-$NR_{3a}R_{3b}$; Hydroxy-$C_{1-4}$-alkyl; Halo-$C_{1-4}$-alkyl; -(C=O)-$C_{1-4}$-Alkyl; -C(=O)-O-$C_{1-4}$-Alkyl steht, wobei $C_{1-4}$-Alkyl wahlweise mit Phenyl substituiert sein kann; $C_{1-4}$-Alkyl, wahlweise substituiert mit Cyano, Carboxyl, $C_{1-4}$-Alkyloxy, -C(=O)-O-$C_{1-4}$-Alkyl, -O-C(=O)-$C_{1-4}$-Alkyl, -$NR_{3e}R_{3f}$, -C(=O)-$NR_{3e}R_{3f}$, -$SO_2$-$NR_{3e}R_{3f}$, Q, -C(=O)-Q oder -$SO_2$-Q substituiert ist; Hydroxy-$C_{1-4}$-Alkyloxy-$C_{1-4}$-Alkyl; $C_{1-4}$-Alkyloxyhydroxy-$C_{1-4}$-Alkyl; Hydroxy-$C_{1-4}$ -alkyloxyhydroxy-$C_{1-4}$-alkyl; oder $C_{1-4}$-Alkyloxy$_{1-4}$-Alkyl, wahlweise substituiert mit Cyano, Carboxyl, $C_{1-4}$-Alkyloxy, -C(=O)-O-$C_{1-4}$-Alkyl, -O-C(=O)-$C_{1-4}$-Alkyl,-$NR_{3e}R_{3f}$, -C(=O)-$NR_{3e}R_{3f}$, -$SO_2$-$NR_{3e}R_{3f}$, $R_{10}$, -C(=O)-$R_{10}$ oder-$SO_2$-$R_{10}$; oder zwei $R_3$ -Substituenten, die an das gleiche Kohlenstoffatom gebunden sind, zusammengenommen werden, um $C_{2-5}$-Alkandiyl oder -$(CH_2)_p$-O-$(CH_2)_p$- zu bilden;

jedes $R_{3a}$ und $R_{3b}$ unabhängig voneinander für Wasserstoff; -(C=O)-$C_{1-4}$-Alkyl; -$SO_2$-$NR_{3c}R_{3d}$; oder $C_{1-4}$-Alkyl, wahlweise substituiert mit $C_{1-4}$-Alkyloxy steht; oder

$R_{3a}$ und $R_{3b}$ zusammen mit dem Stickstoff genommen werden, an den sie gebunden sind, einen 4- bis 7-gliedrigen gesättigten monocyclischen heterocyclischen Ring bilden, der wahlweise 1 oder 2 weitere Heteroatome enthält, die ausgewählt sind aus N, O oder $SO_2$, wobei der heterocyclische Ring wahlweise mit 1 bis 4 Substituenten substituiert ist, die jeweils unabhängig ausgewählt sind aus $C_{1-4}$-Alkyl, Halo, Hydroxyl oder Halo-$C_{1-4}$-Alkyl; jedes $R_{3c}$ und $R_{3d}$ unabhängig für Wasserstoff, $C_{1-4}$-Alkyl oder - (C=O) -$C_{1-4}$-Alkyl stehen; oder $R_{3c}$ und $R_{3d}$ zusammen mit dem Stickstoff genommen werden, an den sie gebunden sind, einen 4- bis 7-gliedrigen gesättigten monocyclischen heterocyclischen Ring bilden, der wahlweise 1 oder 2 weitere Heteroatome enthält, die ausgewählt sind aus N, O oder $SO_2$, wobei der heterocyclische Ring wahlweise mit 1 bis 4 Substituenten substituiert ist, die jeweils unabhängig ausgewählt sind aus $C_{1-4}$-Alkyl, Halo, Hydroxyl oder Halo-$C_{1-4}$-Alkyl;

jedes $R_{3e}$ und $R_{3f}$ unabhängig für Wasserstoff, $C_{1-4}$-Alkyl, wahlweise substituiert mit $C_{1-4}$-Alkyloxy, -(C=O) -$C_{1-4}$-Alkyl oder -$SO_2$-$NR_{3c}R_{3d}$ stehen;

$R_4$ für Wasserstoff, $C_{1-4}$-Alkyl oder $C_{1-4}$-Alkyloxy-$C_{1-4}$-Alkyl steht;

jedes $R_{5a}$ unabhängig für Wasserstoff oder $C_{1-4}$-Alkyl steht; oder

zwei $R_{5a}$-Substituenten, die an dasselbe Kohlenstoffatom gebunden sind, zusammen genommen werden, um $C_{2-5}$-Alkandiyl oder -$(CH_2)_p$-O-$(CH_2)_p$- zu bilden;

$R_{5a'}$ für Wasserstoff oder $C_{1-4}$-Alkyl steht;

jedes $R_{5b}$ unabhängig für Wasserstoff; $C_{1-4}$-Alkyl; $C_{1-4}$-Alkyl, das mit $NR_{5b1}R_{5b2}$ substituiert ist; $C_{1-4}$-Alkyloxy-$C_{1-4}$-Alkyl; Hydroxy-$C_{1-4}$-alkyl; Hydroxyl; $C_{3-6}$-Cycloalkyl; oder Phenyl, das wahlweise mit $C_{1-4}$-Alkyl, Halo, Hydroxy oder $C_{1-4}$-Alkyloxy substituiert ist, steht; oder zwei $R_{5b}$-Substituenten, die an dasselbe Kohlenstoffatom gebunden sind, zusammengenommen werden, um $C_{2-5}$-Alkandiyl oder - $(CH_2)_p$-O-$(CH_2)_p$- zu bilden;

jedes $R_{5b1}$ und $R_{5b2}$ unabhängig voneinander für Wasserstoff, $C_{1-4}$-Alkyl, wahlweise substituiert mit $C_{1-4}$-Alkyloxy, - (C=O) -$C_{1-4}$-Alkyl oder -$SO_2$-$NR_{5b3}R_{5b4}$ stehen;

$R_{5b3}$ und $R_{5b4}$ unabhängig für Wasserstoff, $C_{1-4}$-Alkyl oder - (C=O) -$C_{1-4}$-Alkyl stehen; oder $R_{5b3}$ und $R_{5b4}$ zusammen mit dem Stickstoff genommen werden, an den sie gebunden sind, einen 4- bis 7-gliedrigen gesättigten monocyclischen heterocyclischen Ring bilden, der wahlweise 1 oder 2 weitere Heteroatome enthält, die ausgewählt sind aus N, O oder $SO_2$, wobei der heterocyclische Ring wahlweise mit 1 bis 4 Substituenten substituiert ist, die jeweils unabhängig ausgewählt sind aus $C_{1-4}$-Alkyl, Halo, Hydroxyl oder Halo-$C_{1-4}$-Alkyl; jedes $R_6$ unabhängig für Wasserstoff, Halo, Hydroxyl, Carboxyl, Cyano, $C_{1-4}$-Alkyl, $C_{1-4}$-Alkyloxy-$C_{1-4}$-alkyl, Hydroxy-$C_{1-4}$-alkyl, Halo-$C_{1-4}$-Alkyl, $C_{2-4}$-Alkenyl, $C_{2-4}$-Alkynyl, -$NR_{6a}R_{6b}$ oder -C (=O) -$NR_{6a}R_{6b}$ steht;

jedes $R_{6a}$ und $R_{6b}$ unabhängig für Wasserstoff oder $C_{1-4}$-Alkyl stehen;

jede $R_7$ und $R_8$ unabhängig für Wasserstoff, $C_{1-4}$-Alkyl, Halo-$C_{1-4}$-Alkyl oder $C_{3-6}$-Cycloalkyl stehen; oder

$R_7$ und $R_8$ zusammen mit dem Stickstoff, an den sie gebunden sind, einen 4- bis 7-gliedrigen gesättigten

monocyclischen heterocyclischen Ring bilden, der wahlweise 1 Heteroatom enthält, die ausgewählt sind aus N, O oder $SO_2$, wobei der heterocyclische Ring wahlweise mit 1 bis 4 Substituenten substituiert ist, die jeweils unabhängig ausgewählt sind aus $C_{1-4}$-Alkyl, Halo, Hydroxyl oder Halo-$C_{1-4}$-Alkyl;
$R_9$ für $C_{1-4}$-Alkyl, Halo-$C_{1-4}$-Alkyl oder $C_{3-6}$-Cycloalkyl steht;
jedes $R_{10}$ unabhängig für einen 4- bis 7-gliedrigen gesättigten monocyclischen heterocyclischen Ring steht, der bis zu 2 Heteroatome enthält, die ausgewählt sind aus N, O oder $SO_2$, wobei der heterocyclische Ring wahlweise mit 1 bis 4 Substituenten substituiert ist, die jeweils unabhängig ausgewählt sind aus $C_{1-4}$-Alkyl, Halo, Hydroxyl oder Halo-$C_{1-4}$-Alkyl;
jedes $R_{11}$ unabhängig für $C_{3-6}$-Cycloalkyl, Phenyl oder einen 4- bis 7-gliedrigen gesättigten monocyclischen heterocyclischen Ring steht, der bis zu 3 Heteroatome enthält, die ausgewählt sind aus N, O oder $SO_2$, wobei der heterocyclische Ring wahlweise mit 1 bis 4 Substituenten substituiert ist, die jeweils unabhängig ausgewählt sind aus $C_{1-4}$-Alkyl, Halo, Hydroxyl oder Halo-$C_{1-4}$-Alkyl; jedes $R_{12}$ unabhängig für Wasserstoff oder $C_{1-4}$-Alkyl steht;
Q für einen 4- bis 7-gliedrigen gesättigten monocyclischen heterocyclischen Ring steht, der bis zu 3 Heteroatome enthält, die ausgewählt sind aus N, O oder $SO_2$, wobei der heterocyclische Ring wahlweise mit 1 bis 4 Substituenten substituiert ist, die jeweils unabhängig ausgewählt sind aus $C_{1-4}$-Alkyl, Halo, Hydroxyl oder Halo-$C_{1-4}$-Alkyl ;
n für eine ganze Zahl des Werts 1 oder 2 steht;
m für eine ganze Zahl des Werts 1 oder 2 steht;
p für eine ganze Zahl des Werts 1 oder 2 steht;
p1 für eine ganze Zahl des Werts 1 oder 2 steht;
jedes p2 unabhängig für eine ganze Zahl des Werts 0, 1 oder 2 steht;
r für eine ganze Zahl des Werts 0, 1 oder 2 steht;
jedes p3 unabhängig für eine ganze Zahl des Werts 0 oder 1 steht;
jedes s unabhängig für eine ganze Zahl des Werts 0, 1 oder 2 steht;
oder ein N-Oxid, ein pharmazeutisch verträgliches Salz oder Solvat davon.

2. Verbindung nach Anspruch 1, wobei $X_a$, $X_b$ und $X_c$ jeweils unabhängig für CH oder N stehen;
$-X_1-$ für - $(CHR_{12})_s$-$NR_1$-$X_e$-$C_{1-4}$-Alkandiyl-$(SO_2)_{p3}$-; $-X_e-$ für $-C(R_2)_2-$ steht;
a für $-NR_4-C(=O)-[C(R_{5b})_2]_r-$ oder $-NR_4-C(R_{5b})_2-C(=O)$-steht;
b für Folgendes steht

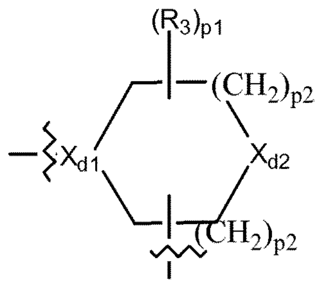

,

wobei der b-Ring zusätzliche Bindungen enthalten kann, um ein verbrücktes Ringsystem zu bilden, das ausgewählt ist aus 2,5-Diazabicyclo[2.2.2]octanyl, 3,8-Diazabicyclo[3.2.1]octanyl, 3,6-Diazabicyclo[3.1.1]heptanyl, 3,9-Diazabicyclo[3.3.1]nonyl;

$X_{d1}$ für CH oder N steht;
$X_{d2}$ für NH steht;
mit der Maßgabe, dass mindestens eines von $X_{d1}$ und $X_{d2}$ für Stickstoff steht;
c für eine Bindung, $-[C(R_{5a})_2]_m-$, -O-, $-NR_{5a'}-$ steht;
Ring

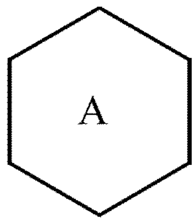

für Phenyl oder Pyridyl steht;

$R_1$ für Wasserstoff, $C_{1-4}$-Alkyl, $C_{2-4}$-Alkenyl, $C_{2-4}$-Alkynyl, Cyano-$C_{1-4}$-alkyl, -C(=O)-$C_{1-4}$-Alkyl, -C(=O)-Halo-$C_{1-4}$-alkyl, Halo-$C_{1-4}$-alkyl, -C(=O)$NR_7R_8$, -$SO_2$-$NR_7R_8$, -$SO_2$-$R_9$, $R_{11}$, $C_{1-4}$-Alkyl, das mit $R_{11}$ substituiert ist, -C(=O)-$R_{11}$ oder -C(=O)-$C_{1-4}$-Alkyl-$R_{11}$ steht;

jedes $R_2$ unabhängig für Wasserstoff, $C_{1-4}$-Alkyl, $C_{1-4}$-Alkyl substituiert mit $C_{3-6}$-Cycloalkyl, Hydroxy-$C_{1-4}$alkyl, $C_{1-4}$-Alkyloxy-$C_{1-4}$-Alkyl, Carboxyl, -C(=O)-O-$C_{1-4}$-Alkyl steht, wobei $C_{1-4}$-Alkyl wahlweise substituiert ist mit $C_{1-4}$-Alkyloxy, -C(=O)-$NH_2$, -C(=O)-NH($C_{1-4}$-Alkyl), wobei $C_{1-4}$-Alkyl wahlweise substituiert ist mit $C_{1-4}$-Alkyloxy oder-C(=O)-N($C_{1-4}$-Alkyl)$_2$, wobei jedes $C_{1-4}$-Alkyl wahlweise substituiert ist mit $C_{1-4}$-Alkyloxy;

oder $R_1$ und ein $R_2$ zusammen genommen werden, um $C_{3-4}$-Alkandiyl oder $C_{3-4}$-Alkendiyl zu bilden, wobei jedes der $C_{3-4}$-Alkandiyle und $C_{3-4}$-Alkendiyl wahlweise mit 1 bis 4 Substituenten substituiert ist, die jeweils unabhängig ausgewählt sind aus Hydroxyl, Oxo, Halo, Cyano, $N_3$, Hydroxy-$C_{1-4}$-alkyl, -$NR_7R_8$, -$SO_2$-$NR_7R_8$, -NH-$SO_2$-$NR_7R_8$, - C(=O) -$NR_7R_8$ oder -NH-C(=O) -$NR_7R_8$;

jedes $R_3$ unabhängig für Wasserstoff; Oxo; Hydroxyl; Carboxyl; -$NR_{3a}R_{3b}$; -C(=O) -$NR_{3a}R_{3b}$; Hydroxy-$C_{1-4}$-alkyl; Halo-$C_{1-4}$-alkyl; -(C=O) -$C_{1-4}$-Alkyl; -C(=O)-O-$C_{1-4}$-Alkyl steht, wobei $C_{1-4}$-Alkyl wahlweise mit Phenyl substituiert sein kann; $C_{1-4}$-Alkyl, wahlweise substituiert mit Cyano, Carboxyl, $C_{1-4}$-Alkyloxy substituiert ist, -C(=O)-O-$C_{1-4}$-Alkyl, -O-C(=O)-$C_{1-4}$-Alkyl, -$NR_{3e}R_{3f}$, -C(=O) -$NR_{3e}R_{3f}$, -$SO_2$-$NR_{3e}R_{3f}$, Q, -C(=O)-Q oder -$SO_2$-Q; Hydroxy-$C_{1-4}$-Alkyloxy-$C_{1-4}$-Alkyl; $C_{1-4}$-Alkyloxyhydroxy-$C_{1-4}$-Alkyl; Hydroxy-$C_{1-4}$ -alkyloxyhydroxy-$C_{1-4}$-alkyl steht; oder $C_{1-4}$-Alkyloxy$_{1-4}$-Alkyl, wahlweise substituiert mit Cyano, Carboxyl, $C_{1-4}$-Alkyloxy, -C (=O) -O-$C_{1-4}$-Alkyl, -O-C(=O)-$C_{1-4}$- Alkyl, -$NR_{3e}R_{3f}$, -C(=O)-$NR_{3e}R_{3f}$, -$SO_2$-$NR_{3e}R_{3f}$, $R_{10}$, -C(=O)-$R_{10}$ oder -$SO_2$-$R_{10}$; oder zwei $R_3$ -Substituenten, die an das gleiche Kohlenstoffatom gebunden sind, zusammengenommen werden, um $C_{2-5}$-Alkandiyl oder -($CH_2$)$_p$-O-($CH_2$)$_p$- zu bilden; jedes $R_{3a}$ und $R_{3b}$ unabhängig voneinander für Wasserstoff; -(C=O)-$C_{1-4}$-Alkyl; -$SO_2$-$NR_{3c}R_{3d}$; oder $C_{1-4}$-Alkyl, wahlweise substituiert mit $C_{1-4}$-Alkyloxy steht; oder

$R_{3a}$ und $R_{3b}$ zusammen mit dem Stickstoff genommen werden, an den sie gebunden sind, einen 4- bis 7-gliedrigen gesättigten monocyclischen heterocyclischen Ring bilden, der wahlweise 1 oder 2 weitere Heteroatome enthält, die ausgewählt sind aus N, O oder $SO_2$, wobei der heterocyclische Ring wahlweise mit 1 bis 4 Substituenten substituiert ist, die jeweils unabhängig ausgewählt sind aus $C_{1-4}$-Alkyl, Halo, Hydroxyl oder Halo-$C_{1-4}$-Alkyl; jedes $R_{3c}$ und $R_{3d}$ unabhängig für Wasserstoff, $C_{1-4}$-Alkyl oder -(C=O)-$C_{1-4}$-Alkyl stehen; oder $R_{3c}$ und $R_{3d}$ zusammen mit dem Stickstoff genommen werden, an den sie gebunden sind, einen 4- bis 7-gliedrigen gesättigten monocyclischen heterocyclischen Ring bilden, der wahlweise 1 oder 2 weitere Heteroatome enthält, die ausgewählt sind aus N, O oder $SO_2$, wobei der heterocyclische Ring wahlweise mit 1 bis 4 Substituenten substituiert ist, die jeweils unabhängig ausgewählt sind aus $C_{1-4}$-Alkyl, Halo, Hydroxyl oder Halo-$C_{1-4}$-Alkyl; jedes $R_{3e}$ und $R_{3f}$ unabhängig für Wasserstoff, $C_{1-4}$-Alkyl, wahlweise substituiert mit $C_{1-4}$-Alkyloxy, -(C=O)-$C_{1-4}$-Alkyl oder -$SO_2$-$NR_{3c}R_{3d}$ stehen;

$R_4$ für Wasserstoff, $C_{1-4}$-Alkyl oder $C_{1-4}$-Alkyloxy-$C_{1-4}$-Alkyl steht;

jedes $R_{5a}$ unabhängig für Wasserstoff oder $C_{1-4}$-Alkyl steht; oder

zwei $R_{5a}$-Substituenten, die an dasselbe Kohlenstoffatom gebunden sind, zusammen genommen werden, um $C_{2-5}$-Alkandiyl oder -($CH_2$)$_p$-O-($CH_2$)$_p$- zu bilden;

$R_{5a'}$ für Wasserstoff oder $C_{1-4}$-Alkyl steht;

jedes $R_{5b}$ unabhängig für Wasserstoff; $C_{1-4}$-Alkyl; $C_{1-4}$-Alkyl, das mit $NR_{5b1}R_{5b2}$ substituiert ist; $C_{1-4}$-Alkyloxy-$C_{1-4}$-Alkyl; Hydroxy-$C_{1-4}$-alkyl; Hydroxyl; $C_{3-6}$-Cycloalkyl; oder Phenyl, das wahlweise mit $C_{1-4}$-Alkyl, Halo, Hydroxyl oder $C_{1-4}$-Alkyloxy substituiert ist, steht; oder zwei $R_{5b}$-Substituenten, die an dasselbe Kohlenstoffatom gebunden sind, zusammengenommen werden, um $C_{2-5}$-Alkandiyl oder - ($CH_2$)$_p$-O-($CH_2$)$_p$- zu bilden;

jedes $R_{5b1}$ und $R_{5b2}$ unabhängig voneinander für Wasserstoff, $C_{1-4}$-Alkyl, wahlweise substituiert mit $C_{1-4}$-Alkyloxy, - (C=O) -$C_{1-4}$-Alkyl oder -$SO_2$-$NR_{5b3}R_{5b4}$ stehen;

$R_{5b3}$ und $R_{5b4}$ unabhängig für Wasserstoff, $C_{1-4}$-Alkyl oder -(C=O)-$C_{1-4}$-Alkyl stehen; oder $R_{5b3}$ und $R_{5b4}$ zusammen mit dem Stickstoff genommen werden, an den sie gebunden sind, einen 4- bis 7-gliedrigen gesättigten monocyclischen heterocyclischen Ring bilden, der wahlweise 1 oder 2 weitere Heteroatome enthält, die ausgewählt sind aus N, O oder $SO_2$, wobei der heterocyclische Ring wahlweise mit 1 bis 4 Substituenten substituiert ist, die jeweils unabhängig ausgewählt sind aus $C_{1-4}$-Alkyl, Halo, Hydroxyl oder Halo-$C_{1-4}$-Alkyl; jedes $R_6$ un-

abhängig für Wasserstoff, Halo, Hydroxyl, Carboxyl, Cyano, $C_{1-4}$-Alkyl, $C_{1-4}$-Alkyloxy-$C_{1-4}$-alkyl, Hydroxy-$C_{1-4}$-alkyl, Halo-$C_{1-4}$-Alkyl, $C_{2-4}$-Alkenyl, $C_{2-4}$-Alkynyl, $-NR_{6a}R_{6b}$ oder -C(=O) $-NR_{6a}R_{6b}$ steht;
jedes $R_{6a}$ und $R_{6b}$ unabhängig für Wasserstoff oder $C_{1-4}$-Alkyl stehen;
jede $R_7$ und $R_8$ unabhängig für Wasserstoff, $C_{1-4}$-Alkyl, Halo-$C_{1-4}$-Alkyl oder $C_{3-6}$-Cycloalkyl stehen; oder
$R_7$ und $R_8$ zusammen mit dem Stickstoff, an den sie gebunden sind, einen 4- bis 7-gliedrigen gesättigten monocyclischen heterocyclischen Ring bilden, der wahlweise 1 Heteroatom enthält, die ausgewählt sind aus N, O oder $SO_2$, wobei der heterocyclische Ring wahlweise mit 1 bis 4 Substituenten substituiert ist, die jeweils unabhängig ausgewählt sind aus $C_{1-4}$-Alkyl, Halo, Hydroxyl oder Halo-$C_{1-4}$-Alkyl;
$R_9$ für $C_{1-4}$-Alkyl, Halo-$C_{1-4}$-Alkyl oder $C_{3-6}$-Cycloalkyl steht;
jedes $R_{10}$ unabhängig für einen 4- bis 7-gliedrigen gesättigten monocyclischen heterocyclischen Ring steht, der bis zu 2 Heteroatome enthält, die ausgewählt sind aus N, O oder $SO_2$, wobei der heterocyclische Ring wahlweise mit 1 bis 4 Substituenten substituiert ist, die jeweils unabhängig ausgewählt sind aus $C_{1-4}$-Alkyl, Halo, Hydroxyl oder Halo-$C_{1-4}$-Alkyl;
jedes $R_{11}$ unabhängig für $C_{3-6}$-Cycloalkyl, Phenyl oder einen 4- bis 7-gliedrigen gesättigten monocyclischen heterocyclischen Ring steht, der bis zu 3 Heteroatome enthält, die ausgewählt sind aus N, O oder $SO_2$, wobei der heterocyclische Ring wahlweise mit 1 bis 4 Substituenten substituiert ist, die jeweils unabhängig ausgewählt sind aus $C_{1-4}$-Alkyl, Halo, Hydroxyl oder Halo-$C_{1-4}$-Alkyl; jedes $R_{12}$ unabhängig für Wasserstoff oder $C_{1-4}$-Alkyl steht; Q für einen 4- bis 7-gliedrigen gesättigten monocyclischen heterocyclischen Ring steht, der bis zu 3 Heteroatome enthält, die ausgewählt sind aus N, O oder $SO_2$, wobei der heterocyclische Ring wahlweise mit 1 bis 4 Substituenten substituiert ist, die jeweils unabhängig ausgewählt sind aus $C_{1-4}$-Alkyl, Halo, Hydroxyl oder Halo-$C_{1-4}$-Alkyl;
n für eine ganze Zahl des Werts 1 oder 2 steht;
m für eine ganze Zahl des Werts 1 oder 2 steht;
p für eine ganze Zahl des Werts 1 oder 2 steht;
p1 für eine ganze Zahl des Werts 1 oder 2 steht;
jedes p2 unabhängig für eine ganze Zahl des Werts 0, 1 oder 2 steht;
r für eine ganze Zahl des Werts 0, 1 oder 2 steht;
jedes p3 unabhängig für eine ganze Zahl des Werts 0 oder 1 steht;
jedes s unabhängig für eine ganze Zahl des Werts 0, 1 oder 2 steht.

**3.** Verbindung nach Anspruch 1, wobei $X_a$, $X_b$ und $X_c$ jeweils unabhängig für CH oder N stehen;
$-X_1-$ für $-(CHR_{12})_s-NR_1-X_e-C_{1-4}$-Alkandiyl-$(SO_2)_{p3}-$ steht; $-X_e-$ für $-C(R_2)_2-$ steht;
a für $-NR_4-C(=O)-[C(R_{5b})_2]_r-$ oder $-NR_4-C(R_{5b})_2-C(=O)-$steht; b für Folgendes steht

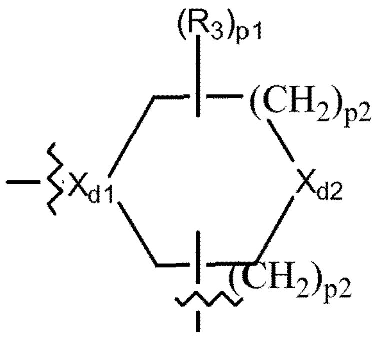

,

wobei der b-Ring zusätzliche Bindungen enthalten kann, um ein verbrücktes Ringsystem zu bilden, das ausgewählt ist aus 2,5-Diazabicyclo[2.2.2]octanyl, 3,8-Diazabicyclo[3.2.1]octanyl;

$X_{d1}$ für CH oder N steht;
$X_{d2}$ für NH steht;
c für eine Bindung, $-[C(R_{5a})_2]_m-$, -O-, $-NR_{5a'}-$ steht;
Ring

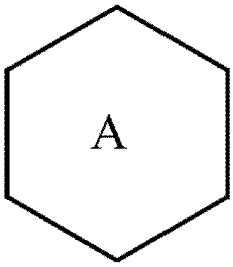

für Phenyl oder Pyridyl steht;
$R_1$ für Wasserstoff, $C_{1-4}$-Alkyl, $C_{2-4}$-Alkenyl, Hydroxy-$C_{1-4}$-alkyl, $C_{1-4}$-Alkyloxy-$C_{1-4}$-alkyl, $C_{1-4}$-Alkyl, das mit $R_{11}$ substituiert ist, oder -C(=O)-$R_{11}$ steht; insbesondere $R_1$ für Wasserstoff, $C_{1-4}$-Alkyl, $C_{2-4}$-Alkenyl, $C_{1-4}$-Alkyl, das mit $R_{11}$ substituiert ist, oder -C(=O)-$R_{11}$ steht;
jedes $R_2$ unabhängig für Wasserstoff, $C_{1-4}$-Alkyl, $C_{1-4}$-Alkyl, das mit $C_{3-6}$-Cycloalkyl substituiert ist, Carboxyl, -C(=O)-O-$C_{1-4}$-Alkyl, -C(=O)-$NH_2$, -C(=O)-NH($C_{1-4}$-Alkyl) steht;
oder $R_1$ und $R_2$ zusammen genommen werden, um $C_{1-4}$-Alkandiyl oder $C_{2-4}$-Alkendiyl zu bilden, wobei jedes der $C_{1-4}$-Alkandiyle und $C_{2-4}$-Alkendiyl wahlweise mit 1 Substituenten substituiert sind, der ausgewählt ist aus Hydroxyl, Oxo, Halo, Cyano, N3, $NR_7R_8$, -NH-$SO_2$-$NR_7R_8$;
oder $R_1$ und $R_{12}$ zusammen genommen werden, um $C_{1-4}$-Alkandiyl zu bilden;
jedes $R_3$ unabhängig für Wasserstoff; Hydroxy-$C_{1-4}$-alkyl; $C_{1-4}$ -Alkyl; oder $C_{1-4}$-Alkyloxy-$C_{1-4}$-Alkyl, das wahlweise mit Cyano oder -$NR_{3e}R_{3f}$ substituiert ist, steht; oder zwei $R_3$-Substituenten, die an dasselbe Kohlenstoffatom gebunden sind, zusammen genommen werden, um $C_{2-5}$-Alkandiyl zu bilden;
jedes $R_{3e}$ und $R_{3f}$ unabhängig für Wasserstoff oder -(C=O)-$C_{1-4}$-Alkyl steht;
$R_4$ für Wasserstoff oder $C_{1-4}$-Alkyl steht;
jedes $R_{5a}$ unabhängig für Wasserstoff oder $C_{1-4}$-Alkyl steht; oder
zwei $R_{5a}$-Substituenten, die an dasselbe Kohlenstoffatom gebunden sind, zusammen genommen werden, um $C_{2-5}$-Alkandiyl oder -$(CH_2)_p$-O-$(CH_2)_p$- zu bilden;
$R_{5a'}$ für Wasserstoff oder $C_{1-4}$-Alkyl steht;
jedes $R_{5b}$ unabhängig für Wasserstoff; $C_{1-4}$-Alkyl; $C_{1-4}$-Alkyl, das mit $NR_{5b1}R_{5b2}$ substituiert ist; $C_{1-4}$-Alkyloxy-$C_{1-4}$-Alkyl; Hydroxy-$C_{1-4}$-alkyl; Hydroxyl; $C_{3-6}$-Cycloalkyl; oder Phenyl, das wahlweise mit $C_{1-4}$-Alkyl, Halo, Hydroxyl oder $C_{1-4}$-Alkyloxy substituiert ist, steht; oder zwei $R_{5b}$-Substituenten, die an dasselbe Kohlenstoffatom gebunden sind, zusammengenommen werden, um $C_{2-5}$-Alkandiyl oder - $(CH_2)_p$-O-$(CH_2)_p$- zu bilden;
$R_{5b1}$ und $R_{5b2}$ unabhängig für Wasserstoff, -(C=O)-$C_{1-4}$-Alkyl stehen;
jedes $R_6$ unabhängig für Wasserstoff, Halo oder -C(=O)-$NR_{6a}R_{6b}$ steht;
jedes $R_{6a}$ und $R_{6b}$ unabhängig für Wasserstoff oder $C_{1-4}$-Alkyl stehen;
jedes $R_7$ und $R_8$ unabhängig für Wasserstoff stehen;
jedes $R_{11}$ unabhängig für $C_{3-6}$-Cycloalkyl steht;
jedes $R_{12}$ unabhängig für Wasserstoff oder $C_{1-4}$-Alkyl steht;
n für eine ganze Zahl des Werts 1 steht;
m für eine ganze Zahl des Werts 1 steht;
p für eine ganze Zahl des Werts 1 steht;
p1 für eine ganze Zahl des Werts 1 oder 2 steht;
jedes p2 unabhängig für eine ganze Zahl des Werts 0, 1 oder 2 steht;
r für eine ganze Zahl des Werts 1 steht;
jedes p3 unabhängig für eine ganze Zahl des Werts 0 oder 1 steht;
jedes s unabhängig für eine ganze Zahl des Werts 0 oder 1 steht.

**4.** Verbindung nach Anspruch 1, wobei
$X_a$ N ist; $X_b$ und $X_c$ für CH stehen;
$R_1$ für Wasserstoff, $C_{1-4}$-Alkyl, $C_{2-4}$-Alkenyl, $C_{2-4}$-Alkynyl, Cyano-$C_{1-4}$-alkyl, -C(=O)-$C_{1-4}$-Alkyl, -C(=O)-Halo-$C_{1-4}$-alkyl, Halo-$C_{1-4}$-alkyl, -C(=O)$NR_7R_8$, -$SO_2$-$NR_7R_8$, -$SO_2$-$R_9$, $R_{11}$, $C_{1-4}$-Alkyl, das mit $R_{11}$ substituiert ist, -C(=O)-$R_{11}$ oder -C(=O)-$C_{1-4}$-Alkyl-$R_{11}$ steht;
jedes $R_2$ unabhängig für Wasserstoff, $C_{1-4}$-Alkyl, $C_{1-4}$-Alkyl, das mit $C_{3-6}$-Cycloalkyl substituiert ist, Hydroxy-$C_{1-4}$-Alkyl, $C_{1-4}$-Alkyloxy-$C_{1-4}$-Alkyl, Carboxyl, -C(=O)-O-$C_{1-4}$-Alkyl steht, wobei $C_{1-4}$-Alkyl wahlweise substituiert ist mit $C_{1-4}$-Alkyloxy, oder -C(=O)-$NH_2$; oder $R_1$ und ein $R_2$ zusammen genommen werden, um $C_{3-4}$-Alkandiyl oder $C_{3-4}$-Alkendiyl zu bilden, wobei jedes der $C_{3-4}$-Alkandiyle und $C_{3-4}$-Alkendiyl wahlweise mit 1 bis 4 Substituenten substituiert ist, die jeweils unabhängig ausgewählt sind aus Hydroxyl, Oxo, Halo, Cyano, $N_3$, Hydroxy-$C_{1-4}$-alkyl,-$NR_7R_8$,

$-SO_2-NR_7R_8$, $-NH-SO_2-NR_7R_8$, $-C(=O)-NR_7R_8$ oder $-NH-C(=O)-NR_7R_8$;
$R_{12}$ Wasserstoff ist.

**5.** Verbindung nach Anspruch 1, wobei
$-X_1-$ für $-CH_2-NR_1-CH_2-C_{1-4}$-Alkandiyl-, $-NR_1-CH_2-C_{2-4}$-Alkanediyl- steht oder $-X_1-$ für eine der folgenden Gruppen steht, wobei $-(CH_2)_2-$ an eine 'Variable a' angehängt ist:

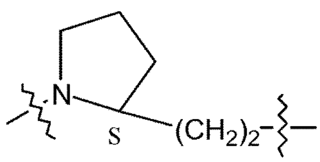
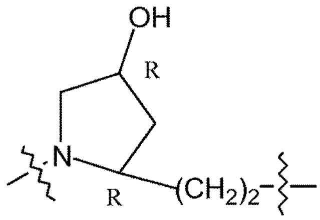
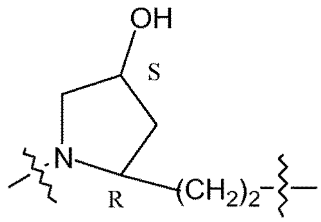

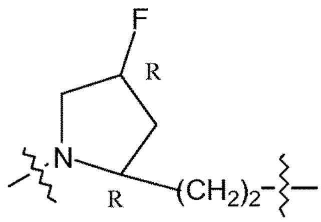
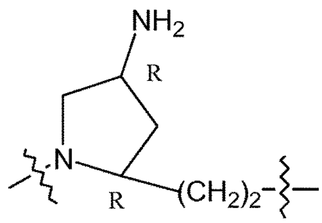
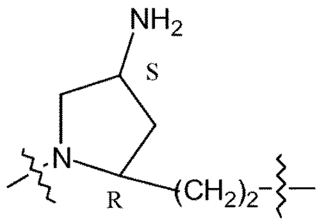

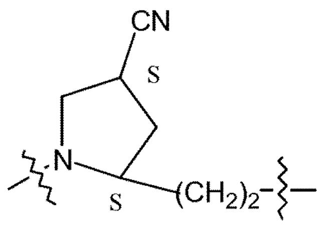
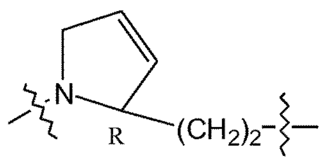
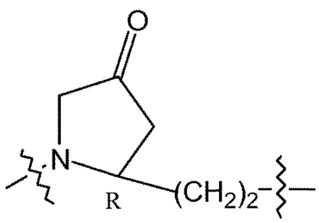

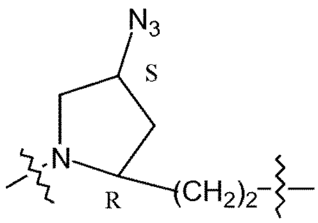
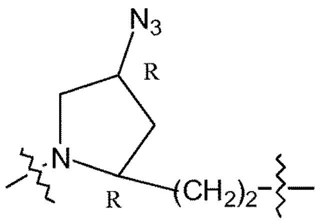
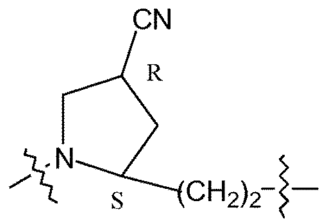

$R_1$ für $C_{1-4}$-Alkyl, $C_{2-4}$-Alkenyl, $C_{2-4}$-Alkynyl, $C_{1-4}$-Alkyloxy-$C_{1-4}$ -Alkyl steht;
a für $-NR_4-C(=O)-[C(R_{5b})_2]_r-$ oder $-NR_4-C(R_{5b})_2-C(=O)$-steht.

**6.** Verbindung nach Anspruch 1, wobei, wenn $R_1$ zusammen mit einem $R_2$ genommen wird, die Bindung in Richtung des zweiten $R_2$-Substituenten wie folgt ausgerichtet ist:

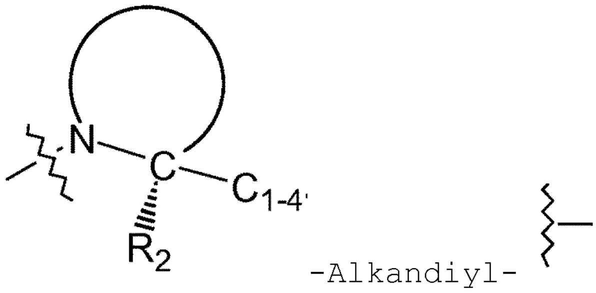

**7.** Verbindung nach Anspruch 1, wobei b für Folgendes steht:

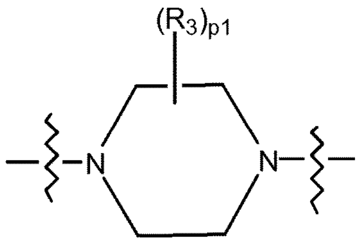

.

**8.** Verbindung nach Anspruch 1, wobei
$R_1$ für Wasserstoff, $C_{1-4}$-Alkyl, $C_{2-4}$-Alkenyl, Hydroxy-$C_{1-4}$-alkyl, $C_{1-4}$-Alkyloxy-$C_{1-4}$-alkyl, $C_{1-4}$-Alkyl, das mit $R_{11}$ substituiert ist, oder -C(=O)-$R_{11}$ steht;
jedes $R_2$ unabhängig für Wasserstoff, $C_{1-4}$-Alkyl, $C_{1-4}$-Alkyl, das mit $C_{3-6}$-Cycloalkyl substituiert ist, Carboxyl, -C(=O)-O-$C_{1-4}$-Alkyl, -C(=O)-$NH_2$, -C(=O)-NH($C_{1-4}$-Alkyl) steht; oder $R_1$ und $R_2$ zusammen genommen werden, um $C_{1-4}$-Alkandiyl oder $C_{2-4}$-Alkendiyl zu bilden, wobei jedes der $C_{1-4}$-Alkandiyle und $C_{2-4}$-Alkendiyl wahlweise mit 1 Substituenten substituiert sind, der ausgewählt ist aus Hydroxyl, Oxo, Halo, Cyano, $N_3$, $NR_7R_8$, -NH-$SO_2$-$NR_7R_8$.

**9.** Verbindung nach Anspruch 1, wobei a für -$NR_4$-C(=O)-$[C(R_{5b})_2]_r$- steht.

**10.** Verbindung nach Anspruch 1, wobei c $CH_2$ ist.

**11.** Verbindung nach einem der Ansprüche 1 bis 10, wobei $X_a$ N ist; $X_b$ und $X_c$ für CH stehen.

**12.** Pharmazeutische Zusammensetzung, umfassend einen pharmazeutisch verträglichen Träger und als Wirkstoff eine therapeutisch wirksame Menge einer Verbindung nach einem der Ansprüche 1 bis 11.

**13.** Verbindung wie in einem der Ansprüche 1 bis 11 definiert, für die Verwendung als ein Medikament.

**14.** Verbindung wie einem der Ansprüche 1 bis 11 definiert, für die Verwendung bei der Behandlung oder Prävention einer Krankheit oder eines Zustands, die ausgewählt sind aus Krebs, Depression, Gedächtnis- und Lernstörungen.

**15.** Verbindung nach Anspruch 14, wobei die Krankheit oder der Zustand ausgewählt ist aus Glioblastom, Medulloblastom, Prostatakrebs, Brustkrebs, Eierstockkrebs und Kolorektalkrebs.

## Revendications

**1.** Composé de Formule (I)

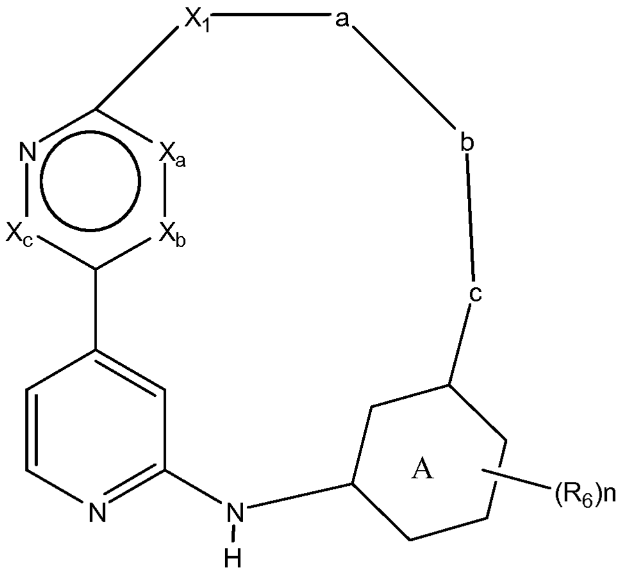

(I)

forme tautomère ou stéréoisomère de celui-ci, où chacun des radicaux $X_a$, $X_b$ et $X_c$ représente indépendamment CH ou N ;

-$X_1$- représente un groupement -$(CHR_{12})_s$-$NR_1$-$X_e$-(alcanediyle en $C_{1-4}$)-$(SO_2)_{p3}$- ou -$(CH_2)_s$-O-$X_e$-(alcanediyle en $C_{1-4}$)-$(SO_2)_{p3}$- ; où chacune desdites entités alcanediyle en $C_{1-4}$ est éventuellement substituée par hydroxyle ou hydroxyalkyle en $C_{1-4}$ ;
-$X_e$- représente -$C(R_2)_2$- ou -C(=O)- ;
a représente -$NR_4$-C(=O)-$[C(R_{5b})_2]_r$-, -$NR_4$-$C(R_{5b})_2$-C(=O) - ou -C (=O)-$NR_4$-$C(R_{5b})_2$- ;
b représente

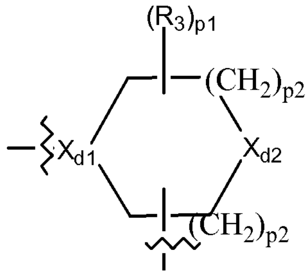

,

où ledit cycle b peut contenir des liaisons supplémentaires pour former un système cyclique ponté choisi parmi les groupements 2,5-diazabicyclo[2.2.2]octanyle, 3,8-diazabicyclo[3.2.1]octanyle, 3,6-diazabicyclo[3.1.1]heptanyle, 3,9-diazabicyclo[3.3.1]nonyle ;
$X_{d1}$ représente CH ou N ;
$X_{d2}$ représente $CH_2$ ou NH ;
à la condition qu'au moins l'un des radicaux $X_{d1}$ et $X_{d2}$ représente un atome d'azote ;
c représente une liaison ou un groupement -$[C(R_{5a})_2]_m$-,-C(=O)-, -O-, -$NR_{5a'}$-, -$SO_2$- ou -SO- ;
le cycle

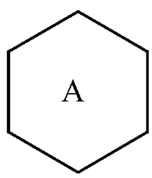

représente un groupement phényle ou pyridyle ;

$R_1$ représente un atome d'hydrogène ou un groupement alkyle en $C_{1-4}$, alcényle en $C_{2-4}$, alcynyle en $C_{2-4}$, cyanoalkyle en $C_{1-4}$, -C(=O)-(alkyle en $C_{1-4}$), -C(=O)-halogénoalkyle en $C_{1-4}$, hydroxyalkyle en $C_{1-4}$, halogénoalkyle en $C_{1-4}$, (alkoxy en $C_{1-4}$)-(alkyle en $C_{1-4}$), (halogénoalkoxy en $C_{1-4}$) - (alkyle en $C_{1-4}$), -C(=O)$NR_7R_8$,-$SO_2$-$NR_7R_8$, -$SO_2$-$R_9$, $R_{11}$, alkyle en $C_{1-4}$ substitué par $R_{11}$, -C(=O)-$R_{11}$ ou -C(=O)-(alkyle en $C_{1-4}$)-$R_{11}$ ;

chacun des radicaux $R_2$ représente indépendamment un atome d'hydrogène ou un groupement alkyle en $C_{1-4}$, alkyle en $C_{1-4}$ substitué par cycloalkyle en $C_{3-6}$, hydroxyalkyle en $C_{1-4}$, (alkoxy en $C_{1-4}$)-(alkyle en $C_{1-4}$), carboxyle, -C(=O)-O-(alkyle en $C_{1-4}$) où le groupement alkyle en $C_{1-4}$ est éventuellement substitué par alkoxy en $C_{1-4}$, -C(=O)-$NH_2$, -C(=O)-NH (alkyle en $C_{1-4}$) où le groupement alkyle en $C_{1-4}$ est éventuellement substitué par alkoxy en $C_{1-4}$, ou -C(=O)-N(alkyle en $C_{1-4}$)$_2$, où chacun des radicaux alkyle en $C_{1-4}$ est éventuellement substitué par alkoxy en $C_{1-4}$ ;

ou $R_1$ et l'un des radicaux $R_2$ forment ensemble un groupement alcanediyle en $C_{1-4}$ ou alcènediyle en $C_{2-4}$, chacun desdits groupements alcanediyle en $C_{1-4}$ et alcènediyle en $C_{2-4}$ étant éventuellement substitué par 1 à 4 substituants, chacun étant indépendamment choisi parmi les groupements hydroxyle, oxo, halogéno, cyano, $N_3$, hydroxyalkyle en $C_{1-4}$, -$NR_7R_8$, -$SO_2$-$NR_7R_8$, -NH-$SO_2$-$NR_7R_8$, -C(=O)-$NR_7R_8$ ou -NH-C (=O) -$NR_7R_8$ ;

ou $R_1$ et $R_{12}$ forment ensemble un groupement alcanediyle en $C_{1-4}$ ou alcènediyle en $C_{2-4}$, chacun desdits groupements alcanediyle en $C_{1-4}$ et alcènediyle en $C_{2-4}$ étant éventuellement substitué par 1 à 4 substituants, chacun étant indépendamment choisi parmi les groupements hydroxyle, oxo, halogéno, cyano, $N_3$, hydroxyalkyle en $C_{1-4}$, -$NR_7R_8$, -$SO_2$-$NR_7R_8$, -NH-$SO_2$-$NR_7R_8$,-C(=O)-$NR_7R_8$ ou -NH-C (=O) -$NR_7R_8$ ;

chacun des radicaux $R_3$ représente indépendamment un atome d'hydrogène ; ou un groupement oxo ; hydroxyle ; carboxyle ; -$NR_{3a}R_{3b}$ ; -C (=O) -$NR_{3a}R_{3b}$ ; hydroxyalkyle en $C_{1-4}$ ; halogénoalkyle en $C_{1-4}$ ; -(C=O)-(alkyle en $C_{1-4}$) ; -C(=O)-O-(alkyle en $C_{1-4}$) où ledit groupement alkyle en $C_{1-4}$ est éventuellement substitué par phényle ; alkyle en $C_{1-4}$ éventuellement substitué par cyano, carboxyle, alkoxy en $C_{1-4}$, -C(=O)-O-(alkyle en $C_{1-4}$) , -O-C(=O)-(alkyle en $C_{1-4}$) , -$NR_{3e}R_{3f}$, -C (=O) -$NR_{3e}R_{3f}$, -$SO_2$-$NR_{3e}R_{3f}$, Q, -C(=O)-Q ou -$SO_2$-Q ; (hydroxyalkoxy en $C_{1-4}$) - (alkyle en $C_{1-4}$) ; (alkoxy en $C_{1-4}$) - (hydroxyalkyle en $C_{1-4}$) ; (hydroxyalkoxy en $C_{1-4}$) - (hydroxyalkyle en $C_{1-4}$) ; ou (alkoxy en $C_{1-4}$) - (alkyle en $C_{1-4}$) éventuellement substitué par cyano, carboxyle, alkoxy en $C_{1-4}$, -C(=O) - O-(alkyle en $C_{1-4}$) , -O-C(=O) - (alkyle en $C_{1-4}$) , -$NR_{3e}R_{3f}$, - C (=O) -$NR_{3e}R_{3f}$, -$SO_2$-$NR_{3e}R_{3f}$, $R_{10}$, -C(=O)-$R_{10}$ ou -$SO_2$-$R_{10}$ ; ou deux substituants $R_3$ liés au même atome de carbone forment ensemble un groupement alcanediyle en $C_{2-5}$ ou - $(CH_2)_p$-O-$(CH_2)_p$- ;

chacun des radicaux $R_{3a}$ et $R_{3b}$ représente indépendamment un atome d'hydrogène ; ou un groupement -(C=O)-(alkyle en $C_{1-4}$) ; -$SO_2$-$NR_{3c}R_{3d}$ ; ou alkyle en $C_{1-4}$ éventuellement substitué par alkoxy en $C_{1-4}$ ; ou

$R_{3a}$ et $R_{3b}$ forment ensemble et avec l'atome d'azote auquel ils sont liés un cycle hétérocyclique monocyclique saturé comportant 4 à 7 chaînons ainsi qu'éventuellement 1 ou 2 hétéroatomes supplémentaires choisis parmi N, O or $SO_2$, ledit cycle hétérocyclique étant éventuellement substitué par 1 à 4 substituants, chacun étant indépendamment choisi parmi les groupements alkyle en $C_{1-4}$, halogéno, hydroxyle ou halogénoalkyle en $C_{1-4}$ ;

chacun des radicaux $R_{3c}$ et $R_{3d}$ représente indépendamment un atome d'hydrogène ou un groupement alkyle en $C_{1-4}$ ou - (C=O)- (alkyle en $C_{1-4}$) ; ou

$R_{3c}$ et $R_{3d}$ forment ensemble et avec l'atome d'azote auquel ils sont liés un cycle hétérocyclique monocyclique saturé comportant 4 à 7 chaînons ainsi qu'éventuellement 1 ou 2 hétéroatomes supplémentaires choisis parmi N, O or $SO_2$, ledit cycle hétérocyclique étant éventuellement substitué par 1 à 4 substituants, chacun étant indépendamment choisi parmi les groupements alkyle en $C_{1-4}$, halogéno, hydroxyle ou halogénoalkyle en $C_{1-4}$ ;

chacun des radicaux $R_{3e}$ et $R_{3f}$ représente indépendamment un atome d'hydrogène ou un groupement alkyle en $C_{1-4}$ éventuellement substitué par alkoxy en $C_{1-4}$, - (C=O)-(alkyle en $C_{1-4}$) ou -$SO_2$-$NR_{3c}R_{3d}$ ;

$R_4$ représente un atome d'hydrogène ou un groupement alkyle en $C_{1-4}$ ou (alkoxy en $C_{1-4}$) - (alkyle en $C_{1-4}$) ;

chacun des radicaux $R_{5a}$ représente indépendamment un atome d'hydrogène ou un groupement alkyle en $C_{1-4}$ ; ou

deux substituants $R_{5a}$ liés au même atome de carbone forment ensemble un groupement alcanediyle en $C_{2-5}$ ou - $(CH_2)_p$-O-$(CH_2)_p$- ;

$R_{5a'}$ représente un atome d'hydrogène ou un groupement alkyle en $C_{1-4}$ ;

chacun des radicaux $R_{5b}$ représente indépendamment un atome d'hydrogène ; ou un groupement alkyle en $C_{1-4}$ ; alkyle en $C_{1-4}$ substitué par $NR_{5b1}R_{5b2}$ ; (alkoxy en $C_{1-4}$)-(alkyle en $C_{1-4}$) ; hydroxyalkyle en $C_{1-4}$ ; hydroxyle ;

cycloalkyle en $C_{3-6}$ ; ou phényle éventuellement substitué par alkyle en $C_{1-4}$, halogéno, hydroxyle ou alkoxy en $C_{1-4}$ ; ou deux substituants $R_{5b}$ liés au même atome de carbone forment ensemble un groupement alcanediyle en $C_{2-5}$ ou - $(CH_2)_p$-O-$(CH_2)_p$- ;

chacun des radicaux $R_{5b1}$ et $R_{5b2}$ représente indépendamment un atome d'hydrogène ou un groupement alkyle en $C_{1-4}$ éventuellement substitué par alkoxy en $C_{1-4}$, - (C=O)- (alkyle en $C_{1-4}$) ou -$SO_2$-$NR_{5b3}R_{5b4}$ ;

chacun des radicaux $R_{5b3}$ et $R_{5b4}$ représente indépendamment un atome d'hydrogène ou un groupement alkyle

en $C_{1-4}$ ou - (C=O)- (alkyle en $C_{1-4}$) ; ou
$R_{5b3}$ et $R_{5b4}$ forment ensemble et avec l'atome d'azote auquel ils sont liés un cycle hétérocyclique monocyclique saturé comportant 4 à 7 chaînons ainsi qu'éventuellement 1 ou 2 hétéroatomes supplémentaires choisis parmi N, O or $SO_2$, ledit cycle hétérocyclique étant éventuellement substitué par 1 à 4 substituants, chacun étant indépendamment choisi parmi les groupements alkyle en $C_{1-4}$, halogéno, hydroxyle ou halogénoalkyle en $C_{1-4}$ ;
chacun des radicaux $R_6$ représente indépendamment un atome d'hydrogène ou un groupement halogéno, hydroxyle, carboxyle, cyano, alkyle en $C_{1-4}$, (alkoxy en $C_{1-4}$)-(alkyle en $C_{1-4}$) , hydroxyalkyle en $C_{1-4}$, halogénoalkyle en $C_{1-4}$, alcényle en $C_{2-4}$, alcynyle en $C_{2-4}$, $-NR_{6a}R_{6b}$ ou - $C(=O)NR_{6a}R_{6b}$ ;
chacun des radicaux $R_{6a}$ et $R_{6b}$ représente indépendamment un atome d'hydrogène ou un groupement alkyle en $C_{1-4}$ ;
chacun des radicaux $R_7$ et $R_8$ représente indépendamment un atome d'hydrogène ou un groupement alkyle en $C_{1-4}$, halogénoalkyle en $C_{1-4}$ ou cycloalkyle en $C_{3-6}$ ; ou
$R_7$ et $R_8$ forment ensemble et avec l'atome d'azote auquel ils sont liés un cycle hétérocyclique monocyclique saturé comportant 4 à 7 chaînons ainsi qu'éventuellement 1 hétéroatome supplémentaire choisi parmi N, O or $SO_2$, ledit cycle hétérocyclique étant éventuellement substitué par 1 à 4 substituants, chacun étant indépendamment choisi parmi les groupements alkyle en $C_{1-4}$, halogéno, hydroxyle ou halogénoalkyle en $C_{1-4}$ ;
Rg représente un groupement alkyle en $C_{1-4}$, halogénoalkyle en $C_{1-4}$ ou cycloalkyle en $C_{3-6}$ ;
chacun des radicaux $R_{10}$ représente indépendamment un cycle hétérocyclique monocyclique saturé comportant 4 à 7 chaînons ainsi que jusqu'à 2 hétéroatomes choisis parmi N, O ou $SO_2$, ledit cycle hétérocyclique étant éventuellement substitué par 1 à 4 substituants, chacun étant indépendamment choisi parmi les groupements alkyle en $C_{1-4}$, halogéno, hydroxyle ou halogénoalkyle en $C_{1-4}$ ;
chacun des radicaux $R_{11}$ représente indépendamment un groupement cycloalkyle en $C_{3-6}$, phényle ou un cycle hétérocyclique monocyclique comportant 4 à 7 chaînons ainsi que jusqu'à 3 hétéroatomes choisis parmi N, O ou $SO_2$, ledit cycle hétérocyclique étant éventuellement substitué par 1 à 4 substituants, chacun étant indépendamment choisi parmi les groupements alkyle en $C_{1-4}$, halogéno, hydroxyle ou halogénoalkyle en $C_{1-4}$ ;
chacun des radicaux $R_{12}$ représente indépendamment un atome d'hydrogène ou un groupement alkyle en $C_{1-4}$ ;
Q représente un cycle hétérocyclique monocyclique saturé comportant 4 à 7 chaînons ainsi que jusqu'à 3 hétéroatomes choisis parmi N, O ou $SO_2$, ledit cycle hétérocyclique étant éventuellement substitué par 1 à 4 substituants, chacun étant indépendamment choisi parmi les groupements alkyle en $C_{1-4}$, halogéno, hydroxyle ou halogénoalkyle en $C_{1-4}$ ;
n représente un entier d'une valeur de 1 ou de 2 ;
m représente un entier d'une valeur de 1 ou de 2 ;
p représente un entier d'une valeur de 1 ou de 2 ;
p1 représente un entier d'une valeur de 1 ou de 2 ;
chacun des nombres p2 représente indépendamment un entier d'une valeur de 0, 1 ou 2 ;
r représente un entier d'une valeur de 0, 1 ou 2 ;
chacun des nombres $p_3$ représente indépendamment un entier d'une valeur de 0 ou de 1 ;
chacun des nombres s représente indépendamment un entier d'une valeur de 0, 1 ou 2 ;
ou N-oxyde, sels d'addition pharmaceutiquement acceptable ou solvate de celui-ci.

2. Composé selon la revendication 1, où chacun des radicaux $X_a$, $X_b$ et $X_c$ représente indépendamment CH ou N ;
$-X_1-$ représente un groupement - $(CHR_{12})_s-NR_1-X_e-$(alcanediyle en $C_{1-4}$)-$(SO_2)_{p3}-$ ;
$-X_e-$ représente $-C(R_2)_2-$ ;
a représente $-NR_4-C(=O)-[C(R_{5b})_2]_r-$ ou $-NR_4-C(R_{5b})_2-C(=O)-$;
b représente

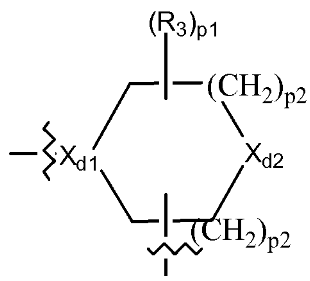

,

où ledit cycle b peut contenir des liaisons supplémentaires pour former un système cyclique ponté choisi parmi les

groupements 2,5-diazabicyclo[2.2.2]octanyle, 3,8-diazabicyclo[3.2.1]octanyle, 3,6-diazabicyclo[3.1.1]heptanyle, 3,9-diazabicyclo[3.3.1]nonyle ;

$X_{d1}$ représente CH ou N ;
$X_{d2}$ représente NH ;
à la condition qu'au moins l'un des radicaux $X_{d1}$ et $X_{d2}$ représente un atome d'azote ;
c représente une liaison ou un groupement $-[C(R_{5a})_2]_m-$,-O-, $-NR_{5a'}-$ ;
le cycle

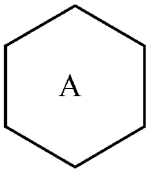

représente un groupement phényle ou pyridyle ;
$R_1$ représente un atome d'hydrogène ou un groupement alkyle en $C_{1-4}$, alcényle en $C_{2-4}$, alcynyle en $C_{2-4}$, cyanoalkyle en $C_{1-4}$, -C (=O) - (alkyle en $C_{1-4}$) , -C(=O)-halogénoalkyle en $C_{1-4}$, halogénoalkyle en $C_{1-4}$,-C (=O) $NR_7R_8$, $-SO_2-NR_7R_8$, $-SO_2-R_9$, $R_{11}$, alkyle en $C_{1-4}$ substitué par $R_{11}$, $-C(=O)-R_{11}$ ou -C(=O)-(alkyle en $C_{1-4}$)-$R_{11}$ ;
chacun des radicaux $R_2$ représente indépendamment un atome d'hydrogène ou un groupement alkyle en $C_{1-4}$, alkyle en $C_{1-4}$ substitué par cycloalkyle en $C_{3-6}$, hydroxyalkyle en $C_{1-4}$, (alkoxy en $C_{1-4}$) - (alkyle en $C_{1-4}$), carboxyle, -C(=O)-O-(alkyle en $C_{1-4}$) où le groupement alkyle en $C_{1-4}$ est éventuellement substitué par alkoxy en $C_{1-4}$, $-C(=O)-NH_2$, -C(=O)-NH(alkyle en $C_{1-4}$) où le groupement alkyle en $C_{1-4}$ est éventuellement substitué par alkoxy en $C_{1-4}$, ou -C(=O)-N(alkyle en $C_{1-4}$)$_2$, où chacun des radicaux alkyle en $C_{1-4}$ est éventuellement substitué par alkoxy en $C_{1-4}$ ;
ou $R_1$ et l'un des radicaux $R_2$ forment ensemble un groupement alcanediyle en $C_{3-4}$ ou alcènediyle en $C_{3-4}$, chacun desdits groupements alcanediyle en $C_{3-4}$ et alcènediyle en $C_{3-4}$ étant éventuellement substitué par 1 à 4 substituants, chacun étant indépendamment choisi parmi les groupements hydroxyle, oxo, halogéno, cyano, $N_3$, hydroxyalkyle en $C_{1-4}$, $-NR_7R_8$, $-SO_2-NR_7R_8$, $-NH-SO_2-NR_7R_8$, $-C(=O)-NR_7R_8$ ou -NH-C (=O) $-NR_7R_8$ ;
chacun des radicaux $R_3$ représente indépendamment un atome d'hydrogène ; ou un groupement oxo ; hydroxyle ; carboxyle ; $-NR_{3a}R_{3b}$ ; -C (=O) $-NR_{3a}R_{3b}$ ; hydroxyalkyle en $C_{1-4}$ ; halogénoalkyle en $C_{1-4}$ ; - (C=O)-(alkyle en $C_{1-4}$) ; -C(=O)-O-(alkyle en $C_{1-4}$) où ledit groupement alkyle en $C_{1-4}$ est éventuellement substitué par phényle ; alkyle en $C_{1-4}$ éventuellement substitué par cyano, carboxyle, alkoxy en $C_{1-4}$, -C(=O)-O-(alkyle en $C_{1-4}$), -O-C(=O)-(alkyle en $C_{1-4}$), $-NR_{3e}R_{3f}$, $-C(=O)-NR_{3e}R_{3f}$, $-SO_2-NR_{3e}R_{3f}$, Q, -C(=O)-Q ou $-SO_2$-Q ; (hydroxyalkoxy en $C_{1-4}$) - (alkyle en $C_{1-4}$) ; (alkoxy en $C_{1-4}$) - (hydroxyalkyle en $C_{1-4}$) ; (hydroxyalkoxy en $C_{1-4}$) - (hydroxyalkyle en $C_{1-4}$) ; ou (alkoxy en $C_{1-4}$) - (alkyle en $C_{1-4}$) éventuellement substitué par cyano, carboxyle, alkoxy en $C_{1-4}$, -C(=O) - O-(alkyle en $C_{1-4}$) , -O-C(=O)-(alkyle en $C_{1-4}$) , $-NR_{3e}R_{3f}$, - C (=O) $-NR_{3e}R_{3f}$, $-SO_2-NR_{3e}R_{3f}$, $R_{10}$, $-C(=O)-R_{10}$ ou $-SO_2-R_{10}$ ; ou deux substituants $R_3$ liés au même atome de carbone forment ensemble un groupement alcanediyle en $C_{2-5}$ ou - $(CH_2)_p-O-(CH_2)_p$- ;
chacun des radicaux $R_{3a}$ et $R_{3b}$ représente indépendamment un atome d'hydrogène ; ou un groupement -(C=O)-(alkyle en $C_{1-4}$) ; $-SO_2-NR_{3c}R_{3d}$ ; ou alkyle en $C_{1-4}$ éventuellement substitué par alkoxy en $C_{1-4}$ ; ou
$R_{3a}$ et $R_{3b}$ forment ensemble et avec l'atome d'azote auquel ils sont liés un cycle hétérocyclique monocyclique saturé comportant 4 à 7 chaînons ainsi qu'éventuellement 1 ou 2 hétéroatomes supplémentaires choisis parmi N, O or $SO_2$, ledit cycle hétérocyclique étant éventuellement substitué par 1 à 4 substituants, chacun étant indépendamment choisi parmi les groupements alkyle en $C_{1-4}$, halogéno, hydroxyle ou halogénoalkyle en $C_{1-4}$ ;
chacun des radicaux $R_{3c}$ et $R_{3d}$ représente indépendamment un atome d'hydrogène ou un groupement alkyle en $C_{1-4}$ ou - (C=O)- (alkyle en $C_{1-4}$) ; ou
$R_{3c}$ et $R_{3d}$ forment ensemble et avec l'atome d'azote auquel ils sont liés un cycle hétérocyclique monocyclique saturé comportant 4 à 7 chaînons ainsi qu'éventuellement 1 ou 2 hétéroatomes supplémentaires choisis parmi N, O or $SO_2$, ledit cycle hétérocyclique étant éventuellement substitué par 1 à 4 substituants, chacun étant indépendamment choisi parmi les groupements alkyle en $C_{1-4}$, halogéno, hydroxyle ou halogénoalkyle en $C_{1-4}$ ;
chacun des radicaux $R_{3e}$ et $R_{3f}$ représente indépendamment un atome d'hydrogène ou un groupement alkyle en $C_{1-4}$ éventuellement substitué par alkoxy en $C_{1-4}$, - (C=O) - (alkyle en $C_{1-4}$) ou $-SO_2-NR_{3c}R_{3d}$ ;
$R_4$ représente un atome d'hydrogène ou un groupement alkyle en $C_{1-4}$ ou (alkoxy en $C_{1-4}$) - (alkyle en $C_{1-4}$) ;
chacun des radicaux $R_{5a}$ représente indépendamment un atome d'hydrogène ou un groupement alkyle en $C_{1-4}$ ; ou
deux substituants $R_{5a}$ liés au même atome de carbone forment ensemble un groupement alcanediyle en $C_{2-5}$

ou - $(CH_2)_p$-O- $(CH_2)_p$- ;
$R_{5a'}$ représente un atome d'hydrogène ou un groupement alkyle en $C_{1-4}$ ;
chacun des radicaux $R_{5b}$ représente indépendamment un atome d'hydrogène ; ou un groupement alkyle en $C_{1-4}$ ; alkyle en $C_{1-4}$ substitué par $NR_{5b1}R_{5b2}$ ; (alkoxy en $C_{1-4}$)-(alkyle en $C_{1-4}$) ; hydroxyalkyle en $C_{1-4}$ ; hydroxyle ; cycloalkyle en $C_{3-6}$ ; ou phényle éventuellement substitué par alkyle en $C_{1-4}$, halogéno, hydroxyle ou alkoxy en $C_{1-4}$ ; ou deux substituants $R_{5b}$ liés au même atome de carbone forment ensemble un groupement alcanediyle en $C_{2-5}$ ou - $(CH_2)_p$-O-$(CH_2)_p$- ;
chacun des radicaux $R_{5b1}$ et $R_{5b2}$ représente indépendamment un atome d'hydrogène ou un groupement alkyle en $C_{1-4}$ éventuellement substitué par alkoxy en $C_{1-4}$, - (C=O) - (alkyle en $C_{1-4}$) ou -$SO_2$-$NR_{5b3}R_{5b4}$ ;
chacun des radicaux $R_{5b3}$ et $R_{5b4}$ représente indépendamment un atome d'hydrogène ou un groupement alkyle en $C_{1-4}$ ou - (C=O) - (alkyle en $C_{1-4}$) ; ou
$R_{5b3}$ et $R_{5b4}$ forment ensemble et avec l'atome d'azote auquel ils sont liés un cycle hétérocyclique monocyclique saturé comportant 4 à 7 chaînons ainsi qu'éventuellement 1 ou 2 hétéroatomes supplémentaires choisis parmi N, O or $SO_2$, ledit cycle hétérocyclique étant éventuellement substitué par 1 à 4 substituants, chacun étant indépendamment choisi parmi les groupements alkyle en $C_{1-4}$, halogéno, hydroxyle ou halogénoalkyle en $C_{1-4}$ ;
chacun des radicaux $R_6$ représente indépendamment un atome d'hydrogène ou un groupement halogéno, hydroxyle, carboxyle, cyano, alkyle en $C_{1-4}$, (alkoxy en $C_{1-4}$)-(alkyle en $C_{1-4}$) , hydroxyalkyle en $C_{1-4}$, halogénoalkyle en $C_{1-4}$, alcényle en $C_{2-4}$, alcynyle en $C_{2-4}$, -$NR_{6a}R_{6b}$ ou - C(=O)$NR_{6a}R_{6b}$ ;
chacun des radicaux $R_{6a}$ et $R_{6b}$ représente indépendamment un atome d'hydrogène ou un groupement alkyle en $C_{1-4}$ ;
chacun des radicaux $R_7$ et $R_8$ représente indépendamment un atome d'hydrogène ou un groupement alkyle en $C_{1-4}$, halogénoalkyle en $C_{1-4}$ ou cycloalkyle en $C_{3-6}$ ; ou
$R_7$ et $R_8$ forment ensemble et avec l'atome d'azote auquel ils sont liés un cycle hétérocyclique monocyclique saturé comportant 4 à 7 chaînons ainsi qu'éventuellement 1 hétéroatome supplémentaire choisi parmi N, O or $SO_2$, ledit cycle hétérocyclique étant éventuellement substitué par 1 à 4 substituants, chacun étant indépendamment choisi parmi les groupements alkyle en $C_{1-4}$, halogéno, hydroxyle ou halogénoalkyle en $C_{1-4}$ ;
$R_9$ représente un groupement alkyle en $C_{1-4}$, halogénoalkyle en $C_{1-4}$ ou cycloalkyle en $C_{3-6}$ ;
chacun des radicaux $R_{10}$ représente indépendamment un cycle hétérocyclique monocyclique saturé comportant 4 à 7 chaînons ainsi que jusqu'à 2 hétéroatomes choisis parmi N, O ou $SO_2$, ledit cycle hétérocyclique étant éventuellement substitué par 1 à 4 substituants, chacun étant indépendamment choisi parmi les groupements alkyle en $C_{1-4}$, halogéno, hydroxyle ou halogénoalkyle en $C_{1-4}$ ;
chacun des radicaux $R_{11}$ représente indépendamment un groupement cycloalkyle en $C_{3-6}$, phényle ou un cycle hétérocyclique monocyclique comportant 4 à 7 chaînons ainsi que jusqu'à 3 hétéroatomes choisis parmi N, O ou $SO_2$, ledit cycle hétérocyclique étant éventuellement substitué par 1 à 4 substituants, chacun étant indépendamment choisi parmi les groupements alkyle en $C_{1-4}$, halogéno, hydroxyle ou halogénoalkyle en $C_{1-4}$ ;
chacun des radicaux $R_{12}$ représente indépendamment un atome d'hydrogène ou un groupement alkyle en $C_{1-4}$ ;
Q représente un cycle hétérocyclique monocyclique saturé comportant 4 à 7 chaînons ainsi que jusqu'à 3 hétéroatomes choisis parmi N, O ou $SO_2$, ledit cycle hétérocyclique étant éventuellement substitué par 1 à 4 substituants, chacun étant indépendamment choisi parmi les groupements alkyle en $C_{1-4}$, halogéno, hydroxyle ou halogénoalkyle en $C_{1-4}$ ;
n représente un entier d'une valeur de 1 ou de 2 ;
m représente un entier d'une valeur de 1 ou de 2 ;
p représente un entier d'une valeur de 1 ou de 2 ;
p1 représente un entier d'une valeur de 1 ou de 2 ;
chacun des nombres p2 représente indépendamment un entier d'une valeur de 0, 1 ou 2 ;
r représente un entier d'une valeur de 0, 1 ou 2 ;
chacun des nombres $p_3$ représente indépendamment un entier d'une valeur de 0 ou de 1 ;
chacun des nombres s représente indépendamment un entier d'une valeur de 0, 1 ou 2.

**3.** Composé selon la revendication 1, où chacun des radicaux $X_a$, $X_b$ et $X_c$ représente indépendamment CH ou N ; -$X_1$- représente un groupement - $(CHR_{12})_s$-$NR_1$-$X_e$-(alcanediyle en $C_{1-4}$) - $(SO_2)_{p3}$ ; -$X_e$- représente -$C(R_2)_2$- ;
a représente -$NR_4$-C(=O) - $[C(R_{5b})_2]_r$- ou -$NR_4$-$C(R_{5b})_2$-C(=O)-;
b représente

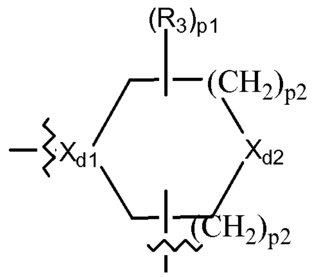

,

où ledit cycle b peut contenir des liaisons supplémentaires pour former un système cyclique ponté choisi parmi les groupements 2,5-diazabicyclo[2.2.2]octanyle, 3,8-diazabicyclo[3.2.1]octanyle ;

$X_{d1}$ représente CH ou N ;
$X_{d2}$ représente NH ;
c représente une liaison ou un groupement $-[C(R_{5a})_2]_m-$,-O-, $-NR_{5a'}-$ ;
le cycle

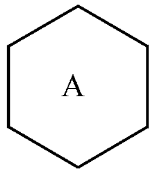

représente un groupement phényle ou pyridyle ;
$R_1$ représente un atome d'hydrogène ou un groupement alkyle en $C_{1-4}$, alcényle en $C_{2-4}$, hydroxyalkyle en $C_{1-4}$, (alkoxy en $C_{1-4}$) - (alkyle en $C_{1-4}$), alkyle en $C_{1-4}$ substitué par $R_{11}$, ou -C (=O)-$R_{11}$ ; en particulier, $R_1$ représente un atome d'hydrogène ou un groupement alkyle en $C_{1-4}$, alcényle en $C_{2-4}$, alkyle en $C_{1-4}$ substitué par $R_{11}$ ou -C (=O) -$R_{11}$ ;
chacun des radicaux $R_2$ représente indépendamment un atome d'hydrogène ou un groupement alkyle en $C_{1-4}$, alkyle en $C_{1-4}$ substitué par cycloalkyle en $C_{3-6}$, carboxyle, -C(=O)-O-(alkyle en $C_{1-4}$), -C(=O)-$NH_2$, -C(=O)-NH(alkyle en $C_{1-4}$) ;
ou $R_1$ et l'un des radicaux $R_2$ forment ensemble un groupement alcanediyle en $C_{1-4}$ ou alcènediyle en $C_{2-4}$, chacun desdits groupements alcanediyle en $C_{1-4}$ et alcènediyle en $C_{2-4}$ étant éventuellement substitué par 1 substituant choisi parmi les groupements hydroxyle, oxo, halogéno, cyano, $N_3$, $-NR_7R_8$, $-NH-SO_2-NR_7R_8$ ;
ou $R_1$ et $R_{12}$ forment ensemble un groupement alcanediyle en $C_{1-4}$ ;
chacun des radicaux $R_3$ représente indépendamment un atome d'hydrogène ; ou un groupement hydroxyalkyle en $C_{1-4}$ ; alkyle en $C_{1-4}$ ; ou (alkoxy en $C_{1-4}$) - (alkyle en $C_{1-4}$) éventuellement substitué par cyano ou $-NR_{3e}R_{3f}$ ; ou deux substituants $R_3$ liés au même atome de carbone forment ensemble un groupement alcanediyle en $C_{2-5}$ ;
chacun des radicaux $R_{3e}$ et $R_{3f}$ représente indépendamment un atome d'hydrogène ou un groupement -(C=O)-(alkyle en $C_{1-4}$) ;
$R_4$ représente un atome d'hydrogène ou un groupement alkyle en $C_{1-4}$ ;
chacun des radicaux $R_{5a}$ représente indépendamment un atome d'hydrogène ou un groupement alkyle en $C_{1-4}$ ; ou
deux substituants $R_{5a}$ liés au même atome de carbone forment ensemble un groupement alcanediyle en $C_{2-5}$ ou - $(CH_2)_p$-O- $(CH_2)_p$- ;
$R_{5a'}$ représente un atome d'hydrogène ou un groupement alkyle en $C_{1-4}$ ;
chacun des radicaux $R_{5b}$ représente indépendamment un atome d'hydrogène ; ou un groupement alkyle en $C_{1-4}$ ; alkyle en $C_{1-4}$ substitué par $NR_{5b1}R_{5b2}$ ; (alkoxy en $C_{1-4}$)-(alkyle en $C_{1-4}$) ; hydroxyalkyle en $C_{1-4}$ ; hydroxyle ; cycloalkyle en $C_{3-6}$ ; ou phényle éventuellement substitué par alkyle en $C_{1-4}$, halogéno, hydroxyle ou alkoxy en $C_{1-4}$ ; ou deux substituants $R_{5b}$ liés au même atome de carbone forment ensemble un groupement alcanediyle en $C_{2-5}$ ou - $(CH_2)_p$-O-$(CH_2)_p$- ;
chacun des radicaux $R_{5b1}$ et $R_{5b2}$ représente indépendamment un atome d'hydrogène ou un groupement -(C=O) - (alkyle en $C_{1-4}$) ;
chacun des radicaux $R_6$ représente indépendamment un atome d'hydrogène ou un groupement halogéno ou - C(=O)$NR_{6a}R_{6b}$ ;

chacun des radicaux $R_{6a}$ et $R_{6b}$ représente indépendamment un atome d'hydrogène ou un groupement alkyle en $C_{1-4}$ ; chacun des radicaux $R_7$ et $R_8$ représente indépendamm1-ent un atome d'hydrogène ;
chacun des radicaux $R_{11}$ représente indépendamment un groupement cycloalkyle en $C_{3-6}$ ;
chacun des radicaux $R_{12}$ représente indépendamment un atome d'hydrogène ou un groupement alkyle en $C_{1-4}$ ;
n représente un entier d'une valeur de 1 ;
m représente un entier d'une valeur de 1 ;
p représente un entier d'une valeur de 1 ;
p1 représente un entier d'une valeur de 1 ou de 2 ;
chacun des nombres p2 représente indépendamment un entier d'une valeur de 0, 1 ou 2 ;
r représente un entier d'une valeur de 1 ;
chacun des nombres $p_3$ représente indépendamment un entier d'une valeur de 0 ou de 1 ;
chacun des nombres s représente indépendamment un entier d'une valeur de 0 ou de 1.

**4.** Composé selon la revendication 1, où
$X_a$ représente N ;
chacun des radicaux $X_b$ et $X_c$ représente CH ;
$R_1$ représente un atome d'hydrogène ou un groupement alkyle en $C_{1-4}$, alcényle en $C_{2-4}$, alcynyle en $C_{2-4}$, cyanoalkyle en $C_{1-4}$, -C(=O)-(alkyle en $C_{1-4}$) , -C(=O)-halogénoalkyle en $C_{1-4}$, halogénoalkyle en $C_{1-4}$,-C(=O) $NR_7R_8$, $-SO_2-NR_7R_8$, $-SO_2-R_9$, $R_{11}$, alkyle en $C_{1-4}$ substitué par $R_{11}$, -C(=O)-$R_{11}$ ou -C(=O)-(alkyle en $C_{1-4}$)-$R_{11}$ ;
chacun des radicaux $R_2$ représente indépendamment un atome d'hydrogène ou un groupement alkyle en $C_{1-4}$, alkyle en $C_{1-4}$ substitué par cycloalkyle en $C_{3-6}$, hydroxyalkyle en $C_{1-4}$, (alkoxy en $C_{1-4}$)-(alkyle en $C_{1-4}$), carboxyle, -C(=O)-O-(alkyle en $C_{1-4}$) où le groupement alkyle en $C_{1-4}$ est éventuellement substitué par alkoxy en $C_{1-4}$, ou $-C(=O)-NH_2$ ; ou
$R_1$ et l'un des radicaux $R_2$ forment ensemble un groupement alcanediyle en $C_{3-4}$ ou alcènediyle en $C_{3-4}$, chacun desdits groupements alcanediyle en $C_{3-4}$ et alcènediyle en $C_{3-4}$ étant éventuellement substitué par 1 à 4 substituants, chacun étant indépendamment choisi parmi les groupements hydroxyle, oxo, halogéno, cyano, $N_3$, hydroxyalkyle en $C_{1-4}$, $-NR_7R_8$, $-SO_2-NR_7R_8$, $-NH-SO_2-NR_7R_8$, $-C(=O)-NR_7R_8$ ou -NH-C (=O) $-NR_7R_8$ ;
$R_{12}$ représente un atome d'hydrogène.

**5.** Composé selon la revendication 1, où
$-X_1-$ représente un groupement $-CH_2-NR_1-CH_2-$ (alcanediyle en $C_{1-4}$) -, $-NR_1-CH_2-$ (alcanediyle en $C_{2-4}$) -, ou $-X_1-$représente l'un des groupements suivants, où $-(CH_2)_2-$est lié à la « variable a » :

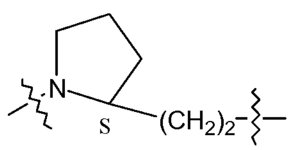 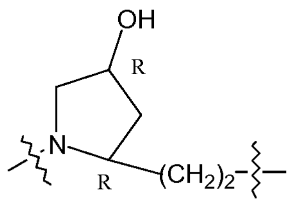 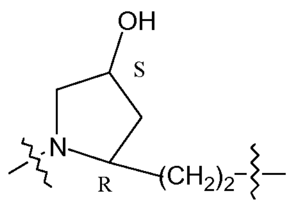

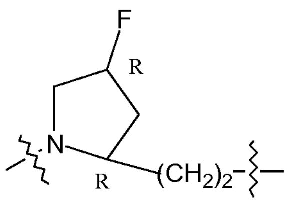 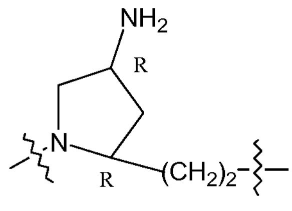 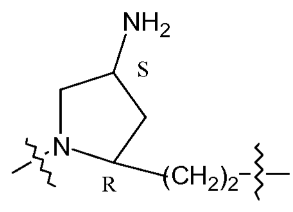

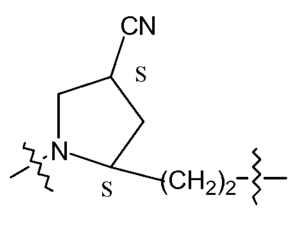 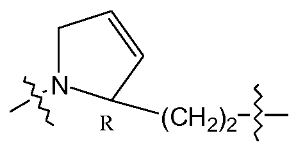 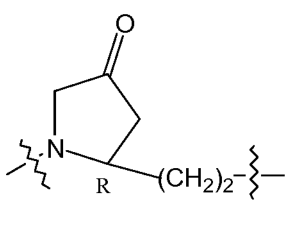

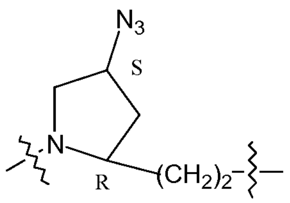 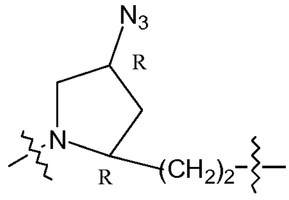 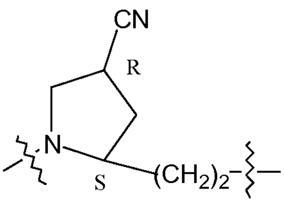

$R_1$ représente un groupement alkyle en $C_{1-4}$, alcényle en $C_{2-4}$, alcynyle en $C_{2-4}$, (alkoxy en $C_{1-4}$)-(alkyle en $C_{1-4}$) ; a représente $-NR_4-C(=O)-[C(R_{5b})_2]_r-$ ou $-NR_4-C(R_{5b})_2-C(=O)-$.

6. Composé selon la revendication 1, où si $R_1$ est lié avec un radical $R_2$, la liaison vers le deuxième substituant $R_2$ est orientée comme illustré ci-après :

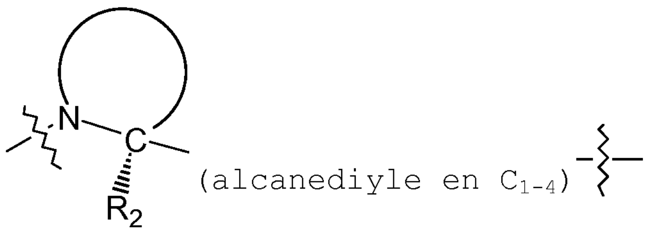

7. Composé selon la revendication 1, où b représente

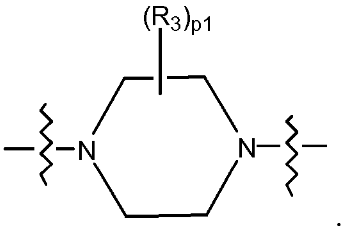

8. Composé selon la revendication 1, où
$R_1$ représente un atome d'hydrogène ou un groupement alkyle en $C_{1-4}$, alcényle en $C_{2-4}$, hydroxyalkyle en $C_{1-4}$, (alkoxy en $C_{1-4}$)-(alkyle en $C_{1-4}$), alkyle en $C_{1-4}$ substitué par $R_{11}$ ou $-C(=O)-R_{11}$ ;
chacun des radicaux $R_2$ représente indépendamment un atome d'hydrogène ou un groupement alkyle en $C_{1-4}$, alkyle en $C_{1-4}$ substitué par cycloalkyle en $C_{3-6}$, carboxyle, -C(=O)-O-(alkyle en $C_{1-4}$), $-C(=O)-NH_2$, -C(=O)-NH (alkyle en $C_{1-4}$) ; ou
$R_1$ et l'un des radicaux $R_2$ forment ensemble un groupement alcanediyle en $C_{1-4}$ ou alcènediyle en $C_{2-4}$, chacun desdits groupements alcanediyle en $C_{1-4}$ et alcènediyle en $C_{2-4}$ étant éventuellement substitué par 1 substituant choisi parmi les groupements hydroxyle, oxo, halo, cyano, $N_3$, $-NR_7R_8$, $-NH-SO_2-NR_7R_8$.

9. Composé selon la revendication 1, où a représente un groupement $-NR_4-C(=O)-[C(R_{5b})_2]_r-$.

10. Composé selon la revendication 1, où c représente $CH_2$.

11. Composé selon l'une quelconque des revendications 1 à 10, où
$X_a$ représente N ;
chacun des radicaux $X_b$ et $X_c$ représente CH.

12. Composition pharmaceutique comprenant un vecteur pharmaceutiquement acceptable et, au titre de principe actif, une quantité thérapeutiquement active d'un composé selon l'une quelconque des revendications 1 à 11.

13. Composé selon l'une quelconque des revendications 1 à 11 pour utilisation en tant que médicament.

14. Composé selon l'une quelconque des revendications 1 à 11 pour utilisation dans le traitement prophylactique ou

thérapeutique d'une maladie ou d'un état pathologique choisis parmi le cancer, la dépression et les troubles de la mémoire et de l'apprentissage.

**15.** Composé selon la revendication 14, où la maladie ou l'état pathologique sont choisis parmi les suivants : glioblastome, médulloblastome, cancer de la prostate, cancer du sein, cancer de l'ovaire et cancer colorectal.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009112439 A
- WO 2009150230 A
- WO 2004105765 A
- WO 2005058318 A
- WO 2005058913 A
- WO 2006061415 A
- WO 2006061417 A
- WO 2009016132 A
- WO 2008155421 A
- WO 2007003525 A
- FR 901228

### Non-patent literature cited in the description


- **BROWNE et al.** *Eur J Biochem.,* 2002, vol. 269 (22), 5360-5368
- **LEPRIVIER et al.** *Cell,* 2013, vol. 153 (5), 1064-1079
- **HAIT et al.** *Clin Cancer Res.,* 2006, vol. 12, 1961-1965
- **JIN et al.** *J Cell Sci.,* 2007, vol. 120 (3), 379-83
- **KAVALALI et al.** *Am J Psychiatry,* 2012, vol. 169 (11), 1150-1156
- **AMARAVADI et al.** *Clin Cancer Res.,* 2011, vol. 17, 654-666
- **BEN-ZVI et al.** *Clin Rev Allergy Immunol.,* 2012, vol. 42 (2), 145-53
- **MAIURI et al.** *Cell Death Differ.,* 2009, vol. 16 (1), 87-93
- *Journal of Medicinal Chemistry,* 1994, vol. 37 (9), 1362-70